# EUROPEAN PATENT SPECIFICATION

(11) **EP 1 105 409 B1**
(45) Date of publication and mention of the grant of the patent: **01.03.2006**
(21) Application number: 00936023.1
(22) Date of filing: 17.05.2000
(51) Int. Cl.: C07K 1/107, C07K 14/135, C07K 14/16, C07K 14/46, C07K 14/575, C07K 14/605, C07K 14/645, C07K 14/655, C12N 9/68

(54) **PROTECTION OF ENDOGENOUS THERAPEUTIC PEPTIDES FROM PEPTIDASE ACTIVITY THROUGH CONJUGATION TO BLOOD COMPONENTS**
SCHUTZ ENDOGENER, THERAPEUTISCH AKTIVER PEPTIDE VOR PEPTIDASEAKTIVITÄT DURCH KONJUGATION AN BLUTBESTANDTEILE
PROTECTION DE PEPTIDES THERAPEUTIQUES ENDOGENES CONTRE L'ACTIVITE PEPTIDASE PAR CONJUGAISON DE COMPOSANTS SANGUINS

(30) Priority: 17.05.1999 US 134406 P; 10.09.1999 US 153406 P; 15.10.1999 US 159783 P
(43) Date of publication of application: 13.06.2001
(60) Divisional application: 05105384.1
(73) Proprietor: ConjuChem, Inc., Montréal, Québec H2X 3Y8 (CA)
(72) Inventor: BRIDON, Dominique, P., Outremont, Quebec H2V 2B2 (CA); EZRIN, Alan, M., Moraga, CA 94556 (US); MILNER, Peter, G., Los Altos Hills, CA 94022 (US); HOLMES, Darren, L., Montreal, Quebec H3G 1T3 (CA); THIBAUDEAU, Karen, Montreal, Quebec H3W 2L5 (CA)
(74) Representative: Larfeldt, Helene
(86) International application number: PCT/US2000/013576
(87) International publication number: WO 2000/069900

(56) References cited:
- EP-A- 0 602 290
- WO-A-95/10302
- WO-A-99/24075
- WO-A-99/24462
- WO-A-99/48536
- US-A- 5 580 853
- US-A- 5 654 276

## Description

### FIELD OF THE INVENTION

This invention relates to modified therapeutic peptides. In particular, this invention relates to protection of endogenous therapeutic peptides from peptidase activity through a modification that enables the peptide to selectively conjugate to blood components, thus protecting the peptide from peptidase activity and increasing the duration of action of the therapeutic peptide for the treatment of various disorders. Specifically, the invention relates to a method of synthesizing such modified therapeutic peptides.

### BACKGROUND OF THE INVENTION

Many endogenous peptides have been described as key components of biological processes. Some of these peptides have been identified as key therapeutic agents for the management of various disorders. In general, endogenous peptides are more desirable as therapeutic agents than synthetic peptides with non-native sequences, because they do not produce an immune response due to their endogenous character. In addition, endogenous peptides are highly specific for their target receptors and are easy to synthesize and manufacture. However, a major difficulty with the delivery of such therapeutic peptides is their short plasma half-life, mainly due to rapid serum clearance and proteolytic degradation via the action of peptidases.

Peptidases break a peptide bond in peptides by inserting a water molecule across the bond. Generally, most peptides are broken down by peptidases in the body in a manner of a few minutes or less. In addition, some peptidases are specific for certain types of peptides, making their degradation even more rapid. Thus, if a peptide is used as a therapeutic agent, its activity is generally reduced as the peptide quickly degrades in the body due to the action of peptidases.

One way to overcome this disadvantage is to administer large dosages of the therapeutic peptide of interest to the patient so that even if some of the peptide is degraded, enough remains to be therapeutically effective. However, this method is quite uncomfortable for the patient. Since most therapeutic peptides cannot be administered orally, the therapeutic peptide would have to be either constantly infused, frequently administered by intravenous injections, or administered frequently by the inconvenient route of subcutaneous injections. The need for frequent administration also results in many potential peptide therapeutics having an unacceptably high projected cost per treatment course. The presence of large amounts of degraded peptide may also generate undesired side effects.

Discomfort in administration and high costs are two reasons why most therapeutic peptides with attractive bioactivity profiles are not developed as drug candidates. Instead, these therapeutic peptides are used as templates for the development of peptidomimetic compounds to substitute for the therapeutic peptide. Biotechnology and large pharmaceutical firms frequently undertake lengthy and expensive optimization programs to attempt to develop non-peptide, organic compounds which mimic the activity seen with therapeutic peptides without incurring an unacceptable side effect profile. For example, cyclic peptides, peptidomimetics and small molecules coming from expensive SAR (Structure Activity Relationship) and molecular modeling studies have led to the development of an incredible amount of peptide mimics. However, these peptide mimics in no way reflect the exact original biological nature of the therapeutic peptide, and thus are inferior to the endogenous therapeutic peptide as therapeutic agents.

An alternative to creating peptide mimics is to block the action of peptidases to prevent degradation of the therapeutic peptide or to modify the therapeutic peptides in such a way that their degradation is slowed down while still maintaining biological activity. Such methods include conjugation with polymeric materials such as dextrans, polyvinyl pyrrolidones, glycopeptides, polyethylene glycol and polyamino acids, conjugation with adroitin sulfates, as well as conjugation with polysaccharides, low molecular weight compounds such as aminolethicin, fatty acids, vitamin B₁₂, and glycosides. These conjugates, however, are still often susceptible to protease activity. In addition, the therapeutic activity of these peptides is often reduced by the addition of the polymeric material. Finally, there is the risk of the conjugates generating an immune response when the material is injected *in vivo.* Several methods include *ex vivo* conjugation with carrier proteins, resulting in the production of randomized conjugates. Since conjugates are difficult to manufacture, and their interest is limited by commercial availability of the carriers, as well as by their poor pharmaco economics.

There is thus a need:for novel methods to modify therapeutic peptides to protect them from peptidase activity and to provide longer duration of action *in vivo*, while maintaining low toxicity yet retaining the therapeutic advantages of the modified peptides.

### PRIOR ART

### US 5,654,276

Described are peptides and peptide mimetics that bind to and the IL-receptor. Such peptides and peptide mimetics are useful in methods for treating disorders that involve improper production of or response to IL-5 and or the production and accumulation of eosinophils, such as asthma, as well as in diagnostic methods employing labeled peptides and peptide mimetics.

### WO 99/24462

Conjugates are prepared from RGD containing peptides, by combining said peptides or analog with a material providing a functionally reactive group capable of reacting with a blood component (preferably a mobile blood cell or endogenous protein). The conjugates may be administered to patients to provide antiplatelet or antiadhesion properties through the inhibition of the binding of fibrinogen to the GPIIb/IIIa receptor, and may also be used as probes for receptor activity. The administration to the patient may be made either in vivo or ex vivo and may be performed by either introducing the RDG containing peptide including the reactive functional group into the patient's vascular system or preparing such a conjugate externally and introducing that conjugate to the patient's vascular system.

### WO 99/48536

Methods of and compositions for localized delivery of therapeutic agents which are capable of forming covalent bonds with a site of interest are disclosed. Therapeutic agents useful in the invention include wound healing agents, antibiotics, anti-inflammatories, anti-oxidants, anti-proliferatives, immunosupressants, anti-infective and anti-cancer agents.

### WO 95/10302

Novel bifunctional reagents useful in providing extended in vivo lifetimes of physiologically active agents are provided. The reagents comprise conjugates of a first binding member specific for a target in a mammalian host, such as a toxin, drug of abuse, microbe, autoreactive immune cell, infected or tumourous cell, antigen presenting cell, or the like, joined to a second binding member specific for along-lived blood component, including cells, such as an erythrocyte, platelet or endothelial cell, and plasma proteins. These conjugates find use by extending the lifetime and availability of the target binding member for coupling the target and the blood component and thereby reducing the concentration free target, modulating the volume of distribution of the target, targeting the target to sites of enhanced immune response, facilitating target clearance from the bloodstream, or extending the stimulation of an immunogen.

### WO 99/24075

Methods and compositions are provided for identifying compounds having affinity or complementarity to a target molecule. Compounds according to the invention may be described by the formula E-Ca-R-Cb-A, wherein E is a therapeutic or diagnostic agent, R is a reactive group, Ca and Cb are connector groups between E and R and between R and A, respectively, and A is a group having an affinity for human serum albumin, wherein affinity group A comprises a sequence of amino acid residues -O1-O2-X1-X2-B in which the amino acid residues are independently selected from the group of all twenty naturally occurring amino acids. Compounds according to the invention may be used for labeling the target molecule, particularly where the target molecule is naturally found in a complex mixture, such as a physiological fluid, like blood. By affinity labeling in vivo, the lifetime of physiologically active entities can be greatly enhanced by becoming bound to long-lived blood components. The covalently bound entity may also serve as an antagonist or agonist of a particular binding protein or as an enzyme inhibitor.

### SUMMARY OF THE INVENTION

This invention is directed to overcoming the problem of peptide degradation in the body by modifying the therapeutic peptide of interest and attaching it to protein carriers, such that the action of peptidases is prevented, or slowed down.

The present invention is directed to modifying and attaching therapeutic peptides to protein carriers, preferentially albumin, through *ex vivo* technology to prevent or reduce the action of peptidases by virtue of a synthetic modification on the first residue to be cleaved. Therapeutic peptides are usually active at the N-terminus portion, at the C-terminus portion, or in an interior portion of the peptide chain. Using the technology of this invention, a site other than the active portion of a therapeutic peptide is modified with certain reactive groups. These reactive groups are capable of forming covalent bonds with functionalities present on blood components. The reactive group is placed at a site such that when the therapeutic peptide is bonded to the blood component, the peptide retains a substantial proportion of the parent compound's activity.

The modification of the therapeutic peptide through the chemical modification used in.the invention is done in such a way that all or most of the peptide specificity is conserved despite attachment to a blood component. This therapeutic peptide-blood component complex is now capable of traveling to various body regions without and being degraded by peptidases, with the peptide still retaining its therapeutic activity. The invention is applicable to all known therapeutic peptides and is easily tested under physiological conditions by the direct comparison of the pharmacokinetic parameters for the free and the modified therapeutic peptide.

The present invention is directed to a method of synthesizing a modified therapeutic peptide capable of forming a peptidase stablilized therapeutic peptide conjugate. The peptide is composed of between 3 and 50 amino acids. The peptide has a carboxy terminal amino acid, an amino terminal amino acid, a therapeutically active region of amino acids and a less therapeutically active region of amino acids. The peptide comprises a reactive group which reacts with amino groups, hydroxyl groups, or thiol groups on blood components to form a stable covalent bond and thereby forms the peptidase stabilized therapeutic peptide. In the peptide of the invention the reactive group is selected from the group consisting of succinimidyl and maleimido groups and the reactive group is attached to an amino acid positioned in the less therapeutically active region of amino acids.

In one embodiment, the therapeutically active region of the peptide includes the carboxy terminal amino acid and the reactive group is attached to said amino terminal amino acid.

In another embodiment, the therapeutically active region of the peptide includes the amino terminal amino acid and the reactive group is attached to the carboxy terminal amino acid.

In another embodiment, the therapeutically active region of the peptide includes the carboxy terminal amino acid and the reactive group is attached to an amino acid positioned between the amino terminal amino acid and the carboxy terminal amino acid.

In yet another embodiment, the therapeutically active region includes the amino terminal amino acid and the reactive group is attached to an amino acid positioned between the amino terminal amino acid and the carboxy terminal amino acid.

The present inventive method of synthesizing the modified therapeutic peptide comprises the following steps; a) choosing a position within the less therapeutically active region; b) if the therapeutic peptide does not contain a cysteine, then the peptide is synthesized from the carboxy terminal amino acid and the reactive group is added to the carboxy terminal amino acid; or if the therapeutic peptide contains only one cysteine, then the cysteine is reacted with a protective group prior to addition of the reactive group to an amino acid in the less therapeutically active region of the peptide; or if the therapeutic peptide containe two cysteines as a disulfide bridge, then the two cysteines are oxidized and the reactive group is added to the amino terminal amino acid, or to the carboxy terminal amino acid, or to an amino acid positioned between the carboxy terminal amino acid and the amino terminal amino acid of the therapeutic peptide; or if the therapeutic peptide contains more than two cysteines as disulfide bridges, the cysteines are sequentially oxidized in the disulfide bridges and the peptide is purified prior to the addition of the reactive groups to the carboxy terminal amino acid; c) forming a stable covalent bond between the reactive group and an amino group, hydroxyl group, or thiol group on albumin *in vivo* or *ex vivo* and providing a peptide-albumin conjugate, thereby generating the peptidase-stabilized therapeutic peptide, while retaining therapeutic activity of the therapeutic peptide; the peptide-albumin conjugate having a higher stability towards peptidase degradation than the therapeutic peptide; and d) analyzing the therapeutic activity of the peptide-albumin conjugate and the stability of the peptide-albumin conjugate towards peptidase degradation; confirming that the peptide-albumin conjugate has a higher stability than the therapeutic peptide; confirming that the peptide-albumin conjugate retains the therapeutic activity of the therapeutic peptide, and confirming the chosen position within said therapeutically less active region. Alternatively, a terminal lysine is added to the carboxy terminal amino acid and the reactive group is added to the terminal lysine.

The present invention is also directed to a method for protecting a therapeutic peptide from peptidase activity in vivo, the peptide being composed of between 3 and 50 amino acids and having a therapeutically active region of amino acids and a less therapeutically active region of amino acids. The method comprises the following steps:
(a) determining the therapeutically active region of amino acids;
(b) modifying the peptide at an amino acid included in the less therapeutically active region of amino acids by attaching a reactive group to the amino acid to form a modified peptide, such that the
modified peptide has therapeutic activity and is capable of forming a covalent bond in vivo with a reactive functionality on a blood component, such as albumin;
(c) forming a covalent bond between the reactive entity and a reactive functionality on albumin to form a peptide-albumin conjugate, thereby protecting the peptide from peptidase activity while retaining therapeutic activity of the therapeutic peptide; and
(d) analyzing the therapeutic activity of the peptide-albumin conjugate and the stability of the peptide-albumin conjugate towards peptidase degradation; confirming that the peptide-albumin conjugate has a higher stability than the terapeutic peptide and confirming that the peptide-albumin conjugate retains the therapeutic activity of the therapeutic peptide. These steps will be performed *ex vivo*.

The peptides useful in the compositions and methods of the present invention include, but are not limited to, the peptides presented in SEQ ID NO:1 to SEQ ID NO:1617.

### DETAILED DESCRIPTION OF THE INVENTION

### DEFINITIONS

To ensure a complete understanding of the invention, the following definitions are provided:
**Reactive Groups**: Reactive groups are entities capable of forming a covalent bond. Such reactive groups are coupled or bonded to a therapeutic peptide of interest. Reactive groups will generally be stable in an aqueous environment and will usually be carboxy, phosphoryl, or convenient acyl group, either as an ester or a mixed anhydride, or an imidate, thereby capable of forming a covalent bond with functionalities such as an amino group, a hydroxy or a thiol at the target site on mobile blood components. For the most part, the esters will involve phenolic compounds, or be thiol esters, alkyl esters, phosphate esters, or the like. Reactive groups include succimidyl and maleimido groups.
**Functionalities**: Functionalities are groups on blood components, including mobile and fixed proteins, to which reactive groups on modified therapeutic peptides react to form covalent bonds. Functionalities usually include hydroxyl groups for bonding to ester reactive groups, thiol groups for bonding to maleimides, imidates and thioester groups; amino groups for bonding to activated carboxyl, phosphoryl or any other acyl groups on reactive groups.
**Blood Components:** Blood components may be either fixed or mobile. Fixed blood components are non-mobile blood components and include tissues, membrane receptors, interstitial proteins, fibrin proteins, collagens, platelets, endothelial cells, epithelial cells and their associated membrane and membraneous receptors, somatic body cells, skeletal and smooth muscle cells, neuronal components, osteocytes and osteoclasts and all body tissues especially those associated with the circulatory and lymphatic systems. Mobile blood components are blood components that do not have a fixed situs for any extended period of time, generally not exceeding 5, more usually one minute. These blood components are not membrane-associated and are present in the blood for extended periods of time and are present in a minimum concentration of at least 0.1 µg/ml. Mobile blood components include serum albumin, transferrin, ferritin and immunoglobulins such as IgM and IgG. The half-life of mobile blood components is at least about 12 hours.
**Protective Groups:** Protective groups are chemical moieties utilized to protect peptide derivatives from reacting with themselves. Various protective groups are disclosed herein and in U.S. 5,493,007 which is hereby incorporated by reference. Such protective groups include acetyl, fluorenylmethyloxycarbonyl (Fmoc), t-butyloxycarbonyl (Boc), benzyloxycarbonyl (Cbz), and the like. The specific protected amino acids are depicted in Table 1.
**Linking Groups**: Linking groups are chemical moieties that link or connect reactive groups to therapeutic peptides. Linking groups may comprise one or more alkyl groups, alkoxy group, alkenyl group, alkynyl group or amino group substituted by alkyl groups, cycloalkyl group, polycyclic group, aryl groups, polyaryl groups, substituted aryl groups, heterocyclic groups, and substituted heterocyclic groups. Linking groups may also comprise poly ethoxy aminoacids such as AEA ((2-amino) ethoxy acetic acid) or a preferred linking group AEEA ([2-(2-amino)ethoxy)]ethoxy acetic acid). A preferred linking group is aminoethoxyethoxyacetic acid (AEEA).
**Sensitive Functional Groups** - A sensitive functional group is a group of atoms that represents a potential reaction site on a therapeutic peptide. If present, a sensitive functional group may be chosen as the attachment point for the linker-reactive group modification. Sensitive functional groups include but are not limited to carboxyl, amino, thiol, and hydroxyl groups.
**Modified Therapeutic Peptides -** A modified therapeutic peptide peptide is a therapeutic peptide that has been modified by attaching a reactive group, and is capable of forming a peptidase stabilized peptide through conjugation to blood components. The reactive group may be attached to the therapeutic peptide either via a linking group, or optionally without using a linking group. It is also contemplated that one or more additional amino acids may be added to the therapeutic peptide to facilitage the attachment of the reactive group. Modified peptides may be administered *in vivo* such that conjugation with blood components occurs *in vivo*, or they may be first conjugated to blood components *in vitro* and the resulting peptidase stabilized peptide (as defined below) administered *in vivo*. The terms "modified therapeutic peptide" and "modified peptide" may be used interchangeably in this application.
**Peptidase Stabilized Therapeutic Peptides** - A peptidase stabilized therapeutic peptide is a modified peptide that has been conjugated to a blood component via a covalent bond formed between the reactive group of the modified peptide and the functionalities of the blood component, with or without a linking group. Peptidase stabilized peptides are more stable in the presence of peptidases *in vivo* than a non-stabilized peptide. A peptidase stabilized therapeutic peptide generally has an increased half life of at least 10-50% as compared to a non-stabilized peptide of identical sequence. Peptidase stability is determined by comparing the half life of the unmodified therapeutic peptide in serum or blood to the half life of a modified counterpart therapeutic peptide in serum or blood. Half life is determined by sampling the serum or blood after administration of the modified and non-modified peptides and determining the activity of the peptide. In addition to determining the activity, the length of the therapeutic peptide may also be measured.
**Therapeutic Peptides** - As used in this invention, therapeutic peptides are amino acid chains of between 2-50 amino acids with therapeutic activity, as defined below. Each therapeutic peptide has an amino terminus (also referred to as N-terminus or amino terminal amino acid), a carboxyl terminus (also referred to as C-terminus terminal carboxyl terminal amino acid) and internal amino acids located between the amino terminus and the carboxyl terminus. The amino terminus is defined by the only amino acid in the therapeutic peptide chain with a free α-amino group. The carboxyl terminus is defined by the only amino acid in the therapeutic peptide chain with a free α-carboxyl group.

Therapeutic peptides used in the present invention contain a therapeutically active region generally located at the amino terminus, at the carboxyl terminus, or at an internal amino acid. The therapeutically active region may be identified using blind or structure activity relationship (SAR) driven substitution, as defined in more detail in this application. SAR is an analysis which defines the relationship between the structure of a molecule and its pharmacological activity for a series of compounds. Alternatively, where the therapeutically active region has previously been defined and is available in the literature, it may be obtained by referring to references such as scientific journals. Knowledge of the location of the therapeutically active region of the peptide is important for modifying the therapeutic peptide, as defined in more detail below.

Therapeutic peptides used in this invention also contain a less therapeutically active region generally located at the amino terminus, at or near the carboxyl terminus, or at or near an internal amino acid. The less therapeutically active region is a region of amino acids that does not coincide with the therapeutically active region of the therapeutic peptide. The less therapeutically active reion is generally located away from the therapeutically active region, such that modification at the less therapeutically active region does not substantially affect the therapeutic activity of the therapeutic peptide. For example, if the therapeutically active region is located at the amino terminus, the therapeutic peptide will be modified at either the carboxyl terminus or at an internal amino acid. Alternatively, if the therapeutically active region is located at the carboxyl terminus, the therapeutic peptide will be modfied at either the amino terminus or at an internal amino acid. Finally, if the therapeutically active region is located at an internal region, the therapeutic peptide will be modified at either the amino terminus or the carboxyl terminus.

"Therapeutic activity" is any activity directed toward healing or curing a biological disorder in a patient. Examples of said therapeutic peptides include pituitary hormones such as vasopressin, oxytocin, melanocyte stimulating hormones, adrenocorticotropic hormones, growth hormones; hypothalamic hormones such as growth hormone releasing factor, corticotropin releasing factor, prolactin releasing peptides, gonadotropin releasing hormone and its associated peptides, luteinizing hormone release hormones, thyrotropin releasing hormone, orexin, and somatostatin; thyroid hormones such as calcitonins, calcitonin precursors, and calcitonin gene related peptides; parathyroid hormones and their related proteins; pancreatic hormones such as insulin and insulin-like peptides, glucagon, somatostatin, pancreatic polypeptides, amylin, peptide YY, and neuropeptide Y; digestive hormones such as gastrin, gastrin releasing peptides, gastrin inhibitory peptides, cholecystokinin, secretin, motilin, and vasoactive intestinal peptide; natriuretic peptides such as atrial natriuretic peptides, brain natriuretic peptides, and C-type natriuretic peptides; neurokinins such as neurokinin A, neurokinin B, and substance P; renin related peptides such as renin substrates and inhibitors and angiotensins; endothelins, including big endothelin, endothelin A receptor antagonists, and sarafotoxin peptides; and other peptides such as adrenomedullin peptides, allatostatin peptides, amyloid beta protein fragments, antibiotic and antimicrobial peptides, apoptosis related peptides, bag cell peptides, bombesin, bone Gla protein peptides, CART peptides, chemotactic peptides, cortistatin peptides, fibronectin fragments and fibrin related peptides, FMRF and analog peptides, galanin and related peptides, growth factors and related peptides, Gtherapeutic peptide-binding protein fragments, guanylin and uroguanylin, inhibin peptides, interleukin and interleukin receptor proteins, laminin fragments, leptin fragment peptides, leucokinins, mast cell degranulating peptides, pituitary adenylate cyclase activating polypeptides, pancreastatin, peptide T, polypeptides, virus related peptides, signal transduction reagents, toxins, and miscellaneous peptides such as adjuvant peptide analogs, alpha mating factor, antiarrhythmic peptide, antifreeze polypeptide, anorexigenic peptide, bovine pineal antireproductive peptide, bursin, C3 peptide P16, tumor necrosis factor, cadherin peptide, chromogranin A fragment, contraceptive tetrapeptide, conantokin G, conantokin T, crustacean cardioactive peptide, C-telopeptide, cytochrome b588 peptide, decorsin, delicioius peptide, delta-sleep-inducing peptide, diazempam-binding inhibitor fragment, nitric oxide synthase blocking peptide, OVA peptide, platelet calpain inhibitor (P1), plasminogen activator inhibitor 1, rigin, schizophrenia related peptide, serum thymic factor, sodium potassium Atherapeutic peptidease inhibiro-1, speract, sperm activating peptide, systemin, thrombin receptor agonist, thymic humoral gamma2 factor, thymopentin, thymosin alpha 1, thymus factor, tuftsin, adipokinetic hormone, uremic pentapeptide and other therapeutic peptides.

Taking into account these definitions, the focus of this invention is to modify therapeutic peptides to protect them from peptidase activity *in vivo* and thereby extend the effective therapeutic life of the therapeutic peptide in question as compared to administration of the peptide per se to a patient.

### 1. Therapeutic Peptides Used in the Present Invention

Peptide fragments chosen from the determined amino acid sequence of a therapeutic peptide as provided in the attached SEQUENCE LISTING constitute the starting point in the development comprising the present invention. The peptides range from 2 to 50 amino acids in length. The interchangeable terms "peptide fragment" and "peptide moiety" are meant to include both synthetic and naturally occurring amino acid sequences derivable from a naturally occurring amino acid sequence.

In one embodiment, peptide and peptide fragments are synthesized by conventional means, either by bench-top methods or by automated peptide synthesis machines as discussed in detail below. However, it is also possible to obtain fragments of the peptides by fragmenting the naturally occurring amino acid sequence, using, for example, a proteolytic enzyme. Further, it is possible to obtain the desired fragments of the therapeutic peptide through the use of recombinant DNA technology, as disclosed by Maniatis, T., et al., Molecular Biology: A Laboratory Manual, Cold Spring Harbor, New York (1982), which is hereby incorporated by reference. The use of other new modifications to existing methodologies is also contemplated.

The present invention includes peptides which are derivable from the naturally occuring sequence of the therapeutic peptide. A peptide is said to be "derivable from a naturally occurring amino acid sequence" if it can be obtained by fragmenting a naturally occurring sequence, or if it can be synthesized based upon a knowledge of the sequence of the naturally occurring amino acid sequence or of the genetic material (DNA or RNA) which encodes this sequence. Included within the scope of the present invention are those molecules which are said to be "derivatives" of a peptide. Such a "derivative" has the following characteristics: (1) it shares substantial homology with the therapeutic peptide or a similarly sized fragment of the peptide and (2) it is capable of functioning with the same therapeutic activity as the peptide.

A derivative of a peptide is said to share "substantial homology" with the peptide if the amino acid sequences of the derivative is at least 80%, and more preferably at least 90%, and most preferably at least 95%, the same as that of either the peptide or a fragment of the peptide having the same number of amino acid residues as the derivative.

The derivatives of the present invention include fragments which, in addition to containing a sequence that is substantially homologous to that of a naturally occurring therapeutic peptide may contain one or more additional amino acids at their amino and/or their carboxy termini as discussed in detail below. Thus, the invention pertains to polypeptide fragments of the therapeutic peptide that may contain one or more amino acids that may not be present in a naturally occurring therapeutic peptide sequence provided that such fragments have a therapeutic activity which exceeds that of the therapeutic peptide.

Similarly, the invention includes polypeptide fragments which, although containing a sequence that is substantially homologous to that of a naturally occurring therapeutic peptide, may lack one or more additional amino acids at their amino and/or their carboxy termini that are naturally found on the therapeutic peptide. Thus, the invention pertains to polypeptide fragments of the therapeutic peptide that may lack one or more amino acids that are normally present in the naturally occurring peptide sequence provided that such polypeptides have a therapeutic activity which exceeds that of the therapeutic peptide.

The invention also encompasses the obvious or trivial variants of the above-described fragments which have inconsequential amino acid substitutions (and thus have amino acid sequences which differ from that of the natural sequence) provided that such variants have an activity which is substantially identical to that of the above-described derivatives. Examples of obvious or trivial substitutions include the substitution of one basic residue for another (i.e. Arg for Lys), the substitution of one hydrophobic residue for another (i.e. Leu for Ile), or the substitution of one aromatic residue for another (i.e. Phe for Tyr), etc.

As is known in the art, the amino acid residues may be in their protected or unprotected form, using appropriate amino or carboxyl protecting groups as discussed in detail below. The variable length peptides may be in the form of the free amines (on the N-terminus), or acid-addition salts thereof. Common acid addition salts are hydrohalic acid salts, i.e., HBr, HI, or, more preferably, HCl. Useful cations are alkali or alkaline earth metallic cations (i.e., Na, K, Li, Ca, Ba, etc.) or amine cations (i.e., tetraalkylammonium, trialkylammonium, where alkyl can be C₁C₁₂).

Any peptide having a therapeutic activity may be used in this invention. The following list of peptides provides examples of peptides that may be used in this invention, but is not exhaustive and in no way limits the number or type of peptides that may be used in this invention. These therapeutic peptides and fragments produced from these peptides may be modified according to the present invention, and used therapeutically in the body.

### A. Pituitary Hormones (SEQ ID NOS: 1-72)

**Adrenocortiocotropic Hormones (ACTH, aka corticotropin) (SEQ ID NOS: 1-22)** - The endocrine functions of the adrenal cortex are regulated by an anterior pituitary hormone, ACTH. ACTH, a 39-amino acid peptide is generated in the corticotrophic cells of the anterior pituitary under the control of corticotropin releasing factor. ACTH is derived by post-translational modification from a 241-amino acid precursor known as pro-opiomelanocortin (POMC).

The biological role of ACTH is to maintain the bulk and the viability of the adrenal cortex and to stimulate the production of adrenal cortex steroids, principally cortisol and costicosterone. The mechanism of action of ACTH involves binding to the ACTH receptor followed by activation of adenylate cyclase, elevation of cyclic AMP (cAMP), and increased protein kinase A (PKA) activity of adrenal cortex tissue. The main effect of these events is to increase the activity of a side chain-cleaving enzyme, which converts cholesterol to pregnenolone. Because of the distribution of enzymes in the various adrenal cortex subdivisions, the principal physiological effect of ACTH is production of the glucocorticosteroids.

Aside from its function controlling adrenal cortical activity, ACTH appears to have diverse biological roles including modulation of endocrine and exocrine glands, temperature regulation and influences on nerve regeneration and development. In addition, ACTH and its fragments affect motivation, learning, and behavior. The use of ACTH as a therapeutic agent may thus help the control of these functions. ACTH release from the anterior pituitary is mediated by corticotropin releasing factor (CRF).

**Growth Hormone Peptides (SEQ ID NOS**: **23-24, 45) -** Human placental lactogen (hPL), growth hormones, and prolactin (Prl) comprise the growth hormone family. All have about 200 amino acids, 2 disulfide bonds, and no glycosylation. Although each has special receptors and unique characteristics to their activity, they all possess growth-promoting and lactogenic activity. Mature GH (22,000 daltons) is synthesized in acidophilic pituitary somatotropes as a single polypeptide chain. Because of alternate RNA splicing, a small amount of a somewhat smaller molecular form is also secreted.

There are a number of genetic deficiencies associated with GH. GH-deficient dwarfs lack the ability to synthesize or secrete GH, and these short-statured individuals respond well to GH therapy. Pygmies lack the IGF-1 response to GH but not its metabolic effects; thus in pygmies the deficiency is post-receptor in nature. Finally, Laron dwarfs have normal or excess plasma GH, but lack liver GH receptors and have low levels of circulating IGF-1. The defect in these individuals is clearly related to an inability to respond to GH by the production of IGF-1. The production of excessive amounts of GH before epiphyseal closure of the long bones leads to gigantism, and when GH becomes excessive after epiphyseal closure, acral bone growth leads to the characteristic features of acromegaly. Using GH as a therapeutic agent would aid in treating these disorders, and potentially stimulate growth in other cases of short stature with low or normal GH levels.

**Melanocyte Stimulating Hormones (MSH) (SEQ ID NOS: 25-39) -** Melanocyte stimulating hormone (MSH) is generated in the intermediary pituitary under the control of dopamine. MSH may have important physiological roles in the control of vertebrate pigment cell melanogenesis, neural functioning related to learning and behavior, and fetal development. See Sawyer, T.K. et al., Proc. Nat. Acad. Sci USA, 79, 1751 (1982).

**Oxytocin (SEQ ID NOS: 40-44)** - Oxytocin is involved in the enhancement of lactation, contraction of the uterus, and relaxation of the pelvis prior to childbirth. Oxytocin secretion in nursing women is stimulated by direct neural feedback obtained by stimulation of the nipple during suckling. Its physiological effects include the contraction of mammary gland myoepithelial cells, which induces the ejection of milk from mammary glands, and the stimulation of uterine smooth muscle contraction leading to childbirth. Oxytocin causes myoepithelial cells surrounding secretory acini of mammary glands to contract, pushing milk through ducts. In addition, it stimulates the release of prolactin, and prolactin is trophic on the breast and stimulates acinar formation of milk. A conjugated oxytocin could thus be used to aid lactation and help relax the pelvis prior to birth. It could also be used to prevent post partum uterine hemorrage.

**Vasopressin (ADH) (SEQ ID NOS: 46-72) -** Vasopressin is also known as antidiuretic hormone (ADH), because it is the main regulator of body fluid osmolarity, causing antidiuresis and increase in blood pressure. Vasopressin binds plasma membrane receptors and acts through G-proteins to activate the cyclic AMP/protein kinase A (cAMP/PKA) regulatory system. The secretion of vasopressin is regulated in the hypothalamus by osmoreceptors, which sense water concentration and stimulate increased vasopressin secretion when plasma osmolarity increases. The secreted vasopressin increases the reabsorption rate of water in kidney tubule cells, causing the excretion of urine that is concentrated in Na⁺ and thus yielding a net drop in osmolarity of body fluids. Vasopressin deficiency leads to watery urine and polydipsia, a condition known as diabetes insipidus. Using conjugated vasopressin or vasopressin fragments would thus prevent these disorders and allow the regular maintenance of the body's osmolarity.

### B. Hypothalamic Hormones (Releasing factors)

**Corticotropin Releasing Factor (CRF) & related peptides (SEQ ID NOS: 73-102)** - Corticotrophin-releasing factor (CRF), a 41 amino acid peptide, plays a significant role in coordinating the overall response to stress through actions both in the brain and the periphery. In the brain, CRF is produced and secreted primarily from parvocellular neurons of the paraventricular hypothalamic nucleus. From there, the CRF-containing neurons project to the portal capillary zone of the median eminence and act to stimulate the secretion of adrenocorticotrophic hormone (ACTH), beta-endorphin, and other proopiomelanocortin (POMC)-derived peptides from the pituitary gland. The subsequent ACTH-induced release of adrenal glucocorticoids represents the final stage in the hypothalamic-pituitary-adrenal axis (HPA), which mediates the endocrine response to stress. Besides its neuroendocrine role, CRF also functions as a neurotransmitter and neuromodulator to elicit a wide spectrum of autonomic, behavioral and immune effects to physiological, pharmacological, and pathological stimuli.

Clinical studies indicated that CRF hypersecretion is associated with various diseases, such as major depression, anxiety-related illness, eating disorder, as well as inflammatory disorder. Low levels of CRF have been found in Alzheimer's disease, dementias, obesity, and many endocrine diseases. Therefore, the use of CRF as a therapeutic agent to counter the effects associated with high levels or low levels of CRF will provide a basis for the treatment of diseases that are associated with abnormal CRF levels. Several peptide antagonists and nonpeptide antagonists have been discovered and widely studied, including a-helical CRF(9-41), Astressin, D-PheCRF(12-41) (peptide antagonist) and CP-154526 (nonpeptide antagonist). These CRF antagonists may provide a novel agent for treatment of depression, anxiety and other CRF related illnesses. Conjugated CRF peptides could thus be used to maintain adrenal health and viability during long term steroid use or as anti-inflamatory agents.

**Gonadotropin Releasing Hormone Associated peptides (GAP) (SEQ ID NOS: 103-110) -** GAP is contained in the precursor molecule to gonadotropin-releasing hormone (GnRH). GAP has prolactin inhibiting properties. Gn-RH is a hormone secreted by the hypothalamus that stimulate the release of gonadotrophic hormones follicle stimulating hormone (FSH) and luteinizing hormone (LH). Low levels of circulating sex hormone reduce feedback inhibition on GnRH synthesis, leading to elevated levels of FSH and LH. The latter peptide hormones bind to gonadal tissue, resulting in sex hormone production via cyclic AMP (cAMP) and protein kinase A (PKA) mediated pathways. A conjugated GnRM could be used to aid fertility, or as a contraceptive in either males or females. This agent would have use in animals as well as humans.

**Growth Hormone Releasing Factor (GRF) (SEQ ID NOS: 111-134)**- GRF is a hypothalamic peptide that plays a critical role in controlling the synthesis and secretion of growth hormone in the anterior pituitary. Some structurally unrelated short peptides have also been reported to elicit growth hormone secretion by a different mechanism.

Under the influence of GRF, growth hormone is released into the systemic circulation, causing the target tissue to secrete IGF-1. Growth hormone also has other more direct metabolic effects; it is both hyperglycemic and lipolytic. The principal source of systemic IGF-1 is the liver, although most other tissues secrete and contribute to systemic IGF-1. Liver IGF-1 is considered to be the principal regulator of tissue growth. In particular, the IGF-1 secreted by the liver is believed to synchronize growth throughout the body, resulting in a homeostatic balance of tissue size and mass. IGF-1 secreted by peripheral tissues is generally considered to be autocrine or paracrine in its biological action. The use of a conjugated GRF as a therapeutic agent to increase GH release, would then help treat disorders involving growth functions regulated by GRF.

**Lutenizing Hormone Release Hormones (LH-RH) (SEQ ID NOS: 135-161) -** Luteinizing hormone releasing hormone is the key mediator in the neuroregulation of the secretion of gonadotropins, luteinizing hormone (LH) and follicle stimulating hormone (FSH). LH-RH can modify sexual behavior by regulating plasma gonadotropin and sex steroid levels. See Vale, W.W. et al., Peptides, Structure and Biological Function, Proceedings of the Sixth American Peptide Symposium, Gross, E. and Meienhofer, M., eds., 781 (1979). A conjugated LH-RH agent could be used to stimulate ovulation in humans or animals as an aid to fertility.

**Orexins (SEQ ID NOS: 162-164) -** Orexins are a family of neuropeptides from the hypothalamus that have been recently discovered and characterized. Orexins stimulate appetite and food consumption. Their genes are expressed bilaterally and symmetrically in the lateral hypothalamus, which was earlier determined to be the "feeding center" of the hypothalamus. In contrast, the so-called satiety center is expressed in the ventromedial hypothalamus and is dominated by the leptin-regulated neuropeptide network.

**Prolactin Releasing Peptides (SEQ ID NOS: 65-170)- Prolactin** is produced by acidophilic pituitary lactotropes. Prolactin releasing peptides act on lactotrope to release prolactin. PRL initiates and maintains lactation in mammals, but normally only in mammary tissue that has been primed with estrogenic sex hormones. A conjugated PRP could be used to increase lactation in humans or animals.

**Somatostatin (SEQ ID NOS: 171-201)** - Also known as Growth Hormone Release Inhibiting Factors (GIF), somatostatin is a 14 amino acid peptide is secreted by both the hypothatamus and by d cells of the pancreas (its pancreatic version is discussed below). Somatostatin has been reported to modulate physiological functions at various sites including pituitary, pancreas, gut and brain. It inhibits the release of growth hormone, insulin, and glucagon. It has many biological roles, including: inhibition of basal and stimulated hormone secretion from endocrine and exocrine cells, an effect on locomotor activity and cognitive function, and possible therapeutic value in small cell lung cancer. See Reubi, J. C. et al, Endocrinology, 110, 1049 (1982). A conjugated somatostatin could be used to treat giantism in children or acromegaly in the adult.

**Thyrotropin Releasing Hormone (THR) and Analogs (SEQ ID NOS: 202-214) -** THR stimulates the production of thyroid stimulating hormone (TSH, also known as thyrotropin) and prolactin secretion. In adults, TSH is responsible for up-regulating general protein synthesis and inducing a state of positive nitrogen balance. In the embryo, it is necessary for normal development. Hypothyroidism in the embryo is responsible for cretinism, which is characterized by multiple congenital defects and mental retardation. A conjugated THR could then be used as a therapeutic agent in the treatment of these disorders. It could also be used to treat pituitary causes of thyroid insufficiency or in the diagnosis of human tumors of the thyroid.

### C. Thyroid Hormones

**Calcitonins (CT) & Caltitonins Precursor Peptides (SEQ ID NOS: 215-224) -** Calcitonin (CT) is a 32-amino acid peptide secreted by C cells of the thyroid gland. Calcitonin is employed therapeutically to relieve the symptoms of osteoporosis, although details of its mechanism of action remain unclear. However, it has been observed that CT induces the synthesis of parathyroid hormone (PTH) in isolated cells, which leads *in vivo* to increased plasma Ca²⁺ levels. In addition, CT has been shown to reduce the synthesis of osteoporin (Opn), a protein made by osteoclasts and responsible for attaching osteoclasts to bone. Thus, using conjugated CT as a therapeutic peptide would elevate plasma Ca²⁺ via PTH induction and reduce bone reabsorption by decreasing osteoclast binding to bone.

**Calcitonins Gene Related Peptide (CGRP) (SEQ ID NOS: 225-253)-** CGRP is a 37 amino acid peptide that results from alternative splicing of calcitonin gene transcripts. It exists in at least two forms: alpha-CGRP (or CGRP-I) and beta-CGRP (or CGRP-II). CGRP has considerable homology with amylin and adrenomedullin, and is widely distributed both centrally and peripherally in organs including the skin, the heart, the pancreas, the lungs, and the kidneys. CGRP has many biological roles, affecting the nervous and cardiovascular systems, inflammation and metabolism.

### D. Parathyroid Hormones and Related Proteins

**Parathyroid Hormones (PTH) (SEQ ID NOS: 254-293) -** Parathyroid hormone (PTH) is synthesized and secreted by chief cells of the parathyroid in response to systemic Ca²⁺ levels. It plays a major role in the modulation of serum calcium concentration and thereby affect the physiology of mineral and bone metabolism. The Ca²⁺ receptor of the parathyroid gland responds to Ca²⁺ by increasing intracellular levels of PKC, Ca²⁺ and IP₃; this stage is followed, after a period of protein synthesis, by PTH secretion. The synthesis and secretion of PTH in chief cells is constitutive, but Ca²⁺ regulates the level of PTH in chief cells (and thus its secretion) by increasing the rate of PTH proteolysis when plasma Ca²⁺ levels rise and by decreasing the proteolysis of PTH when Ca²⁺ levels fall. The role of PTH is to regulate Ca²⁺ concentration in extracellular fluids. The feedback loop that regulates PTH secretion therefore involves the parathyroids, Ca²⁺, and the target tissues described below.

PTH acts by binding to cAMP-coupled plasma membrane receptors, initiating a cascade of reactions that culminates in the biological response. The body's response to PTH is complex but is aimed in all tissues at increasing Ca²⁺ levels in extracellular fluids. PTH induces the dissolution of bone by stimulating osteoclast activity, which leads to elevated plasma Ca²⁺ and phosphate. In the kidney, PTH reduces renal Ca²⁺ clearance by stimulating its reabsorption; at the same time, PTH reduces the reabsorption of phosphate and thereby increases its clearance. Finally, PTH acts on the liver, kidney, and intestine to stimulate the production of the steroid hormone 1,25-dihydroxycholecalciferol (calcitriol), which is responsible for Ca²⁺ absorption in the intestine. A conjugated PTH could be used to regulate calcium homeostasis in patients with parathyroid hormone deficiency states. Inhibitor analogues could be used to block PTH action in renal failure or other patients with excessive PTH levels.

**Parathyroid Hormone Related Proteins (PTHrP) (SEQ ID NOS: 294-309) -** Parathyroid hormone-related protein (PTHrP) has received attention as a physiological regulator attenuating chondrocytic differentiation and preventing apoptotic cell death. PTHrP was initially identified as a tumor-derived, secretory protein with structural similarity to parathyroid hormone (PTH), the major regulator of calcium homeostasis. PTH and PTHrP bind to a common G protein-coupled cell surface receptor (PTH/PTHrP or PTH-1 receptor) that recognizes the N-terminal (1-34) region of these peptides. Hence, when tumor-derived PTHrP enters the circulation, it activates receptors in classic PTH target organs such as bone and kidney and elicits PTH-like bioactivity. By promoting bone resorption and inhibiting calcium excretion, circulating PTHrP gives rise to the common paraneoplastic syndrome of malignancy-associated humoral hypercalcemia.

Although initially discovered in tumors, PTHrP was subsequently shown to be expressed in a remarkable variety of normal tissues including the fetal and adult skeleton, where acting in concert with its amino terminal PTH-1 receptor, it serves to regulate cellular growth and differentiation. The anabolic effects of intermittent PTH administration on bone and its therapeutic potential in osteoporosis have been extensively explored. With the recognition that PTHrP is the endogenous ligand for the PTH/PTHrP receptor in osteoblasts, its use as an anabolic agent has also been investigated. Modified PTHrP peptides could be used for similar indications as PTH.

**E. Pancreatic Hormones -** The principal role of the pancreatic hormones is the regulation of whole-body energy metabolism, principally by regulating the concentration and activity of numerous enzymes involved in catabolism and anabolism of the major cell energy supplies.

**Amylin (SEQ ID NOS: 310-335)-** Pancreatic beta-cell hormone amylin is a 37-amino-acid peptide related to CGRP and calcitonin. It is co-secreted with insulin from pancreatic beta-cells. Amylin is deficient with type 1 diabetes mellitus. Amylin appears to work with insulin to regulate plasma glucose concentrations in the bloodstream, suppressing the postherapeutic peptiderandial secretion of glucagon and restraining the rate of gastric emptying. People with diabetes have a deficiency in the secretion of amylin that parallels the deficiency in insulin secretion, resulting in an excessive inflow of glucose into the bloodstream during the postherapeutic peptiderandial period.

While insulin replacement therapy is a cornerstone of diabetes treatment, replacement of the function of both amylin and insulin may allow a more complete restoration of the normal physiology of glucose control. Type 2 diabetes is characterized by islet amyloid deposits, which are primarily composed of the amyloidogenic human form of islet amyloid polypeptide. A conjugated amylin could be used in the management of diabetes to limit post prandial hypergtysemia.

**Glucagon (SEQ ID NOS: 336-376) -** Glucagon is a 29-amino acid hormone synthesized by the a cells of the islets of Langerhans as a very much larger proglucagon molecule. Like insulin, glucagon lacks a plasma carrier protein, and like insulin its circulating half life is also about 5 minutes. As a consequence of the latter trait, the principal effect of glucagon is on the liver, which is the first tissue perfused by blood containing pancreatic secretions. Glucagon binds to plasma membrane receptors and is coupled through G-proteins to adenylate cyclase. The resultant increases in cAMP and PKA reverse all of the effects described above that insulin has on liver. The increases also lead to a marked elevation of circulating glucose, with the glucose being derived from liver gluconeogenesis and liver glycogenolysis. A conjugated glucagon construct could be used to manage brittle diabetes with recurrent hypoglycemia or to prevent or treat iatrogenic hypoglycemia.

**Insulin and Insulin-Like peptides (SEQ ID NOS: 377-382)-**The earliest of these hormones recognized was insulin, a disulfide bonded dipeptide of 21 and 30 amino acids produced by the pancreas, whose major function is to counter the concerted action of a number of hyperglycemia-generating hormones and to maintain low blood glucose levels. Insulin is a member of a family of structurally and functionally similar molecules that include IGF-1, IGF-2, and relaxin. The tertiary structure of all 4 molecules is similar, and all have growth-promoting activities, but the dominant role of insulin is metabolic while the dominant roles of the IGFs and relaxin are in the regulation of cell growth and differentiation.

Insulin is synthesized as a preprohormone in the b cells of the islets of Langerhans. Its signal peptide is removed in the cistemae of the endoplasmic reticulum and it is packaged into secretory vesicles in the Golgi, folded to its native structure, and locked in this conformation by the formation of 2 disulfide bonds. Specific protease activity cleaves the center third of the molecule, which dissociates as C peptide, leaving the amino terminal B peptide disulfide bonded to the carboxy terminal A peptide.

Insulin generates its intracellular effects by binding to a plasma membrane receptor, which is the same in all cells. The receptor is a disulfide-bonded glycoprotein. One function of insulin (aside from its role in signal transduction) is to increase glucose transport in extrahepatic tissue is by increasing the number of glucose transport molecules in the plasma membrane. Glucose transporters are in a continuous state of turnover. Increases in the plasma membrane content of transporters stem from an increase in the rate of recruitment of new transporters into the plasma membrane, deriving from a special pool of preformed transporters localized in the cytoplasm.

In addition to its role in regulating glucose metabolism (and its therapeutic use in treating diabetes), insulin stimulates lipogenesis, diminishes lipolysis, and increases amino acid transport into cells. Insulin also modulates transcription, altering the cell content of numerous mRNAs. It stimulates growth, DNA synthesis, and cell replication, effects that it holds in common with the IGFs and relaxin. A conjugated insulin could thus be used to manage diabetes.

**NeuroPeptide Y (SEQ ID NOS: 383-389) -** Neuropeptide Y (NPY), a peptide with 36 amino acid residues, is one of the most abundant neuropeptides in both the peripheral and the central nervous systems. It belongs to the pancreatic polypeptide family of peptides. Like its relatives, peptide YY (PYY) and pancreatic polypeptide (PP). NPY is bent into hairpin configuration that is important in bringing the free ends of the molecule together for binding to the receptors.

NPY exerts a wide range of effects in the central nervous system (CNS) and the periphery. Its CNS actions include major effects on feeding and energy expenditure, and alterations in heart rate, blood pressure, arousal and mood. In the periphery, NPY causes vasoconstriction and hypertension; it is also found in the gastrointestinal and urogenital tract, implicating its functions by action upon gastrointestinal and renal targets. In recent studies, hypothalamic NPY has been found to play a fundamental role in developing the features of obesity, it is a major transducer in the pathways signalling body fat to the hypothalamus, and in regulating body fat content. Leptin, an obese gene product, has been found to decrease NPY gene expression in obese (ob/ob) mice. Insulin and corticosteroids are also involved in the regulation of hypothalamic NPY synthesis, with insulin decreasing and corticosteroids increasing NPY expression. A conjugated NPY could be used to treat obesity and MODM (Type II DM) in obese patients.

**Pancreatic Polypeptides (PP) (SEQ ID NOS: 390-396)-**Pancreatic polypeptide (PP) is a 36-amino acid hormone produced by F cells within the pancreatic islets and the exocrine pancreas. It is a member of the PP fold family of regulatory peptides, and increases glycogenolysis and regulates gastrointestinal activity. A conjugated pancreatic polypeptide could thus be used to alter absorption and metabolism of foods.

**Peptide YY (SEQ ID NOS: 397-400) -** PYY is a thirty six amino acid long peptide, first isolated from porcine intestinal tissue and mainly localized in intestinal endocrine cells. It has many biological activities, including a range of activities within the digestive system and potent inhibition of intestinal electrolyte and fluid secretion.

**Somatostatin (SEQ ID NOS: 171-201) -** The somatostatin secreted by d cells of the pancreas is a 14-amino acid peptide identical to somatostatin secreted by the hypothalamus. In neural tissue somatostatin inhibits GH secretion and thus has systemic effects. In the pancreas, somatostatin acts a paracrine inhibitor of other pancreatic hormones and thus also has systemic effects. It has been speculated that somatostatin secretion responds principally to blood glucose levels, increasing as blood glucose levels rise and thus leading to down-regulation of glucagon secretion. A conjugated somatostatin could then be used to aid in the management of diabetes.

### F. Digestive Hormones

**Cholecystokinin (CCK) & related peptides (SEQ ID NOS: 401-416) -** CCK is a polypeptide of 33 amino acids originally isolated from pig small intestine that stimulates gallbladder contraction and bile flow and increases secretion of digestive enzymes from pancreas. It exists in multiple forms, including CCK-4 and CCK-8, with the octapeptide representing the dominant molecular species showing the greatest activity. It belongs to the CCK/gastrin peptide family and is distributed centrally in the nervous system and peripherally in the gastrointestinal system. It has many biological roles, including stimulation of pancreatic secretion, gall bladder contraction and intestinal mobility in the GI tract as well as the possible mediation of satiety and painful stimuli. A conjugated CCK could be used in diagnostic studies of the gall bladder or in chronic cholecystisis.

**Gastrin Releasing Peptide (GRP) (SEQ ID NOS: 417-429) -** GRP is a 27-amino acid peptide originally isolated from porcine non-antral gastric tissue, and is the homolog of the frog skin peptide named bombesin growth. It is widely distributed both centrally and peripherally in tissues including brain, lung and gastrointestinal tract It regulates a variety of cell physiological processes including secretion, smooth muscle contraction, neurotransmission and cell growth. A conjugated GRP could be used in the treatment of adynamic ileus or constipation in the elderly.

**Gastrin & related peptides (SEQ ID NOS: 417-429) - Gastrin is** a polypeptide of 17 amino acids produced by stomach antrum, which stimulates acid and pepsin secretion. Gastrin also stimulates pancreatic secretions. Multiple active products are generated from the gastrin precursor, and there are multiple control points in gastrin biosynthesis. Biosynthetic precursors and intermediates (progastrin and Gly-gastrins) are putative growth factors; their products, the amidated gastrins, regulate epithelial cell proliferation, the differentiation of acid-producing parietal cells and histamine-secreting enterochromaffin-like (ECL) cells, and the expression of genes associated with histamine synthesis and storage in ECL cells, as well as acutely stimulating acid secretion. Gastrin also stimulates the production of members of the epidermal growth factor (EGF) family, which in turn inhibit parietal cell function but stimulate the growth of surface epithelial cells. Plasma gastrin concentrations are elevated in subjects with *Helicobacter pylori,* who are known to have increased risk of duodenal ulcer disease and gastric cancer. The use of gastrin or gastrin antagonists as a therapeutic agent may therefore contribute to treating major upper gastrointestinal tract disease.

**Gastrin Inhibitory peptides (SEQ ID NOS: 417-429)** - Gastrin inhibitory peptide is a polypeptide of 43 amino acids that inhibits secretion of gastrin. A conjugated GIP could be used to treat severe peptic ulcer disease.

**Motilin (SEQ ID NOS: 430-433)** - Motilin is a polypeptide of 22 amino acids that controls gastrointestinal muscles. Motitin-producing cells are distributed in the duodenum, upper jejunum, and colorectal adenocarinomas and in midgut carcinoids. Motilin stimulates gut motility.

**Secretin (SEQ ID NOS: 434-441)** - Secretin is a polypeptide of 27 amino acids secreted from duodenum at pH values below 4.5, stimulates pancreatic acinar cells to release bicarbonate and H₂O. Secretin is a neurotransmitter (a chemical messenger) in the neuropeptide group. It is one of the hormones that controls digestion (gastrin and cholecystokinin are the others). It is a polypeptide composed of 27 amino acids and is secreted by cells in the digestive system when the stomach empties. Secretin stimulates the pancreas to emit digestive fluids that are rich in bicarbonate which neutralizes the acidity of the intestines, the stomach to produce pepsin (an enzyme that aids digestion of protein), and the liver to produce bile.

Secretin may be useful in treating autism. In one study, children with autistic spectrum disorders underwent upper gastrointestinal endoscopy and intravenous administration of secretin to stimulate pancreaticobiliary secretion. All three had an increased pancreaticobiliary secretory response when compared with nonautistic patients (7.5 to 10 mL/min versus 1 to 2 mL/min). Within 5 weeks of the secretin infusion, a significant amelioration of the children's gastrointestinal symptoms was observed, as was a dramatic improvement in their behavior, manifested by improved eye contact, alertness, and expansion of expressive language. These clinical observations suggest an association between gastrointestinal and brain function in patients with autistic behavior.

**Vasoactive Intestinal Peptide (VIP) and related peptides (SEQ ID NOS: 442-464)** - VIP is a polypeptide of 28 residues produced by hypothalamus and GI tract. It relaxes the GI, inhibits acid and pepsin secretion, acts as a neurotransmitter in peripheral autonomic nervous system, and increases secretion of H₂O and electrolytes from pancreas and gut. It was originally discovered in lung and intestine and is also found in tissues including brain, liver, pancreas, smooth muscle and lymphocytes. It is structurally related to a family of peptides which include PACAP, PHI, secretin and glucagon. It has a diverse range of biological actions including vasodilation, electrolyte secretion, modulation of immune function and neurotransmission. A conjugated VIP may be useful in the treatment of achlorhydria, ischemic colitis and irritable bowel syndrome (IBS).

**G. Natriuretic Peptides -** There are three members in the natriuretic peptide hormone family, atrial natriuretic peptide (ANP), B-type natriuretic peptide (BNP, brain natriuretic peptide), and C-type natriuretic peptide (CNP), that are involved in the regulation of blood pressure and fluid homeostasis.

**Atrial-Natriuretic Peptides (ANP) (SEQ ID NOS: 465-507)-**ANP is a 28-amino acid peptide hormone containing a disulfide bond. It exerts natriuretic, diuretic, and vasorelaxant effects and play an important role in the body's blood volume and blood pressure homeostasis. See Smith, F.G. et al., J. Dev. Physiol. 12, 55 (1989). The mechanisms controlling ANP release have been the subject of intense research, and are now fairly well understood. The major determinant of ANP secretion is myocyte stretch. Although much less is known about the factors regulating BNP release from the heart, myocyte stretch has also been reported to stimulate BNP release from both atria and ventricles. However, whether wall stretch acts directly or via factors such as endothelin-1, nitric oxide, or angiotensin II liberated in response to distension has not been established. Recent studies show that by stimulating endothelin type A receptors endothelin plays an important physiological role as a mediator of acute-volume load-induced ANP secretion from atrial myocytes in conscious animals. In fact, endogenous paracrine/autocrine factors liberated in response to atrial wall stretch rather than direct stretch appears to be responsible for activation of ANP secretion in response to volume load, as evidenced by almost complete blockade of ANP secretion during combined inhibition of endothelin type A/B and angiotensin II receptors. Furthermore, under certain experimental conditions angiotensin II and nitric oxide may also exert a significant modulatory effect on stretch-activated ANP secretion. The molecular mechanisms by which endothelin-1, angiotensin 11, and nitric oxide synergistically regulate stretch-activated ANP release are yet unclear. Abstract Volume 75 Issue 11/12 (1997) pp 876-885, Journal of Molecular Medicine. A conjugated would be useful in the management of malignant hypertension or severe hypertension and renal failure.

**Brain Natriuretic Peptides (BNP) (SEQ ID NOS: 507-516) -** Brain natriuretic peptide (BNP), a member of the natriuretic peptide family, is produced and released from cardiac ventricles. BNP regulates the body fluid volume, blood pressure, and vascular tones through the A-type guanylate cyclase-coupled receptor. The BNP plays a role in electrolyte-fluid homeostasis such as atrial natriuretic peptides (ANP). A conjugated BNP could be useful in the management of heart failure.

**C-Type Natriuretic Peptides (CNP) (SEQ ID NOS: 517-624)** - C-type natriuretic peptide (CNP), the third member of the natriuretic peptide family, is produced in vascular endothelial cells (ECs) and acts as an endothelium-derived relaxing peptide. Although atrial and brain natriuretic peptides are well known to be involved in the regulation of cardiovascular and endocrine functions as circulating hormones, the roles of the C-type natriuretic peptide (CNP) remain unknown.

CNP is found principally in the central nervous system and vascular endothelial cells while ANP and BNP are cardiac hormones. ANP is synthesized mainly in the atria of the normal adult heart, while BNP is produced by both the atria and ventricles.

**H. Tachykinins (SEQ ID NOS: 525-627) -** A family of peptides, including neurokinin A and substance P, that share a common C terminal sequence (F-X-GLM-NH2) which is required for full biological activity including Neurokinin A, B, and Substance P.

**Neurokinin A** - Neurokinin A is a decapeptide, previously known as substance K. It is is a member of the tachykinin family of neuropeptides which includes substance P and neurokinin B. It exhibits a variety of activities related to smooth muscle contraction, pain transmission, bronchoconstriction, vasodilation and modulation of the immune system.

**Neuromedin**- Neuromedins, smooth-muscle-stimulating peptides, are commonly divided into four groups: bombesin-like, kassinin-like, neurotensin-like and neuromedins U. These neuropeptides and their receptors are localized to all components of the HPA hypophyseal pituitary axis, the only exemption seems to be neurokinin B, which is not detected in the adenohypophysis. Neuromedins exert a manifold effect on HPA axis, and their action on the adrenal suggests their involvement in the regulation of growth, structure and function of the adrenal cortex. Neuromedins may exert both direct and indirect effects on the adrenal cortex. Direct effect is proven by the stimulation of mineralo- and glucocorticoid therapeutic peptides by isolated or cultured adrenocortical cells and by mobilisation of intracellular [Ca2+]. Indirect effects, on the other hand, may be mediated by ACTH, arginine-vasopressin, angiotensin II, catecholamines or by other regulatory substances of medullary origin.

**Substance P and Related Peptides** - Substance P is an eleven amino acid peptide, first isolated from brain and intestine. It has been proposed as a neuromodulator involved in pain transmission in the spinal cord. It also affects contraction of smooth muscle, reduction of blood pressure, stimulation of secretory tissue, and release of histimine from mast cells.

### I. Renin Related Peptides

**Angiotensins (SEQ ID NOS: 628-677)**- Angiotensin is a 10 amino acid peptide derived from enzymatic cleavage of a2-globin by the kidney enzyme renin. The C-terminal 2 amino acids are then released to yield angiotensin I, which is responsible for essential hypertension through stimulated synthesis and release of aldosterone from adrenal cells. It is a multifunctional hormone regulating blood pressure, plasma volume, neuronal function thirst, and water intake.

Angiotensin II is an octapeptide derived from angiotensin I by angiotensin converting enzyme, and is widely distributed both centrally and peripherally in organs such as the heart, the kidneys, and the liver. Angiotensin IV is the temninal hexapeptide fragment of angiotensin II formed metabolically by proteolytic cleavage from either angiotensin I or angiotensin II. It plays a role in vascular control, cardiac growth, renal blood flow and memory function.

Angiotensin II is the key peptide hormone that regulates vascular smooth muscle tone, blood pressure, free water intake and sodium retension. It controls vascular homeostasis compensating for loss of intravascular volume by stimulating increased vasospastic tone, increase sodium retention and increased free water intake.

**Renin substrates and Inhibitors (SEQ ID NOS: 678-684) -** Renin is a very specific aspartic protease, which is synthesized and released by differentiated smooth muscle cells in the vasculature of the kidney called granular epithelial cells. Renin is specific for its substrate, angiotensinogen, which it cleaves specifically at the Leu¹⁰-Val¹¹ bond to form the decapeptide, angiotensin I (Al). The renin-angiotensin system is involved in the control of fluid and mineral balance throughout the vertebrates. Renin can be found in mammals, birds, reptiles, amphibians, bony fishes, cartilaginous fishes, and agnathans. Specific renin inhibitors can also be designed, with therapeutic applications for treatment of for example hypertension and congestive heart failure (Blundell et al., 1987).

**J. Endothelins and Related Peptides (SEQ ID NOS: 685-744) -** The endothelin peptide family consists of the 21 amino acid isoforms endothelin-1, endothelin-2, endothelin-3, sarafotoxin (a snake venom) and scorpion toxin.

**Endothelins (ET) and Big Endothelins -** Endothelins are found on endothelial cells in a wide variety of organ systems. Examples of pathologies and physiological processes associated with changes in endothelin levels and synthetics include: atherosclerosis and hypertension, coronary vasospasm, acute renal failure, changes in intracellular Ca2⁺ levels, and effects on the renin-angiotensin system. Endothelins are released in reponse to variations in angiotensin II, vasopressin, and cytokines (e.g. TGF-βm TNF-α, IL-β-) levels as well as other physiological events including increase blood flow.

The endothelin family of peptides consists of highly potent endogenous vasoconstrictor agents first isolated from endothelial cell supernatant. They regulate blood flow to organs by exporting a vasoconstrictive effect on arteries. Endothelins are derived from big-endothelin, which is cleaved by a unique membrane-bound metalloprotease, endothelin-converting enzyme, into the 21-amino-acid bioactive forms (ET-1, ET-2 and ET-3).

Of the 3 isoforms (ET-1, ET-2, ET-3), endothelin-1 is the major isoform and plays an important role for regulation of vascular function. Endogenous endothelin peptides and their receptors are diferentially distributed throughout the many smooth muscle tissues including blood vessels, uterus, bladder and intestine. Through this widespread distribution and localization, they exert biological functions in regulating vascular tone and causing mitogenesis. ETs and their receptor subtypes are also present in various endocrine organs. It appears to act as a modulator of secretion of prolactin, gonadotropins GH and TSH. Endothelin may also be the disease marker or an etiologic factor in ischemic heart disease, atherosclerosis, congestive heart failure, renal failure, systemic hypertension, pulmonary hypertension, cerebral vasospasm.

Exogenously administered endothelin-1 has been demonstrated to increase peripheral resistance and blood pressure in a dose-dependent manner. However, during the first minutes of intravenous administration endothelins also decrease peripheral resistance and blood pressure, presumably due to the release of vasodilatory compounds such as nitric oxide, prostacyclin, and atrial natriuretic peptide.

**ET(A) Receptor Antagonists** - Endothelin receptors exist as two types: A (ET-A) and B (ET-B1 and ET-B2). ET-A receptors are responsible for while ET-B1 and ET-B2 mediate vasorelaxation and vasoconstriction respectively.

**Sarafotoxin peptides** - As already described, endothelin (ET) peptides are potent growth factors binding to G protein-coupted receptors. Sarafotoxins (S6) isolated from *Atractaspis engaddensis* are highly homologous to endothelins. Sarafotoxin peptides have marked vasoconstrictive activity and are responsible for the ischemic limb loss that follows snake or scorpion bites. They could be used therapeutically as a peptidase stabilized peptide as a vasopressive agent in shock and sepsis.

**K. Opioid Peptides (SEQ ID NOS: 745-927) -** Opioids are a large class of drugs, used clinically as painkillers, that include both plant-derived and synthetic alkaloids and peptides found endogenously in the mammalian brain. While the plant-derived alkaloids have been known and used for thousands of years, the endogenous opioid peptides were discovered only in the mid-1970s.

Opioids include casomorphin peptides, demorphins, endorphins, enkephalins, deltorphins, dynorphins, and analogs and derivatives of these.

**Casomorphin Peptides** - Casomorphin peptides are novel opioid peptides derived from casein(§-casomorphins). Beta casomorphins are the more exstensive studied opioid peptides arising from food proteins (beta-caseins). They were originally isolated from bovine beta-casein, the same sequences occur in ovine and buffalo beta-caseins.

**Dermorphins** - Demorphin is a is a seven amino acid peptide, originally isolated from *Phylomedusa sauvagei* frog skin. It is a ligand which binds with high affinity to the µ opioid receptor, and has many biological roles including analgesia, endocrine modulation, immunomodulation, increased K⁺ conductance and inhibition of action potentials.

**Dynorphin/New-Endorphin Precursor Related Peptides -** Dynorphins are a class of endogenous opioids that exist in multiple forms in the central nervous system. Dynorphins are derived from the precursor prodynorphin (proenkephalin B). Dynorphin, also known as Dynorphin A1-17, is a well known opioid which has the sequence Tyr-Gly-Gly-Phe-Leu⁵-Arg-Arg-Ile-Arg-Pro¹⁰-Lys-Leu-Lys-Trp-Asp¹⁵-Asn-Gln. SEQ ID NO:1. A number of derivatives and analogs of dynorphin are known including Dyn A1-13, SEQ ID NO: 2 Dyn A2-13, SEQ ID NO:3, Dyn A1-12, Dyn A2-12 and Dyn A2-17 as well as amide analogs such as those mentioned in U.S. Patent 4,462,941 of Lee et al., N-terminus truncated dynorphin analogs such as those described in International Patent Application WO 96/06626 of Lee et al. and des-Tyr or des-Tyr Gly analogs such as those disclosed in International Patent Application WO 93/25217 also of Lee et al. The dynorphis are highly potent opioids, and demonstrate selective affinity for the kappa receptor. See Goldstein, A., Peptides, Structure and Function, Proceedings of the 8th American Peptides Symposium, Hruby, V.J. and Rich, D.H., eds., 409 (1983).

**Endorphins** - The endorphis are derived from the precursor protein -lipotropin. They have been found to elicit several biological reactions such as analgesia, behavioral changes and growth hormone release. See Akil, H. et al., Ann. Rev. Neurosci., 7, 223 (1984).

**Enkepalins & related peptides** - Enkephalins and endorphins are neurohormones that inhibit transmission of pain impulses. The activity of neurons in both the central and peripheral nervous systems is affected by a large number of neurohormones that act on cells quite distant from their site of release. Neurohormones can modify the ability of nerve cells to respond to synaptic neurotransmitters. Several small peptides with profound effects on the nervous system have been discovered recently, for example enkephalins (e.g. Met-enkephalin and Leu-enkephalin) and endorphins (e.g. β endorphin). These three contain a common tetrapeptide sequence (Tyr-Gly-Gly-Phe) that is essential to their functions. Enkephalins and endorphins function as natural pain killers or opiates and decrease the pain responses in the central nervous system. See also Akil, H. etl al., Ann. Rev. Neurosci., 7, 223 (1984).

**L. Thymic peptides (SEQ ID NOS: 928-934) -** The thymus is thought to be responsible for the development and regulation of T cell immunity in both infants and adults. The thymus seems to exert its regulatory functions through the secretion of various noncellular, hormonelike products via its epithelial cells, called thymic peptides.

Thymic peptides are reported to have many effects on T cells. Several studies have reported that thymic peptides can assist development of immature, precursor cells into fully competent T cells. Thymic peptides seem to regulate the expression of various cytokine and monokine receptors on T cells and induce secretion of IL-2, interferon alpha, and interferon gamma (disease-fighting substances) when the immune system is challenged. There are reports that the use of thymic hormones in children with immuno-deficiencies caused by chemotherapy has resulted in an increase in circulating T cells, normalization of T cell subsets, and restoration of delayed hypersensitivity reactions.

**Thymopoietin** - Thymopoietin is the largest of the known thymic hormones and consists of 49 amino acids.

**Thymulin** - Previously known as thymic serum factor, thymulin is the smallest of the chemically characterized thymic hormones and consists of 9 amino acids. Thymulin is the hormone responsible for stimulating the production of immune-system T cells

**Thymopentin -** Thymopentin is a small, synthesized thymic peptide drug, also known as therapeutic peptide-5 or Timunox. In the U.S. it is being developed as an AIDS therapy by the Immunobiology Research Institute. Thymopentin has been studied more extensively than most other thymic peptide drugs. At least one study has claimed a significant rise in T cells and slight clinical improvement in those patients who received thymopentin three times a week, compared to untreated control participants. Compared to the 14 untreated control participants, those taking the drug showed greater "immunologic stability" and some clinical improvement.

**Thymosin -** Thymosin is a mixture of 15 or more proteins. One of these proteins is thymosin alpha-1 which consists of 28 amino acids. Thymosin has therapeutic use for the treatment of primary immunodeficiencies and as a booster for influenza vaccine in renal dialysis patients. It is also being tested in ongoing clinical trials for activity against chronic hepatitis B and C, HIV infection, and certain forms of cancer.

**Thyrmic Humoral Factor (THF) -** THF is a thymic peptide currently being examined as an anti- HIV treatment. In preclinical studies in rats with CMV-related immunosuppression, THF restored immune competence through modulation of T cells. In addition, it may have therapeutic use in the treatment of herpes, causing (at least in one study) the viral infection's rapid regression and increase of T-cell populations.

### L. Other Peptides

**Adrenomedullin Peptides (AM) (SEQ ID NOS: 935-945) -** Adrenomedullin is a potent vasodilator peptide that exerts major effects on cardiovascular functions. Its systemic administration causes a rapid and profound fall in blood pressure and an increase in pulmonary blood flow. Its other actions are bronchodilatation, being an inhibitor of drinking behavior and an inhibitor of angiotensin-induced aldosterone secretion. See The Journal of Biological Chemistry, Vol. 270, No. 43, pp 25344-25347, 1995 and in the references cited therein

**Allatostatin Peptides (SEQ ID NOS: 946-949)** - Allatostatins are 6-18 amino acid peptides synthesized by insects to control production of juvenile hormones, which in turn regulate functions including metamorphosis and egg production. While neuropeptides of the allatostatin family inhibit *in vitro* production of juvenile hormone, which modulates aspects of development and reproduction in the cockroach, *Diploptera punctata,* they are susceptible to inactivation by peptidases in the hemolymph, gut, and bound to internal tissues.

**Amyloid Beta-Protein Fragments (Aβ fragments) (SEQ ID NOS: 950-1010)** - These are the principle component of the amyloid plaques that accumulate intracellularly and extracellularly in the neuritic plaques in the brain in Alzheimer's Disease. Aβ is a 4.5 kD protein, about 40-42 amino acids long, that is derived from the C-terminus of amyloid precursor protein (APP). APP is a membrane-spanning glycoprotein that, in the normal processing pathway, is cleaved inside the Aβ protein to produce α-sAPP, a secreted form of APP. Formation of α-sAPP precludes formation of Aβ. It has been proposed that Aβ accumulates by virtue of abnormal processing of APP, so that compounds that inhibit the activity of enzymes responsible for Aβ production are being sought. See, e.g., Wagner et al. Biotech. Report (1994/1995), pp. 106-107; and Selkoe (1993) TINS 16:403-409. Under certain conditions Aβ peptides first aggregate and then are deposited as a folded β-sheet structure that is characteristic of amyloid fibrils. β-amyloid (1-42) forms aggregates at a significantly greater rate and to a greater extent than β-amyloid (1-40).

**Antimicrobial peptides (SEQ ID NOS: 1011-1047) -** Antimicrobial peptides are a key component of the innate immune systems of most multicellular organisms, being active against one or more microorganisms such as bacteria, fungi, protozoa, yeast, and mycobacteria. Examples of such peptides include defensin, cecropin, buforin, and magainin. Despite broad divergences in sequence and taxonomy, most antimicrobial peptides share a common mechanism of action, *i.e..* membrane permeabilization of the pathogen. They are classified in two broad groups: linear and cyclic. In the linear antimicrobial peptides, there are two subgroups: linear peptides tending to adopt α-helical amphipathic conformation and linear peptides of unusual composition, rich in amino acids such as Pro, Arg, or Trp. The cyclic group encompasses all cysteine-containing peptides, and can be further divided into two subgroups corresponding to single or multiple disulfide structures.

Most antimicrobial peptides provoke an increase in plasma membrane permeability. There is also evidence of other mechanisms, such as inhibition of specific membrane proteins, synthesis of stress proteins, arrest of DNA synthesis, breakage of single-strand DNA by defensins, interaction with DNA (without arrest of synthesis) by buforins, or production of hydrogen peroxide. Antimicrobial peptides can also act by triggering self-destructive mechanisms such as apoptosis in eukaryotic cells or autolysis in bacterial targets. Antimicrobial peptides are also known to act as inhibitors of enzymes produced by pathogenic organisms, either by serving as pseudo-substrates or by tight binding to the active sight that disturbs the access of the substrate.

Increased levels of antimicrobial peptides have been reported for several animal and human infections for example for α-defensins in *Mycobacterium, Pasteurella,* or *Cryptoporidium* infections and for a variety of peptides in blisters and wound fluid. Inflammatory situations or stimuli are also associated with induction of antibiotic peptides.

Depleted levels of antimicrobial peptides are associated to several pathologies. Thus, patients of specific granule-deficiency syndrome, completely lacking in α-defensins, suffer from frequent and severe bacterial infections. Low levels of histatins from saliva in HIV patients has been correlated with a higher incidence of oral candidiasis and fungal infections. Perhaps the most compelling illustration of the implication of antimicrobial peptides in human pathology comes from cystic fibrosis, a genetic disease associated with recurrent bacterial infections of the airways. The defective chloride channel causing the disease increases the salinity of the alveolar fluid, and thus impairs the bactericidal activity of β-defensins, which are salt sensitive. Andreu D, (Ed.)(1998) "*Antimicrobial peptides*" Biopolymers (Peptide Science) vol 47, N° 6, pp413-491. A. Andreu, L. Rivas (1998) *Animal Antimicrobial Peptides: An Overview*, Biopolymers (Pep. Sci.) 47: pp415-433.

**Antioxidant Peptides (SEQ ID NOS: 1048-1050) -** Antioxidants are agents that prevents oxidative damage to tissue. Mammalian cells are continuously exposed to activated oxygen species such as superoxide, hydrogen peroxide, hydroxyl radical, and singlet oxygen. These reactive oxygen intermediates are generated *in vivo* by cells in response to aerobic metabolism, catabolism of drugs and other xenobiotics, ultraviolet and x-ray radiation, and the respiratory burst of phagocytic cells (such as white blood cells) to kill invading bacteria such as those introduced through wounds. Hydrogen peroxide, for example, is produced during respiration of most living organisms especially by stressed and injured cells.

One example of antioxidant peptides is natural killer-enhancing factor B (NKEF-B), which belongs to a highly conserved family of recently discovered antioxidants. Natural killer-enhancing factor (NKEF) was identified and cloned on the basis of its ability to increase NK cytotoxicity. Two genes, NKEF-A and -B, encode NKEF proteins and sequence analysis presented suggests that each belongs to a highly conserved family of antioxidants. The role of NKEF-B as an antioxidant has been demonstrated by its protection of transfected cells to oxidative damage by hydrogen peroxide. NKEF-B has antioxidant activities toward prooxidants such as alkyl hydroperoxide and MeHg. Together with its antioxidant activity, the induction of NKEF-B by HP indicates that NKEF-B is an important oxidative stress protein providing protection against a variety of xenobiotic toxic agents.

**Apoptosis Related Peptides (SEQ ID NOS: 1051-1075) -** Animal cells can self-destruct via an intrinsic program of cell death (Steller, 1995). Apoptosis is a form of programmed cell death that is characterized by specific morphologic and biochemical properties (Wyllie *et al.,* 1980). Morphologically, apoptosis is characterized by a series of structural changes in dying cells: blebbing (i.e. blistering) of the plasma membrane, condensation of the cytoplasm and nucleus, and cellular fragmentation into membrane apoptotic bodies (Steller, 1995; Wyllie *et al.,* 1980).

Biochemically, apoptosis is characterized by the degradation of chromatin, initially into large fragments of 50-300 kilobases and subsequently into smaller fragments that are monomers and multimers of 200 bases (Oberhammer *et al.,* 1993; Wyllie, 1980). Other biochemical indicators of apoptosis are induced or increased levels of the protein clusterin (Pearse *et al*., 1992), also known as TRPM-2 or SGP-2, and activation of the enzyme typell transglutaminase, which crosslinks proteins to the envelope of apoptotic bodies (Fesus *et al*., 1991). Apoptosis is a complex phenomenon of related morphological and biochemical processes that can vary with tissue and cell type (Zakeri *et al*., 1995).

The execution of apoptosis minimizes the leakage of cellular constituents from dying cells (apoptosis causes the cell to involute). For example, proteases could damage adjacent cells or stimulate an inflammatory response. This cardinal feature of apoptosis distinguishes it from necrosis, which usually results from trauma that causes injured cells to swell and lyse, releasing the cytoplasmic material that stimulates an inflammatory response (Steller, 1995; Wyllie *et al*., 1980)

**Bag Cell Peptides (BCP) (SEQ ID NOS: 1076-1080) -** The neuropeptidergic bag cells of the marine mollusc *Aplysia califomica* are involved in the egg-laying behavior of the animal. These neurosecretory cells synthesize an egg-laying hormone (ELH) precursor protein, yielding multiple bioactive peptides, including ELH, several bag cell peptides (BCP) and acidic peptide (AP). The bag cells of the marine mollusc *Aplysia califomica* are well-characterized neuroendocrine cells that initiate egg laying. During sexual maturation, these cells (bag cell neurons), develop the capability of storing hormones that are released during periods of nervous system stimulation. The hormones are important to the process of egg laying, and so must not be released before the animal is sexually mature. Alpha-bag cell peptide belong to a small family of structurally related peptides that can elicit bag-cell activity in vitro.

**Bombesin (SEQ ID NOS: 1081-1090) -** Bombesin is a bioactive tetradecapeptide neuropeptide that belongs to a family of peptides sharing a common C terminal sequence, Trp-Ala-X-Gly-His-Met-NH2, and the N terminal region. It has a modulatory role found in nerves of the brain and gut that prevents gastric injury by release of endogenous gastrin. The mammalian homologue of bombesin is gastrin-releasing peptide (GRP).

**Bone Gla Protein Peptides (SEQ ID NOS: 1091-1097)** - Osteocalcin (bone Gla-protein, or BGP) is produced and secreted by osteoblasts in the process of bone formation. As with collagen, this protein is a component of bone matrix. Serum osteocalcin rises when bone formation rates increase. Levels are high during puberty when bone growth is most rapid. Often levels are also high in diseases having high bone turnover, such as hyperparathyroidism and hyperthyroidism. In postmenopausal osteoporosis, osteocalcin levels are sometimes increased, reflecting the increased turnover of bone secondary to rapid bone resorption. In senile osteoporosis, occurring in more elderly subjects, osteocalcin levels are more likely to be low, reflecting reduced rates of both bone turnover and bone formation. A treatment regimen that increases bone formation also raises the serum osteocalcin levels.

**CART Peptides (SEQ ID NOS: 1098-1100)** - Cocaine and amphetamine regulated transcript peptide (CART), is a recently discovered hypothalamic peptide with a potent appetite suppressing activity. In the rat the CART gene encodes a peptide of either 129 or 116 amino acid residues whereas only the short form exists in humans. The predicted signal sequence is 27 amino acid residues resulting in a prohormone of 102 or 89 residues. The C-terminal end of CART, consisting of 48 amino acid residues and 3 disulphide bonds, is thought to constitute a biologically active part of the molecule.

In the central nervous system CART is highly expressed in many hypothalamic nuclei, some of which are involved in regulating feeding behavior. The CART mRNA is regulated by leptin, and the expressed CART is a potent inhibitor of feeding that even overrides the feeding response induced by neuropeptide Y. The putative CART receptor is therefore a potential therapeutic target for an anti-obesity drug. See CART, a new anorectic peptide Thim L; Kristensen P; Larsen PJ; Wulff BS, Int J Biochem Cell Biol, 30(12):1281-4 1998 Dec.

**Cell Adhesion Peptides (SEQ ID NO: 1101)** - Cellular adhesion peptides are directly involved in the cellular response to external stimuli. For example, during an inflammatory response, leukocytes must leave the plasma compartment and migrate to the point of antigenic insult. The mechanism of this migratory event is a complex interplay between soluble mediators and membrane-bound cellular adhesion molecules. Soluble cellular chemotactic factors, which are produced in the damaged tissue by a variety of resident cells, set up a chemical concentration gradient out to the plasma compartment. Interaction of these factors with their receptors on leukocytes leads to a directional migration of the leukocytes toward increasing concentrations of the chemotactic factor. Simultaneously, various adhesion peptides are upregulated on the leukocyte which mediate the initial rolling on the endothelial tissue, binding to a specific ligand on the activated endothelial tissue, and finally migration between endothelial cells into the tissue. The steps in this cascade of events are mediated by the interaction of specific cell surface proteins, termed "cell adhesion molecules such as. E-selectin (ELAM-1, endothelial leukocyte adhesion molecule-1), ICAM-1 (intercellular adhesion molecule-1), and VCAM-1 (vascular cell adhesion molecule-1).

**Chemotactic Peptides (SEQ ID NOS: 1102-1113)** - Chemotactic peptides are peptides that stimulate the migration of white cells, leukocytes and macrophages into tissues at the site of infection or injury or alternatively the prevent the migration of these same cells away from these sites.

**Complement Inhibitors (SEQ ID NOS: 1114-1120) -** Inhibition of complement attack on xenotransplants may be accomplished by the use of complement inhibitors. The rejection of transplanted organs may involve both an extremely rapid hyperacute rejection (HAR) phase and a slower cellular rejection phase. HAR of xenotransplants is initiated by preformed "natural" antibodies that bind to donor organ endothelium and activate complement attack by the recipient immune system. Activation of complement leads to the generation of fluid phase (C3a, C5a) and membrane bound (C3b and C5b-9, i.e., C5b, C6, C7, C8, and C9) proteins with chemotactic, procoagulant, proinflammatory, adhesive, and cytolytic properties. Complement inhibitors inhibit this process.

**Cortistatin Peptides (SEQ ID NOS: 1121-1124)** - Cortistatin, whose mRNA accumulates during sleep deprivation, apparently acts by antagonizing the effects of acetylcholine on cortical excitability, thereby causing synchronization brain slow waves. Cortistatin-14 (CST-14) shares 11 of its 14 residues with somatostatin-14 (SRIF-14), yet its effects on sleep physiology, locomotor behavior and hippocampal function are quite different from those of somatostatin.

**Fibronecdtin Fragments & Fibrin Related Peptides (SEQ ID NOS: 1125-1174)** - Fibronectin is a large glycoprotein that is composed of blocks of three types of repeating, homologous peptide sequences. Several of the homologous blocks form functional domains that are organized in a linear array on two nearly identical subunit arms. Each arm can be divided into functional domains, which are often referred to by one of the substances which bind in that region, for example the heparin-binding fragment, the fibrin binding fragment, and the cell-binding fragment. In several cell types, the Arg-Gly-Asp (RGD) sequence in the cell-binding domain of fibronectin interacts with a cell-surface glycoprotein designated Iib/IIIa. Fibronectin also binds to extracellular and basement-membrane components, to the envelope glycoprotein of viruses, to a variety of bacteria including staphylococci and streptococci, and to parasites such as *Trypanosoma cruzi* and *Leishmania* species.

Fibronectin has several adhesive functions, for example cell-to-cell adhesion, cell-to-basement-membrane attachment, and clot stabilization. In addition, fibronectin promotes embryogenesis, nerve regeneration, fibroblast migration, macrophage function, and pathogen (virus, fungus, bacteria, and protozoa) binding to mammalian cells and extracellular matrix. Thus, fibronectin is involved in the pathogenesis of infections from the initiation of the infection through the final stages of wound healing. See Proctor, R.A., Rev. Infect. Dis., 9, 317 (1987).

**FMRF and Analog peptides (SEQ ID NOS: 1175-1187) -** FMRF are neuropeptides encoded in the FMRF amide gene and have a common C-terminal FMRFamide but different N-terminal extensions. FMRF amide-related peptides (FaRPs) are present throughout the animal kingdom and affect both neural and gastrointestinal functions. Organisms have several genes encoding numerous FaRPs with a common C-terminal structure but different N-terminal amino acid extensions.

**Galanin & related peptides (SEQ ID NOS: 1188-1208)-** Galanin is a 29-30 amino acid peptide originally isolated from pig small intestine. It is found in two biologically active forms: GAL (1-19), and GAL (1-30), a non-amidated form. It has many biological roles including: the inhibition of the release of biogenic amines in the hypothalamus, the pre- and post-synaptic inhibition of cholinergic function, the maintenance of gastrointestinal homeostasis, and the regulation of insulin and glucagon secretion.

**Growth Factors & related peptides (SEQ ID NOS: 1209-1240)** - Growth factors are a family of proteins that regulate cell division. Some growth factors are cell type specific, stimulating division of only those cells with appropriate receptors. Other growth factors are more general in their effects. There are also extracellular factors that antagonize the effects of growth factors, slowing or preventing division (for example transforming growth factor beta and tumor necrosis factor). These extracellular signals act through cell surface receptors very similar to those for hormones, and by similar mechanisms: the production of intracellular second messangers, protein phosphorylation, and ultimately, alteration of gene expression.

**Gtherapeutic peptide-Binding protein fragments (SEQ ID NOS: 1241-1246)** - Members of a family of Gtherapeutic peptide-binding regulatory proteins (G-proteins) transduce signals from membrane-bound receptors to intracellular effectors. The family includes Gₛ and Gᵢ, which are responsible for stimulation and inhibition, respectively, of adenylate cyclase. Transducin (T), localized in the disc membranes of retinal rod outer segments, couples activation of rhodopsin by light to increased cyclinc GMP phosphodiesterase activity. Gₒ, found originally in bovine brain, is a fourth member of the family.

Purified G proteins have similar physical properties. They are heterodimers composed of α, β, and γ subunits. The α subunits bind and hydrolyze Gtherapeutic peptide. See S. M. Mumby et al., PNAS 83, 265 (1986) and Lehninger p. 764.

**Guanylin and Uroguanylin (SEQ ID NOS: 1247-1249) -** Guanylin and uroguanylin are peptides isolated from intestinal mucosa, and urine, which regulate cyclic GMP production in enterocytes bind to and activate guanylate cyclase C and control salt and water transport in many epithelia in vertebrates, mimicking the action of several heat-stable bacteria enterotoxins. In the kidney, both of them have well-documented natriuretic and kaliuretic effects.

Chloride secretion in the intestine is regulated by these hormones via activation of guanylate cyclase C (GC-C). Both peptides are expressed in a variety of tissues and organs, including the kidney. In the isolated perfused kidney and in vivo these hormones induce natriuresis and diuresis, however, localisation and cellular mechanisms of their action in the kidney are still unknown.

**Inhibin Peptides (SEQ ID NOS: 1250-1255) -** Inhibin is composed of two subunits (α is 134 amino acids; β is 115 and 116 amino acids). Its role is inhibition of FSH secretion. The two inhibin isoforms, inhibin A and inhibin B, are produced by the gonads in the course of gamete maturation and have different patterns of secretion during the menstrual cycle. Inhibins are also produced by the placenta and fetal membranes and may be involved in physiological adaptation of pregnancy. Clinically, inhibins may serve as sensitive tumor markers in postmenopausal women, or as useful tools for evaluating ovarian reserve in infertile women; they may also be used in the diagnosis of materno-fetat disorders and to prevent maturation of the ovum or to inhibit ovulation.

**Interleukin (IL) and Interleukin Receptor Proteins (SEQ ID NOS: 1256-1263)** - Interleukins are growth factors targeted to cells of hematopoietic origin. A variety of biological activities associated with immune and inflammatory responses have been ascribed to interleukins. These responses include fever, cartilage breakdown, bone resorption, thymocyte proliferation, activation of T and B lymphocytes, induction of acute-phase protein synthesis from hepatocytes, fibroblast proliferation, and differentiation and proliferation of bone marrow cells.

**Laminin Fragments (SEQ ID NOS: 1264-1284)** - Laminin, the major noncollagenous glycoprotein of basement membranes, has been shown to promote the adhesion, spreading, and migration of a variety of tumor cell types in vitro. In particular, the major current studies in the laboratory utilize intact laminin, purified proteolytic fragments of laminin, and synthetic peptides of laminin to identify functionally active sites on this large protein. Components of such basement membranes are important modulators of growth, development, and differentiation for various cell types. A conjugated laminin could be used to prevent inflamation or fibrosis in tissues.

This category also includes the peptide kringle-5 (or K-5). As used herein, the term "kringle 5" refers to the region of mammalian plasminogen having three disulfide bonds which contribute to the specific three-dimensional confirmation defined by the fifth kringle region of the mammalian plasminogen molecule. One such disulfide bond links the cysteine residues located at amino acid positions 462 and 541, a second links the cysteine residues located at amino acid positions 483 and 524 and a third links the cysteine residues located at amino acid positions 512 and 536. The term "kringle 5 peptide peptides" refers to peptides with anti-angiogenic activity of between 4 and 104 amino acids (inclusive) with a substantial sequence homology to the corresponding peptide fragment of mammalian plasminogen.

**Leptin Fragment Peptides (SEQ ID NOS: 1286-1288) -** Leptin**,** the protein product of the obesity gene, is secreted by fat cells. Leptin is involved in the regulation of bodyweight and metabolism in man and might also be involved in the pathophysiology of the insulin resistance syndrome, which is associated with the development of cardiovascular diseases

**Leucokinins (SEQ ID NOS: 1289-98) -** Leucokinins are a group of widespread insect hormones that stimulate gut motility and tubule fluid secretion rates. In tubules, their major action is to raise chloride permeability by binding to a receptor on the basolateral membrane.

**Pituitary Adenylate Cyclase Activating Polypeptide (PACAP) (SEQ ID NOS: 1299-1311)** - It is a thirty-eight amino acid peptide first isolated from ovine hypothalamus, which also occurs in a 27 amino acid form called PACAP-27. PACAP has been localized in the hypothalamus, elsewhere in the brain, respiratory tract and gastrointestinal system. It has many biological actions, including neurotransmitter and hormonal functions, involvement in regulation of energy metabolism, and neuronal cytoprotective activity.

**Pancreastatin (SEQ ID NOS: 1312-1324) -** Pancreastatin is a 49 amino acid peptide first isolated, purified and characterized from porcine pancreas. Its biological activity in different tissues can be assigned to the C-terminal part of the molecule. Pancreastatin has a prohormonal precursor, chromogranin A, which is a glycoprotein present in neuroendocrine cells, including the endocrine pancreas

**Polypeptides (SEQ ID NOS: 1325-1326) -** these are repetitive chains. Two examples are provided: (pro-Hyp-Gly)10*20H20 and Poly-L-Lysine Hydrochloride.

**Signal Transduction Reagents (SEQ ID NOS: 1327-1367)** - Signal transduction is the process by which an extracellular signal (for example chemical, mechanical, or electrical) is amplfied and converted to a cellular response. Many reagents are involved in this process, for example achatin-1, glycogen synthase, autocamtide 2, calcineurin autoinhibitory peptide, calmodulin dependent protein kinase II, calmodulin dependent protein kinase substrate, calmodulin dependent protein kinase substrate analog, CKS-17, Cys-Kemptide, autocamtide 2, malantide, melittin, phosphate acceptor peptide, protein kinase C fragments, P34cd2 kinase fragment, P60c-src substrate II, protein kinase A fragments, tyrosine protein kinase substrate, syntide 2, S6 kinase substrate peptide 32, tyrosine specific protein kinase inhibitor, and their derivatives and fragments.

**Thrombin Inhibitors (SEQ ID NOS: 1368-1377)** - Thrombin is a key regulatory enzyme in the coagulation cascade; it serves a pluralistic role as both a positive and negative feedback regulator. In addition to its direct effect on hemostasis, thrombin exerts direct effects on diverse cell types that support and amplify pathogenesis of arterial thrombus disease. The enzyme is the strongest activator of platelets causing them to aggregate and release substances (eg. ADP TXA.sub.2 NE) that further propagate the thrombotic cycle. Platelets in a fibrin mesh comprise the principal framework of a white thrombus. Thrombin also exerts direct effects on endothelial cells causing release of vasoconstrictor substances and translocation of adhesion molecules that become sites for attachment of immune cells. In addition, the enzyme causes mitogenesis of smooth muscle cells and proliferation of fibroblasts. From this analysis, it is apparent that inhibition of thrombin activity by thrombin inhibitors constitutes a viable therapeutic approach towards the attenuation of proliferative events associated with thrombosis.

**Toxins (SEQ ID NOS: 1378-1415)** -A toxin can be conjugated using the present invention to target cancer cells, receptors, viruses, or blood cells. Once the toxin binds to the target cells the toxin is allowed to internalize and cause cell toxicity and eventually cell death. Toxins have been widely used as cancer therapeutics.

One example of a class of toxins is the mast cell degranulating peptide, a cationic 22-amino acid residue peptide with two disulfide bridges isolated from bee venom, causes mast cell degranulation and histamine release at low concentrations and has anti-inflammatory activity at higher concentrations. It is a powerful anti-inflammatory, more than 100 times more effective than hydrocortisone in reducing inflammation. Because of these unique immunologic properties, MCD peptide may serve as a useful tool for studying secretory mechanisms of inflammatory cells such as mast cells, basophils, and leukocytes, leading to the design of compounds with therapeutic potential. An example of a mast cell degranulating peptide is mastoparans, originating from wasp venom. It degranulates mast cells in the concentration of 0.5 µg/ml and releases histamine from the cells in the same concentration. See IY. Hirai et al., Chem. Pharm. Bull. 27, 1942 (1979).

Other examples of such toxins include omega-agatoxin TK, agelenin, apamin, calcicudine, calciseptine, charbdotoxin, chlorotoxin, conotoxins, endotoxin inhibitors, gegraphutoxins, iberiotoxin, kaliotoxin, mast cell degranulating peptides, margatoxin, neurotoxin NSTX-3, PLTX-II, scyllatoxin, stichodactyla toxin, and derivatives and fragments thereof.

**Trypsin Inhibitors (SEQ ID NOS: 1416-1418)** - Trypsin inhibitors functions as an inhibitors of trypsin, as well as other serine proteases. Useful for treatment of lung inflammation, pancreatitis, myocardial infarction, cerebrovascular ischemia

**Virus Related Peptides (SEQ ID NOS:1419-1529)** - Virus related peptides are proteins related to viruses, for example virus receptors, virus inhibitors, and envelope proteins. Examples include but are not limited to peptide inhibitors of human immunodeficiency virus (HIV), respiratory syncytial virus (RSV), human parainfluenza virus (HPV), measles virus (MeV), and simian immunodeficiency virus (SIV). fluorogenic Human CMV Protease Substrate, HCV Core Protein, HCV NS4A Protein, Hepatitis B Virus Receptor Binding Fragment, Hepatitis B Virus Pre-S Region, Herpes Virus Inhibitor 2, HIV Envelope Protein Fragment, HIV gag fragment, HIV substrate, HIV-1 Inhibitory Peptide, peptide T, T21, V3 decapeptide, Virus Replication Inhibitor Peptide, and their fragments and derivatives.

These peptides can be administered therapeutically. For example, peptide T is a chain of 8 amino acids from the V2 region of HIV-1 gp120. These amino acids look like a portion of HIV's outer envelope. It is under investigation as a treatment for HIV-related neurological and constitutional symptoms, as peptide T may be able to alleviate symptoms like fevers, night sweats, weight loss, and fatigue. It has also been shown to resolve psoriatic lesions.

**Miscellaneous peptides (SEQ ID NOS: 1529-1617)** - Including adjuvant peptide analogs, alpha mating factor, antiarrhythmic peptide, anorexigenic peptide, alpha-1 antitrypsin, bovine pineal antireproductive peptide, bursin, C3 peptide P16, cadherin peptide, chromogranin A fragment, contraceptive tetrapeptide, conantokin G, conantokin T, crustacean cardioactive peptide, C-telopeptide, cytochrome b588 peptide, decorsin; delicious peptide, delta-sleep-inducing peptide, diazempam-binding inhibitor fragment, nitric oxide synthase blocking peptide, OVA peptide, platelet calpain inhibitor (P1), plasminogen activator inhibitor 1, rigin, schizophrenia related peptide, sodium potassium Atherapeutic peptidease inhibitor-1, speract, sperm activating peptide, systemin, thrombin receptor agonist (three peptides), tuftsin, adipokinetic hormone, uremic pentapeptide, Antifreeze Polypeptide, tumor necrosis factor, leech [Des Asp10]Decorsin, L-Ornithyltaurine Hydrochloride, p-Aminophenylacetyl Tuftsin, Ac-Glu-Glu-Val-Val-Ala-Cys-pNA, Ac-Ser-Asp-Lys-Pro, Ac-rfwink-NH2, Cys-Gly-Tyr-Gly-Pro-Lys-Lys-Lys-Arg-Lys-Val-Gly-Gly, DAla-Leu, D-D-D-D-D, D-D-D-D-D-D, N-P-N-A-N-P-N-A, V-A-I-T-V-L-V-K, V-G-V-R-V-R, V-I-H-S, V-P-D-P-R, Val-Thr-Cys-Gly, R-S-R, Sea Urchin Sperm Activating Peptide, SHU-9119 MC3-R & MC4-R Antagonist, glaspimod (irnmunostimutant, useful against bacterial infections, fungal infections, immune deficiency immune disorder, leukopenia), HP-228 (melanocortin, useful against chemotherapy induced emesis, toxicity, pain, diabetes mellitus, inflammation, rheumatoid arthritis, obesity), alpha 2-plasmin inhibitor (plasmin inhibitor), APC tumor suppressor (tumor suppressor, useful against neoplasm), early pregnancy factor (immunosuppressor), endozepine diazepam binding inhibitor (receptor peptide), gamma interferon (useful against leukemia), glandular kallikrei n-1 (immunostimulant), placental ribonuclease inhibitor, sarcolecin binding protein, surfactant protein D, wilms' tumor suppressor, wilm's tumor suppressor, GABAB 1b receptor peptide, prion related peptide (iPrP13), choline binding protein fragment (bacterial related peptide), telomerase inhibitor, cardiostatin peptide, endostatin derived peptide (angiogenesis inhibitor), prion inhibiting peptide, N-methyl D-aspartate receptor antagonist, C-peptide analog (useful against diabetic complications).

### 2. Modified Therapeutic Peptides

This invention relates to a method of synthesizing modified therapeutic peptides and their derivatives. The modified therapeutic peptides synthesized by the method of the invention include reactive groups which can react with available reactive functionalities on blood components to form covalent bonds.

To form covalent bonds with functionalities on a protein, one may use as a reactive group a wide variety of active carboxyl groups, particularly esters, where the hydroxyl moiety is physiologically acceptable at the levels required to modify the therapeutic peptide. While a number of different hydroxyl groups may be employed in these linking agents, the most convenient would be N-hydroxysuccinimide (NHS), and N-hydroxy-sulfosuccinimide (sulfo-NHS).

Primary amines are the principal targets for NHS esters as diagramed in schematic 1A below. Accessible α-amine groups present on the N-termini of proteins react with NHS esters. However, α-amino groups on a protein may not be desirable or available for the NHS coupling. While five amino acids have nitrogen in their side chains, only the ε-amine of lysine reacts significantly with NHS esters. An amide bond is formed when the NHS ester conjugation reaction reacts with primary amines releasing N-hydroxysuccinimide as demonstrated in schematic 1A below.

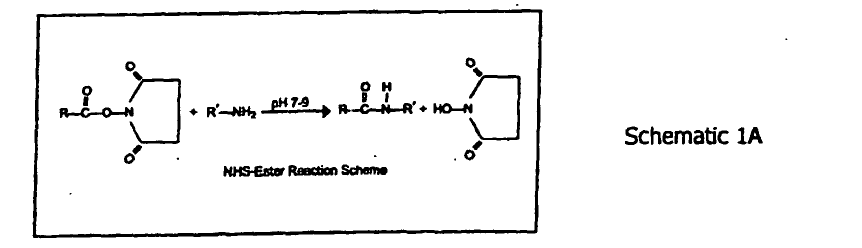

In the preferred embodiments of this invention, the functionality on the protein will be a thiol group and the reactive group will be a maleimido-containing group such as gamma-maleimide-butyralamide (GMBA) or MPA. The maleimido group is most selective for sulfhydryl groups on peptides when the pH of the reaction mixture is kept between 6.5 and 7.4 as shown in schematic 1B below. At pH 7.0, the rate of reaction of maleimido groups with sulfhydryls is 1000-fold faster than with amines. A stable thioether linkage between the maleimido group and the sulfhydryl is formed which cannot be cleaved under physiological conditions.

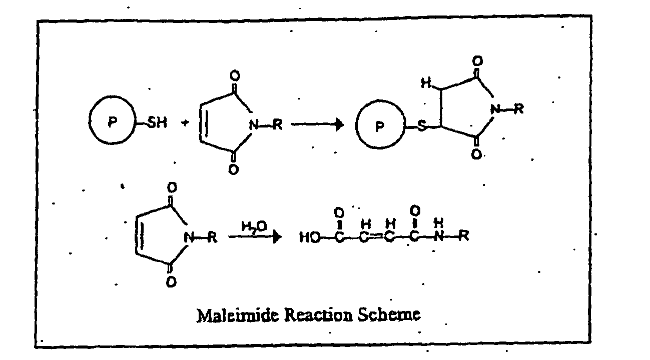

The therapeutic peptides and peptide derivatives of the invention may be modified for specific labeling and non-specific labeling of blood components.

### A. Specific Labeling

Preferably, the therapeutic peptides synthesized or protected by the methods of this invention are designed to specifically react with thiol groups on mobile blood proteins. Such reaction is preferably established by covalent bonding of a therapeutic peptide modified with a maleimide link (e.g. prepared from GMBS, MPA or other maleimides) to a thiol group on a mobile blood protein such as serum albumin or IgG.

Under certain circumstances, specific labeling with maleimides offers several advantages over non-specific labeling of mobile proteins with groups such as NHS and sulfo-NHS. Thiol groups are less abundant *in vivo* than amino groups. Therefore, the maleimide derivatives of this invention will covalently bond to fewer proteins. For example, in albumin (the most abundant blood protein) there is only a single thiol group. Thus, therapeutic peptide-maleimide-albumin conjugates will tend to comprise approximately a 1:1 molar ratio of therapeutic peptide to albumin. In addition to albumin, IgG molecules (class II) also have free thiols. Since IgG molecules and serum albumin make up the majority of the soluble protein in blood they also make up the majority of the free thiol groups in blood that are available to covalently bond to maleimide-modified therapeutic peptides.

Further, even among free thiol-containing blood proteins, specific labeling with maleimides leads to the preferential formation of therapeutic peptide-maleimide-albumin conjugates, due to the unique characteristics of albumin itself. The single free thiol group of albumin, highly conserved among species, is located at amino acid residue 34 (Cys³⁴). It has been demonstrated recently that the Cys³⁴ of albumin has increased reactivity relative to free thiols on other free thiol-containing proteins. This is due in part to the very low pK value of 5.5 for the Cys³⁴ of albumin. This is much lower than typical pK values for cysteines residues in general, which are typically about 8. Due to this low pK, under normal physiological conditions Cys³⁴ of albumin is predominantly in the ionized form, which dramatically increases its reactivity. In addition to the low pK value of Cys³⁴, another factor which enhances the reactivity of Cys³⁴ is its location, which is in a crevice close to the surface of one loop of region V of albumin. This location makes Cys³⁴ very available to ligands of all kinds, and is an important factor in Cys³⁴'s biological role as free radical trap and free thiol scavenger. These properties make Cys³⁴ highly reactive with therapeutic peptide-maleimides, and the reaction rate acceleration can be as much as 1000-fold relative to rates of reaction of therapeutic peptide-maleimides with other free-thiol containing proteins.

Another advantage of therapeutic peptide-maleimide-albumin conjugates is the reproducibility associated with the 1:1 loading of peptide to albumin specifically at Cys³⁴. Other techniques, such as glutaraldehyde, DCC, EDC and other chemical activations of, for example, free amines lack this selectivity. For example, albumin contains 52 lysine residues, 25-30 of which are located on the surface of albumin and accessible for conjugation. Activating these lysine residues, or alternatively modifying peptides to couple through these lysine residues, results in a heterogenous population of conjugates. Even if 1:1 molar ratios of peptide to albumin are employed, the yield will consist of multiple conjugation products, some containing 0, 1, 2 or more peptides per albumin, and each having peptides randomly coupled at any one of the 25-30 available lysine sites. Given the numerous combinations possible, characterization of the exact composition and nature of each batch becomes difficult, and batch-to-batch reproducibility is all but impossible, making such conjugates less desirable as a therapeutic. Additionally, while it would seem that conjugation through lysine residues of albumin would at least have the advantage of delivering more therapeutic agent per albumin molecule, studies have shown that a 1:1 ratio of therapeutic agent to albumin is preferred. In an article by Stehle, et al., "The Loading Rate Determines Tumor Targeting Properties of Methotrexate-Albumin Conjugates in Rats," Anti-Cancer Drugs, Vol. 8, pp. 677-685 (1997), incorporated herein in its entirety, the authors report that a 1:1 ratio of the anti-cancer methotrexate to albumin conjugated via glutaraldehyde gave the most promising results. These conjugates were taken up by tumor cells, whereas conjugates bearing 5:1 to 20:1 methotrexate molecules had altered HPLC profiles and were quickly taken up by the liver *in vivo*. It is postulated that at these higher ratios, conformational changes to albumin diminish its effectiveness as a therapeutic carrier.

Through controlled administration of maleimide-therapeutic peptides *in vivo*, one can control the specific labeling of albumin and IgG *in vivo*. In typical administrations, 80-90% of the administered maleimide-therapeutic peptides will label albumin and less than 5% will label IgG. Trace labeling of free thiols such as glutathione will also occur. Such specific labeling is preferred for *in vivo* use as it permits an accurate calculation of the estimated half-life of the administered agent.

In addition to providing controlled specific *in vivo* labeling, maleimide-therapeutic peptides can provide specific labeling of serum albumin and IgG *ex vivo.* Such *ex vivo* labeling involves the addition of maleimide-therapeutic peptides to blood, serum or saline solution containing serum albumin and/or IgG. Once modified *ex vivo* with maleimide-therapeutic peptides, the blood, serum or saline solution can be readministered to the blood for *in vivo* treatment.

In contrast to NHS-peptides, maleimide-therapeutic peptides are generally quite stable in the presence of aqueous solutions and in the presence of free amines. Since maleimide-therapeutic peptides will only react with free thiols, protective groups are generally not necessary to prevent the maleimide-therapeutic peptides from reacting with itself. In addition, the increased stability of the peptide permits the use of further purification steps such as HPLC to prepare highly purified products suitable for *in vivo* use. Lastly, the increased chemical stability provides a product with a longer shelf life.

### B. Non-Specific Labeling

The therapeutic peptides synthesized or protected by the methods of the invention may also be modified for non-specific labeling of blood components. Bonds to amino groups will generally be employed, particularly with the formation of amide bonds for non-specific labeling. To form such bonds, one may use as a chemically reactive group coupled to the therapeutic peptide a wide variety of active carboxyl groups, particularly esters, where the hydroxyl moiety is physiologically acceptable at the levels required. While a number of different hydroxyl groups may be employed in these linking agents, the most convenient would be N-hydroxysuccinimide (NHS) and N-hydroxy-sulfosuccinimide (sulfo-NHS).

Other linking agents which may be utilized are described in U.S. Patent 5,612,034, which is hereby incorporated herein.

The various sites with which the chemically reactive groups of the non-specific therapeutic peptides may react *in vivo* include cells, particularly red blood cells (erythrocytes) and platelets, and proteins, such as immunoglobulins, including IgG and IgM, serum albumin, ferritin, steroid binding proteins, transferrin, thyroxin binding protein, α-2-macroglobulin, and the like. Those receptors with which the derivatized therapeutic peptides react, which are not long-lived, will generally be eliminated from the human host within about three days. The proteins indicated above (including the proteins of the cells) will remain in the bloodstream at least three days, and may remain five days or more (usually not exceeding 60 days, more usually not exceeding 30 days) particularly as to the half life, based on the concentration in the blood.

For the most part, reaction will be with mobile components in the blood, particularly blood proteins and cells, more particularly blood proteins and erythrocytes. By "mobile" is intended that the component does not have a fixed situs for any extended period of time, generally not exceeding 5 minutes, more usually one minute, although some of the blood components may be relatively stationary for extended periods of time. Initially, there will be a relatively heterogeneous population of labeled proteins and cells. However, for the most part, the population within a few days after administration will vary substantially from the initial population, depending upon the half-life of the labeled proteins in the blood stream. Therefore, usually within about three days or more, IgG will become the predominant labeled protein in the blood stream.

Usually, by day 5 post-administration, IgG, serum albumin and erythrocytes will be at least about 60 mole %, usually at least about 75 mole %, of the conjugated components in blood, with IgG, IgM (to a substantially lesser extent) and serum albumin being at least about 50 mole %, usually at least about 75 mole %, more usually at least about 80 mole %, of the non-cellular conjugated components.

The desired conjugates of non-specific therapeutic peptides to blood components may be prepared *in vivo* by administration of the therapeutic peptides directly to the patient, which may be a human or other mammal. The administration may be done in the form of a bolus or introduced slowly over time by infusion using metered flow or the like.

If desired, the subject conjugates may also be prepared *ex vivo* by combining blood with modified therapeutic peptides of the present invention, allowing covalent bonding of the modified therapeutic peptides to reactive functionalities on blood components and then returning or administering the conjugated blood to the host. Moreover, the above may also be accomplished by first purifying an individual blood component or limited number of components, such as red blood cells, immunoglobulins, serum albumin, or the like, and combining the component or components *ex vivo* with the chemically reactive therapeutic peptides. The labeled blood or blood component may then be retumed to the host to provide *in vivo* the subject therapeutically effective conjugates. The blood also may be treated to prevent coagulation during handling *ex vivo.*

### 3. Synthesis of Therapeutic Peptides Used in the Present Invention

Peptide fragments may be synthesized by standard methods of solid phase peptide chemistry known to those of ordinary skill in the art. For example, peptide fragments may be synthesized by solid phase chemistry techniques following the procedures described by Steward and Young (Steward, J. M. and Young, J. D., Solid Phase Peptide Synthesis, 2nd Ed., Pierce Chemical Company, Rockford, III., (1984) using an Applied Biosystem synthesizer. Similarly, multiple fragments may be synthesized then linked together to form larger fragments. These synthetic peptide fragments can also be made with amino acid substitutions at specific locations.

For solid phase peptide synthesis, a summary of the many techniques may be found in J. M. Stewart and J. D. Young, Solid Phase Peptide Synthesis, W. H. Freeman Co. (San Francisco), 1963 and J. Meienhofer, Hormonal Proteins and Peptides, vol. 2, p. 46, Academic Press (New York), 1973. For classical solution synthesis see G. Schroder and K. Lupke, The Peptides, Vol. 1, Acacemic Press (New York). In general, these methods comprise the sequential addition of one or more amino acids or suitably protected amino acids to a growing peptide chain. Normally, either the amino or carboxyl group of the first amino acid is protected by a suitable protecting group. The protected or derivatized amino acid is then either attached to an inert solid support or utilized in solution by adding the next amino acid in the sequence having the complimentary (amino or carboxyl) group suitably protected and under conditions suitable for forming the amide linkage. The protecting group is then removed from this newly added amino acid residue and the next amino acid (suitably protected) is added, and so forth.

After all the desired amino acids have been linked in the proper sequence, any remaining protecting groups (and any solid support) are removed sequentially or concurrently to afford the final polypeptide. By simple modification of this general procedure, it is possible to add more than one amino acid at a time to a growing chain, for example, by coupling (under conditions which do not racemize chiral centers) a protected tripeptide with a properly protected dipeptide to form, after deprotection, a pentapeptide.

A particularly preferred method of preparing compounds of the present invention involves solid phase peptide synthesis wherein the amino acid α-N-terminal is protected by an acid or base sensitive group. Such protecting groups should have the properties of being stable to the conditions of peptide linkage formation while being readily removable without destruction of the growing peptide chain or racemization of any of the chiral centers contained therein. Suitable protecting groups are 9-fluorenylmethyloxycarbonyl (Fmoc), t-butyloxycarbonyl (Boc), benzyloxycarbonyl (Cbz), biphenylisopropyloxycarbonyl, t-amyloxycarbonyl, isobornyloxycarbonyl, α, α-dimethyl-3,5-dimethoxybenzyloxycarbonyl, o-nitrophenylsulfenyl, 2-cyano-t-butyloxycarbonyl, and the like. The 9-fluorenyl-methyloxycarbonyl (Fmoc) protecting group is particularly preferred for the synthesis of Itherapeutic peptide fragments. Other preferred side chain protecting groups are, for side chain amino groups like lysine and arginine, 2,2,5,7,8-pentamethylchroman-6-sulfonyl (pmc), nitro, p-toluenesulfonyl, 4-methoxybenzene-sulfonyl, Cbz, Boc, and adamantyloxycarbonyl; for tyrosine, benzyl, o-bromobenzyloxycarbonyl, 2,6-dichlorobenzyl, isopropyl, t-butyl (t-Bu), cyclohexyl, cyclopenyl and acetyl (Ac); for serine, t-butyl, benzyl and tetrahydropyranyl; for histidine, trityl, benzyl, Cbz, p-toluenesulfonyl and 2,4-dinitrophenyl; for tryptophan, formyl; for asparticacid and glutamic acid, benzyl and t-butyl and for cysteine, triphenylmethyl (trityl).

In the solid phase peptide synthesis method, the α-C-terminal amino acid is attached to a suitable solid support or resin. Suitable solid supports useful for the above synthesis are those materials which are inert to the reagents and reaction conditions of the stepwise condensation-deprotection reactions, as well as being insoluble in the media used. The preferred solid support for synthesis of α-C-terminal carboxy peptides is 4-hydroxymethylphenoxymethyl-copoly(styrene-1% divinylbenzene). The preferred solid support for α-C-terminal amide peptides is the 4-(2',4'-dimethoxyphenyl-Fmoc-aminomethyl)phenoxyacetamidoethyl resin available from Applied Biosystems (Foster City, Catif.). The α-C-terminal amino acid is coupled to the resin by means of N,N'-dicyclohexylcarbodiimide (DCC), N,N'-diisopropylcarbodiimide (DIC) or O-benzotriazol-1-yl-N,N,N',N'-tetramethyluronium-hexafluorophosphate (HBTU), with or without 4-dimethylaminopyridine (DMAP), 1-hydroxybenzotriazole (HOBT), benzotriazol-1-yloxy-tris(dimethylamino)phosphonium-hexafluorophosphate (BOP) or bis(2-oxo-3-oxazolidinyl)phosphine chloride (BOPCI), mediated coupling for from about 1 to about 24 hours at a temperature of between 10° and 50°C. in a solvent such as dichloromethane or DMF.

When the solid support is 4-(2',4'-dimethoxyphenyl-Fmoc-aminomethyl)phenoxy-acetamidoethyl resin, the Fmoc group is cleaved with a secondary amine, preferably piperidine, prior to coupling with the α-C-terminal amino acid as described above. The preferred method for coupling to the deprotected 4-(2',4'-dimethoxyphenyl-Fmoc-aminomethyl)phenoxy-acetamidoethyl resin is O-benzotriazol-1-yl-N,N,N',N'-tetramethyluroniumhexafluoro-phosphate (HBTU, 1 equiv.) and 1-hydroxybenzotriazole (HOBT, 1 equiv.) in DMF. The coupling of successive protected amino acids can be carried out in an automatic polypeptide synthesizer as is well known in the art. In a preferred embodiment, the α-N-terminal amino acids of the growing peptide chain are protected with Fmoc. The removal of the Fmoc protecting group from the α-N-terminal side of the growing peptide is accomplished by treatment with a secondary amine, preferably piperidine. Each protected amino acid is then introduced in about 3-fold molar excess, and the coupling is preferably carried out in DMF. The coupling agent is normally O-benzotriazol-1-yl-N,N,N',N'-tetramethyluroniumhexafluorophosphate (HBTU, 1 equiv.) and 1-hydroxybenzotriazole (HOBT, 1 equiv.).

At the end of the solid phase synthesis, the polypeptide is removed from the resin and deprotected, either in successively or in a single operation. Removal of the polypeptide and deprotection can be accomplished in a single operation by treating the resin-bound polypeptide with a cleavage reagent comprising thianisole, water, ethanedithiol and trifluoroacetic acid. In cases wherein the α-C-terminal of the polypeptide is an alkylamide, the resin is cleaved by aminolysis with an alkylamine. Alternatively, the peptide may be removed by transesterification, e.g. with methanol, followed by aminolysis or by direct transamidation. The protected peptide may be purified at this point or taken to the next step directly. The removal of the side chain protecting groups is accomplished using the cleavage cocktail described above. The fully deprotected peptide is purified by a sequence of chromatographic steps employing any or all of the following types: ion exchange on a weakly basic resin (acetate form); hydrophobic adsorption chromatography on underivitized polystyrene-divinylbenzene (for example, Amberlite XAD); silica gel adsorption chromatography; ion exchange chromatography on carboxymethylcellulose; partition chromatography, e.g. on Sephadex G-25, LH-20 or countercurrent distribution; high performance liquid chromatography (HPLC), especially reverse-phase HPLC on octyl- or octadecylsilyl-silica bonded phase column packing.

Molecular weights of these therapeutic peptides are determined using Fast Atom Bombardment (FAB) Mass Spectroscopy.

The therapeutic peptides of the invention may be synthesized with N- and C-terminal protecting groups for use as pro-drugs.

### (1) N-Terminal Protective Groups

As discussed above, the term "N-protecting group" refers to those groups intended to protect the α-N-terminal of an amino acid or peptide or to otherwise protect the amino group of an amino acid or peptide against undesirable reactions during synthetic procedures. Commonly used N-protecting groups are disclosed in Greene, "Protective Groups In Organic Synthesis," (John Wiley & Sons, New York (1981)), which is hereby incorporated by reference. Additionally, protecting groups can be used as pro-drugs which are readily cleaved *in vivo,* for example, by enzymatic hydrolysis, to release the biologically active parent. α-N-protecting groups comprise loweralkanoyl groups such as formyl, acetyl ("Ac"), propionyl, pivaloyl, t-butylacetyl and the like; other acyl groups include 2-chloroacetyl, 2-bromoacetyl, trifluoroacetyl, trichloroacetyl, phthalyl, o-nitrophenoxyacetyl, -chlorobutyryl, benzoyl, 4-chlorobenzoyl, 4-bromobenzoyl, 4-nitrobenzoyl and the like; sulfonyl groups such as benzenesulfonyl, p-toluenesulfonyl and the like; carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4-dimethoxybenzyloxycarbonyl, 4-ethoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxycarbonyl, t-butyloxycarbonyl, diisopropylmethoxycarbonyl, isopropyloxycarbonyl, ethoxycarbonyl, methoxycarbonyl, allyloxycarbonyl, 2,2,2,-trichloroethoxycarbonyl, phenoxycarbonyl, 4-nitrophenoxycarbonyl, fluorenyl-9-methoxycarbonyl, cyclopentyloxycarbonyl, adamantyloxycarbonyl, cyclohexyloxycarbonyl, phenylthiocarbonyl and the like; arylalkyl groups such as benzyl, triphenylmethyl, benzyloxymethyl, 9-fluorenylmethyloxycarbonyl (Fmoc) and the like and silyl groups such as trimethylsilyl and the like.

### (2) Carboxy Protective Groups

As discussed above, the term "carboxy protecting group" refers to a carboxylic acid protecting ester or amide group employed to block or protect the carboxylic acid functionality while the reactions involving other functional sites of the compound are performed. Carboxy protecting groups are disclosed in Greene, "Protective Groups in Organic Synthesis" pp. 152-186 (1981), which is hereby incorporated by reference. Additionally, a carboxy protecting group can be used as a pro-drug whereby the carboxy protecting group can be readily cleaved *in vivo*, for example by enzymatic hydrolysis, to release the biologically active parent. Such carboxy protecting groups are well known to those skilled in the art, having been extensively used in the protection of carboxyl groups in the penicillin and cephalosporin fields as described in U.S. Pat. Nos. 3,840,556 and 3,719,667, the disclosures of which are hereby incorporated herein by reference. Representative carboxy protecting groups are C₁ -C₈ loweralkyl (e.g., methyl, ethyl or t-butyl and the like); arylalkyl such as phenethyl or benzyl and substituted derivatives thereof such as alkoxybenzyl or nitrobenzyl groups and the like; arylalkenyl such as phenylethenyl and the like; aryl and substituted derivatives thereofsuch as 5-indanyl and the like; dialkylaminoalkyl such as dimethylaminoethyl and the like); alkanoyloxyalkyl groups such as acetoxymethyl, butyryloxymethyl, valeryloxymethyl, isobutyryloxymethyl, isovaleryloxymethyl, 1-(propionyloxy)-1-ethyl, 1-(pivaloyloxyl)-1-ethyl, 1-methyl-1-(propionyloxy)-1-ethyl, pivaloyloxymethyl, propionyloxymethyl and the like; cycloalkanoyloxyalkyl groups such as cyclopropylcarbonyloxymethyl, cyclobutylcarbonyloxymethyl, cyclopentylcarbonyloxymethyl, cyclohexylcarbonyloxymethyl and the like; aroyloxyalkyl such as benzoyloxymethyl, benzoyloxyethyl and the like; arylalkylcarbonyloxyalkyl such as benzylcarbonyloxymethyl, 2-benzylcarbonyloxyethyl and the like; alkoxycarbonylalkyl or cycloalkyloxycarbonylalkyl such as methoxycarbonylmethyl, cyclohexyloxycarbonylmethyl, 1-methoxycarbonyl-1-ethyl and the like; alkoxycarbonyloxyalkyl or cycloalkyloxycarbonyloxyalkyl such as methoxycarbonyloxymethyl, t-butyloxycarbonyloxymethyl, 1-ethoxycarbonyloxy-1-ethyl, 1-cyclohexyloxycarbonyloxy-1-ethyl and the like; aryloxycarbonyloxyalkyl such as 2-(phenoxycarbonyloxy)ethyl, 2-(5-indanyloxycarbonyloxy)ethyl and the like; alkoxyalkylcarbonyloxyalkyl such as 2-(1-methoxy-2-methylpropan-2-oyloxy)ethyl and like; arylalkyloxycarbonyloxyalkyl such as 2-(benzyloxycarbonyloxy)ethyl and the like; arylalkenyloxycarbonyloxyalkyl such as 2-(3-phenylpropen-2-yloxycarbonyloxy)ethyl and the like; alkoxycarbonylaminoalkyl such as t-butyloxycarbonylaminomethyl and the like; alkylaminocarbonylaminoalkyl such as methylaminocarbonylaminomethyl and the like; alkanoylaminoalkyl such as acetylaminomethyl and the like; heterocycliccarbonyloxyalkyl such as 4-methylpiperazinylcarbonyloxymethyl and the like; dialkylaminocarbonylalkyl such as dimethylaminocarbonylmethyl, diethylaminocarbonylmethyl and the like; (5-(loweralkyl)-2-oxo-1,3-dioxolen-4-yl)alkyl such as (5-t-butyl-2-oxo-1,3-dioxolen-4-yl)methyl and the like, and (5-phenyl-2-oxo-1,3-dioxolen-4-yl)alkyl such as (5-phenyl-2-oxo-1,3-dioxolen-4-yl)methyl and the like.

Representative amide carboxy protecting groups are aminocarbonyl and loweralkylaminocarbonyl groups.

Preferred carboxy-protected compounds of the invention are compounds wherein the protected carboxy group is a loweralkyl, cycloalkyl or arylalkyl ester, for example, methyl ester, ethyl ester, propyl ester, isopropyl ester, butyl ester, sec-butyl ester, isobutyl ester, amyl ester, isoamyl ester, octyl ester, cyclohexyl ester, phenylethyl ester and the like or an alkanoyloxyalkyl, cycloalkanoyloxyalkyl, aroyloxyalkyl or an arylalkylcarbonyloxyalkyl ester. Preferred amide carboxy protecting groups are loweralkylaminocarbonyl groups. For example, aspartic acid may be protected at the α-C-terminal by an acid labile group (e.g. t-butyl) and protected at the β-C-terminal by a hydrogenation labile group (e.g. benzyl) then deprotected selectively during synthesis.

Alternatively, it is also possible to obtain fragments of the peptides by fragmenting the naturally occurring amino acid sequence, using, for example, a proteolytic enzyme according to methods well known in the art. Further, it is possible to obtain the desired fragments of the therapeutic peptide through the use of recombinant DNA technology using methods well known in the art.

### 4. Modification of Therapeutic Peptides

The manner of producing the modified therapeutic peptides of the present invention will vary widely, depending upon the nature of the various elements comprising the molecule. The synthetic procedures will be selected so as to be simple, provide for high yields, and allow for a highly purified stable product. Normally, the reactive group will be created as the last stage, for example, with a carboxyl group, esterification to form an active ester will be the last step of the synthesis. Specific methods for the production of modified therapeutic peptides of the present invention are described below.

Generally, the modified therapeutic peptides of the present invention may be made using blind or structure activity relationship (SAR) driven substitution. SAR is an analysis which defines the relationship between the structure of a molecule and its pharmacological activity for a series of compounds. Various studies relative to individual therapeutic peptides show how the activity of the peptide varies according to the variation of chemical structure or chemical properties. More specifically, first the therapeutic activity of the free peptide is assayed. Next, the peptide is modified according to the invention, either at the N-terminus, at the C-terminus, or in the interior of the peptide with the linking group only. The linking group will include the reactive group as discussed above. The therapeutic activity of this modified peptide-linking group is assayed next, and based on the detected activity a decision is made regarding the modification site. Next, the peptide conjugate is prepared and its therapeutic ativity is determined. If the therapeutic activity of the peptide after conjugation is not substantially reduced (i.e. if the therapeutic activity is reduced by less than 10 fold), then the stability of the peptide is measured as indicated by its *in vivo* lifetime. If the stability is not improved to a desired level, then the peptide is modified at an alternative site, and the procedure is repeated until a desired level of therapeutic activity and a desired stability are achieved.

More specifically, each therapeutic peptide selected to undergo the derivatization with a linker and a reactive group will be modified according to the following criteria: if a terminal carboxylic group is available on the therapeutic peptide and is not critical for the retention of pharmacological activity, and no other sensitive functional group is present on the therapeutic peptide, then the carboxylic acid will be chosen as attachment point for the linker-reactive group modification. If the terminal carboxylic group is involved in pharmacological activity, or if no carboxylic acids are available, then any other sensitive functional group not critical for the retention of pharmacological activity will be selected as the attachment point for the linker-reactive group modification. If several sensitive functional groups are available on a therapeutic peptide, a combination of protecting groups will be used in such a way that after addition of the linker/reactive group and deprotection of all the protected sensitive functional groups, retention of pharmacological activity is still obtained. If no sensitive functional groups are available on the therapeutic peptide, or if a simpler modification route is desired, synthetic efforts will allow for a modification of the original peptide in such a way that retention of biological activity and retention of receptor or target specificity is obtained. In this case the modification will occur at the opposite end of the peptide.

An NHS derivative may be synthesized from a carboxylic acid in absence of other sensitive functional groups in the therapeutic peptide. Specifically, such a therapeutic peptide is reacted with N-hydroxysuccinimide in anhydrous CH₂ Cl₂ and EDC, and the product is purified by chromatography or recrystallized from the appropriate solvent system to give the NHS derivative.

Alternatively, an NHS derivative may be synthesized from a therapeutic peptide that contains an amino and/or thiol group and a carboxylic acid. When a free amino or thiol group is present in the molecule, it is preferable to protect these sensitive functional groups prior to perform the addition of the NHS derivative. For instance, if the molecule contains a free amino group, a transformation of the amine into a Fmoc or preferably into a tBoc protected amine is necessary prior to perform the chemistry described above. The amine functionality will not be deprotected after preparation of the NHS derivative. Therefore this method applies only to a compound whose amine group is not required to be freed to induce a pharmacological desired effect. If the amino group needs to be freed to retain the original biological properties of the molecule, then another type of chemistry described in example 3-6 has to be performed.

In addition, an NHS derivative may be synthesized from a therapeutic peptide containing an amino or a thiol group and no carboxylic acid. When the selected molecule contains no carboxylic acid, an array of bifunctional linkers can be used to convert the molecule into a reactive NHS derivative. For instance, ethylene glycol-bis(succinimydylsuccinate) (EGS) and triethylamine dissolved in DMF and added to the free amino containing molecule (with a ratio of 10:1 in favor of EGS) will produce the mono NHS derivative. To produce an NHS derivative from a thiol derivatized molecule, one can use N-[-maleimidobutyryloxy]succinimide ester (GMBS) and triethylamine in DMF. The maleimido group will react with the free thiol and the NHS derivative will be purified from the reaction mixture by chromatography on silica or by HPLC.

An NHS derivative may also be synthesized from a therapeutic peptide containing multiple sensitive functional groups. Each case will have to be analyzed and solved in a different manner. However, thanks to the large array of protecting groups and bifunctional linkers that are commercially available, this invention is applicable to any therapeutic peptide with preferably one chemical step only to derivatize the therapeutic peptide (as described in example 1 or 3) or two steps (as described in example 2 and involving prior protection of a sensitive group) or three steps (protection, activation and deprotection). Under exceptional circumstances only, would we require to use multiple steps (beyond three steps) synthesis to transform a therapeutic peptide into an active NHS or maleimide derivative.

A maleimide derivative may also be synthesized from a therapeutic peptide containing a free amino group and a free carboxylic acid. To produce a maleimide derivative from a amino derivatized molecule, one can use N-[γ-m a leimidobutyryloxy]succinimide ester (GMBS) and triethylamine in DMF. The succinimide ester group will react with the free amino and the maleimide derivative will be purified from the reaction mixture by crystallization or by chromatography on silica or by HPLC.

Finally, a maleimide derivative may be synthesized from a therapeutic peptide containing multiple other sensitive functional groups and no free carboxylic acids. When the selected molecule contains no carboxylic acid, an array of bifunctional crosslinking reagents can be used to convert the molecule into a reactive NHS derivative. For instance maleimidopropionic acid (MPA) can be coupled to the free amine to produce a maleimide derivative through reaction of the free amine with the carboxylic group of MPA using HBTU/HOBt/DIEA activation in DMF. Alternatively, a lysine residue can be added on the C-terminus end of the peptide to allow for conjugation onto the -amino group of the lysine as described in the examples below. This added lysine allows for simple and effcient modification at the C-terminus of the peptide while keeping the terminal end capped by an amide function as designed by the initial choice of the resin

Many other commercially available heterobifunctional crosslinking reagents can alternatively be used when needed. A large number of bifunctional compounds are available for linking to entities. Illustrative reagents include: azidobenzoyl hydrazide, N-[4-(p-azidosalicylamino)butyl]-3'-[2'-pyridyldithio)propionamide), bis-sulfosuccinimidyl suberate, dimethyl adipimidate, disuccinimidyl tartrate, N-y-maleimidobutyryloxysuccinimide ester, N-hydroxy sulfosuccinimidyl-4-azidobenzoate, N-succinimidyl [4-azidophenyl]-1,3'-dithiopropionate, N-succinimidyl [4-iodoacetyl]aminobenzoate, glutaraldehyde, and succinimidyl 4-[N-maleimidomethyl]cyclohexane-1-carboxylate.

Even more specifically, the peptides are preferably modified according to the nature of their substituants and the presence or absence of free cysteines. Most peptides can be gathered into three distinct categories: (1) peptides that contain no cysteines; (2) peptides that contain one cysteine, (3) peptides that contain two cysteines as a disulfide bridge (cystine); and (4) peptides that contain multiple cysteines.

### A. Peptides that Contain No Cysteines

Where the peptide contains no cysteine, addition from the C terminus is performed with all residues cleaved from the support resin and fully protected. Solution phase activation of C-terminus with EDC and NHS can be reacted with an amino-alkyl-maleimide in one pot. The peptide is then fully deprotected. Alternatively, a lysine residue can be added on the C-terminus of the peptide to allow modification at the epsilon amino group of the lysine while keeping the carboxy terminus capped with an amide group. Such an addition of a lysine residue is preferably performed only where the addition does not substantially affect the therapeutic activity of the peptide. The generalized reaction scheme for C-terminus modification of peptides that contain no cysteines is illustrated in the schematic diagram below.

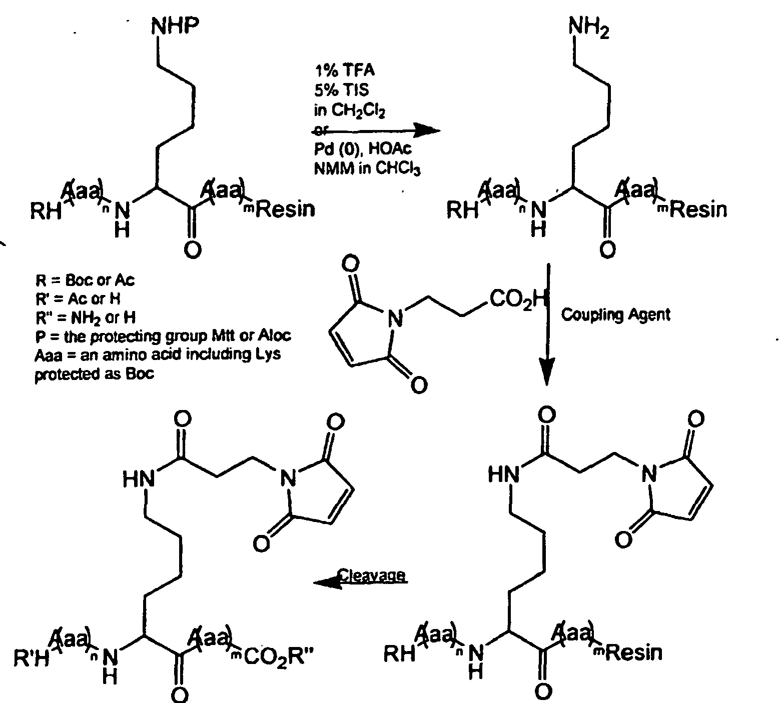

If an N-terminus modfication is favored, and again for a peptide containing no cysteine, addition on the N terminus is performed with all residues still on the support resin and fully protected. Addition of activated NHS-Mal bifunctional linker could be performed on deprotected N-terminus with peptide still on resin. The peptide is then fully deprotected. Examples of therapeutic peptides that contain no cystein and undergo a C-terminus modification are described in examples 7-26. Examples of therapeutic peptides that contain no cystein and undergo a N-terminus modification are described in examples 27-38. The generalized reaction scheme for N-terminus modification of peptides that contain no cysteines is illustrated in the schematic diagrams below, using hetero NHS maleimide (GMBS like) and 3-MPA, respectively.

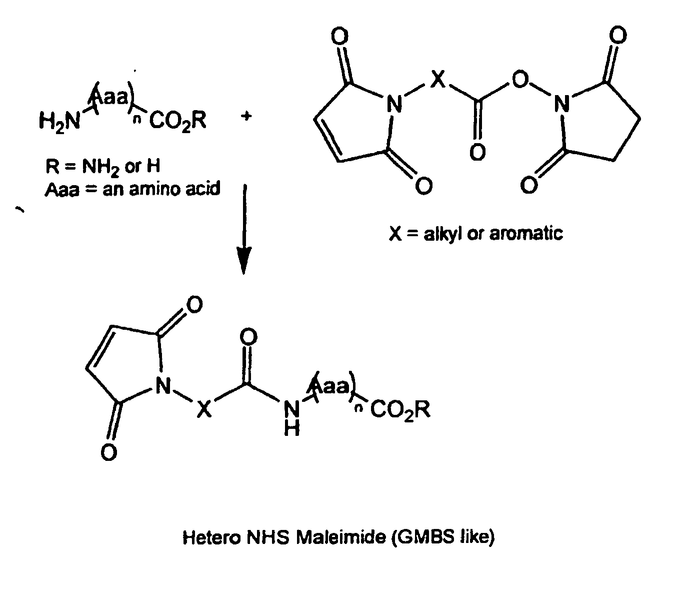

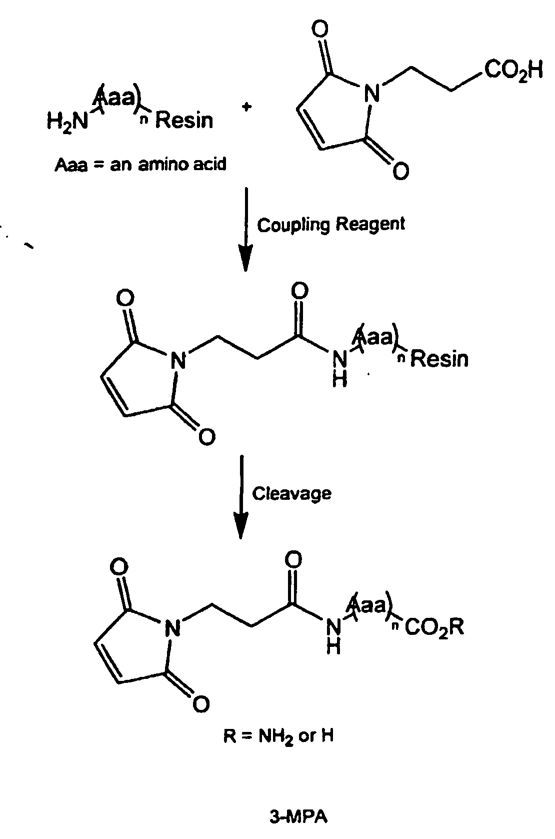

Alternatively, the peptide may be modified at an internal amino acid (i.e. neither at the C-terminus nor at the N-terminus). The generalized reaction scheme for modification at an internal amino acid of a peptide that contains no free cysteines is illustrated in the schematic diagrams below, using homo bis NHS and hetero NHS maleimide.

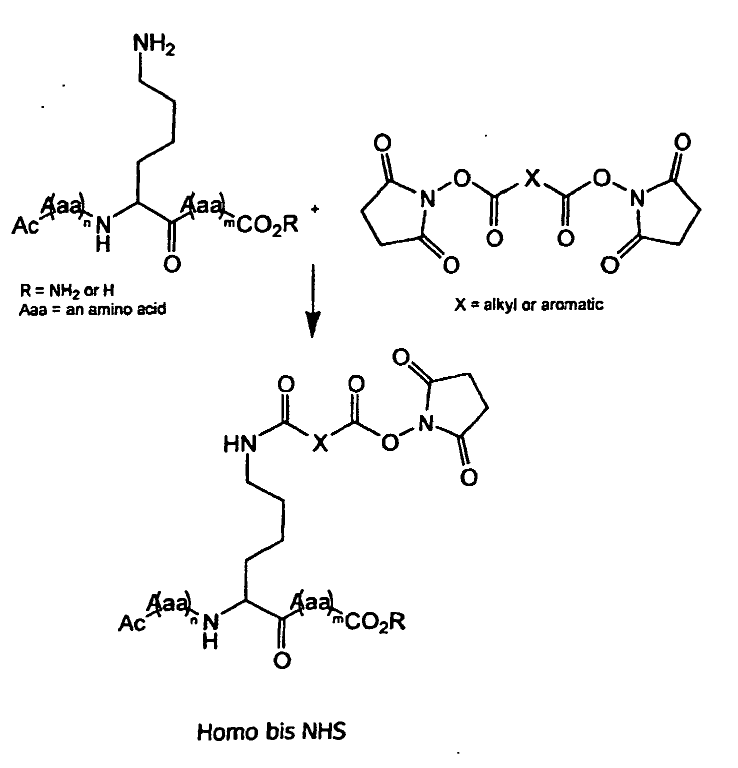

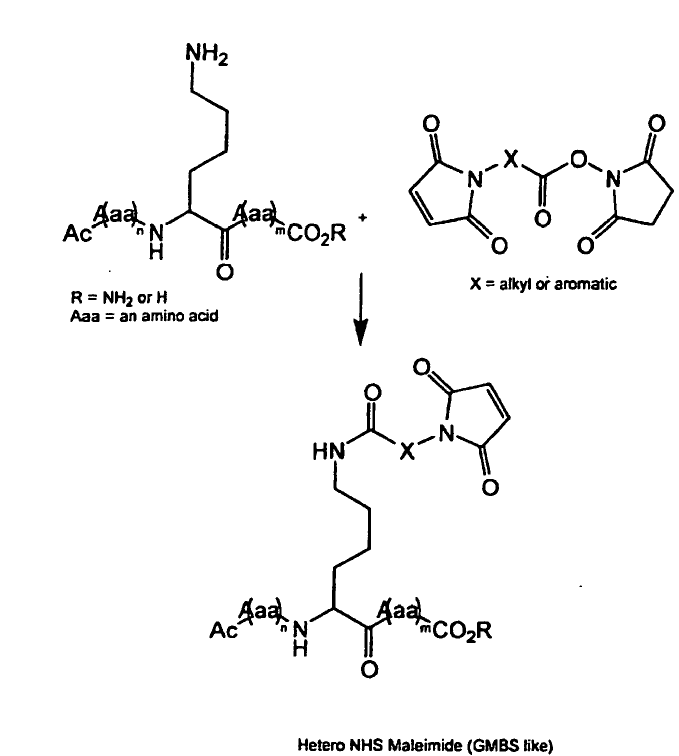

Peptides that contain no cysteine and can be modified as described above include fragments of the Kringle 5 peptide, of the GLP-1 peptide, of dynorphin A, human growth hormone releasing factor, the 1-24 fragment of human neuropeptide Y, and human secretin. Full description of the chemistry for each of these peptides is reported in the Example section.

### B. Peptides that Contain One Cysteine

Where the peptide contains one cysteine, the cysteine must stay capped after addition of the maleimide. If the cysteine is involved in binding site, assessment has to be made of how much potency is lost is cysteine is capped by a protecting group. If the cysteine can stay capped, then the synthetic path is similar to that described in section A above for either a C or an N terminus modification.

Alternatively, the peptide may be modified at an internal amino acid (i.e. neither at the C-terminus nor at the N-terminus). The generalized reaction scheme for modification at an internal amino acid of a peptide that contains no cysteines is illustrated in the schematic diagram below, using homobis maleimide and hetero NHS maleimide (GMBS like).

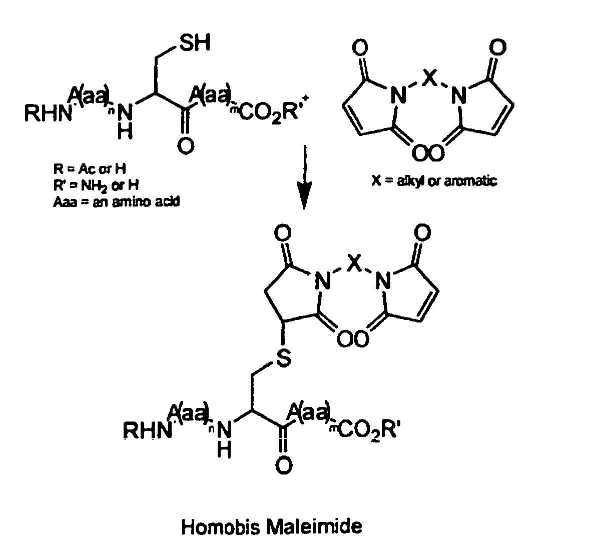

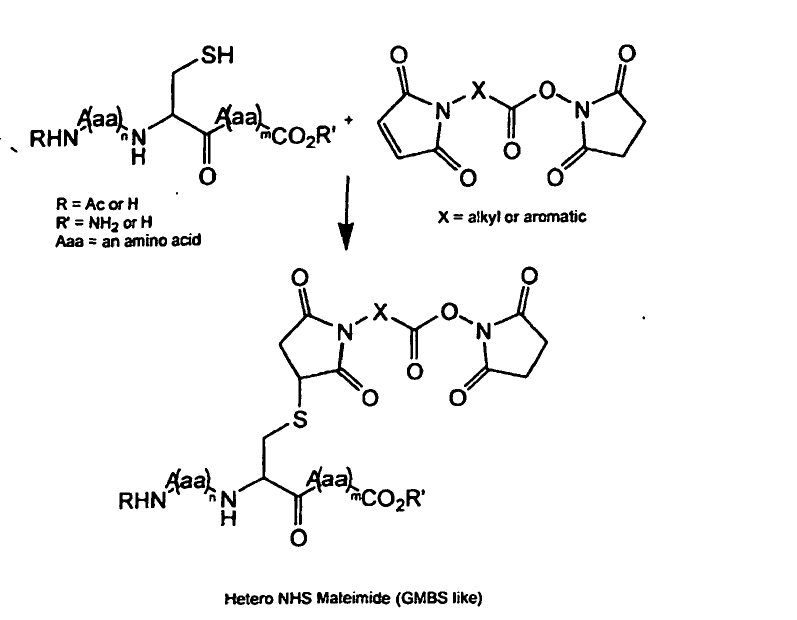

Examples of therapeutic peptides that contain one cysteine include G_{α} (the alpha subunit of Gtherapeutic peptide binding protein), the 724-739 fragment of rat brain nitric oxide synthase blocking peptide, the alpha subunit 1-32 fragment of human [Tyr0] inhibin, the 254-274 fragment of HIV envelope protein, and P34cdc2 kinase fragment.

### C. Peptides that Contain Two Cysteines as a Disulfide Bridge (Cystine)

Where the peptide contains two cysteines as a disulfide bridge, the peptide is cleaved from the support resin before addition of the maleimide. For a modification of the peptide from the C terminus end, all protecting groups are present except at the carboxy terminus (which stays unprotected due to cleavage from the support resin) and at the two cysteines, which need to be deprotected when peptide is cleaved from resin. Mild air oxidation yields the disulfide bridge, and the peptide can be purified at that stage. Solution phase chemistry is then required to activate the C-terminus in presence of the disulfide bridge and add the maleimide (through an amino-alkyl-maleimide) to the C-terminus. The peptide is then fully deprotected.

For a modification of the peptide at the N-terminus, the peptide can remain on the support resin. The two cysteines are selectively deprotected before addition of the maleimide. Air oxidation, potentially helped by a catalyst (heterogeneous) can yield the disulfide with the peptide still on the resin. Maleimide is then added on the N-terminus and peptide cleaved from resin and fully deprotected. The generalized reaction scheme for modification at an internal amino acid of a peptide that contains two cysteines in a disulfide bridge is illustrated in the schematic diagram below.

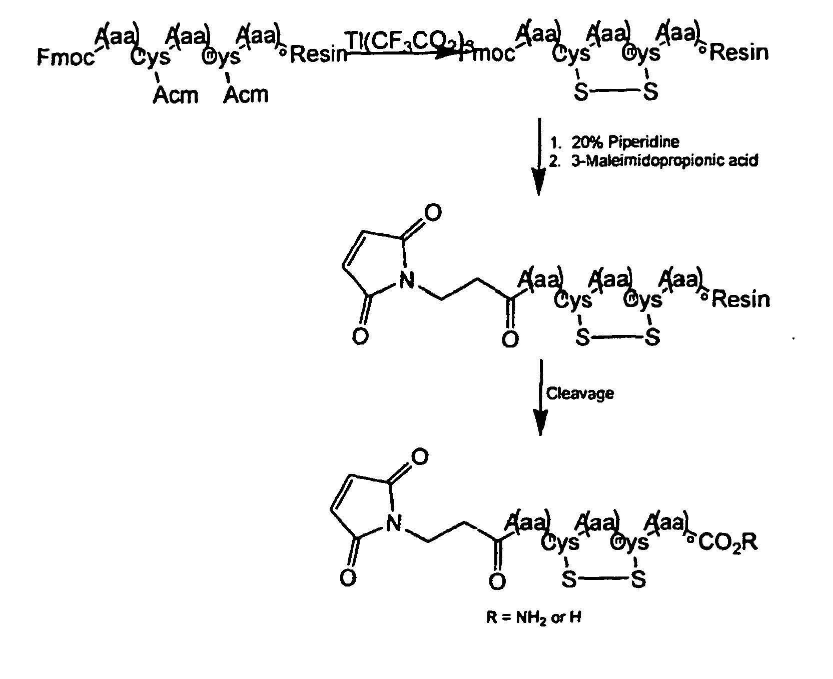

Alternatively, the peptide may be modified at an internal amino acid (i.e. neither at the C-terminus nor at the N-terminus). The generalized reaction scheme for modification at an internal amino acid of a peptide that contains two cysteines in a disulfide bridge is illustrated in the schematic diagram below.

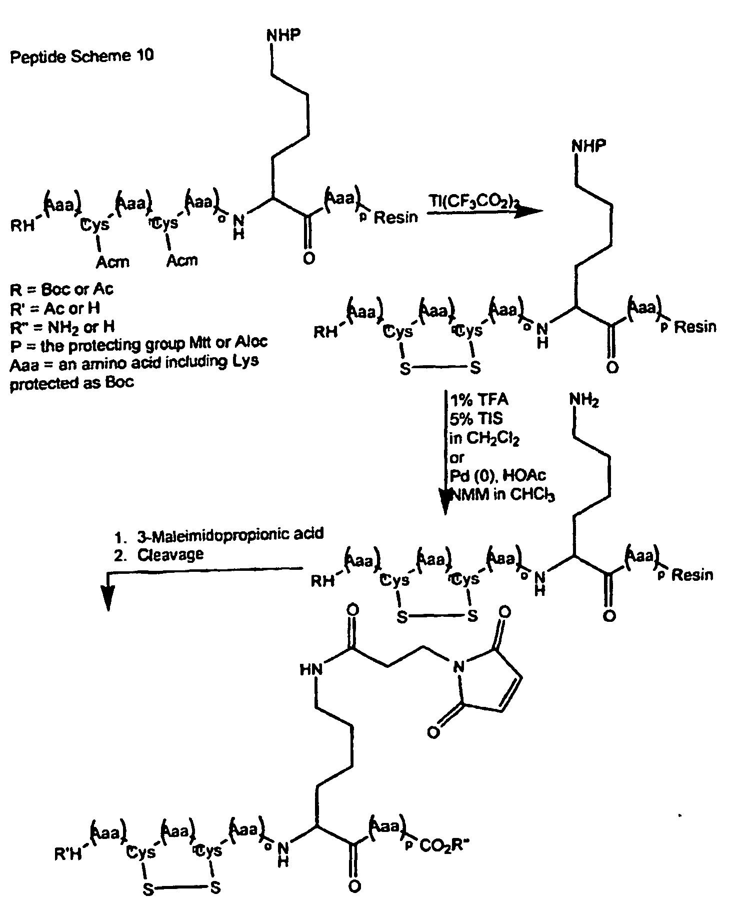

Examples of therapeutic peptides that contain two cysteines as a disulfide bridge include human osteocalcin 1-49, human diabetes associated peptide, the 5-28 fragment of human/canine atrial natriuretic peptide, bovine bactenecin, and human [Tyr0]-cortistatin 29,

### D. Peptides Containing Multiple Cysteines

Where the peptide contains multiple cysteines as disulfide bridges, the peptide is cleaved from the support resin before addition of the maleimide. For a modification of the peptide from the C terminus end, all protecting groups are present except at the carboxy terminus (which stays unprotected due to cleavage from the support resin) and at the two cysteines that are supposed to build a disulfide bridge. Cysteines that are involved in other disulfide bridges are deprotected sequencially in pairs using a choice of protecting groups. It is recommended to build and purify each bridge one at a time prior to moving on to the next bridge. Mild air oxidation yields the disulfide bridge, and the peptide should be purified at each stage. Solution phase chemistry is then required to activate the C-terminus in presence of the disulfide bridge and add the maleimide (through an amino-alkyl-maleimide) to the C-terminus. The peptide is then fully deprotected.

For a modification of the peptide from the N terminus end, one can leave the peptide on the support resin and selectively deprotect the first two cysteines to build the disulfide under mild air oxidation. Subsequent deprotection will offer the other disulfides before addition of the maleimide. Air oxidation, potentially helped by a catalyst (heterogeneous) can yield the disulfides with the peptide still on the resin. Maleimide is then added on the N-terminus and peptide cleaved from resin and fully deprotected.

Alternatively, the peptide may be modified at an internal amino acid (i.e. neither at the C-terminus nor at the N-terminus).

Peptides containing multiple cysteines include human endothelins and [Lys4] Sarafotoxin S6c.

### 5. Administration of the Modified Therapeutic Peptides

The modified therapeutic peptide will be administered in a physiologically acceptable medium, e.g. deionized water, phosphate buffered saline (PBS), saline, aqueous ethanol or other alcohol, plasma, proteinaceous solutions, mannitol, aqueous glucose, alcohol, vegetable oil, or the like. Other additives which may be included include buffers, where the media are generally buffered at a pH in the range of about 5 to 10, where the buffer will generally range in concentration from about 50 to 250 mM, salt, where the concentration of salt will generally range from about 5 to 500 mM, physiologically acceptable stabilizers, and the like. The compositions may be lyophilized for convenient storage and transport.

The modified Itherapeutic peptides will for the most part be administered orally, parenterally, such as intravascularly (IV), intraarterially (IA), intramuscularly (IM), subcutaneously (SC), or the like. Administration may in appropriate situations be by transfusion. In some instances, where reaction of the functional group is relatively slow, administration may be oral, nasal, rectal, transdermal or aerosol, where the nature of the conjugate allows for transfer to the vascular system. Usually a single injection will be employed although more than one injection may be used, if desired. The modified therapeutic peptides may be administered by any convenient means, including syringe, trocar, catheter, or the like. The particular manner of administration will vary depending upon the amount to be administered, whether a single bolus or continuous administration, or the like. Preferably, the administration will be intravascularly, where the site of introduction is not critical to this invention, preferably at a site where there is rapid blood flow, e.g., intravenously, peripheral or central vein. Other routes may find use where the administration is coupled with slow release techniques or a protective matrix. The intent is that the Itherapeutic peptides be effectively distributed in the blood, so as to be able to react with the blood components. The concentration of the conjugate will vary widely, generally ranging from about 1 pg/ml to 50 mg/ml. The total administered intravascularly will generally be in the range of about 0.1 mg/ml to about 10 mg/ml, more usually about 1 mg/ml to about 5 mg/ml.

By bonding to long-lived components of the blood, such as immunoglobulin, serum albumin, red blood cells and platelets, a number of advantages ensue. The activity of the modified therapeutic peptides compound is extended for days to weeks. Only one administration need be given during this period of time. Greater specificity can be achieved, since the active compound will be primarily bound to large molecules, where it is less likely to be taken up intracellularly to interfere with other physiological processes.

The formation of the covalent bond between the blood component may occur *in vivo* or *ex vivo*. For *ex vivo* covalent bond formation, the modified Itherapeutic peptide is added to blood, serum or saline solution containing human serum albumin or IgG to permit covalent bond formation between the modified therapeutic peptide and the blood component. In a preferred format, the therapeutic peptide is modified with maleimide and it is reacted with human serum albumin in saline solution. Once the modified therapeutic peptide has reacted with the blood component, to form a therapeutic peptide-protein conjugate, the conjugate may be administered to the patient.

Alternatively, the modified therapeutic peptide may be administered to the patient directly so that the covalent bond forms between the modified Itherapeutic peptide and the blood component *in vivo*.

In addition, where localized delivery of therapeutic peptides is desired, several methods of delivery may be used:

### A. Open Surgical Field Lavage

There are a number of indications for local therapeutic compounds which would entail administration of the therapeutic compound as an adjunct to open surgery. In these cases, the therapeutic compound would either be lavaged in the surgical site (or a portion of that site) prior to closure, or the therapeutic compound would be incubated for a short time in a confined space (e.g., the interior of a section of an artery following an endarterectomy procedure or a portion of GI tract during resection) and the excess fluid subsequently evacuated.

### B. Incubation of Tissue Grafts

Tissue grafts such as autologous and xenobiotic vein/artery and valve grafts as well as organ grafts can be pretreated with therapeutic compounds that have been modified to permit covalent bond formation by either incubating them in a therapeutic solution and/or perfusing them with such a solution.

### C. Catheter Delivery

A catheter is used to deliver the therapeutic compound either as part of an endoscopic procedure into the interior of an organ (e.g., bladder, GI tract, vagina/uterus) or adjunctive to a cardiovascular catheter procedure such as a balloon angioplasty. Standard catheters as well as newer drug delivery and iontophoretic catheters can be utilized.

### D. Direct Injection

For certain poorly vascularized spaces such as intra-articular joint spaces, a direct injection of a therapeutic compound may be able to bioconjugate to surface tissues and achieve a desirable duration of drug effect. Other applications could include intra medullary, intratumor, intravaginal, intrauterine, intra intestinal, intra eustachian tube, intrathecal, subcutaneous, intrarticular, intraperitoneal or intraocular injections as weel as via bronchoscope, via nasogastiric tube and via nophrostomy.

### 6. Monitoring the Presence of Modified Therapeutic Peptide Derivatives

Another aspect of this invention relates to methods for determining the concentration of the therapeutic peptides and/or analogs, or their derivatives and conjugates in biological samples (such as blood) and determining the peptidase stability of the modified peptides. The blood of the mammalian host may be monitored for the presence of the modified therapeutic peptide compounds one or more times. By taking a portion or sample of the blood of the host, one may determine whether the therapeutic peptide has become bound to the long-lived blood components in sufficient amount to be therapeutically active and, thereafter, the level of therapeutic peptide compound in the blood. If desired, one may also determine to which of the blood components the therapeutic peptide derivative molecule is bound. This is particularly important when using non-specific therapeutic peptides. For specific maleimide-therapeutic peptides, it is much simpler to calculate the half life of serum albumin and IgG.

One method for determining the concentration of the therapeutic peptide, analogs, derivatives and conjugates is to use antibodies specific to the therapeutic peptides or therapeutic peptide analogs or their derivatives and conjugates, and to use such antibodies as a treatment for toxicity potentially associated with such therapeutic peptides, analogs, and/or their derivatives or conjugates. This is advantageous because the increased stability and life of the therapeutic peptides *in vivo* in the patient might lead to novel problems during treatment, including increased possibility for toxicity. It should be mentioned, however, that in some cases, the traditional antibody assay may not recognize the difference between cleaved and uncleaved therapeutic peptides. In such cases, other assay techniques may be employed, for example LC/MS (Liquid Chromatography / Mass Spectrometry).

The use of antibodies, either monoclonal or polyclonal, having specificity for a particular therapeutic peptide, analog or derivative thereof, can assist in mediating any such problem. The antibody may be generated or derived from a host immunized with the particular therapeutic peptide, analog or derivative thereof, or with an immunogenic fragment of the agent, or a synthesized immunogen corresponding to an antigenic determinant of the agent. Preferred antibodies will have high specificity and affinity for native, derivatized and conjugated forms of the therapeutic peptide or therapeutic peptide analog. Such antibodies can also be labeled with enzymes, fluorochromes, or radiolabels.

Antibodies specific for derivatized therapeutic peptides may be produced by using purified therapeutic peptides for the induction of derivatized therapeutic peptide-specific antibodies. By induction of antibodies, it is intended not only the stimulation of an immune response by injection into animals, but analogous steps in the production of synthetic antibodies or other specific binding molecules such as screening of recombinant immunoglobulin libraries. Both monoclonal and polyclonal antibodies can be produced by procedures well known in the art. In some cases, the use of monoclonal antibodies may be preferred over polyclonal antibodies, such as when degradation occurs over an area not covered by epitope/antibody recognition.

The antibodies may be used to treat toxicity induced by administration of the therapeutic peptide, analog or derivative thereof, and may be used *ex vivo* or *in vivo. Ex vivo* methods would include immuno-dialysis treatment for toxicity employing antibodies fixed to solid supports. *In vivo* methods include administration of antibodies in amounts effective to induce clearance of antibody-agent complexes.

The antibodies may be used to remove the therapeutic peptides, analogs or derivatives thereof, and conjugates thereof, from a patient's blood *ex vivo* by contacting the blood with the antibodies under sterile conditions. For example, the antibodies can be fixed or otherwise immobilized on a column matrix and the patient's blood can be removed from the patient and passed over the matrix. The therapeutic peptide analogs, derivatives or conjugates, will bind to the antibodies and the blood containing a low concentration of the therapeutic peptide, analog, derivative or conjugate, then may be returned to the patient's circulatory system. The amount of therapeutic peptide compound removed can be controlled by adjusting the pressure and flow rate. Preferential removal of the therapeutic peptides, analogs, derivatives and conjugates from the plasma component of a patient's blood can be affected, for example, by the use of a semipermeable membrane, or by otherwise first separating the plasma component from the cellular component by ways known in the art prior to passing the plasma component over a matrix containing the anti-therapeutic antibodies. Alternatively the preferential removal of therapeutic peptide-conjugated blood cells, including red blood cells, can be effected by collecting and concentrating the blood cells in the patient's blood and contacting those cells with fixed anti-therapeutic antibodies to the exclusion of the serum component of the patient's blood.

The antibodies can be administered *in vivo,* parenterally, to a patient that has received the therapeutic peptide, analogs, derivatives or conjugates for treatment. The antibodies will bind the therapeutic peptide compounds and conjugates. Once bound the therapeutic peptide, activity will be hindered if not completely blocked thereby reducing the biologically effective concentration of therapeutic peptide compound in the patient's bloodstream and minimizing harmful side effects. In addition, the bound antibody-therapeutic peptide complex will facilitate clearance of the therapeutic peptide compounds and conjugates from the patient's blood stream.

The invention having been fully described is now exemplified by the following non-limiting examples.

### EXAMPLES

### A. General Method of Synthesis of a Modified Therapeutic Peptide

Solid phase peptide synthesis of the modified peptide on a 100 µmole scale was performed on a Symphony Peptide Synthesizer using Fmoc protected Rink Amide MBHA resin, Fmoc protected amino acids, O-benzotriazol-1-yl-*N*, *N*, *N*', *N*'-tetramethyl-uronium hexafluorophosphate (HBTU) in *N*,*N*-dimethylformamide (DMF) solution and activation with *N*-methyl morpholine (NMM), and piperidine deprotection of Fmoc groups (Step 1). The deprotection of the terminal Fmoc group is accomplished using 20% piperidine (Step 2) followed by either the coupling of 3-maleimidopropionic acid (3-MPA), the coupling of acetic acid or the coupling of one or multiple Fmoc-AEEA followed by the coupling of 3-MPA (Step 3). Resin cleavage and products isolation are performed using 86% TFA/5% TIS/5% H₂O/2% thioanisole and 2% phenol, followed by precipitation by dry-ice cold Et₂O (Step 4). The products are purified by preparative reverse phase HPLC using a Varian (Rainin) preparative binary HPLC system using a Dynamax C₁₈, 60Å, 8 µm, 21 mm x 25 cm column equipped with a Dynamax C₁₈, 60Å. 8 µm guard module, 21 mm x 25 cm column and UV detector (Varian Dynamax UVD II) at λ 214 and 254 nm. The product should have >95% purity as determined by RP-HPLC mass spectrometry using a Hewlett Packard LCMS-1100 series spectrometer equipped with a diode array detector and using electro-spray ionization.

### B. Alteration of the Native Peptide Chain

To facilitate modification of the peptide, one or more amino acid residues may be added to the peptide as described in examples 1 to 5, and/or one or more amino acid residues may be replaced with other amino acid residues. This alteration aids attachment of the reactive group.

### Example 1 - Addition of Lys at C-Terminus of Kringle-5

### Preparation of NAc-Pro-Arg-Lys-Leu-Tyr-Asp-Tyr-Lys-NH₂.3TFA

Using automated peptide synthesis, the following protected amino acids were sequentially added to Rink Amide MBHA resin: Fmoc-Lys(Boc)-OH, Fmoc-Tyr(tBu)OH, Fmoc-Asp(OtBu)-OH, Fmoc-Tyr(tBu)OH, Fmoc-Leu-OH, Fmoc-Lys(Boc)-OH, Fmoc-Arg(Pbf)-OH, Fmoc-Pro-OH. Deblocking of the Fmoc group the the N-terminal of the resin-bound amino acid was performed with 20% piperidine in DMF for about 15-20 minutes. Coupling of the acetic acid was performed under conditions similar to amino acid coupling. Final cleavage from the resin was performed using cleavage mixture as described above. The product was isolated by precipitation and purified by preparative HPLC to afford the desired product as a white solid upon lyophilization .

### Example 2 - Addition of Lys at C-Terminus of Kringle-5

### Preparation of NAc-Arg-Lys-Leu-Tyr-Asp-Tyr-Lys-NH₂.2TFA.3TFA

Using automated peptide synthesis, the following protected amino acids were sequentially added to Rink Amide MBHA resin: Fmoc-Lys(Boc)-OH, Fmoc-Tyr(tBu)OH, Fmoc-Asp(OtBu)-OH, Fmoc-Tyr(tBu)OH, Fmoc-Leu-OH, Fmoc-Lys(Boc)-OH, Fmoc-Arg(Pbf)-OH. Deblocking of the Fmoc group the the N-terminal of the resin-bound amino acid was performed with 20% piperidine in DMF for about 15-20 minutes. Coupling of the acetic acid was performed under conditions similar to amino acid coupling. Final cleavage from the resin was performed using cleavage mixture as described above. The product was isolated by precipitation and purified by preparative HPLC to afford the desired product as a white solid upon lyophilization.

### Example 3 - Addition of Lys at N-Terminus of Kringle-5

### Preparation of NAc-Tyr-Thr-Thr-Asn-Pro-Arg-Lys-Leu-Tyr-Asp-Tyr-Lys-NH₂.3TFA

Using automated peptide synthesis, the following protected amino acids were sequentially added to Rink Amide MBHA resin: Fmoc-Lys(Boc)-OH, Fmoc-Tyr(tBu)OH, Fmoc-Asp(OtBu)-OH, Fmoc-Tyr(tBu)OH, Fmoc-Leu-OH, Fmoc-Lys(Boc)-OH, Fmoc-Arg(Pbf)-OH, Fmoc-Pro-OH, Fmoc-Asn(Trt)-OH, Fmoc-Thr(tBu)-OH, Fmoc-Thr(tBu)-OH, Fmoc-Tyr(tBu)OH. Deblocking of the Fmoc group the the N-terminal of the resin-bound amino acid was performed with 20% piperidine in DMF for about 15-20 minutes. Coupling of the acetic acid was performed under conditions similar to amino acid coupling. Final cleavage from the resin was performed using cleavage mixture as described above. The product was isolated by precipitation and purified by preparative HPLC to afford the desired product as a white solid upon lyophilization.

### Example 4 - Addition of Lys at N-Terminus of Kringle-5,

### Substitution of Cys with Ala at Position 524

### Preparation of NAc-Arg-Asn-Pro-Asp-Gly-Asp-Val-Gly-Gly-Pro-Trp-Ala -Tyr-Thr-Thr-Asn-Pro-Arg-Lys-Leu-Tyr-Asp-Tyr-Lys-NH₂.4TFA

Using automated peptide synthesis, the following protected amino acids were sequentially added to Rink Amide MBHA resin: Fmoc-Lys(Boc)-OH, Fmoc-Tyr(tBu)OH, Fmoo-Asp(OtBu)-OH, Fmoc-Tyr(tBu)OH, Fmoc-Leu-OH, Fmoc-Lys(Boc)-OH, Fmoc-Arg(Pbf)-OH, Fmoc-Pro-OH, Fmoc-Asn(Trt)-OH, Fmoc-Thr(tBu)-OH, Fmoc-Thr(tBu)-OH, Fmoc-Tyr(tBu)OH, Fmoc-Ala-OH, Fmoc-Trp-OH, Fmoc-Pro-OH, Fmoc-Gly-OH, Fmoc-Gly-OH, Fmoc-Val-OH, Fmoc-Asp(OtBu)-OH, Fmoc-Asp(OtBu)-OH, Fmoc-Pro-OH, Fmoc-Asn(Trt)-OH, Fmoc-Arg(Pbf)-OH. Deblocking of the Fmoc group the the N-terminal of the resin-bound amino acid was performed with 20% piperidine in DMF for about 15-20 minutes. Coupling of the acetic acid was performed under conditions similar to amino acid coupling. Final cleavage from the resin was performed using cleavage mixture as described above. The product was isolated by precipitation and purified by preparative HPLC to afford the desired product as a white solid upon lyophilization.

### Example 5 - Addition of Lys at N-Terminus of Kringle-5

### Preparation of NAc-Arg-Asn-Pro-Asp-Gly-Asp-Val-Gly-Gly-Pro-Trp-Lys-NH₂.2TFA

Using automated peptide synthesis, the following protected amino acids were sequentially added to Rink Amide MBHA resin: Fmoc-Lys(Boc)-OH, Fmoc-Trp-OH, Fmoc-Pro-OH, Fmoc-Gly-OH, Fmoc-Gly-OH, Fmoc-Asp(OtBu)-OH, Fmoc-Gly-OH, Fmoc-Val-OH, Fmoc-Asp(OtBu)-OH, Fmoc-Pro-OH, Fmoc-Asn(Trt)-OH, Fmoc-Arg(Pbf)-OH. Deblocking of the Fmoc group the the N-terminal of the resin-bound amino acid was performed with 20% piperidine in DMF for about 15-20 minutes. Coupling of the acetic acid was performed under conditions similar to amino acid coupling. Final cleavage from the resin was performed using cleavage mixture as described above. The product was isolated by precipitation and purified by preparative HPLC to afford the desired product as a white solid upon lyophilization.

### Example 6 - Preparation of D-Ala2 GLP-1 (7-36) Amide

Solid phase peptide synthesis of the GLP-1 analog on a 100 µmole scale is performed using manual solid-phase synthesis and a Symphony Peptide Synthesizer using Fmoc protected Rink Amide MBHA resin, Fmoc protected amino acids, O-benzotriazol-1-yl-*N*, *N*, *N*', *N*'-tetramethyl-uronium hexafluorophosphate (HBTU) in *N,N-*dimethylformamide (DMF) solution and activation with *N*-methyl morpholine (NMM), and piperidine deprotection of Fmoc groups (Step 1). Resin cleavage and product isolation is performed using 85% TFA/5% TIS/5% thioanisole and 5% phenol, followed by precipitation by dry-ice cold Et₂O (Step 2). The product is purified by preparative reversed phased HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm to afford the desired peptide in >95% purity, as determined by RP-HPLC. These steps are illustrated in the schematic diagram below.

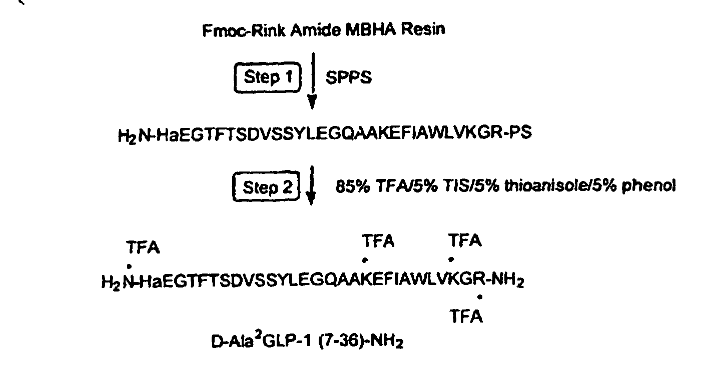

### C. Preparation of Modified Peptides From Peptides Containing No Cysteines

Preparation of maleimido peptides from therapeutic peptides containing multiple protected functional groups and no Cysteine is exempted by the synthesis of peptides as described below. The peptide may be modified at the N-terminus, the C-terminus, or at an amino acid located between the N-terminus and the C-terminus. The modified peptide is synthesized by linking off the N-terminus of the natural peptide sequence or by linking off the modified C-terminus of the natural peptide sequence. One or more additional amino acids may be added to the therapeutic peptide to facilitate attachment of the reactive group.

### 1. Modification of the Therapeutic Peptide at the C-Terminus

### Example 7 - Modification of RSV Peptide at the ε-Amino Group of the Added C-terminus Lysine Residue

### Preparation of Val-Ile-Thr-Ile-Glu-Leu-Ser-Asn-Ile-Lys-Glu-Asn-Lys-Met-Asn-Gly-Ala-Lys-Val-Lys-Leu-Ile-Lys-Gln-Glu-Leu-Asp-Lys-Tyr-Lys-Asn-Ala-Val-Lys-(Nε-MPA)

Solid phase peptide synthesis of the DAC analog on a 100 µmole scale is performed using manual solid-phase synthesis, a Symphony Peptide Synthesizer and Fmoc protected Rink Amide MBHA. The following protected amino acids are sequentially added to resin: Fmoc-Lys(Aloc)-OH, Fmoc-Val-OH, Fmoc-Ala-OH, Fmoc-Asn(Trt)-OH, Fmoc-Lys(Boc)-OH, Fmoc-Tyr(tBu)-OH, Fmoc-Lys(Boc)-OH, Fmoc-Asp(tBu)-OH, Fmoc-Leu-OH, Fmoc-Glu(tBu)-OH, Fmoc-Gln(Trt)-OH, Fmoc-Lys(Boc)-OH, Fmoc-Ile-OH, Fmoc-Leu-OH, Fmoc-Lys(Boc)-OH, Fmoc-Val-OH, Fmoc-Lys(Boc)-OH, Fmoc-Ala-OH, Fmoc-Gly-OH, Fmoc-Asn(Trt)-OH, Fmoc-Met-OH, Fmoc-Lys(Boc)-OH, Fmoc-Asn(Trt)-OH, Fmoc-Glu(tBu)-OH, Fmoc-Lys(Boc)-OH, Fmoc-Ile-OH, Fmoc-Asn(Trt)-OH, Fmoc-Ser(tBu)-OH, Fmoc-Leu-OH, Fmoc-Glu(tBu)-OH, Fmoc-Ile-OH, Fmoc-Thr(tBu)-OH, Fmoc-Ile-OH, Fmoc-Val-OH.They are dissolved in N,N-dimethylformamide (DMF) and, according to the sequence, activated using O-benzotriazol-1-yl-N, N, N', N'-tetramethyl-uronium hexafluorophosphate (HBTU) and Diisopropylethylamine (DIEA). Removal of the Fmoc protecting group is achieved using a solution of 20% (V/V) piperidine in N,N-dimethylformamide (DMF) for 20 minutes (step 1).The selective deprotection of the Lys (Aloc) group is performed manually and accomplished by treating the resin with a solution of 3 eq of Pd(PPh3)4 dissolved in 5 mL of CHCl3:NMM:HOAc (18:1:0.5) for 2 h (Step 2). The resin is then washed with CHCl3 (6 x 5 mL), 20% HOAc in DCM (6 x 5 mL), DCM (6 x 5 mL), and DMF (6 x 5 mL). The synthesis is then re-automated for the addition of the 3-maleimidopropionic acid (Step 3). Between every coupling, the resin is washed 3 times with N,N-dimethylformamide (DMF) and 3 times with isopropanol. The peptide is cleaved from the resin using 85% TFA/5% TIS/5% thioanisole and 5% phenol, followed by precipitation by dry-ice cold Et2O (Step 4). The product is purified by preparative reverse phase HPLC using a Varian (Rainin) preparative binary HPLC system: gradient elution of 30-55% B (0.045% TFA in H2O (A) and 0.045% TFA in CH3CN (B)) over 180 min at 9.5 mUmin using a Phenomenex Luna 10 µ phenyl-hexyl, 21 mm x 25 cm column and UV detector (Varian Dynamax UVD II) at λ 214 and 254 nm to afford the desired DAC in >95% purity, as determined by RP-HPLC.

### Example 8 - Modification of Dyn A 1-13 at the ε-Amino Group of the

### Added C-terminus Lysine Residue - Synthesis of Dyn A 1-13(Nε-MPA)-NH₂ Tyr-Gly-Gly-Phe-Leu-Arg-Arg-Ile-Arg-Pro-Lys-Leu-Lys-(Nε-MPA)-NH₂

Solid phase peptide synthesis of a modified Dyn A 1-13 on a 100 µmole scale was performed using manual solid-phase synthesis, a Symphony Peptide Synthesizer and Fmoc protected Rink Amide MBHA. The following protected amino acids were sequentially added to resin: Fmoc-Lys(Aloc)-OH, Fmoc-Leu-OH, Fmoc-Lys(Boc)-OH, Fmoc-Pro-OH, Fmoc-Arg(Pbf)-OH, Fmoc-Ile-OH, Fmoc-Arg(Pbf)-OH, Fmoc-Arg(Pbf)-OH, Fmoc-Leu-OH, Fmoc-Phe-OH, Fmoc-Gly-OH, Fmoc-Gly-OH, Fmoc-Tyr(tBu)-OH. They were dissolved in *N*,*N*-dimethylformamide (DMF) and, according to the sequence, activated using O-benzotriazol-1-yl-*N*, *N, N', N*'-tetramethyl-uronium hexafluorophosphate (HBTU) and Diisopropylethylamine (DIEA). Removal of the Fmoc protecting group is achieved using a solution of 20% (V/V) piperidine in *N,N-*dimethylformamide (DMF) for 20 minutes (Step 1).The selective deprotection of the Lys (Aloc) group is performed manually and accomplished by treating the resin with a solution of 3 eq of Pd(PPh₃)₄ dissolved in 5 mL of CHCl₃:NMM:HOAc (18:1:0.5) for 2 h (Step 2). The resin is then washed with CHCl₃ (6 x 5 mL), 20% HOAc in DCM (6 x 5 mL), DCM (6 x 5 mL), and DMF (6 x 5 mL). The synthesis is then re-automated for the addition of the 3-maleimidopropionic acid (Step 3). Between every coupling, the resin is washed 3 times with *N,N-*dimethylformamide (DMF) and 3 times with isopropanol. The peptide is cleaved from the resin using 85% TFA/5% TIS/5% thioanisole and 5% phenol, followed by precipitation by dry-ice cold Et₂O (Step 4). The product is purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm to afford the desired DAC in >95% purity, as determined by RP-HPLC.

The structure of this product is

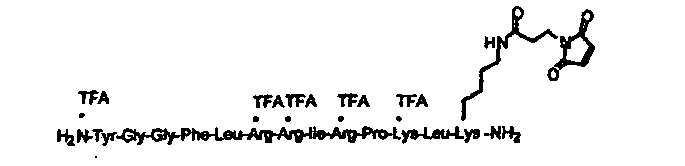

### Example 9 - Modification of Dyn A 2-13 at the ε-Amino Group of the Added C-terminus Lysine Residue - Synthesis of Dyn A 2-13(Nε-MPA)-NH₂ Gly-Gly-Phe-Leu-Arg-Arg-Ile-Arg-Pro-Lys-Leu-Lys-(Nε-MPA)-NH₂

Using automated peptide synthesis, the following protected amino acids were sequentially added to Rink Amide MBHA resin: Fmoc-Lys(Mtt)-OH, Fmoc-Leu-OH, Fmoo-Lys(Boc)-OH, Fmoc-Pro-OH, Fmoc-Arg(Pbf)-OH, Fmoc-Ile-OH, Fmoc-Arg(Pbf)-OH, fmoc-Arg(Fbf)-OH, Fmoc-Leu-OH, Fmoc-Phe-OH, Fmoc-Gly-OH, and Boc-Gly-OH. Manual synthesis was employed for the remaining steps: selective removal of the Mtt group and coupling of MPA using HBTU/HOBt/DIEA activation in DMF. The target dynorphin analog was removed from the resin; the product was isolated by precipitation and purified by preparative HPLC to afford the desired product as a white solid upon lyophilization in a 35% yield. Anal. HPLC indicated product to be >95% pure with Rₜ = 30.42 min. ESI-MS *m*/*z* for C₇₃H₁₂₃N₂₅O₁₅ (MH⁺), calcd 1590.0, found MH³⁺ 531.3.

### Example 10 - Modification of Dyn A 1-13 at the ε-Amino Group of the Added C-terminus Lysine Residue - Synthesis of Dyn A 1-13(AEA₃-MPA)-NH₂ Tyr-Gly-Gly-Phe-Leu-Arg-Arg-Ile-Arg-Pro-Lys-Leu-Lys-(AEA₃-MPA)-NH₂

Using automated peptide synthesis, the following protected amino acids were sequentially added to Rink Amide MBHA resin: Fmoc-Lys(Mtt)-OH, Fmoc-Leu-OH, Fmoc-Lys(Boc)-OH, Fmoc-Pro-OH, Fmoc-Arg(Pbf)-OH. Fmoc-Ile-OH, Fmoc-Arg(Pbf)-OH, Fmoc-Arg(Pbf)-OH, Fmoc-Leu-OH, Fmoc-Phe-OH, Fmoc-Gly-OH, Fmoc-Gly-OH, and Boc-Tyr(Boc)-OH. Manual synthesis was employed for the remaining steps: selective removal of the Mtt group, the coupling of three-Fmoc-AEA-OH groups (AEA = aminoethoxyacetic acid) with Fmoc removal in-between each coupling, and MPA acid using HBTU/HOBt/DIEA activation in DMF. The target dynorphin analog was removed from the resin; the product was isolated by precipitation and purified by preparative HPLC to afford the desired product as a white solid upon lyophilization in a 29% yield. Anal. HPLC indicated product to be >95% pure with Rₜ = 33.06 min. ESI-MS *m*/*z* for C₉₄H₁₅₄N₂₉O₂₃ (MH⁺), calcd 2057.2, found MH⁴⁺ 515.4, MH³⁺ 686.9, MH²⁺ 1029.7.

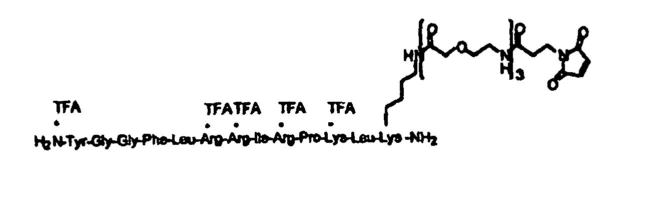

### Example 11 - Modification of Dyn A 2-13 at the ε-Amino Group of the Added C-terminus Lysine Residue - Synthesis of Dyn A 2-13(AEA₃-MPA)-NH₂ Gly-Gly-Phe-Leu-Arg-Arg-Ile-Arg-Pro-Lys-Leu-Lys-(AEA₃-MPA)-NH₂

Using automated peptide synthesis, the following protected amino acids were sequentially added to Rink Amide MBHA resin: Fmoc-Lys(Mtt)-OH, Fmoc-Leu-OH, Fmoc-Lys(Boc)-OH, Fmoc-Pro-OH, Fmoc-Arg(Pbf)-OH, Fmoc-Ile-OH, Fmoc-Arg(Pbf)-OH, Fmoc-Arg(Pbf)-OH, Fmoc-Leu-OH, Fmoc-Phe-OH, Fmoc-Gly-OH, and Fmoc-Gly-OH. Manual synthesis was employed for the remaining steps: selective removal of the Mtt group, the coupling of three-Fmoc-AEA-OH groups, with Fmoc removal in-between each coupling, and MPA using HBTU/HOBt/DIEA activation in DMF. The target dynorphin analog was removed from the resin; the product was isolated by precipitation and purified by preparative HPLC to afford the desired product as a white solid upon lyophilization in a 29% yield. Anal. HPLC indicated product to be >95% pure with Rₜ = 31.88 min. ESI-MS *m*/*z* for C₈₅H₁₄₅N₂₅O₂₁ (MH⁺), calcd 1894.3, found MH⁴⁺ 474.6, MH³⁺ 632.4, MH²⁺ 948.10.

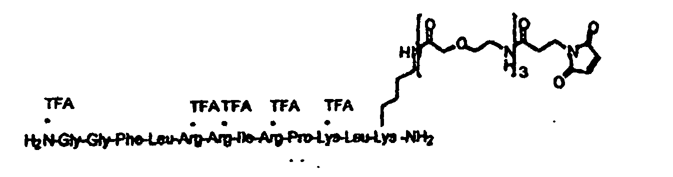

### Example 12 - Modification of Neuropeptide Y at the ε-Amino Group of the Added C-terminus Lysine Residue

### Preparation of Tyr-Pro-Ser-Lys-Pro-Asp-Asn-Pro-Gly-Glu-Asp-Ala-Pro-Ala-Glu-Asp-Met-Ala-Arg-Tyr-Tyr-Ser-Ala-Leu-Lys-(N-εMPA)-NH₂

Solid phase peptide synthesis of a modified neuropeptide Y analog on a 100 µmole scale is performed using manual solid-phase synthesis, a Symphony Peptide Synthesizer and Fmoc protected Rink Amide MBHA. The following protected amino acids were sequentially added to resin: Fmoc-Lys(Aloc)-OH, Fmoc-Leu-OH, Fmoc-Ala-OH, Fmoc-Ser(tBu)-OH, Fmoc-Tyr(tBu)-OH, Fmoc-Tyr(tBu)-OH, Fmoc-Arg(Pbf)-OH, Fmoc-Ala-OH, Fmoc-Met-OH, Fmoc-Asp(tBu)-OH, Fmoc-Glu(tBu)-OH, Fmoc-Ala-OH, Fmoc-Pro-OH, Fmoc-Ala-OH, Fmoc-Asp(tBu)-OH, Fmoc-Glu(tBu)-OH, Fmoc-Gly-OH, Fmoc-Pro-OH, Fmoc-Asn(Trt)-OH, Fmoc-Asp(tBu)-OH, Fmoc-Pro-OH, Fmoc-Lys(Boc)-OH, Fmoc-Ser(tBu)-OH, Fmoc-Pro-OH, Fmoc-Tyr(tBu)-OH, They are dissolved in *N,N*-dimethylformamide (DMF) and, according to the sequence, activated using O-benzotriazol-1-yl-*N*, *N, N'*, *N'*-tetramethyl-uronium hexafluorophosphate (HBTU) and Diisopropylethylamine (DIEA). Removal of the Fmoc protecting group is achieved using a solution of 20% (V/V) piperidine in *N*,*N*-dimethylformamide (DMF) for 20 minutes (Step 1).The selective deprotection of the Lys (Aloc) group is performed manually and accomplished by treating the resin with a solution of 3 eq of Pd(PPh₃)₄ dissolved in 5 mL of CHCl₃:NMM:HOAc (18:1:0.5) for 2 h (Step 2). The resin is then washed with CHCl₃ (6 x 5 mL), 20% HOAc in DCM (6 x 5 mL), DCM (6 x 5 mL), and DMF (6 x 5 mL). The synthesis is then re-automated for the addition of the 3-maleimidopropionic acid (Step 3). Between every coupling, the resin is washed 3 times with *N,N*-dimethylformamide (DMF) and 3 times with isopropanol. The peptide is cleaved from the resin using 85% TFA/5% TIS/5% thioanisole and 5% phenol, followed by precipitation by dry-ice cold Et₂O (Step 4). The product is purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm to afford the desired DAC in >95% purity, as determined by RP-HPLC.

### Example 13 - Modification of GLP-1 (7-36) at the C-Terminus Arginine

### Preparation of GLP-1 (7-36)-EDA-MPA

Solid phase peptide synthesis of a modified GLP-1 analog on a 100 µmole scale is performed manually and on a Symphony Peptide Synthesizer SASRIN (super acid sensitive resin). The following protected amino acids are sequentially added to the resin: Fmoc-Arg(Pbf)-OH, Fmoc-Gly-OH, Fmoc-Lys(Boc)-OH, Fmoc-Val-OH, Fmoc-Leu-OH, Fmoo-Trp(Boc)-OH, Fmoc-Ala-OH, Fmoc-Ile-OH, Fmoc-Phe-OH, Fmoc-Glu(OtBu)-OH, Fmoc-Lys(Boc)-OH, Fmoc-Ala-OH, Fmoc-Ala-OH, Fmoc-Gln(Trt)-OH, Fmoc-Gly-OH, Fmco-Glu(OtBu)-OH, Fmoc-Leu-OH, Fmoc-Tyr(tBu)-OH, Fmoc-Ser(tBu)-OH, Fmoc-Ser(tBu)-OH, Fmoc-Val-OH, Fmoc-Asp(tBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Ala-OH, Boc-His(Trt)-OH. They are dissolved in *N,N*-dimethylformamide (DMF) and, according to the sequence, activated using O-benzotriazol-1-yl-*N*, *N*, *N', N*'-tetramethyl-uronium hexafluorophosphate (HBTU) and Diisopropylethylamine (DIEA). Removal of the Fmoc protecting group is achieved using a solution of 20% (V/V) piperidine in *N,N*-dimethylformamide (DMF) for 20 minutes (Step 1). The fully protected peptide is cleaved from the resin by treatment with 1% TEA / DCM (Step 2). Ethylenediamine and 3-maleimidopropionic acid are then sequentially added to the free C-terminus (Step 3). The protecting groups are then cleaved and the product isolated using 86% TFA/5% TIS/5% H₂O/2% thioanisole and 2% phenol, followed by precipitation by dry-ice cold Et₂O (Step 4). The product is purified by preparative reverse phase HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm to afford the desired DAC in >95% purity, as determined by RP-HPLC. These steps are illustrated in the schematic diagram below.

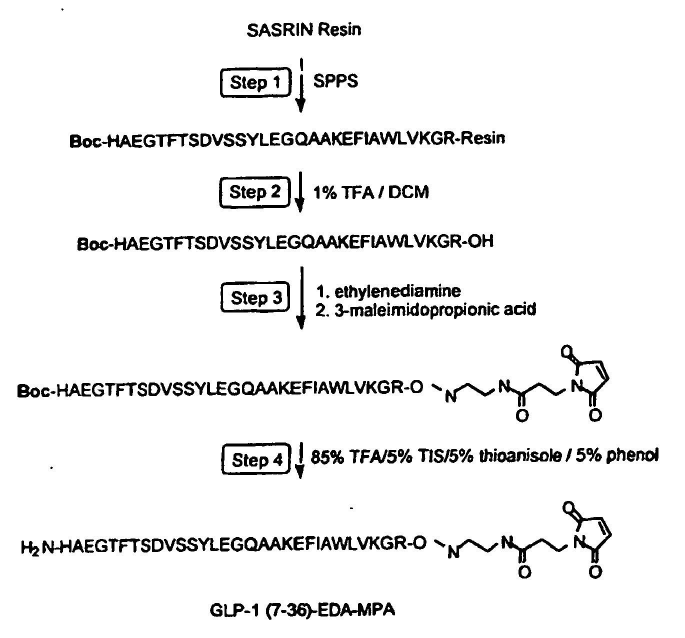

### Example 14 - Modification of Exendin-4 at the C-terminus Serine

### Preparation of Exendin-4 (1-39)-EDA-MPA

Solid phase peptide synthesis of a modified Exendin-4 analog on a 100 µmole scale is performed manually and on a Symphony Peptide Synthesizer SASRIN (super acid sensitive resin). The following protected amino acids are sequentially added to the resin: Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH, Boc-His(Trt)-OH. They are dissolved in *N,N*-dimethylformamide (DMF) and, according to the sequence, activated using O-benzotriazol-1-yl-*N, N, N', N'-*tetramethyl-uronium hexafluorophosphate (HBTU) and Diisopropylethylamine (DIEA). Removal of the Fmoc protecting group is achieved using a solution of 20% (V/V) piperidine in *N,N-*dimethylformamide (DMF) for 20 minutes (Step 1). The fully protected peptide is cleaved from the resin by treatment with 1% TFA / DCM (Step 2). Ethylenediamine and 3-maleimidopropionic acid are then sequentially added to the free *C*-terminus (Step 3). The protecting groups are then cleaved and the product isolated using 86% TFA/5% TIS/5% H₂O/2% thioanisole and 2% phenol, followed by precipitation by dry-ice cold Et₂O (Step 4). The product is purified by preparative reverse phase HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm to afford the desired DAC in >95% purity, as determined by RP-HPLC.

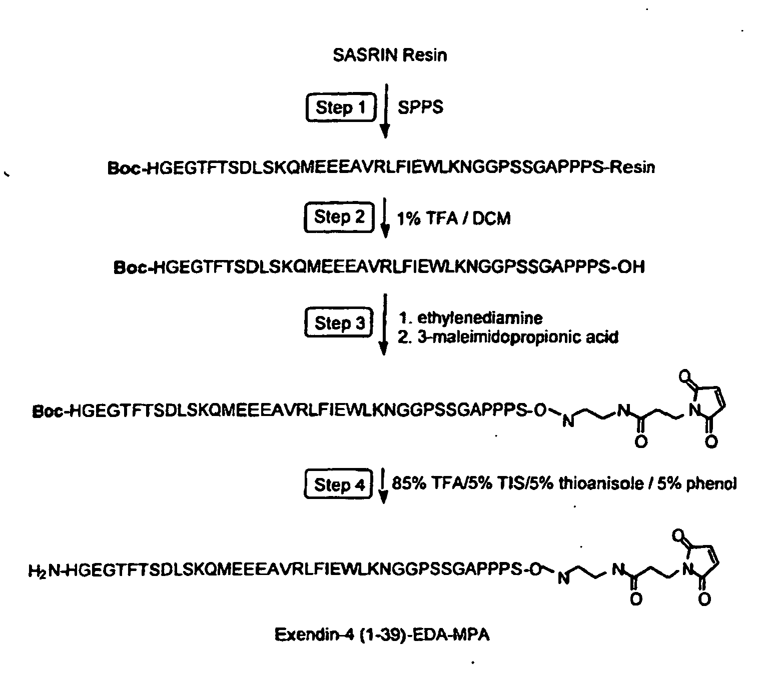

### Example 15 - Modification of Secretin Peptide at the ε-Amino Group of the Added C-terminus Lysine Residue

### Preparation of His-Ser-Asp-Gly-Thr-Phe-Thr-Ser-Glu-Leu-Ser-Arg-Leu-Arg-Glu-Gly-Ala-Arg-Leu-Glu-Arg-Leu-Leu-Gln-Gly-Leu-Val-Lys-(Nε-MPA)-NH₂

Solid phase peptide synthesis of a modified secretin peptide analog on a 100 µmole scale is performed using manual solid-phase synthesis, a Symphony Peptide Synthesizer and Fmoc protected Rink Amide MBHA. The following protected amino acids are sequentially added to resin: Fmoc-Lys(Aloc)-OH, Fmoc-Val-OH, Fmoc-Leu-OH, Fmoc-Gly-OH, Fmoc-Gln(Trt)-OH, Fmoc-Leu-OH, Fmoc-Leu-OH, Fmoc-Arg(Pbf)-OH, Fmoc-Glu(tBu)-OH, Fmoc-Leu-OH, Fmoc-Arg(Pbf)-OH, Fmoc-Ala-OH, Fmoc-Gly-OH, Fmoc-Glu(tBu)-OH, Fmoc-Arg(Pbf)-OH, Fmoc-Leu-OH, Fmoc-Arg(Pbf)-OH, Fmoc-Ser(tBu)-OH, Fmoc-Leu-OH, Fmoc-Glu(tBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Asp(tBu)-OH, Fmoc-Ser(tBu)-OH, Fmoc-His(Boc)-OH.They are dissolved in *N*,*N-*dimethylformamide (DMF) and, according to the sequence, activated using O-benzotriazol-1-yl-*N*, *N, N'*, *N'*-tetramethyl-uronium hexafluorophosphate (HBTU) and Diisopropylethylamine (DIEA). Removal of the Fmoc protecting group is achieved using a solution of 20% (V/V) piperidine in *N,N*-dimethylformamide (DMF) for 20 minutes (Step 1).The selective deprotection of the Lys (Aloc) group is performed manually and accomplished by treating the resin with a solution of 3 eq of Pd(PPh₃)₄ dissolved in 5 mL of CHCl₃:NMM:HOAc (18:1:0.5) for 2 h (Step 2). The resin is then washed with CHCl₃ (6 x 5 mL), 20% HOAc in DCM (6 x 5 mL), DCM (6 x 5 mL), and DMF (6 x 5 mL). The synthesis is then re-automated for the addition of the 3-maleimidopropionic acid (Step 3). Between every coupling, the resin is washed 3 times with *N,N-*dimethylformamide (DMF) and 3 times with isopropanol. The peptide is cleaved from the resin using 85% TFA/5% TIS/5% thioanisole and 5% phenol, followed by precipitation by dry-ice cold Et₂O (Step 4). The product is purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mUmin using a Phenomenex Luna 10 µ phenyl-hexyl, 21 mm x 25 cm column and UV detector (Varian Dynamax UVD II) at λ 214 and 254 nm to afford the desired DAC in >95% purity, as determined by RP-HPLC.

### Example 16 - Modification of Kringle-5 at the ε-Amino Group of the Added C-terminus Lysine Residue

### Preparation of NAc-Pro-Arg-Lys-Leu-Tyr-Asp-Tyr-Lys-(Nε-MPA)-NH₂.2TFA

Solid phase peptide synthesis of a modified Kringle-5 peptide on a 100 µmole scale was performed using manual solid-phase synthesis, a Symphony Peptide Synthesizer and Fmoc protected Rink Amide MBHA. The following protected amino acids are sequentially added to resin: Fmoc-Lys(Aloc)-OH, Fmoc-Tyr(tBu)-OH, Fmoc-Asp(tBu)-OH, Fmoc-Tyr(tBu)-OH, Fmoc-Leu-OH, Fmoc-Lys(Boc)-OH, Fmoc-Arg(Pbf)-OH, Fmoc-Pro-OH. They were dissolved in *N,N*-dimethylformamide (DMF) and, according to the sequence, activated using O-benzotriazol-1-yl-*N*, *N, N', N'*-tetramethyl-uronium hexafluorophosphate (HBTU) and Diisopropylethylamine (DIEA). Removal of the Fmoc protecting group is achieved using a solution of 20% (V/V) piperidine in *N,N-*dimethylformamide (DMF) for 20 minutes (step 1). At the end of the synthesis Acetic Anhydride was added to acetylate the N-terminal.The selective deprotection of the Lys (Aloc) group is performed manually and accomplished by treating the resin with a solution of 3 eq of Pd(PPh₃)₄ dissolved in 5 mL of CHCl₃:NMM:HOAc (18:1:0.5) for 2 h (Step 2). The resin is then washed with CHCl₃ (6 x 5 mL), 20% HOAc in DCM (6 x 5 mL), DCM (6 x 5 mL), and DMF (6 x 5 mL). The synthesis is then re-automated for the addition of the 3-maleimidopropionic acid (Step 3). Between every coupling, the resin is washed 3 times with *N,N-*dimethylformamide (DMF) and 3 times with isopropanol. The peptide is cleaved from the resin using 85% TFA/5% TIS/5% thioanisole and 5% phenol, followed by precipitation by dry-ice cold Et₂O (Step 4). The product is purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and W detector (Varian Dynamax UVD II) at λ 214 and 254 nm to afford the desired DAC in >95% purity, as determined by RP-HPLC.

### Example 17 - Modification of Kringle-5 at the ε-Amino Group of the Added C-terminus Lysine Residue

### Preparation of NAc-Tyr-Thr-Thr-Asn-Pro-Arg-Lys-Leu-Tyr-Asp-Tyr-Lys-(Nε-MPA)-NH₂.2TFA

Using automated peptide synthesis, the following protected amino acids were sequentially added to Rink Amide MBHA resin: Fmoc-Lys(Aloc)-OH, Fmoc-Tyr(tBu)OH, Fmoc-Asp(OtBu)-OH, Fmoc-Tyr(tBu)OH, Fmoc-Leu-OH, Fmoc-Lys(Boc)-OH, Fmoc-Arg(Pbf)-OH, Fmoc-Pro-OH, Fmoc-Asn(Trt)-OH, Fmoc-Thr(tBu)-OH, Fmoc-Thr(tBu)-OH, Fmoc-Tyr(tBu)OH (step 1). Deblocking of the Fmoc group the the N-terminal of the resin-bound amino acid was performed with 20% piperidine in DMF for about 15-20 minutes. Final cleavage from the resin was performed using cleavage mixture as described above. The product was isolated by precipitation and purified by preparative HPLC to afford the desired product as a white solid upon lyophilization The selective deprotection of the Lys(Aloc) group was performed manually and accomplished by treating the resin with a solution of 3 eq of Pd(PPh₃)₄ dissolved in 5 mL of CHC)₃:NMM:HOAc (18:1:0.5) for 2 h (Step 2). The resin was then washed with CHCl₃ (6 x 5 mL), 20% HOAc in DCM (6 x 5 mL), DCM (6 x 5 mL), and DMF (6 x 5 mL). The synthesis was then re-automated for the addition of the 3-maleimidopropionic acid (Step 3). Resin cleavage and product isolation was performed using 85% TFA/5% TIS/5% thioanisole and 5% phenol, followed by precipitation by dry-ice cold Et₂O (Step 4). The product was purified by preparative reversed phased HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm.

### Example 18 - Modification of Kringle-5 at the ε-Amino Group of the Added C-terminus Lysine Residue

### Preparation of NAc-Arg-Asn-Pro-Asp-Gly-Asp-Val-Gly-Gly-Pro-Trp- Ala-Tyr-Thr-Thr-Asn-Pro-Arg-Lys-Leu-Tyr-Asp-Tyr-Lys-(Nε-MPA)-NH₂.3TFA

Using automated peptide synthesis, the following protected amino acids were sequentially added to Rink Amide MBHA resin: Fmoc-Lys(Aloc)-OH, Fmoc-Tyr(tBu)OH, Fmoc-Asp(OtBu)-OH, Fmoc-Tyr(tBu)OH, Fmoc-Leu-OH, Fmoc-Lys(Boc)-OH, Fmoc-Arg(Pbf)-OH, Fmoc-Pro-OH, Fmoc-Asn(Trt)-OH, Fmoc-Thr(tBu)-OH, Fmoc-Thr(tBu)-OH, Fmoc-Tyr(tBu)OH, Fmoc-Ala-OH, Fmoc-Trp-OH, Fmoc-Pro-OH, Fmoc-Gly-OH, Fmoc-Gly-OH, Fmoc-Val-OH, Fmoc-Asp(OtBu)-OH, Fmoc-Gly-OH, Fmoc-Asp(OtBu)-OH, Fmoc-Pro-OH, Fmoc-Asn(Trt)-OH, Fmoc-Arg(Pbf)-OH (step 1). Deblocking of the Fmoc group the the N-terminal of the resin-bound amino acid was performed with 20% piperidine in DMF for about 15-20 minutes. Coupling of the acetic acid was performed under conditions similar to amino acid coupling. Final cleavage from the resin was performed using cleavage mixture as described above. The product was isolated by precipitation and purified by preparative HPLC to afford the desired product as a white solid upon lyophilization.

The selective deprotection of the Lys(Aloc) group was performed manually and accomplished by treating the resin with a solution of 3 eq of Pd(PPh₃)₄ dissolved in 5 mL of CHCl₃:NMM:HOAc (18:1:0.5) for 2 h (Step 2). The resin was then washed with CHCl₃ (6 x 5 mL), 20% HOAc in DCM (6 x 5 mL), DCM (6 x 5 mL), and DMF (6 x 5 mL). The synthesis was then re-automated for the addition of the 3-maleimidopropionic acid (Step 3). Resin cleavage and product isolation was performed using 85% TFA/5% TIS/5% thioanisole and 5% phenol, followed by precipitation by dry-ice cold Et₂O (Step 4). The product was purified by preparative reversed phased HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm.

### Example 19 - Modification of Kringle-5 at the ε-Amino Group of the Added C-terminus Lysine Residue

### Preparation of NAc-Arg-Asn-Pro-Asp-Gly-Asp-Val-Gly-Gly-Pro-Trp-Lys-(Nε-MPA)-NH₂.TFA

Using automated peptide synthesis, the following protected amino acids were sequentially added to Rink Amide MBHA resin: Fmoc-Lys(Aloc)-OH, Fmoc-Trp-OH, Fmoc-Pro-OH, Fmoc-Gly-OH, Fmoc-Gly-OH, Fmoc-Val-OH, Fmoc-Asp(OtBu)-OH, Fmoc-Gly-OH, Fmoc-Asp(OtBu)-OH, Fmoc-Pro-OH, Fmoc-Asn(Trt)-OH, Fmoo-Arg(Pbf)-OH (Step 1). The selective deprotection of the Lys(Aloc) group was performed manually and accomplished by treating the resin with a solution of 3 eq of Pd(PPh₃)₄ dissolved in 5 mL of CHCl₃:NMM:HOAc (18:1:0.5) for 2 h (Step 2). The resin was then washed with CHCl₃ (6 x 5 mL), 20% HOAc in DCM (6 x 5 mL), DCM (6 x 5 mL), and DMF (6 x 5 mL). The synthesis was then re-automated for the addition of the 3-maleimidopropionic acid (Step 3). Resin cleavage and product isolation was performed using 85% TFA/5% TIS/5% thioanisole and 5% phenol, followed by precipitation by dry-ice cold Et₂O (Step 4). The product was purified by preparative reversed phased HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm.

### Example 20 - Modification of Kringle-5 at the ε-Amino Group of the Added C-terminus Lysine Residue

### Preparation of NAc-Arg-Lys-Leu-Tyr-Asp-Tyr-Lys-(Nε-MPA)-NH₂.2TFA

Using automated peptide synthesis, the following protected amino acids were sequentially added to Rink Amide MBHA resin: Fmoc-Lys(Aloc)-OH, Fmoc-Tyr(tBu)OH, Fmoc-Asp(OtBu)-OH, Fmoc-Tyr(tBu)OH, Fmoc-Leu-OH, Fmoc-Lys(Boc)-OH, Fmoc-Arg(Pbf)-OH (Step 1). Deblocking of the Fmoc group the N-terminal of the resin-bound amino acid was performed with 20% piperidine in DMF for about 15-20 minutes. Coupling of the acetic acid was performed under conditions similar to amino acid coupling. Final cleavage from the resin was performed using cleavage mixture as described above. The product was isolated by precipitation and purified by preparative HPLC to afford the desired product as a white solid upon lyophilization The selective deprotection of the Lys(Aloc) group was performed manually and accomplished by treating the resin with a solution of 3 eq of Pd(PPh₃)₄ dissolved in 5 mL of CHCl₃:NMM:HOAc (18:1:0.5) for 2 h (Step 2). The resin was then washed with CHCl₃ (6 x 5 mL), 20% HOAc in DCM (6 x 5 mL), DCM (6 x 5 mL), and DMF (6 x 5 mL). The synthesis was then re-automated for the addition of the 3-maleimidopropionic acid (Step 3). Resin cleavage and product isolation was performed using 85% TFA/5% TIS/5% thioanisole and 5% phenol, followed by precipitation by dry-ice cold Et₂O (Step 4). The product was purified by preparative reversed phased HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm.

### Example 21 - Modification of Kringle-5 at the ε-Amino Group of the Added C-terminus Lysine Residue

### Preparation of NAc-Pro-Arg-Lys-Leu-Tyr-Asp-Lys-(Nε-MPA)-NH₂.2TFA

Using automated peptide synthesis, the following protected amino acids were sequentially added to Rink Amide MBHA resin: Fmoc-Lys(Aloc)-OH, Fmoc-Asp(OtBu)-OH, Fmoc-Tyr(tBu)OH, Fmoc-Leu-OH, Fmoc-Lys(Boc)-OH, Fmoc-Arg(Pbf)-OH, Fmoc-Pro-OH (Step 1). Deblocking of the Fmoc group the N-terminal of the resin-bound amino acid was performed with 20% piperidine in DMF for about 15-20 minutes. Coupling of the acetic acid was performed under conditions similar to amino acid coupling. Final cleavage from the resin was performed using cleavage mixture as described above. The product was isolated by precipitation and purified by preparative HPLC to afford the desired product as a white solid upon lyophilization

The selective deprotection of the Lys(Aloc) group was performed manually and accomplished by treating the resin with a solution of 3 eq of Pd(PPh₃)₄ dissolved in 5 mL of CHCl₃:NMM:HOAc (18:1:0.5) for 2 h (Step 2). The resin was then washed with CHCl₃ (6 x 5 mL), 20% HOAc in DCM (6 x 5 mL), DCM (6 x 5 mL), and DMF (6 x 5 mL). The synthesis was then re-automated for the addition of the 3-maleimidopropionic acid (Step 3). Resin cleavage and product isolation was performed using 85% TFA/5% TIS/5% thioanisole and 5% phenol, followed by precipitation by dry-ice cold Et₂O (Step 4). The product was purified by preparative reversed phased HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm.

### Example 22 - Modification of Kringle-5 at the ε-Amino Group of the Added C-terminus Lysine Residue

### Preparation of NAc-Pro-Arg-Lys-Leu-Tyr-Asp-Tyr-Lys-(Nε-AEEA-MPA)-NH₂.2TFA

Using automated peptide synthesis, the following protected amino acids were sequentially added to Rink Amide MBHA resin: Fmoc-Lys(Aloc)-OH, Fmoc-Tyr(tBu)OH, Fmoc-Asp(OtBu)-OH, Fmoc-Tyr(tBu)OH, Fmoc-Leu-OH, Fmoc-Lys(Boe)-OH, Fmoc-Arg(Pbf)-OH, Fmoc-Pro-OH (Step 1). Deblocking of the Fmoc group at the N-terminal of the resin-bound amino acid was performed with 20% piperidine in DMF for about 15-20 minutes. Coupling of the acetic acid was performed under conditions similar to amino acid coupling. The selective deprotection of the Lys(Aloc) group was performed manually and accomplished by treating the resin with a solution of 3 eq of Pd(PPh₃)₄ dissolved in 5 mL of CHCl₃:NMM:HOAc (18:1:0.5) for 2 h (Step 2). The resin was then washed with CHCl₃ (6 x 5 mL), 20% HOAc in DCM (6 x 5 mL), DCM (6 x 5 mL), and DMF (6 x 5 mL). The synthesis was then re-automated for the addition. The synthesis was then re-automated for the addition of the AEEA (aminoethoxyethoxyacetic acid) groupand of the 3-maleimidopropionic acid (MPA) (Step 3). Resin cleavage and product isolation was performed using 85% TFA/5% TIS/5% thioanisole and 5% phenol, followed by precipitation by dry-ice cold Et₂O (Step 4). The product was purified by preparative reversed phased HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm.

### Example 23 - Modification of Kringle-5 at the ε-Amino Group of the Added C-terminus Lysine Residue

### Preparation of NAc-Pro-Arg-Lys-Leu-Tyr-Asp-Tyr-Lys-(Nε-AEEAₙ₋MPA)-NH₂.2TFA

Using automated peptide synthesis, the following protected amino acids were sequentially added to Rink Amide MBHA resin: Fmoc-Lys(Aloc)-OH, Fmoc-Tyr(tBu)OH, Fmoc-Asp(OtBu)-OH, Fmoc-Tyr(tBu)OH, Fmoc-Leu-OH, Fmoc-Lys(Boc)-OH, Fmoc-Arg(Pbf)-OH, Fmoc-Pro-OH (Step 1). Deblocking of the Fmoc group at the N-terminal of the resin-bound amino acid was performed with 20% piperidine in DMF for about 15-20 minutes. Coupling of the acetic acid was performed under conditions similar to amino acid coupling.

The selective deprotection of the Lys(Aloc) group was performed manually and accomplished by treating the resin with a solution of 3 eq of Pd(PPh₃)₄ dissolved in 5 mL of CHCl₃:NMM:HOAc (18:1:0.5) for 2 h (Step 2). The resin was then washed with CHCl₃ (6 x 5 mL), 20% HOAc in DCM (6 x 5 mL), DCM (6 x 5 mL), and DMF (6 x 5 mL). The synthesis was then re-automated for the addition. The synthesis was then re-automated for the addition of n AEEA (aminoethoxyethoxyacetic acid) groups and of the 3-maleimidopropionic acid (MPA) (Step 3). Resin cleavage and product isolation was performed using 85% TFA/5% TIS/5% thioanisole and 5% phenol, followed by precipitation by dry-ice cold Et₂O (Step 4). The product was purified by preparative reversed phased HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm.

### Example 24 - Modification of GLP-1 at the ε-Amino Group of the Added C-terminus Lysine Residue

### Preparation of GLP-1 (1-36)-Lys³⁷(Nε-MPA)-NH₂.5TFA; His-Asp-Glu-Phe-Glu-Arg-His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Lys(Nε-MPA)-NH₂.5TFA

Using automated peptide synthesis, the following protected amino acids were sequentially added to Rink Amide MBHA resin: Fmoc-Lys(Aloc)-OH, Fmoc-Arg(Pbf)-OH, Fmoc-Gly-OH, Fmoc-Lys(tBoc)-OH, Fmoc-Val-OH, Fmoc-Leu-OH, Fmoc-Trp-OH, Fmoc-Ala-OH, Fmoc-Ile-OH, Fmoc-Phe-OH, Fmoc-Glu(OtBu)-OH, Fmoc-Lys(tBoc)-OH, Fmoc-Ala-OH, Fmoc-Ala-OH, Fmoc-Gln(Trt)-OH, Fmoc-Gly-OH, Fmoc-Glu(OtBu)-OH, Fmoc-Leu-OH, Fmoc-Tyr(Pbf)-OH, Fmoc-Ser(tBu)-OH, Fmoc-Ser(tBu)-OH, Fmoc-Val-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Ala-OH, Boc-His(N-Trt)-OH, Fmoc-Arg(Pbf)-OH, Fmoc-Glu(OtBu)-OH, Fmoc-Phe-OH, Fmoc-Glu(OtBu)-OH, Fmoc-Asp(OtBu)-OH, Boc-His(N-Trt)-OH (step 1)

The selective deprotection of the Lys(Aloc) group was performed manually and accomplished by treating the resin with a solution of 3 eq of Pd(PPh₃)₄ dissolved in 5 mL of CHCl₃:NMM:HOAc (18:1:0.5) for 2 h (Step 2). The resin was then washed with CHCl₃ (6 x 5 mL), 20% HOAc in DCM (6 x 5 mL), DCM (6 x 5 mL), and DMF (6 x 5 mL). The synthesis was then re-automated for the addition of the 3-maleimidopropionic acid (Step 3). Resin cleavage and product isolation was performed using 85% TFA/5% TIS/5% thioanisole and 5% phenol, followed by precipitation by dry-ice cold Et₂O (Step 4). The product was purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization. These steps are illustrated in the schematic diagram below.

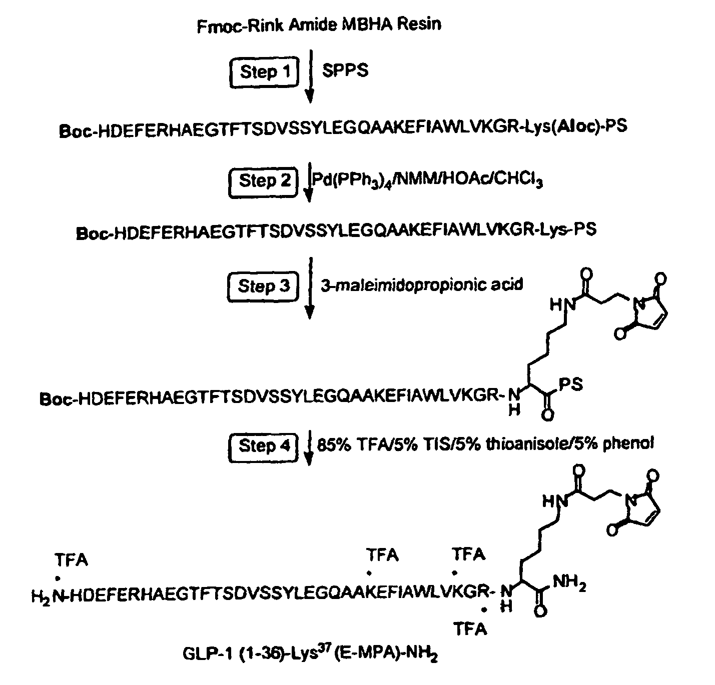

### Example 25 - Modification of GLP-1 at the ε-Amino Group of the Added C-terminus Lysine Residue

### Preparation of GLP-1 (1-36)-Lys³⁷(Nε-AEEA-AEEA-MPA)-NH₂.5TFA; His-Asp-Glu-Phe-Glu-Arg-His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Lys(Nε-AEEA-AEEA-MPA)-NH₂.5TFA

Using automated peptide synthesis, the following protected amino acids were sequentially added to Rink Amide MBHA resin: Fmoc-Lys(Aloc)-OH, Fmoc-Arg(Pbf)-OH, Fmoc-Gly-OH, Fmoc-Lys(tBoc)-OH, Fmoc-Val-OH, Fmoc-Leu-OH, Fmoc-Trp-OH, Fmoc-Ala-OH, Fmoc-Ile-OH, Fmoc-Phe-OH, Fmoc-Glu(OtBu)-OH, Fmoc-Lys(tBoc)-OH, Fmoc-Ala-OH, Fmoc-Ala-OH, Fmoc-Gln(Trt)-OH, Fmoc-Gly-OH, Fmoc-Glu(OtBu)-OH, Fmoc-Leu-OH, Fmoc-Tyr(Pbf)-OH, Fmoc-Ser(tBu)-OH, Fmoc-Ser(tBu)-OH, Fmoc-Val-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Ala-OH, Boc-His(N-Trt)-OH, Fmoc-Arg(Pbf)-OH, Fmoc-Glu(OtBu)-OH, Fmoc-Phe-OH, Fmoc-Glu(OtBu)-OH, Fmoc-Asp(OtBu)-OH, Boc-His(N-Trt)-OH (step 1).

The selective deprotection of the Lys(Aloc) group was performed manually and accomplished by treating the resin with a solution of 3 eq of Pd(PPh₃)₄ dissolved in 5 mL of CHCl₃:NMM:HOAc (18:1:0.5) for 2 h (Step 2). The resin was then washed with CHCl₃ (6 x 5 mL), 20% HOAc in DCM (6 x 5 mL), DCM (6 x 5 mL), and DMF (6 x 5 mL). The synthesis was then re-automated for the addition of the two AEEA (aminoethoxyethoxyacetic acid) groups and the 3-maleimidopropionic acid (Step 3). Resin cleavage and product isolation was performed using 85% TFA/5% TIS/5% thioanisole and 5% phenol, followed by precipitation by dry-ice cold Et₂O (Step 4). The product was purfied by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization, ESI-MS *m*/*z* for C₁₇₄H₂₆₅N₄₄O₅₆ (MH⁺), calcd 3868, found [M+H₂]²⁺ 1934, [M+H₃]³⁺ 1290, [M+H₄]⁴⁺ 967. These steps are illustrated in the schematic diagram below.

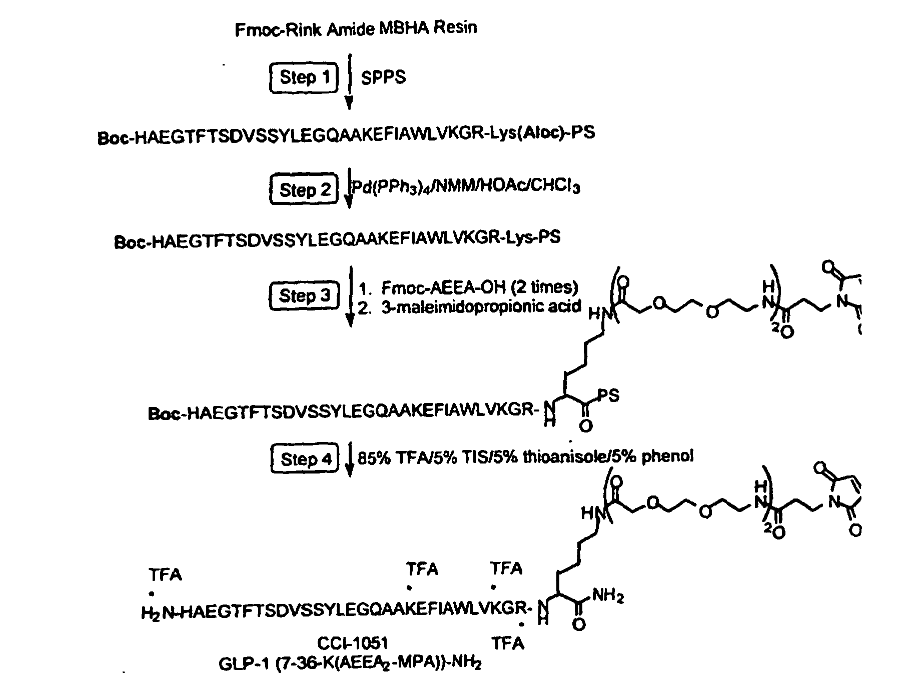

### Example 26 - Modification of GLP-1 at the ε-Amino Group of the Added C-terminus Lysine Residue

### Preparation of GLP-1 (7-36)-Lys³⁷(Nε-MPA)-NH₂.4TFA; His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Lys(Nε-MPA)-NH₂.4TFA

Using automated peptide synthesis, the following protected amino acids were sequentially added to Rink Amide MBHA resin: Fmoc-Lys(Aloc)-OH, Fmoc-Arg(Pbf)-OH, Fmoc-Gly-OH, Fmoc-Lys(tBoc)-OH, Fmoc-Val-OH, Fmoc-Leu-OH, Fmoc-Trp-OH, Fmoc-Ala-OH, Fmoc-Ile-OH, Fmoc-Phe-OH, Fmoc-Glu(OtBu)-OH, Fmoc-Lys(tBoc)-OH, Fmoc-Ala-OH, Fmoc-Ala-OH, Fmoc-Gln(Trt)-OH, Fmoc-Gly-OH, Fmoc-Glu(OtBu)-OH, Fmoc-Leu-OH, Fmoc-Tyr(Pbf)-OH, Fmoc-Ser(tBu)-OH, Fmoc-Ser(tBu)-OH, Fmoc-Val-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Ala-OH, Boc-His(N-Trt)-OH (Step 1).

The selective deprotection of the Lys(Aloc) group was performed manually and accomplished by treating the resin with a solution of 3 eq of Pd(PPh₃)₄ dissolved in 5 mL of CHCl₃:NMM:HOAc (18:1:0.5) for 2 h (Step 2). The resin was then washed with CHCl₃ (6 x 5 mL), 20% HOAc in DCM (6 x 5 mL), DCM (6 x 5 mL), and DMF (6 x 5 mL). The synthesis was then re-automated for the addition of the 3-maleimidopropionic acid (Step 3). Resin cleavage and product isolation was performed using 85% TFA/5% TIS/5% thioanisole and 5% phenol, followed by precipitation by dry-ice cold Et₂O (Step 4). The product was purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization.

### Example 27 - Modification of GLP-1 at the ε-Amino Group of the Added C-terminus Lysine Residue

### Preparation of GLP-1 (7-36)-Lys³⁷(Nε-AEEA-AEEA-MPA)-NH₂.4TFA His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Lys(Nε-AEEA-AEEA-MPA)-NH₂.4TFA

Using automated peptide synthesis, the following protected amino acids were sequentially added to Rink Amide MBHA resin: Fmoc-Lys(Aloc)-OH, Fmoc-Arg(Pbf)-OH, Fmoc-Gly-OH, Fmoc-Lys(tBoc)-OH, Fmoc-Val-OH, Fmoc-Leu-OH, Fmoc-Trp-OH, Fmoc-Ala-OH, Fmoc-Ile-OH, Fmoc-Phe-OH, Fmoc-Glu(OtBu)-OH, Fmoc-Lys(tBoc)-OH, Fmoc-Ala-OH, Fmoc-Ala-OH, Fmoc-Gln(Trt)-OH, Fmoc-Gly-OH, Fmoc-Glu(OtBu)-OH, Fmoc-Leu-OH, Fmoc-Tyr(Pbf)-OH, Fmoc-Ser(tBu)-OH, Fmoc-Ser(tBu)-OH, Fmoc-Val-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Ala-OH, Boc-His(N-Trt)-OH (Step 1).

The selective deprotection of the Lys(Aloc) group was performed manually and accomplished by treating the resin with a solution of 3 eq of Pd(PPh₃)₄ dissolved in 5 mL of CHCl₃:NMM:HOAc (18:1:0.5) for 2 h (Step 2). The resin was then washed with CHCl₃ (6 x 5 mL), 20% HOAc in DCM (6 x 5 mL), DCM (6 x 5 mL), and DMF (6 x 5 mL). The synthesis was then re-automated for the addition of the two AEEA (aminoethoxyethoxyacetic acid) groups and the 3-maleimidopropionic acid (Step 3). Resin cleavage and product isolation was performed using 85% TFA/5% TIS/5% thioanisole and 5% phenol, followed by precipitation by dry-ice cold Et₂O (Step 4). The product was purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization.

### Example 28 - Modification of D-Ala² GLP-1 at the ε-Amino Group of the Added C-terminus Lysine Residue

### Preparation of D-Ala² GLP-1 (7-36)-Lys³⁷(Nε-MPA)-NH₂.4TFA His-d-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Lys(Nε-MPA)-NHh₂.4TFA

Using automated peptide synthesis, the following protected amino acids were sequentially added to Rink Amide MBHA resin: Fmoc-Lys(Aloc)-OH, Fmoc-Arg(Pbf)-OH, Fmoc-Gly-OH, Fmoc-Lys(tBoc)-OH, Fmoc-Val-OH, Fmoc-Leu-OH, Fmoc-Trp-OH, Fmoc-Ala-OH, Fmoc-Ile-OH, Fmoc-Phe-OH, Fmoc-Glu(OtBu)-OH, Fmoc-Lys(tBoc)-OH, Fmoc-Ala-OH, Fmoc-Ala-OH, Fmoc-Gln(Trt)-OH, Fmoc-Gly-OH, Fmoc-Glu(OtBu)-OH, Fmoc-Leu-OH, Fmoc-Tyr(Pbf)-OH, Fmoc-Ser(tBu)-OH, Fmoc-Ser(tBu)-OH, Fmoc-Val-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-d-Ala-OH, Boc-His(N-Trt)-OH (Step1).

The selective deprotection of the Lys(Aloc) group was performed manually and accomplished by treating the resin with a solution of 3 eq of Pd(PPh₃)₄ dissolved in 5 mL of CHCl₃:NMM:HOAc (18:1:0.5) for 2 h (Step 2). The resin was then washed with CHCl₃ (6 x 5 mL), 20% HOAc in DCM (6 x 5 mL), DCM (6 x 5 mL), and DMF (6 x 5 mL). The synthesis was then re-automated for the addition of the 3-maleimidopropionic acid (Step 3). Resin cleavage and product isolation was performed using 85% TFA/5% TIS/5% thioanisole and 5% phenol, followed by precipitation by dry-ice cold Et₂O (Step 4). The product was purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization. These steps are illustrated in the schematic diagram below.

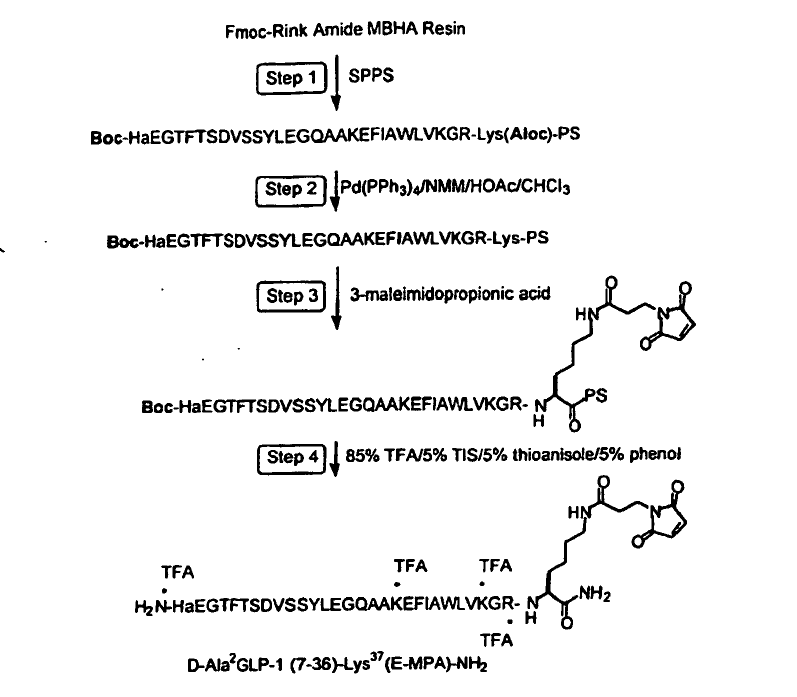

### Example 29 - Modification of D-Ala² GLP-1 at the ε-Amino Group of the Added C-terminus Lysine Residue

### Preparation of D-Ala² GLP-1 (7-36)-Lys³⁷(Nε-AEEA-AEEA-MPA)-NH₂.4TFA His-D-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Lys (Nε-AEEA-AEEA-MPA)-NH₂.4TFA

Using automated peptide synthesis, the following protected amino acids were sequentially added to Rink Amide MBHA resin: Fmoc-Lys(Aloc)-OH, Fmoc-Arg(Pbf)-OH, Fmoc-Gly-OH, Fmoc-Lys(tBoe)-OH, Fmoc-Val-OH, Fmoc-Leu-OH, Fmoc-Trp-OH, Fmoc-Ala-OH, Fmoc-Ile-OH, Fmoc-Phe-OH, Fmoc-Glu(OtBu)-OH, Fmoc-Lys(tBoc)-OH, Fmoc-Ala-OH, Fmoc-Ala-OH, Fmoc-Gln(Trt)-OH, Fmoc-Gly-OH, Fmoc-Glu(OtBu)-OH, Fmoc-Leu-OH, Fmoc-Tyr(Pbf)-OH, Fmoc-Ser(tBu)-OH, Fmoc-Ser(tBu)-OH, Fmoc-Val-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-d-Ala-OH, Boc-His(N-Trt)-OH (Step 1).

The selective deprotection of the Lys(Aloc) group was performed manually and accomplished by treating the resin with a solution of 3 eq of Pd(PPh₃)₄ dissolved in 5 mL of CHCl₃:NMM:HOAc (18:1:0.5) for 2 h (Step 2). The resin was then washed with CHCl₃ (6 x 5 mL), 20% HOAc in DCM (6 x 5 mL), DCM (6 x 5 mL), and DMF (6 x 5 mL). The synthesis was then re-automated for the addition of the two AEEA (aminoethoxyethoxyacetic acid) groups and the 3-maleimidopropionic acid (Step 3). Resin cleavage and product isolation was performed using 85% TFA/5% TIS/5% thioanisole and 5% phenol, followed by precipitation by dry-ice cold Et₂O (Step 4). The product was purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization. These steps are illustrated in the schematic diagram below.

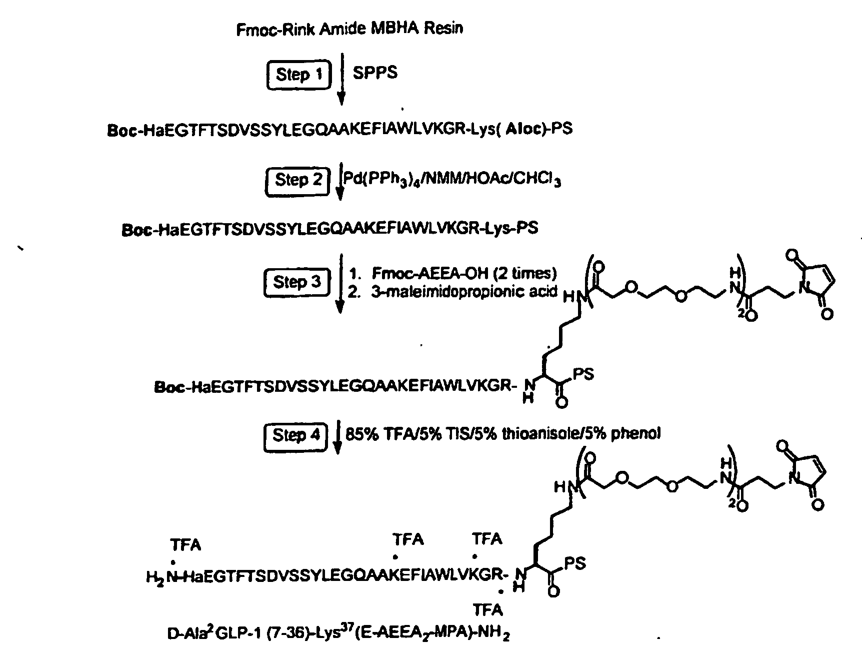

### Example 30 - Modification of Exendin-4(1-39) at the ε-Amino Group of the Added C-terminus Lysine Residue

### Preparation of Exendin-4 (1-39)-Lys⁴⁰(Nε-MPA)-NH₂; His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-Lys (Nε-MPA)-NH₂.5TFA

Using automated peptide synthesis, the following protected amino acids were sequentially added to Rink Amide MBHA resin: Fmoc-Lys(Aloc)-OH, Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp-OH, Fmoc-Glu(OtBu)-OH, Fmoc-Ile-OH, Fmoc-Phe-OH, Fmoc-Leu-OH, Fmoc-Arg(Bpf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoe-Glu(OtBu)-OH, Fmoc-Gly-OH, Boc-His(Trt)-OH (Step 1).

The selective deprotection of the Lys(Aloc) group was performed manually and accomplished by treating the resin with a solution of 3 eq of Pd(PPh₃)₄ dissolved in 5 mL of CHCl₃:NMM:HOAc (18:1:0.5) for 2 h (Step 2). The resin was then washed with CHCl₃ (6 x 5 mL), 20% HOAc in DCM (6 x 5 mL), DCM (6 x 5 mL), and DMF (6 x 5 mL). The synthesis was then re-automated for the addition of the 3-maleimidopropionic acid (Step 3). Resin cleavage and product isolation was performed using 85% TFA/5% TIS/5% thioanisole and 5% phenol, followed by precipitation by dry-ice cold Et₂O (Step 4). The product was purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization. These steps are illustrated in the schematic diagram below.

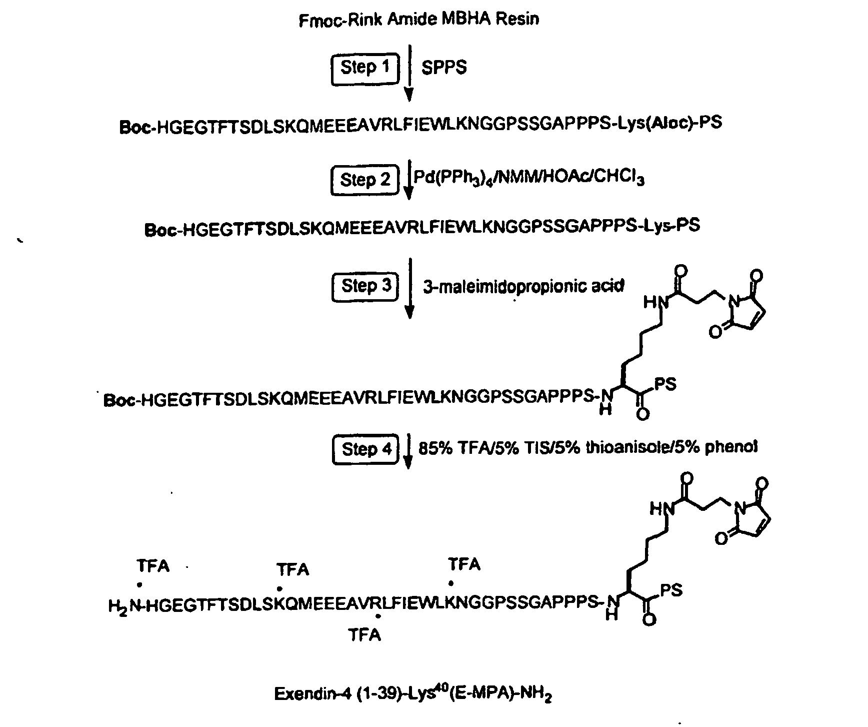

### Example 31 - Modification of Exendin-4(1-39) at the ε-Amino Group of the Added C-terminus Lysine Residue

### Preparation of Exendin-4 (1-39)-Lys⁴⁰(Nε-AEEA-AEEA-MPA)-NH₂.5TFA; His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-Lys(Nε-AEEA-AEEA-MPA)-NH₂.5TFA

Using automated peptide synthesis, the following protected amino acids were sequentially added to Rink Amide MBHA resin: Fmoc-Lys(Aloc)-OH, Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp-OH, Fmoc-Glu(OtBu)-OH, Fmoc-Ile-OH, Fmoc-Phe-OH, Fmoc-Leu-OH, Fmoc-Arg(Bpf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH, Boc-His(Trt)-OH (Step 1).

The selective deprotection of the Lys(Aloc) group was performed manually and accomplished by treating the resin with a solution of 3 eq of Pd(PPh₃)₄ dissolved in 5 mL of CHCl₃:NMM:HOAc (18:1:0.5) for 2 h (Step 2). The resin was then washed with CHCl₃ (6 x 5 mL), 20% HOAc in DCM (6 x 5 mL), DCM (6 x 5 mL), and DMF (6 x 5 mL). The synthesis was then re-automated for the addition of the two AEEA (aminoethoxyethoxyacetic acid) groups and the 3-maleimidopropionic acid (Step 3). Resin cleavage and product isolation was performed using 85% TFA/5% TIS/5% thioanisole and 5% phenol, followed by precipitation by dry-ice cold Et₂O (Step 4). The product was purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mUmin using a Phenomenex Luna 10 µ phenyl-hexyl, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization. These steps are illustrated in the schematic diagram below.

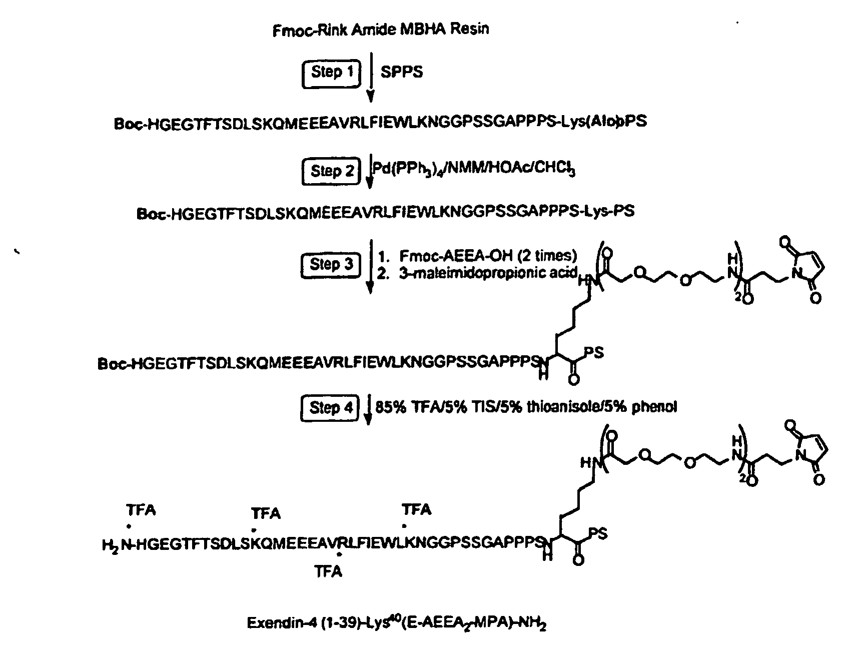

### Example 32 - Modification of Exendin-3(1-39) at the ε-Amino Group of the Added C-terminus Lysine Residue

### Preparation of Exendin-3 (1-39)-Lys⁴⁰(Nε-MPA)-NH₂.5TFA; His-Ser-Asp-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-Lys(Nε-MPA)-NH₂.5TFA

Using automated peptide synthesis, the following protected amino acids were sequentially added to Rink Amide MBHA resin: Fmoc-Lys(Aloc)-OH, Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser-OH, Fmoc-Ser(tBu)-OH, Fmoc-Pro-OH. Fmoc-Gly-OH, Fmoc-Gly-OH, Fmoc-Asn(Trt)-OH, Fmoc-Lys(Boc)-OH. Fmoc-Leu-OH, Fmoc-Trp-OH, Fmoc-Glu(OtBu)-OH, Fmoc-Ile-OH, Fmoc-Phe-OH, Fmoc-Leu-OH, Fmoc-Arg(Bpf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)-OH. Fmoc-Ser(tBu)-OH, Fmoc-Thr(tBu)-OH, Fmoc-Phe-OH, Fmoc-Thr(t8u)-OH, Fmoc-Gly-OH, Fmoc-Asp(OtBu)-OH, Fmoc-Ser(OtBu)-OH, Boc-His(Trt)-OH (Step 1).

The selective deprotection of the Lys(Aloc) group was performed manually and accomplished by treating the resin with a solution of 3 eq of Pd(PPh₃)₄ dissolved in 5 mL of CHCl₃:NMM:HOAc (18:1:0.5) for 2 h (Step 2). The resin was then washed with CHCl₃ (6 x 5 mL), 20% HOAc in DCM (6 x 5 mL), DCM (6 x 5 mL), and DMF (6 x 5 mL). The synthesis was then re-automated for the addition of the 3-maleimidopropionic acid (Step 3). Resin cleavage and product isolation was performed using 85% TFA/5% TIS/5% thioanisole and 5% phenol, followed by precipitation by dry-ice cold Et₂O (Step 4). The product was purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization. These steps are illustrated in the schematic diagram below.

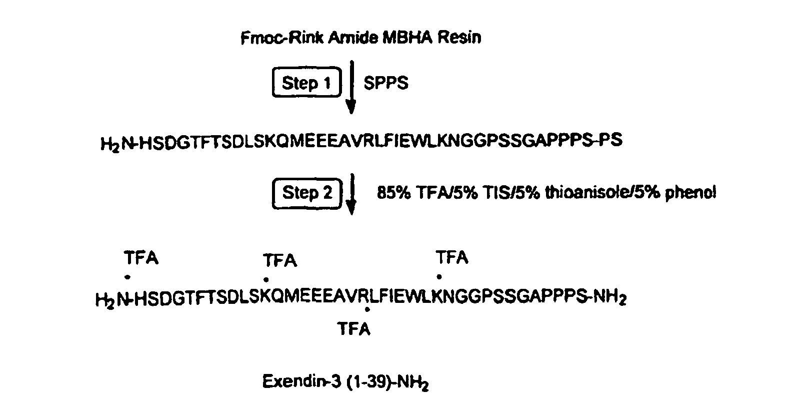

### Example 33 - Modification of Exendin-3(1-39) at the ε-Amino Group of the Added C-terminus Lysine Residue

### Preparation of Exendin-3 (1-39)-Lys⁴⁰(Nε-AEEA-AEEA-MPA)-NH₂.5TFA; His-Ser-Asp-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-Lys(Nε-AEEA-AEEA-MPA)-NH₂.5TFA

Using automated peptide synthesis, the following protected amino acids were sequentially added to Rink Amide MBHA resin: Fmoc-Lys(Aloc)-OH, Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser-OH, Fmoc-Ser(tBu)-OH, Fmoc-Pro-OH, Fmoc-Gly-OH, Fmoc-Gly-OH. Fmoc-Asn(Trt)-OH, Fmoc-Lys(Boc)-OH, Fmoc-Leu-OH, Fmoc-Trp-OH, Fmoc-Glu(OtBu)-OH, Fmoc-Ile-OH, Fmoc-Phe-OH, Fmoc-Leu-OH, Fmoc-Arg(Bpf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Asp(OtBu)-OH, Fmoc-Ser(OtBu)-OH, Boc-His(Trt)-OH (Step 1).

The selective deprotection of the Lys(Aloc) group was performed manually and accomplished by treating the resin with a solution of 3 eq of Pd(PPh₃)₄ dissolved in 5 mL of CHCl₃:NMM:HOAc (18:1:0.5) for 2 h (Step 2). The resin was then washed with CHCl₃ (6 x 5 mL), 20% HOAc in DCM (6 x 5 mL), DCM (6 x 5 mL), and DMF (6 x 5 mL. The synthesis was then re-automated for the addition of the two AEEA (aminoethoxyethoxyacetic acid) groups and the 3-maleimidopropionic acid (Step 3). Resin cleavage and product isolation was performed using 85% TFA/5% TIS/5% thioanisole and 5% phenol, followed by precipitation by dry-ice cold Et₂O (Step 4). The product was purfied by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization.

### Example 34 - Modification of HIV-1 DP 178 at the C-Terminus

### Preparation of modified HIV-1 DP 178 antifusogenic peptide Tyr-Thr-Ser-Leu-Ile-His-Ser-Leu-Ile-Glu-Glu-Ser-Gln-Asn-Glu-Glu-Glu-Lys-Asn-Glu-Glu-Glu-Leu-Leu-Glu-Leu-Asp-Lys-Trp-Ala-Ser-Leu-Trp-Asn-Trp-Phe-Lys-(Nε-MPA)-NH₂

Using automated peptide synthesis, the following protected amino acids are sequentially added to Rink Amide MBHA resin: Fmoc-Lys(Mtt)-OH, Fmoc-Phe-OH , Fmoc-Trp(Boc)-OH, Fmoc-Asn(Trt)-OH, Fmoc-Trp(Boc)-OH, Fmoc-Leu-OH, Fmoc-Ser(tBu)-OH, Fmoc-Ala-OH, Fmoc-Trp(Boc)-OH , Fmoc-Lys(Boc)-OH, Fmoc-Asp(tBu)-OH, Fmoc-Leu-OH, Fmoe-Glu(tBu)-OH, Fmoc-Leu-OH, Fmoc-Leu-OH, Fmoc-Glu(Tbu)-OH, Fmoc-Gln(Trt)-OH, Fmoc-Glu(tBu)-OH, Fmoc-Asn(Trt)-OH, Fmoc-Lys(Boc)-OH, Fmoc-Glu(tBu)-OH, Fmoc-Gln(Trt)-OH, Fmoc-Gln(Trt)-OH, Fmoc-Asn(Trt)-OH; Fmoc-Gln(Trt)-OH, Fmoc-Ser(tBu)-OH, Fmoc-Glu(tBu)-OH, Fmoc-Glu(tBu)-OH, Fmoc-Ile-OH, Fmoc-Leu-OH, Fmoc-Ser(tBu)-OH, Fmoc-His(Trt)-OH, Fmoc-Ile-OH, Fmoc-Leu-OH, Fmoc-Ser(tBu)-OH, Fmoc-Thr(tBu)-OH and Boc-Tyr(tBu)-OH. Manual synthesis is employed for the remaining steps: selective removal of the Mtt group and coupling of maleimidopropionic acid (MPA) using HBTU/HOBt/DIEA activation in DMF. The target molecule is removed from the resin; the product is isolated by precipitation and purified by preparative HPLC to afford the desired product as a white solid upon lyophilization.

### Example 35 - Modification of HIV-1 DP 107 at the C-Terminus

### Preparation of modified HIV-1 DP 107 antifusogenic peptide Asn-Asn-Leu-Leu-Arg-Ala-Ile-Glu-Ala-Glu-Glu-His-Leu-Leu-Glu-Leu-Thr-Val-Trp-Glu-Ile-Lys-Glu-Leu-Glu-Ala-Arg-Ile-Leu-Ala-Val-Glu-Arg-Tyr-Leu-Lys-Asp-Glu-Lys-(Nε-MPA)NH₂

Using automated peptide synthesis, the following protected amino acids are sequentially added to Rink Amide MBHA resin: Fmoc-Lys(Mtt)-OH, Fmoc-Gln(Trt)-OH, Fmoc-Asp(tBu)-OH , Fmoc-Lys(Boc)-OH, Fmoc-Leu-OH, Fmoc-Tyr(tBu)-OH, Fmoc-Arg(Pbf)-OH, Fmoc-Glu(tBu)-OH, Fmoc-Val-OH, Fmoc-Ala-OH, Fmoc-Leu-OH, Fmoc-Ile-OH, Fmoc-Arg(Pbf)-OH, Fmoc-Ala-OH, Fmoc-Gln(Trt)-OH, Fmoc-Leu-OH, Fmoc-Gln(Trt)-OH, Fmoc-Lys(Boc)-OH, Fmoc-Ile-OH, Fmoc-Gln(Trt)-OH, Fmoc-Trp(Boc)-OH, Fmoc-Val-OH, Fmoc-Thr(tBu)-OH, Fmoc-Leu-OH, Fmoc-Gln(Trt)-OH, Fmoc-Leu-OH, Fmoc-Leu-OH, Fmoc-His(Trt)-OH, Fmoc-Gln(Trt)-OH, Fmoc-Gln(Trt)-OH, Fmoc-Ala-OH, Fmoc-Glu(tBu)-OH, Fmoc-Ile-OH, Fmoc-Ala-OH, Fmoc-Arg(Pbf)-OH, Fmoc-Leu-OH, Fmoc-Leu-OH, Fmoc-Asn(Trt)-OH, Fmoc-Asn(Trt)-OH. Manual synthesis is employed for the remaining steps: selective removal of the Mtt group and coupling of maleimidopropionic acid (MPA) using HBTU/HOBt/DIEA activation in DMF. The target analog is removed from the resin; the product is isolated by precipitation and purified by preparative HPLC to afford the desired product as a white solid upon lyophilization.

### 2. Modification of the Therapeutic Peptide at the N-Terminus

### Example 36 - Modification of RSV Peptide at the ε-Amino Group of the Added N-terminus Lysine Residue

### Preparation of (Nε-MPA)-Lys-Val-Ile-Thr-Ile-Glu-Leu-Ser-Asn-Ile-Lys-Glu-Asn-Lys-Met-Asn-Gly-Ala-Lys-Val-Lys-Leu-Ile-Lys-Gln-Glu-Leu-Asp-Lys-Tyr-Lys-Asn-Ala-Val

Solid phase peptide synthesis of a modified RSV peptide on a 100 µmole scale is performed using manual solid-phase synthesis, a Symphony Peptide Synthesizer and Fmoc protected Rink Amide MBHA. The following protected amino acids are sequentially added to resin: Fmoc-Val-OH, Fmoc-Ala-OH, Fmoc-Asn(Trt)-OH, Fmoc-Lys(Boc)-OH, Fmoc-Tyr(tBu)-OH, Fmoc-Lys(Boc)-OH, Fmoc-Asp(tBu)-OH, Fmoc-Leu-OH, Fmoc-Glu(tBu)-OH, Fmoc-Gln(Trt)-OH, Fmoc-Lys(Boc)-OH, Fmoc-Ile-OH, Fmoc-Leu-OH, Fmoc-Lys(Boc)-OH, Fmoc-Val-OH, Fmoc-Lys(Boc)-OH, Fmoc-Ala-OH, Fmoc-Gly-OH, Fmoc-Asn(Trt)-OH, Fmoc-Met-OH, Fmoc-Lys(Boc)-OH, Fmoc-Asn(Trt)-OH, Fmoc-Glu(tBu)-OH, Fmoc-Lys(Boc)-OH, Fmoc-Ile-OH, Fmoc-Asn(Trt)-OH, Fmoc-Ser(tBu)-OH, Fmoc-Leu-OH, Fmoc-Glu(tBu)-OH, Fmoc-Ile-OH, Fmoc-Thr(tBu)-OH, Fmoc-Ile-OH, Fmoc-Val-OH, Fmoc-Lys(Aloc)-OH.They are dissolved in N,N-dimethylformamide (DMF) and, according to the sequence, activated using O-benzotriazol-1-yl-N, N, N', N'-tetramethyl-uronium hexafluorophosphate (HBTU) and Diisopropylethylamine (DIEA). Removal of the Fmoc protecting group is achieved using a solution of 20% (V/V) piperidine in N,N-dimethylformamide (DMF) for 20 minutes (Step 1).The selective deprotection of the Lys (Aloc) group is performed manually and accomplished by treating the resin with a solution of 3 eq of Pd(PPh3)4 dissolved in 5 mL of CHCl3:NMM:HOAc (18:1:0.5) for 2 h (Step 2). The resin is then washed with CHCl3 (6 x 5 mL), 20% HOAc in DCM (6 x 5 mL), DCM (6 x 5 mL), and DMF (6 x 5 mL). The synthesis is then re-automated for the addition of the 3-maleimidopropionic acid (Step 3). Between every coupling, the resin is washed 3 times with N,N-dimethylformamide (DMF) and 3 times with isopropanol. The peptide is cleaved from the resin using 85% TFA/5% TIS/5% thioanisole and 5% phenol, followed by precipitation by dry-ice cold Et2O (Step 4). The product is purified by preparative reversed phased HPLC using a Varian (Rainin) preparative binary HPLC system: gradient elution of 30-55% B (0.045% TFA in H2O (A) and 0.045% TFA in CH3CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm to afford the desired DAC in >95% purity, as determined by RP-HPLC.

### Example 37 - Modification of Neuropeptide Y at the ε-Amino Group of the Added N-terminus Lysine Residue

### Preparation of (N-εMPA)-Lys-Tyr-Pro-Ser-Lys-Pro-Glu-Asn-Pro-Gly-Glu-Asp-Ala-Pro-Ala-Glu-Asp-Met-Ala-Arg-Tyr-Tyr-Ser-Ala-Leu

Solid phase peptide synthesis of a modified neuropeptide Y on a 100 µmole scale was performed using manual solid-phase synthesis, a Symphony Peptide Synthesizer and Fmoc protected Rink Amide MBHA. The following protected amino acids are sequentially added to resin: Fmoc-Leu-OH, Fmoc-Ala-OH, Fmoc-Ser(tBu)-OH, Fmoc-Tyr(tBu)-OH, Fmoc-Tyr(tBu)-OH, Fmoc-Arg(Pbf)-OH. Fmoc-Ala-OH, Fmoc-Met-OH, Fmoc-Asp(tBu)-OH, Fmoc-Glu(tBu)-OH, Fmoc-Ala-OH, Fmoc-Pro-OH, Fmoc-Ala-OH, Fmoc-Asp(tBu)-OH, Fmoc-Glu(tBu)-OH, Fmoc-Gly-OH, Fmoc-Pro-OH, Fmoc-Asn(Trt)-OH, Fmoc-Glu(tBu)-OH, Fmoc-Pro-OH, Fmoc-Lys(Boc)-OH, Fmoc-Ser(tBu)-OH, Fmoc-Pro-OH, Fmoc-Tyr(tBu)-OH, Fmoc-Lys(Aloc)-OH. They are dissolved in N,N-dimethylformamide (DMF) and, according to the sequence, activated using O-benzotriazol-1-yl-N, N, N', N'-tetramethyl-uronium hexafluorophosphate (HBTU) and Diisopropylethylamine (DIEA). Removal of the Fmoc protecting group is achieved using a solution of 20% (V/V) piperidine in N,N-dimethylformamide (DMF) for 20 minutes (Step 1).The selective deprotection of the Lys (Aloc) group is performed manually and accomplished by treating the resin with a solution of 3 eq of Pd(PPh₃)₄ dissolved in 5 mL of CHCl₃:NMM:HOAc (18:1:0.5) for 2 h (Step 2). The resin is then washed with CHCl₃ (6 x 5 mL), 20% HOAc in DCM (6 x 5 mL), DCM (6 x 5 mL), and DMF (6 x 5 mL). The synthesis is then re-automated for the addition of the 3-maleimidopropionic acid (Step 3). Between every coupling, the resin is washed 3 times with N,N-dimethyformamide (DMF) and 3 times with isopropanol. The peptide is cleaved from the resin using 85% TFA/5% TIS/5% thioanisole and 5% phenol, followed by precipitation by dry-ice cold Et₂O (Step 4). The product is purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm to afford the desired DAC in >95% purity, as determined by RP-HPLC.

### Example 38 - Modification of Neuropeptide Y at the ε-Amino Group of the Added N-terminus Lysine Residue

### Preparation of (N-εMPA)-Lys-Tyr-Pro-Ser-Lys-Pro-Asp-Asn-Pro-Gly-Glu-Asp-Ala-Pro-Ala-Glu-Asp-Met-Ala-Arg-Tyr-Tyr-Ser-Ala-Leu

Solid phase peptide synthesis of a modified neuropeptide Y on a 100 µmole scale was performed using manual solid-phase synthesis, a Symphony Peptide Synthesizer and Fmoc protected Rink Amide MBHA. The following protected amino acids are sequentially added to resin: Fmoc-Leu-OH, Fmoc-Ala-OH, Fmoc-Ser(tBu)-OH, Fmoc-Tyr(tBu)-OH, Fmoc-Tyr(tBu)-OH, Fmoc-Arg(Pbf)-OH, Fmoc-Ala-OH, Fmoc-Met-OH, Fmoc-Asp(tBu)-OH, Fmoc-Glu(tBu)-OH, Fmoc-Ala-OH, Fmoc-Pro-OH, Fmoc-Ala-OH, Fmoc-Asp(tBu)-OH, Fmoc-Glu(tBu)-OH, Fmoc-Gly-OH, Fmoc-Pro-OH, Fmoc-Asn(Trt)-OH, Fmoc-Asp(tBu)-OH, Fmoc-Pro-OH, Fmoc-Lys(Boc)-OH, Fmoc-Ser(tBu)-OH, Fmoc-Pro-OH, Fmoc-Tyr(tBu)-OH, Fmoc-Lys(Aloc)-OH. They are dissolved in N,N-dimethylformamide (DMF) and, according to the sequence, activated using O-benzotriazol-1-yl-N, N, N', N'-tetramethyl-uronium hexafluorophosphate (HBTU) and Diisopropylethylamine (DIEA). Removal of the Fmoc protecting group is achieved using a solution of 20% (V/V) piperidine in N,N-dimethylformamide (DMF) for 20 minutes (Step 1).The selective deprotection of the Lys (Aloc) group is performed manually and accomplished by treating the resin with a solution of 3 eq of Pd(PPh₃)4 dissolved in 5 mL of CHCI₃:NMM:HOAc (18:1:0.5) for 2 h (Step 2). The resin is then washed with CHCl₃ (6 x 5 mL), 20% HOAc in DCM (6 x 5 mL), DCM (6 x 5 mL), and DMF (6 x 5 mL). The synthesis is then re-automated for the addition of the 3-maleimidopropionic acid (Step 3). Between every coupling, the resin is washed 3 times with N,N-dimethylformamide (DMF) and 3 times with isopropanol. The peptide is cleaved from the resin using 85% TFA/5% TIS/5% thioanisole and 5% phenol, followed by precipitation by dry-ice cold Et2O (Step 4). The product is purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm to afford the desired DAC in >95% purity, as determined by RP-HPLC.

### Example 39 - Modification of Dyn A 1-13 at the ε-Amino Group of the Added N-terminus Lysine Residue - Synthesis of (Nε-MPA)-Dyn A 1-13-NH₂ (Nε-MPA)-Lys-Tyr-Gly-Gly-Phe-Leu-Arg-Arg-Ile-Arg-Pro-Lys-Leu

Solid phase peptide synthesis of a modified Dyn A 1-13 analog on a 100 µmole scale is performed using manual solid-phase synthesis, a Symphony Peptide Synthesizer and Fmoc protected Rink Amide MBHA. The following protected amino acids are sequentially added to resin: Fmoc-Lys(Boc)-OH, Fmoc-Leu-OH, Fmoc-Lys(Boc)-OH, Fmoc-Pro-OH, Fmoc-Arg(Pbf)-OH, Fmoc-Ile-OH, Fmoc-Arg(Pbf)-OH, Fmoc-Arg(Pbf)-OH, Fmoc-Leu-OH, Fmoc-Phe-OH, Fmoc-Gly-OH, Fmoc-Gly-OH, Fmoc-Tyr(tBu)-OH, Fmoc-Lys(Aloc)-OH. They were dissolved in *N,N-*dimethylformamide (DMF) and, according to the sequence, activated using O-benzotriazol-1-yl-*N, N, N', N'*-tetramethyl-uronium hexafluorophosphate (HBTU) and Diisopropylethylamine (DIEA). Removal of the Fmoc protecting group is achieved using a solution of 20% (V/V) piperidine in *N,N*-dimethylformamide (DMF) for 20 minutes (Step 1).The selective deprotection of the Lys (Aloc) group is performed manually and accomplished by treating the resin with a solution of 3 eq of Pd(PPh₃)₄ dissolved in 5 mL of CHCl₃:NMM:HOAc (18:1:0.5) for 2 h (Step 2). The resin is then washed with CHCl₃ (6 x 5 mL), 20% HOAc in DCM (6 x 5 mL), DCM (6 x 5 mL), and DMF (6 x 5 mL). The synthesis is then re-automated for the addition of the 3-maleimidopropionic acid (Step 3). Between every coupling, the resin is washed 3 times with *N,N-*dimethylformamide (DMF) and 3 times with isopropanol. The peptide is cleaved from the resin using 85% TFA/5% TIS/5% thioanisole and 5% phenol, followed by precipitation by dry-ice cold Et₂O (Step 4). The product is purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and W detector (Varian Dynamax UVD II) at λ 214 and 254 nm to afford the desired DAC in >95% purity, as determined by RP-HPLC.

### Example 40 - Modification of Dyn A 2-17-NH₂ at the N-terminus Glycine - Synthesis of MPA-AEA₃-Dyn A 2-17-NH₂ (MPA-AEA-AEA-AEA)-Gly-Gly-Phe-Leu-Arg-Arg-Ile-Arg-Pro-Lys-Leu-Lys-Trp-Asp-Asn-Glu

Using automated peptide synthesis, the following protected amino acids and maleimide were sequentially added to Rink Amide MBHA resin: Fmoc-Gln(Trt)-OH, Fmoc-Asn(Trt)-OH, Fmoc-Asp(OtBu)-OH, Fmoc-Trp(Boc)-OH, Fmoc-Lys(Boc)-OH, Fmoc-Leu-OH, Fmoc-Lys(Boc)-OH, Fmoc-Pro-OH, Fmoc-Arg(Pbf)-OH, Fmoc-Ile-OH, Fmoc-Arg(Pbf)-OH, Fmoc-Arg(Pbf)-OH, Fmoc-Leu-OH, Fmoc-Phe-OH, Fmoc-Gly-OH, Fmoc-Gly-OH, Fmoc-AEA-OH, Fmoc-AEA-OH, Fmoc-AEA-OH, and MPA. The target dynorphin analog was then removed from the resin; the product was isolated by precipitation and purified by preparative HPLC to afford the desired product as a pale yellow solid upon lyophilization in a 32% yield. Anal. HPLC indicated product to be >95% pure with Rₜ = 33.44 min. ESI-MS *m*/*z* for C₁₀₉H₁₇₂N₃₅O₂₉ (MH⁺), calcd 2436.8, found MH³⁺ 813.6.

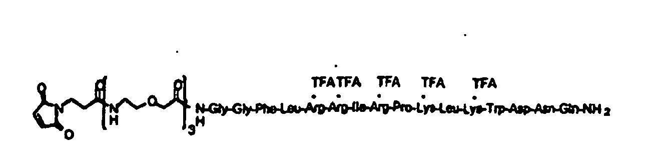

### Example 42 - Modification of Kringle-5 at the ε-Amino Group of the Added N-Terminus Lysine Residue

### Preparation of (Nε-MPA)-Lys-Pro-Arg-Lys-Leu-Tyr-Asp-Tyr-NH₂.2TFA

Solid phase peptide synthesis of a modified Kringle-5 analog on a 100 µmole scale was performed using manual solid-phase synthesis, a Symphony Peptide Synthesizer and Fmoc protected Rink Amide MBHA. The following protected amino acids are sequentially added to resin: Fmoc-Tyr(tBu)-OH, Fmoc-Asp(tBu)-OH, Fmoc-Tyr(tBu)-OH, Fmoc-Leu-OH, Fmoc-Lys(Boc)-OH, Fmoc-Arg(Pbf)-OH, Fmoc-Pro-OH, Fmoc-Lys(Aloc)-OH. They were dissolved in *N,N*-dimethylformamide (DMF) and, according to the sequence, activated using O-benzotriazol-1-yl-*N, N*, *N*', *N*'-tetramethyl-uronium hexafluorophosphate (HBTU) and Diisopropylethylamine (DIEA). Removal of the Fmoc protecting group is achieved using a solution of 20% (V/V) piperidine in *N,N-*dimethylformamide (DMF) for 20 minutes (step 1).The selective deprotection of the Lys (Aloc) group is performed manually and accomplished by treating the resin with a solution of 3 eq of Pd(PPh₃)₄ dissolved in 5 mL of CHCl₃:NMM:HOAc (18:1:0.5) for 2 h (Step 2). The resin is then washed with CHCl₃ (6 x 5 mL), 20% HOAc in DCM (6 x 5 mL), DCM (6 x 5 mL), and DMF (6 x 5 mL). The synthesis is then re-automated for the addition of the 3-maleimidopropionic acid (Step 3). Between every coupling, the resin is washed 3 times with *N,N-*dimethylformamide (DMF) and 3 times with isopropanol. The peptide is cleaved from the resin using 85% TFA/5% TIS/5% thioanisole and 5% phenol, followed by precipitation by dry-ice cold Et₂O (Step 4). The product is purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm to afford the desired DAC in >95% purity, as determined by RP-HPLC.

### Example 43 - Modification of Kringle-5 at the N-Terminus Proline

### Preparation of (MPA-AEEA)-Pro-Arg-Lys-Leu-Tyr-Asp-Tyr-Lys-NH₂.2TFA

Using automated peptide synthesis, the following protected amino acids were sequentially added to Rink Amide MBHA resin: Fmoc-Lys(Boc)-OH, Fmoc-Tyr(tBu)OH, Fmoc-Asp(OtBu)-OH, Fmoc-Tyr(tBu)OH, Fmoc-Leu-OH, Fmoc-Lys(Boc)-OH, Fmoc-Arg(Pbf)-OH, Fmoc-Pro-OH (step 1). The deprotection of the terminal Fmoc group is accomplished using 20% piperidine (Step 2) followed by the coupling of Fmoc-AEEA. Deprotection of the resulting Fmoc-AEEA-peptide with piperidine 20% in DMF allow for the subsequent addition of the 3-MPA (Step 3). Resin cleavage and product isolation was performed using 86% TFA/5% TIS/5% H₂O/2% thioanisole and 2% phenol, followed by precipitation by dry-ice cold Et₂O (Step 4). The product was purified by preparative reversed phased HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization.

### Example 44 - Modification of Kringle-5 at the N-Terminus Proline

### Preparation of (MPA)-Pro-Arg-Lys-Leu-Tyr-Asp-Tyr-Lys-NH₂.2TFA

Using automated peptide synthesis, the following protected amino acids were sequentially added to Rink Amide MBHA resin: Fmoc-Lys(Boc)-OH, Fmoc-Tyr(tBu)OH, Fmoc-Asp(OtBu)-OH, Fmoc-Tyr(tBu)OH, Fmoc-Leu-OH, Fmoc-Lys(Boc)-OH, Fmoc-Arg(Pbf)-OH, Fmoc-Pro-OH (step 1). The deprotection of the terminal Fmoc group is accomplished using 20% piperidine (Step 2) followed by the coupling of 3-MPA (Step 3). Resin cleavage and product isolation was performed using 86% TFA/5% TIS/5% H₂O/2% thioanisole and 2% phenol, followed by precipitation by dry-ice cold Et₂O (Step 4). The product was purified by preparative reversed phased HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization

### Example 45 - Modification of Kringle-5 at the N-Terminus Tyrosine

### Preparation of (MPA-AEEA)-Tyr-Thr-Thr-Asn-Pro-Arg-Lys-Leu-Tyr-Asp-Tyr-NH₂.2TFA

Using automated peptide synthesis, the following protected amino acids were sequentially added to Rink Amide MBHA resin: Fmoc-Lys(Boc)-OH. Fmoc-Tyr(tBu)OH, Fmoc-Asp(OtBu)-OH, Fmoc-Tyr(tBu)OH, Fmoc-Leu-OH, Fmoc-Lys(Boc)-OH, Fmoc-Arg(Pbf)-OH, Fmoe-Pro-OH, Fmoc-Asn(Trt)-OH, Fmoc-Thr(tBu)-OH, Fmoc-Thr(tBu)-OH, Fmoc-Tyr(tBu)OH (Step 1). The deprotection of the terminal Fmoc group is accomplished using 20% piperidine (Step 2) followed by the coupling of Fmoc-AEEA. Deprotection of the resulting Fmoc-AEEA-peptide with piperidine 20% in DMF allow for the subsequent addition of the 3-MPA (Step 3). Resin cleavage and product isolation was performed using 86% TFA/5% TIS/5% H₂O/2% thioanisole and 2% phenol, followed by precipitation by dry-ice cold Et₂O (Step 4). The product was purified by preparative reversed phased HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization.

### Example 46 - Modification of Kringle-5 at the N-Terminus Tyrosine

### Preparation of (MPA)-Tyr-Thr-Thr-Asn-Pro-Arg-Lys-Leu-Tyr-Asp-Tyr-NH₂.2TFA

Using automated peptide synthesis, the following protected amino acids were sequentially added to Rink Amide MBHA resin: Fmoc-Lys(Boc)-OH, Fmoc-Tyr(tBu)OH, Fmoc-Asp(OtBu)-OH, Fmoc-Tyr(tBu)OH, Fmoc-Leu-OH, Fmoc-Lys(Boc)-OH, Fmoc-Arg(Pbf)-OH, Fmoc-Pro-OH, Fmoc-Asn(Trt)-OH, Fmoc-Thr(tBu)-OH, Fmoc-Thr(tBu)-OH, Fmoc-Tyr(tBu)OH (Step1). The deprotection of the terminal Fmoc group is accomplished using 20% piperidine (Step 2) followed by the coupling of 3-MPA (Step 3). Resin cleavage and product isolation was performed using 86% TFA/5% TIS/5% H₂O/2% thioanisole and 2% phenol, followed by precipitation by dry-ice cold Et₂O (Step 4). The product was purified by preparative reversed phased HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization.

### Example 47 - Modification of Kringle-5 at the N-Terminus Arginine

### Preparation of (MPA-AEEA)-Arg-Asn-Pro-Asp-Gly-Asp-Gly-Pro-Trp-Ala-Tyr-Thr-Thr-Asn-Pro-Arg-Lys-Leu-Tyr-Asp-Tyr-NH₂.3TFA

Using automated peptide synthesis, the following protected amino acids were sequentially added to Rink Amide MBHA resin: Fmoc-Lys(Boc)-OH, Fmoc-Tyr(tBu)OH, Fmoc-Asp(OtBu)-OH, Fmoc-Tyr(tBu)OH, Fmoc-Leu-OH, Fmoc-Lys(Boc)-OH, Fmoc-Arg(Pbf)-OH, Fmoc-Pro-OH, Fmoc-Asn(Trt)-OH, Fmoc-Thr(tBu)-OH, Fmoc-Thr(tBu)-OH, Fmoc-Tyr(tBu)OH, Fmoc-Ala-OH, Fmoc-Trp-OH, Fmoc-Pro-OH, Fmoc-Gly-OH, Fmoe-Gly-OH, Fmoc-Val-OH, Fmoc-Asp(OtBu)-OH, Fmoc-Gly-OH, Fmoc-Asp(OtBu)-OH, Fmoc-Pro-OH, Fmoc-Asn(Trt)-OH, Fmoc-Arg(Pbf)-OH (step 1). The deprotection of the terminal Fmoc group is accomplished using 20% piperidine (Step 2) followed by the coupling of Fmoc-AEEA. Deprotection of the resulting Fmoc-AEEA-peptide with piperidine 20% in DMF allow for the subsequent addition of the 3-MPA (Step 3). Resin cleavage and product isolation was performed using 86% TFA/5% TIS/5% H₂O/2% thioanisole and 2% phenol, followed by precipitation by dry-ice cold Et₂O (Step 4). The product was purified by preparative reversed phased HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization.

### Example 48 - Modification of Kringle-5 at the N-Terminus Arginine

### Preparation of (MPA)-Arg-Asn-Pro-Asp-Gly-Asp-Val-Gly-Gly-Pro-Trp-Ala-Tyr-Thr-Thr-Asn-Pro-Arg-Lys-Leu-Tyr-Asp-Tyr-NH₂.3TFA

Using automated peptide synthesis, the following protected amino acids were sequentially added to Rink Amide MBHA resin: Fmoc-Lys(Boc)-OH, Fmoc-Tyr(tBu)OH, Fmoc-Asp(OtBu)-OH, Fmoc-Tyr(tBu)OH, Fmoc-Leu-OH, Fmoc-Lys(Boc)-OH, Fmoc-Arg(Pbf)-OH, Fmoc-Pro-OH, Fmoc-Asn(Trt)-OH, Fmoc-Thr(tBu)-OH, Fmoc-Thr(tBu)-OH, Fmoc-Tyr(tBu)OH, Fmoc-Ala-OH, Fmoc-Trp-OH, Fmoc-Pro-OH, Fmoc-Gly-OH, Fmoc-Gly-OH, Fmoc-Val-OH, Fmoc-Asp(OtBu)-OH, Fmoc-Gly-OH, Fmoc-Asp(OtBu)-OH, Fmoc-Pro-OH, Fmoc-Asn(Trt)-OH, Fmoc-Arg(Pbf)-OH (Step 1). The deprotection of the terminal Fmoc group is accomplished using 20% piperidine (Step 2) followed by the coupling of 3-MPA (Step 3). Resin cleavage and product isolation was performed using 86% TFA/5% TIS/5% H₂O/2% thioanisole and 2% phenol, followed by precipitation by dry-ice cold Et₂O (Step 4). The product was purified by preparative reversed phased HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization.

### Example 49 - Modification of Kringle-5 at the N-Terminus Arginine

### Preparation of (MPA-AEEA)-Arg-Asn-Pro-Asp-Gly-Asp-Val-Gly-Gly-Pro-Trp-NH₂.TFA

Using automated peptide synthesis, the following protected amino acids were sequentially added to Rink Amide MBHA resin: Fmoc-Lys(Boc)-OH, Fmoc-Trp-OH, Fmoc-Pro-OH, Fmoc-Gly-OH, Fmoc-Gly-OH, Fmoc-Val-OH, Fmoc-Asp(OtBu)-OH, Fmoc-Gly-OH, Fmoc-Asp(OtBu)-OH, Fmoc-Pro-OH, Fmoc-Asn(Trt)-OH, Fmoc-Arg(Pbf)-OH (step 1). The deprotection of the terminal Fmoc group is accomplished using 20% piperidine (Step 2) followed by the coupling of Fmoc-AEEA. Deprotection of the resulting Fmoc-AEEA-peptide with piperidine 20% in DMF allow for the subsequent addition of the 3-MPA (Step 3). Resin cleavage and product isolation was performed using 86% TFA/5% TIS/5% H₂O/2% thioanisole and 2% phenol, followed by precipitation by dry-ice cold Et₂O (Step 4). The product was purified by preparative reversed phased HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization.

### Example 50 - Modification of Kringle-5 at the N-Terminus Arginine

### Preparation of (MPA)-Arg-Asn-Pro-Asp-Gly-Asp-Val-Gly-Gly-Pro-Trp-NH₂. TFA

Using automated peptide synthesis, the following protected amino acids were sequentially added to Rink Amide MBHA resin: Fmoc-Lys(Boc)-OH, Fmoc-Trp-OH, Fmoc-Pro-OH, Fmoc-Gly-OH, Fmoc-Gly-OH, Fmoc-Val-OH, Fmoc-Asp(OtBu)-OH, Fmoc-Gly-OH, Fmoc-Asp(OtBu)-OH, Fmoc-Pro-OH, Fmoc-Asn(Trt)-OH, Fmoc-Arg(Pbf)-OH (Step 1). The deprotection of the terminal Fmoc group is accomplished using 20% piperidine (Step 2) followed by the coupling of 3-MPA (Step 3). Resin cleavage and product isolation was performed using 86% TFA/5% TIS/5% H₂O/2% thioanisole and 2% phenol, followed by precipitation by dry-ice cold Et₂O (Step 4). The product was purified by preparative reversed phased HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization.

### Example 51 - Modification of Kringle-5 at the N-Terminus Arginine

### Preparation of (MPA-AEEA)-Arg-Lys-Leu-Tyr-Asp-Tyr-NH₂.2TFA

Using automated peptide synthesis, the following protected amino acids were sequentially added to Rink Amide MBHA resin: Fmoc-Lys(Boc)-OH, Fmoc-Tyr(tBu)OH, Fmoc-Asp(OtBu)-OH, Fmoc-Tyr(tBu)OH, Fmoc-Leu-OH, Fmoc-Lys(Boc)-OH, Fmoc-Arg(Pbf)-OH (Step 1). The deprotection of the terminal Fmoc group is accomplished using 20% piperidine (Step 2) followed by the coupling of Fmoc-AEEA. Deprotection of the resulting Fmoc-AEEA-peptide with piperidine 20% in DMF allow for the subsequent addition of the 3-MPA (Step 3). Resin cleavage and product isolation was performed using 86% TFA/5% TIS/5% H₂O/2% thioanisole and 2% phenol, followed by precipitation by dry-ice cold Et₂O (Step 4). The product was purified by preparative reversed phased HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization

### Example 52 - Modification of Kringle-5 at the N-Terminus Arginine

### Preparation of (MPA)-Arg-Lys-Leu-Tyr-Asp-Tyr-NH₂.2TFA

Using automated peptide synthesis, the following protected amino acids were sequentially added to Rink Amide MBHA resin: Fmoc-Lys(Boc)-OH, Fmoc-Tyr(tBu)OH, Fmoc-Asp(OtBu)-OH, Fmoc-Tyr(tBu)OH, Fmoc-Leu-OH, Fmoc-Lys(Boc)-OH, Fmoc-Arg(Pbf)-OH (Step 1). The deprotection of the terminal Fmoc group is accomplished using 20% piperidine (Step 2) followed by the coupling of 3-MPA (Step 3). Resin cleavage and product isolation was performed using 86% TFA/5% TIS/5% H₂O/2% thioanisole and 2% phenol, followed by precipitation by dry-ice cold Et₂O (Step 4). The product was purified by preparative reversed phased HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization.

### Example 53 - Modification of Kringle-5 at the N-Terminus Proline

### Preparation of (MPA-AEEA)-Pro-Arg-Lys-Leu-Tyr-Asp-NH₂.2TFA

Using automated peptide synthesis, the following protected amino acids were sequentially added to Rink Amide MBHA resin: Fmoc-Lys(Boc)-OH, Fmoc-Asp(OtBu)-OH, Fmoc-Tyr(tBu)OH, Fmoo-Leu-OH, Fmoc-Lys(Boc)-OH, Fmoc-Arg(Pbf)-OH, Fmoc-Pro-OH (step 1). The deprotection of the terminal Fmoc group is accomplished using 20% piperidine (Step 2) followed by the coupling of Fmoc-AEEA. Deprotection of the resulting Fmoc-AEEA-peptide with piperidine 20% in DMF allow for the subsequent addition of the 3-MPA (Step 3). Resin cleavage and product isolation was performed using 86% TFA/5% TIS/5% H₂O/2% thioanisole and 2% phenol, followed by precipitation by dry-ice cold Et₂O (Step 4). The product was purified by preparative reversed phased HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization.

### Example 54 - Modification of Kringle-5 at the N-Terminus Proline

### Preparation of (MPA)-Pro-Arg-Lys-Leu-Tyr-Asp-NH₂.2TFA

Using automated peptide synthesis, the following protected amino acids were sequentially added to Rink Amide MBHA resin: Fmoc-Lys(Boc)-OH, Fmoc-Asp(OtBu)-OH, Fmoc-Tyr(tBu)OH, FmocrLeu-OH, Fmoc-Lys(Boc)-OH, Fmoc-Arg(Pbf)-OH, Fmoc-Pro-OH (Step 1). The deprotection of the terminal Fmoc group is accomplished using 20% piperidine (Step 2) followed by the coupling of 3-MPA (Step 3). Resin cleavage and product isolation was performed using 86% TFA/5% TIS/5% H₂O/2% thioanisole and 2% phenol, followed by precipitation by dry-ice cold Et₂O (Step 4). The product was purified by preparative reversed phased HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization.

### 3. Modification at an Internal Amino Acid

### Example 55 - Synthesis of Lys²⁶(ε-MPA)GLP-1(7-36)-NH₂

Solid phase peptide synthesis of a modified GLP-1 analog on a 100 µmole scale was performed manually and on a Symphony Peptide Synthesizer using Fmoc protected Rink amide MBHA resin. The following protected amino acids are sequentially added to the resin: Fmoc-Arg(Pbf)-OH, Fmoc-Gly-OH, Fmoc-Lys(Boc)-OH, Fmoc-Val-OH, Fmoc-Leu-OH, Fmoo-Trp(Boc)-OH, Fmoc-Ala-OH, Fmoc-Ile-OH, Fmoc-Phe-OH, Fmoc-Glu(OtBu)-OH, Fmoc-Lys(Aloc)-OH, Fmoc-Ala-OH, Fmoc-Ala-OH, Fmoc-Gln(Trt)-OH, Fmoc-Gly-OH, Fmoc-Glu(OtBu)-OH, Fmoc-Leu-OH, Fmoc-Tyr(tBu)-OH, Fmoc-Ser(tBu)-OH, Fmoc-Ser(tBu)-OH, Fmoc-Val-OH, Fmoc-Asp(tBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Ala-OH, Boc-His(TrtrOH. They are dissolved in *N*,*N*-dimethylformamide (DMF) and, according to the sequence, activated using O-benzotriazol-1-yl-*N*, *N*, *N*', *N*'-tetramethyl-uronium hexafluorophosphate (HBTU) and Diisopropylethylamine (DIEA). Removal of the Fmoc protecting group is achieved using a solution of 20% (V/V) piperidine in *N,N*-dimethylformamide (DMF) for 20 minutes (Step 1). Selective deprotection of the Lys(Aloc) group is performed manually and accomplished by treating the resin with a solution of 3eq of Pd(PPh₃)₄ dissolved in 5mL of CHCl₃:NMM:HOAc (18:1:0.5) for 2h (Step 2). The resin is then washed with CHCl₃ (6 x 5mL), 20% HOAc in DCM (6 x 5mL), DCM (6 x 5mL), and DMF (6 x 5mL). The synthesis is then re-automated for the addition of the 3-maleimidopropionic acid (Step 3). Resin cleavage and product isolation is performed using 86% TFA/5% TIS/5% H₂O/2% thioanisole and 2% phenol, followed by precipitation by dry-ice cold Et₂O (Step 4). The product is purified by preparative reversed phase HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm to afford the desired DAC in >95% purity, as determined by RP-HPLC. These steps are illustrated in the schematic diagram below.

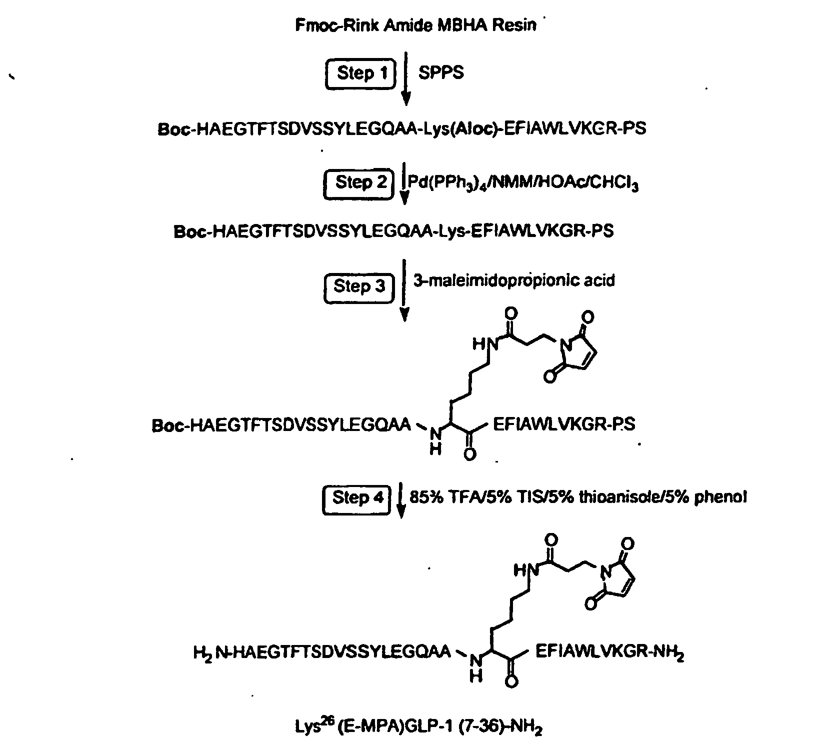

### D. Preparation of Modified Peptides From Peptides Containing One Free Cysteine

Preparation of maleimido peptides from therapeutic peptides containing one free Cysteine is exemplified by the synthesis of peptides as described below. The peptide may be modified at the N-terminus, the C-terminus, or at an amino acid located between the N-terminus and the C-terminus.

Preparation of maleimido peptides from peptides containing multiple protected functional groups and multiple Cysteine residues all with one free Cysteine residues (*i*.*e*. all Cysteine residues, except one, are tied up as disulfides). Linking from an internal amino acid in the natural sequence as in Example 5. The free Cysteine residue must be capped or replaced with another amino acid (e.g. Alanine, Methionine, etc.).

Where the peptide contains one cysteine, the cysteine must stay capped after addition of the maleimide. If the cysteine is involved in binding site, assessment has to be made of how much potency is lost is cysteine is capped by a protecting group. If the cysteine can stay capped, then the synthetic path is similar to example (i) above. Examples of therapeutic peptides that contain one cystein include G_{α} (the alpha subunit of Gtherapeutic peptide binding protein), the 724-739 fragment of rat brain nitric oxide synthase blocking peptide, the alpha subunit 1-32 fragment of human [Tyr0] inhibin, the 254-274 fragment of HIV envelope protein, and P34cdc2 kinase fragment.

### 1. Modification at the N-Terminus

### Example 56 - Modification of Inhibin Peptide at the Added N-Terminus Lysine

### Preparation of (Nε-MPA)-Lys-Tyr-Ser-Thr-Pro-Leu-Met-Ser-Trp-Pro-Trp-Ser-Pro-Ser-Ala-Leu-Arg-Leu-Leu-Gln-Arg-Pro-Pro-Glu-Glu-Pro-Ala-Ala-Ala-His-Ala-Asn-Cys-His-Arg

Solid phase peptide synthesis of a modified inhibin peptide analog on a 100 µmole scale is performed using manual solid-phase synthesis, a Symphony Peptide Synthesizer and Fmoc protected Rink Amide MBHA. The following protected amino acids are sequentially added to resin: Fmoc-Arg(Pbf)-OH, Fmoo-His(Boc)-OH, Fmoc-Cys(Trt)-OH, Fmoc-Asn(Trt)-OH, Fmoc-Ala-OH, Fmoc-His(Boc)-OH. Fmoc-Ala-OH, Fmoc-Ala-OH, Fmoc-Pro-OH, Fmoc-Glu(tBu)-OH, Fmoc-Glu(tBu)-OH, Fmoc-Pro-OH, Fmoc-Pro-OH, Fmoc-Arg(Pbf)-OH, Fmoc-Gln(Trt)-OH, Fmoc-Leu-OH, Fmoc-Leu-OH, Fmoc-Arg(Pbf)-OH, Fmoc-Leu-OH, Fmoc-Ala-OH, Fmoc-Ser(tBu)-OH, Fmoc-Pro-OH, Fmoc-Ser(tBu)-OH, Fmoc-Trp(Boc)-OH, Fmoc-Pro-OH, Fmoc-Trp(Boc)-OH, Fmoc-Ser(tBu)-OH, Fmoc-Met-OH, Fmoc-Leu-OH, Fmoc-Pro-OH, Fmoc-Thr(tBu)-OH, Fmoc-Ser(tBu)-OH, Fmoc-Tyr(tBu)-OH, Fmoc-Lys(Aloc)-OH,They are dissolved in *N,N*-dimethylformamide (DMF) and, according to the sequence, activated using O-benzotriazol-1-yl-*N*, *N, N*', *N*'-tetramethyl-uronium hexafluorophosphate (HBTU) and Diisopropylethylamine (DIEA). Removal of the Fmoc protecting group is achieved using a solution of 20% (V/V) piperidine in *N*,*N*-dimethylformamide (DMF) for 20 minutes (Step 1).The selective deprotection of the Lys (Aloc) group is performed manually and accomplished by treating the resin with a solution of 3 eq of Pd(PPh₃)₄ dissolved in 5 mL of CHCI₃:NMM:HOAc (18:1:0.5) for 2 h (Step 2). The resin is then washed with CHC)₃ (6 x 5 mL), 20% HOAc in DCM (6 x 5 mL), DCM (6 x 5 mL), and DMF (6 x 5 mL). The synthesis is then re-automated for the addition of the 3-maleimidopropionic acid (Step 3). Between every coupling, the resin is washed 3 times with *N,N*-dimethylformamide (DMF) and 3 times with isopropanol. The peptide is cleaved from the resin using 85% TFA/5% TIS/5% thioanisole and 5% phenol, followed by precipitation by dry-ice cold Et₂O (Step 4). The product is purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm to afford the desired DAC in >95% purity, as determined by RP-HPLC.

### Example 57 - Modification of RSV Antifusogenic Peptide at the N-Terminus

### Preparation of 3-(MPA)-Val-Ile-Thr-Ile-Glu-Leu-Ser-Asn-Ile-Lys-Glu-Asn-Lys-Cys-Asn-Glu-Ala-Lys-Val-Lys-Leu-Ile-Lys-Glu-Glu-Leu-Asp-Lys-Tyr-Lys-Asn-Ala-Val

Initially, (Cysteine (Cys) was replaced with Methionine (Met) within the native sequence. Solid phase peptide synthesis of a modified anti RSV analog on a 100 µmole scale was performed on a Symphony Peptide Synthesizer using Fmoc protected Rink Amide MBHA resin, Fmoc protected amino acids, O-benzotriazol-1-yl-*N*, *N, N*', *N*'-tetramethyl-uronium hexafluorophosphate (HBTU) in *N*,*N-*dimethylformamide (DMF) solution and activation with *N*-methyl morpholine (NMM), and piperidine deprotection of Fmoc groups (Step 1). The deprotection of the terminal Fmoc group is accomplished using 20% piperidine (Step 2) followed by the coupling of 3-MPA (Step 3). Resin cleavage and product isolation was performed using 85% TFA/5% TIS/5% thioanisole and 5% phenol, followed by precipitation by dry-ice cold Et₂O (Step 4). The product is purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm to afford the desired DAC in >95% purity, as determined by RP-HPLC. These steps are illustrated by the schematic diagram below.

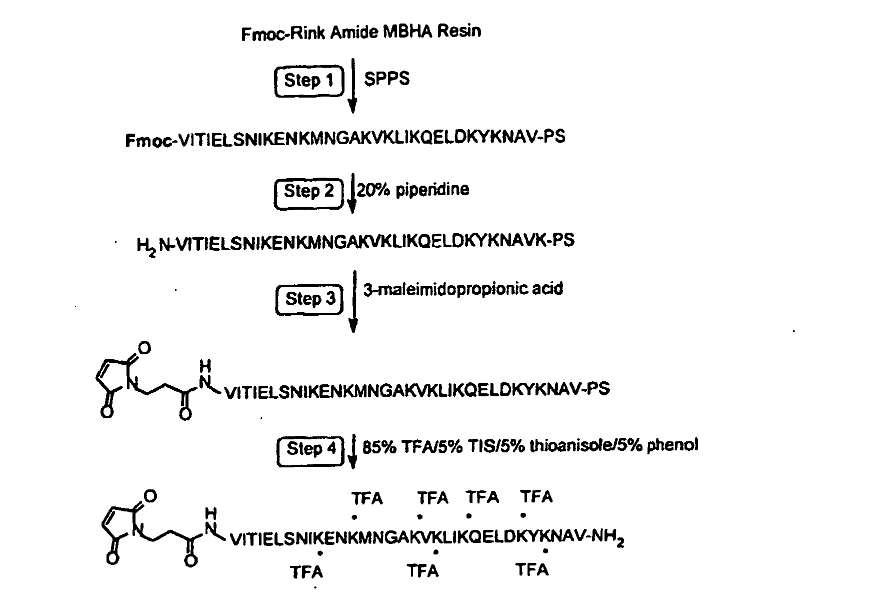

### 2. Modification at the C-Terminus

### Example 58 - Modification of Inhibin Peptide at the Added C-Terminus Lysine

### Preparation of (Nε-MPA)-Lys-Cys-Asn-Leu-Lys-Glu-Asp-Gly-Ile-Ser-Ala-Ala-Lys-Asp-Val-Lys

Solid phase peptide synthesis of a modified inhibin peptide analog on a 100 µmole scale is performed using manual solid-phase synthesis, a Symphony Peptide Synthesizer and Fmoc protected Rink Amide MBHA. The following protected amino acids are sequentially added to resin: Fmoc-Lys(Boc)-OH, Fmoc-Val-OH, Fmoc-Asp(tBu)-OH, Fmoc-Lys(Boc)-OH, Fmoc-Ala-OH, Fmoc-Ala-OH, Fmoc-Ser(tBu)-OH, Fmoc-Ile-OH, Fmoc-Gly-OH, Fmoc-Asp(tBu)-OH, Fmoc-Glu(tBu)-OH, Fmoc-Lys(Boc)-OH, Fmoc-Leu-OH, Fmoc-Asn(Trt)-OH, Fmoc-Cys(Trt)-OH, Fmoc-Lys(Aloc)-OH, They are dissolved in *N,N*-dimethylformamide (DMF) and, according to the sequence, activated using O-benzotriazol-1-yl-*N, N, N', N*'-tetramethyl-uronium hexafluorophosphate (HBTU) and Diisopropylethylamine (DIEA). Removal of the Fmoc protecting group is achieved using a solution of 20% (V/V) piperidine in *N,N-*dimethylformamide (DMF) for 20 minutes (Step 1).The selective deprotection of the Lys (Aloc) group is performed manually and accomplished by treating the resin with a solution of 3 eq of Pd(PPh₃)₄ dissolved in 5 mL of CHCl₃:NMM:HOAc (18:1:0.5) for 2 h (Step 2). The resin is then washed with CHCl₃ (6 x 5 mL), 20% HOAc in DCM (6 x 5 mL), DCM (6 x 5 mL), and DMF (6 x 5 mL). The synthesis is then re-automated for the addition of the 3-maleimidopropionic acid (Step 3). Between every coupling, the resin is washed 3 times with *N,N-*dimethytformamide (DMF) and 3 times with isopropanol. The peptide is cleaved from the resin using 85% TFA/5% TIS/5% thioanisole and 5% phenol, followed by precipitation by dry-ice cold Et₂O (Step 4). The product is purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm to afford the desired DAC in >95% purity, as determined by RP-HPLC.

### Example 59 - Modification of RSV Fusogenic Peptide at the C-Terminus Val-Ile-Thr-Ile-Glu-Leu-Ser-Asn-Ile-Lys-Glu-Asn-Lys-Cys-Asn-Gly-Ala-Lys-Val-Lys-Leu- Ile- Lys-Gln-Glu-Leu-Asp-Lys-Tyr-Asn-Ala-Val-(AEEA.MPA)

Preparation of maleimido peptides from peptides containing multiple protected functional groups and one cysteine is exemplified by the synthesis of a modified RSV fusogenic peptide. The modified RSV fusogenic peptide was synthesized by linking off the C-terminus by the addition of a lysine residue to the natural peptide sequence as illustrated by the schematic diagram below. In cases where a cysteine residue is contained within the peptide sequence and is not essentially to the biological activity of the peptide, this residue must be replaced with another amino acid (e.g. alanine, methionine, etc.). In the following synthesis, the cysteine (Cys) was replaced with a methionine (Met) within the native RSV sequence.

Solid phase peptide synthesis of the maleimido RSV fusogenic peptide on a 100 µmole scale is performed using manual solid-phase synthesis and a Symphony Peptide Synthesizer using Fmoc protected Rink Amide MBHA resin, Fmoc protected amino acids, O-benzotriazol-1-yl-*N*, *N, N', N*'-tetramethyl-uronium hexafluorophosphate (HBTU) in *N,N-*dimethylformamide (DMF) solution and activation with *N*-methyl morpholine (NMM), and piperidine deprotection of Fmoc groups (Step 1). The selective deprotection of the Lys(Aloc) group is performed manually and accomplished by treating the resin with a solution of 3 eq of Pd(PPh₃)₄ dissolved in 5 mL of CHCl₃:NMM:HOAc (18:1:0.5) for 2 h (Step 2). The resin is then washed with CHCl₃ (6 x 5 mL), 20% HOAc in DCM (6 x 5 mL), DCM (6 x 5 mL), and DMF (6 x 5 mL). The synthesis is then re-automated for the addition of 3-maleimidopropionic acid (Step 3). Resin cleavage and product isolation is performed using 85% TFA/5% TIS/5% thioanisole and 5% phenol, followed by precipitation by dry-ice cold Et₂O (Step 4). The product is purified by preparative reverse phase HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm to afford the desired DAC in >95% purity, as determined by RP-HPLC. These steps are illustrated in the schematic diagram below.

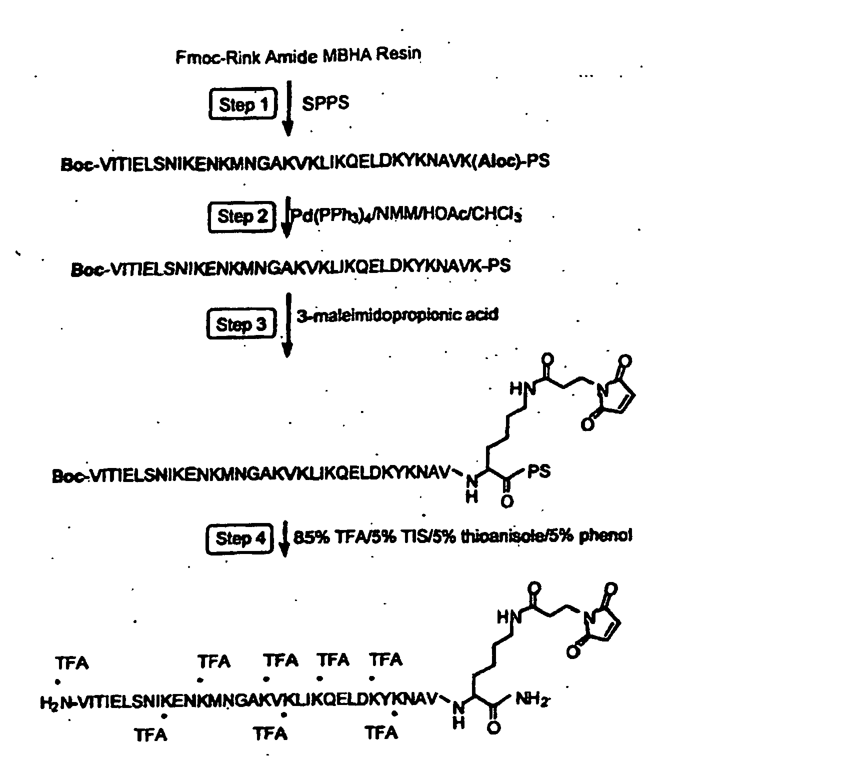

### 3. Modification at an Internal Amino Acid

### Example 60 - Modification of Gα Peptide at Cys-Asn-Leu-Lys-Glu-Asp-Gly-Ile-Ser-Ala-Ala-Lys-Asp-Val

Preparation of maleimido peptides from peptides containing multiple protected functional groups and one cysteine is exemplified by the synthesis of a modified Gα peptide. The modified Gα peptide is synthesized by linking at an internal amino acid as described below.

In cases where a cysteine residue is contained within the peptide sequence and is not essentially to the biological activity of the peptide, this residue must be capped or replaced with another amino acid (e.g. alanine, methionine, etc.). Solid phase peptide synthesis of the modified Gα peptide on a 100 µmole scale is performed using manual solid-phase synthesis and a Symphony Peptide Synthesizer using Fmoc protected Rink Amide MBHA resin, Fmoc protected amino acids, O-benzotriazol-1-yl-*N*, *N*, *N*', *N*'-tetramethyl-uronium hexafluorophosphate (HBTU) in *N,N*-dimethylformamide (DMF) solution and activation with *N-*methyl morpholine (NMM), and piperidine deprotection of Fmoc groups (Step 1). The selective deprotection of the Lys(Aloc) group is performed manually and accomplished by treating the resin with a solution of 3 eq of Pd(PPh₃)₄ dissolved in 5 mL of CHCl₃:NMM:HOAc (18:1:0.5) for 2 h (Step 2). The resin is then washed with CHCl₃(6 x 5 mL), 20% HOAc in DCM (6 x 5 mL), DCM (6 x 5 mL), and DMF (6 x 5 mL). The synthesis is then re-automated for the addition of 3-maleimidopropionic acid (Step 3). Resin cleavage and product isolation is performed using 85% TFA/5% TIS/5% thioanisole and 5% phenol, followed by precipitation by dry-ice cold Et₂O (Step 4). The product is purified by preparative reversed phased HPLC using a Varian (Rainin) preparative binary HPLC system: gradient elution of 30-55% B (0.045% TFA in H₂O (A) and 0.045% TFA in CH₃CN (B)) over 180 min at 9.5 mL/min using a Phenomenex Luna 10 µ phenyl-hexyl, 21 mm x 25 cm column and UV detector (Varian Dynamax UVD II) at λ 214 and 254 nm to afford the desired DAC in >95% purity, as determined by RP-HPLC.

### E. Preparation of Modified Peptides From Peptides Containing Two Cysteines In Disulfide Bridge

Where the peptide contains two cysteines as a disulfide bridge, the peptide is cleaved from the support resin before addition of the maleimide. We need to add a Lys protected with a Mtt group in order to selectively deprotect the lysine in presence of other t-Boc protected lysine. All protecting groups are present except at the carboxy terminus (which stays unprotected due to cleavage from the support resin) and at the two cysteines, which need to be deprotected when peptide is cleaved from resin. Mild air oxidation yield the disulfide bridge, and the peptide can be purified at that stage. Solution phase chemistry is then required to activate the C-terminus in presence of the disulfide bridge and add the maleimide (through an amino-alkyl-maleimide) to the C-terminus. The peptide is then fully deprotected. Examples of therapeutic peptides that contain two cysteins as a disulfide bridge include human osteocalcin 1-49, human diabetes associated peptide, the 5-28 fragment of human/canine atrial natriuretic peptide, bovine bactenecin, and human [Tyr0]-cortistatin 29.

Preparation of maleimido peptides from therapeutic peptides containing two Cysteines in a disulfide bridge is exemplified by the synthesis of peptides as described below. The peptide may be modified at the N-terminus, the C-terminus, or at an amino acid located between the N-terminus and the C-terminus.

### 1. Modification at the N-Terminus

### Example 61 - Modification of TH-1 Peptide at N-Terminus

### Preparation of (Nε-MPA)NH₂-Lys-Arg-Gly-Asp-Ala-Cys-Glu-Gly-Asp-Ser-Gly-Gly-Pro-Phe-Cys

Preparation of thiol cyclized maleimido peptides from peptides containing multiple protected functional groups and no free cysteine residues (*i*.*e*. all cysteine residues are tied up as disulfide bridges) is illustrated by the synthesis of a modified TH-1 peptide.

Solid phase peptide synthesis of the modified TH-1 peptide on a 100 µmole scale was performed manually and on a Symphony Peptide Synthesizer using Fmoc protected Rink Amide MBHA resin, Fmoc protected amino acids, O-benzotriazol-1-yl-*N*, *N*, *N*', *N*'-tetramethyl-uronium hexafluorophosphate (HBTU) in *N,N*-dimethylformamide (DMF) solution and activation with *N*-methyl morpholine (NMM), and piperidine deprotection of Fmoc groups (Step 1). The removal of the Acm group and resulting oxidation of the two Cys residues to form the cyclized on resin DAC was accomplished using TI(CF₃CO)₂ (Step 2). The deprotection of the terminal Fmoc group is accomplished using 20% piperidine followed by the coupling of 3-MPA (Step 3). Resin cleavage and product isolation was performed using 86% TFA/5% TIS/5% H₂O/2% thioanisole and 2% phenol, followed by precipitation by dry-ice cold Et₂O (Step 4). The product was purified by preparative reverse phase HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm. The product had >95% purity as determined by RP-HPLC mass spectrometry using a Hewlett Packard LCMS-1100 series spectrometer equipped with a diode array detector and using electro-spray ionization, ESI-MS *m*/*z* for C₆₆H₉₅N₂₀O₂₆S₂ (MH⁺). 1646.8. Found: 1646.7. These steps are illustrated in the schematic diagram below.

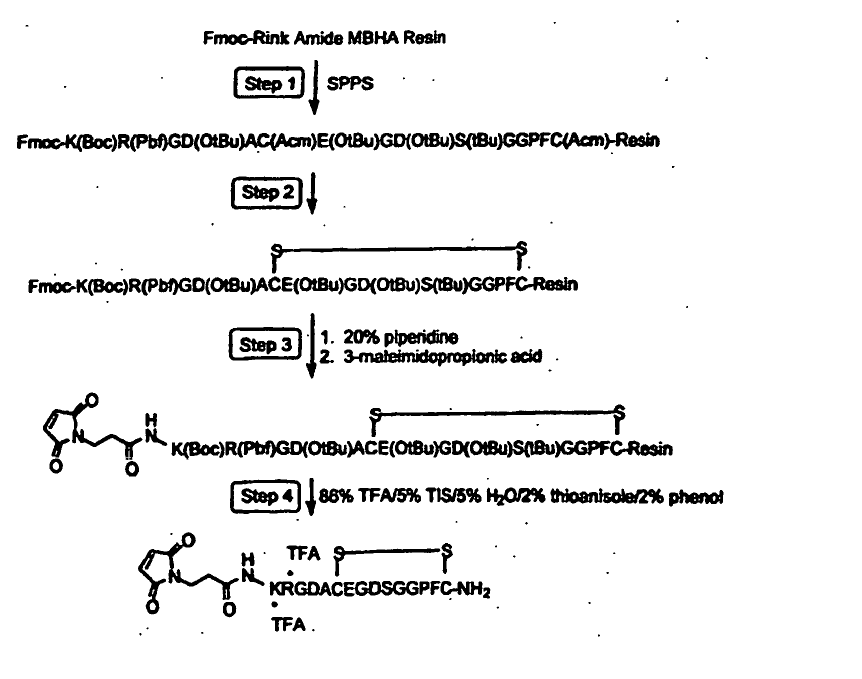

### Example 62 - Synthesis of N-MPA-Ser¹-Somatostatin-28

Solid phase peptide synthesis of the DAC:Somatostatin-28 analog on a 100 µmole scale is performed manually and on a Symphony Peptide Synthesizer using Fmoc protected Rink amide MBHA resin. The following protected amino acids are sequentially added to the resin: Fmoc-Cys(Acm)-OH, Fmoc-Ser(tBu)-OH, Fmoc-Thr(tBu)-OH, Fmoc-Phe-OH, Fmoc-Thr(tBu)-OH, Fmoc-Lys(Boc)-OH, Fmoc-Trp(Boc)-OH, Fmoc-Phe-OH, Fmoc-Phe-OH, Fmoc-Asn(Trt)-OH, Fmoc-Lys(Boc)-OH, Fmoc-Cys(Acm)-OH, Fmoc-Gly-OH, Fmoc-Ala-OH, Fmoc-Lys(Soc)-OH, Fmoc-Arg(Pbf)-OH, Fmoc-Glu(OtBu)-OH, Fmoc-Arg(Pbf)-OH, Fmoc-Pro-OH, Fmoc-Ala-OH, Fmoc-Met-OH, Fmoc-Ala-OH, Fmoc-Pro-OH, Fmoc-Asn(Trt)-OH, Fmoc-Ser(tBu)-OH, Fmoc-Asn(Trt)-OH, Fmoc-Ala-OH, Fmoc-Ser(tBu)-OH. They are dissolved in *N,N*-dimethylformamide (DMF) and, according to the sequence, activated using O-benzotriazol-1-yl-*N*, *N, N'*, *N*'-tetramethyl-uronium hexafluorophosphate (HBTU) and Diisopropylethylamine (DIEA). Removal of the Fmoc protecting group is achieved using a solution of 20% (V/V) piperidine in *N,N-*dimethylformamide (DMF) for 20 minutes (Step 1). Removal of the Acm groups and resulting oxidation of the two Cys residues to form the disulfide bridge is accomplished using iodine (Step 2). Deprotection of the terminal Fmoc group is accomplished using 20% piperidine followed by the coupling of 3-MPA (Step 3). Resin cleavage and product isolation is performed using 86% TFA/5% TIS/5% H₂O/2% thioanisole and 2% phenol, followed by precipitation by dry-ice cold Et₂O (Step 4). The product is purified by preparative reversed phase HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm to afford the desired DAC in >95% purity, as determined by RP-HPLC. These steps are illustrated in the schematic diagram below.

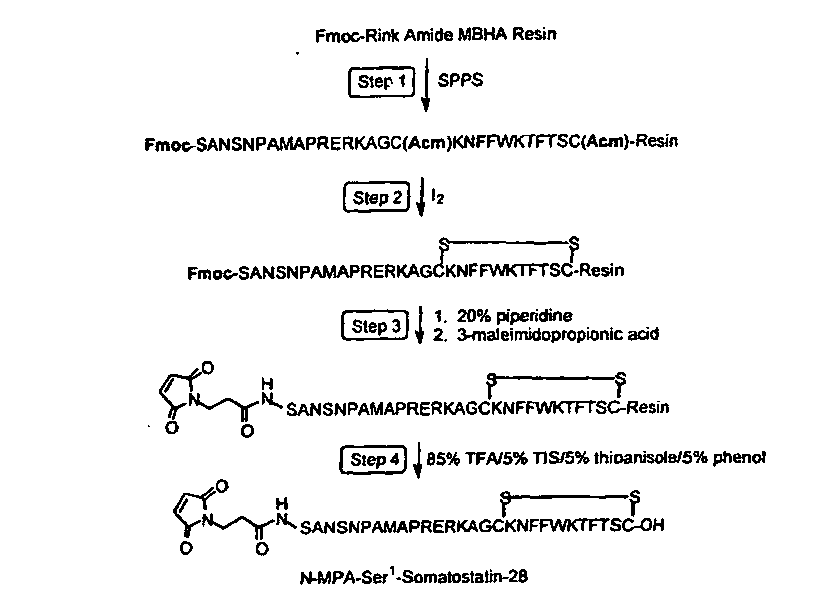

### 2. Modification at the C-Terminus

### Example 63 - Synthesis of Somatostatin-28-EDA-MPA

Solid phase peptide synthesis of the DAC:Somatostatin-28 analog on a 100 µmole scale is performed manually and on a Symphony Peptide Synthesizer using SASRIN (super acid sensitive resin). The following protected amino acids are sequentially added to the resin: Fmoc-Cys(Acm)-OH, Fmoc-Ser(tBu)-OH, Fmoc-Thr(tBu)-OH, Fmoc-Phe-OH, Fmoc-Thr(tBu)-OH, Fmoc-Lys(Boc)-OH, Fmoc-Trp(Boc)-OH, Fmoc-Phe-OH, Fmoc-Phe-OH, Fmoc-Asn(Trt)-OH, Fmoc-Lys(Boc)-OH, Fmoc-Cys(Acm)-OH, Fmoc-Gly-OH, Fmoc-Ala-OH, Fmoc-Lys(Boc)-OH, Fmoc-Arg(Pbf)-OH, Fmoc-Glu(OtBu)-OH, Fmoc-Arg(Pbf)-OH, Fmoc-Pro-OH, Fmoc-Ala-OH, Fmoc-Met-OH, Fmoc-Ala-OH, Fmoc-Pro-OH, Fmoc-Asn(Trt)-OH, Fmoc-Ser(tBu)-OH, Fmoc-Asn(Trt)-OH, Fmoc-Ala-OH, Boc-Ser(tBu)-OH. They are dissolved in *N,N*-dimethylformamide (DMF) and, according to the sequence, activated using O-benzotriazol-1-yl-*N, N, N', N*'-tetramethyl-uronium hexafluorophosphate (HBTU) and Diisopropylethylamine (DIEA). Removal of the Fmoc protecting group is achieved using a solution of 20% (V/V) piperidine in *N,N-*dimethylformamide (DMF) for 20 minutes (Step 1). The fully protected peptide is cleaved from the resin by treatment with 1% TFA / DCM (Step 2). Removal of the Acm groups and resulting oxidation of the two Cys residues to form the disulfide bridge is accomplished using iodine (Step 3). Ethylenediamine and 3-maleimidopropionic acid are then sequentially added to the free C-terminus (Step 4). The protecting groups are then cleaved and the product isolated using 86% TFA/5% TIS/5% H₂O/2% thioanisole and 2% phenol, followed by precipitation by dry-ice cold Et₂O (Step 5). The product is purified by preparative reversed phase HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm to afford the desired DAC in >95% purity, as determined by RP-HPLC. These steps are illustrated in the schematic diagram below.

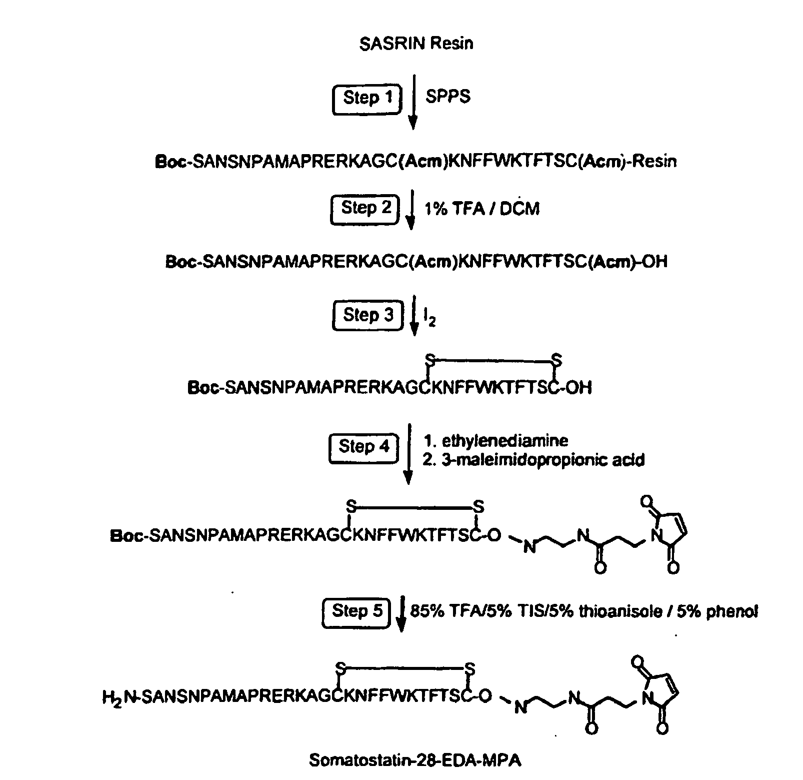

### 3. Modification at an Internal Amino Acid

### Example 64 - Synthesis of Lys¹⁴(ε-MPA)-Somatostatin-28

Solid phase peptide synthesis of the DAC:Somatostatin-28 analog on a 100 µmole scale is performed manually and on a Symphony Peptide Synthesizer using Fmoc protected Rink amide MBHA resin. The following protected amino acids are sequentially added to the resin: Fmoc-Cys(Acm)-OH, Fmoc-Ser(tBu)-OH, Fmoc-Thr(tBu)-OH, Fmoc-Phe-OH, Fmoc-Thr(tBu)-OH, Fmoc-Lys(Boc)-OH, Fmoc-Trp(Boc)-OH, Fmoc-Phe-OH, Fmoc-Phe-OH, Fmoc-Asn(Trt)-OH, Fmoc-Lys(Boc)-OH, Fmoc-Cys(Acm)-OH, Fmoc-Gly-OH, Fmoc-Ala-OH, Fmoc-Lys(Aloc)-OH, Fmoc-Arg(Pbf)-OH, Fmoc-Glu(OtBu)-OH, Fmoc-Arg(Pbf)-OH, Fmoc-Pro-OH, Fmoc-Ala-OH, Fmoc-Met-OH, Fmoc-Ala-OH, Fmoc-Pro-OH, Fmoc-Asn(Trt)-OH, Fmoc-Ser(tBu)-OH, Fmoc-Asn(Trt)-OH, Fmoc-Ala-OH, Fmoc-Ser(tBu)-OH. They are dissolved in *N,N*-dimethylformamide (DMF) and, according to the sequence, activated using O-benzotriazol-1-yl-*N, N, N', N*'-tetramethyl-uronium hexafluorophosphate (HBTU) and Diisopropylethylamine (DIEA). Removal of the Fmoc protecting group is achieved using a solution of 20% (V/V) piperidine in *N,N-*dimethylformamide (DMF) for 20 minutes (Step 1). Removal of the Acm groups and resulting oxidation of the two Cys residues to form the disulfide bridge is accomplished using iodine (Step 2). Selective deprotection of the Lys(Aloc) group is performed manually and accomplished by treating the resin with a solution of 3 eq of Pd(PPh₃)₄ dissolved in 5mL of CHCl₃:NMM:HOAc (18:1:0.5) for 2h (Step 3). The resin is then washed with CHCl₃ (6 x 5mL), 20% HOAc in DCM (6 x 5mL), DCM (6 x 5mL), and DMF (6 x 5mL). The synthesis is then re-automated for the addition of the 3-maleimidopropionic acid (Step 4). Resin cleavage and product isolation is performed using 86% TFA/5% TIS/5% H₂O/2% thioanisole and 2% phenol, followed by precipitation by dry-ice cold Et₂O (Step 5). The product is purified by preparative reversed phase HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm to afford the desired DAC in >95% purity, as determined by RP-HPLC. These steps are illustrated in the following schematic diagram.

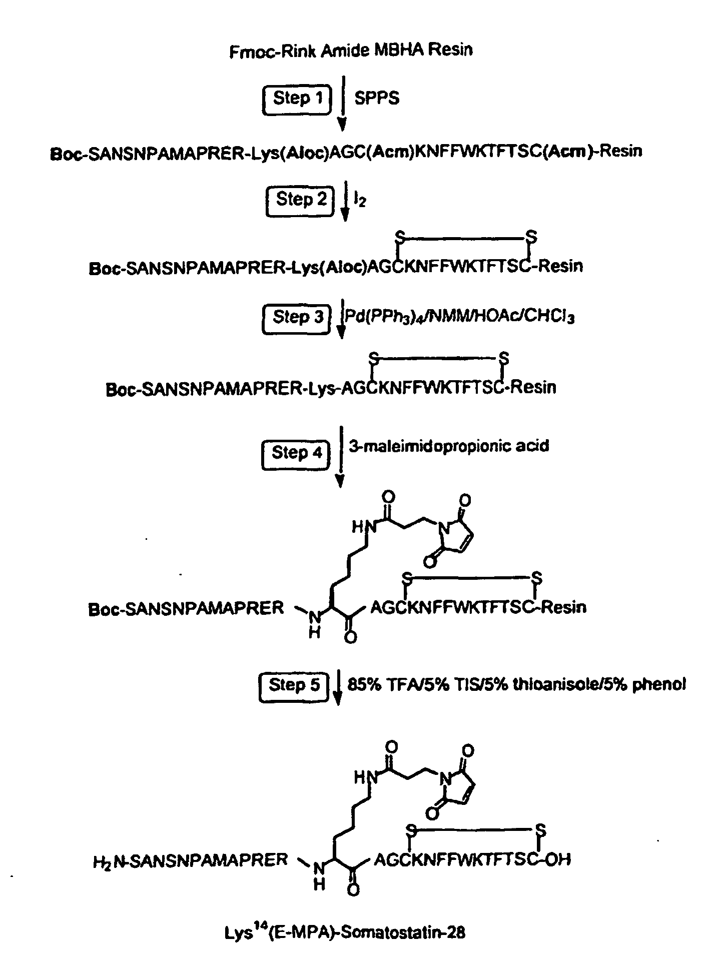

### F. Preparation of Modified Peptides From Peptides Containing Multiple Cysteines

### 1. Modification at the N-Terminus

### Example 65- Synthesis of N-MPA-Cys¹-Endothelin-1 (1-21)-OH

Solid phase peptide synthesis of a modified Endothelin-1 analog on a 100 µmole scale is performed manually and on a Symphony Peptide Synthesizer using Fmoc protected Rink Amide MBHA resin. The following protected amino acids are sequentially added to the resin: Fmoc-Trp(Boc)-OH, Fmoc-Ile-OH, Fmoc-Ile-OH, Fmoc-Asp(OtBu)-OH, Fmoc-Leu-OH, Fmoc-His(Trt)-OH, Fmoc-Cys(Acm)-OH, Fmoc-Phe-OH, Fmoc-Tyr(tBu)-OH, Fmoc-Val-OH, Fmoc-Cys(tBu)-OH, Fmoc-Glu(OtBu)-OH, Fmoc-Lys(Boc)-OH, Fmoc-Asp(OtBu)-OH, Fmoc-Met-OH, Fmoc-Leu-OH, Fmoc-Ser(tBu)-OH, Fmoc-Ser(tBu)-OH, Fmoc-Cys(tBu)-OH, Fmoc-Ser(tBu)-OH, Fmoc-Cys(Acm)-OH. They are dissolved in *N,N*-dimethylformamide (DMF) and, according to the sequence, activated using O-benzotriazol-1-yl-*N*, *N, N*', *N*'-tetramethyl-uronium hexafluorophosphate (HBTU) and Diisopropylethylamine (DIEA). Removal of the Fmoc protecting groups is achieved using a solution of 20% (V/V) piperidine in *N,N*-dimethylformamide (DMF) for 20 minutes (Step 1). The removal of the Acm groups and resulting oxidation of the first two Cys residues to form the first disulfide bridge on resin is accomplished using iodine (Step 2). The removal of the tBu groups and resulting oxidation of the other two Cys residues to form the second disulfide bridge on resin is accomplished using thallium (III) trifluoroacetate (Step 3). The deprotection of the terminal Fmoc group is accomplished using 20% piperidine followed by the coupling of 3-MPA (Step 4). Resin cleavage and product isolation is performed using 86% TFA/5% TIS/5% H₂O/2% thioanisole and 2% phenol, followed by precipitation by dry-ice cold Et₂O (Step 5). The product is purified by 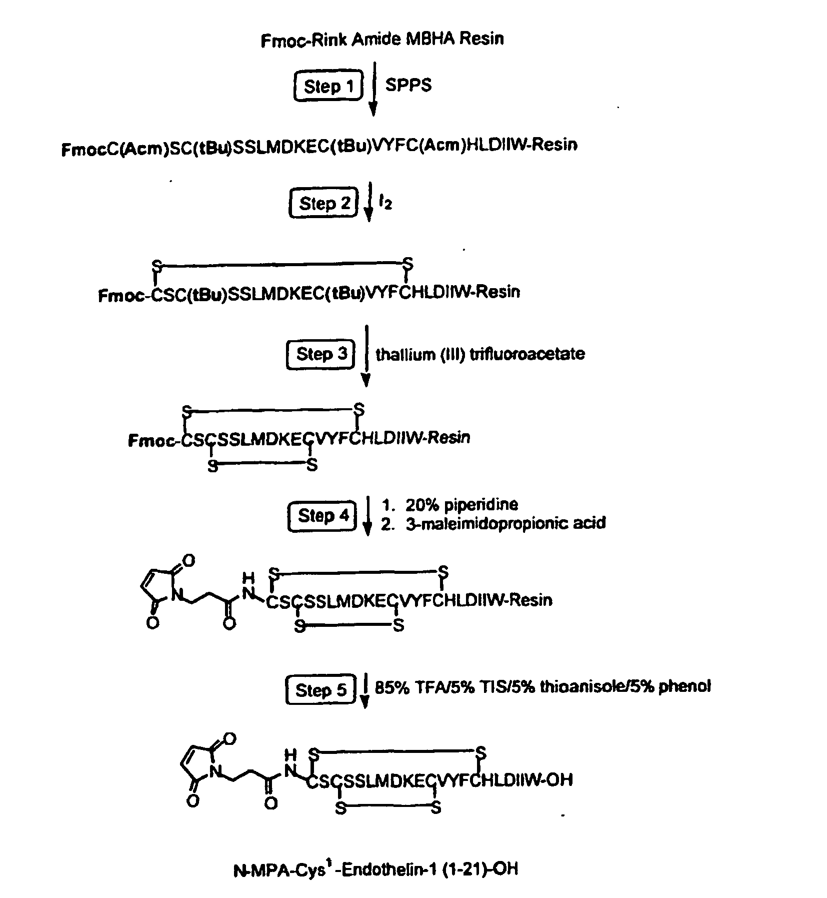 preparative reverse phase HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm to afford the desired DAC in >95% purity, as determined by RP-HPLC. These steps are illustrated in the schematic diagram above.

### 2. Modification at the C-Terminus

### Example 66 - Synthesis of Endothelin-1 (1-21)Lys²²-(Nε-MPA)-OH

Solid phase peptide synthesis of a modified Endothelin-1 analog on a 100 µmole scale is performed manually and on a Symphony Peptide Synthesizer using Fmoc protected Rink Amide MBHA resin. The following protected amino acids are sequentially added to the resin: Fmoc-Lys(Aloc)-OH, Fmoc-Trp(Boc)-OH, Fmoc-Ile-OH, Fmoc-Ile-OH, Fmoc-Asp(OtBu)-OH, Fmoc-Leu-OH, Fmoc-His(Trt)-OH, Fmoc-Cys(Acm)-OH, Fmoc-Phe-OH, Fmoc-Tyr(tBu)-OH, Fmoc-Val-OH, Fmoc-Cys(tBu)-OH, Fmoc-Glu(OtBu)-OH, Fmoc-Lys(Boc)-OH, Fmoc-Asp(OtBu)-OH, Fmoc-Met-OH, Fmoc-Leu-OH, Fmoc-Ser(tBu)-OH, Fmoc-Ser(tBu)-OH, Fmoc-Cys(tBu)-OH, Fmoc-Ser(tBu)-OH, Boc-Cys(Acm)-OH. They are dissolved in *N,N*-dimethylformamide (DMF) and, according to the sequence, activated using O-benzotriazol-1-yl-*N, N*, *N*', *N*'-tetramethyl-uronium hexafluorophosphate (HBTU) and Diisopropylethylamine (DIEA). Removal of the Fmoc protecting group is achieved using a solution of 20% (V/V) piperidine in *N,N-*dimethylformamide (DMF) for 20 minutes (Step 1). The removal of the Acm groups and resulting oxidation of the first two Cys residues to form the first disulfide bridge on resin is accomplished using iodine (Step 2). The removal of the tBu groups and resulting oxidation of the other two Cys residues to form the second disulfide bridge on resin is accomplished using thallium (III) trifluoroacetate (Step 3). Selective deprotection of the Lys(Aloc) group is performed manually and accomplished by treating the resin with a solution of 3 eq of Pd(PPh3)4 dissolved in 5 mL of CHCl₃:NMM:HOAc (18:1:0.5) for 2h (Step 4). The resin is then washed with CHCl3 (6 x 5mL), 20% HOAc in DCM (6 x 5mL), DCM (6 x 5mL), and DMF (6 x 5mL). The synthesis is then re-automated for the addition of the 3-maleimidopropionic acid (Step 5). Resin cleavage and product isolation is performed using 86% TFA/5% TIS/5% H₂O/2% thioanisole and 2% phenol, followed by precipitation by dry-ice cold Et₂O (Step 5). The product is purified by preparative reverse phase HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm to afford the desired DAC in >95% purity, as determined by RP-HPLC. These steps are illustrated in the schematic diagram below.

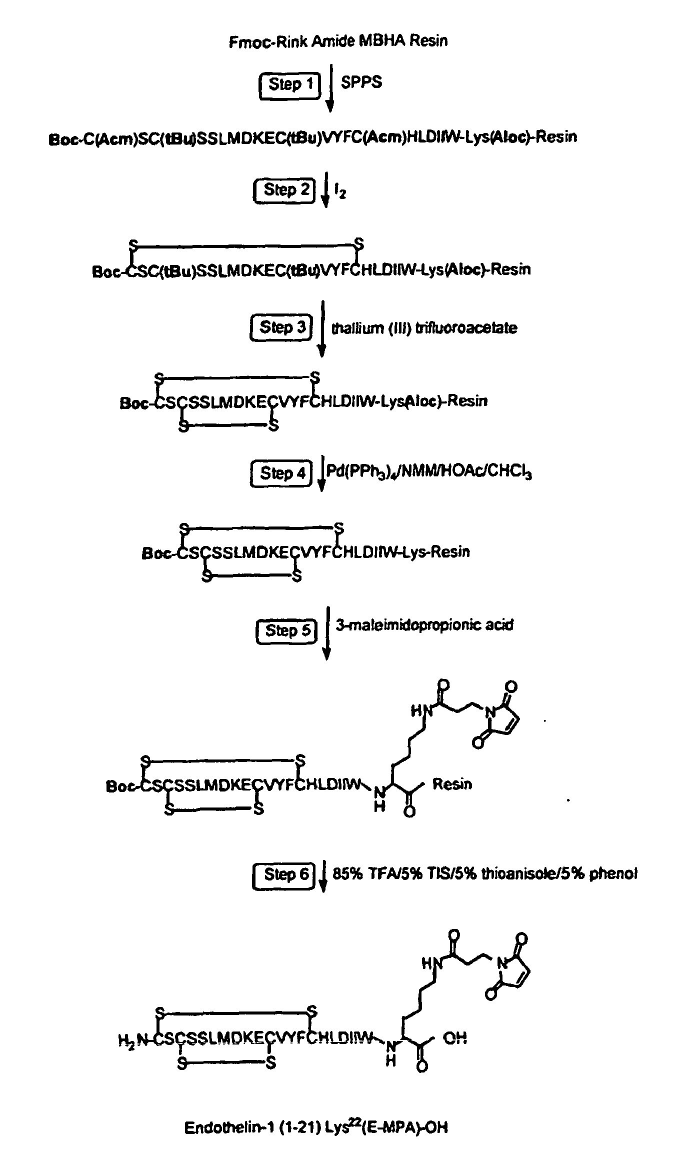

### 3. Modification at an Internal Amino Acid

### Example 67- Synthesis of Lys⁴(Nε-MPA)Sarafotoxin B(1-21)-OH

Solid phase peptide synthesis of a modified Sarafotoxin-B analog on a 100 µmole scale is performed manually and on a Symphony Peptide Synthesizer using Fmoc protected Rink Amide MBHA resin. The following protected amino acids are sequentially added to the resin: Fmoc-Trp(Boc)-OH, Fmoc-Ile-OH, Fmoc-Val-OH, Fmoc-Asp(OtBu)-OH, Fmoc-Gln(Trt)-OH, Fmoc-His(Trt)-OH, Fmoc-Cys(Acm)-OH, Fmoc-Phe-OH, Fmoc-Tyr(tBu)-OH, Fmoc-Leu-OH, Fmoc-Cys(tBu)-OH, Fmoc-Glu(OtBu)-OH, Fmoc-Lys(Boc)-OH, Fmoc-Asp(OtBu)-OH, Fmoc-Thr(tBu)-OH, Fmoc-Met-OH, Fmoc-Asp(OtBu)-OH, Fmoc-Lys(Aloc)-OH, Fmoc-Cys(tBu)-OH, Fmoc-Ser(tBu)-OH, Boc-Cys(Acm)-OH. They are dissolved in *N,N*-dimethylformamide (DMF) and, according to the sequence, activated using O-benzotriazol-1-yl-*N, N, N'*, *N*'-tetramethyl-uronium hexafluorophosphate (HBTU) and Diisopropylethylamine (DIEA). Removal of the Fmoc protecting group is achieved using a solution of 20% (V/V) piperidine in *N,N*-dimethylformamide (DMF) for 20 minutes (Step 1). The removal of the Acm groups and resulting oxidation of the first two Cys residues to form the first disulfide bridge on resin is accomplished using iodine (Step 2). The removal of the *t*Bu groups and resulting oxidation of the other two Cys residues to form the second disulfide bridge on resin is accomplished using thallium (III) trifluoroacetate (Step 3). Selective deprotection of the Lys(Aloc) group is performed manually and accomplished by treating the resin with a solution of 3 eq of Pd(PPh3)4 dissolved in 5 mL of CHCl₃:NMM:HOAc (18:1:0.5) for 2h (Step 4). The resin is then washed with CHCl3 (6 x 5mL), 20% HOAc in DCM (6 x 5mL), DCM (6 x 5mL), and DMF (6 x 5mL). The synthesis is then re-automated for the addition of the 3-maleimidopropionic acid (Step 5). Resin cleavage and product isolation is performed using 86% TFA/5% TIS/5% H₂O/2% thioanisole and 2% phenol, followed by precipitation by dry-ice cold Et₂O (Step 5). The product is purified by preparative reverse phase HPLC using a Varian (Rainin) preparative binary HPLC system using a Dynamax C₁₈, 60Å, 8 µm, 21 mm x 25 cm column equipped with a Dynamax C₁₈, 60Å, 8 µm guard module, 21 mm x 25 cm column and UV detector (Varian Dynamax UVD II) at λ 214 and 254 nm to afford the desired DAC in >95% purity, as determined by RP-HPLC. These steps are illustrated in the schematic diagram below.

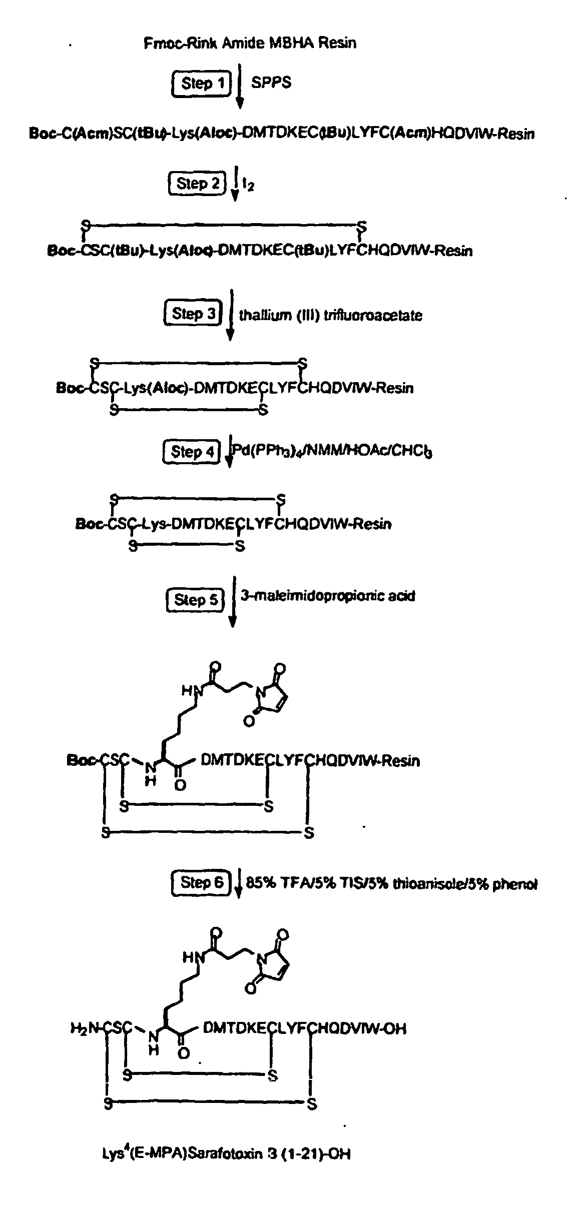

### F. Peptide Stability Assays

### Example 68 - Peptide Stability Assay of K5

A peptide stability assay was performed. (MPA)-Pro-Arg-Lys-Leu-Tyr-Asp-Lys-NH₂. 2TFA was synthesized as described above and was identified MPA-K5. The non-modified counterpart peptide Pro-Arg-Lys-Leu-Tyr-Asp-Lys was also synthesized as described in Example 20 without the addition of 3-MPA and identified as K5.

K5 (MW1260.18, 918.12 freebase) was prepared as a 100 mM stock solution in water. MPA-K5 (MW = 1411.17, 1069.11 freebase) was prepared as a 100 mM stock solution in water. Human Serum Albumin (HSA) was obtained as a 25% solution (ca 250 mg/ml, 3.75 mM) as Albutein® available from Alpha Therapeutic. Human plasma was obtained from Golden West Biologicals.

### (1) Stability of K5 in Human Plasma

K5 was prepared as a 1 µM solution and dissolved in 25% human serum albumin. The mixture was then incubated at 37°C in the presence of human plasma to final concentration of 160 mM K5. Aliquots of 100 µl were withdrawn from the plasma at 0, 4 hours and 24 hours. The 100 µl aliquots were mixed with 100 µl of blocking solution (5 vol. 5%ZnSO₄/3 vol. Acetonitrile/2 vol. Methanol) in order to precipitate all proteins. The sample was centrifuged for 5 min at 10,000 g and the supematant containing the peptide was recovered and filtered through a 0.22 µm filter. The presence of free intact K5 peptide was assayed by the HPLC/MS. The results are presented below. The HPLC parameters for detection of K5 peptide in serum were as follows.

The HPLC method was as follows: A Vydac C18 250 X 4.6 mm, 5 µ particle size column was utilized. The column temperature was 30°C with a flow rate of 0.5 ml/min. Mobile Phase A was 0.1 % TFA/water. Mobile Phase B was 0.1% TFA/acetonitrite. The injection volume was 10µl.

The gradient was as follows:

| Time(Minutes) | %A | %B |
|---|---|---|
| 0 | 95 | 5 |
| 20 | 70 | 30 |
| 25 | 10 | 90 |
| 30 | 10 | 90 |
| 35 | 95 | 5 |
| 45 | 95 | 5 |

The proteins were detected at 214, 254 and 334 nm. For mass spectral analysis, the ionization mode was API-electrospray (positive mode) at an M/Z range of 300 to 2000. The gain was 3.0, fragmentor 120v, threshold 20, stepsize 0.1. The gas temp was 350°C and the drying gas volume was 10.0 l/min. The Neb pressure was 24 psi and the Vcap was 3500V. The HPLC method was as follows: A Vydac C18 250 X 4.6 mm, 5 µ particle size column was utilized. The column temperature was 30°C with a flow rate of 0.5 ml/min. Mobile Phase A was 0.1% TFA/water. Mobile Phase B was 0.1% TFA/acetonitrite. The injection volume was 10µl.

The gradient was as follows:

| Time(Minutes) | %A | %B |
|---|---|---|
| 0 | 95 | 5 |
| 20 | 70 | 30 |
| 25 | 10 | 90 |
| 30 | 10 | 90 |
| 35 | 95 | 5 |
| 45 | 95 | 5 |

The proteins were detected at 214, 254 and 334 nm. For mass spectral analysis, the ionization mode was API-electrospray (positive mode) at an M/Z range of 300 to 2000. The gain was 3.0, fragmentor 120v, threshold 20, stepsize 0.1. The gas temp was 350°C and the drying gas volume was 10.0 l/min. The Neb pressure was 24 psi and the Vcap was 3500V.

| Time | %K5 peptide in Plasma |
|---|---|
| 0 hrs. | 100% |
| 4 hrs | 9% |
| 24 hrs | 0% |

The results demonstrate that unmodified K5 peptide is unstable in plasma likely as a result of protease activity.

### (2) Stability of MPA-K5-HSA Conjugate in Plasma

MPA-K5 (modified K5 peptide) was incubated with 25% HSA for 2 hours at room temperature. The MPA-K5-HSA conjugate was then incubated at 37° in the presence of human plasma at a final concentration of 160 µm. After the specific incubation period (0, 4 and 24 hours) an aliquot of 100 µl was withdrawn and filtered through a 0.22 µm filter. The presence of intact conjugate was assayed by HPLC-MS.

The column was an Aquapore RP-300, 250 x 4.6 mm, 7µ particle size. The column temperature was 50° C. The mobile phase A was 0.1 % TFA/water. The mobile phase B was 0.1 % TFA/acetonitrile. The injection volume was 1 µl. The gradient was as follows:

| Time (minutes) | %A | %B | Flow(ml/min) |
|---|---|---|---|
| 0 | 66 | 34 | 0.700 |
| 1 | 66 | 34 | 0.700 |
| 25 | 58.8 | 41.2 | 0.700 |
| 30 | 50 | 50 | 0.70 |
| 35 | 5 | 95 | 1.00 |
| 41 | 5 | 95 | 1.00 |
| 45 | 66 | 34 | 1.00 |
| 46 | 66 | 34 | 0.70 |

The peptide was detected at 214 mm for quantification. For mass spectral analysis of the peptide, the ionization mode was API-electrospray at 1280 to 1500 m/z range, gain 1.0, fragmentor 125V, threshold 100, stepsize 0.40. The gas temperature was 350°C the drying gas was 13.0 l/min. The pressure was 60psi and the Vcap was 6000V. The results are presented below.

Approximately 33% of circulating albumin in the bloodstream is mercaptalbumin (SH-albumin) which is not blocked by endogenous sulfhydryl compounds such as cysteine or glutathione and is therefore available for reaction with maleimido groups. The remaining 66% of the circulating albumin is capped or blocked by sulfhydryl compounds. The HPLC MS assay permits the identification of capped-HSA, SH-albumin and K5-MPA-albumin. The MPA covalently bonds to the free thiol on the albumin. The stability of the three forms of albumin in plasma is presented below.

| Time | %capped HSA | % SH-Albumin | %K5-MPA-HSA |
|---|---|---|---|
| 0 hrs. | 61.3 | 16.6 | 22.1 |
| 4 hrs. | 64.6 | 16.05 | 19.35 |
| 24 hrs. | 63 | 16.8 | 20.2 |

The percentage of K5-MPA-HSA remained relatively constant throughout the 24 hour plasma assay in contrast to unmodified K5 which decreased to 9% of the original amount of K5 in only 4 hours in plasma. The results demonstrate that in contrast to K5 which is quite unstable in plasma, K5-MPA-HSA is quite stable from peptidase activity in plasma.

### Example 70 - Peptide Stability Assay of Dynorphin

In order to determine the stability of peptide conjugates in the presence of serum peptidases the serum stability of Dyn A-(1-13)-OH, Dyn A-(1-13)-NH₂ and Dyn A 1-13(MPA)-NH₂ were compared. Dyn A-(1-13)-OH, Dyn A-(1-13)-NH₂ and Dyn A 1-13(MPA)-NH₂ were synthesized as described above. The Dynorphin peptides were mixed with human heparinized plasma to a final concentration of 4 mg/mL. After the required incubation time at 37°C, 0, 20, 20, 60, 120, 180, 360 and 480 minutes) a 100 µL-aliquot was added to 100 µL of blocking solution (5 vol. of a 5% aqueous ZnSO₄ solution, 3 vol. of acetonitrile, 2 vol. of methanol) that precipitates all proteins. After centrifugation (10,000 g for 2.5 min), clear supernatant was recovered, filtered through a 0.45 µm filter and stored on ice until LC/MS analysis.

The samples were analyzed using an LC at 214 nm to detect the presence of the different compounds and MS to determine the identity of the detected compound. The integrated area % for each peak from the LC chromatogram was then plotted against time and the relative stabilities determined in human plasma.

The stability data for Dyn A-(1-13)-OH and Dyn A-(1-13)-NH₂ were consistent with that reported in literature: the proteolytic breakdown of the dynorphin peptides is quite rapid. Dyn A-(1-13)-OH had a half life of about 10 minutes. Dyn A-(1-13)-NH₂ had a half life of about 30 minutes. In contrast Dyn A 1-13(MPA)-NH₂ exhibited striking stabilization in the presence of serum peptidase activity. Unmodified dynorphin peptides are degraded within 60 minutes. In contrast, modified dynorphin peptides (Dyn A 1-13(MPA)-NH₂) are stable from serum peptidase activity for up to 480 minutes.

The stability determination of the dynorphin conjugate is determined by ELISA. In order to determine if the observed signal is due to a dynorphin conjugate and what the conjugate is, LC mass spectrometrytral analysis of the reaction mixture after 8 h was performed. The use of mass spectrometry permits a determination of the molecular weight of the conjugate and allows the determination whether there are any truncated forms of the dynorphin conjugate. Mass spectrometry of human plasma shows the two forms of albumin, the free thiol at 66436 Da and the oxidized form at 66557 Da. Also, mass spectrometry can distinguish between a Dyn 2-13 truncated conjugate (68046 Da) and the intact Dyn 1-13 conjugate, (68207 Da) in an equal mixture.

Mass spectrometry analysis of dynorphin samples taken from the serum after 480 minutes of exposure to the serum peptidases identifies only the presence of the intact conjugate (68192 Da) and not the breakdown products thereby demonstrating the stability of the dynorphin conjugate from serum peptidase activity.

| **TABLE 1** | | | |
|---|---|---|---|
| **NATURAL AMINO ACIDS AND THEIR ABBREVIATIONS** | | | |
| Name | 3-Letter Abbreviation | 1-Letter Abbreviation | Protected Amino Acids |
| Alanine | Ala | A | Fmoc-Ala-OH |
| Arginine | Arg | R | Fmoc-Arg(Pbt)-OH |
| Asparagine | Asn | N | Fmoc-Asn(Trt)-OH |
| Aspartic acid | Asp | D | Asp(tBu)-OH |
| Cysteine | Cys | C | Fmoc-Cys(Trt) |
| Glutamic acid | Glu | E | Fmoc-Glu(tBu)-OH |
| Glutamine | Gln | Q | Fmoc-Gln(Trt)-OH |
| Glycine | Gly | G | Fmoc-Gly-OH |
| Histidine | His | H | Fmoc-His(Trt)-OH |
| Isoleucine | Ile | I | Fmoc-Ile-OH |
| Leucine | Leu | L | Fmoc-Leu-OH |
| Lysine | Lys | K | Fmoc-Lys(Mtt)-OH |
| Methionine | Met | M | Fmoc-Met-OH |
| Phenylalanine | Phe | F | Fmoc-Phe-OH |
| Proline | Pro | P | Fmoc-Pro-OH |
| Serine | Ser | S | Fmoc-Ser(tBu)-OH |
| Threonine | Thr | T | Fmoc-Thr(tBu)-OH |
| Tryptophan | Trp | W | Fmoc-Trp(Boc)-OH |
| Tyrosine | Tyr | Y | Boe-Tyr(tBu)-OH |
| Valine | Val | V | Fmoc-Val-OH |

## Claims

1. A method of synthesizing a modified therapeutic peptide capable of forming a peptidase-stabilized therapeutic peptide conjugate, the peptide comprising between 3 and 50 amino acids, said peptide having a carboxy terminal amino acid, an amino terminal amino acid, a therapeutically active region and a less therapeutically active region, the therapeutically active region being identified by structure activity relationship analysis, the method comprising the steps of:
a) choosing a position within the less therapeutically active region, and
b)
1) if said therapeutic peptide does not contain a cysteine, then synthesizing said peptide from said carboxy terminal amino acid and coupling a reactive group to an amino acid at said position, in the less therapeutically active region; or
2) if said therapeutic peptide contains only one cysteine, then synthesizing said peptide from said carboxy terminal amino acid; protecting said cysteine with a protective group; coupling a reactive group to an amino acid at said position in the less therapeutically active region, and deprotecting the protected cysteine; or
3) if said therapeutic peptide contains two cysteines, then synthesizing said peptide from said carboxy terminal amino acid; oxidizing said two cysteines to form a disulfide bridge; and coupling a reactive group to an amino acid at said position in the less therapeutically active region; or
4) if said therapeutic peptide contains more than two cysteines as disulfide bridges, then synthesizing said peptide from said carboxy terminal amino acid; sequentially oxidizing said cysteines to form disulfide bridge; purifying said peptide; and coupling a reactive group to an amino acid at said position in the less therapeutically active region;
c) forming a stable covalent bond between the reactive group and an amino group, hydroxyl group, or thiol group on albumin in *vivo* or *ex vivo* and providing a peptide-albumin conjugate, thereby generating the peptidase-stabilized therapeutic peptide, while retaining therapeutic activity of the therapeutic peptide; the peptide-albumin conjugate having a higher stability towards peptidase degradation than the therapeutic peptide; and
d) analyzing the therapeutic activity of the peptide-albumin conjugate and the stability of the peptide-albumin conjugate towards peptidase degradation; confirming that the pepode-albumin conjugate has a higher stability than the therapeutic peptide; confirming that the peptide-albumin conjugate retains the therapeutic activity of the therapeutic peptide, and confirming the chosen position within said therapeutically less active region

2. A method as claimed in claim 1 wherein the reactive goup is coupled to the therapeutic peptide via a lysine and/or a linking group.

3. A method as claimed in claim 3 wherein the reactive group comprises a maleimide.

4. An *ex vivo* method for protecting from peptidase degradation a therapeutic peptide, said peptide comprising between 3 and 50 amino acids and having a therapeutically active region of amino acids and a less therapeutically active region of amino acids, comprising:
(a) identifying the therapeutically active region of the peptide by structure activity relationship analysis;
(b) modifying said peptide at an amino acid included in the less therapeutically active region by coupling thereto a reactive group to said amino acid to form a modified peptide, such that said modified peptide has therapeutic activity, the reactive group being capable of forming a covalent bond with a reactive functionality on albumin;
(c) forming a covalent bond between said reactive entity and a reactive functionality on albumin to form a peptide-albumin conjugate, thereby protecting said peptide from peptidase activity, while retaining therapeutic activity of the therapeutic peptide; and
(d) analyzing the therapeutic activity of the peptide-albumin conjugate and the stability of the peptide-albumin conjugate towards peptidase degradation; confirming that the peptide-albumin conjugate bas a higher stability than the therapeutic peptide and confirming that the peptide-albumin conjugate retains the therapeutic activity of the therapeutic peptide.

5. A method according to claim 4, wherein said peptide has a carboxy terminus, an amino terminus, a carboxy terminal amino acid and an amino terminal amino acid, and wherein step (b) further comprises:
(1) if said less therapeutically active region is located at the carboxy terminus of said peptide, then modifying said peptide at the carboxy terminal amino acid of said peptide; or
(2) if said less therapeutically active region is located at the amino terminus of said peptide, then modifying said peptide at the amino terminal amino acid of said peptide; or
(3) if said less therapeutically active region is located at neither the amino terminus nor the carboxy terminus of said peptide, then modifying said peptide at an amino acid located between the carboxy terminus and the amino terminus.

6. A method according to claim 4, wherein said reactive group is a maleimide group.

7. A method according to claim 4, wherein said reactive group is coupled to said peptide via a lysine and/or linking group.

8. A method according to claim 4, wherein one or more of said amino acids is synthetic.

## Patentansprüche

1. Verfahren zur Synthese eines modifizierten therapeutischen Peptids, geeignet zur Bildung eines Peptidase-stabilisierten therapeutischen Peptidkonjugats, wobei das Peptid zwischen 3 und 50 Aminosäuren umfaßt und besagtes Peptid eine Carboxyterminale Aminosäure, eine Amino-terminale Aminosäure, eine therapeutisch aktive Region und eine weniger therapeutisch aktive Region hat, wobei die therapeutisch aktive Region durch Struktur-Aktivitäts-Verhältnis-Analyse identifiziert wird, umfassend die Schritte:
a) Auswählen einer Position innerhalb der weniger therapeutisch aktiven Region, und
b) 1) wenn besagtes therapeutisches Peptid kein Cystein enthält, Synthetisieren von besagtem Peptid von besagter Carboxy-terminaler Aminosäure und Koppeln einer reaktiven Gruppe an die Aminosäure an besagter Position in der weniger therapeutisch aktiven Region; oder
2) wenn besagtes therapeutisches Peptid nur ein Cystein enthält, Synthetisieren von besagtem Peptid von besagter Carboxy-terminaler Aminosäure, Schützen von besagtem Cystein mit einer Schutzgruppe, Koppeln einer reaktiven Gruppe an eine Aminosäure an besagter Position in der weniger therapeutisch aktiven Region und Entschützen des geschützten Cysteins; oder
3) wenn besagtes therapeutisches Peptid zwei Cysteine enthält, Synthetisieren von besagtem Peptid von besagter Carboxy-terminaler Aminosäure, Oxidieren von besagten zwei Cysteinen, um eine Disulfidbrücke zu bilden, und Koppeln einer reaktiven Gruppe an eine Aminosäure an besagter Position in der weniger therapeutisch aktiven Region; oder
4) wenn besagtes therapeutisches Peptid mehr als zwei Cysteine als Disulfidbrücken enthält, Synthetisieren von besagtem Peptid von besagter Carboxy-terminaler Aminosäure, sequentielles Oxidieren besagter Cysteine, um Disulfidbrücken zu bilden, Reinigen von besagtem Peptid und Koppeln einer reaktiven Gruppe an eine Aminosäure an besagter Position in der weniger therapeutisch aktiven Region;
c) Bilden einer stabilen kovalenten Bindung zwischen der reaktiven Gruppe und einer Aminogruppe, Hydroxylgruppe oder Thiolgruppe auf Albumin in vivo oder ex vivo und Zurverfügungstellen eines Peptid-Albumin-Konjugats, wodurch das Peptidasestabilisierte therapeutische Peptid generiert wird, während die therapeutische Aktivität des therapeutischen Peptids erhalten bleibt, wobei das Peptid-Albumin-Konjugat eine höhere Stabilität gegenüber Peptidaseabbau hat als das therapeutische Peptid; und
d) Analysieren der therapeutischen Aktivität des Peptid-Albumin-Konjugats und der Stabilität des Peptid-Albumin-Konjugats gegenüber Peptidaseabbau; Bestätigen, daß das Peptid-Albumin-Konjugat eine höhere Stabilität als das therapeutische Peptid hat; Bestätigen, daß das Peptid-Albumin-Konjugat die therapeutische Aktivität des therapeutischen Peptids beibehält und Bestätigen der ausgewählten Position innerhalb besagter weniger therapeutisch aktiver Region.

2. Verfahren gemäß Anspruch 1, worin die reaktive Gruppe an das therapeutische Peptid über ein Lysin und/oder eine Verbindungsgruppe gekoppelt wird.

3. Verfahren gemäß Anspruch 3, worin die reaktive Gruppe ein Maleimid umfaßt.

4. Ex-vivo-Verfahren zum Schützen eines therapeutischen Peptids vor Peptidaseabbau, wobei besagtes Peptid zwischen 3 und 50 Aminosäuren umfaßt und eine therapeutisch aktive Region von Aminosäuren und eine weniger therapeutisch aktive Region von Aminosäuren hat, umfassend:
(a) Identifizieren der therapeutisch aktiven Region des Peptids durch Struktur-Aktivitäts-Verhältnis-Analyse;
(b) Modifizieren von besagtem Peptid an einer Aminosäure, die in der weniger therapeutisch aktiven Region enthalten ist, durch Koppeln einer reaktiven Gruppe an besagte Aminosäure, um ein modifiziertes Peptid zu bilden, so daß besagtes modifiziertes Peptid therapeutische Aktivität hat, wobei die reaktive Gruppe in der Lage ist, eine kovalente Bindung mit einer reaktiven Funktionalität auf Albumin zu bilden;
(c) Bilden einer kovalenten Bindung zwischen besagter reaktiver Einheit und einer reaktiven Funktionalität auf Albumin, um ein Peptid-Albumin-Konjugat zu bilden, wodurch besagtes Peptid von Peptidaseaktivität geschützt wird, während die therapeutische Aktivität des therapeutischen Peptids erhalten bleibt; und
(d) Analysieren der therapeutischen Aktivität des Peptid-Albumin-Konjugats und der Stabilität des Peptid-Albumin-Konjugats gegenüber Peptidaseabbau; Bestätigen, daß das Peptid-Albumin-Konjugat eine höhere Stabilität hat als das therapeutische Peptid und Bestätigen, daß das Peptid-Albumin-Konjugat die therapeutische Aktivität des therapeutischen Peptids beibehält.

5. Verfahren gemäß Anspruch 4, worin besagtes Peptid einen Carboxyterminus, einen Aminoterminus, eine Carboxyterminale Aminosäure und eine Amino-terminale Aminosäure hat und worin Schritt (b) des weiteren umfaßt:
(1) wenn besagte weniger therapeutisch aktive Region an dem Carboxyterminus besagten Peptids lokalisiert ist, Modifizieren von besagtem Peptid an der Carboxy-terminalen Aminosäure von besagtem Peptid; oder
(2) wenn besagte weniger therapeutisch aktive Region an dem Aminoterminus des besagten Peptids lokalisiert ist, Modifizieren von besagtem Peptid an der Aminoterminalen Aminosäure von besagtem Peptid; oder
(3) wenn besagte weniger therapeutisch aktive Region weder am Amino- noch am Carboxyterminus von besagtem Peptid lokalisiert ist, Modifizieren von besagtem Peptid an einer Aminosäure, welche zwischen dem Carboxyterminus und dem Aminoterminus liegt.

6. Verfahren gemäß Anspruch 4, worin besagte reaktive Gruppe eine Maleimidgruppe ist.

7. Verfahren gemäß Anspruch 4, worin besagte reaktive Gruppe an besagtes Peptid über ein Lysin und/oder eine Verbindungsgruppe gekoppelt ist.

8. Verfahren gemäß Anspruch 4, worin eine oder mehrere besagte Aminosäuren synthetisch ist/sind.

## Revendications

1. Procédé de synthèse d'un peptide thérapeutique modifié capable de former un conjugué de peptide thérapeutique stabilisé vis-à-vis des peptidases, le peptide comprenant entre 3 et 50 acides aminés, ledit peptide ayant un acide aminé carboxy-terminal, un acide aminé amino-terminal, une région thérapeutiquement active et une région thérapeutiquement moins active, la région thérapeutiquement active étant identifiée par analyse de la relation structure-activité, le procédé comprenant les étapes consistant à :
a) choisir une position dans la région thérapeutiquement moins active, et
b)
1) si ledit peptide thérapeutique ne contient pas de cystéine, synthétiser alors ledit peptide à partir dudit acide aminé carboxy-terminal et coupler un groupe réactif à un acide aminé au niveau de ladite position dans la région thérapeutiquement moins active ; ou
2) si ledit peptide thérapeutique ne contient qu'une cystéine, synthétiser alors ledit peptide à partir dudit acide aminé carboxy-terminal ; protéger ladite cystéine avec un groupe protecteur ; coupler un groupe réactif à un acide aminé au niveau de ladite position dans la région thérapeutiquement moins active ; et déprotéger la cystéine protégée ; ou
3) si ledit peptide thérapeutique contient deux cystéines, synthétiser alors ledit peptide à partir dudit acide aminé carboxy-terminal ; oxyder lesdites deux cystéines pour former un pont disulfure ; et coupler un groupe réactif à un acide aminé au niveau de ladite position dans la région thérapeutiquement moins active ; ou
4) si ledit peptide thérapeutique contient plus de deux cystéines sous forme de ponts disulfure, synthétiser alors ledit peptide à partir dudit acide aminé carboxy-terminal ; oxyder successivement lesdites cystéines pour former un pont disulfure ; purifier ledit peptide ; et coupler un groupe réactif à un acide aminé au niveau de ladite position dans la région thérapeutiquement moins active ;
c) former une liaison covalente stable entre le groupe réactif et un groupe amino, un groupe hydroxyle, ou un groupe thiol sur l'albumine *in vivo* ou *ex vivo* et obtenir un conjugué peptide-albumine pour ainsi produire le peptide thérapeutique stabilisé vis-à-vis des peptidases tout en conservant l'activité thérapeutique du peptide thérapeutique ; le conjugué peptide-albumine ayant une plus grande stabilité vis-à-vis de la dégradation par les peptidases que le peptide thérapeutique ; et
d) analyser l'activité thérapeutique du conjugué peptide-albumine et la stabilité du conjugué peptide-albumine vis-à-vis de la dégradation par les peptidases ; confirmer que le conjugué peptide-albumine a une plus grande stabilité que le peptide thérapeutique ; confirmer que le conjugué peptide-albumine conserve l'activité thérapeutique du peptide thérapeutique, et confirmer la position choisie dans ladite région thérapeutiquement moins active.

2. Procédé selon la revendication 1, dans lequel le groupe réactif est couplé au peptide thérapeutique par l'intermédiaire d'une lysine et/ou d'un groupe de liaison.

3. Procédé selon la revendication 3, dans lequel le groupe réactif comprend un maléimide.

4. Procédé *ex vivo* de protection contre la dégradation par les peptidases d'un peptide thérapeutique, ledit peptide comprenant entre 3 et 50 acides aminés et ayant une région thérapeutiquement active d'acides aminés et une région thérapeutiquement moins active d'acides aminés, comprenant les étapes consistant à :
(a) identifier la région thérapeutiquement active du peptide par analyse de la relation structure-activité ;
(b) modifier ledit peptide au niveau d'un acide aminé inclus dans la région thérapeutiquement moins active en couplant un groupe réactif audit acide aminé pour former un peptide modifié, de façon à ce que ledit peptide modifié ait une activité thérapeutique, le groupe réactif étant capable de former une liaison covalente avec un groupe fonctionnel réactif sur l'albumine ;
(c) former une liaison covalente entre ladite entité réactive et un groupe fonctionnel réactif sur l'albumine pour former un conjugué peptide-albumine et ainsi protéger ledit peptide de l'activité des peptidases tout en conservant l'activité thérapeutique du peptide thérapeutique ; et
(d) analyser l'activité thérapeutique du conjugué peptide-albumine et la stabilité du conjugué peptide-albumine vis-à-vis de la dégradation par les peptidases ; confirmer que le conjugué peptide-albumine a une plus grande stabilité que le peptide thérapeutique et confirmer que le conjugué peptide-albumine conserve l'activité thérapeutique du peptide thérapeutique.

5. Procédé selon la revendication 4, dans lequel ledit peptide a une extrémité carboxy, une extrémité amino, un acide aminé carboxy-terminal et un acide aminé amino-terminal, et dans lequel l'étape (b) comprend en outre les étapes consistant à :
(1) si ladite région thérapeutiquement moins active est située au niveau de l'extrémité carboxy dudit peptide, modifier alors ledit peptide au niveau de l'acide aminé carboxy-terminal dudit peptide ; ou
(2) si ladite région thérapeutiquement moins active est située au niveau de l'extrémité amino dudit peptide, modifier alors ledit peptide au niveau de l'acide aminé amino-terminal dudit peptide ; ou
(3) si ladite région thérapeutiquement moins active n'est située ni au niveau de l'extrémité amino ni au niveau de l'extrémité carboxy dudit peptide, modifier alors ledit peptide au niveau d'un acide aminé situé entre l'extrémité carboxy et l'extrémité amino.

6. Procédé selon la revendication 4, dans lequel ledit groupe réactif est un groupe maléimide.

7. Procédé selon la revendication 4, dans lequel ledit groupe réactif est couplé audit peptide par l'intermédiaire d'une lysine et/ou d'un groupe de liaison.

8. Procédé selon la revendication 4, dans lequel un ou plusieurs desdits acides aminés est/sont synthétique(s).


### SEQUENCE LISTING

<110> Conjuchem, Inc.
   Bridon, Dominique
   Ezrin, Alan
   Milner, Peter
   Holmes, Darren
   Thibaudeau, Karen
<120> PROTECTION OF ENDOGENOUS THERAPEUTIC PEPTIDES FROM PEPTIDASE ACTIVITY THROUGH CONJUGATION TO BLOOD COMPONENTS
<130> 2110
<140>
   <141>
<150> 60/134,406
   <151> 1999-05-17
<150> 60/153,406
   <151> 1999-09-10
<150> 60/159,783
   <151> 1999-10-18
<160> 1617
<170> PatentIn Ver. 2.1
<210> 1
   <211> 19
   <212> PRT
   <213> Artificial Sequence
<220>
   <223> Description of Artificial Sequence: Synthetic Peptide
<400> 1
   His Phe Arg Trp Gly Lys Pro Val Gly Lys Lys Arg Arg Pro Val Lys
   1 5 10 15
   Val Tyr Pro
<210> 2
   <211> 8
   <212> PRT
   <213> Artificial Sequence
<220>
   <223> Description of Artificial Sequence: Synthetic Peptide
<400> 2
   Met Glu His Phe Arg Trp Gly Lys
   1 5
<210> 3
   <211> 39
   <212> PRT
   <213> Artificial Sequence
<220>
   <223> Description of Artificial Sequence: Synthetic Peptide
<400> 3
   Ser Tyr Ser Met Glu His Phe Arg Trp Gly Lys Pro Val Gly Lys Lys
   1 5 10 15
   Arg Arg Pro Val Lys Val Tyr Pro Asn Gly Ala Glu Asp Glu Ser Ala
   20 25 30
   Glu Ala Phe Pro Leu Glu Phe
   35
<210> 4
   <211> 13
   <212> PRT
   <213> Artificial Sequence
<220>
   <223> Description of Artificial Sequence: Synthetic Peptide
<400> 4
   Ser Tyr Ser Met Glu His Phe Arg Trp Gly Lys Pro Val
   1 5 10
<210> 5
   <211> 16
   <212> PRT
   <213> Artificial Sequence
<220>
   <223> Description of Artificial Sequence: Synthetic Peptide
<400> 5
   Ser Tyr Ser Met Glu His Phe Arg Trp Gly Lys Pro Val Gly Lys Lys
   1 5 10 15
<210> 6
   <211> 17
   <212> PRT
   <213> Artificial Sequence
<220>
   <223> Description of Artificial Sequence: Synthetic Peptide
<400> 6
   Ser Tyr Ser Met Glu His Phe Arg Trp Gly Lys Pro Val Gly Lys Lys
   1 5 10 15
   Arg
<210> 7
   <211> 24
   <212> PRT
   <213> Artificial Sequence
<220>
   <223> Description of Artificial Sequence: Synthetic Peptide
<400> 7
   Ser Tyr Ser Met Glu His Phe Arg Trp Gly Lys Pro Val Gly Lys Lys
   1 5 10 15
   Arg Arg Pro Val Lys Val Tyr Pro
   20
<210> 8
   <211> 7
   <212> PRT
   <213> Artificial Sequence
<220>
   <223> Description of Artificial Sequence: Synthetic Peptide
<400> 8
   Met Glu His Phe Arg Trp Gly
   1 5
<210> 9
   <211> 32
   <212> PRT
   <213> Artificial Sequence
<220>
   <223> Description of Artificial Sequence: Synthetic Peptide
<400> 9
   Phe Arg Trp Gly Lys Pro Val Gly Lys Lys Arg Arg Pro Val Lys Val
   1 5 10 15
   Tyr Pro Asn Gly Ala Glu Asp Glu Ser Ala Glu Ala Phe Pro Leu Glu
   20 25 30
<210> 10
   <211> 22
   <212> PRT
   <213> Artificial Sequence
<220>
   <223> Description of Artificial Sequence: Synthetic Peptide
<400> 10
   Arg Pro Val Lys Val Tyr Pro Asn Gly Ala Glu Asp Glu Ser Ala Glu
   1 5 10 15
   Ala Phe Pro Leu Glu Phe
   20
<210> 11
   <211> 39
   <212> PRT
   <213> Artificial Sequence
<220>
   <223> Description of Artificial Sequence: Synthetic Peptide
<400> 11
   Ser Tyr Ser Met Glu His Phe Arg Trp Gly Lys Pro Val Gly Lys Lys
   1 5 10 15
   Arg Arg Pro Val Lys Val Tyr Pro Asn Val Ala Glu Asn Glu Ser Ala
   20 25 30
   Glu Ala Phe Pro Leu Glu Phe
   35
<210> 12
   <211> 28
   <212> PRT
   <213> Artificial Sequence
<220>
   <223> Description of Artificial Sequence: Synthetic Peptide
<400> 12
   Pro Val Gly Lys Lys Arg Arg Pro Val Lys Val Tyr Pro Asn Val Ala
   1 5 10 15
   Glu Asn Glu Ser Ala Glu Ala Phe Pro Leu Glu Phe
   20 25
<210> 13
   <211> 18
   <212> PRT
   <213> Artificial Sequence
<220>
   <223> Description of Artificial Sequence: synthetic Peptide
<400> 13
   Val Tyr Pro Asn Gly Ala Glu Asp Glu Ser Ala Glu Ala Phe Pro Leu
   1 5 10 15
   Glu Phe
<210> 14
   <211> 39
   <212> PRT
   <213> Artificial Sequence
<220>
   <223> Description of Artificial Sequence: Synthetic Peptide
<400> 14
   Ser Tyr Ser Met Glu His Phe Arg Trp Gly Lys Pro Val Gly Lys Lys
   1 5 10 15
   Arg Arg Pro Val Lys Val Tyr Pro Asn Val Ala Glu Asn Glu Ser Ala
   20 25 30
   Glu Ala Phe Pro Leu Glu Phe
   35
<210> 15
   <211> 28
   <212> PRT
   <213> Artificial Sequence
<220>
   <223> Description of Artificial Sequence: synthetic. Peptide
<400> 15
   Pro Val Gly Lys Lys Arg Arg Pro Val Lys Val Tyr Pro Asn Val Ala
   1 5 10 15
   Glu Asn Glu Ser Ala Glu Ala Phe Pro Leu Glu Phe
   20 25
<210> 16
   <211> 18
<212> PRT
   <213> Artificial sequence
<220>
   <223> Description of Artificial Sequence: synthetic Peptide
<400> 16
   Val Tyr Pro Asn Gly Ala Glu Asp Glu Ser Ala Glu Ala Phe Pro Leu
   1 5 10 15
   Glu Phe
<210> 17
   <211> 33
   <212> PRT
   <213> Artificial Sequence
<220>
   <223> Description of Artificial Sequence: Synthetic Peptide
<400> 17
   Val Cys Ser Cys Arg Leu Val Phe Cys Arg Arg Thr Glu Leu Arg Val
   1 5 10 15
   Gly Asn Cys Leu Ile Gly Gly Val Ser Phe Thr Tyr Cys Cys Thr Arg
   20 25 30
   Val
<210> 18
   <211> 34
   <212> PRT
   <213> Artificial Sequence
<220>
   <223> Description of Artificial Sequence: Synthetic Peptide
<400> 18
   Gly Ile Cys Ala Cys Arg Arg Arg Phe Cys Pro Asn Ser Glu Arg Phe
   1 5 10 15
   Ser Gly Tyr Cys Arg Val Asn Gly Ala Arg Tyr Val Arg Cys Cys Ser
   20 25 30
   Arg Arg
<210> 19
   <211> 5
   <212> PRT
   <213> Artificial Sequence
<220>
   <223> Description of Artificial Sequence: Synthetic Peptide
<400> 19
   Met Glu His Phe Phe
   1 5
<210> 20
   <211> 5
   <212> PRT
   <213> Artificial Sequence
<220>
   <223> Description of Artificial Sequence: Synthetic Peptide
<400> 20
   Met Glu His Phe Phe
   1 5
<210> 21
   <211> 17
   <212> PRT
   <213> Artificial Sequence
<220>
   <223> Description of Artificial Sequence: Synthetic Peptide
<400> 21
   Ser Tyr Ser Met Glu His Phe Arg Trp Gly Lys Pro Val Gly Lys Lys
   1 5 10 15
   Arg
<210> 22
   <211> 30
   <212> PRT
   <213> Artificial Sequence
<220>
   <223> Description of Artificial Sequence: Synthetic Peptide
<400> 22
   Arg Lys Tyr Val Met Gly His Phe Arg Trp Asp Arg Phe Gly Arg Arg
   1 5 10 15
   Asn Ser Ser Ser Ser Gly Ser Ser Gly Ala Gly Gln Lys Arg
   20 25 30
<210> 23
   <211> 43
   <212> PRT
   <213> Artificial Sequence
<220>
   <223> Description of Artificial Sequence: Synthetic Peptide
<400> 23
   Phe Pro Thr Ile Pro Leu Ser Arg Leu Phe Asp Asn Ala Met Leu Arg
   1 5 10 15
   Ala His Arg Leu His Gln Leu Ala Phe Asp Thr Tyr Gln Glu Phe Glu
   20 25 30
   Glu Ala Tyr Ile Pro Lys Glu Gln Lys Tyr Ser
   35 40
<210> 24
   <211> 6
   <212> PRT
   <213> Artificial Sequence
<220>
   <223> Description of Artificial Sequence: Synthetic Peptide
<210> 25
   <211> 9
   <212> PRT
   <213> Artificial Sequence
<220>
   <223> Description of Artificial Sequence: Synthetic Peptide
<400> 25
   Cys Glu His Arg Trp Cys Lys Pro Val
   1 5
<210> 26
   <211> 13
   <212> PRT
   <213> Artificial Sequence
<220>
   <223> Description of Artificial Sequence: Synthetic Peptide
<400> 26
   Ser Tyr Ser Met Glu His Phe Arg Trp Gly Lys Pro Val
   1 5 10
<210> 27
   <211> 13
   <212> PRT
   <213> Artificial Sequence
<220>
   <223> Description of Artificial Sequence: synthetic Peptide
<400> 27
   Ser Tyr Ser Met Glu His Phe Arg Trp Gly Lys Pro Val
   1 5 10
<210> 28
   <211> 18
   <212> PRT
   <213> Artificial Sequence
<220>
   <223> Description of Artificial Sequence: Synthetic Peptide
<400> 28
   Asp Glu Gly Pro Tyr Lys Met Glu His Phe Arg Trp Gly Ser Pro Pro
   1 5 10 15
   Lys Asp
<210> 29
   <211> 13
   <212> PRT
   <213> Artificial Sequence
<220>
   <223> Description of Artificial Sequence: Synthetic Peptide
<400> 29
   Ser Tyr Ser Met Glu His Phe Arg Trp Gly Lys Pro Val
   1 5 10
<210> 30
   <211> 12
   <212> PRT
   <213> Artificial Sequence
<220>
   <223> Description of Artificial Sequence: Synthetic Peptide
<400> 30
   Ser Tyr Ser Met Glu His Arg Trp Gly Lys Pro Val
   1 5 10
<210> 31
   <211> 11
<212> PRT
   <213> Artificial Sequence
<220>
   <223> Description of Artificial Sequence: Synthetic Peptide
<400> 31
   Tyr Val Met Gly His Phe Arg Trp Asp Arg Phe
   1 5 10
<210> 32
   <211> 12
   <212> PRT
   <213> Artificial Sequence
<220>
   <223> Description of Artificial Sequence: Synthetic Peptide
<400> 32
   Lys Tyr Val Met Gly His Phe Arg Trp Asp Arg Phe
   1 5 10
<210> 33
   <211> 3
   <212> PRT
   <213> Artificial Sequence
<220>
   <223> Description of Artificial Sequence: Synthetic Peptide
<400> 33
   Pro Leu Gly
   1
<210> 34
   <211> 6
   <212> PRT
   <213> Artificial Sequence
<220>
   <223> Description of Artificial Sequence: Synthetic Peptide
<400> 34
   Asp His Phe Arg Trp Lys
   1 5
<210> 35
   <211> 12
   <212> PRT
   <213> Artificial Sequence
<220>
   <223> Description of Artificial Sequence: Synthetic Peptide
<400> 35
   Ser Tyr Ser Glu His Phe Arg Trp Gly Lys Pro Val
   1 5 10
<210> 36
   <211> 12
   <212> PRT
   <213> Artificial Sequence
<220>
   <223> Description of Artificial Sequence: Synthetic Peptide
<400> 36
   Ser Tyr Ser Glu His Phe Arg Trp Gly Lys Pro Val
   1 5 10
<210> 37
   <211> 6 .
   <212> PRT
   <213> Artificial Sequence
<220>
   <223> Description of Artificial Sequence: Synthetic Peptide
<400> 37
   Glu His Phe Arg Trp Gly
   1 5
<210> 38
   <211> 22
   <212> PRT
   <213> Artificial Sequence
<220>
   <223> Description of Artificial Sequence: Synthetic Peptide
<400> 38
   Ala Glu Lys Lys Asp Glu Gly Pro Tyr Arg Met Glu His Phe Arg Trp
   1 5 10 15
   Gly Ser Pro Pro Lys Asp
   20
<210> 39
   <211> 12
   <212> PRT
   <213> Artificial Sequence
<220>
   <223> Description of Artificial Sequence: Synthetic Peptide
<400> 39
   Tyr Val Met Gly His Phe Arg Trp Asp Arg Phe Gly
   1 5 10
<210> 40
   <211> 7
   <212> PRT
   <213> Artificial Sequence
<220>
   <223> Description of Artificial Sequence: Synthetic Peptide
<400> 40
   Tyr Ile Gln Asn Pro Leu Gly
   1 5
<210> 41
   <211> 9
   <212> PRT
<213> Artificial Sequence
<220>
   <223> Description of Artificial Sequence: Synthetic Peptide
<400> 41
   Cys Tyr Ile Gln Asn Cys Pro Leu Gly
   1 5
<210> 42
   <211> 7
   <212> PRT
   <213> Artificial Sequence
<220>
   <223> Description of Artificial Sequence: Synthetic Peptide
<400> 42
   Tyr Ile Thr Asn Cys Pro Tyr
   1 5
<210> 43
   <211> 6
   <212> PRT
   <213> Artificial Sequence
<220>
   <223> Description of Artificial Sequence: Synthetic Peptide
<400> 43
   Cys Tyr Ile Gln Asn Cys
   1 5
<210> 44
   <211> 9
   <212> PRT
   <213> Artificial Sequence
<220>
   <223> Description of Artificial Sequence: Synthetic Peptide
<400> 44
   Cys Tyr Ile Gln Asn Cys Pro Leu Gly
   1 5
<210> 45
   <211> 9
   <212> PRT
   <213> Artificial Sequence
<220>
   <223> Description of Artificial Sequence: Synthetic Peptide
<400> 45
   Met Asn Ile Lys Gly Ser Pro Trp Lys
   1 5
<210> 46
   <211> 7
   <212> PRT
   <213> Artificial Sequence
<220>
   <223> Description of Artificial Sequence: Synthetic Peptide
<400> 46
   Tyr Phe Gln Asn Pro Arg Gly
   1 5
<210> 47
   <211> 7
   <212> PRT
   <213> Artificial Sequence
<220>
   <223> Description of Artificial Sequence: Synthetic Peptide
<400> 47
   Tyr Ile Gln Asn Pro Arg Gly
   1 5
<210> 48
   <211> 9
   <212> PRT
   <213> Artificial Sequence
<220>
   <223> Description of Artificial Sequence: Synthetic Peptide
<400> 48
   Cys Tyr Phe Gln Asn Cys Pro Lys Gly
   1 5
<210> 49
   <211> 6
   <212> PRT
   <213> Artificial Sequence
<220>
   <223> Description of Artificial Sequence: Synthetic Peptide
<400> 49
   Cys Tyr Phe Gln Asn Cys
   1 5
<210> 50
   <211> 7
   <212> PRT
   <213> Artificial Sequence
<220>
   <223> Description of Artificial Sequence: Synthetic Peptide
<400> 50
   Tyr Phe Val Asn Cys Pro Gly
   1 5
<210> 51
   <211> 7
   <212> PRT
   <213> Artificial Sequence
<220>
   <223> Description of Artificial Sequence: Synthetic Peptide
<400> 51
   Tyr Phe Gln Asn Cys Pro Arg
   1 5
<210> 52
   <211> 9
   <212> PRT
   <213> Artificial Sequence
<220>
   <223> Description of Artificial Sequence: Synthetic Peptide
<400> 52
   Cys Tyr Phe Gln Asn Cys Pro Arg Gly
   1 5
<210> 53
   <211> 8
   <212> PRT
   <213> Artificial Sequence
<220>
   <223> Description of Artificial Sequence: Synthetic Peptide
<400> 53
   Cys Tyr Phe Gln Asn Cys Pro Arg
   1 5
<210> 54
   <211> 9
   <212> PRT
   <213> Artificial Sequence
<220>
   <223> Description of Artificial Sequence: Synthetic Peptide
<400> 54
   Cys Tyr Phe Gln Asn Cys Pro Arg Gly
   1 5
<210> 55
   <211> 8
   <212> PRT
   <213> Artificial Sequence
<220>
   <223> Description of Artificial Sequence: Synthetic Peptide
<400> 55
   Tyr Phe Gln Asn Cys Pro Arg Gly
   1 5
<210> 56
   <211> 8
   <212> PRT
   <213> Artificial Sequence
<220>
   <223> Description of Artificial Sequence: Synthetic' Peptide
<400> 56
   Tyr Phe Gln Asn Cys Pro Arg Gly
   1 5
<210> 57
   <211> 8
   <212> PRT
   <213> Artificial Sequence
<220>
   <223> Description of Artificial Sequence: Synthetic Peptide
<400> 57
   Ala Phe Gln Asn Cys Pro Arg Gly
   1 5
<210> 58
   <211> 7
   <212> PRT
   <213> Artificial Sequence
<220>
   <223> Description of Artificial Sequence: Synthetic Peptide
<400> 58
   Tyr Phe Val Asn Cys Pro Gly
   1 5
<210> 59
   <211> 8
   <212> PRT
   <213> Artificial Sequence
<220>
   <223> Description of Artificial Sequence: Synthetic Peptide
<400> 59
   Tyr Phe Gln Asn Cys Pro Arg Tyr
   1 5
<210> 60
   <211> 7
   <212> PRT
   <213> Artificial Sequence
<220>
   <223> Description of Artificial Sequence: Synthetic Peptide
<400> 60
   Ile Phe Ile Asn Cys Pro Arg
   1 5
<210> 61
   <211> 8
   <212> PRT
   <213> Artificial Sequence
<220>
   <223> Description of Artificial Sequence: Synthetic Peptide
<400> 61
   Tyr Phe Val Asn Cys Pro Arg Gly
   1 5
<210> 62
   <211> 8
   <212> PRT
   <213> Artificial Sequence
<220>
   <223> Description of Artificial Sequence: Synthetic Peptide
<400> 62
   Tyr Phe Gln Asn Cys Pro Arg Gly
   1 5
<210> 63
   <211> 6
   <212> PRT
   <213> Artificial Sequence
<220>
   <223> Description of Artificial Sequence: Synthetic Peptide
<400> 63
   Glu Asn Cys Cys Pro Arg
   1 5
<210> 64
   <211> 8
   <212> PRT
   <213> Artificial Sequence
<220>
   <223> Description of Artificial Sequence: Synthetic Peptide
<400> 64
   Phe Phe Ile Asn Cys Pro Arg Ala
   1 5
<210> 65
   <211> 7
   <212> PRT
<213> Artificial Sequence
<220>
   <223> Description of Artificial Sequence: Synthetic Peptide
<400> 65
   Tyr Phe Gln Asn Cys Pro Lys
   1 5
<210> 66
   <211> 8
   <212> PRT
   <213> Artificial Sequence
<220>
   <223> Description of Artificial Sequence: Synthetic Peptide
<400> 66
   Phe Phe Ile Asn Cys Pro Arg Ala
   1 5
<210> 67
   <211> 9
   <212> PRT
   <213> Artificial Sequence
<220>
   <223> Description of Artificial Sequence: Synthetic Peptide
<400> 67
   Cys Tyr Phe Gln Asn Cys Pro Lys Gly
   1 5
<210> 68
   <211> 7
   <212> PRT
   <213> Artificial Sequence
<220>
   <223> Description of Artificial Sequence: Synthetic Peptide
<400> 68
   Tyr Phe Val Asn Cys Pro Gly
   1 5
<210> 69
   <211> 8
   <212> PRT
   <213> Artificial Sequence
<220>
   <223> Description of Artificial Sequence: Synthetic Peptide
<400> 69
   Cys Phe Ile Gln Asn Cys Pro Gly
   1 5
<210> 70
   <211> 9
   <212> PRT
   <213> Artificial Sequence
<220>
   <223> Description of Artificial Sequence: Synthetic Peptide
<400> 70
   Cys Tyr Ile Gln Asn Cys Pro Arg Gly
   1 5
<210> 71
   <211> 7
   <212> PRT
   <213> Artificial Sequence
<220>
   <223> Description of Artificial Sequence: Synthetic Peptide
<400> 71
   Tyr Ile Gln Asn Cys Pro Gly
   1 5
<210> 72
   <211> 25
   <212> PRT
   <213> Artificial Sequence
<220>
   <223> Description of Artificial Sequence: Synthetic Peptide
<400> 72
   Ser Glu Ala Ala Ala Leu Pro Arg Ala Ser Ala Ala Ala Met Arg Ala
   1 5 10 15
   Ala Trp Pro Ser Pro Ser Val Glu Gly
   20 25
<210> 73
   <211> 28
   <212> PRT
   <213> Artificial Sequence
<220>
   <223> Description of Artificial Sequence: Synthetic Peptide
<400> 73
   Phe His Leu Leu Arg Glu Val Leu Glu Ala Arg Ala Glu Gln Leu Ala
   1 5 10 15
   Gln Glu Ala His Lys Asn Arg Lys Leu Glu Ile Ile
   20 25
<210> 74
   <211> 30
   <212> PRT
   <213> Artificial Sequence
<220>
   <223> Description of Artificial Sequence: Synthetic Peptide
<400> 74
   Phe His Leu Leu Arg Glu Met Leu Glu Met Ala Lys Ala Glu Gln Glu
   1 5 10 15
   Ala Glu Gln Ala Ala Leu Asn Arg Leu Leu Leu Glu Glu Ala
   20 25 30
<210> 75
   <211> 41
   <212> PRT
   <213> Artificial Sequence
<220>
   <223> Description of Artificial Sequence: Synthetic Peptide
<400> 75
   Ser Gln Glu Pro Pro Ile Ser Leu Asp Leu Thr Phe His Leu Leu Arg
   1 5 10 15
   Glu Val Leu Glu Met Thr Lys Ala Asp Gln Leu Ala Gln Gln Ala His
   20 25 30
   Asn Asn Arg Lys Leu Leu Asp Ile Ala
   35 40
<210> 76
   <211> 41
   <212> PRT
   <213> Artificial Sequence
<220>
   <223> Description of Artificial Sequence: Synthetic Peptide
<400> 76
   Ser Glu Glu Pro Pro Ile Ser Leu Asp Leu Thr Phe His Leu Leu Arg
   1 5 10 15
   Glu Val Leu Glu Met Ala Arg Ala Glu Gln Leu Ala Gln Gln Ala His
   20 25 30
   Ser Asn Arg Lys Leu Met Glu Ile Ile
   35 40
<210> 77
   <211> 41
   <212> PRT
   <213> Artificial Sequence
<220>
   <223> Description of Artificial Sequence: Synthetic Peptide
<400> 77
   Ser Gln Glu Pro Pro Ile Ser Leu Asp Leu Thr Phe His Leu Leu Arg
   1 5 10 15
   Glu Val Leu Glu Met Thr Lys Ala Asp Gln Leu Ala Gln Gln Ala His
   20 25 30
   Ser Asn Arg Lys Leu Leu Asp Ile Ala
   35 40

<210> 78
   <211> 41
   <212> PRT
   <213> Artificial Sequence
<220>
   <223> Description of Artificial Sequence: Synthetic Peptide
<400> 78
   Ser Glu Glu Pro Pro Ile Ser Leu Asp Leu Thr Phe His Leu Leu Arg
   1 5 10 15
   Glu Val Leu Glu Met Ala Arg Ala Glu Gln Leu Ala Gln Gln Ala His
   20 25 30
   Ser Asn Arg Lys Leu Met Glu Asn Phe
   35 40
<210> 79
   <211> 21
   <212> PRT
   <213> Artificial Sequence
<220>
   <223> Description of Artificial Sequence: Synthetic Peptide
<400> 79
   Ser Glu Glu Pro Pro Ile Ser Leu Asp Leu Thr Phe His Leu Leu Arg
   1 5 10 15
   Glu Val Leu Glu Cys
   20
<210> 80
   <211> 33
   <212> PRT
   <213> Artificial Sequence
<220>
   <223> Description of Artificial Sequence: Synthetic Peptide
<400> 80
   Asp Leu Thr Phe His Leu Leu Arg Glu Met Leu Glu Met Ala Lys Ala
   1 5 10 15
   Glu Gln Glu Ala Glu Gln Ala Ala Leu Asn Arg Leu Leu Leu Glu Glu
   20 25 30
   Ala
<210> 81
   <212> PRT
   <213> Artificial Sequence
<220>
   <223> Description of Artificial sequence: Synthetic Peptide
<400> 81
<210> 82
   <211> 28
   <212> PRT
   <213> Artificial Sequence
<220>
   <223> Description of Artificial Sequence: Synthetic Peptide
<400> 82
   Phe His Leu Leu Arg Glu Val Leu Glu Ala Arg Ala Glu Gln Leu Ala
   1 5 10 15
   Gln Gln Ala His Ser Asn Arg Lys Leu Glu Ile Ile
   20 25
<210> 83
   <211> 28
   <212> PRT
   <213> Artificial Sequence
<220>
   <223> Description of Artificial Sequence: Synthetic Peptide
<400> 83
   Phe His Leu Leu Arg Glu Val Leu Glu Ala Arg Ala Glu Gln Leu Ala
   1 5 10 15
   Gln Glu Ala His Lys Asn Arg Lys Leu Glu Ile Ile
   20 25
<210> 84
   <211> 40
   <212> PRT
   <213> Artificial Sequence
<220>
   <223> Description of Artificial Sequence: Synthetic Peptide
<400> 84
   Ser Glu Glu Pro Ile Ser Leu Asp Leu Thr Phe His Leu Leu Arg Glu
   1 5 10 15
   Val Leu Glu Met Ala Arg Ala Glu Gln Leu Ala Gln Gln Ala His Ser
   20 25 30
   Asn Arg Lys Leu Met Glu Ile Ile
   35 40
<210> 85
   <211> 4
   <212> PRT
   <213> Artificial Sequence
<220>
   <223> Description of Artificial Sequence: Synthetic Peptide
<400> 85
   Val Gly Ser Glu
   1
<210> 86
   <211> 4
   <212> PRT
   <213> Artificial Sequence
<220>
   <223> Description of Artificial Sequence: Synthetic Peptide
<400> 86
   Ala Gly Ser Glu
   1
<210> 87
   <211> 41
   <212> PRT
   <213> Artificial Sequence
<220>
   <223> Description of Artificial Sequence: Synthetic Peptide
<400> 87
   Ser Gln Glu Pro Pro Ile Ser Leu Asp Leu Thr Phe His Leu Leu Arg
   1 5 10 15
   Glu Val Leu Glu Met Thr Lys Ala Asp Gln Leu Ala Gln Gln Ala His
   20 25 30
   Ser Asn Arg Lys Leu Leu Asp Ile Ala
   35 40
<210> 88
   <211> 40
   <212> PRT
   <213> Artificial Sequence
<220>
   <223> Description of Artificial Sequence: Synthetic Peptide
<400> 88
   Ser Gln Glu Pro Pro Ile Ser Leu Asp Leu Thr Phe His Leu Leu Arg
   1 5 10 15
   Glu Val Leu Glu Thr Lys Ala Asp Gln Leu Ala Gln Gln Ala Tyr Ser
   20 25 30
   Asn Arg Lys Leu Leu Asp Ile Ala
   35 40
<210> 89
   <211> 27
   <212> PRT
   <213> Artificial Sequence
<220>
   <223> Description of Artificial Sequence: Synthetic Peptide
<400> 89
   Ser Leu Asp Ser Pro Ala Ala Leu Ala Glu Arg Gly Ala Arg Asn Ala
   1 5 10 15
   Leu Gly Gly His Gln Glu Ala Pro Glu Arg Glu
   20 25
<210> 90
   <211> 40
   <212> PRT
   <213> Artificial Sequence
<220>
   <223> Description of Artificial Sequence: Synthetic Peptide
<400> 90
   Glu Gly Pro Pro Ile Ser Ile Asp Leu Ser Leu Glu Leu Leu Arg Lys
   1 5 10 15
   Met Ile Glu Ile Glu Lys Gln Glu Lys Glu Lys Gln Gln Ala Ala Asn
   20 25 30
   Asn Arg Leu Leu Leu Asp Thr Ile
   35 40
<210> 91
   <211> 42
   <212> PRT
   <213> Artificial Sequence
<220>
   <223> Description of Artificial Sequence: Synthetic Peptide
<400> 91
   Tyr Ser Glu Glu Pro Pro Ile Ser Leu Asp Leu Thr Phe His Leu Leu
   1 5 10 15
   Arg Glu Val Leu Glu Met Ala Arg Ala Glu Gln Leu Ala Gln Gln Ala
   20 25 30
   His Ser Asn Arg Lys Leu Met Glu Ile Ile
   35 40
<210> 92
   <211> 42
   <212> PRT
   <213> Artificial Sequence
<220>
   <223> Description of Artificial Sequence: Synthetic Peptide
<400> 92
   Tyr Ser Gln Glu Pro Pro Ile Ser Leu Asp Leu Thr Phe His Leu Leu
   1 5 10 15
   Arg Glu Val Leu Glu Met Thr Lys Ala Asp Gln Leu Ala Gln Gln Ala
   20 25 30
   His Ser Asn Arg Lys Leu Leu Asp Ile Ala
   35 40
<210> 93
   <211> 9
   <212> PRT
   <213> Artificial Sequence
<220>
   <223> Description of Artificial Sequence: Synthetic Peptide
<400> 93
   Tyr Asn Arg Lys Leu Leu Asp Ile Ala
   1 5
<210> 94
   <211> 41
   <212> PRT
   <213> Artificial Sequence
<220>
   <223> Description of Artificial Sequence: Synthetic Peptide
<400> 94
   Tyr Asp Asp Pro Pro Leu Ser Ile Asp Leu Thr Phe His Leu Leu Arg
   1 5 10 15
   Thr Leu Leu Glu Leu Ala Arg Thr Gln Ser Gln Arg Glu Arg Ala Glu
   20 25 30
   Gln Asn Arg Ile Ile Phe Asp Ser Val
   35 40
<210> 95
   <211> 41
   <212> PRT
   <213> Artificial Sequence
<220>
   <223> Description of Artificial Sequence: Synthetic Peptide
<400> 95
   Tyr Asp Asp Pro Pro Leu Ser Ile Asp Leu Thr Phe His Leu Leu Arg
   1 5 10 15
   Thr Leu Leu Glu Leu Ala Arg Thr Gln Ser Gln Arg Glu Arg Ala Glu
   20 25 30
   Gln Asn Arg Ile Ile Phe Asp Ser Val
   35 40
<210> 96
   <211> 40
   <212> PRT
   <213> Artificial Sequence
<220>
   <223> Description of Artificial Sequence: Synthetic Peptide
<400> 96
   Asp Asn Pro Pro Leu Ser Ile Asp Leu Thr Phe His Leu Leu Arg Thr
   1 5 10 15
   Leu Leu Glu Leu Ala Arg Thr Gln Ser Gln Arg Glu Arg Ala Glu Gln
   20 25 30
   Asn Arg Ile Ile Phe Asp Ser Val
   35 40
<210> 97
   <211> 40
   <212> PRT
   <213> Artificial Sequence
<220>
   <223> Description of Artificial Sequence: Synthetic Peptide
<400> 97
   Asp Asp Pro Pro Leu Ser Ile Asp Leu Thr Phe His Leu Leu Arg Thr
   1 5 10 15
   Leu Leu Glu Leu Ala Arg Thr Gln Ser Gln Arg Glu Arg Ala Glu Gln
   20 25 30
   Asn Arg Ile Ile Phe Asp Ser Val
   35 40
<210> 98
   <211> 41
   <212> PRT
   <213> Artificial Sequence
<220>
   <223> Description of Artificial Sequence: Synthetic Peptide
<400> 98
   Asn Asp Asp Pro Pro Ile Ser Ile Asp Leu Thr Phe His Leu Leu Arg
   1               5                   10                  15
   Asn Met Ile Glu Met Ala Arg Ile Glu Asn Glu Arg Glu Gln Ala Gly
               20                  25                  30
   Leu Asn Arg Lys Tyr Leu Asp Glu Val
           35                  40
<210> 99
   <211> 12
   <212> PRT
   <213> Artificial Sequence
<220>
   <223> Description of Artificial Sequence: Synthetic Peptide
<400> 99
   Ala Gly Thr Ala Asp Cys Phe Trp Lys Tyr Cys Val
   1               5                   10
<210> 100
   <211> 13
   <212> PRT
   <213> Artificial Sequence
<220>
   <223> Description of Artificial Sequence: Synthetic Peptide
<400> 100
   Ala Gly Asn Leu Ser Glu Cys Phe Trp Lys Tyr Cys Val
   1               5                   10
<210> 101
   <211> 46
   <212> PRT
   <213> Artificial Sequence
<220>
   <223> Description of Artificial Sequence: Synthetic Peptide
<400> 101
   Thr Gly Ser Gly Pro Ser Leu Ser Ile Val Asn Pro Leu Asp Val Leu
   1 5 10 15
   Arg Gln Arg Leu Leu Leu Glu Ile Ala Arg Arg Arg Met Arg Gln Ser
   20 25 30
   Gln Asp Gln Ile Gln Ala Asn Arg Glu Ile Leu Gln Thr Ile
   35 40 45
<210> 102
   <211> 41
   <212> PRT
   <213> Artificial Sequence
<220>
   <223> Description of Artificial Sequence: Synthetic Peptide
<400> 102
   Ser Glu Asp Pro Pro Met Ser Ile Asp Leu Thr Phe His Met Leu Arg
   1 5 10 15
   Asn Met Ile His Met Ala Lys Met Glu Gly Glu Arg Glu Gln Ala Gln
   20 25 30
   Ile Asn Arg Asn Leu Leu Asp Glu Val
   35 40
<210> 103
   <211> 29
   <212> PRT
   <213> Artificial Sequence
<220>
   <223> Description of Artificial Sequence: Synthetic Peptide
<400> 103
   Phe Glu Cys Thr Thr His Gln Pro Arg Ser Pro Leu Arg Asp Leu Lys
   1 5 10 15
   Gly Ala Leu Glu Ser Leu Ile Glu Glu Glu Thr Gly Gln
   20 25
<210> 104
   <211> 29
   <212> PRT
   <213> Artificial Sequence
<220>
   <223> Description of Artificial Sequence: Synthetic Peptide
<400> 104
   Phe Glu Cys Thr Thr His Gln Pro Arg Ser Pro Leu Arg Asp Leu Lys
   1 5 10 15
   Gly Ala Leu Glu Ser Leu Ile Glu Glu Glu Thr Gly Gln
   20 25
<210> 105
   <211> 13
   <212> PRT
   <213> Artificial Sequence
<220>
   <223> Description of Artificial Sequence: Synthetic Peptide
<400> 105
   Asp Ala Glu Asn Leu Ile Asp Ser Phe Gln Glu Ile Val
   1 5 10
<210> 106
   <211> 13
   <212> PRT
   <213> Artificial Sequence
<220>
   <223> Description of Artificial Sequence: Synthetic Peptide
<400> 106
   Asn Thr Glu His Leu Val Asp Ser Phe Gln Glu Met Gly
   1 5 10
<210> 107
   <211> 13
   <212> PRT
   <213> Artificial Sequence
<220>
   <223> Description of Artificial Sequence: Synthetic Peptide
<400> 107
   Asp Thr Ser His His Asp Gln Asp His Pro Thr Phe Asp
   1 5 10
<210> 108
   <212> PRT
   <213> Artificial Sequence
<220>
   <223> Description of Artificial Sequence: Synthetic Peptide
<400> 108
<210> 109
   <211> 26
   <212> PRT
   <213> Artificial Sequence
<220>
   <223> Description of Artificial Sequence: Synthetic Peptide
<400> 109
   Trp Cys Leu Glu Ser Ser Gln Cys Gln Asp Leu Ser Thr Glu Ser Asn
   1 5 10 15
   Leu Leu Ala Cys Ile Arg Ala Cys Lys Pro
   20 25
<210> 110
   <211> 10
   <212> PRT
   <213> Artificial Sequence
<220>
   <223> Description of. Artificial Sequence: Synthetic Peptide
<400> 110
   Gln His Trp Ser Tyr Gly Leu Ser Pro Gly
   1 5 10
<210> 111
   <211> 44
   <212> PRT
   <213> Artificial Sequence
<220>
   <223> Description of Artificial Sequence: Synthetic Peptide
<400> 111
   Tyr Ala Asp Ala Ile Phe Thr Asn Ser Tyr Arg Lys Val Leu Gly Gln
   1 5 10 15
   Leu Ser Ala Arg Lys Leu Leu Gln Asp Ile Met Ser Arg Gln Gln Gly
   20 25 30
   Glu Ser Asn Gln Glu Arg Gly Ala Arg Ala Arg Leu
   35 40
<210> 112
   <211> 44
   <212> PRT
   <213> Artificial Sequence
<220>
   <223> Description of Artificial Sequence: Synthetic Peptide
<400> 112
   Tyr Ala Asp Ala Ile Phe Thr Asn Ser Tyr Arg Lys Val Leu Gly Gln
   1 5 10 15
   Leu Ser Ala Arg Lys Leu Leu Gln Asp Ile Met Asn Arg Gln Gln Gly
   20 25 30
   Glu Arg Asn Gln Glu Gln Gly Ala Lys Val Arg Leu
   35 40
<210> 113
   <211> 44
   <212> PRT
   <213> Artificial Sequence
<220>
   <223> Description of Artificial Sequence: Synthetic Peptide
<400> 113
   Tyr Ala Asp Ala Ile Phe Thr Asn Ser Tyr Arg Lys Val Leu Gly Gln
   1 5 10 15
   Leu Ser Ala Arg Lys Leu Leu Gln Asp Ile Met Ser Arg Gln Gln Gly
   20 25 30
   Glu Arg Asn Gln Glu Gln Gly Ala Arg Val Arg Leu
   35 40
<210> 114
   <211> 29
   <212> PRT
   <213> Artificial Sequence
<220>
   <223> Description of Artificial Sequence: Synthetic Peptide
<400> 114
   His Ala Asp Ala Ile Phe Thr Ser Ser Tyr Arg Arg Ile Leu Gly Gln
   1 5 10 15
   Leu Tyr Ala Arg Lys Leu Leu His Glu Ile Met Asn Arg
   20 25
<210> 115
   <211> 30
   <212> PRT
   <213> Artificial Sequence
<220>
   <223> Description of Artificial Sequence: Synthetic Peptide
<400> 115
   Val Asp Ser Met Trp Ala Glu Gln Lys Gln Met Glu Leu Glu Ser Ile
   1 5 10 15
   Leu Val Ala Leu Leu Gln Lys His Ser Arg Asn Ser Gln Gly
   20 25 30
<210> 116
   <211> 29
   <212> PRT
   <213> Artificial Sequence
<220>
   <223> Description of Artificial Sequence: Synthetic Peptide
<400> 116
   Tyr Ala Asp Ala Ile Phe Thr Asn Ser Tyr Arg Lys Val Leu Gly Gln
   1 5 10 15
   Leu Ser Ala Arg Lys Leu Leu Gln Asp Ile Met Ser Arg
   20 25
<210> 117
   <211> 29
   <212> PRT
   <213> Artificial Sequence
<220>
   <223> Description of Artificial Sequence: Synthetic Peptide
<400> 117
   Tyr Ala Asp Ala Ile Phe Thr Asn Ser Tyr Arg Lys Val Leu Gly Gln
   1 5 10 15
   Leu Ser Ala Arg Lys Leu Leu Gln Asp Ile Met Ser Arg
   20 25
<210> 118
   <211> 29
   <212> PRT
   <213> Artificial Sequence
<220>
   <223> Description of Artificial Sequence: Synthetic Peptide
<400> 118
   Tyr Arg Asp Ala Ile Phe Thr Asn Ser Tyr Arg Lys Val Leu Gly Gln
   1 5 10 15
   Leu Ser Ala Arg Lys Leu Leu Gln Asp Ile Met Ser Arg
   20 25
<210> 119
   <211> 31
   <212> PRT
   <213> Artificial Sequence
<220>
   <223> Description of Artificial Sequence: Synthetic Peptide
<400> 119
   His Ala Asp Ala Ile Phe Thr Asn Ser Tyr Arg Lys Val Leu Gly Gln
   1 5 10 15
   Leu Ser Ala Arg Lys Leu Leu Gln Asp Ile Ser Arg Gln Gln Gly
   20 25 30
<210> 120
   <211> 37
   <212> PRT
   <213> Artificial Sequence
<220>
   <223> Description of Artificial Sequence: Synthetic Peptide
<400> 120
   Tyr Ala Asp Ala Ile Phe Thr Asn Ser Tyr Arg Lys Val Leu Gly Gln
   1 5 10 15
   Leu Ser Ala Arg Lys Leu Leu Gln Asp Ile Met Ser Arg Gln Gln Gly
   20 25 30
   Glu Ser Asn Gln Glu
   35
<210> 121
   <211> 40
   <212> PRT
   <213> Artificial Sequence
<220>
   <223> Description of Artificial Sequence: Synthetic Peptide
<400> 121
   Tyr Ala Asp Ala Ile Phe Thr Asn Ser Tyr Arg Lys Val Leu Gly Gln
   1 5 10 15
   Leu Ser Ala Arg Lys Leu Leu Gln Asp Ile Met Ser Arg Gln Gln Gly
   20 25 30
   Glu Ser Asn Gln Glu Arg Gly Ala
   35 40
<210> 122
   <211> 40
   <212> PRT
   <213> Artificial Sequence
<220>
   <223> Description of Artificial Sequence: Synthetic Peptide
<400> 122
   Tyr Ala Asp Ala Ile Phe Thr Asn Ser Tyr Arg Lys Val Leu Gly Gln
   1 5 10 15
   Leu Ser Ala Arg Lys Leu Leu Gln Asp Ile Met Ser Arg Gln Gln Gly
   20 25 30
   Glu Ser Asn Gln Glu Arg Gly Ala
   35 40
<210> 123
   <211> 15
   <212> PRT
   <213> Artificial Sequence
<220>
   <223> Description of Artificial Sequence: Synthetic Peptide
<400> 123
   Glu Gln Gly Glu Ser Asn Gln Glu Arg Gly Ala Arg Ala Arg Leu
   1 5 10 15
<210> 124
   <211> 42
   <212> PRT
   <213> Artificial Sequence
<220>
   <223> Description of Artificial Sequence: Synthetic Peptide
<400> 124
   His Val Asp Ala Ile Phe Thr Thr Asn Tyr Arg Lys Leu Leu Ser Gln
   1 5 10 15
   Leu Tyr Ala Arg Lys Val Ile Gln Asp Ile Met Asn Lys Gln Gly Glu
   20 25 30
   Arg Ile Gln Glu Gln Arg Ala Arg Leu Ser
   35 40
<210> 125
   <211> 44
   <212> PRT
   <213> Artificial Sequence
<220>
   <223> Description of Artificial Sequence: Synthetic Peptide
<400> 125
   Tyr Ala Asp Ala Ile Phe Thr Asn Ser Tyr Arg Lys Ile Leu Gly Gln
   1 5 10 15
   Leu Ser Ala Arg Lys Leu Leu Gln Asp Ile Met Asn Arg Gln Gln Gly
   20 25 30
   Glu Arg Asn Gln Glu Gln Gly Ala Lys Val Arg Leu
   35 40
<210> 126
   <211> 43
   <212> PRT
   <213> Artificial Sequence
<220>
   <223> Description of Artificial Sequence: Synthetic Peptide
<400> 126
   His Ala Asp Ala Ile Phe Thr Ser Ser Tyr Arg Arg Ile Leu Gly Gln
   1 5 10 15
   Leu Tyr Ala Arg Lys Leu Leu His Glu Ile Met Asn Arg Gln Gln Gly
   20 25 30
   Glu Arg Asn Gln Glu Gln Arg Ser Arg Phe Asn
   35 40
<210> 127
   <211> 6
   <212> PRT
   <213> Artificial Sequence
<220>
   <223> Description of Artificial Sequence: Synthetic Peptide
<400> 127
   His Trp Ala Trp Phe Lys
   1 5
<210> 128
   <211> 6
   <212> PRT
   <213> Artificial Sequence
<220>
   <223> Description of Artificial Sequence: Synthetic Peptide
<400> 128
   His Trp Ala Trp Phe Lys
   1 5
<210> 129
   <211> 5
   <212> PRT
   <213> Artificial Sequence
<220>
   <223> Description of Artificial Sequence: Synthetic Peptide
<400> 129
   Ala Ala Trp Phe Lys
   1 5
<210> 130
   <211> 6
   <212> PRT
   <213> Artificial Sequence
<220>
   <223> Description of Artificial Sequence: Synthetic Peptide
<400> 130
   His Trp Lys Trp Phe Lys
   1 5
<210> 131
   <211> 6
   <212> PRT
   <213> Artificial Sequence
<220>
   <223> Description of Artificial Sequence: Synthetic Peptide
<400> 131
   Ala Ala Ala Trp Phe Leu
   1 5
<210> 132
   <211> 45
   <212> PRT
   <213> Artificial Sequence
<220>
   <223> Description of Artificial Sequence: Synthetic Peptide
<400> 132
   His Ala Asp Gly Met Phe Asn Lys Ala Tyr Arg Lys Ala Leu Gly Gln
   1 5 10 15
   Leu Ser Ala Arg Lys Tyr Leu His Thr Leu Met Ala Lys Arg Val Gly
   20 25 30
   Gly Gly Ser Met Ile Glu Asp Asp Asn Glu Pro Leu Ser
   35 40 45
<210> 133
   <211> 44
   <212> PRT
   <213> Artificial Sequence
<220>
   <223> Description of Artificial Sequence: Synthetic Peptide
<400> 133
   Tyr Ala Asp Ala Ile Phe Thr Asn Ser Tyr Arg Lys Val Leu Gly Gln
   1 5 10 15
   Leu Ser Ala Arg Lys Leu Leu Gln Asp Ile Asn Ser Arg Gln Gln Gly
   20 25 30
   Glu Ser Asn Gln Glu Arg Gly Ala Arg Ala Arg Leu
   35 40
<210> 134
   <211> 28
   <212> PRT
   <213> Artificial Sequence
<220>
   <223> Description of Artificial Sequence: synthetic Peptide
<400> 134
   Tyr Ala Asp Ala Ile Phe Thr Asn Cys Tyr Arg Lys Val Leu Cys Gln
   1 5 10 15
   Leu Ser Ala Arg Lys Leu Leu Gln Asp Ile Ser Arg
   20 25
<210> 135
   <211> 8
   <212> PRT
   <213> Artificial Sequence
<220>
   <223> Description of Artificial Sequence: Synthetic Peptide
<400> 135
   Phe Ser Lys Lys Leu Lys Pro Ala
   1 5
<210> 136
   <211> 10
   <212> PRT
   <213> Artificial Sequence
<220>
   <223> Description of Artificial Sequence: Synthetic Peptide
<400> 136
   Glu His Trp Ser His Gly Trp Tyr Pro Gly
   1 5 10
<210> 137
   <211> 10
   <212> PRT
   <213> Artificial Sequence
<220>
   <223> Description of Artificial Sequence: Synthetic Peptide
<400> 137
   Glu His Trp Ser Tyr Gly Leu Arg Pro Gly
   1 5 10
<210> 138
   <211> 7
   <212> PRT
   <213> Artificial Sequence
<220>
   <223> Description of Artificial Sequence: Synthetic Peptide
<400> 138
   Phe Ser Tyr Leu Arg Pro Ala
   1 5
<210> 139
   <211> 10
   <212> PRT
   <213> Artificial Sequence
<220>
   <223> Description of Artificial Sequence: Synthetic Peptide
<400> 139
   Glu His Trp Ser Tyr Ala Leu Arg Pro Gly
   1 5 10
<210> 140
   <211> 10
   <212> PRT
   <213> Artificial Sequence
<220>
   <223> Description of Artificial Sequence: Synthetic Peptide
<400> 140
   Glu His Trp Ser Tyr Gly Leu Gln Pro Gly
   1 5 10
<210> 141
   <211> 7
   <212> PRT
   <213> Artificial Sequence
<220>
   <223> Description of Artificial Sequence: Synthetic Peptide
<400> 141
   His Trp Ser Tyr Val Arg Pro
   1 5
<210> 142
   <211> 10
   <212> PRT
   <213> Artificial Sequence
<220>
   <223> Description of Artificial Sequence: Synthetic Peptide
<400> 142
   Glu His Trp Ser Tyr Lys Leu Arg Pro Gly
   1 5 10
<210> 143
   <211> 10
   <212> PRT
   <213> Artificial Sequence
<220>
   <223> Description of Artificial Sequence: Synthetic Peptide
<400> 143
   Glu Phe Pro Ser Tyr Phe Leu Arg Pro Gly
   1 5 10
<210> 144
   <211> 9
   <212> PRT
   <213> Artificial Sequence
<220>
   <223> Description of Artificial Sequence: Synthetic Peptide
<400> 144
   Phe Trp Ser Tyr Ala Leu Arg Pro Gly
   1 5
<210> 145
   <211> 10
   <212> PRT
   <213> Artificial Sequence
<220>
   <223> Description of Artificial Sequence: Synthetic Peptide
<400> 145
   Glu Phe Trp Ser Tyr Trp Leu Arg Pro Gly
   1 5 10
<210> 146
   <211> 9
   <212> PRT
   <213> Artificial Sequence
<220>
   <223> Description of Artificial Sequence: Synthetic Peptide
<400> 146
   Glu His Trp Ser Tyr Gly Leu Arg Pro
   1 5
<210> 147
   <211> 9
   <212> PRT
   <213> Artificial Sequence
<220>
   <223> Description of Artificial Sequence: Synthetic Peptide
<400> 147
   Glu His Trp Ser Tyr Ala Leu Arg Pro
   1 5
<210> 148
   <211> 9
   <212> PRT
   <213> Artificial Sequence
<220>
   <223> Description of Artificial Sequence: Synthetic Peptide
<400> 148
   His Trp Ser Tyr Trp Leu Arg Pro Gly
   1 5
<210> 149
   <211> 9
   <212> PRT
   <213> Artificial Sequence
<220>
   <223> Description of Artificial Sequence: Synthetic Peptide
<400> 149
   Glu His Trp Ser Tyr Trp Leu Arg Pro
   1 5

<210> 150
   <211> 8
   <212> PRT
   <213> Artificial Sequence
<220>
   <223> Description of Artificial Sequence: Synthetic Peptide
<400> 150
   His Trp Ser Tyr Ser Leu Arg Pro
   1 5
<210> 151
   <211> 9
   <212> PRT
   <213> Artificial Sequence
<220>
   <223> Description of Artificial Sequence: Synthetic Peptide
<400> 151
   Glu His Trp Ser Tyr Arg Trp Leu Pro
   1 5
<210> 152
   <211> 4
   <212> PRT
   <213> Artificial Sequence
<220>
   <223> Description of Artificial Sequence: Synthetic Peptide
<400> 152
   Leu Arg Pro Gly
   1
<210> 153
   <211> 9
   <212> PRT
   <213> Artificial Sequence
<220>
   <223> Description of Artificial Sequence: Synthetic Peptide
<400> 153
   His Trp Ser Tyr Gly Leu Arg Pro Gly
   1 5
<210> 154
   <211> 10
   <212> PRT
   <213> Artificial Sequence
<220>
   <223> Description of Artificial Sequence: Synthetic Peptide
<400> 154
   Glu His Tyr Ser Leu Glu Trp Lys Pro Gly
   1 5 10
<210> 155
   <211> 10
   <212> PRT
   <213> Artificial Sequence
<220>
   <223> Description of Artificial Sequence: Synthetic Peptide
<400> 155
   Glu His Trp Ser Tyr Gly Trp Leu Pro Gly
   1 5 10
<210> 156
   <211> 7
   <212> PRT
   <213> Artificial Sequence
<220>
   <223> Description of Artificial Sequence: Synthetic Peptide
<400> 156
   Ser Tyr Gly Leu Arg Pro Gly
   1 5
<210> 157
   <211> 9
   <212> PRT
   <213> Artificial Sequence
<220>
   <223> Description of Artificial Sequence: Synthetic Peptide
<400> 157
   His Trp Ser Tyr Gly Leu Lys Pro Gly
   1 5
<210> 158
   <211> 7
   <212> PRT
   <213> Artificial Sequence
<220>
   <223> Description of Artificial Sequence: Synthetic Peptide
<400> 158
   Phe Phe Ser Tyr Leu Arg Pro
   1 5
<210> 159
   <211> 9
   <212> PRT
   <213> Artificial Sequence
<220>
   <223> Description of Artificial Sequence: Synthetic peptide
<400> 159
   His Trp Ser Tyr Gly Trp Leu Pro Gly
   1 5
<210> 160
   <211> 9
   <212> PRT
   <213> Artificial Sequence
<220>
   <223> Description of Artificial Sequence: Synthetic Peptide
<400> 160
   His Trp Ser Tyr Ser Leu Arg Pro Gly
   1 5
<210> 161
   <211> 9
   <212> PRT
   <213> Artificial Sequence
<220>
   <223> Description of Artificial Sequence: Synthetic Peptide
<400> 161
   Ala Phe Trp Ser Tyr Ser Leu Arg Pro
   1 5
<210> 162
   <211> 31
   <212> PRT
   <213> Artificial Sequence
<220>
   <223> Description of Artificial Sequence: Synthetic Peptide
<400> 162
   Glu Pro Asp Cys Cys Arg Gln Lys Thr Cys Ser Cys Arg Leu Tyr Glu
   1 5 10 15
   Leu Leu His Gly Ala Gly Asn His Ala Ala Gly Ile Leu Thr Leu
   20 25 30
<210> 163
   <211> 28
   <212> PRT
   <213> Artificial Sequence
<220>
   <223> Description of Artificial Sequence: Synthetic Peptide
<400> 163
   Arg Ser Gly Pro Pro Gly Leu Gln Gly Arg Leu Gln Arg Leu Leu Gln
   1 5 10 15
   Ala Ser Gly Asn His Ala Ala Gly Ile Leu Thr Met
   20 25
<210> 164
   <211> 28
   <212> PRT
   <213> Artificial Sequence
<220>
   <223> Description of Artificial Sequence: Synthetic Peptide
<400> 164
   Arg Pro Gly Pro Pro Gly Leu Gln Gly Arg Leu Gln Arg Leu Leu Gln
   1 5 10 15
   Ala Asn Gly Asn His Ala Ala Gly Ile Leu Thr Met
   20 25
<210> 165
   <211> 31
   <212> PRT
   <213> Artificial Sequence
<220>
   <223> Description of Artificial Sequence: Synthetic Peptide
<400> 165
   Ser Arg Thr His Arg His Ser Met Glu Ile Arg Thr Pro Asp Ile Asn
   1 5 10 15
   Pro Ala Trp Tyr Ala Ser Arg Gly Ile Arg Pro Val Gly Arg Phe
   20 25 30
<210> 166
   <211> 20
   <212> PRT
   <213> Artificial Sequence
<220>
   <223> Description of Artificial Sequence: Synthetic Peptide
<400> 166
   Thr Pro Asp Ile Asn Pro Ala Trp Tyr Ala Ser Arg Gly Ile Arg Pro
   1 5 10 15
   Val Gly Arg Phe
   20
<210> 167
   <211> 31
   <212> PRT
   <213> Artificial Sequence
<220>
   <223> Description of Artificial Sequence: Synthetic Peptide
<400> 167
   Ser Arg Ala His Gln His Ser Met Glu Thr Arg Thr Pro Asp Ile Asn
   1 5 10 15
   Pro Ala Trp Tyr Thr Gly Arg Gly Ile Arg Pro Val Gly Arg Phe
   20 25 30
<210> 168
   <211> 20
   <212> PRT
   <213> Artificial Sequence
<220>
   <223> Description of Artificial Sequence: Synthetic Peptide
<400> 168
   Thr Pro Asp Ile Asn Pro Ala Trp Tyr Thr Gly Arg Gly Ile Arg Pro
   1 5 10 15
   Val Gly Arg Phe
   20
<210> 169
   <211> 31
   <212> PRT
   <213> Artificial Sequence
<220>
   <223> Description of Artificial Sequence: Synthetic Peptide
<400> 169
   Ser Arg Ala His Gln His Ser Met Glu Ile Arg Thr Pro Asp Ile Asn
   1 5 10 15
   Pro Ala Trp Tyr Ala Ser Arg Gly Ile Arg Pro Val Gly Arg Phe
   20 25 30
<210> 170
   <211> 20
   <212> PRT
   <213> Artificial Sequence
<220>
   <223> Description of Artificial Sequence: Synthetic Peptide
<400> 170
   Thr Pro Asp Ile Asn Pro Ala Trp Tyr Ala Gly Arg Gly Ile Arg Pro
   1 5 10 15
   Val Gly Arg Phe
   20
<210> 171
   <211> 5
   <212> PRT
   <213> Artificial Sequence
<220>
   <223> Description of Artificial Sequence: Synthetic Peptide
<400> 171
   Ala Tyr Trp Lys Phe
   1 5
<210> 172
   <211> 14
   <212> PRT
   <213> Artificial Sequence
<220>
   <223> Description of Artificial Sequence: Synthetic Peptide
<400> 172
   Pro Cys Lys Asn Phe Phe Trp Lys Thr Phe Ser Ser Cys Lys
   1 5 10
<210> 173
   <211> 17
   <212> PRT
   <213> Artificial Sequence
<220>
   <223> Description of Artificial Sequence: Synthetic Peptide
<400> 173
   Asp Arg Met Pro Cys Arg Asn Phe Phe Trp Lys Thr Phe Ser Ser Cys
   1 5 10 15
   Lys
<210> 174
   <211> 17
   <212> PRT
   <213> Artificial Sequence
<220>
   <223> Description of Artificial Sequence: Synthetic Peptide
<400> 174
   Asp Arg Met Pro Cys Arg Asn Phe Phe Trp Lys Thr Phe Ser Ser Cys
   1 5 10 15
   Lys
<210> 175
   <211> 14
   <212> PRT
   <213> Artificial Sequence
<220>
   <223> Description of Artificial Sequence: Synthetic Peptide
<400> 175
   Ala Gly Cys Lys Asn Phe Phe Trp Lys Thr Phe Thr Ser Cys
   1 5 10
<210> 176
   <211> 13
   <212> PRT
   <213> Artificial Sequence
<220>
   <223> Description of Artificial Sequence: Synthetic Peptide
<400> 176
   Ala Gly Cys Lys Asn Phe Phe Lys Thr Phe Thr Ser Cys
   1 5 10
<210> 177
   <211> 13
   <212> PRT
   <213> Artificial Sequence
<220>
   <223> Description of Artificial Sequence: Synthetic Peptide
<400> 177
   Ala Gly Cys Lys Asn Phe Phe Lys Thr Tyr Thr Ser Cys
   1 5 10
<210> 178
   <211> 11
   <212> PRT
   <213> Artificial Sequence
<220>
   <223> Description of Artificial Sequence: Synthetic Peptide
<400> 178
   Cys His His Phe Phe Lys Thr Phe Thr Ser Cys
   1 5 10
<210> 179
   <211> 14
   <212> PRT
   <213> Artificial Sequence
<220>
   <223> Description of Artificial Sequence: Synthetic Peptide
<400> 179
   Ala Gly Cys Lys Asn Phe Phe Trp Lys Thr Trp Thr Ser Cys
   1 5 10
<210> 180
   <211> 5
   <212> PRT
   <213> Artificial Sequence
<220>
   <223> Description of Artificial Sequence: Synthetic Peptide
<400> 180
   Phe Cys Tyr Thr Thr
   1 5
<210> 181
   <211> 27
   <212> PRT
   <213> Artificial Sequence
<220>
   <223> Description of Artificial Sequence: Synthetic Peptide
<400> 181
   Ser Ala Asn Ser Asn Pro Ala Ala Pro Arg Glu Arg Lys Ala Gly Cys
   1 5 10 15
   Lys Asn Phe Phe Trp Lys Thr Phe Thr Ser Cys
   20 25
<210> 182
   <211> 7
   <212> PRT
   <213> Artificial Sequence
<220>
   <223> Description of Artificial Sequence: Synthetic Peptide
<400> 182
   Phe Cys Phe Lys Thr Cys Thr
   1 5
<210> 183
   <211> 32
   <212> PRT
   <213> Artificial Sequence
<220>
   <223> Description of Artificial Sequence: Synthetic Peptide
<400> 183
   Ala Pro Ser Asp Pro Arg Leu Arg Gln Phe Leu Gln Lys Ser Leu Ala
   1 5 10 15
   Ala Ala Ala Gly Lys Gln Glu Leu Ala Lys Tyr Phe Leu Ala Glu Leu
   20 25 30
<210> 184
   <211> 15
   <212> PRT
   <213> Artificial Sequence
<220>
   <223> Description of Artificial Sequence: Synthetic Peptide
<400> 184
   Tyr Ala Gly Cys Lys Asn Phe Phe Trp Lys Thr Phe Thr Ser Cys
   1 5 10 15
<210> 185
   <211> 14
   <212> PRT
   <213> Artificial Sequence
<220>
   <223> Description of Artificial Sequence: Synthetic Peptide
<400> 185
   Tyr Gly Cys Lys Asn Phe Phe Trp Lys Thr Phe Thr Ser Cys
   1 5 10
<210> 186
   <211> 14
   <212> PRT
   <213> Artificial Sequence
<220>
   <223> Description of Artificial Sequence: Synthetic Peptide
<400> 186
   Ser Ala Asn Ser Asn Pro Ala Met Ala Pro Arg Tyr Arg Lys
   1 5 10
<210> 187
   <211> 14
   <212> PRT
   <213> Artificial Sequence
<220>
   <223> Description of Artificial Sequence: Synthetic Peptide
<400> 187
   Ala Gly Cys Lys Asn Phe Phe Trp Lys Thr Tyr Thr Ser Cys
   1 5 10
<210> 188
   <211> 15
   <212> PRT
   <213> Artificial Sequence
<220>
   <223> Description of Artificial Sequence: Synthetic Peptide
<400> 188
   Tyr Ala Gly Cys Lys Asn Phe Phe Trp Lys Thr Phe Thr Ser Cys
   1 5 10 15
<210> 189
   <211> 14
   <212> PRT
   <213> Artificial Sequence
<220>
   <223> Description of Artificial Sequence: Synthetic Peptide
<400> 189
   Ala Gly Cys Lys Asn Phe Phe Trp Lys Thr Phe Thr Ser Cys
   1 5 10
<210> 190
   <211> 4
   <212> PRT
   <213> Artificial Sequence
<220>
   <223> Description of Artificial Sequence: Synthetic Peptide
<400> 190
   Phe Trp Lys Thr
   1
<210> 191
   <211> 25
   <212> PRT
   <213> Artificial Sequence
<220>
   <223> Description of Artificial Sequence: Synthetic Peptide
<400> 191
   Ser Asn Pro Ala Met Ala Pro Arg Glu Arg Lys Ala Gly Cys Lys Asn
   1 5 10 15
   Phe Phe Trp Lys Thr Phe Thr Ser Cys
   20 25
<210> 192
   <211> 28
   <212> PRT
   <213> Artificial Sequence
<220>
   <223> Description of Artificial Sequence: Synthetic Peptide
<400> 192
   Ser Ala Asn Ser Asn Pro Ala Met Ala Pro Arg Glu Arg Lys Ala Gly
   1 5 10 15
   Cys Lys Asn Phe Phe Trp Lys Thr Phe Thr Ser Cys
   20 25
<210> 193
   <211> 12
   <212> PRT
   <213> Artificial Sequence
<220>
   <223> Description of Artificial Sequence: Synthetic Peptide
<400> 193
   Ser Ala Asn Ser Asn Pro Ala Met Ala Pro Arg Glu
   1 5 10
<210> 194
   <211> 29
   <212> PRT
   <213> Artificial Sequence
<220>
   <223> Description of Artificial Sequence: Synthetic Peptide
<400> 194
   Tyr Ser Ala Asn Ser Asn Pro Ala Met Ala Pro Arg Glu Arg Lys Ala
   1 5 10 15
   Gly Cys Lys Asn Phe Phe Trp Lys Thr Phe Thr Ser Cys
   20 25
<210> 195
   <211> 28
   <212> PRT
   <213> Artificial Sequence
<220>
   <223> Description of Artificial Sequence: Synthetic Peptide
<400> 195
   Ser Ala Asn Ser Asn Pro Ala Leu Ala Pro Arg Glu Arg Lys Ala Gly
   1 5 10 15
   Cys Lys Asn Phe Phe Trp Lys Thr Tyr Thr Ser Cys
   20 25
<210> 196
   <211> 14
   <212> PRT
   <213> Artificial Sequence
<220>
   <223> Description of Artificial Sequence: Synthetic Peptide
<400> 196
   Ser Ala Asn Ser Asn Pro Ala Met Ala Pro Arg Glu Arg Lys
   1 5 10
<210> 197
   <211> 8
   <212> PRT
   <213> Artificial Sequence
<220>
   <223> Description of Artificial Sequence: Synthetic Peptide
<400> 197
   Phe Cys Tyr Trp Lys Val Cys Trp
   1 5
<210> 198
   <211> 8
   <212> PRT
   <213> Artificial Sequence
<220>
   <223> Description of Artificial Sequence: Synthetic Peptide
<400> 198
   Phe Cys Tyr Trp Lys Val Cys Trp
   1 5
<210> 199
   <211> 6
   <212> PRT
   <213> Artificial Sequence
<220>
   <223> Description of Artificial Sequence: Synthetic Peptide
<400> 199
   Phe Cys Tyr Trp Thr Thr
   1 5
<210> 200
   <211> 8
   <212> PRT
   <213> Artificial Sequence
<220>
   <223> Description of Artificial Sequence: Synthetic Peptide
<400> 200
   Ala Cys Tyr Trp Leu Val Cys Thr
   1 5
<210> 201
   <211> 14
   <212> PRT
   <213> Artificial Sequence
<220>
   <223> Description of Artificial Sequence: Synthetic Peptide
<400> 201
   Ala Gly Cys Lys Asn Phe Phe Trp Lys Thr Phe Thr Ser Cys
   1 5 10
<210> 202
   <211> 3
   <212> PRT
   <213> Artificial Sequence
<220>
   <223> Description of Artificial Sequence: Synthetic Peptide
<400> 202
   His Pro Gly
   1
<210> 203
   <211> 1
   <212> PRT
   <213> Artificial Sequence
<220>
   <223> Description of Artificial Sequence: Synthetic Peptide
<400> 203
   Thr
   1
<210> 204
   <211> 3
   <212> PRT
   <213> Artificial Sequence
<220>
   <223> Description of Artificial Sequence: Synthetic Peptide
<400> 204
   Thr His Pro
   1
<210> 205
   <211> 3
   <212> PRT
   <213> Artificial Sequence
<220>
   <223> Description of Artificial Sequence: Synthetic Peptide
<400> 205
   Glu His Pro
   1
<210> 206
   <211> 3
   <212> PRT
   <213> Artificial Sequence
<220>
   <223> Description of Artificial Sequence: Synthetic Peptide
<400> 206
   Glu Gln Pro
   1
<210> 207
   <211> 2
   <212> PRT
   <213> Artificial Sequence
<220>
   <223> Description of Artificial Sequence: Synthetic Peptide
<400> 207
   Glu His
   1
<210> 208
   <211> 4
   <212> PRT
   <213> Artificial Sequence
<220>
   <223> Description of Artificial Sequence: Synthetic Peptide
<400> 208
   His Pro Gly Lys
   1
<210> 209
   <211> 3
   <212> PRT
   <213> Artificial Sequence
<220>
   <223> Description of Artificial Sequence: Synthetic Peptide
<400> 209
   His Pro Gly
   1
<210> 210
   <211> 2
   <212> PRT
   <213> Artificial Sequence
<220>
   <223> Description of Artificial Sequence: Synthetic Peptide
<400> 210
   Glu Pro
   1
<210> 211
   <211> 2
   <212> PRT
   <213> Artificial Sequence
<220>
   <223> Description of Artificial Sequence: Synthetic Peptide
<400> 211
   Phe Pro
   1
<210> 212
   <211> 22
   <212> PRT
   <213> Artificial Sequence
<220>
   <223> Description of Artificial Sequence: Synthetic Peptide
<400> 212
   Phe Leu Trp Lys Asp Leu Gln Arg Val Arg Gly Asp Leu Gly Ala Ala
   1 5 10 15
   Leu Asp Ser Trp Ile Thr
   20
<210> 213
   <211> 24
   <212> PRT
   <213> Artificial Sequence
<220>
   <223> Description of Artificial Sequence: Synthetic Peptide
<400> 213
   Glu Glu Glu Glu Lys Asp Ile Glu Ala Glu Glu Arg Gly Asp Leu Gly
   1 5 10 15
   Glu Gly Gly Ala Trp Arg Leu His
   20
<210> 214
   <211> 10
   <212> PRT
   <213> Artificial Sequence
<220>
   <223> Description of Artificial Sequence: Synthetic Peptide
<400> 214
   Ser Phe Pro Trp Met Glu Ser Asp Val Thr
   1 5 10
<210> 215
   <211> 32
   <212> PRT
   <213> Artificial Sequence
<220>
   <223> Description of Artificial Sequence: Synthetic Peptide
<400> 215
   Cys Ala Ser Leu Ser Thr Cys Val Leu Gly Lys Leu Ser Gln Glu Leu
   1 5 10 15
   His Lys Leu Gln Thr Tyr Pro Arg Thr Asp Val Gly Ala Gly Thr Pro
   20 25 30
<210> 216
   <211> 32
   <212> PRT
   <213> Artificial Sequence
<220>
   <223> Description of Artificial Sequence: Synthetic Peptide
<400> 216
   Cys Ser Asn Leu Ser Thr Cys Val Leu Gly Lys Leu Ser Gln Glu Leu
   1 5 10 15
   His Lys Leu Gln Thr Tyr Pro Arg Thr Asp Val Gly Ala Gly Thr Pro
   20 25 30
<210> 217
   <211> 32
   <212> PRT
   <213> Artificial Sequence
<220>
   <223> Description of Artificial Sequence: Synthetic Peptide
<400> 217
   Cys Gly Asn Leu Ser Thr Cys Met Leu Gly Thr Tyr Thr Gln Asp Phe
   1 5 10 15
   Asn Lys Phe His Thr Phe Pro Gln Thr Ala Ile Gly Val Gly Ala Pro
   20 25 30
<210> 218
   <211> 32
   <212> PRT
   <213> Artificial Sequence
<220>
   <223> Description of Artificial Sequence: Synthetic Peptide
<400> 218
   Cys Ser Asn Leu Ser Thr Cys Val Leu Ser Ala Tyr Trp Arg Asn Leu
   1 5 10 15
   Asn Asn Phe His Arg Phe Ser Gly Met Gly Phe Gly Pro Glu Thr Pro
   20 25 30
<210> 219
   <211> 32
   <212> PRT
   <213> Artificial Sequence
<220>
   <223> Description of Artificial Sequence: Synthetic Peptide
<400> 219
   Cys Gly Asn Leu Ser Thr Cys Met Leu Gly Thr Tyr Thr Gln Asp Leu
   1 5 10 15
   Asn Lys Phe His Thr Phe Pro Gln Thr Ser Ile Gly Val Gly Ala Pro
   20 25 30
<210> 220
   <211> 32
   <212> PRT
   <213> Artificial Sequence
<220>
   <223> Description of Artificial Sequence: Synthetic Peptide
<400> 220
   Cys Ser Asn Leu Ser Thr Cys Val Leu Gly Lys Leu Ser Gln Glu Leu
   1 5 10 15
   His Lys Leu Gln Thr Tyr Pro Arg Thr Asn Thr Gly Ser Gly Thr Pro
   20 25 30
<210> 221
   <211> 25
   <212> PRT
   <213> Artificial Sequence
<220>
   <223> Description of Artificial Sequence: Synthetic Peptide
<400> 221
   Val Leu Gly Lys Leu Ser Gln Glu Leu His Lys Leu Gln Thr Tyr Pro
   1 5 10 15
   Arg Thr Asn Thr Gly Ser Gly Thr Pro
   20 25
<210> 222
   <211> 21
   <212> PRT
   <213> Artificial Sequence
<220>
   <223> Description of Artificial Sequence: Synthetic Peptide
<400> 222
   Asp Met Ser Ser Asp Leu Glu Arg Asp His Arg Pro His Val Ser Met
   1 5 10 15
   Pro Gln Asn Ala Asn
   20
<210> 223
   <211> 57
   <212> PRT
   <213> Artificial Sequence
<220>
   <223> Description of Artificial Sequence: Synthetic Peptide
<400> 223
   Ala Pro Phe Arg Ser Ala Leu Glu Ser Ser Pro Ala Asp Pro Ala Thr
   1 5 10 15
   Leu Ser Glu Asp Glu Ala Arg Leu Leu Leu Ala Ala Leu Val Gln Asp
   20 25 30
   Tyr Val Gln Met Lys Ala Ser Glu Leu Glu Gln Glu Gln Glu Arg Glu
   35 40 45
   Gly Ser Ser Leu Asp Ser Pro Arg Ser
   50 55
<210> 224
   <211> 37
   <212> PRT
   <213> Artificial Sequence
<220>
   <223> Description of Artificial Sequence: Synthetic Peptide
<400> 224
   Ser Cys Asn Thr Ala Thr Cys Val Thr His Arg Leu Ala Gly Leu Leu
   1 5 10 15
   Ser Arg Ser Gly Gly Val Val Lys Ser Asn Phe Val Pro Thr Asn Val
   20 25 30
   Gly Ser Gln Ala Phe
   35
<210> 225
   <211> 19
   <212> PRT
   <213> Artificial Sequence
<220>
   <223> Description of Artificial Sequence: Synthetic Peptide
<400> 225
   Ser Gly Gly Val Val Lys Asn Asn Phe Val Pro Thr Asn Val Gly Ser
   1 5 10 15
   Lys Ala Phe

<210> 226
   <211> 19
   <212> PRT
   <213> Artificial Sequence
<220>
   <223> Description of Artificial Sequence: Synthetic Peptide
<400> 226
   Ser Gly Gly Val Val Lys Asn Asn Phe Val Pro Thr Asn Val Gly Ser
   1 5 10 15
   Lys Ala Phe
<210> 227
   <211> 15
   <212> PRT
   <213> Artificial Sequence
<220>
   <223> Description of Artificial Sequence: Synthetic Peptide
<400> 227
   Val Lys Asn Asn Phe Val Pro Thr Asn Val Gly Ser Lys Ala Phe
   1 5 10 15
<210> 228
   <211> 37
   <212> PRT
   <213> Artificial Sequence
<220>
   <223> Description of Artificial Sequence: Synthetic Peptide
<400> 228
   Ser Cys Asn Thr Ala Thr Cys Val Thr His Arg Leu Ala Gly Leu Leu
   1 5 10 15
   Ser Arg Ser Gly Gly Val Val Lys Asp Asn Phe Val Pro Thr Asn Val
   20 25 30
   Gly Ser Lys Ala Phe
   35
<210> 229
   <211> 37
   <212> PRT
   <213> Artificial Sequence
<220>
   <223> Description of Artificial Sequence: Synthetic Peptide
<400> 229
   Ala Cys Asn Thr Ala Thr Cys Val Thr His Arg Leu Ala Asp Phe Leu
   1 5 10 15
   Ser Arg Ser Gly Gly Val Gly Lys Asn Asn Phe Val Pro Thr Asn Val
   20 25 30
   Gly Ser Lys Ala Phe
   35
<210> 230
   <211> 37
   <212> PRT
   <213> Artificial Sequence
<220>
   <223> Description of Artificial Sequence: Synthetic Peptide
<400> 230
   Ala Cys Asp Thr Ala Thr Cys Val Thr His Arg Leu Ala Gly Leu Leu
   1 5 10 15
   Ser Arg Ser Gly Gly Val Val Lys Asn Asn Phe Val Pro Thr Asn Val
   20 25 30
   Gly Ser Lys Ala Phe
   35
<210> 231
   <211> 7
   <212> PRT
   <213> Artificial Sequence
<220>
   <223> Description of Artificial Sequence: Synthetic Peptide
<400> 231
   Cys Gly Asn Leu Ser Thr Cys
   1 5
<210> 232
   <211> 16
   <212> PRT
   <213> Artificial Sequence
<220>
   <223> Description of Artificial Sequence: Synthetic Peptide
<400> 232
   Asp Met Ala Lys Asp Leu Glu Thr Asn His His Pro Tyr Phe Gly Asn
   1 5 10 15
<210> 233
   <211> 19
   <212> PRT
   <213> Artificial Sequence
<220>
   <223> Description of Artificial Sequence: Synthetic Peptide
<400> 233
   Ala Cys Asp Thr Ala Thr Cys Val Thr His Arg Leu Ala Gly Leu Leu
   1 5 10 15
   Ser Arg Ser
<210> 234
   <211> 18
   <212> PRT
   <213> Artificial Sequence
<220>
   <223> Description of Artificial Sequence: Synthetic Peptide
<400> 234
   Gly Gly Val Val Lys Asn Asn Phe Val Pro Thr Asn Val Gly Ser Lys
   1 5 10 15
   Ala Phe
<210> 235
   <211> 30
   <212> PRT
   <213> Artificial Sequence
<220>
   <223> Description of Artificial Sequence: Synthetic Peptide
<400> 235
   Val Thr His Arg Leu Ala Gly Leu Leu Ser Arg Ser Gly Gly Val Val
   1 5 10 15
   Lys Asn Asn Phe Val Pro Thr Asn Val Gly Ser Lys Ala Phe
   20 25 30
<210> 236
   <211> 37
   <212> PRT
   <213> Artificial Sequence
<220>
   <223> Description of Artificial Sequence: Synthetic Peptide
<400> 236
   Ala Cys Asn Thr Ala Thr Cys Val Thr His Arg Leu Ala Gly Leu Leu
   1 5 10 15
   Ser Arg Ser Gly Gly Met Val Lys Ser Asn Phe Val Pro Thr Asn Val
   20 25 30
   Gly Ser Lys Ala Phe
   35
<210> 237
   <211> 37
   <212> PRT
   <213> Artificial Sequence
<220>
   <223> Description of Artificial Sequence: Synthetic Peptide
<400> 237
   Ser Cys Asn Thr Ala Thr Cys Val Thr His Arg Leu Ala Gly Leu Leu
   1 5 10 15
   Ser Arg Ser Gly Gly Val Val Lys Asp Asn Phe Val Pro Thr Asn Val
   20 25 30
   Gly Ser Glu Ala Phe
   35
<210> 238
   <211> 37
   <212> PRT
   <213> Artificial Sequence
<220>
   <223> Description of Artificial Sequence: Synthetic Peptide
<400> 238
   Ser Cys Asn Thr Ala Thr Cys Val Thr His Arg Leu Ala Gly Leu Leu
   1 5 10 15
   Ser Arg Ser Gly Gly Val Val Lys Asp Asn Phe Val Pro Thr Asn Val
   20 25 30
   Gly Ser Glu Ala Phe
   35
<210> 239
   <211> 30
   <212> PRT
   <213> Artificial sequence
<220>
   <223> Description of Artificial Sequence: Synthetic Peptide
<400> 239
   Val Thr His Arg Leu Ala Gly Leu Leu Ser Arg Ser Gly Gly Val Val
   1 5 10 15
   Lys Asp Asn Phe Val Pro Thr Asn Val Gly Ser Glu Ala Phe
   20 25 30
<210> 240
   <211> 9
   <212> PRT
   <213> Artificial Sequence
<220>
   <223> Description of Artificial Sequence: Synthetic Peptide
<400> 240
   Pro Thr Asn Val Gly Ser Glu Ala Phe
   1 5
<210> 241
   <211> 8
   <212> PRT
   <213> Artificial Sequence
<220>
   <223> Description of Artificial Sequence: Synthetic Peptide
<400> 241
   Thr Asn Val Gly Ser Glu Ala Phe
   1 5
<210> 242
   <211> 7
   <212> PRT
   <213> Artificial Sequence
<220>
   <223> Description of Artificial Sequence: Synthetic Peptide
<400> 242
   Asn Val Gly Ser Glu Ala Phe
   1 5
<210> 243
   <211> 6
   <212> PRT
   <213> Artificial Sequence
<220>
   <223> Description of Artificial Sequence: Synthetic Peptide
<400> 243
   Val Gly Ser Glu Ala Phe
   1 5
<210> 244
   <211> 6
   <212> PRT
   <213> Artificial Sequence
<220>
   <223> Description of Artificial Sequence: Synthetic Peptide
<400> 244
   Val Gly Ser Glu Ala Phe
   1 5
<210> 245
   <211> 4
   <212> PRT
   <213> Artificial Sequence
<220>
   <223> Description of Artificial Sequence: Synthetic Peptide
<400> 245
   Ser Glu Ala Phe
   1
<210> 246
   <211> 37
   <212> PRT
   <213> Artificial Sequence
<220>
   <223> Description of Artificial Sequence: Synthetic Peptide
<400> 246
   Ala Cys Asp Thr Ala Thr Cys Val Thr His Arg Leu Ala Gly Leu Leu
   1 5 10 15
   Ser Arg Ser Gly Gly Val Val Lys Asn Asn Phe Val Pro Thr Asn Val
   20 25 30
   Gly Ser Lys Ala Phe
   35
<210> 247
   <211> 30
   <212> PRT
   <213> Artificial Sequence
<220>
   <223> Description of Artificial Sequence: Synthetic Peptide
<400> 247
   Ser Asn Leu Ser Thr Val Leu Gly Lys Leu Ser Gln Glu Leu His Lys
   1 5 10 15
   Leu Gln Thr Tyr Pro Arg Thr Asp Val Gly Ala Gly Thr Pro
   20 25 30
<210> 248
   <211> 38
   <212> PRT
   <213> Artificial Sequence
<220>
   <223> Description of Artificial Sequence: Synthetic Peptide
<400> 248
   Tyr Ala Cys Asp Thr Ala Thr Cys Val Thr His Arg Leu Ala Gly Leu
   1 5 10 15
   Leu Ser Arg Ser Gly Gly Val Val Lys Asn Asn Phe Val Pro Thr Asn
   20 25 30
   Val Gly Ser Lys Ala Phe
   35
<210> 249
   <211> 38
   <212> PRT
   <213> Artificial Sequence
<220>
   <223> Description of Artificial Sequence: Synthetic Peptide
<400> 249
   Tyr Ala Cys Asn Thr Ala Thr Cys Val Thr His Arg Leu Ala Gly Leu
   1 5 10 15
   Leu Ser Arg Ser Gly Gly Met Val Lys Ser Asn Phe Val Pro Thr Asn
   20 25 30
   Val Gly Ser Lys Ala Phe
   35
<210> 250
   <211> 11
   <212> PRT
   <213> Artificial Sequence
<220>
   <223> Description of Artificial Sequence: Synthetic Peptide
<400> 250
   Tyr Val Pro Thr Asn Val Gly Ser Glu Ala Phe
   1 5 10
<210> 251
   <211> 38
   <212> PRT
   <213> Artificial Sequence
<220>
   <223> Description of Artificial Sequence: Synthetic Peptide
<400> 251
   Tyr Ser Cys Asn Thr Ala Thr Cys Val Thr His Arg Leu Ala Gly Leu
   1 5 10 15
   Leu Ser Arg Ser Gly Gly Val Val Lys Asp Asn Phe Val Pro Thr Asn
   20 25 30
   Val Gly Ser Glu Ala Phe
   35
<210> 252
   <211> 16
   <212> PRT
   <213> Artificial Sequence
<220>
   <223> Description of Artificial Sequence: Synthetic Peptide
<400> 252
   Tyr Val Lys Asp Asn Phe Val Pro Thr Asn Val Gly Ser Glu Ala Phe
   1 5 10 15
<210> 253
   <211> 37
   <212> PRT
   <213> Artificial Sequence
<220>
   <223> Description of Artificial Sequence: Synthetic Peptide
<400> 253
   Ser Cys Asn Thr Ala Thr Cys Val Thr His Arg Leu Ala Gly Leu Leu
   1               5                   10                  15
   Ser Arg Ser Gly Gly Val Val Lys Asp Asn Phe Val Pro Thr Asn Val
               20                  25                  30
   Gly Ser Glu Ala Phe
           35
<210> 254
   <211> 46
   <212> PRT
   <213> Artificial Sequence
<220>
   <223> Description of Artificial Sequence: Synthetic Peptide
<400> 254
   Ala Pro Leu Ala Pro Arg Asp Ala Gly Ser Gln Arg Pro Arg Lys Lys
   1               5                   10                  15
   Glu Asp Asn Val Leu Val Glu Ser His Glu Lys Ser Leu Gly Glu Ala
               20                  25                  30
   Asp Lys Ala Asp Val Asn Val Leu Thr Lys Ala Lys Ser Gln
           35                  40                  45
<210> 255
   <211> 32
   <212> PRT
   <213> Artificial Sequence
<220>
   <223> Description of Artificial Sequence: Synthetic Peptide
<400> 255
   Lys Lys Glu Asp Asn Val Leu Val Glu Ser His Glu Lys Ser Leu Gly
   1               5                   10                  15
   Glu Ala Asp Lys Ala Asp Val Asp Val Leu Thr Lys Ala Lys Ser Gln
               20                  25                  30
<210> 256
   <211> 84
   <212> PRT
   <213> Artificial Sequence
<220>
   <223> Description of Artificial Sequence: Synthetic Peptide
<400> 256
   Ser Val Ser Glu Ile Gln Leu Met His Asn Leu Gly Lys His Leu Asn
   1 5 10 15
   Ser Met Glu Arg Val Glu Trp Leu Arg Lys Lys Leu Gln Asp Val His
   20 25 30
   Asn Phe Val Ala Leu Gly Ala Pro Leu Ala Pro Arg Asp Ala Gly Ser
   35 40 45
   Gln Arg Pro Arg Lys Lys Glu Asp Asn Val Leu Val Glu Ser His Glu
   50 55 60
   Lys Ser Leu Gly Glu Ala Asp Lys Ala Asp Val Asn Val Leu Thr Lys
   65 70 75 80
   Ala Lys Ser Gln
<210> 257
   <211> 21
   <212> PRT
   <213> Artificial Sequence
<220>
   <223> Description of Artificial Sequence: Synthetic Peptide
<400> 257
   Glu Lys Ser Leu Gly Glu Ala Asp Lys Ala Asp Val Asn Val Leu Thr
   1 5 10 15
   Lys Ala Lys Ser Gln
   20
<210> 258
   <211> 34
   <212> PRT
   <213> Artificial sequence
<220>
   <223> Description of Artificial Sequence: Synthetic Peptide
<400> 258
   Ser Val Ser Glu Ile Gln Leu Asn His Asn Leu Gly Lys His Leu Asn
   1 5 10 15
   Ser Leu Glu Arg Val Glu Trp Leu Arg Lys Lys Leu Gln Asp Val His
   20 25 30
   Asn Phe
<210> 259
   <211> 34
   <212> PRT
   <213> Artificial Sequence
<220>
   <223> Description of Artificial Sequence: Synthetic Peptide
<400> 259
   Ser Val Ser Glu Cys Gln Leu Met His Asn Leu Gly Lys His Leu Asn
   1 5 10 15
   Ser Met Glu Arg Val Glu Trp Leu Arg Lys Lys Cys Gln Asp Val His
   20 25 30
   Asn Phe
<210> 260
   <211> 40
   <212> PRT
   <213> Artificial Sequence
<220>
   <223> Description of Artificial sequence: Synthetic Peptide
<400> 260
   Ala Val Ser Glu His Gln Leu Leu His Asp Lys Gly Lys Ser Ile Gln
   1 5 10 15
   Asp Leu Arg Arg Arg Phe Phe Leu His His Leu Ile Ala Glu Ile His
   20 25 30
   Thr Ala Glu Ile Arg Ala Thr Ser
   35 40
<210> 261
   <211> 34
   <212> PRT
   <213> Artificial Sequence
<220>
   <223> Description of Artificial Sequence: Synthetic Peptide
<400> 261
   Ser Val Ser Glu Ile Gln Leu Met His Asn Leu Gly Lys His Leu Asn
   1 5 10 15
   Ser Leu Glu Arg Val Glu Trp Leu Arg Lys Lys Leu Gln Asp Val His
   20 25 30
   Asn Phe
<210> 262
   <211> 32
   <212> PRT
   <213> Artificial Sequence
<220>
   <223> Description of Artificial Sequence: Synthetic Peptide
<400> 262
   Ala Val Ser Glu Ile Gln Phe His Asn Leu Gly Lys His Leu Ser Ser
   1 5 10 15
   Glu Arg Val Glu Trp Leu Arg Lys Lys Leu Gln Asp Val His Asn Tyr
   20 25 30
<210> 263
   <211> 30
   <212> PRT
   <213> Artificial Sequence
<220>
   <223> Description of Artificial Sequence: Synthetic Peptide
<400> 263
   Ser Glu Ile Gln Phe His Asn Leu Gly Lys His Leu Ser Ser Glu Arg
   1 5 10 15
   Val Glu Trp Leu Arg Lys Lys Leu Gln Asp Val His Asn Tyr
   20 25 30
<210> 264
   <211> 32
   <212> PRT
   <213> Artificial Sequence
<220>
   <223> Description of Artificial Sequence: Synthetic Peptide
<400> 264
   Ser Val Ser Glu Ile Gln Leu His Asn Leu Gly Lys His Leu Asn Ser
   1 5 10 15
   Glu Arg Val Glu Trp Leu Arg Lys Lys Leu Gln Asp Val His Asn Tyr
   20 25 30
<210> 265
   <211> 32
   <212> PRT
   <213> Artificial Sequence
<220>
   <223> Description of Artificial Sequence: Synthetic Peptide
<400> 265
   Ser Val Ser Glu Ile Gln Leu His Asn Leu Gly Lys His Leu Asn Ser
   1 5 10 15
   Glu Arg Val Glu Trp Leu Arg Lys Lys Leu Gln Asp Val His Asn Tyr
   20 25 30
<210> 266
   <211> 30
   <212> PRT
   <213> Artificial Sequence
<220>
   <223> Description of Artificial Sequence: Synthetic Peptide
<400> 266
   Ser Glu Ile Gln Leu His Asn Leu Gly Lys His Leu Asn Ser Glu Arg
   1 5 10 15
   Val Glu Trp Leu Arg Lys Lys Leu Gln Asp Val His Asn Tyr
   20 25 30
<210> 267
   <211> 26
   <212> PRT
   <213> Artificial Sequence
<220>
   <223> Description of Artificial Sequence: Synthetic Peptide
<400> 267
   Phe His Asn Leu Gly Lys His Leu Ser Ser Glu Arg Val Glu Trp Leu
   1 5 10 15
   Arg Lys Lys Leu Gln Asp Val His Asn Tyr
   20 25
<210> 268
   <211> 32
   <212> PRT
   <213> Artificial Sequence
<220>
   <223> Description of Artificial Sequence: Synthetic Peptide
<400> 268
   Ala Val Ser Glu Ile Gln Leu His Asn Leu Gly Lys His Leu Ala Ser
   1 5 10 15
   Val Glu Arg Gln Trp Leu Arg Lys Lys Leu Gln Asp Val His Asn Tyr
   20 25 30
<210> 269
   <211> 25
   <212> PRT
   <213> Artificial Sequence
<220>
   <223> Description of Artificial Sequence: Synthetic Peptide
<400> 269
   Arg Asp Ala Gly Ser Gln Arg Pro Arg Lys Lys Glu Asp Asn Val Leu
   1 5 10 15
   Val Glu Ser His Glu Lys Ser Leu Gly
   20 25
<210> 270
   <211> 32
   <212> PRT
   <213> Artificial Sequence
<220>
   <223> Description of Artificial Sequence: Synthetic Peptide
<400> 270
   Ser Glu Ile Gln Phe Met His Asn Leu Gly Lys His Leu Ser Ser Met
   1 5 10 15
   Glu Arg Val Glu Trp Leu Arg Lys Lys Leu Gln Asp Val His Asn Phe
   20 25 30
<210> 271
   <211> 31
   <212> PRT
   <213> Artificial Sequence
<220>
   <223> Description of Artificial Sequence: Synthetic Peptide
<400> 271
   Ser Val Ser Glu Ile Gln Leu Met His Asn Leu Gly Lys His Leu Asn
   1 5 10 15
   Ser Met Glu Arg Val Glu Trp Leu Arg Lys Lys Leu Gln Asp Val
   20 25 30
<210> 272
   <211> 34
   <212> PRT
   <213> Artificial Sequence
<220>
   <223> Description of Artificial Sequence: Synthetic Peptide
<400> 272
   Ser Val Ser Glu Ile Gln Leu Met His Asn Leu Gly Lys His Leu Asn
   1 5 10 15
   Ser Met Glu Arg Val Glu Trp Leu Arg Lys Lys Leu Gln Asp Val His
   20 25 30
   Asn Phe
<210> 273
   <211> 22
   <212> PRT
   <213> Artificial Sequence
<220>
   <223> Description of Artificial Sequence: Synthetic Peptide
<400> 273
   Lys His Leu Asn Ser Met Glu Arg Val Glu Trp Leu Arg Lys Lys Leu
   1 5 10 15
   Gln Asp Val His Asn Phe
   20
<210> 274
   <211> 34
   <212> PRT
   <213> Artificial Sequence
<220>
   <223> Description of Artificial Sequence: Synthetic Peptide
<400> 274
   Ala Val Ser Glu Ile Gln Leu Met His Asn Leu Gly Lys His Leu Ala
   1 5 10 15
   Ser Val Glu Arg Met Gln Trp Leu Arg Lys Lys Leu Gln Asp Val His
   20 25 30
   Asn Phe
<210> 275
   <211> 38
   <212> PRT
   <213> Artificial Sequence
<220>
   <223> Description of Artificial Sequence: Synthetic Peptide
<400> 275
   Ser Val Ser Glu Ile Gln Leu Met His Asn Leu Gly Lys His Leu Asn
   1 5 10 15
   Ser Met Glu Arg Val Glu Trp Leu Arg Lys Lys Leu Gln Asp Val His
   20 25 30
   Asn Phe Val Ala Leu Gly
   35
<210> 276
   <211> 44
   <212> PRT
   <213> Artificial Sequence
<220>
   <223> Description of Artificial Sequence: Synthetic Peptide
<400> 276
   Ser Val Ser Glu Ile Gln Leu Met His Asn Leu Gly Lys His Leu Asn
   1 5 10 15
   Ser Met Glu Arg Val Glu Trp Leu Arg Lys Lys Leu Gln Asp Val His
   20 25 30
   Asn Phe Val Ala Leu Gly Ala Pro Leu Ala Pro Arg
   35 40
<210> 277
   <211> 21
   <212> PRT
   <213> Artificial Sequence
<220>
   <223> Description of Artificial Sequence: Synthetic Peptide
<400> 277
   Leu Gln Asp Val His Asn Phe Val Ala Leu Gly Ala Pro Leu Ala Pro
   1 5 10 15
   Arg Asp Ala Gly Ser
   20
<210> 278
   <211> 30
   <212> PRT
   <213> Artificial Sequence
<220>
   <223> Description of Artificial Sequence: Synthetic Peptide
<400> 278
   Ala Pro Leu Ala Pro Arg Asp Ala Gly Ser Gln Arg Pro Arg Lys Lys
   1 5 10 15
   Glu Asp Asn Val Leu Val Glu Ser His Glu Lys Ser Leu Gly
   20 25 30
<210> 279
   <211> 46
   <212> PRT
   <213> Artificial Sequence
<220>
   <223> Description of Artificial Sequence: Synthetic Peptide
<400> 279
   Ala Pro Leu Ala Pro Arg Asp Ala Gly Ser Gln Arg Pro Arg Lys Lys
   1 5 10 15
   Glu Asp Asn Val Leu Val Glu Ser His Glu Lys Ser Leu Gly Glu Ala
   20 25 30
   Asp Lys Ala Asp Val Asn Val Leu Thr Lys Ala Lys Ser Gln
   35 40 45
<210> 280
   <211> 32
   <212> PRT
   <213> Artificial Sequence
<220>
   <223> Description of Artificial Sequence: Synthetic Peptide
<400> 280
   Lys Lys Glu Asp Asn Val Leu Val Glu Ser His Glu Lys Ser Leu Gly
   1 5 10 15
   Glu Ala Asp Lys Ala Asp Val Asp Val Leu Thr Lys Ala Lys Ser Gln
   20 25 30
<210> 281
   <211> 16
   <212> PRT
   <213> Artificial Sequence
<220>
   <223> Description of Artificial Sequence: Synthetic Peptide
<400> 281
   Glu Ala Asp Lys Ala Asp Val Asn Val Leu Thr Lys Ala Lys Ser Gln
   1 5 10 15
<210> 282
   <211> 15
   <212> PRT
   <213> Artificial Sequence
<220>
   <223> Description of Artificial Sequence: Synthetic Peptide
<400> 282
   Ala Asp Lys Ala Asp Val Asn Val Leu Thr Lys Ala Lys Ser Gln
   1 5 10 15
<210> 283
   <211> 36
   <212> PRT
   <213> Artificial Sequence
<220>
   <223> Description of Artificial Sequence: Synthetic Peptide
<400> 283
   Tyr Pro Ile Lys Pro Glu Ala Pro Gly Glu Asp Ala Ser Pro Glu Glu
   1 5 10 15
   Leu Asn Arg Tyr Tyr Ala Ser Leu Arg His Tyr Leu Asn Leu Val Thr
   20 25 30
   Arg Pro Arg Tyr
   35
<210> 284
   <211> 41
   <212> PRT
   <213> Artificial Sequence
<220>
   <223> Description of Artificial Sequence: Synthetic Peptide
<400> 284
   Tyr Ala Val Ser Glu His Gln Leu Leu His Asp Lys Gly Lys Ser Ile
   1 5 10 15
   Gln Asp Leu Arg Arg Arg Phe Phe Leu His His Leu Ile Ala Glu Ile
   20 25 30
   His Thr Ala Glu Ile Arg Ala Thr Ser
   35 40
<210> 285
   <211> 45
   <212> PRT
   <213> Artificial Sequence
<220>
   <223> Description of Artificial Sequence: Synthetic Peptide
<400> 285
   Tyr Ser Val Ser Glu Ile Gln Leu Met His Asn Leu Gly Lys His Leu
   1 5 10 15
   Asn Ser Met Glu Arg Val Glu Trp Leu Arg Lys Lys Leu Gln Asp Val
   20 25 30
   His Asn Phe Val Ala Leu Gly Ala Pro Leu Ala Pro Arg
   35 40 45
<210> 286
   <211> 35
   <212> PRT
   <213> Artificial Sequence
<220>
   <223> Description of Artificial Sequence: Synthetic Peptide
<400> 286
   Tyr Ser Val Ser Glu Ile Gln Leu Met His Asn Leu Gly Lys His Leu
   1 5 10 15
   Asn Ser Met Glu Arg Val Glu Trp Leu Arg Lys Lys Leu Gln Asp Val
   20 25 30
   His Asn Phe
   35
<210> 287
   <211> 34
   <212> PRT
   <213> Artificial Sequence
<220>
   <223> Description of Artificial Sequence: Synthetic Peptide
<400> 287
   Tyr Val Ser Glu Ile Gln Leu Met His Asn Leu Gly Lys His Leu Asn
   1 5 10 15
   Ser Met Glu Arg Val Glu Trp Leu Arg Lys Lys Leu Gln Asp Val His
   20 25 30
   Asn Phe
<210> 288
   <211> 22
   <212> PRT
   <213> Artificial Sequence
<220>
   <223> Description of Artificial Sequence: Synthetic Peptide
<400> 288
   Tyr Leu Gln Asp Val His Asn Phe Val Ala Leu Gly Ala Pro Leu Ala
   1 5 10 15
   Pro Arg Asp Ala Gly Ser
   20
<210> 289
   <211> 28
   <212> PRT
   <213> Artificial Sequence
<220>
   <223> Description of Artificial Sequence: Synthetic Peptide
<400> 289
   Phe Met His Asn Leu Gly Lys His Leu Ser Ser Met Glu Arg Val Glu
   1               5                   10                  15
   Trp Leu Arg Lys Lys Leu Gln Asp Val His Asn Tyr
               20                  25
<210> 290
   <211> 26
   <212> PRT
   <213> Artificial Sequence
<220>
   <223> Description of Artificial Sequence: Synthetic Peptide
<400> 290
   Tyr Arg Asp Ala Gly Ser Gln Arg Pro Arg Lys Lys Glu Asp Asn Val
   1               5                   10                  15
   Leu Val Glu Ser His Glu Lys Ser Leu Gly
               20                  25
<210> 291
   <211> 33
   <212> PRT
   <213> Artificial Sequence
<220>
   <223> Description of Artificial Sequence: Synthetic Peptide
<400> 291
   Tyr Lys Lys Glu Asp Asn Val Leu Val Glu Ser His Glu Lys Ser Leu
   1               5                   10                  15
   Gly Glu Ala Asp Lys Ala Asp Val Asn Val Leu Thr Lys Ala Lys Ser
               20                  25                  30
   Gln
<210> 292
   <211> 22
   <212> PRT
   <213> Artificial Sequence
<220>
   <223> Description of Artificial Sequence: Synthetic Peptide
<400> 292
   Tyr Glu Lys Ser Leu Gly Glu Ala Asp Lys Ala Asp Val Asn Val Leu
   1 5 10 15
   Thr Lys Ala Lys Ser Gln
   20

<210> 293
   <211> 34
   <212> PRT
   <213> Artificial Sequence
<220>
   <223> Description of Artificial Sequence: Synthetic Peptide
<400> 293
   Ala Val Ser Glu His Gln Leu Leu His Asp Lys Gly Lys Ser Ile Gln
   1 5 10 15
   Asp Leu Arg Arg Arg Phe Phe Leu His His Leu Ile Ala Glu Ile His
   20 25 30
   Thr Ala
<210> 294
   <211> 5
   <212> PRT
   <213> Artificial Sequence
<220>
   <223> Description of Artificial sequence: Synthetic Peptide
<400> 294
   Ala Arg Ser Ala Trp
   1 5
<210> 295
   <211> 34
   <212> PRT
   <213> Artificial Sequence
<220>
   <223> Description of Artificial Sequence: Synthetic Peptide
<400> 295
   Ala Val Ser Glu His Gln Leu Leu His Asp Lys Gly Lys Ser Ile Gln
   1 5 10 15
   Asp Leu Arg Arg Arg Phe Phe Leu His His Leu Ile Ala Glu Ile His
   20 25 30
   Thr Ala
<210> 296
   <211> 33
   <212> PRT
   <213> Artificial Sequence
<220>
   <223> Description of Artificial Sequence: Synthetic Peptide
<400> 296
   Val Ser Glu His Gln Leu Leu His Asp Lys Gly Lys Ser Ile Gln Asp
   1 5 10 15
   Leu Arg Arg Arg Phe Phe Leu His His Leu Ile Ala Glu Ile His Thr
   20 25 30
   Ala
<210> 297
   <211> 35
   <212> PRT
   <213> Artificial Sequence
<220>
   <223> Description of Artificial Sequence: Synthetic Peptide
<400> 297
   Tyr Ala Val Ser Glu His Gln Leu Leu His Asp Lys Gly Lys Ser Ile
   1 5 10 15
   Gln Asp Leu Arg Arg Arg Phe Phe Leu His His Leu Ile Ala Glu Ile
   20 25 30
   His Thr Ala
   35
<210> 298
   <211> 34
   <212> PRT
   <213> Artificial Sequence
<220>
   <223> Description of Artificial Sequence: Synthetic Peptide
<400> 298
   Ala Val Ser Glu His Gln Leu Leu His Asp Lys Gly Lys Ser Ile Gln
   1 5 10 15
   Asp Leu Arg Arg Arg Phe Phe Leu His His Leu Ile Ala Glu Ile His
   20 25 30
   Thr Tyr
<210> 299
   <211> 37
   <212> PRT
   <213> Artificial Sequence
<220>
   <223> Description of Artificial Sequence: Synthetic Peptide
<400> 299
   Ala Val Ser Glu His Gln Leu Leu His Asp Lys Gly Lys Ser Ile Gln
   1 5 10 15
   Asp Leu Arg Arg Arg Phe Phe Leu His His Leu Ile Ala Glu Ile His
   20 25 30
   Thr Ala Glu Ile Arg
   35
<210> 300
   <211> 28
   <212> PRT
   <213> Artificial Sequence
<220>
   <223> Description of Artificial Sequence: Synthetic Peptide
<400> 300
   Leu Leu His Asp Lys Gly Lys Ser Ile Gln Asp Leu Arg Arg Arg Phe
   1 5 10 15
   Phe Leu His His Leu Ile Ala Glu Ile His Thr Ala
   20 25
<210> 301
   <211> 27
   <212> PRT
   <213> Artificial Sequence
<220>
   <223> Description of Artificial Sequence: Synthetic Peptide
<400> 301
   Ala Thr Ser Glu Val Ser Pro Asn Ser Lys Pro Ser Pro Asn Thr Lys
   1 5 10 15
   Asn His Pro Val Arg Phe Gly Ser Asp Asp Glu
   20 25
<210> 302
   <211> 20
   <212> PRT
   <213> Artificial Sequence
<220>
   <223> Description of Artificial Sequence: Synthetic Peptide
<400> 302
   Tyr Leu Thr Gln Glu Thr Asn Lys Val Glu Thr Tyr Lys Glu Gln Pro
   1 5 10 15
   Leu Lys Thr Pro
   20
<210> 303
   <211> 5
   <212> PRT
   <213> Artificial Sequence
<220>
   <223> Description of Artificial Sequence: Synthetic Peptide
<400> 303
   Thr Arg Ser Ala Trp
   1 5
<210> 304
   <211> 5
   <212> PRT
   <213> Artificial Sequence
<220>
   <223> Description of Artificial Sequence: Synthetic Peptide
<400> 304
   Thr Arg Ser Ala Trp
   1 5
<210> 305
   <211> 32
   <212> PRT
   <213> Artificial Sequence
<220>
   <223> Description of Artificial Sequence: Synthetic Peptide
<400> 305
   Thr Arg Ser Ala Trp Leu Asp Ser Gly Val Thr Gly Ser Gly Leu Glu
   1 5 10 15
   Gly Asp His Leu Ser Asp Thr Ser Thr Thr Ser Leu Glu Leu Asp Ser
   20 25 30
<210> 306
   <211> 33
   <212> PRT
   <213> Artificial Sequence
<220>
   <223> Description of Artificial Sequence: synthetic Peptide
<400> 306
   Ser Ala Trp Leu Asp Ser Gly Val Thr Gly Ser Gly Leu Glu Gly Asp
   1 5 10 15
   His Leu Ser Asp Thr Ser Thr Thr Ser Leu Glu Leu Asp Ser Arg Arg
   20 25 30
   His
<210> 307
   <211> 34
   <212> PRT
   <213> Artificial Sequence
<220>
   <223> Description of Artificial Sequence: Synthetic Peptide
<400> 307
   Thr Ala Leu Leu Trp Gly Leu Lys Lys Lys Lys Glu Asn Asn Arg Arg
   1 5 10 15
   Thr His His Met Gln Leu Met Ile Ser Leu Phe Lys Ser Pro Leu Leu
   20 25 30
   Leu Leu
<210> 308
   <211> 31
   <212> PRT
   <213> Artificial Sequence
<220>
   <223> Description of Artificial Sequence: Synthetic Peptide
<400> 308
<210> 309
   <211> 27
   <212> PRT
   <213> Artificial Sequence
<220>
   <223> Description of Artificial Sequence: Synthetic Peptide
<400> 309
   Thr Ala Leu Leu Trp Gly Leu Lys Lys Lys Lys Gly Lys Gln Gln Lys
   1 5 10 15
   Asn Thr Ser Tyr Ala Thr Asn Asp Leu Ile Ile
   20 25
<210> 310
   <211> 30
   <212> PRT
   <213> Artificial Sequence
<220>
   <223> Description of Artificial Sequence: Synthetic Peptide
<400> 310
   Ala Thr Gln Arg Leu Ala Asn Phe Leu Val His Ser Ser Asn Asn Phe
   1 5 10 15
   Gly Ala Ile Leu Ser Ser Thr Asn Val Gly Ser Asn Thr Tyr
   20 25 30
<210> 311
   <211> 30
   <212> PRT
   <213> Artificial Sequence
<220>
   <223> Description of Artificial Sequence: Synthetic Peptide
<400> 311
   Ala Thr Gln Arg Leu Ala Asn Phe Leu Val Arg Ser Ser Asn Asn Leu
   1 5 10 15
   Gly Pro Val Leu Pro Pro Thr Asn Val Gly Ser Asn Thr Tyr
   20 25 30
<210> 312
   <211> 25
   <212> PRT
   <213> Artificial Sequence
<220>
   <223> Description of Artificial Sequence: Synthetic Peptide
<400> 312
   Val Leu Gly Lys Leu Ser Gln Glu Leu His Lys Leu Gln Thr Tyr Pro
   1 5 10 15
   Arg Thr Asn Thr Gly Ser Asn Thr Tyr
   20 25
<210> 313
   <211> 24
   <212> PRT
   <213> Artificial Sequence
<220>
   <223> Description of Artificial Sequence: Synthetic Peptide
<400> 313
   Leu Gly Arg Leu Ser Gln Glu Leu His Arg Leu Gln Thr Tyr Pro Arg
   1 5 10 15
   Thr Asn Thr Gly Ser Asn Thr Tyr
   20
<210> 314
   <211> 25
   <212> PRT
   <213> Artificial Sequence
<220>
   <223> Description of Artificial Sequence: Synthetic Peptide
<400> 314
   Ala Thr Gln Arg Leu Ala Asn Glu Leu Val Arg Leu Gln Thr Tyr Pro
   1 5 10 15
   Arg Thr Asn Val Gly Ser Asn Thr Tyr
   20 25
<210> 315
   <211> 29
   <212> PRT
   <213> Artificial Sequence
<220>
   <223> Description of Artificial Sequence: Synthetic Peptide
<400> 315
   Thr Gln Arg Leu Ala Asn Phe Leu Val His Ser Ser Asn Asn Phe Gly
   1 5 10 15
   Ala Ile Leu Ser Ser Thr Asn Val Gly Ser Asn Thr Tyr
   20 25
<210> 316
   <211> 37
   <212> PRT
   <213> Artificial Sequence
<220>
   <223> Description of Artificial Sequence: Synthetic Peptide
<400> 316
   Lys Cys Asn Thr Ala Thr Cys Ala Thr Gln Arg Leu Ala Asn Phe Leu
   1 5 10 15
   Ile Arg Ser Ser Asn Asn Leu Gly Ala Ile Leu Ser Pro Thr Asn Val
   20 25 30
   Gly Ser Asn Thr Tyr
   35
<210> 317
   <211> 37
   <212> PRT
   <213> Artificial Sequence
<220>
   <223> Description of Artificial Sequence: Synthetic Peptide
<400> 317
<210> 318
   <211> 37
   <212> PRT
   <213> Artificial Sequence
<220>
   <223> Description of Artificial Sequence: Synthetic Peptide
<400> 318
<210> 319
   <211> 13
   <212> PRT
   <213> Artificial Sequence
<220>
   <223> Description of Artificial Sequence: Synthetic Peptide
<400> 319
   Lys Cys Asn Thr Ala Thr Cys Ala Thr Gln Arg Leu Ala
   1 5 10
<210> 320
   <211> 10
   <212> PRT
   <213> Artificial Sequence
<220>
   <223> Description of Artificial Sequence: Synthetic Peptide
<400> 320
   Ser Asn Asn Phe Gly Ala Ile Leu Ser Ser
   1 5 10
<210> 321
   <211> 37
   <212> PRT
   <213> Artificial Sequence
<220>
   <223> Description of Artificial Sequence: Synthetic Peptide
<400> 321
   Lys Cys Asn Thr Ala Thr Cys Ala Thr Gln Arg Leu Ala Asn Phe Leu
   1 5 10 15
   Val Arg Ser Ser Asn Asn Leu Gly Pro Val Leu Pro Pro Thr Asn Val
   20 25 30
   Gly Ser Asn Thr Tyr
   35
<210> 322
   <211> 30
   <212> PRT
   <213> Artificial Sequence
<220>
   <223> Description of Artificial Sequence: Synthetic Peptide
<400> 322
   Ala Thr Gln Arg Leu Ala Asn Phe Leu Val Arg Ser Ser Asn Asn Leu
   1 5 10 15
   Gly Pro Val Leu Pro Pro Thr Asn Val Gly Ser Asn Thr Tyr
   20 25 30
<210> 323
   <211> 30
   <212> PRT
   <213> Artificial Sequence
<220>
   <223> Description of Artificial Sequence: Synthetic Peptide
<400> 323
   Ala Thr Gln Arg Leu Ala Asn Phe Leu Val His Ser Ser Asn Asn Phe
   1 5 10 15
   Gly Ala Ile Leu Ser Ser Thr Asn Val Gly Ser Asn Thr Tyr
   20 25 30
<210> 324
   <211> 30
   <212> PRT
   <213> Artificial Sequence
<220>
   <223> Description of Artificial Sequence: Synthetic Peptide
<400> 324
   Ala Thr Gln Arg Leu Ala Asn Phe Leu Val Arg Ser Ser Asn Asn Leu
   1 5 10 15
   Gly Pro Val Leu Pro Pro Thr Asn Val Gly Ser Asn Thr Tyr
   20 25 30
<210> 325
   <211> 25
   <212> PRT
   <213> Artificial Sequence
<220>
   <223> Description of Artificial Sequence: Synthetic Peptide
<400> 325
   Val Leu Gly Lys Leu Ser Gln Glu Leu His Lys Leu Gln Thr Tyr Pro
   1 5 10 15
   Arg Thr Asn Thr Gly Ser Asn Thr Tyr
   20 25
<210> 326
   <211> 24
   <212> PRT
   <213> Artificial Sequence
<220>
   <223> Description of Artificial Sequence: Synthetic Peptide
<400> 326
   Leu Gly Arg Leu Ser Gln Glu Leu His Arg Leu Gln Thr Tyr Pro Arg
   1 5 10 15
   Thr Asn Thr Gly Ser Asn Thr Tyr
   20
<210> 327
   <211> 25
   <212> PRT
   <213> Artificial Sequence
<220>
   <223> Description of Artificial Sequence: Synthetic Peptide
<400> 327
   Ala Thr Gln Arg Leu Ala Asn Glu Leu Val Arg Leu Gln Thr Tyr Pro
   1 5 10 15
   Arg Thr Asn Val Gly Ser Asn Thr Tyr
   20 25
<210> 328
   <211> 29
   <212> PRT
   <213> Artificial Sequence
<220>
   <223> Description of Artificial Sequence: Synthetic Peptide
<400> 328
   Thr Gln Arg Leu Ala Asn Phe Leu Val His Ser Ser Asn Asn Phe Gly
   1 5 10 15
   Ala Ile Leu Ser Ser Thr Asn Val Gly Ser Asn Thr Tyr
   20 25
<210> 329
   <211> 37
   <212> PRT
   <213> Artificial Sequence
<220>
   <223> Description of Artificial Sequence: Synthetic Peptide
<400> 329
   Lys Cys Asn Thr Ala Thr Cys Ala Thr Gln Arg Leu Ala Asn Phe Leu
   1 5 10 15
   Ile Arg Ser Ser Asn Asn Leu Gly Ala Ile Leu Ser Pro Thr Asn Val
   20 25 30
   Gly Ser Asn Thr Tyr
   35
<210> 330
   <211> 37
   <212> PRT
   <213> Artificial Sequence
<220>
   <223> Description of Artificial Sequence: Synthetic Peptide
<400> 330
<210> 331
   <211> 37
   <212> PRT
   <213> Artificial Sequence
<220>
   <223> Description of Artificial Sequence: Synthetic Peptide
<400> 331
<210> 332
   <211> 13
   <212> PRT
   <213> Artificial Sequence
<220>
   <223> Description of Artificial Sequence: Synthetic Peptide
<400> 332
   Lys Cys Asn Thr Ala Thr Cys Ala Thr Gln Arg Leu Ala
   1 5 10
<210> 333
   <211> 10
   <212> PRT
   <213> Artificial Sequence
<220>
   <223> Description of Artificial Sequence: Synthetic Peptide
<400> 333
   Ser Asn Asn Phe Gly Ala Ile Leu Ser Ser
   1 5 10
<210> 334
   <211> 37
   <212> PRT
   <213> Artificial Sequence
<220>
   <223> Description of Artificial Sequence: Synthetic Peptide
<400> 334
   Lys Cys Asn Thr Ala Thr Cys Ala Thr Gln Arg Leu Ala Asn Phe Leu
   1 5 10 15
   Val Arg Ser Ser Asn Asn Leu Gly Pro Val Leu Pro Pro Thr Asn Val
   20 25 30
   Gly Ser Asn Thr Tyr
   35
<210> 335
   <211> 30
   <212> PRT
   <213> Artificial Sequence
<220>
   <223> Description of Artificial Sequence: Synthetic Peptide
<400> 335
   Ala Thr Gln Arg Leu Ala Asn Phe Leu Val Arg Ser Ser Asn Asn Leu
   1 5 10 15
   Gly Pro Val Leu Pro Pro Thr Asn Val Gly Ser Asn Thr Tyr
   20 25 30
<210> 336
   <211> 28
   <212> PRT
   <213> Artificial Sequence
<220>
   <223> Description of Artificial Sequence: Synthetic Peptide
<400> 336
   Ser Gln Gly Thr Phe Thr Ser Glu Tyr Ser Lys Tyr Leu Asp Ser Arg
   1 5 10 15
   Arg Ala Gln Asp Phe Val Gln Trp Leu Met Asn Thr
   20 25
<210> 337
   <211> 35
   <212> PRT
   <213> Artificial Sequence
<220>
   <223> Description of Artificial Sequence: Synthetic Peptide
<400> 337
   His Gly Glu Gly Thr Ser Asp Leu Ser Lys Gln Met Glu Glu Ala Arg
   1 5 10 15
   Leu Phe Ile Glu Trp Leu Lys Asn Gly Gly Pro Ser Ser Gly Ala Pro
   20 25 30
   Pro Pro Ser
   35
<210> 338
   <211> 29
   <212> PRT
   <213> Artificial Sequence
<220>
   <223> Description of Artificial Sequence: Synthetic Peptide
<400> 338
   His Ser Gln Gly Thr Phe Thr Ser Asp Tyr Ser Lys Tyr Leu Asp Ser
   1 5 10 15
   Arg Arg Ala Gln Asp Phe Val Gln Trp Leu Met Asn Thr
   20 25
<210> 339
   <211> 11
   <212> PRT
   <213> Artificial Sequence
<220>
   <223> Description of Artificial Sequence: Synthetic Peptide
<400> 339
   Ala Gln Asp Phe Val Gln Trp Leu Met Asn Thr
   1 5 10
<210> 340
   <211> 8
   <212> PRT
   <213> Artificial Sequence
<220>
   <223> Description of Artificial Sequence: Synthetic Peptide
<400> 340
   Phe Val Gln Trp Leu Met Asn Thr
   1 5
<210> 341
   <211> 28
   <212> PRT
   <213> Artificial Sequence
<220>
   <223> Description of Artificial Sequence: Synthetic Peptide
<210> 342
   <211> 36
   <212> PRT
   <213> Artificial Sequence
<220>
   <223> Description of Artificial Sequence: Synthetic Peptide
<400> 342
   His Asp Glu Phe Glu Arg His Ala Glu Gly Thr Phe Thr Ser Asp Val
   1 5 10 15
   Ser Ser Tyr Leu Glu Gly Gln Ala Ala Lys Glu Phe Ile Ala Trp Leu
   20 25 30
   Val Lys Gly Arg
   35
<210> 343
   <211> 37
   <212> PRT
   <213> Artificial Sequence
<220>
   <223> Description of Artificial Sequence: Synthetic Peptide
<400> 343
   His Asp Glu Phe Glu Arg His Ala Glu Gly Thr Phe Thr Ser Asp Val
   1 5 10 15
   Ser Ser Tyr Leu Glu Gly Gln Ala Ala Lys Glu Phe Ile Ala Trp Leu
   20 25 30
   Val Lys Gly Arg Gly
   35
<210> 344
   <211> 30
   <212> PRT
   <213> Artificial Sequence
<220>
   <223> Description of Artificial Sequence: Synthetic Peptide
<400> 344
   His Ala Glu Gly Thr Phe Thr Ser Asp Val Ser Ser Tyr Leu Glu Gly
   1 5 10 15
   Gln Ala Ala Lys Glu Phe Ile Ala Trp Leu Val Lys Gly Arg
   20 25 30
<210> 345
   <211> 33
   <212> PRT
   <213> Artificial Sequence
<220>
   <223> Description of Artificial Sequence: Synthetic Peptide
<400> 345
   His Ala Asp Gly Ser Phe Ser Asp Glu Met Asn Thr Ile Leu Asp Asn
   1 5 10 15
   Leu Ala Thr Arg Asp Phe Ile Asn Trp Leu Ile Gln Thr Lys Ile Thr
   20 25 30
   Asp
<210> 346
   <211> 34
   <212> PRT
   <213> Artificial Sequence
<220>
   <223> Description of Artificial Sequence: Synthetic Peptide
<400> 346
   His Ala Asp Gly Ser Phe Ser Asp Glu Met Asn Thr Ile Leu Asp Asn
   1 5 10 15
   Leu Ala Ala Arg Asp Phe Ile Asn Trp Leu Ile Gln Thr Lys Ile Thr
   20 25 30
   Asp Arg
<210> 347
   <211> 37
   <212> PRT
   <213> Artificial Sequence
<220>
   <223> Description of Artificial Sequence: Synthetic Peptide
<400> 347
   His Ser Glu Gly Thr Phe Thr Ser Asp Tyr Ser Lys Tyr Leu Asp Ser
   1 5 10 15
   Arg Arg Ala Glu Asp Phe Val Glu Trp Leu Met Asn Thr Lys Arg Asn
   20 25 30
   Lys Asn Asn Ile Ala
   35
<210> 348
   <211> 37
   <212> PRT
   <213> Artificial Sequence
<220>
   <223> Description of Artificial Sequence: Synthetic Peptide
<400> 348
   His Ser Glu Gly Thr Phe Thr Ser Asp Tyr Ser Lys Tyr Leu Asp Ser
   1               5                   10                  15
   Arg Arg Ala Glu Asp Phe Val Glu Trp Leu Met Asn Thr Lys Arg Asn
               20                  25                  30
   Lys Asn Asn Ile Ala
           35
<210> 349
   <211> 39
   <212> PRT
   <213> Artificial Sequence
<220>
   <223> Description of Artificial Sequence: Synthetic Peptide
<400> 349
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 350
   <211> 34
   <212> PRT
   <213> Artificial Sequence
<220>
   <223> Description of Artificial Sequence: Synthetic Peptide
<400> 350
   His Ala Asp Gly Thr Leu Thr Ser Asp Ile Ser Ser Phe Leu Glu Lys
   1               5                   10                  15
   Gln Ala Thr Lys Glu Phe Ile Ala Trp Leu Val Ser Gly Arg Gly Arg
               20                  25                  30
   Arg Gln
<210> 351
   <211> 29
   <212> PRT
   <213> Artificial Sequence
<220>
   <223> Description of Artificial Sequence: Synthetic Peptide
<400> 351
   His Ser Gln Gly Thr Phe Thr Ser Asp Tyr Ser Lys Tyr Leu Asp Ser
   1               5                   10                  15
   Lys Lys Ala Gln Glu Phe Val Gln Trp Leu Met Asn Thr
               20                  25
<210> 352
   <211> 39
   <212> PRT
   <213> Artificial Sequence
<220>
   <223> Description of Artificial Sequence: Synthetic Peptide
<400> 352
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 353
   <211> 33
   <212> PRT
   <213> Artificial Sequence
<220>
   <223> Description of Artificial Sequence: Synthetic Peptide
<400> 353
   His Ala Asp Gly Ser Phe Thr Ser Asp Ile Asn Lys Val Leu Asp Thr
   1 5 10 15
   Ile Ala Ala Lys Glu Phe Leu Asn Trp Leu Ile Ser Thr Lys Val Thr
   20 25 30
   Glu
<210> 354
   <211> 36
   <212> PRT
   <213> Artificial Sequence
<220>
   <223> Description of Artificial Sequence: Synthetic peptide
<400> 354
   His Asp Glu Phe Glu Arg His Ala Glu Gly Thr Phe Thr Ser Asp Val
   1 5 10 15
   Ser Ser Tyr Leu Glu Gly Gln Ala Ala Lys Glu Phe Ile Ala Trp Leu
   20 25 30
   Val Lys Gly Arg
   35
<210> 355
   <211> 30
   <212> PRT
   <213> Artificial Sequence
<220>
   <223> Description of Artificial Sequence: Synthetic Peptide
<400> 355
   His Ala Glu Gly Thr Phe Thr Ser Asp Val Ser Ser Tyr Leu Glu Gly
   1 5 10 15
   Gln Ala Ala Lys Glu Phe Ile Ala Trp Leu Val Lys Gly Arg
   20 25 30
<210> 356
   <211> 19
   <212> PRT
   <213> Artificial Sequence
<220>
   <223> Description of Artificial sequence: Synthetic Peptide
<400> 356
   Ser Tyr Leu Glu Gly Gln Ala Ala Lys Glu Phe Ile Ala Trp Leu Val
   1 5 10 15
   Xaa Gly Arg
<210> 357
   <211> 4
   <212> PRT
   <213> Artificial Sequence
<220>
   <223> Description of Artificial Sequence: Synthetic Peptide
<400> 357
   Ser Asp Val Ser
   1
<210> 358
   <211> 5
   <212> PRT
   <213> Artificial Sequence
<220>
   <223> Description of Artificial Sequence: Synthetic Peptide
<400> 358
   Thr Ser Asp Val Ser
   1 5
<210> 359
   <211> 6
   <212> PRT
   <213> Artificial Sequence
<220>
   <223> Description of Artificial Sequence: Synthetic Peptide
<400> 359
   Phe Thr Ser Asp Val Ser
   1 5
<210> 360
   <211> 7
   <212> PRT
   <213> Artificial Sequence
<220>
   <223> Description of Artificial Sequence: Synthetic Peptide
<400> 360
   Thr Phe Thr Ser Asp Val Ser
   1 5
<210> 361
   <211> 8
   <212> PRT
   <213> Artificial Sequence
<220>
   <223> Description of Artificial Sequence: Synthetic Peptide
<400> 361
   Gly Thr Phe Thr Ser Asp Val Ser
   1 5
<210> 362
   <211> 9
   <212> PRT
   <213> Artificial Sequence
<220>
   <223> Description of Artificial Sequence: Synthetic Peptide
<400> 362
   Glu Gly Thr Phe Thr Ser Asp Val Ser
   1 5
<210> 363
   <211> 10
   <212> PRT
   <213> Artificial Sequence
<220>
   <223> Description of Artificial Sequence: Synthetic Peptide
<400> 363
   Ala Glu Gly Thr Phe Thr Ser Asp Val Ser
   1 5 10
<210> 364
   <211> 39
   <212> PRT
   <213> Artificial Sequence
<220>
   <223> Description of Artificial Sequence: Synthetic Peptide
<400> 364
   His Ser Asp Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 365
   <211> 39
   <212> PRT
   <213> Artificial Sequence
<220>
   <223> Description of Artificial Sequence: Synthetic Peptide
<400> 365
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 366
   <211> 31
   <212> PRT
   <213> Artificial Sequence
<220>
   <223> Description of Artificial Sequence: Synthetic Peptide
<400> 366
   His Gly Glu Gly Thr Phe Thr Ser Asp Leu Ser Lys Gln Met Glu Glu
   1 5 10 15
   Ala Val Arg Leu Phe Ile Glu Trp Leu Lys Asn Gly Gly Pro Tyr
   20 25 30
<210> 367
   <211> 31
   <212> PRT
   <213> Artificial Sequence
<220>
   <223> Description of Artificial Sequence: Synthetic Peptide
<400> 367
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Tyr
   20 25 30
<210> 368
   <211> 31
   <212> PRT
   <213> Artificial Sequence
<220>
   <223> Description of Artificial Sequence: Synthetic Peptide
<400> 368
   Asp Leu Ser Lys Gln Met Glu Glu Glu Ala Val Arg Leu Met Ile Glu
   1 5 10 15
   Trp Leu Lys Asn Gly Gly Pro Ser Ser Gly Ala Pro Pro Pro Ser
   20 25 30
<210> 369
   <211> 37
   <212> PRT
   <213> Artificial Sequence
<220>
   <223> Description of Artificial Sequence: Synthetic Peptide
<400> 369
   His Asp Glu Phe Glu Arg His Ala Glu Gly Thr Phe Thr Ser Asp Val
   1 5 10 15
   Ser Ser Tyr Leu Glu Gly Gln Ala Ala Lys Glu Phe Ile Ala Trp Leu
   20 25 30
   Val Lys Gly Arg Lys
   35
<210> 370
   <211> 31
   <212> PRT
   <213> Artificial Sequence
<220>
   <223> Description of Artificial Sequence: Synthetic Peptide
<400> 370
   His Ala Glu Gly Thr Phe Thr Ser Asp Val Ser Ser Tyr Leu Glu Gly
   1 5 10 15
   Gln Ala Ala Lys Glu Phe Ile Ala Trp Leu Val Lys Gly Arg Lys
   20 25 30
<210> 371
   <211> 40
   <212> PRT
   <213> Artificial Sequence
<220>
   <223> Description of Artificial Sequence: Synthetic Peptide
<400> 371
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser Lys
   35 40
<210> 372
   <211> 40
   <212> PRT
   <213> Artificial Sequence
<220>
   <223> Description of Artificial Sequence: Synthetic Peptide
<400> 372
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser Lys
   35 40
<210> 373
   <211> 40
   <212> PRT
   <213> Artificial Sequence
<220>
   <223> Description of Artificial Sequence: Synthetic Peptide
<400> 373
   His Ser Asp Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser Lys
   35 40

<210> 374
   <211> 31
   <212> PRT
   <213> Artificial Sequence
<220>
   <223> Description of Artificial Sequence: Synthetic Peptide
<400> 374
   His Gly Glu Gly Thr Phe Thr Ser Asp Leu Ser Lys Glu Met Glu Glu
   1 5 10 15
   Glu Val Arg Leu Phe Ile Glu Trp Leu Lys Asn Gly Gly Pro Tyr
   20 25 30
<210> 375
   <211> 30
   <212> PRT
   <213> Artificial Sequence
<220>
   <223> Description of Artificial Sequence: Synthetic Peptide
<400> 375
   His Gly Glu Gly Thr Phe Thr Ser Asp Leu Ser Lys Glu Met Glu Glu
   1 5 10 15
   Glu Val Arg Leu Phe Ile Glu Trp Leu Lys Asn Gly Gly Tyr
   20 25 30
<210> 376
   <211> 29
   <212> PRT
   <213> Artificial Sequence
<220>
   <223> Description of Artificial Sequence: Synthetic Peptide
<400> 376
   Asp Leu Ser Lys Gln Met Glu Glu Glu Ala Val Arg Leu Phe Ile Glu
   1 5 10 15
   Trp Leu Lys Gly Gly Pro Ser Ser Gly Pro Pro Pro Ser
   20 25
<210> 377
   <211> 31
   <212> PRT
   <213> Artificial Sequence
<220>
   <223> Description of Artificial Sequence: Synthetic Peptide
<400> 377
   Glu Ala Glu Asp Leu Gln Val Gly Gln Val Glu Leu Gly Gly Gly Pro
   1 5 10 15
   Gly Ala Gly Ser Leu Gln Pro Leu Ala Leu Glu Gly Ser Leu Gln
   20 25 30
<210> 378
   <211> 32
   <212> PRT
   <213> Artificial Sequence
<220>
   <223> Description of Artificial Sequence: Synthetic Peptide
<400> 378
   Tyr Glu Ala Glu Asp Leu Gln Val Gly Gln Val Glu Leu Gly Gly Gly
   1 5 10 15
   Pro Gly Ala Gly Ser Leu Gln Pro Leu Ala Leu Glu Gly Ser Leu Gln
   20 25 30
<210> 379
   <211> 51
   <212> PRT
   <213> Artificial Sequence
<220>
   <223> Description of Artificial Sequence: Synthetic Peptide
<400> 379
   Gly Ile Val Glu Gln Cys Cys Thr Ser Ile Cys Ser Leu Tyr Gln Leu
   1 5 10 15
   Glu Asn Tyr Cys Asn Phe Val Asn Gln His Leu Cys Gly Ser His Leu
   20 25 30
   Val Glu Ala Leu Tyr Leu Val Cys Gly Glu Arg Gly Phe Phe Tyr Thr
   35 40 45
   Pro Lys Thr
   50
<210> 380
   <211> 16
   <212> PRT
   <213> Artificial Sequence
<220>
   <223> Description of Artificial Sequence: Synthetic Peptide
<400> 380
   Asp Val Ser Thr Pro Pro Thr Val Leu Pro Asp Asn Phe Pro Arg Tyr
   1 5 10 15
<210> 381
   <212> 35
   <212> PRT
   <213> Artificial Sequence
<220>
   <223> Description of Artificial Sequence: Synthetic Peptide
<400> 381
<210> 382
   <211> 24
   <212> PRT
   <213> Artificial Sequence
<220>
   <223> Description of Artificial Sequence: Synthetic Peptide
<400> 382
   Gly Leu Pro Ala Leu Leu Arg Ala Arg Arg Gly His Val Leu Ala Lys
   1 5 10 15
   Glu Leu Glu Ala Phe Arg Glu Ala
   20
<210> 383
   <211> 36
   <212> PRT
   <213> Artificial Sequence
<220>
   <223> Description of Artificial Sequence: synthetic Peptide
<400> 383
   Tyr Pro Ser Lys Pro Asp Asn Pro Gly Glu Asp Ala Pro Ala Glu Asp
   1 5 10 15
   Met Ala Arg Tyr Tyr Ser Ala Leu Arg His Tyr Ile Asn Leu Leu Thr
   20 25 30
   Arg Pro Arg Tyr
   35
<210> 384
   <211> 39
   <212> PRT
   <213> Artificial Sequence
<220>
   <223> Description of Artificial Sequence: Synthetic Peptide
<400> 384
   Pro Asp Lys Asp Phe Ile Val Asn Pro Ser Asp Leu Val Leu Asp Asn
   1 5 10 15
   Lys Ala Ala Leu Arg Asp Tyr Leu Arg Gln Ile Asn Glu Tyr Phe Ala
   20 25 30
   Ile Ile Gly Arg Pro Arg Phe
   35
<210> 385
   <211> 3
   <212> PRT
   <213> Artificial Sequence
<220>
   <223> Description of Artificial Sequence: Synthetic Peptide
<400> 385
   Phe Met Arg
   1
<210> 386
   <211> 8
   <212> PRT.
   <213> Artificial Sequence
<220>
   <223> Description of Artificial Sequence: Synthetic Peptide
<400> 386
   Cys Trp Arg Tyr Cys Trp Arg Tyr
   1 5
<210> 387
   <211> 36
   <212> PRT
   <213> Artificial Sequence
<220>
   <223> Description of Artificial Sequence: Synthetic Peptide
<400> 387
   Tyr Pro Ser Lys Pro Asp Asn Pro Gly Glu Asp Ala Pro Ala Glu Asp
   1 5 10 15
   Met Ala Arg Tyr Tyr Ser Ala Leu Arg His Tyr Ile Asn Leu Ile Thr
   20 25 30
   Arg Gln Arg Tyr
   35
<210> 388
   <211> 24
   <212> PRT
   <213> Artificial Sequence
<220>
   <223> Description of Artificial Sequence: Synthetic Peptide
<400> 388
   Tyr Pro Ser Lys Pro Asp Asn Pro Gly Glu Asp Ala Pro Ala Glu Asp
   1 5 10 15
   Met Ala Arg Tyr Tyr Ser Ala Leu
   20
<210> 389
   <211> 8
   <212> PRT
   <213> Artificial Sequence
<220>
   <223> Description of Artificial Sequence: Synthetic Peptide
<400> 389
   Ile Gly Pro Tyr Arg Leu Arg Tyr
   1 5
<210> 390
   <211> 36
   <212> PRT
   <213> Artificial Sequence
<220>
   <223> Description of Artificial Sequence: Synthetic Peptide
<400> 390
   Gly Pro Ser Gln Pro Thr Tyr Pro Gly Asp Asp Ala Pro Val Glu Asp
   1 5 10 15
   Leu Ile Arg Phe Tyr Asp Asn Leu Gln Gln Tyr Leu Asn Val Val Thr
   20 25 30
   Arg His Arg Tyr
   35
<210> 391
   <211> 36
   <212> PRT
   <213> Artificial Sequence
<220>
   <223> Description of Artificial Sequence: Synthetic Peptide
<400> 391
   Ala Pro Leu Glu Pro Val Tyr Pro Gly Asp Asn Ala Thr Pro Glu Gln
   1 5 10 15
   Met Ala Gln Tyr Ala Ala Asp Leu Arg Arg Tyr Ile Asn Met Leu Thr
   20 25 30
   Arg Pro Arg Tyr
   35
<210> 392
   <211> 6
   <212> PRT
   <213> Artificial Sequence
<220>
   <223> Description of Artificial Sequence: Synthetic Peptide
<400> 392
   Leu Thr Arg Pro Arg Tyr
   1 5
<210> 393
   <211> 6
   <212> PRT
   <213> Artificial Sequence
<220>
   <223> Description of Artificial Sequence: Synthetic Peptide
<400> 393
   Leu Thr Arg Pro Arg Tyr
   1 5
<210> 394
   <211> 36
   <212> PRT
   <213> Artificial Sequence
<220>
   <223> Description of Artificial Sequence: Synthetic Peptide
<400> 394
   Ala Pro Ser Glu Pro His His Pro Gly Asp Gln Ala Thr Gln Asp Gln
   1 5 10 15
   Leu Ala Gln Tyr Tyr Ser Asp Leu Tyr Gln Tyr Ile Thr Phe Val Thr
   20 25 30
   Arg Pro Arg Phe
   35
<210> 395
   <211> 36
   <212> PRT
   <213> Artificial Sequence
<220>
   <223> Description of Artificial Sequence: Synthetic Peptide
<400> 395
   Ala Pro Leu Glu Pro Met Tyr Pro Gly Asp Tyr Ala Thr His Glu Gln
   1               5                   10                  15
   Arg Ala Gln Tyr Glu Thr Gln Leu Arg Arg Tyr Ile Asn Thr Leu Thr
               20                  25                  30
   Arg Pro Arg Tyr
           35
<210> 396
   <211> 36
   <212> PRT
   <213> Artificial Sequence
<220>
   <223> Description of Artificial Sequence: Synthetic Peptide
<400> 396
   Tyr Pro Pro Lys Pro Glu Asn Pro Gly Glu Asp Ala Pro Pro Glu Glu
   1               5                   10                  15
   Leu Ala Lys Tyr Tyr Thr Ala Leu Arg His Tyr Ile Asn Leu Ile Thr
               20                  25                  30
   Arg Gln Arg Tyr
           35
<210> 397
   <211> 36
   <212> PRT
   <213> Artificial Sequence
<220>
   <223> Description of Artificial Sequence: Synthetic Peptide
<400> 397
   Tyr Pro Ile Lys Pro Glu Ala Pro Gly Glu Asp Ala Ser Pro Glu Glu
   1               5                   10                  15
   Leu Asn Arg Tyr Tyr Ala Ser Leu Arg His Tyr Leu Asn Leu Val Thr
               20                  25                  30
   Arg Gln Arg Tyr
           35
<210> 398
   <211> 34
   <212> PRT
   <213> Artificial Sequence
<220>
   <223> Description of Artificial Sequence: Synthetic Peptide
<400> 398
   Ile Lys Pro Glu Ala Pro Gly Glu Asp Ala Ser Pro Glu Glu Leu Asn
   1 5 10 15
   Arg Tyr Tyr Ala Ser Leu Arg His Tyr Leu Asn Leu Val Thr Arg Gln
   20 25 30
   Arg Tyr
<210> 399
   <211> 36
   <212> PRT
   <213> Artificial Sequence
<220>
   <223> Description of Artificial Sequence: Synthetic Peptide
<400> 399
   Tyr Pro Ala Lys Pro Glu Ala Pro Gly Glu Asp Ala Ser Pro Glu Glu
   1 5 10 15
   Leu Ser Arg Tyr Tyr Ala Ser Leu Arg His Tyr Leu Asn Leu Val Thr
   20 25 30
   Arg Gln Arg Tyr
   35
<210> 400
   <211> 36
   <212> PRT
   <213> Artificial Sequence
<220>
   <223> Description of Artificial Sequence: Synthetic Peptide
<400> 400
   Tyr Pro Ile Lys Pro Glu Ala Pro Gly Glu Asp Ala Ser Pro Glu Glu
   1 5 10 15
   Leu Asn Arg Tyr Tyr Ala Ser Leu Arg His Tyr Leu Asn Leu Leu Thr
   20 25 30
   Arg Pro Arg Tyr
   35
<210> 401
   <211> 10
   <212> PRT
   <213> Artificial Sequence
<220>
   <223> Description of Artificial Sequence: Synthetic Peptide
<400> 401
   Glu Gln Asp Tyr Thr Gly Trp Met Asp Phe
   1 5 10
<210> 402
   <211> 33
   <212> PRT
   <213> Artificial Sequence
<220>
   <223> Description of Artificial Sequence: Synthetic Peptide
<400> 402
   Lys Ala Pro Ser Gly Arg Met Ser Ile Val Lys Asn Leu Gln Asn Leu
   1 5 10 15
   Asp Pro Ser His Arg Ile Ser Asp Arg Asp Tyr Met Gly Trp Met Asp
   20 25 30
   Phe
<210> 403
   <211> 4
   <212> PRT
   <213> Artificial Sequence
<220>
   <223> Description of Artificial Sequence: Synthetic Peptide
<400> 403
   Asp Tyr Met Gly
   1
<210> 404
   <211> 8
   <212> PRT
   <213> Artificial Sequence
<220>
   <223> Description of Artificial Sequence: Synthetic Peptide
<400> 404
<210> 405
   <211> 8
   <212> PRT
   <213> Artificial Sequence
<220>
   <223> Description of Artificial Sequence: Synthetic Peptide
<400> 405
<210> 406
   <211> 7
   <212> PRT
   <213> Artificial Sequence
<220>
   <223> Description of Artificial Sequence: Synthetic Peptide
<400> 406
   Tyr Met Gly Trp Met Asp Phe
   1 5
<210> 407
   <211> 4
   <212> PRT
   <213> Artificial Sequence
<220>
   <223> Description of Artificial Sequence: Synthetic Peptide
<400> 407
   Trp Met Asp Phe
   1
<210> 408
   <211> 33
   <212> PRT
   <213> Artificial Sequence
<220>
   <223> Description of Artificial Sequence: Synthetic Peptide
<400> 408
   Lys Ala Pro Ser Gly Arg Val Ser Met Ile Lys Asn Leu Gln Ser Leu
   1 5 10 15
   Asp Pro Ser His Arg Ile Ser Asp Arg Asp Tyr Met Gly Trp Met Asp
   20 25 30
   Phe
<210> 409
   <212> PRT
   <213> Artificial Sequence
<220>
   <223> Description of Artificial Sequence: Synthetic Peptide
<400> 409
<210> 410
   <211> 9
   <212> PRT
   <213> Artificial Sequence
<220>
   <223> Description of Artificial Sequence: Synthetic Peptide
<400> 410
   Ser Ala Glu Glu Tyr Glu Tyr Pro Ser
   1 5
<210> 411
   <211> 5
   <212> PRT
   <213> Artificial Sequence
<220>
   <223> Description of Artificial Sequence: Synthetic Peptide
<400> 411
   Asp Tyr Met Gly Trp
   1 5
<210> 412
   <211> 6
   <212> PRT
   <213> Artificial Sequence
<220>
   <223> Description of Artificial Sequence: Synthetic Peptide
<400> 412
   Asp Tyr Met Gly Trp Met
   1 5
<210> 413
   <211> 9
   <212> PRT
   <213> Artificial Sequence
<220>
   <223> Description of Artificial Sequence: Synthetic Peptide
<400> 413
   Val Pro Val Glu Ala Val Asp Pro Met
   1 5
<210> 414
   <212> PRT
   <213> Artificial Sequence
<220>
   <223> Description of Artificial Sequence: Synthetic Peptide
<400> 414
<210> 415
   <211> 8
   <212> PRT
   <213> Artificial Sequence
<220>
   <223> Description of Artificial Sequence: Synthetic Peptide
<400> 415
<210> 416
   <211> 23
   <212> PRT
   <213> Artificial Sequence
<220>
   <223> Description of Artificial Sequence: Synthetic Peptide
<400> 416
   Met Lys Val Ala Ile Ile Phe Leu Leu Ser Ala Leu Ala Leu Leu Asn
   1 5 10 15
   Leu Ala Gly Asn Thr Thr Ala
   20
<210> 417
   <211> 27
   <212> PRT
   <213> Artificial Sequence
<220>
   <223> Description of Artificial Sequence: Synthetic Peptide
<400> 417
   Val Pro Leu Pro Ala Gly Gly Gly Thr Val Leu Thr Lys Met Tyr Pro
   1 5 10 15
   Arg Gly Asn His Trp Ala Val Gly His Leu Met
   20 25
<210> 418
   <211> 16
   <212> PRT
   <213> Artificial Sequence
<220>
   <223> Description of Artificial Sequence: Synthetic Peptide
<400> 418
   Val Pro Leu Pro Ala Gly Gly Gly Thr Val Leu Thr Lys Met Tyr Pro
   1 5 10 15
<210> 419
   <211> 27
   <212> PRT
   <213> Artificial Sequence
<220>
   <223> Description of Artificial Sequence: Synthetic Peptide
<400> 419
   Ala Pro Val Ser Val Gly Gly Gly Thr Val Leu Ala Lys Met Tyr Pro
   1 5 10 15
   Arg Gly Asn His Trp Ala Val Gly His Leu Met
   20 25
<210> 420
   <211> 14
   <212> PRT
   <213> Artificial Sequence
<220>
   <223> Description of Artificial Sequence: synthetic Peptide
<400> 420
   Met Tyr Pro Arg Gly Asn His Trp Ala Val Gly His Leu Met
   1 5 10
<210> 421
   <211> 36
   <212> PRT
   <213> Artificial Sequence
<220>
   <223> Description of Artificial Sequence: Synthetic Peptide
<400> 421
   Phe Leu Pro His Val Phe Ala Glu Leu Ser Asp Arg Lys Gly Phe Val
   1 5 10 15
   Gln Gly Asn Gly Ala Val Glu Ala Leu His Asp His Phe Tyr Pro Asp
   20 25 30
   Trp Met Asp Phe
   35
<210> 422
   <211> 17
   <212> PRT
   <213> Artificial Sequence
<220>
   <223> Description of Artificial Sequence: Synthetic Peptide
<400> 422
   Glu Gly Pro Trp Leu Glu Glu Glu Glu Glu Ala Tyr Gly Trp Met Asp
   1 5 10 15
   Phe
<210> 423
   <211> 34
   <212> PRT
   <213> Artificial Sequence
<220>
   <223> Description of Artificial Sequence: Synthetic Peptide
<400> 423
   Glu Leu Gly Pro Gln Gly Pro Pro His Leu Val Ala Asp Pro Ser Lys
   1 5 10 15
   Lys Gln Gly Pro Trp Leu Glu Glu Glu Glu Glu Ala Tyr Gly Trp Met
   20 25 30
   Asp Phe
<210> 424
   <211> 17
   <212> PRT
   <213> Artificial Sequence
<220>
   <223> Description of Artificial Sequence: Synthetic Peptide
<400> 424
   Glu Arg Pro Pro Met Glu Glu Glu Glu Glu Ala Tyr Gly Trp Met Asp
   1 5 10 15
   Phe
<210> 425
   <211> 5
   <212> PRT
   <213> Artificial Sequence
<220>
   <223> Description of Artificial Sequence: Synthetic Peptide
<400> 425
   Ala Trp Met Asp Phe
   1 5
<210> 426
   <211> 42
   <212> PRT
   <213> Artificial Sequence
<220>
   <223> Description of Artificial Sequence: Synthetic Peptide
<400> 426
   Tyr Ala Glu Gly Thr Phe Ile Ser Asp Tyr Ser Ile Ala Met Asp Lys
   1               5                   10                  15
   Ile His Gln Gln Asp Phe Val Asn Trp Leu Leu Ala Gln Lys Gly Lys
               20                  25                  30
   Lys Asn Asp Trp Lys His Asn Ile Thr Gln
           35                  40
<210> 427
   <211> 42
   <212> PRT
   <213> Artificial Sequence
<220>
   <223> Description of Artificial Sequence: Synthetic Peptide
<400> 427
   Tyr Ala Glu Gly Thr Phe Ile Ser Asp Tyr Ser Ile Ala Met Asp Lys
   1               5                   10                  15
   Ile Arg Gln Gln Asp Phe Val Asn Trp Leu Leu Ala Gln Lys Gly Lys
               20                  25                  30
   Lys Ser Asp Trp Lys His Asn Ile Thr Gln
           35                  40
<210> 428
   <211> 30
   <212> PRT
   <213> Artificial Sequence
<220>
   <223> Description of Artificial Sequence: Synthetic peptide
<400> 428
   Tyr Ala Glu Gly Thr Phe Ile Ser Asp Tyr Ser Ile Ala Met Asp Lys
   1               5                   10                  15
   Ile Arg Gln Gln Asp Phe Val Asn Trp Leu Leu Ala Gln Lys
               20                  25                  30
<210> 429
   <211> 30
   <212> PRT
   <213> Artificial Sequence
<220>
   <223> Description of Artificial Sequence: Synthetic Peptide
<400> 429
   Tyr Ala Glu Gly Thr Phe Ile Ser Asp Tyr Ser Ile Ala Met Asp Lys
   1 5 10 15
   Ile Arg Gln Gln Asp Phe Val Asn Trp Leu Leu Ala Gln Lys
   20 25 30
<210> 430
   <211> 22
   <212> PRT
   <213> Artificial Sequence
<220>
   <223> Description of Artificial Sequence: Synthetic Peptide
<400> 430
   Phe Val Pro Ile Phe Thr His Ser Glu Leu Gln Lys Ile Arg Glu Lys
   1 5 10 15
   Glu Arg Asn Lys Gly Gln
   20
<210> 431
   <211> 22
   <212> PRT
   <213> Artificial Sequence
<220>
   <223> Description of Artificial Sequence: Synthetic Peptide
<400> 431
   Phe Val Pro Ile Phe Thr Tyr Gly Glu Leu Gln Arg Met Gln Glu Lys
   1 5 10 15
   Glu Arg Asn Lys Gly Gln
   20
<210> 432
   <211> 22
   <212> PRT
   <213> Artificial Sequence
<220>
   <223> Description of Artificial Sequence: Synthetic Peptide
<400> 432
   Phe Val Pro Ile Phe Thr Tyr Gly Glu Leu Gln Arg Leu Gln Glu Lys
   1 5 10 15
   Glu Arg Asn Lys Gly Gln
   20
<210> 433
   <211> 21
   <212> PRT
   <213> Artificial Sequence
<220>
   <223> Description of Artificial Sequence: Synthetic Peptide
<400> 433
   Phe Val Pro Ile Phe Thr Tyr Gly Glu Leu Arg Leu Gln Glu Lys Glu
   1 5 10 15
   Arg Asn Lys Gly Gln
   20
<210> 434
   <211> 9
   <212> PRT
   <213> Artificial Sequence
<220>
   <223> Description of Artificial Sequence: Synthetic Peptide
<400> 434
   Ile Ala Arg Arg His Pro Tyr Phe Leu
   1 5
<210> 435
   <211> 27
   <212> PRT
   <213> Artificial Sequence
<220>
   <223> Description of Artificial Sequence: Synthetic Peptide
<400> 435
   His Ser Asp Gly Thr Phe Thr Ser Glu Leu Ser Arg Leu Arg Glu Ser
   1 5 10 15
   Ala Arg Leu Gln Arg Leu Leu Gln Gly Leu Val
   20 25
<210> 436
   <211> 27
   <212> PRT
   <213> Artificial Sequence
<220>
   <223> Description of Artificial Sequence: Synthetic Peptide
<400> 436
   His Ser Asp Gly Leu Phe Thr Ser Glu Tyr Ser Lys Met Arg Gly Asn
   1 5 10 15
   Ala Gln Val Gln Lys Phe Ile Gln Asn Leu Met
   20 25
<210> 437
   <211> 27
   <212> PRT
   <213> Artificial Sequence
<220>
   <223> Description of Artificial Sequence: Synthetic Peptide
<400> 437
   His Ser Asp Gly Thr Phe Thr Ser Glu Leu Ser Arg Leu Arg Glu Gly
   1 5 10 15
   Ala Arg Leu Gln Arg Leu Leu Gln Gly Leu Val
   20 25
<210> 438
   <211> 27
   <212> PRT
   <213> Artificial Sequence
<220>
   <223> Description of Artificial Sequence: Synthetic Peptide
<400> 438
   His Ser Asp Gly Thr Phe Thr Ser Glu Leu Ser Arg Leu Arg Asp Ser
   1 5 10 15
   Ala Arg Leu Gln Arg Leu Leu Gln Gly Leu Val
   20 25
<210> 439
   <211> 27
   <212> PRT
   <213> Artificial Sequence
<220>
   <223> Description of Artificial Sequence: Synthetic Peptide
<400> 439
   His Ser Asp Gly Thr Phe Thr Ser Glu Leu Ser Arg Leu Gln Asp Ser
   1 5 10 15
   Ala Arg Leu Gln Arg Leu Leu Gln Gly Leu Val
   20 25
<210> 440
   <211> 26
   <212> PRT
   <213> Artificial Sequence
<220>
   <223> Description of Artificial Sequence: Synthetic Peptide
<400> 440
   Ser Asp Gly Thr Phe Thr Ser Glu Leu Ser Arg Leu Arg Asp Ser Ala
   1 5 10 15
   Arg Leu Gln Arg Leu Leu Gly Gly Leu Val
   20 25
<210> 441
   <211> 32
   <212> PRT
   <213> Artificial Sequence
<220>
   <223> Description of Artificial Sequence: Synthetic Peptide
<400> 441
   Ala Ala Met Leu Ala Ser Gln Thr Glu Ala Phe Val Pro Ile Phe Thr
   1 5 10 15
   Tyr Gly Glu Leu Gln Arg Met Gln Glu Lys Glu Arg Asn Lys Gly Gln
   20 25 30
<210> 442
   <211> 28
   <212> PRT
   <213> Artificial sequence
<220>
   <223> Description of Artificial Sequence: Synthetic Peptide
<400> 442
   His Ser Asp Ala Val Phe Thr Asp Asn Tyr Thr Arg Leu Arg Arg Gln
   1 5 10 15
   Leu Ala Val Arg Arg Tyr Leu Asn Ser Ile Leu Asn
   20 25
<210> 443
   <211> 31
   <212> PRT
   <213> Artificial Sequence
<220>
   <223> Description of Artificial Sequence: Synthetic Peptide
<400> 443
   His Ser Asp Ala Val Phe Thr Asp Asn Tyr Thr Arg Leu Arg Arg Gln
   1 5 10 15
   Leu Ala Val Arg Arg Tyr Leu Asn Ser Ile Leu Asn Gly Lys Arg
   20 25 30
<210> 444
   <211> 12
   <212> PRT
   <213> Artificial Sequence
<220>
   <223> Description of Artificial Sequence: Synthetic Peptide
<400> 444
   Met Ala Val Lys Lys Tyr Leu Asn Ser Ile Leu Asn
   1 5 10

<210> 445
   <211> 27
   <212> PRT
   <213> Artificial Sequence
<220>
   <223> Description of Artificial Sequence: Synthetic Peptide
<400> 445
   His Ala Asp Gly Val Phe Thr Ser Asp Phe Ser Arg Leu Leu Gly Gln
   1 5 10 15
   Leu Ser Ala Lys Lys Tyr Leu Glu Ser Leu Ile
   20 25
<210> 446
   <211> 27
   <212> PRT
   <213> Artificial Sequence
<220>
   <223> Description of Artificial Sequence: Synthetic Peptide
<400> 446
   His Ala Asp Gly Val Phe Thr Ser Asp Tyr Ser Arg Leu Leu Gly Gln
   1 5 10 15
   Ile Ser Ala Lys Lys Tyr Leu Glu Ser Leu Ile
   20 25
<210> 447
   <211> 27
   <212> PRT
   <213> Artificial Sequence
<220>
   <223> Description of Artificial Sequence: Synthetic Peptide
<400> 447
   His Ala Asp Gly Val Phe Thr Ser Asp Phe Ser Lys Leu Leu Gly Gln
   1 5 10 15
   Leu Ser Ala Lys Lys Tyr Leu Glu Ser Leu Met
   20 25
<210> 448
   <211> 42
   <212> PRT
   <213> Artificial Sequence
<220>
   <223> Description of Artificial Sequence: Synthetic Peptide
<400> 448
   His Ala Asp Gly Val Phe Thr Ser Asp Phe Ser Lys Leu Leu Gly Gln
   1 5 10 15
   Leu Ser Ala Lys Lys Tyr Leu Glu Ser Leu Met Gly Lys Arg Val Ser
   20 25 30
   Ser Asn Ile Ser Glu Asp Pro Val Pro Val
   35 40
<210> 449
   <211> 12
   <212> PRT
   <213> Artificial Sequence
<220>
   <223> Description of Artificial Sequence: Synthetic Peptide
<400> 449
   Val Ser Ser Asn Ile Ser Glu Asp Pro Val Pro Val
   1 5 10
<210> 450
   <211> 15
   <212> PRT
   <213> Artificial Sequence
<220>
   <223> Description of Artificial Sequence: Synthetic Peptide
<400> 450
   Ser Ser Glu Gly Glu Ser Pro Asp Phe Pro Glu Glu Leu Glu Lys
   1 5 10 15
<210> 451
   <211> 21
   <212> PRT
   <213> Artificial Sequence
<220>
   <223> Description of Artificial Sequence: Synthetic Peptide
<400> 451
   Cys Ser Cys Asn Ser Trp Leu Asp Lys Glu Cys Val Tyr Phe Cys His
   1 5 10 15
   Leu Asp Ile Ile Trp
   20
<210> 452
   <211> 28
   <212> PRT
   <213> Artificial Sequence
<220>
   <223> Description of Artificial Sequence: Synthetic Peptide
<400> 452
   His Ser Asp Ala Val Phe Thr Asp Asn Tyr Ser Arg Phe Arg Lys Gln
   1 5 10 15
   Met Ala Val Lys Lys Tyr Leu Asn Ser Val Leu Thr
   20 25
<210> 453
   <211> 28
   <212> PRT
   <213> Artificial Sequence
<220>
   <223> Description of Artificial Sequence: Synthetic Peptide
<400> 453
   His Ser Asp Ala Leu Phe Thr Asp Thr Tyr Thr Arg Leu Arg Lys Gln
   1 5 10 15
   Met Ala Met Lys Lys Tyr Leu Asn Ser Val Leu Asn
   20 25
<210> 454
   <211> 28
   <212> PRT
   <213> Artificial Sequence
<220>
   <223> Description of Artificial Sequence: Synthetic Peptide
<400> 454
   His Ser Asp Ala Val Phe Thr Asp Asn Tyr Thr Arg Leu Arg Lys Gln
   1 5 10 15
   Met Ala Val Lys Lys Tyr Leu Asn Ser Ile Leu Asn
   20 25
<210> 455
   <211> 28
   <212> PRT
   <213> Artificial Sequence
<220>
   <223> Description of Artificial Sequence: Synthetic Peptide
<400> 455
   His Ser Asp Ala Val Phe Thr Asp Asn Tyr Thr Arg Leu Arg Lys Gln
   1 5 10 15
   Met Ala Val Lys Lys Tyr Leu Asn Ser Ile Leu Asn
   20 25
<210> 456
   <211> 12
   <212> PRT
   <213> Artificial Sequence
<220>
   <223> Description of Artificial Sequence: Synthetic Peptide
<400> 456
   His Ser Asp Ala Val Phe Thr Asp Asn Tyr Thr Arg
   1 5 10
<210> 457
   <211> 19
   <212> PRT
   <213> Artificial Sequence
<220>
   <223> Description of Artificial Sequence: Synthetic Peptide
<400> 457
   Tyr Thr Arg Leu Arg Lys Gln Met Ala Val Lys Lys Tyr Leu Asn Ser
   1 5 10 15
   Ile Leu Asn
<210> 458
   <211> 18
   <212> PRT
   <213> Artificial Sequence
<220>
   <223> Description of Artificial Sequence: Synthetic Peptide
<400> 458
   Thr Arg Leu Arg Lys Gln Met Ala Val Lys Lys Tyr Leu Asn Ser Ile
   1 5 10 15
   Leu Asn
<210> 459
   <211> 27
   <212> PRT
   <213> Artificial Sequence
<220>
   <223> Description of Artificial Sequence: Synthetic Peptide
<400> 459
   His Ser Asp Ala Val Phe Thr Asp Asn Ser Arg Phe Arg Lys Gln Met
   1 5 10 15
   Ala Ala Lys Lys Tyr Leu Asn Ser Val Leu Ala
   20 25
<210> 460
   <211> 23
   <212> PRT
   <213> Artificial Sequence
<220>
   <223> Description of Artificial Sequence: Synthetic Peptide
<400> 460
   Phe Thr Asp Asn Tyr Thr Arg Leu Arg Lys Gln Met Ala Val Lys Lys
   1 5 10 15
   Tyr Leu Asn Ser Ile Leu Asn
   20
<210> 461
   <211> 28
   <212> PRT
   <213> Artificial Sequence
<220>
   <223> Description of Artificial Sequence: Synthetic Peptide
<400> 461
   Lys Pro Arg Arg Pro Tyr Thr Asp Asn Tyr Thr Arg Leu Arg Lys Gln
   1 5 10 15
   Met Ala Val Lys Lys Tyr Leu Asn Ser Ile Leu Asn
   20 25
<210> 462
   <211> 29
   <212> PRT
   <213> Artificial Sequence
<220>
   <223> Description of Artificial Sequence: Synthetic Peptide
<400> 462
   Tyr Phe Asp Ala Ile Phe Thr Asn Ser Tyr Arg Lys Val Leu Gly Gln
   1 5 10 15
   Leu Ser Ala Arg Lys Leu Leu Gln Asp Ile Met Ser Arg
   20 25
<210> 463
   <211> 28
   <212> PRT
   <213> Artificial Sequence
<220>
   <223> Description of Artificial Sequence: Synthetic Peptide
<400> 463
   His Ser Asp Ala Val Phe Thr Asp Asn Tyr Thr Arg Leu Arg Lys Gln
   1               5                   10                  15
   Leu Ala Val Lys Lys Tyr Leu Asn Ser Ile Leu Asn
               20                  25
<210> 464
   <211> 8
   <212> PRT
   <213> Artificial Sequence
<220>
   <223> Description of Artificial Sequence: Synthetic Peptide
<400> 464
   Leu Met Tyr Pro Thr Tyr Leu Lys
   1               5
<210> 465
   <211> 29
   <212> PRT
   <213> Artificial Sequence
<220>
   <223> Description of Artificial Sequence: Synthetic Peptide
<400> 465
   Met Met Arg Asp Ser Gly Cys Phe Gly Arg Arg Ile Asp Arg Ile Gly
   1               5                   10                  15
   Ser Leu Ser Gly Met Gly Cys Asn Gly Ser Arg Lys Asn
               20                  25
<210> 466
   <211> 15
   <212> PRT
   <213> Artificial Sequence
<220>
   <223> Description of Artificial Sequence: Synthetic Peptide
<400> 466
   Gly Phe Ile Trp Gly Asn Ile Phe Gly His Tyr Ser Gly Asp Phe
   1 5 10 15
<210> 467
   <211> 11
   <212> PRT
   <213> Artificial Sequence
<220>
   <223> Description of Artificial Sequence: Synthetic Peptide
<400> 467
   Ser Leu Arg Arg Ser Ser Cys Phe Gly Gly Arg
   1 5 10
<210> 468
   <211> 24
   <212> PRT
   <213> Artificial Sequence
<220>
   <223> Description of Artificial Sequence: Synthetic Peptide
<400> 468
   Ser Ser Asp Cys Phe Gly Ser Arg Ile Asp Arg Ile Gly Ala Gln Ser
   1 5 10 15
   Gly Met Gly Cys Gly Arg Arg Phe
   20
<210> 469
   <211> 20
   <212> PRT
   <213> Artificial Sequence
<220>
   <223> Description of Artificial Sequence: synthetic Peptide
<400> 469
   Cys Phe Gly Ser Arg Ile Asp Arg Ile Gly Ala Gln Ser Gly Met Gly
   1 5 10 15
   Cys Gly Arg Phe
   20
<210> 470
   <211> 21
   <212> PRT
   <213> Artificial Sequence
<220>
   <223> Description of Artificial Sequence: Synthetic Peptide
<400> 470
   Cys Phe Gly Ser Arg Ile Asp Arg Ile Gly Ala Gln Ser Gly Met Gly
   1 5 10 15
   Cys Gly Arg Arg Phe
<210> 471
   <211> 24
   <212> PRT
   <213> Artificial Sequence
<220>
   <223> Description of Artificial Sequence: Synthetic Peptide
<400> 471
   Ser Ser Asp Cys Phe Gly Ser Arg Ile Asp Arg Ile Gly Ala Gln Ser
   1 5 10 15
   Gly Met Gly Cys Gly Arg Arg Phe
   20
<210> 472
   <211> 30
   <212> PRT
   <213> Artificial Sequence
<220>
   <223> Description of Artificial Sequence: Synthetic Peptide
<400> 472
   Ala Pro Arg Ser Met Arg Arg Ser Ser Asp Cys Phe Gly Ser Arg Ile
   1 5 10 15
   Asp Arg Ile Gly Ala Gln Ser Gly Met Gly Cys Gly Arg Phe
   20 25 30
<210> 473
   <211> 24
   <212> PRT
   <213> Artificial Sequence
<220>
   <223> Description of Artificial-Sequence: Synthetic Peptide
<400> 473
   Ser Ser Cys Phe Gly Gly Arg Met Asp Arg Ile Gly Ala Gln Ser Gly
   1 5 10 15
   Leu Gly Cys Asn Ser Phe Arg Tyr
   20
<210> 474
   <211> 17
   <212> PRT
   <213> Artificial Sequence
<220>
   <223> Description of Artificial Sequence: Synthetic Peptide
<400> 474
   Cys Phe Gly Gly Arg Met Asp Arg Ile Gly Ala Gln Ser Gly Leu Gly
   1 5 10 15
   Cys
<210> 475
   <211> 22
   <212> PRT
   <213> Artificial Sequence
<220>
   <223> Description of Artificial Sequence: Synthetic Peptide
<400> 475
   Cys Phe Gly Gly Arg Met Asp Arg Ile Gly Ala Gln Ser Gly Leu Gly
   1 5 10 15
   Cys Asn Ser Phe Arg Tyr
   20
<210> 476
   <211> 28
   <212> PRT
   <213> Artificial Sequence
<220>
   <223> Description of Artificial Sequence: Synthetic Peptide
<400> 476
   Ser Leu Arg Arg Ser Ser Cys Phe Gly Gly Arg Met Asp Arg Ile Gly
   1 5 10 15
   Ala Gln Ser Gly Leu Gly Cys Asn Ser Phe Arg Tyr
   20 25
<210> 477
   <211> 11
   <212> PRT
   <213> Artificial Sequence
<220>
   <223> Description of Artificial Sequence: Synthetic Peptide
<400> 477
   Arg Cys Gly Gly Arg Ile Asp Arg Ile Arg Cys
   1 5 10
<210> 478
   <211> 26
   <212> PRT
   <213> Artificial Sequence
<220>
   <223> Description of Artificial Sequence: Synthetic Peptide
<400> 478
   Arg Arg Ser Ser Cys Phe Gly Gly Arg Ile Asp Arg Ile Gly Ala Gln
   1 5 10 15
   Ser Gly Leu Gly Cys Asn Ser Phe Arg Tyr
   20 25
<210> 479
   <211> 26
   <212> PRT
   <213> Artificial Sequence
<220>
   <223> Description of Artificial Sequence: Synthetic Peptide
<400> 479
   Arg Arg Ser Ser Cys Phe Gly Gly Arg Met Asp Arg Ile Gly Ala Gln
   1 5 10 15
   Ser Gly Leu Gly Cys Asn Ser Phe Arg Tyr
   20 25
<210> 480
   <211> 25
   <212> PRT
   <213> Artificial Sequence
<220>
   <223> Description of Artificial Sequence: Synthetic Peptide
<400> 480
   Arg Ser Ser Cys Phe Gly Gly Arg Met Asp Arg Ile Gly Ala Gln Ser
   1 5 10 15
   Gly Leu Gly Cys Asn Ser Phe Arg Tyr
   20 25
<210> 481
   <211> 23
   <212> PRT
   <213> Artificial Sequence
<220>
   <223> Description of Artificial Sequence: Synthetic Peptide
<400> 481
   Ser Ser Cys Phe Gly Gly Arg Met Asp Arg Ile Gly Ala Gln Ser Gly
   1 5 10 15
   Leu Gly Cys Asn Ser Phe Arg
   20
<210> 482
   <211> 27
   <212> PRT
   <213> Artificial Sequence
<220>
   <223> Description of Artificial Sequence: Synthetic Peptide
<400> 482
   Ser Lys Ser Ser Ser Pro Cys Phe Gly Gly Lys Leu Asp Arg Ile Gly
   1 5 10 15
   Ser Tyr Ser Gly Leu Gly Cys Asn Ser Arg Lys
   20 25
<210> 483
   <211> 21
   <212> PRT
   <213> Artificial Sequence
<220>
   <223> Description of Artificial Sequence: Synthetic Peptide
<400> 483
   Ser Ser Cys Phe Gly Gly Arg Ile Asp Arg Ile Gly Ala Gln Ser Gly
   1 5 10 15
   Leu Gly Cys Asn Ser
   20
<210> 484
   <211> 23
   <212> PRT
   <213> Artificial Sequence
<220>
   <223> Description of Artificial Sequence: Synthetic Peptide
<400> 484
   Ser Ser Cys Phe Gly Gly Arg Ile Asp Arg Ile Gly Ala Gln Ser Gly
   1 5 10 15
   Leu Gly Cys Asn Ser Phe Arg
   20
<210> 485
   <211> 24
   <212> PRT
   <213> Artificial Sequence
<220>
   <223> Description of Artificial Sequence: Synthetic Peptide
<400> 485
   Ser Ser Cys Phe Gly Gly Arg Ile Asp Arg Ile Gly Ala Gln Ser Gly
   1 5 10 15
   Leu Gly Cys Asn Ser Phe Arg Tyr
   20
<210> 486
   <211> 28
   <212> PRT
   <213> Artificial Sequence
<220>
   <223> Description of Artificial Sequence: Synthetic Peptide
<400> 486
   Ser Leu Arg Arg Ser Ser Cys Phe Gly Gly Arg Ile Asp Arg Ile Gly
   1 5 10 15
   Ala Gln Ser Gly Leu Gly Cys Asn Ser Phe Arg Tyr
   20 25
<210> 487
   <211> 24
   <212> PRT
   <213> Artificial Sequence
<220>
   <223> Description of Artificial Sequence: synthetic Peptide
<400> 487
   Arg Ser Ser Cys Phe Gly Gly Arg Ile Asp Arg Ile Gly Ala Gln Ser
   1 5 10 15
   Gly Leu Gly Cys Asn Ser Phe Arg
   20
<210> 488
   <211> 25
   <212> PRT
   <213> Artificial Sequence
<220>
   <223> Description of Artificial Sequence: Synthetic Peptide
<400> 488
   Arg Ser Ser Cys Phe Gly Gly Arg Ile Asp Arg Ile Gly Ala Gln Ser
   1 5 10 15
   Gly Leu Gly Cys Asn Ser Phe Arg Tyr
   20 25
<210> 489
   <211> 56
   <212> PRT
   <213> Artificial Sequence
<220>
   <223> Description of Artificial Sequence: Synthetic Peptide
<400> 489
   Ser Leu Arg Arg Ser Ser Cys Phe Gly Gly Arg Met Asp Arg Ile Gly
   1 5 10 15
   Ala Gln Ser Gly Leu Gly Cys Asn Ser Phe Arg Tyr Ser Leu Arg Arg
   20 25 30
   Ser Ser Cys Phe Gly Gly Arg Met Asp Arg Ile Gly Ala Gln Ser Gly
   35 40 45
   Leu Gly Cys Asn Ser Phe Arg Tyr
   50 55
<210> 490
   <211> 32
   <212> PRT
   <213> Artificial Sequence
<220>
   <223> Description of Artificial Sequence: Synthetic Peptide
<400> 490
   Ala Gly Pro Arg Ser Leu Arg Arg Ser Ser Cys Phe Gly Gly Arg Ile
   1 5 10 15
   Asp Arg Ile Gly Ala Gln Ser Gly Leu Gly Cys Asn Ser Phe Arg Tyr
   20 25 30
<210> 491
   <211> 27
   <212> PRT
   <213> Artificial Sequence
<220>
   <223> Description of Artificial Sequence: Synthetic Peptide
<400> 491
   Ser Lys Ser Ser Ser Pro Cys Phe Gly Gly Lys Leu Asp Arg Ile Gly
   1 5 10 15
   Ser Tyr Ser Gly Leu Gly Cys Asn Ser Arg Lys
   20 25
<210> 492
   <211> 16
   <212> PRT
   <213> Artificial Sequence
<220>
   <223> Description of Artificial Sequence: Synthetic Peptide
<400> 492
   Asn Pro Met Tyr Asn Ala Val Ser Asn Ala Asp Leu Met Asp Phe Lys
   1 5 10 15
<210> 493
   <211> 15
   <212> PRT
   <213> Artificial Sequence
<220>
   <223> Description of Artificial Sequence: Synthetic Peptide
<400> 493
   Arg Ser Ser Cys Phe Gly Gly Arg Ile Asp Arg Ile Gly Ala Cys
   1 5 10 15
<210> 494
   <211> 21
   <212> PRT
   <213> Artificial Sequence
<220>
   <223> Description of Artificial Sequence: Synthetic Peptide
<400> 494
   Ser Phe Gly Gly Arg Ile Asp Arg Ile Gly Ala Gln Ser Gly Leu Gly
   1 5 10 15
   Asn Ser Phe Arg Tyr
   20
<210> 495
   <211> 28
   <212> PRT
   <213> Artificial Sequence
<220>
   <223> Description of Artificial Sequence: Synthetic Peptide
<400> 495
   Ser Leu Arg Arg Ser Ser Cys Phe Gly Gly Arg Met Asp Arg Ile Gly
   1 5 10 15
   Ala Gln Ser Gly Leu Gly Cys Asn Ser Phe Arg Tyr
   20 25
<210> 496
   <211> 21
   <212> PRT
   <213> Artificial Sequence
<220>
   <223> Description of Artificial Sequence: Synthetic Peptide
<400> 496
   Phe Ala Gly Arg Ile Asp Arg Ile Gly Ala Gln Ser Gly Leu Gly Cys
   1 5 10 15
   Asn Ser Phe Arg Tyr
   20
<210> 497
   <211> 13
   <212> PRT
   <213> Artificial Sequence
<220>
   <223> Description of Artificial Sequence: Synthetic Peptide
<400> 497
   Ser Ser Asp Arg Ser Ala Leu Leu Lys Ser Lys Leu Arg
   1 5 10
<210> 498
   <211> 30
   <212> PRT
   <213> Artificial Sequence
<220>
   <223> Description of Artificial Sequence: Synthetic Peptide
<400> 498
   Asn Pro Met Tyr Asn Ala Val Ser Asn Ala Asp Leu Met Asp Phe Lys
   1 5 10 15
   Asn Leu Leu Asp His Leu Glu Glu Lys Met Pro Leu Glu Asp
   20 25 30
<210> 499
   <211> 37
   <212> PRT
   <213> Artificial Sequence
<220>
   <223> Description of Artificial Sequence: Synthetic Peptide
<400> 499
   Glu Val Val Pro Pro Gln Val Leu Ser Glu Pro Asn Glu Glu Ala Gly
   1 5 10 15
   Ala Ala Leu Ser Pro Leu Pro Glu Val Pro Pro Trp Thr Gly Glu Val
   20 25 30
   Ser Pro Ala Gln Arg
   35
<210> 500
   <211> 20
   <212> PRT
   <213> Artificial Sequence
<220>
   <223> Description of Artificial Sequence: Synthetic Peptide
<400> 500
   Ser Ser Asp Arg Ser Ala Leu Leu Lys Ser Lys Leu Arg Ala Leu Leu
   1 5 10 15
   Thr Ala Pro Arg
   20
<210> 501
   <211> 27
   <212> PRT
   <213> Artificial Sequence
<220>
   <223> Description of Artificial Sequence: Synthetic Peptide .
<400> 501
   Ser Lys Ser Ser Ser Pro Cys Phe Gly Gly Lys Leu Asp Arg Ile Gly
   1 5 10 15
   Ser Tyr Ser Gly Leu Gly Cys Asn Ser Arg Lys
   20 25
<210> 502
   <211> 24
   <212> PRT
   <213> Artificial Sequence
<220>
   <223> Description of Artificial Sequence: Synthetic Peptide
<400> 502
   Tyr Ser Ser Cys Phe Gly Gly Arg Ile Asp Arg Ile Gly Ala Gln Ser
   1 5 10 15
   Gly Leu Gly Cys Asn Ser Phe Arg
   20
<210> 503
   <211> 21
   <212> PRT
   <213> Artificial Sequence
<220>
   <223> Description of Artificial Sequence: Synthetic Peptide
<400> 503
   Tyr Ser Ser Asp Arg Ser Ala Leu Leu Lys Ser Lys Leu Arg Ala Leu
   1 5 10 15
   Leu Thr Ala Pro Arg
   20
<210> 504
   <211> 32
   <212> PRT
   <213> Artificial Sequence
<220>
   <223> Description of Artificial Sequence: Synthetic Peptide
<400> 504
   Thr Ala Pro Arg Ser Leu Arg Arg Ser Ser Cys Phe Gly Gly Arg Met
   1 5 10 15
   Asp Arg Ile Gly Ala Gln Ser Gly Leu Gly Cys Asn Ser Phe Arg Tyr
   20 25 30
<210> 505
   <211> 24
   <212> PRT
   <213> Artificial Sequence
<220>
   <223> Description of Artificial Sequence: Synthetic Peptide
<400> 505
   Tyr Ser Ser Cys Phe Gly Gly Arg Ile Asp Arg Ile Gly Ala Gln Ser
   1 5 10 15
   Gly Leu Gly Cys Asn Ser Phe Arg
   20
<210> 506
   <211> 35
   <212> PRT
   <213> Artificial Sequence
<220>
   <223> Description of Artificial Sequence: Synthetic Peptide
<400> 506
   Ser Phe Asn Ser Cys Phe Gly Asn Arg Ile Glu Arg Ile Gly Ser Trp
   1 5 10 15
   Ser Gly Leu Gly Cys Asn Asn Val Lys Thr Gly Asn Lys Lys Arg Ile
   20 25 30
   Phe Gly Asn
   35
<210> 507
   <211> 32
   <212> PRT
   <213> Artificial Sequence
<220>
   <223> Description of Artificial Sequence: Synthetic Peptide
<400> 507
   Ser Pro Lys Met Met His Lys Ser Gly Cys Phe Gly Arg Arg Leu Asp
   1 5 10 15
   Arg Ile Gly Ser Leu Ser Gly Leu Gly Cys Asn Val Leu Arg Lys Tyr
   20 25 30
<210> 508
   <211> 22
   <212> PRT
   <213> Artificial Sequence
<220>
   <223> Description of Artificial Sequence: Synthetic Peptide
<400> 508
   Gly Trp Asn Arg Gly Cys Phe Gly Leu Lys Leu Asp Arg Ile Gly Ser
   1 5 10 15
   Leu Ser Gly Leu Gly Cys
   20
<210> 509
   <211> 32
   <212> PRT
   <213> Artificial Sequence
<220>
   <223> Description of Artificial Sequence: Synthetic Peptide
<400> 509
   Ser Pro Lys Met Val Gln Gly Ser Gly Cys Phe Gly Arg Lys Met Asp
   1 5 10 15
   Arg Ile Ser Ser Ser Ser Gly Leu Gly Cys Lys Val Leu Arg Arg His
   20 25 30
<210> 510
   <211> 45
   <212> PRT
   <213> Artificial Sequence
<220>
   <223> Description of Artificial Sequence: Synthetic Peptide
<400> 510
   Ser Gln Gly Ser Thr Leu Arg Val Gln Gln Arg Pro Gln Asn Ser Lys
   1 5 10 15
   Val Thr His Ile Ser Ser Cys Phe Gly His Lys Ile Asp Arg Ile Gly
   20 25 30
   Ser Val Ser Arg Leu Gly Cys Asn Ala Leu Lys Leu Leu
   35 40 45
<210> 511
   <211> 26
   <212> PRT
   <213> Artificial Sequence
<220>
   <223> Description of Artificial Sequence: Synthetic Peptide
<400> 511
   Asp Ser Gly Cys Phe Gly Arg Arg Leu Asp Arg Ile Gly Ser Leu Ser
   1 5 10 15
   Gly Leu Gly Cys Asn Val Leu Arg Arg Tyr
   20 25
<210> 512
   <211> 32
   <212> PRT
   <213> Artificial Sequence
<220>
   <223> Description of Artificial Sequence: Synthetic Peptide
<400> 512
   Ser Pro Lys Thr Met Arg Asp Ser Gly Cys Phe Gly Arg Arg Leu Asp
   1 5 10 15
   Arg Ile Gly Ser Leu Ser Gly Leu Gly Cys Asn Val Leu Arg Arg Tyr
   20 25 30
<210> 513
   <211> 32
   <212> PRT
   <213> Artificial Sequence
<220>
   <223> Description of Artificial Sequence: Synthetic Peptide
<400> 513
   Asn Ser Lys Met Ala His Ser Ser Ser Cys Phe Gly Gln Lys Ile Asp
   1 5 10 15
   Arg Ile Gly Ala Val Ser Arg Leu Gly Cys Asp Gly Leu Arg Leu Phe
   20 25 30
<210> 514
   <211> 45
   <212> PRT
   <213> Artificial Sequence
<220> .
   <223> Description of Artificial Sequence: Synthetic Peptide
<400> 514
   Ser Gln Asp Ser Ala Phe Arg Ile Gln Glu Arg Leu Arg Asn Ser Lys
   1 5 10 15
   Met Ala His Ser Ser Ser Cys Phe Gly Gln Lys Ile Asp Arg Ile Gly
   20 25 30
   Ala Val Ser Arg Leu Gly Cys Asp Gly Leu Arg Leu Phe
   35 40 45

<210> 515
   <211> 33
   <212> PRT
   <213> Artificial Sequence
<220>
   <223> Description of Artificial Sequence: Synthetic Peptide
<400> 515
   Tyr Ser Pro Lys Met Val Gln Gly Ser Gly Cys Phe Gly Arg Lys Met
   1 5 10 15
   Asp Arg Leu Ser Ser Ser Ser Gly Leu Gly Cys Lys Val Leu Arg Arg
   20 25 30
   His
<210> 516
   <211> 32 .
   <212> PRT
   <213> Artificial Sequence
<220>
   <223> Description of Artificial Sequence: Synthetic Peptide
<400> 516
   Ser Pro Lys Met Val Gln Gly Ser Gly Cys Phe Gly Arg Lys Met Asp
   1 5 10 15
   Arg Ile Ser Ser Ser Ser Gly Leu Gly Cys Lys Val Leu Arg Arg His
   20 25 30
<210> 517
   <211> 22
   <212> PRT
   <213> Artificial Sequence
<220>
   <223> Description of Artificial Sequence: Synthetic Peptide
<400> 517
   Gly Leu Ser Lys Gly Cys Phe Gly Leu Lys Leu Asp Arg Ile Gly Ser
   1 5 10 15
   Met Ser Gly Leu Gly Cys
   20
<210> 518
   <211> 22
   <212> PRT
   <213> Artificial Sequence
<220>
   <223> Description of Artificial Sequence: Synthetic Peptide
<400> 518
   Gly Leu Ser Arg Ser Cys Phe Gly Val Lys Leu Asp Arg Ile Gly Ser
   1 5 10 15
   Met Ser Gly Leu Gly Cys
   20
<210> 519
   <211> 53
   <212> PRT
   <213> Artificial Sequence
<220>
   <223> Description of Artificial Sequence: Synthetic Peptide
<400> 519
   Asp Leu Arg Val Asp Thr Lys Ser Arg Ala Ala Trp Ala Arg Leu Leu
   1 5 10 15
   Gln Glu His Pro Asn Ala Arg Lys Tyr Lys Gly Ala Asn Lys Lys Gly
   20 25 30
   Leu Ser Lys Gly Cys Phe Gly Leu Lys Leu Asp Arg Ile Gly Ser Met
   35 40 45
   Ser Gly Leu Gly Cys
   50
<210> 520
   <211> 53
   <212> PRT
   <213> Artificial Sequence
<220>
   <223> Description of Artificial Sequence: Synthetic Peptide
<400> 520
   Asp Leu Arg Val Asp Thr Lys Ser Arg Ala Ala Trp Ala Arg Leu Leu
   1 5 10 15
   His Glu His Pro Asn Ala Arg Lys Tyr Lys Gly Gly Asn Lys Lys Gly
   20 25 30
   Leu Ser Lys Gly Cys Phe Gly Leu Lys Leu Asp Arg Ile Gly Ser Met
   35 40 45
   Ser Gly Leu Gly Cys
   50
<210> 521
   <211> 27
   <212> PRT
   <213> Artificial Sequence
<220>
   <223> Description of Artificial Sequence: Synthetic Peptide
<400> 521
   Lys Pro Gly Thr Pro Pro Lys Val Pro Arg Thr Pro Pro Gly Glu Glu
   1 5 10 15
   Leu Ala Glu Pro Gln Ala Ala Gly Gly Asn Gln
   20 25
<210> 522
   <211> 21
   <212> PRT
   <213> Artificial Sequence
<220>
   <223> Description of Artificial Sequence: Synthetic Peptide
<400> 522
   Gly Asp Lys Thr Pro Gly Gly Gly Gly Ala Asn Leu Lys Gly Asp Arg
   1 5 10 15
   Ser Arg Leu Leu Arg
   20
<210> 523
   <211> 2.7
   <212> PRT
   <213> Artificial Sequence
<220>
   <223> Description of Artificial Sequence: Synthetic Peptide
<400> 523
   Gly Leu Ser Lys Gly Cys Phe Gly Leu Lys Leu Asp Arg Ile Gly Ser
   1 5 10 15
   Met Ser Gly Leu Gly Cys Asn Ser Phe Arg Tyr
   20 25
<210> 524
   <211> 23
   <212> PRT
   <213> Artificial Sequence
<220>
   <223> Description of Artificial Sequence: Synthetic Peptide
<400> 524
   Tyr Gly Leu Ser Lys Gly Cys Phe Gly Leu Lys Leu Asp Arg Ile Gly
   1 5 10 15
   Ser Met Ser Gly Leu Gly Cys
   20
<210> 525
   <211> 7
   <212> PRT
   <213> Artificial Sequence
<220>
   <223> Description of Artificial Sequence: Synthetic Peptide
<400> 525
   Asp Ala Phe Val Ala Leu Met
   1 5
<210> 526
   <211> 7
   <212> PRT
   <213> Artificial Sequence
<220>
   <223> Description of Artificial Sequence: Synthetic Peptide
<400> 526
   Asp Ala Phe Val Ala Leu Met
   1 5
<210> 527
   <211> 11
   <212> PRT
   <213> Artificial Sequence
<220>
   <223> Description of Artificial Sequence: Synthetic Peptide
<400> 527
   Glu Pro Ser Lys Asp Ala Phe Ile Gly Leu Met
   1 5 10
<210> 528
   <211> 9
   <212> PRT
   <213> Artificial Sequence
<220>
   <223> Description of Artificial Sequence: Synthetic Peptide
<400> 528
   Gly Pro Ser Gly Phe Tyr Gly Val Arg
   1 5
<210> 529
   <211> 10
   <212> PRT
   <213> Artificial Sequence
<220>
   <223> Description of Artificial Sequence: Synthetic Peptide
<400> 529
   Ala Pro Leu Ser Gly Phe Tyr Gly Val Arg
   1 5 10
<210> 530
   <211> 7
   <212> PRT
   <213> Artificial Sequence
<220>
   <223> Description of Artificial Sequence: Synthetic Peptide
<400> 530
   Asp Ser Phe Val Gly Leu Met
   1 5
<210> 531
   <211> 10
   <212> PRT
   <213> Artificial Sequence
<220>
   <223> Description of Artificial Sequence: Synthetic Peptide
<400> 531
   His Lys Thr Asp Ser Phe Val Gly Leu Met
   1 5 10
<210> 532
   <211> 10
   <212> PRT
   <213> Artificial Sequence
<220>
   <223> Description of Artificial Sequence: Synthetic Peptide
<400> 532
   His Lys Ile Asn Ser Phe Val Gly Leu Met
   1 5 10
<210> 533
   <211> 11
   <212> PRT
   <213> Artificial Sequence
<220>
   <223> Description of Artificial Sequence: Synthetic Peptide
<400> 533
   Tyr His Lys Thr Asp Ser Phe Val Gly Leu Met
   1 5 10
<210> 534
   <211> 10
   <212> PRT
   <213> Artificial Sequence
<220>
   <223> Description of Artificial Sequence: Synthetic Peptide
<400> 534
   His Lys Thr Asp Ser Tyr Val Gly Leu Met
   1 5 10
<210> 535
   <211> 7
   <212> PRT
   <213> Artificial Sequence
<220>
   <223> Description of Artificial Sequence: Synthetic Peptide
<400> 535
   Lys Asp Ser Phe Val Gly Met
   1 5
<210> 536
   <211> 6
   <212> PRT
   <213> Artificial Sequence
<220>
   <223> Description of Artificial Sequence: Synthetic Peptide
<400> 536
   Arg Ala Trp Phe Pro Pro
   1 5
<210> 537
   <211> 6
   <212> PRT
   <213> Artificial Sequence
<220>
   <223> Description of Artificial Sequence: Synthetic Peptide
<400> 537
   Ala Ala Trp Phe Pro Pro
   1 5
<210> 538
   <211> 6
   <212> PRT
   <213> Artificial Sequence
<220>
   <223> Description of Artificial Sequence: Synthetic Peptide
<400> 538
   Ala Ala Trp Phe Pro Pro
   1 5
<210> 539
   <211> 7
   <212> PRT
   <213> Artificial Sequence
<220>
   <223> Description of Artificial Sequence: Synthetic Peptide
<400> 539
   Asp Ser Phe Val Ala Leu Met
   1 5
<210> 540
   <211> 6
   <212> PRT
   <213> Artificial Sequence
<220>
   <223> Description of Artificial Sequence: Synthetic Peptide
<400> 540
   Asp Ser Phe Val Gly Leu
   1 5
<210> 541
   <211> 7
   <212> PRT
   <213> Artificial Sequence
<220>
   <223> Description of Artificial Sequence: Synthetic Peptide
<400> 541
   Asp Ser Phe Trp Ala Leu Met
   1 5
<210> 542
   <211> 7
   <212> PRT
   <213> Artificial Sequence
<220>
   <223> Description of Artificial Sequence: Synthetic Peptide
<400> 542
   Asp Tyr Trp Val Trp Trp Arg
   1 5
<210> 543
   <211> 7
   <212> PRT
   <213> Artificial Sequence
<220>
   <223> Description of Artificial Sequence: Synthetic Peptide
<400> 543
   Asp Tyr Trp Val Trp Trp Lys
   1 5
<210> 544
   <211> 10
   <212> PRT
   <213> Artificial Sequence
<220>
   <223> Description of Artificial Sequence: Synthetic Peptide
<400> 544
   Asp Met His Asp Phe Phe Val Gly Leu Met
   1 5 10
<210> 545
   <211> 9
   <212> PRT
   <213> Artificial Sequence
<220>
   <223> Description of Artificial Sequence: Synthetic Peptide
<400> 545
   Asp Met His Asp Phe Phe Gly Leu Met
   1 5
<210> 546
   <211> 10
   <212> PRT
   <213> Artificial Sequence
<220>
   <223> Description of Artificial Sequence: Synthetic Peptide
<400> 546
   Asp Met His Asp Phe Phe Pro Gly Leu Met
   1 5 10
<210> 547
   <211> 11
   <212> PRT
   <213> Artificial Sequence
<220>
   <223> Description of Artificial Sequence: Synthetic Peptide
<400> 547
   Tyr Asp Met His Asp Phe Phe Val Gly Leu Met
   1 5 10
<210> 548
   <211> 9
   <212> PRT
   <213> Artificial Sequence
<220>
   <223> Description of Artificial Sequence: Synthetic Peptide
<400> 548
   Asp Pro His Asp Phe Trp Val Trp Leu
   1 5
<210> 549
   <211> 10
   <212> PRT
   <213> Artificial Sequence
<220>
   <223> Description of Artificial Sequence: Synthetic Peptide
<400> 549
   Gly Asn Leu Trp Ala Thr Gly His Phe Met
   1 5 10
<210> 550
   <211> 30
   <212> PRT
   <213> Artificial Sequence
<220>
   <223> Description of Artificial Sequence: Synthetic Peptide
<400> 550
   Leu Ser Trp Asp Leu Pro Glu Pro Arg Ser Arg Ala Gly Lys Ile Arg
   1 5 10 15
   Val His Pro Arg Gly Asn Leu Trp Ala Thr Gly His Phe Met
   20 25 30
<210> 551
   <211> 32
   <212> PRT
   <213> Artificial Sequence
<220>
   <223> Description of Artificial Sequence: Synthetic Peptide
<400> 551
   Ala Pro Leu Ser Trp Asp Leu Pro Glu Pro Arg Ser Arg Ala Gly Lys
   1 5 10 15
   Ile Arg Val His Pro Arg Gly Asn Leu Trp Ala Thr Gly His Phe Met
   20 25 30
<210> 552
   <211> 5
   <212> PRT
   <213> Artificial Sequence
<220>
   <223> Description of Artificial Sequence: Synthetic Peptide
<400> 552
   Cys Tyr Trp Val Cys
   1 5
<210> 553
   <211> 10
   <212> PRT
   <213> Artificial Sequence
<220>
   <223> Description of Artificial Sequence: Synthetic Peptide
<400> 553
   Gly Asn His Trp Ala Val Gly His Leu Met
   1 5 10
<210> 554
   <211> 6
   <212> PRT
   <213> Artificial Sequence
<220>
   <223> Description of Artificial Sequence: Synthetic Peptide
<400> 554
   Lys Ile Pro Tyr Ile Leu
   1 5
<210> 555
   <211> 8
   <212> PRT
   <213> Artificial Sequence
<220>
   <223> Description of Artificial Sequence: Synthetic Peptide
<400> 555
   Tyr Phe Leu Phe Arg Pro Arg Asn
   1 5
<210> 556
   <211> 25
   <212> PRT
   <213> Artificial Sequence
<220>
   <223> Description of Artificial Sequence: Synthetic Peptide
<400> 556
   Phe Lys Val Asp Glu Glu Phe Gln Gly Pro Ile Val Ser Gln Asn Arg
   1 5 10 15
   Arg Tyr Phe Leu Phe Arg Pro Arg Asn
   20 25
<210> 557
   <211> 23
   <212> PRT
   <213> Artificial Sequence
<220>
   <223> Description of Artificial Sequence: synthetic Peptide
<400> 557
   Tyr Lys Val Asn Glu Tyr Gln Gly Pro Val Ala Pro Ser Gly Gly Phe
   1 5 10 15
   Phe Leu Phe Arg Pro Arg Asn
   20
<210> 558
   <211> 21
   <212> PRT
   <213> Artificial Sequence
<220>
   <223> Description of Artificial Sequence: Synthetic Peptide
<400> 558
   Asp Ala Gly His Gly Gln Ile Ser His Lys Arg His Lys Thr Asp Ser
   1 5 10 15
   Phe Val Gly Leu Met
   20
<210> 559
   <211> 10
   <212> PRT
   <213> Artificial Sequence
<220>
   <223> Description of Artificial Sequence: Synthetic Peptide
<400> 559
   Pro Asn Pro Asp Glu Phe Val Gly Leu Met
   1 5 10
<210> 560
   <211> 9
   <212> PRT
   <213> Artificial Sequence
<220>
   <223> Description of Artificial Sequence: Synthetic Peptide
<400> 560
   Glu Leu Trp Ala Val Gly Ser Phe Met
   1 5
<210> 561
   <211> 9
   <212> PRT
   <213> Artificial Sequence
<220>
   <223> Description of Artificial Sequence: Synthetic Peptide
<400> 561
   Glu Leu Trp Ala Val Gly Ser Leu Met
   1 5
<210> 562
   <211> 11
   <212> PRT
   <213> Artificial Sequence
<220>
   <223> Description of Artificial Sequence: synthetic Peptide
<400> 562
   Glu Ala Asp Pro Asn Lys Phe Tyr Gly Leu Met
   1 5 10
<210> 563
   <211> 8
   <212> PRT
   <213> Artificial Sequence
<220>
   <223> Description of Artificial Sequence: Synthetic Peptide
<400> 563
   Glu Ala Asp Pro Asn Lys Phe Tyr
   1 5
<210> 564
   <211> 18
   <212> PRT
   <213> Artificial Sequence
<220>
   <223> Description of Artificial Sequence: Synthetic Peptide
<400> 564
   Ser Pro Ser Asn Ser Lys Cys Pro Asp Gly Pro Asp Cys Phe Val Gly
   1 5 10 15
   Leu Met
<210> 565
   <211> 5
   <212> PRT
   <213> Artificial Sequence
<220>
   <223> Description of Artificial Sequence: Synthetic Peptide
<400> 565
   Asp Phe Gly Leu Met
   1 5
<210> 566
   <211> 6
   <212> PRT
   <213> Artificial Sequence
<220>
   <223> Description of Artificial Sequence: Synthetic Peptide
<400> 566
   Asp Asp Phe Gly Leu Met
   1 5
<210> 567
   <211> 10
   <212> PRT
   <213> Artificial Sequence
<220>
   <223> Description of Artificial Sequence: Synthetic Peptide
<400> 567
   Gly Ser His Trp Ala Val Gly His Leu Met
   1 5 10
<210> 568
   <211> 23
   <212> PRT
   <213> Artificial Sequence
<220>
   <223> Description of Artificial Sequence: Synthetic Peptide
<400> 568
   Gly Lys Arg Asp Ala Asp Ser Ser Ile Glu Lys Gln Val Ala Leu Leu
   1 5 10 15
   Lys Ala Leu Tyr Gly His Gly
   20
<210> 569
   <211> 19
   <212> PRT
   <213> Artificial Sequence
<220>
   <223> Description of Artificial Sequence: Synthetic Peptide
<400> 569
   Ala Leu Asn Ser Val Ala Tyr Glu Arg Ser Ala Met Gln Asn Tyr Glu
   1 5 10 15
   Arg Arg Arg
<210> 570
   <211> 17
   <212> PRT
   <213> Artificial Sequence
<220>
   <223> Description of Artificial Sequence: Synthetic Peptide
<400> 570
   Lys Trp Cys Phe Arg Val Cys Tyr Arg Gly Ile Cys Tyr Arg Arg Cys
   1 5 10 15
   Arg
<210> 571
   <211> 8
   <212> PRT
   <213> Artificial Sequence
<220>
   <223> Description of Artificial Sequence: Synthetic Peptide
<400> 571
   Glu Gly Lys Arg Pro Trp Ile Leu
   1 5
<210> 572
   <211> 25
   <212> PRT
   <213> Artificial Sequence
<220>
   <223> Description of Artificial Sequence: Synthetic Peptide
<400> 572
   Met Leu Thr Lys Phe Glu Thr Lys Ser Ala Arg Val Lys Gly Leu Ser
   1 5 10 15
   Phe His Pro Lys Arg Pro Trp Ile Leu
   20 25
<210> 573
   <211> 10
   <212> PRT
   <213> Artificial Sequence
<220>
   <223> Description of Artificial Sequence: Synthetic Peptide
<400> 573
   Asp Met His Asp Phe Phe Val Gly Leu Met
   1 5 10
<210> 574
   <211> 10
   <212> PRT
   <213> Artificial Sequence
<220>
   <223> Description of Artificial Sequence: Synthetic Peptide
<400> 574
   His Lys Thr Asp Ser Phe Val Gly Leu Met
   1 5 10
<210> 575
   <211> 24
   <212> PRT
   <213> Artificial Sequence
<220>
   <223> Description of Artificial Sequence: Synthetic Peptide
<400> 575
   Asp Ala Asp Ser Ser Ile Glu Lys Gln Val Ala Leu Leu Lys Ala Leu
   1 5 10 15
   Tyr Gly His Gly Gln Ile Ser His
   20
<210> 576
   <211> 7
   <212> PRT
   <213> Artificial Sequence
<220>
   <223> Description of Artificial Sequence: Synthetic Peptide
<400> 576
   Glu Gln Trp Phe Trp Trp Met
   1 5
<210> 577
   <211> 5
   <212> PRT
   <213> Artificial Sequence
<220>
   <223> Description of Artificial Sequence: Synthetic Peptide
<400> 577
   Arg Phe Phe Leu Met
   1 5
<210> 578
   <211> 11
   <212> PRT
   <213> Artificial Sequence
<220>
   <223> Description of Artificial Sequence: synthetic Peptide
<400> 578
   Arg Pro Arg Pro Gln Gln Phe Phe Gly Leu Met
   1 5 10
<210> 579
   <211> 6
   <212> PRT
   <213> Artificial Sequence
<220>
   <223> Description of Artificial Sequence: Synthetic Peptide
<400> 579
   Arg Trp Phe Trp Leu Met
   1 5
<210> 580
   <211> 2
   <212> PRT
   <213> Artificial Sequence
<220>
   <223> Description of Artificial Sequence: Synthetic Peptide
<400> 580
   Trp Met
   1
<210> 581
   <211> 5
   <212> PRT
   <213> Artificial Sequence
<220>
   <223> Description of Artificial Sequence: Synthetic Peptide
<400> 581
   Arg Phe Phe Leu Met
   1 5
<210> 582
   <211> 6
   <212> PRT
   <213> Artificial Sequence
<220>
   <223> Description of Artificial Sequence: Synthetic Peptide
<400> 582
   Arg Trp Phe Trp Leu Met
   1 5
<210> 583
   <211> 8
   <212> PRT
   <213> Artificial Sequence
<220>
   <223> Description of Artificial Sequence: Synthetic Peptide
<400> 583
   Ala Gln Gln Phe Phe Gly Leu Met
   1 5
<210> 584
   <211> 6
   <212> PRT
   <213> Artificial Sequence
<220>
   <223> Description of Artificial Sequence: Synthetic Peptide
<400> 584
   Tyr Phe Phe His Leu Met
   1 5
<210> 585
   <211> 11
   <212> PRT
   <213> Artificial Sequence
<220>
   <223> Description of Artificial Sequence: Synthetic Peptide
<400> 585
   Arg Pro Lys Pro Gln Gln Phe Tyr Gly Leu Met
   1 5 10
<210> 586
   <211> 10
   <212> PRT
   <213> Artificial Sequence
<220>
   <223> Description of Artificial Sequence: Synthetic Peptide
<400> 586
   Arg Pro Lys Pro Gln Gln Phe Phe Leu Met
   1 5 10
<210> 587
   <211> 11
   <212> PRT
   <213> Artificial Sequence
<220>
   <223> Description of Artificial Sequence: synthetic Peptide
<400> 587
   Arg Pro Lys Pro Gln Gln Phe Phe His Leu Met
   1 5 10
<210> 588
   <211> 11
   <212> PRT
   <213> Artificial Sequence
<220>
   <223> Description of Artificial Sequence: Synthetic Peptide
<400> 588
   Arg Pro Lys Pro Gln Gln Phe Phe Trp Leu Met
   1 5 10
<210> 589
   <211> 11
   <212> PRT
   <213> Artificial Sequence
<220>
   <223> Description of Artificial Sequence: Synthetic Peptide
<400> 589
   Arg Pro Lys Pro Gln Gln Trp Phe Trp Leu Met
   1 5 10
<210> 590
   <211> 11
   <212> PRT
   <213> Artificial Sequence
<220>
   <223> Description of Artificial Sequence: Synthetic Peptide
<400> 590
   Arg Pro Lys Pro Phe Gln Trp Phe Trp Leu Leu
   1 5 10
<210> 591
   <211> 11
   <212> PRT
   <213> Artificial Sequence
<220>
   <223> Description of Artificial Sequence: Synthetic Peptide
<400> 591
   Arg Pro Lys Pro Gln Gln Phe Phe Pro Leu Met
   1 5 10
<210> 592
   <211> 8
   <212> PRT
   <213> Artificial Sequence
<220>
   <223> Description of Artificial Sequence: synthetic Peptide
<400> 592
   Pro Gln Gln Phe Phe Gly Leu Met
   1 5
<210> 593
   <211> 5
   <212> PRT
   <213> Artificial Sequence
<220>
   <223> Description of Artificial Sequence: Synthetic Peptide
<400> 593
   Gln Phe Phe Leu Met
   1 5
<210> 594
   <211> 5
   <212> PRT
   <213> Artificial Sequence
<220>
   <223> Description of Artificial Sequence: Synthetic Peptide
<400> 594
   Gln Phe Phe Leu Met
   1 5

<210> 595
   <211> 6
   <212> PRT
   <213> Artificial Sequence
<220>
   <223> Description of Artificial Sequence: Synthetic Peptide
<400> 595
   Gln Phe Phe Gly Leu Met
   1 5
<210> 596
   <211> 7
   <212> PRT
   <213> Artificial Sequence
<220>
   <223> Description of Artificial Sequence: Synthetic Peptide
<400> 596
   Gln Gln Phe Phe Gly Leu Met
   1 5
<210> 597
   <211> 6
   <212> PRT
   <213> Artificial Sequence
<220>
   <223> Description of Artificial Sequence: Synthetic Peptide
<400> 597
   Gln Phe Phe Gly Leu Met
   1 5
<210> 598
   <211> 10
   <212> PRT
   <213> Artificial Sequence
<220>
   <223> Description of Artificial Sequence: Synthetic Peptide
<400> 598
   Arg Pro Lys Pro Gln Gln Phe Phe Leu Met
   1 5 10
<210> 599
   <211> 10
   <212> PRT
   <213> Artificial Sequence
<220>
   <223> Description of Artificial Sequence: Synthetic Peptide
<400> 599
   Arg Pro Lys Pro Gln Gln Phe Phe Gly Leu
   1 5 10
<210> 600
   <211> 8
   <212> PRT
   <213> Artificial Sequence
<220>
   <223> Description of Artificial Sequence: Synthetic Peptide
<400> 600
   Pro Gln Gln Phe Phe Gly Leu Met
   1 5
<210> 601
   <211> 6
   <212> PRT
   <213> Artificial Sequence
<220>
   <223> Description of Artificial Sequence: Synthetic Peptide
<400> 601
   Glu Phe Phe Gly Leu Met
   1 5
<210> 602
   <211> 6
   <212> PRT
   <213> Artificial Sequence
<220>
   <223> Description of Artificial Sequence: Synthetic Peptide
<400> 602
   Glu Phe Phe Pro Leu Met
   1 5
<210> 603
   <211> 10
   <212> PRT
   <213> Artificial Sequence
<220>
   <223> Description of Artificial Sequence: Synthetic Peptide
<400> 603
   Arg Pro Lys Pro Gln Gln Phe Phe Gly Leu
   1 5 10
<210> 604
   <211> 5
   <212> PRT
   <213> Artificial Sequence
<220>
   <223> Description of Artificial Sequence: Synthetic Peptide
<400> 604
   Phe Phe Gly Leu Met
   1 5
<210> 605
   <211> 11
   <212> PRT
   <213> Artificial Sequence
<220>
   <223> Description of Artificial Sequence: Synthetic Peptide
<400> 605
   Arg Pro Lys Pro Gln Gln Phe Phe Pro Leu Met
   1 5 10
<210> 606
   <211> 5
   <212> PRT
   <213> Artificial Sequence
<220>
   <223> Description of Artificial Sequence: Synthetic Peptide
<400> 606
   Phe Phe Pro Leu Met
   1 5
<210> 607
   <211> 7
   <212> PRT
   <213> Artificial Sequence
<220>
   <223> Description of Artificial Sequence: Synthetic Peptide
<400> 607
   Gln Gln Phe Phe Gly Leu Met
   1 5
<210> 608
   <211> 10
   <212> PRT
   <213> Artificial Sequence
<220>
   <223> Description of Artificial Sequence: Synthetic Peptide
<400> 608
   Arg Pro Lys Pro Gln Gln Phe Phe Leu Met
   1 5 10
<210> 609
   <211> 6
   <212> PRT
   <213> Artificial Sequence
<220>
   <223> Description of Artificial Sequence: Synthetic Peptide
<400> 609
   Glu Phe Phe Pro Leu Met
   1 5
<210> 610
   <211> 11
   <212> PRT
   <213> Artificial Sequence
<220>
   <223> Description of Artificial Sequence: Synthetic Peptide
<400> 610
   Arg Pro Lys Pro Gln Gln Trp Phe Trp Leu Leu
   1 5 10
<210> 611
   <211> 9
   <212> PRT
   <213> Artificial Sequence
<220>
   <223> Description of Artificial Sequence: Synthetic Peptide
<400> 611
   Lys Pro Pro Phe Asn Trp Phe Trp Leu
   1 5
<210> 612
   <211> 11
   <212> PRT
   <213> Artificial Sequence
<220>
   <223> Description of Artificial Sequence: Synthetic Peptide
<400> 612
   Arg Pro Lys Pro Gln Gln Phe Phe Gly Leu Met
   1 5 10
<210> 613
   <211> 11
   <212> PRT
   <213> Artificial Sequence
<220>
   <223> Description of Artificial Sequence: Synthetic Peptide
<400> 613
   Lys Pro Arg Pro Gln Gln Phe Ile Gly Leu Met
   1 5 10
<210> 614
   <211> 11
   <212> PRT
   <213> Artificial Sequence
<220>
   <223> Description of Artificial Sequence: Synthetic Peptide
<400> 614
   Lys Pro Arg Pro His Gln Phe Phe Gly Leu Met
   1 5 10
<210> 615
   <211> 3
   <212> PRT
   <213> Artificial Sequence
<220>
   <223> Description of Artificial Sequence: Synthetic Peptide
<400> 615
   Gln Trp Phe
   1
<210> 616
   <211> 14
   <212> PRT
   <213> Artificial Sequence
<220>
   <223> Description of Artificial Sequence: Synthetic Peptide
<400> 616
   Arg Pro Lys Pro Gln Gln Phe Phe Gly Leu Met Gly Lys Arg
   1 5 10
<210> 617
   <211> 4
   <212> PRT
   <213> Artificial Sequence
<220>
   <223> Description of Artificial Sequence: Synthetic Peptide
<400> 617
   Arg Pro Lys Pro
   1
<210> 618
   <211> 6
   <212> PRT
   <213> Artificial Sequence
<220>
   <223> Description of Artificial Sequence: Synthetic Peptide
<400> 618
   Arg Pro Lys Pro Gln Gln
   1 5
<210> 619
   <211> 7
   <212> PRT
   <213> Artificial Sequence
<220>
   <223> Description of Artificial Sequence: Synthetic Peptide
<400> 619
   Arg Pro Lys Pro Gln Gln Phe
   1 5
<210> 620
   <211> 9
   <212> PRT
   <213> Artificial Sequence
<220>
   <223> Description of Artificial Sequence: Synthetic Peptide
<400> 620
   Arg Pro Lys Pro Gln Gln Phe Phe Gly
   1 5
<210> 621
   <211> 10
   <212> PRT
   <213> Artificial Sequence
<220>
   <223> Description of Artificial Sequence: Synthetic Peptide
<400> 621
   Pro Lys Pro Gln Gln Phe Phe Gly Leu Met
   1 5 10
<210> 622
   <211> 9
   <212> PRT
   <213> Artificial Sequence
<220>
   <223> Description of Artificial Sequence: Synthetic Peptide
<400> 622
   Lys Pro Gln Gln Phe Phe Gly Leu Met
   1 5
<210> 623
   <211> 4
   <212> PRT
   <213> Artificial Sequence
<220>
   <223> Description of Artificial Sequence: Synthetic Peptide
<400> 623
   Phe Gly Leu Met
   1
<210> 624
   <211> 3
   <212> PRT
   <213> Artificial Sequence
<220>
   <223> Description of Artificial Sequence: Synthetic Peptide
<400> 624
   Gly Leu Met
   1
<210> 625
   <211> 11
   <212> PRT
   <213> Artificial Sequence
<220>
   <223> Description of Artificial Sequence: Synthetic Peptide
<400> 625
   Arg Pro Lys Pro Gln Gln Phe Phe Gly Leu Met
   1 5 10
<210> 626
   <211> 11
   <212> PRT
   <213> Artificial Sequence
<220>
   <223> Description of Artificial Sequence: Synthetic Peptide
<400> 626
   Arg Pro Lys Pro Gln Gln Phe Phe Gly Leu Met
   1 5 10
<210> 627
   <211> 10
   <212> PRT
   <213> Artificial Sequence
<220>
   <223> Description of Artificial Sequence: Synthetic Peptide
<400> 627
   Arg Pro Lys Pro Gln Gln Phe Tyr Gly Leu
   1 5 10
<210> 628
   <211> 7
   <212> PRT
   <213> Artificial Sequence
<220>
   <223> Description of Artificial Sequence: Synthetic Peptide
<400> 628
   Asp Arg Val Tyr Ile His Ala
   1 5
<210> 629
   <211> 11
   <212> PRT
   <213> Artificial Sequence
<220>
   <223> Description of Artificial Sequence: Synthetic Peptide
<400> 629
   Ala Pro Gly Asp Arg Ile Tyr Val His Pro Phe
   1 5 10
<210> 630
   <211> 7
   <212> PRT
   <213> Artificial Sequence
<220>
   <223> Description of Artificial Sequence: Synthetic Peptide
<400> 630
   Gly Val Tyr Val His Pro Val
   1 5
<210> 631
   <211> 15
   <212> PRT
   <213> Artificial Sequence
<220>
   <223> Description of Artificial Sequence: Synthetic Peptide
<400> 631
   Glu Asn Gly Leu Pro Val His Leu Asp Gln Ser Ile Phe Arg Arg
   1 5  10 15
<210> 632
   <211> 15
   <212> PRT
   <213> Artificial Sequence
<220>
   <223> Description of Artificial Sequence: Synthetic Peptide
<400> 632
   Glu Asn Gly Leu Pro Val His Leu Asp Gln Ser Ile Phe Arg Arg
   1 5 10 15
<210> 633
   <211> 10
   <212> PRT
   <213> Artificial Sequence
<220>
   <223> Description of Artificial Sequence: Synthetic Peptide
<400> 633
   Gly Leu Pro Pro Arg Pro Lys Ile Pro Pro
   1 5 10
<210> 634
   <211> 10
   <212> PRT
   <213> Artificial Sequence
<220>
   <223> Description of Artificial Sequence: Synthetic Peptide
<400> 634
   Gly Leu Pro Pro Gly Pro Pro Ile Pro Pro
   1 5 10
<210> 635
   <211> 8
   <212> PRT
   <213> Artificial Sequence
<220>
   <223> Description of Artificial Sequence: Synthetic Peptide
<400> 635
   Arg Pro Gly Phe Ser Pro Phe Arg
   1 5
<210> 636
   <211> 8
   <212> PRT
   <213> Artificial Sequence
<220>
   <223> Description of Artificial Sequence: Synthetic Peptide
<400> 636
   Arg Pro Gly Phe Ser Pro Phe Arg
   1 5
<210> 637
   <211> 10
   <212> PRT
   <213> Artificial Sequence
<220>
   <223> Description of Artificial Sequence: Synthetic Peptide
<400> 637
   Asp Arg Val Tyr Ile His Pro Phe His Leu
   1 5 10
<210> 638
   <211> 3
   <212> PRT
   <213> Artificial Sequence
<220>
   <223> Description of Artificial Sequence: Synthetic Peptide
<400> 638
   Gly Gly Gly
   1
<210> 639
   <211> 7
   <212> PRT
   <213> Artificial Sequence
<220>
   <223> Description of Artificial Sequence: Synthetic Peptide
<400> 639
   Asp Arg Val Tyr Ile His Pro
   1 5
<210> 640
   <211> 7
   <212> PRT
   <213> Artificial Sequence
<220>
   <223> Description of Artificial Sequence: Synthetic Peptide
<400> 640
   Cys Tyr Trp Lys Val Cys Thr
   1 5
<210> 641
   <211> 8
   <212> PRT
   <213> Artificial Sequence
<220>
   <223> Description of Artificial Sequence: Synthetic Peptide
<400> 641
   Asp Arg Val Tyr Ile His Pro Phe
   1 5
<210> 642
   <211> 8
   <212> PRT
   <213> Artificial Sequence
<220>
   <223> Description of Artificial Sequence: Synthetic Peptide
<400> 642
   Glu Gly Val Tyr Val His Pro Val
   1 5
<210> 643
   <211> 4
   <212> PRT
   <213> Artificial Sequence
<220>
   <223> Description of Artificial Sequence: Synthetic Peptide
<400> 643
   Asp Arg Val Tyr
   1
<210> 644
   <211> 6
   <212> PRT
   <213> Artificial Sequence
<220>
   <223> Description of Artificial Sequence: Synthetic Peptide
<400> 644
   Val Tyr Ile His Pro Phe
   1 5
<210> 645
   <211> 5
   <212> PRT
   <213> Artificial Sequence
<220>
   <223> Description of Artificial Sequence: Synthetic Peptide
<400> 645
   Tyr Ile His Pro Phe
   1 5
<210> 646
   <211> 4
   <212> PRT
   <213> Artificial Sequence
<220>
   <223> Description of Artificial Sequence: Synthetic Peptide
<400> 646
   Ile His Pro Phe
   1
<210> 647
   <211> 7
   <212> PRT
   <213> Artificial Sequence
<220>
   <223> Description of Artificial Sequence: Synthetic Peptide
<400> 647
   Arg Val Tyr Ile His Pro Phe
   1 5
<210> 648
   <211> 7
   <212> PRT
   <213> Artificial Sequence
<220>
   <223> Description of Artificial Sequence: Synthetic Peptide
<400> 648
   Arg Val Tyr Ile His Pro Ile
   1 5
<210> 649
<211> 8
   <212> PRT
   <213> Artificial Sequence
<220>
   <223> Description of Artificial Sequence: Synthetic Peptide
<400> 649
   Pro Thr His Ile Lys Trp Gly Asp
   1 5
<210> 650
   <211> 9
   <212> PRT
   <213> Artificial Sequence
<220>
   <223> Description of Artificial Sequence: Synthetic Peptide
<400> 650
   Glu Trp Pro Arg Pro Gln Ile Pro Pro
   1 5
<210> 651
   <211> 10
   <212> PRT
   <213> Artificial Sequence
<220>
   <223> Description of Artificial Sequence: Synthetic Peptide
<400> 651
   Asn Arg Val Tyr Val His Pro Phe His Leu
   1 5 10
<210> 652
   <211> 10
   <212> PRT
   <213> Artificial Sequence
<220>
   <223> Description of Artificial Sequence: Synthetic Peptide
<400> 652
   Asn Arg Val Tyr Val His Pro Phe Asn Leu
   1 5 10
<210> 653
   <211> 10
   <212> PRT
   <213> Artificial Sequence
<220>
   <223> Description of Artificial Sequence: Synthetic Peptide
<400> 653
   Asn Arg Val Tyr Val His Pro Phe Gly Leu
   1 5 10
<210> 654
   <211> 7
   <212> PRT
   <213> Artificial Sequence
<220>
   <223> Description of Artificial Sequence: Synthetic Peptide
<400> 654
   Asn Arg Val Tyr Val His Pro
   1 5
<210> 655
   <211> 8
   <212> PRT
   <213> Artificial Sequence
<220>
   <223> Description of Artificial Sequence: Synthetic Peptide
<400> 655
   Asn Arg Val Tyr Val His Pro Phe
   1 5
<210> 656
   <211> 9
   <212> PRT
   <213> Artificial Sequence
<220>
   <223> Description of Artificial Sequence: Synthetic Peptide
<400> 656
   Arg Val Tyr Ile His Pro Phe His Leu
   1 5
<210> 657
   <211> 6
   <212> PRT
   <213> Artificial Sequence
<220>
   <223> Description of Artificial Sequence: Synthetic Peptide
<400> 657
   Tyr Lys Arg His Pro Ile
   1 5
<210> 658
   <211> 8
   <212> PRT
   <213> Artificial Sequence
<220>
   <223> Description of Artificial Sequence: Synthetic Peptide
<400> 658
   Asp Arg Val Tyr Ile Phe Pro Phe
   1 5
<210> 659
   <211> 13
   <212> PRT
   <213> Artificial Sequence
<220>
   <223> Description of Artificial Sequence: Synthetic Peptide
<400> 659
   Asp Arg Val Tyr Ile His Pro Phe His Leu Val Ile His
   1 5 10
<210> 660
   <211> 14
   <212> PRT
   <213> Artificial Sequence
<220>
   <223> Description of Artificial Sequence: Synthetic Peptide
<400> 660
   Asp Arg Val Tyr Ile His Pro Phe His Leu Val Ile His Asn
   1 5 10
<210> 661
   <211> 14
   <212> PRT
   <213> Artificial Sequence
<220>
   <223> Description of Artificial Sequence: Synthetic Peptide
<400> 661
   Asp Arg Val Tyr Ile His Pro Phe His Leu Leu Val Tyr Ser
   1 5 10
<210> 662
   <211> 7
   <212> PRT
   <213> Artificial Sequence
<220>
   <223> Description of Artificial Sequence: Synthetic Peptide
<400> 662
   Arg Val Tyr Ile His Pro Phe
   1 5
<210> 663
   <211> 6
   <212> PRT
   <213> Artificial Sequence
<220>
   <223> Description of Artificial Sequence: Synthetic Peptide
<400> 663
   Arg Val Tyr Ile His Pro
   1 5
<210> 664
   <211> 7
   <212> PRT
   <213> Artificial Sequence
<220>
   <223> Description of Artificial Sequence: Synthetic Peptide
<400> 664
   Arg Val Tyr Ile His Pro Ala
   1 5
<210> 665
   <211> 7
   <212> PRT
   <213> Artificial Sequence
<220>
   <223> Description of Artificial Sequence: Synthetic Peptide
<400> 665
   Arg Val Tyr Ile His Pro Ile
   1 5
<210> 666
   <211> 7
   <212> PRT
   <213> Artificial Sequence
<220>
   <223> Description of Artificial Sequence: Synthetic Peptide
<400> 666
   Arg Val Tyr Ile His Pro Thr
   1 5
<210> 667
   <211> 7
   <212> PRT
   <213> Artificial Sequence
<220>
   <223> Description of Artificial Sequence: Synthetic Peptide
<400> 667
   Arg Val Tyr Ile His Pro Phe
   1 5
<210> 668
   <211> 7
   <212> PRT
   <213> Artificial Sequence
<220>
   <223> Description of Artificial Sequence: Synthetic Peptide
<400> 668
   Arg Val Tyr Val His Pro Ala
   1 5
<210> 669
   <211> 6
   <212> PRT
   <213> Artificial Sequence
<220>
   <223> Description of Artificial Sequence: Synthetic Peptide
<400> 669
   Val Tyr Ile His Pro Ile
   1 5
<210> 670
   <211> 14
   <212> PRT
   <213> Artificial Sequence
<220>
   <223> Description of Artificial Sequence: Synthetic Peptide
<400> 670
   Asp Arg Val Tyr Ile His Pro Phe His Leu Val Ile His Ser
   1 5 10
<210> 671
   <211> 7
   <212> PRT
   <213> Artificial Sequence
<220>
   <223> Description of Artificial Sequence: Synthetic Peptide
<400> 671
   Arg Val Tyr Val His Pro Phe
   1 5
<210> 672
   <211> 8
   <212> PRT
   <213> Artificial Sequence
<220>
   <223> Description of Artificial Sequence: Synthetic Peptide
<400> 672
   Asp Arg Val Tyr Val His Pro Phe
   1 5
<210> 673
   <211> 8
   <212> PRT
   <213> Artificial Sequence
<220>
   <223> Description of Artificial Sequence: Synthetic Peptide
<400> 673
   Asp Arg Val Tyr Val His Pro Phe
   1 5
<210> 674
   <211> 10
   <212> PRT
   <213> Artificial Sequence
<220>
   <223> Description of Artificial Sequence: Synthetic Peptide
<400> 674
   Asp Arg Val Tyr Val His Pro Phe His Leu
   1 5 10
<210> 675
   <211> 10
   <212> PRT
   <213> Artificial Sequence
<220>
   <223> Description of Artificial Sequence: Synthetic Peptide
<400> 675
   Asp Arg Val Tyr Val His Pro Phe Asn Leu
   1 5 10
<210> 676
   <211> 10
   <212> PRT
   <213> Artificial Sequence
<220>
   <223> Description of Artificial Sequence: Synthetic Peptide
<400> 676
   Asp Arg Val Tyr Val His Pro Phe Ser Leu
   1 5 10
<210> 677
   <211> 10
   <212> PRT
   <213> Artificial Sequence
<220>
   <223> Description of Artificial Sequence: Synthetic Peptide
<400> 677
   Asp Arg Val Tyr Val His Pro Phe His Leu
   1 5 10
<210> 678
   <211> 14
   <212> PRT
   <213> Artificial Sequence
<220>
   <223> Description of Artificial Sequence: Synthetic Peptide
<400> 678
   Asp Arg Val Tyr Ile His Pro Phe His Leu Val Ile His Asn
   1 5 10
<210> 679
   <211> 14
   <212> PRT
   <213> Artificial Sequence
<220>
   <223> Description of Artificial Sequence: Synthetic Peptide
<400> 679
   Asp Arg Val Tyr Ile His Pro Phe His Leu Leu Val Tyr Ser
   1 5 10

<210> 680
   <211> 10
   <212> PRT
   <213> Artificial Sequence
<220>
   <223> Description of Artificial Sequence: Synthetic Peptide
<400> 680
   Pro His Pro Phe His Phe Phe Val Tyr Lys
   1 5 10
<210> 681
   <211> 14
   <212> PRT
   <213> Artificial Sequence
<220>
   <223> Description of Artificial Sequence: Synthetic Peptide
<400> 681
   Asp Arg Val Tyr Ile His Pro Phe His Leu Leu Tyr Tyr Ser
   1 5 10
<210> 682
   <211> 14
   <212> PRT
   <213> Artificial Sequence
<220>
   <223> Description of Artificial Sequence: Synthetic Peptide
<400> 682
   Asp Arg Val Tyr Ile His Pro Cys His Leu Leu Tyr Tyr Ser
   1 5 10
<210> 683
   <211> 14
   <212> PRT
   <213> Artificial Sequence
<220>
   <223> Description of Artificial Sequence: Synthetic Peptide
<400> 683
   Asp Arg Val Tyr Ile His Pro Leu His Leu Leu Tyr Tyr Ser
   1 5 10
<210> 684
   <211> 14
   <212> PRT
   <213> Artificial Sequence
<220>
   <223> Description of Artificial Sequence: Synthetic Peptide
<400> 684
   Asp Arg Val Tyr Ile His Pro Val His Leu Leu Tyr Tyr Ser
   1 5 10
<210> 685
   <211> 38
   <212> PRT
   <213> Artificial Sequence
<220>
   <223> Description of Artificial Sequence: Synthetic Peptide
<400> 685
   Cys Ser Cys Ser Ser Leu Met Asp Lys Glu Cys Val Tyr Phe Cys His
   1 5 10 15
   Leu Asp Ile Ile Trp Val Asn Thr Pro Glu His Val Val Pro Tyr Gly
   20 25 30
   Leu Gly Ser Pro Arg Ser
   35
<210> 686
   <211> 20
   <212> PRT
   <213> Artificial Sequence
<220>
   <223> Description of Artificial Sequence: Synthetic Peptide
<400> 686
   Ile Asn Thr Pro Glu Gln Thr Val Pro Tyr Gly Leu Ser Asn Tyr Arg
   1 5 10 15
   Gly Ser Phe Arg
   20
<210> 687
   <211> 1o
   <212> PRT
   <213> Artificial Sequence
<220>
   <223> Description of Artificial Sequence: Synthetic Peptide
<400> 687
<210> 688
   <211> 39
   <212> PRT
   <213> Artificial Sequence
<220>
   <223> Description of Artificial Sequence: Synthetic Peptide
<400> 688
   Cys Ser Cys Ser Ser Leu Met Asp Lys Glu Cys Val Tyr Phe Cys His
   1 5 10 15
   Leu Asp Ile Ile Trp Val Asn Thr Pro Glu His Val Val Pro Tyr Gly
   20 25 30
   Leu Gly Ser Pro Ser Arg Ser
   35
<210> 689
   <211> 18
   <212> PRT
   <213> Artificial Sequence
<220>
   <223> Description of Artificial Sequence: Synthetic Peptide
<400> 689
   Val Asn Thr Pro Glu His Val Val Pro Tyr Gly Leu Gly Ser Pro Ser
   1 5 10 15
   Arg Ser
<210> 690
   <211> 20
   <212> PRT
   <213> Artificial Sequence
<220>
   <223> Description of Artificial Sequence: Synthetic Peptide
<400> 690
   Ile Ile Trp Val Asn Thr Pro Glu His Val Val Pro Tyr Gly Leu Gly
   1 5 10 15
   Ser Pro Arg Ser
   20
<210> 691
   <211> 17
   <212> PRT
   <213> Artificial Sequence
<220>
   <223> Description of Artificial Sequence: Synthetic Peptide
<400> 691
   Val Asn Thr Pro Glu His Val Val Pro Tyr Gly Leu Gly Ser Pro Arg
   1 5 10 15
   Ser
<210> 692
   <211> 37
   <212> PRT
   <213> Artificial Sequence
<220>
   <223> Description of Artificial Sequence: Synthetic Peptide
<400> 692
   Cys Ser Cys Ser Ser Trp Leu Asp Lys Glu Cys Val Tyr Phe Cys His
   1 5 10 15
   Leu Asp Ile Ile Trp Val Asn Thr Pro Glu Gln Thr Ala Pro Tyr Gly
   20 25 30
   Leu Gly Asn Pro Pro
   35
<210> 693
   <211> 16
   <212> PRT
   <213> Artificial Sequence
<220>
   <223> Description of Artificial Sequence: Synthetic Peptide
<400> 693
   Val Asn Thr Pro Glu Gln Thr Ala Pro Tyr Gly Leu Gly Asn Pro Pro
   1 5 10 15
<210> 694
   <211> 41
   <212> PRT
   <213> Artificial Sequence
<220>
   <223> Description of Artificial Sequence: Synthetic Peptide
<400> 694
   Cys Thr Cys Phe Thr Tyr Lys Asp Lys Glu Cys Val Tyr Tyr Cys His
   1 5 10 15
   Leu Asp Ile Ile Trp Ile Asn Thr Pro Glu Gln Thr Val Pro Tyr Gly
   20 25 30
   Leu Ser Asn Tyr Arg Gly Ser Phe Arg
   35 40
<210> 695
   <211> 39
   <212> PRT
   <213> Artificial Sequence
<220>
   <223> Description of Artificial Sequence: Synthetic Peptide
<400> 695
   Cys Ser Cys Ser Ser Leu Met Asp Lys Glu Cys Val Tyr Phe Cys His
   1 5 10 15
   Leu Asp Ile Ile Trp Val Asn Thr Pro Glu His Ile Val Pro Tyr Gly
   20 25 30
   Leu Gly Ser Pro Ser Arg Ser
   35
<210> 696
   <211> 18
   <212> PRT
   <213> Artificial Sequence
<220>
   <223> Description of Artificial Sequence: Synthetic Peptide
<400> 696
   Val Asn Thr Pro Glu His Val Val Pro Tyr Gly Leu Gly Ser Pro Ser
   1 5 10 15
   Arg Ser
<210> 697
   <211> 39
   <212> PRT
   <213> Artificial Sequence
<220>
   <223> Description of Artificial Sequence: Synthetic Peptide
<400> 697
   Cys Ser Cys Ser Ser Leu Met Asp Lys Glu Cys Val Tyr Phe Cys His
   1 5 10 15
   Leu Asp Ile Ile Trp Val Asn Thr Pro Glu Arg Val Val Pro Tyr Gly
   20 25 30
   Leu Gly Ser Pro Ser Arg Ser
   35
<210> 698
   <211> 38
   <212> PRT
   <213> Artificial Sequence
<220>
   <223> Description of Artificial Sequence: Synthetic Peptide
<400> 698
   Cys Ser Cys Ser Ser Trp Leu Asp Lys Glu Cys Val Tyr Phe Cys His
   1 5 10 15
   Leu Asp Ile Ile Trp Val Asn Thr Pro Glu Gln Thr Ala Pro Tyr Gly
   20 25 30
   Leu Gly Asn Pro Pro Arg
   35
<210> 699
   <211> 17
   <212> PRT
   <213> Artificial Sequence
<220>
   <223> Description of Artificial Sequence: Synthetic Peptide
<400> 699
   Val Asn Thr Pro Glu Gln Thr Ala Pro Tyr Gly Leu Gly Asn Pro Pro
   1 5 10 15
   Arg
<210> 700
   <211> 41
   <212> PRT
   <213> Artificial Sequence
<220>
   <223> Description of Artificial Sequence: Synthetic Peptide
<400> 700
   Cys Thr Cys Phe Thr Tyr Lys Asp Lys Glu Cys Val Tyr Tyr Cys His
   1 5 10 15
   Leu Asp Ile Ile Trp Ile Asn Thr Pro Glu Gln Thr Val Pro Tyr Gly
   20 25 30
   Leu Ser Asn His Arg Gly Ser Leu Arg
   35 40
<210> 701
   <211> 20
   <212> PRT
   <213> Artificial Sequence
<220>
   <223> Description of Artificial Sequence: Synthetic Peptide
<400> 701
   Cys Gln Cys Ala Ser Gln Lys Asp Lys Lys Trp Ser Tyr Cys Gln Ala
   1 5 10 15
   Gly Lys Glu Ile
   20
<210> 702
   <211> 21
   <212> PRT
   <213> Artificial Sequence
<220>
   <223> Description of Artificial Sequence: Synthetic Peptide
<400> 702
   Cys Ser Cys Ser Ser Leu Met Asp Lys Glu Cys Val Tyr Phe Cys His
   1 5 10 15
   Leu Asp Ile Ile Trp
   20
<210> 703
   <211> 15
   <212> PRT
   <213> Artificial Sequence
<220>
   <223> Description of Artificial Sequence: Synthetic Peptide
<400> 703
   Cys Ser Cys Ser Ser Leu Met Asp Lys Glu Cys Val Tyr Phe Cys
   1 5 10 15
<210> 704
   <211> 15
   <212> PRT
   <213> Artificial Sequence
<220>
   <223> Description of Artificial Sequence: Synthetic Peptide
<400> 704
   Cys Ser Cys Ser Ser Leu Met Asp Lys Glu Cys Val Tyr Phe Cys
   1 5 10 15
<210> 705
   <211> 6
   <212> PRT
   <213> Artificial Sequence
<220>
   <223> Description of Artificial Sequence: Synthetic Peptide
<400> 705
   Trp Leu Asp Ile Ile Trp
   1 5
<210> 706
   <211> 21
   <212> PRT
   <213> Artificial Sequence
<220>
   <223> Description of Artificial Sequence: Synthetic Peptide
<400> 706
   Cys Ser Ala Ser Ser Leu Met Asp Lys Glu Ala Val Tyr Phe Cys His
   1 5 10 15
   Leu Asp Ile Ile Trp
   20
<210> 707
   <211> 20
   <212> PRT
   <213> Artificial Sequence
<220>
   <223> Description of Artificial Sequence: Synthetic Peptide
<400> 707
   Ser Cys Ser Ser Leu Met Asp Lys Glu Cys Val Tyr Phe Asp His Leu
   1 5 10 15
   Asp Ile Ile Trp
   20
<210> 708
   <211> 21
   <212> PRT
   <213> Artificial Sequence
<220>
   <223> Description of Artificial Sequence: Synthetic Peptide
<400> 708
   Ala Ser Ala Ser Ser Leu Met Asp Lys Glu Ala Val Tyr Phe Ala His
   1 5 10 15
   Leu Asp Ile Ile Trp
   20
<210> 709
   <211> 16
   <212> PRT
   <213> Artificial Sequence
<220>
   <223> Description of Artificial Sequence: Synthetic Peptide
<400> 709
   Leu Met Asp Lys Glu Ala Val Tyr Phe Ala His Leu Asp Ile Ile Trp
   1 5 10 15
<210> 710
   <211> 21
   <212> PRT
   <213> Artificial Sequence
<220>
   <223> Description of Artificial Sequence: Synthetic Peptide
<400> 710
   Cys Ala Cys Phe Thr Tyr Lys Asp Lys Glu Cys Val Tyr Tyr Cys His
   1 5 10 15
   Leu Asp Ile Ile Trp
   20
<210> 711
   <211> 20
   <212> PRT
   <213> Artificial Sequence
<220>
   <223> Description of Artificial Sequence: Synthetic Peptide
<400> 711
   Cys Ser Ala Ser Ser Leu Asp Lys Glu Ala Val Tyr Phe Cys His Leu
   1 5 10 15
   Ala Ile Ile Trp
   20
<210> 712
   <211> 21
   <212> PRT
   <213> Artificial Sequence
<220>
   <223> Description of Artificial sequence: Synthetic Peptide
<400> 712
   Cys Ser Cys Ser Ser Leu Met Asp Lys Glu Cys Val Tyr Phe Cys His
   1 5 10 15
   Leu Asn Ile Ile Trp
   20
<210> 713
   <211> 15
   <212> PRT
   <213> Artificial Sequence
<220>
   <223> Description of Artificial Sequence: Synthetic Peptide
<400> 713
   Asn Trp His Gly Thr Ala Pro Asp Trp Phe Phe Asn Tyr Tyr Trp
   1 5 10 15
<210> 714
   <211> 14
   <212> PRT
   <213> Artificial Sequence
<220>
   <223> Description of Artificial Sequence: Synthetic Peptide
<400> 714
   Asp Glu Glu Ala Val Tyr Phe Ala His Leu Asp Ile Ile Trp
   1 5 10
<210> 715
   <211> 6
   <212> PRT
   <213> Artificial Sequence
<220>
   <223> Description of Artificial Sequence: Synthetic Peptide
<400> 715
   His Leu Asp Ile Ile Trp
   1 5
<210> 716
   <211> 23
   <212> PRT
   <213> Artificial Sequence
<220>
   <223> Description of Artificial Sequence: Synthetic Peptide
<400> 716
   His Leu Asp Ile Ile Trp Val Asn Thr Pro Glu His Val Val Pro Tyr
   1 5 10 15
   Gly Leu Gly Ser Pro Arg Ser
   20
<210> 717
   <211> 21
   <212> PRT
   <213> Artificial Sequence
<220>
   <223> Description of Artificial Sequence: Synthetic Peptide
<400> 717
   Cys Ser Cys Ser Ser Trp Leu Asp Lys Glu Cys Val Tyr Phe Cys His
   1 5 10 15
   Leu Asp Ile Ile Trp
   20
<210> 718
   <211> 21
   <212> PRT
   <213> Artificial Sequence
<220>
   <223> Description of Artificial Sequence: Synthetic Peptide
<400> 718
   Cys Thr Cys Phe Thr Tyr Lys Asp Lys Glu Cys Val Tyr Tyr Cys His
   1 5 10 15
   Leu Asp Ile Ile Trp
   20
<210> 719
   <211> 16
   <212> PRT
   <213> Artificial Sequence
<220>
   <223> Description of Artificial Sequence: Synthetic Peptide
<400> 719
   Ser Leu Lys Asp Leu Phe Pro Ala Lys Ala Ala Asp Arg Arg Asp Arg
   1 5 10 15
<210> 720
   <211> 3
   <212> PRT
   <213> Artificial Sequence
<220>
   <223> Description of Artificial Sequence: Synthetic Peptide
<400> 720
   Leu Trp Trp
   1
<210> 721
   <211> 4
   <212> PRT
   <213> Artificial Sequence
<220>
   <223> Description of Artificial Sequence: Synthetic Peptide
<400> 721
   Met Leu Met Trp
   1
<210> 722
   <211> 1
   <212> PRT
   <213> Artificial Sequence
<220>
   <223> Description of Artificial Sequence: Synthetic Peptide
<400> 722
   Arg
   1
<210> 723
   <211> 5
   <212> PRT
   <213> Artificial Sequence
<220>
   <223> Description of Artificial Sequence: Synthetic Peptide
<400> 723
   Asp Pro Ile Leu Trp
   1 5
<210> 724
   <211> 5
   <212> PRT
   <213> Artificial Sequence
<220>
   <223> Description of Artificial Sequence: Synthetic Peptide
<400> 724
   Ser Pro Val Leu Trp
   1 5
<210> 725
   <211> 5
   <212> PRT
   <213> Artificial Sequence
<220>
   <223> Description of Artificial Sequence: Synthetic Peptide
<400> 725
   Leu Asp Ile Ile Trp
   1 5
<210> 726
   <211> 5
   <212> PRT
   <213> Artificial Sequence
<220>
   <223> Description of Artificial Sequence: Synthetic Peptide
<400> 726
   Leu Asp Ile Ile Trp
   1 5
<210> 727
   <211> 21
   <212> PRT
   <213> Artificial Sequence
<220>
   <223> Description of Artificial Sequence: Synthetic Peptide
<400> 727
   Cys Ser Cys Lys Asp Met Thr Asp Lys Glu Cys Leu Asn Phe Cys His
   1 5 10 15
   Gln Asp Val Ile Trp
   20
<210> 728
   <211> 21
   <212> PRT
   <213> Artificial Sequence
<220>
   <223> Description of Artificial Sequence: Synthetic Peptide
<400> 728
   Cys Ser Cys Lys Asp Met Thr Asp Lys Glu Cys Leu Tyr Phe Cys His
   1 5 10 15
   Gln Asp Val Ile Trp
   20
<210> 729
   <211> 21
   <212> PRT
   <213> Artificial Sequence
<220>
   <223> Description of Artificial Sequence: Synthetic Peptide
<400> 729
   Cys Thr Cys Asn Asp Met Thr Asp Glu Glu Cys Leu Asn Phe Cys His
   1 5 10 15
   Gln Asp Val Ile Trp
   20
<210> 730
   <211> 16
   <212> PRT
   <213> Artificial Sequence
<220>
   <223> Description of Artificial Sequence: Synthetic Peptide
<400> 730
   Gly Asn Trp His Gly Thr Ala Pro Asp Trp Phe Phe Asn Tyr Tyr Trp
   1 5 10 15
<210> 731
   <211> 6
   <212> PRT
   <213> Artificial Sequence
<220>
   <223> Description of Artificial Sequence: Synthetic Peptide
<400> 731
   His Leu Asp Ile Ile Trp
   1 5
<210> 732
   <211> 45
   <212> PRT
   <213> Artificial Sequence
<220>
   <223> Description of Artificial Sequence: Synthetic Peptide
<400> 732
   Thr Leu Asp Glu Glu Asp Leu Val Asp Ser Leu Ser Glu Gly Asp Ala
   1 5 10 15
   Tyr Thr Asn Gly Leu Gln Val Asn Phe His Ser Pro Arg Ser Gly Gln
   20 25 30
   Arg Cys Trp Ala Ala Arg Thr Gln Val Glu Lys Arg Leu
   35 40 45
<210> 733
   <211> 16
   <212> PRT
   <213> Artificial Sequence
<220>
   <223> Description of Artificial Sequence: Synthetic Peptide
<400> 733
   Cys Pro Pro Gly Ser Pro Met Asn Pro His His Lys Cys Glu Val Trp
   1 5 10 15
<210> 734
   <211> 5
   <212> PRT
   <213> Artificial Sequence
<220>
   <223> Description of Artificial Sequence: Synthetic Peptide
<400> 734
   Asp Pro Val Leu Trp
   1 5
<210> 735
   <211> 5
   <212> PRT
   <213> Artificial Sequence
<220>
   <223> Description of Artificial Sequence: Synthetic Peptide
<400> 735
   Glu Ala Ile Trp Leu
   1 5
<210> 736
   <211> 4
   <212> PRT
   <213> Artificial Sequence
<220>
   <223> Description of Artificial Sequence: Synthetic Peptide
<400> 736
   Ala Val Leu Trp
   1
<210> 737
   <211> 3
   <212> PRT
   <213> Artificial Sequence
<220>
   <223> Description of Artificial Sequence: Synthetic Peptide
<400> 737
   Leu Trp Trp
   1
<210> 738
   <211> 2
   <212> PRT
   <213> Artificial Sequence
<220>
   <223> Description of Artificial Sequence: Synthetic Peptide
<400> 738
   Leu Trp
   1
<210> 739
   <211> 3
   <212> PRT
   <213> Artificial Sequence
<220>
   <223> Description of Artificial Sequence: Synthetic Peptide
<400> 739
   Leu Trp Tyr
   1
<210> 740
   <211> 6
   <212> PRT
   <213> Artificial Sequence
<220>
   <223> Description of Artificial Sequence: Synthetic Peptide
<400> 740
   Leu Trp Ala Ala Tyr Phe
   1 5
<210> 741
   <211> 16
   <212> PRT
   <213> Artificial Sequence
<220>
   <223> Description of Artificial Sequence: Synthetic Peptide
<400> 741
   Gly Asn Trp His Gly Thr Ser Pro Asp Trp Phe Phe Asn Tyr Tyr Trp
   1 5 10 15
<210> 742
   <211> 14
   <212> PRT
   <213> Artificial Sequence
<220>
   <223> Description of Artificial Sequence: Synthetic Peptide
<400> 742
   Asp Lys Glu Ala Val Tyr Phe Ala His Leu Asp Ile Ile Trp
   1 5 10
<210> 743
   <211> 21
   <212> PRT
   <213> Artificial Sequence
<220>
   <223> Description of Artificial Sequence: Synthetic Peptide
<400> 743
   Cys Thr Cys Lys Asp Met Thr Asp Lys Glu Cys Leu Tyr Phe Cys His
   1 5 10 15
   Gln Asp Ile Ile Trp
   20
<210> 744
   <211> 21
   <212> PRT
   <213> Artificial Sequence
<220>
   <223> Description of Artificial Sequence: Synthetic Peptide
<400> 744
   Cys Thr Cys Lys Asp Met Thr Asp Glu Glu Cys Leu Asn Phe Cys His
   1 5 10 15
   Gln Asp Val Ile Trp
   20
<210> 745
   <211> 7
   <212> PRT
   <213> Artificial Sequence
<220>
   <223> Description of Artificial Sequence: Synthetic Peptide
<400> 745
   Tyr Pro Phe Val Glu Pro Ile
   1 5
<210> 746
   <211> 3
   <212> PRT
   <213> Artificial Sequence
<220>
   <223> Description of Artificial Sequence: Synthetic Peptide
<400> 746
   Tyr Pro Phe
   1
<210> 747
   <211> 3
   <212> PRT
   <213> Artificial Sequence
<220>
   <223> Description of Artificial Sequence: Synthetic Peptide
<400> 747
   Tyr Pro Phe
   1
<210> 748
   <211> 7
   <212> PRT
   <213> Artificial Sequence
<220>
   <223> Description of Artificial Sequence: Synthetic Peptide
<400> 748
   Tyr Pro Phe Pro Gly Pro Ile
   1 5
<210> 749
   <211> 4
   <212> PRT
   <213> Artificial Sequence
<220>
   <223> Description of Artificial Sequence: Synthetic Peptide
<400> 749
   Tyr Pro Phe Pro
   1
<210> 750
   <211> 5
   <212> PRT
   <213> Artificial Sequence
<220>
   <223> Description of Artificial Sequence: Synthetic Peptide
<400> 750
   Tyr Pro Phe Pro Gly
   1 5
<210> 751
   <211> 5
   <212> PRT
   <213> Artificial Sequence
<220>
   <223> Description of Artificial Sequence: Synthetic Peptide
<400> 751
   Tyr Pro Phe Pro Gly
   1 5
<210> 752
   <211> 5
   <212> PRT
   <213> Artificial Sequence
<220>
   <223> Description of Artificial Sequence: Synthetic Peptide
<400> 752
   Tyr Pro Phe Pro Gly
   1 5
<210> 753
   <211> 7
   <212> PRT
   <213> Artificial Sequence
<220>
   <223> Description of Artificial Sequence: Synthetic Peptide
<400> 753
   Tyr Pro Phe Val Glu Pro Ile
   1 5
<210> 754
   <211> 2
   <212> PRT
   <213> Artificial Sequence
<220>
   <223> Description of Artificial Sequence: Synthetic Peptide
<400> 754
   Tyr Phe
   1
<210> 755
   <211> 3
   <212> PRT
   <213> Artificial Sequence
<220>
   <223> Description of Artificial Sequence: Synthetic Peptide
<400> 755
   Tyr Phe Tyr
   1
<210> 756
   <211> 3
   <212> PRT
   <213> Artificial Sequence
<220>
   <223> Description of Artificial Sequence: Synthetic Peptide
<400> 756
   Tyr Phe Tyr
   1

<210> 757
   <211> 4
   <212> PRT
   <213> Artificial Sequence
<220>
   <223> Description of Artificial Sequence: Synthetic Peptide
<400> 757
   Tyr Phe Pro Tyr
   1
<210> 758
   <211> 3
   <212> PRT
   <213> Artificial Sequence
<220>
   <223> Description of Artificial Sequence: Synthetic Peptide
<400> 758
   Tyr Phe Pro
   1
<210> 759
   <211> 3
   <212> PRT
   <213> Artificial Sequence
<220>
   <223> Description of Artificial Sequence: Synthetic Peptide
<400> 759
   Tyr Phe Pro
   1
<210> 760
   <211> 3
   <212> PRT
   <213> Artificial Sequence.
<220>
   <223> Description of Artificial Sequence: Synthetic Peptide
<400> 760
   Tyr Phe Pro
   1
<210> 761
   <211> 4
   <212> PRT
   <213> Artificial Sequence
<220>
   <223> Description of Artificial Sequence: Synthetic Peptide
<400> 761
   Tyr Phe Pro Gly
   1
<210> 762
   <211> 4
   <212> PRT
   <213> Artificial Sequence
<220>
   <223> Description of Artificial Sequence: Synthetic Peptide
<400> 762
   Tyr Phe Pro Gly
   1
<210> 763
   <211> 4
   <212> PRT
   <213> Artificial Sequence
<220>
   <223> Description of Artificial Sequence: Synthetic Peptide
<400> 763
   Tyr Phe Pro Gly
   1
<210> 764
   <211> 5
   <212> PRT
   <213> Artificial Sequence
<220>
   <223> Description of Artificial Sequence: Synthetic Peptide
<400> 764
   Tyr Phe Pro Gly Pro
   1 5
<210> 765
   <211> 3
   <212> PRT
   <213> Artificial Sequence
<220>
   <223> Description of Artificial Sequence: Synthetic Peptide
<400> 765
   Tyr Phe Pro
   1
<210> 766
   <211> 6
   <212> PRT
   <213> Artificial Sequence
<220>
   <223> Description of Artificial Sequence: Synthetic Peptide
<400> 766
   Pro Phe Pro Gly Pro Ile
   1 5
<210> 767
   <211> 6
   <212> PRT
   <213> Artificial Sequence
<220>
   <223> Description of Artificial Sequence: Synthetic Peptide
<400> 767
   Pro Phe Pro Gly Pro Ile
   1 5
<210> 768
   <211> 4
   <212> PRT
   <213> Artificial Sequence
<220>
   <223> Description of Artificial Sequence: Synthetic Peptide
<400> 768
   Tyr Pro Val Pro
   1
<210> 769
   <211> 4
   <212> PRT
   <213> Artificial Sequence
<220>
   <223> Description of Artificial Sequence: Synthetic Peptide
<400> 769
   Tyr Pro Phe Pro
   1
<210> 770
   <211> 4
   <212> PRT
   <213> Artificial Sequence
<220>
   <223> Description of Artificial Sequence: Synthetic Peptide
<400> 770
   Tyr Pro Phe Pro
   1
<210> 771
   <211> 3
   <212> PRT
   <213> Artificial Sequence
<220>
   <223> Description of Artificial Sequence: Synthetic Peptide
<400> 771
   Tyr Pro Pro
   1
<210> 772
   <211> 7
   <212> PRT
   <213> Artificial Sequence
<220>
   <223> Description of Artificial Sequence: Synthetic Peptide
<400> 772
   Tyr Ala Phe Gly Tyr Pro Ser
   1 5
<210> 773
   <211> 3
   <212> PRT
   <213> Artificial Sequence
<220>
   <223> Description of Artificial Sequence: Synthetic Peptide
<400> 773
   Tyr Arg Phe
   1
<210> 774
   <211> 32
   <212> PRT
   <213> Artificial Sequence
<220>
   <223> Description of Artificial Sequence: Synthetic Peptide
<400> 774
   Tyr Gly Gly Phe Leu Arg Arg Ile Arg Pro Lys Leu Lys Trp Asp Asn
   1 5 10 15
   Gln Lys Arg Tyr Gly Gly Phe Leu Arg Arg Gln Phe Lys Val Val Thr
   20 25 30
<210> 775
   <211> 17
   <212> PRT
   <213> Artificial Sequence
<220>
   <223> Description of Artificial Sequence: Synthetic Peptide
<400> 775
   Tyr Gly Gly Phe Leu Arg Arg Ile Arg Pro Lys Leu Lys Trp Asp Asn
   1 5 10 15
   Gln
<210> 776
   <211> 11
   <212> PRT
   <213> Artificial Sequence
<220>
   <223> Description of Artificial Sequence: Synthetic Peptide
<400> 776
   Tyr Gly Phe Leu Arg Ile Arg Pro Lys Leu Lys
   1 5 10
<210> 777
   <211> 17
   <212> PRT
   <213> Artificial Sequence
<220>
   <223> Description of Artificial Sequence: Synthetic Peptide
<400> 777
   Tyr Ala Gly Phe Leu Arg Arg Ile Arg Pro Lys Leu Lys Trp Asp Asn
   1 5 10 15
   Gln
<210> 778
   <211> 17
   <212> PRT
   <213> Artificial Sequence
<220>
   <223> Description of Artificial Sequence: Synthetic Peptide
<400> 778
   Tyr Ala Gly Phe Leu Arg Arg Ile Arg Pro Lys Leu Lys Trp Asp Asn
   1 5 10 15
   Gln
<210> 779
   <211> 17
   <212> PRT
   <213> Artificial Sequence
<220>
   <223> Description of Artificial Sequence: Synthetic Peptide
<400> 779
   Tyr Ala Gly Phe Leu Arg Arg Ile Arg Pro Lys Leu Lys Trp Asp Asn
   1 5 10 15
   Gln
<210> 780
   <211> 12
   <212> PRT
   <213> Artificial Sequence
<220>
   <223> Description of Artificial Sequence: Synthetic Peptide
<400> 780
   Tyr Gly Phe Leu Arg Arg Ile Arg Pro Lys Leu Lys
   1 5 10
<210> 781
   <211> 8
   <212> PRT
   <213> Artificial Sequence
<220>
   <223> Description of Artificial Sequence: Synthetic Peptide
<400> 781
   Tyr Gly Phe Leu Arg Arg Ile Arg
   1 5
<210> 782
   <211> 12
   <212> PRT
   <213> Artificial Sequence
<220>
   <223> Description of Artificial Sequence: Synthetic Peptide
<400> 782
   Tyr Gly Gly Phe Leu Arg Ile Arg Pro Lys Leu Lys
   1 5 10
<210> 783
   <211> 12
   <212> PRT
   <213> Artificial Sequence
<220>
   <223> Description of Artificial Sequence: Synthetic Peptide
<400> 783
   Tyr Gly Gly Phe Leu Arg Arg Arg Pro Lys Leu Lys
   1 5 10
<210> 784
   <211> 7
   <212> PRT
   <213> Artificial Sequence
<220>
   <223> Description of Artificial Sequence: Synthetic Peptide
<400> 784
   Gly Gly Phe Leu Arg Arg Ile
   1 5
<210> 785
   <211> 12
   <212> PRT
   <213> Artificial Sequence
<220>
   <223> Description of Artificial Sequence: Synthetic Peptide
<400> 785
   Tyr Gly Gly Phe Leu Arg Arg Ile Arg Pro Lys Leu
   1 5 10
<210> 786
   <211> 13
   <212> PRT
   <213> Artificial Sequence
<220>
   <223> Description of Artificial Sequence: Synthetic Peptide
<400> 786
   Tyr Gly Gly Phe Leu Arg Arg Ile Arg Pro Lys Leu Lys
   1 5 10
<210> 787
   <211> 13
   <212> PRT
   <213> Artificial Sequence
<220>
   <223> Description of Artificial Sequence: Synthetic Peptide
<400> 787
   Tyr Gly Gly Phe Leu Arg Arg Ile Arg Pro Lys Leu Lys
   1 5 10
<210> 788
   <211> 10
   <212> PRT
   <213> Artificial Sequence
<220>
   <223> Description of Artificial Sequence: Synthetic Peptide
<400> 788
   Tyr Gly Gly Phe Leu Arg Arg Ile Arg Pro
   1 5 10
<210> 789
   <211> 14
   <212> PRT
   <213> Artificial Sequence
<220>
   <223> Description of Artificial Sequence: Synthetic Peptide
<400> 789
   Tyr Gly Gly Phe Leu Arg Arg Ile Arg Pro Arg Leu Arg Gly
   1 5 10
<210> 790
   <211> 16
   <212> PRT
   <213> Artificial Sequence
<220>
   <223> Description of Artificial Sequence: Synthetic Peptide
<400> 790
   Gly Gly Phe Leu Arg Arg Ile Arg Pro Lys Leu Lys Trp Asp Asn Gln
   1 5 10 15
<210> 791
   <211> 16
   <212> PRT
   <213> Artificial Sequence
<220>
   <223> Description of Artificial Sequence: Synthetic Peptide
<400> 791
   Gly Gly Phe Leu Arg Arg Ile Arg Pro Lys Leu Lys Trp Asp Asn Gln
   1 5 10 15
<210> 792
   <211> 11
   <212> PRT
   <213> Artificial Sequence
<220>
   <223> Description of Artificial Sequence: Synthetic Peptide
<400> 792
   Gly Gly Phe Leu Arg Arg Ile Arg Pro Lys Leu
   1 5 10
<210> 793
   <211> 15
   <212> PRT
   <213> Artificial Sequence
<220>
   <223> Description of Artificial Sequence: Synthetic Peptide
<400> 793
   Gly Phe Leu Arg Arg Ile Arg Pro Lys Leu Lys Trp Asp Asn Gln
   1 5 10 15
<210> 794
   <211> 6
   <212> PRT
   <213> Artificial Sequence
<220>
   <223> Description of Artificial Sequence: Synthetic Peptide
<400> 794
   Gly Phe Leu Arg Arg Ile
   1 5
<210> 795
   <211> 11
   <212> PRT
   <213> Artificial Sequence
<220>
   <223> Description of Artificial Sequence: Synthetic Peptide
<400> 795
   Gly Phe Leu Arg Arg Ile Arg Pro Lys Leu Lys
   1 5 10
<210> 796
   <211> 15
   <212> PRT
   <213> Artificial Sequence
<220>
   <223> Description of Artificial Sequence: Synthetic Peptide
<400> 796
   Gly Phe Leu Arg Arg Ile Arg Pro Lys Leu Lys Trp Asp Asn Gln
   1 5 10 15
<210> 797
   <211> 11
   <212> PRT
   <213> Artificial Sequence
<220>
   <223> Description of Artificial Sequence: Synthetic Peptide
<400> 797
   Arg Ile Arg Pro Lys Leu Lys Trp Asp Asn Gln
   1 5 10
<210> 798
   <211> 10
   <212> PRT
   <213> Artificial Sequence
<220>
   <223> Description of Artificial Sequence: Synthetic Peptide
<400> 798
   Ile Arg Pro Lys Leu Lys Trp Asp Asn Gln
   1 5 10
<210> 799
   <211> 12
   <212> PRT
   <213> Artificial Sequence
<220>
   <223> Description of Artificial Sequence: Synthetic Peptide
<400> 799
   Arg Arg Ile Arg Pro Lys Leu Lys Trp Asp Asn Gln
   1 5 10
<210> 800
   <211> 16
   <212> PRT
   <213> Artificial Sequence
<220>
   <223> Description of Artificial Sequence: Synthetic Peptide
<400> 800
   Tyr Pro Phe Pro Arg Arg Ile Arg Pro Lys Leu Lys Trp Asp Asn Gln
   1 5 10 15
<210> 801
   <211> 9
   <212> PRT
   <213> Artificial Sequence
<220>
   <223> Description of Artificial Sequence: Synthetic Peptide
<400> 801
   Arg Pro Lys Leu Lys Trp Asp Asn Gln
   1 5
<210> 802
   <211> 5
   <212> PRT
   <213> Artificial Sequence
<220>
   <223> Description of Artificial Sequence: Synthetic Peptide
<400> 802
   Lys Trp Asp Asn Gln
   1 5
<210> 803
   <211> 17
   <212> PRT
   <213> Artificial Sequence
<220>
   <223> Description of Artificial Sequence: Synthetic Peptide
<400> 803
   Tyr Gly Gly Phe Leu Arg Arg Ile Arg Pro Lys Leu Lys Trp Asp Asn
   1 5 10 15
   Gln
<210> 804
   <211> 13
   <212> PRT
   <213> Artificial Sequence
<220>
   <223> Description of Artificial Sequence: Synthetic Peptide
<400> 804
   Tyr Gly Gly Phe Leu Arg Arg Gln Phe Lys Val Val Thr
   1 5 10
<210> 805
   <211> 9
   <212> PRT
   <213> Artificial Sequence
<220>
   <223> Description of Artificial Sequence: Synthetic Peptide
<400> 805
   Tyr Gly Gly Phe Leu Arg Arg Gln Phe
   1 5
<210> 806
   <211> 29
   <212> PRT
   <213> Artificial Sequence
<220>
   <223> Description of Artificial Sequence: Synthetic Peptide
<400> 806
   Tyr Gly Gly Phe Leu Arg Arg Gln Phe Lys Val Val Thr Arg Ser Gln
   1 5 10 15
   Glu Asp Pro Asn Ala Tyr Ser Gly Glu Leu Phe Asp Ala
   20 25
<210> 807
   <211> 10
   <212> PRT
   <213> Artificial Sequence
<220>
   <223> Description of Artificial Sequence: Synthetic Peptide
<400> 807
   Tyr Gly Gly Phe Leu Arg Lys Tyr Pro Lys
   1 5 10
<210> 808
   <211> 9
   <212> PRT
   <213> Artificial Sequence
<220>
   <223> Description of Artificial Sequence: Synthetic Peptide
<400> 808
   Tyr Gly Gly Phe Leu Arg Lys Tyr Pro
   1 5
<210> 809
   <211> 13
   <212> PRT
   <213> Artificial Sequence
<220>
   <223> Description of Artificial Sequence: Synthetic Peptide
<400> 809
   Tyr Gly Gly Phe Leu Arg Arg Ile Arg Pro Lys Leu Lys
   1 5 10
<210> 810
   <211> 7
   <212> PRT
   <213> Artificial Sequence
<220>
   <223> Description of Artificial Sequence: Synthetic Peptide
<400> 810
   Tyr Gly Gly Phe Leu Arg Phe
   1 5
<210> 811
   <211> 7
   <212> PRT
   <213> Artificial Sequence
<220>
   <223> Description of Artificial Sequence: Synthetic Peptide
<400> 811
   Tyr Gly Gly Phe Leu Arg Phe
   1 5
<210> 812
   <211> 29
   <212> PRT
   <213> Artificial Sequence
<220>
   <223> Description of Artificial Sequence: Synthetic Peptide
<400> 812
   Tyr Gly Gly Phe Leu Arg Arg Gln Phe Lys Val Val Thr Arg Ser Gln
   1 5 10 15
   Glu Asp Pro Asn Ala Tyr Tyr Glu Glu Leu Phe Asp Val
   20 25
<210> 813
   <211> 31
   <212> PRT
   <213> Artificial Sequence
<220>
   <223> Description of Artificial Sequence: Synthetic Peptide
<400> 813
   Tyr Gly Gly Phe Met Thr Ser Glu Lys Ser Gln Thr Pro Leu Val Thr
   1 5 10 15
   Leu Phe Lys Asn Ala Ile Ile Lys Asn Ala His Lys Lys Gly Gln
   20 25 30
<210> 814
   <211> 27
   <212> PRT
   <213> Artificial Sequence
<220>
   <223> Description of Artificial Sequence: Synthetic Peptide
<400> 814
   Tyr Gly Gly Phe Met Thr Ser Glu Lys Ser Gln Thr Pro Leu Val Thr
   1 5 10 15
   Leu Phe Lys Asn Ala Ile Ile Lys Asn Ala His
   20 25
<210> 815
   <211> 31
   <212> PRT
   <213> Artificial Sequence
<220>
   <223> Description of Artificial Sequence: Synthetic Peptide
<400> 815
   Tyr Gly Gly Phe Met Thr Ser Glu Lys Ser Gln Thr Pro Leu Val Thr
   1 5 10 15
   Leu Phe Lys Asn Ala Ile Ile Lys Asn Ala Tyr Lys Lys Gly Glu
   20 25 30
<210> 816
   <211> 26
   <212> PRT
   <213> Artificial Sequence
<220>
   <223> Description of Artificial Sequence: Synthetic Peptide
<400> 816
   Tyr Gly Gly Phe Met Thr Ser Glu Lys Ser Gln Thr Pro Leu Val Thr
   1 5 10 15
   Leu Phe Lys Asn Ala Ile Ile Lys Asn Ala
   20 25
<210> 817
   <211> 27
   <212> PRT
   <213> Artificial Sequence
<220>
   <223> Description of Artificial Sequence: Synthetic Peptide
<400> 817
   Tyr Gly Gly Phe Met Thr Ser Glu Lys Ser Gln Thr Pro Leu Val Thr
   1 5 10 15
   Leu Phe Lys Asn Ala Ile Ile Lys Asn Ala Tyr
   20 25
<210> 818
   <211> 17
   <212> PRT
   <213> Artificial Sequence
<220>
   <223> Description of Artificial Sequence: Synthetic Peptide
<400> 818
   Tyr Gly Gly Phe Met Thr Ser Glu Lys Ser Gln Thr Pro Leu Val Thr
   1 5 10 15
   Leu
<210> 819
   <211> 16
   <212> PRT
   <213> Artificial Sequence
<220>
   <223> Description of Artificial Sequence: Synthetic Peptide
<400> 819
   Tyr Gly Gly Phe Met Thr Ser Glu Lys Ser Gln Thr Pro Leu Val Thr
   1 5 10 15
<210> 820
   <211> 16
   <212> PRT
   <213> Artificial Sequence
<220>
   <223> Description of Artificial Sequence: Synthetic Peptide
<400> 820
   Tyr Gly Gly Phe Met Thr Ser Glu Lys Ser Gln Thr Pro Leu Val Thr
   1 5 10 15
<210> 821
   <211> 11
   <212> PRT
   <213> Artificial Sequence
<220>
   <223> Description of Artificial Sequence: Synthetic Peptide
<400> 821
   Tyr Gly Gly Phe Leu Arg Lys Tyr Arg Pro Lys
   1 5 10
<210> 822
   <211> 7
   <212> PRT
   <213> Artificial Sequence
<220>
   <223> Description of Artificial Sequence: Synthetic Peptide
<400> 822
   Tyr Gly Gly Phe Leu Arg Lys
   1 5
<210> 823
   <211> 8
   <212> PRT
   <213> Artificial Sequence
<220>
   <223> Description of Artificial Sequence: Synthetic Peptide
<400> 823
   Tyr Gly Gly Phe Leu Arg Lys Arg
   1 5
<210> 824
   <211> 31
   <212> PRT
   <213> Artificial Sequence
<220>
   <223> Description of Artificial Sequence: Synthetic Peptide
<400> 824
   Tyr Gly Gly Phe Met Thr Ser Glu Lys Ser Gln Thr Pro Leu Val Thr
   1 5 10 15
   Leu Phe Lys Asn Ala Ile Ile Lys Asn Ala His Lys Lys Gly Gln
   20 25 30
<210> 625
   <211> 27
   <212> PRT
   <213> Artificial Sequence
<220>
   <223> Description of Artificial Sequence: Synthetic Peptide
<400> 825
<210> 826
   <211> 31
   <212> PRT
   <213> Artificial Sequence
<220>
   <223> Description of Artificial Sequence: Synthetic Peptide
<400> 826
   Tyr Gly Gly Phe Met Ser Ser Glu Lys Ser Gln Thr Pro Leu Val Thr
   1 5 10 15
   Leu Phe Lys Asn Ala Ile Ile Lys Asn Ala His Lys Lys Gly Gln
   20 25 30
<210> 827
   <211> 31
   <212> PRT
   <213> Artificial Sequence
<220>
   <223> Description of Artificial Sequence: Synthetic Peptide
<400> 827
   Tyr Gly Gly Phe Met Thr Ser Glu Lys Ser Gln Thr Pro Leu Val Thr
   1 5 10 15
   Leu Phe Lys Asn Ala Ile Ile Lys Asn Ala Tyr Lys Lys Gly Glu
   20 25 30
<210> 828
   <211> 21
   <212> PRT
   <213> Artificial Sequence
<220>
   <223> Description of Artificial Sequence: Synthetic Peptide
<400> 828
   Tyr Gly Gly Phe Met Thr Leu Phe Lys Asn Ala Ile Ile Lys Asn Ala
   1 5 10 15
   Tyr Lys Lys Gly Glu
   20
<210> 829
   <211> 26
   <212> PRT
   <213> Artificial Sequence
<220>
   <223> Description of Artificial Sequence: Synthetic Peptide
<400> 829
   Tyr Gly Gly Phe Met Thr Ser Glu Lys Ser Gln Thr Pro Leu Val Thr
   1 5 10 15
   Leu Phe Lys Asn Ala Ile Ile Lys Asn Ala
   20 25
<210> 830
   <211> 27
   <212> PRT
   <213> Artificial Sequence
<220>
   <223> Description of Artificial Sequence: Synthetic Peptide
<400> 830
   Tyr Gly Gly Phe Met Thr Ser Glu Lys Ser Gln Thr Pro Leu Val Thr
   1 5 10 15
   Leu Phe Lys Asn Ala Ile Ile Lys Asn Ala Tyr
   20 25
<210> 831
   <211> 26
   <212> PRT
   <213> Artificial Sequence
<220>
   <223> Description of Artificial Sequence: Synthetic Peptide
<400> 831
   Thr Ser Glu Lys Ser Gln Thr Pro Leu Val Thr Leu Phe Lys Asn Ala
   1 5 10 15
   Ile Ile Lys Asn Ala Tyr Lys Lys Gly Glu
   20 25
<210> 832
   <211> 14
   <212> PRT
   <213> Artificial Sequence
<220>
   <223> Description of Artificial Sequence: Synthetic Peptide
<400> 832
   Phe Lys Asn Ala Ile Ile Lys Asn Ala Tyr Lys Lys Gly Glu
   1 5 10
<210> 833
   <211> 31
   <212> PRT
   <213> Artificial Sequence
<220>
   <223> Description of Artificial Sequence: Synthetic Peptide
<400> 833
   Tyr Gly Gly Phe Met Thr Ser Glu Lys Ser Gln Thr Pro Leu Val Thr
   1 5 10 15
   Leu Phe Lys Asn Ala Ile Val Lys Asn Ala His Lys Lys Gly Gln
   20 25 30
<210> 834
   <211> 31
   <212> PRT
   <213> Artificial Sequence
<220>
   <223> Description of Artificial Sequence: Synthetic Peptide
<400> 834
   Tyr Gly Gly Phe Met Thr Ser Glu Lys Ser Gln Thr Pro Leu Val Thr
   1 5 10 15
   Leu Phe Lys Asn Ala Ile Ile Lys Asn Val His Lys Lys Gly Gln
   20 25 30
<210> 835
   <211> 10
   <212> PRT
   <213> Artificial Sequence
<220>
   <223> Description of Artificial Sequence: Synthetic Peptide
<400> 835
   Glu Leu Ala Gly Ala Pro Pro Glu Pro Ala
   1 5 10
<210> 836
   <211> 4
   <212> PRT
   <213> Artificial Sequence
<220>
   <223> Description of Artificial Sequence: Synthetic Peptide
<400> 836
   Tyr Gly Gly Phe
   1
<210> 837
   <211> 9
   <212> PRT
   <213> Artificial Sequence
<220>
   <223> Description of Artificial Sequence: Synthetic Peptide
<400> 837
   Tyr Gly Gly Phe Met Thr Ser Glu Lys
   1 5

<210> 838
   <211> 4
   <212> PRT
   <213> Artificial Sequence
<220>
   <223> Description of Artificial Sequence: Synthetic Peptide
<400> 838
   Lys Lys Gly Glu
   1
<210> 839
   <211> 31
   <212> PRT
   <213> Artificial Sequence
<220>
   <223> Description of Artificial Sequence: Synthetic Peptide
<400> 839
   Tyr Gly Gly Phe Met Thr Ser Glu Lys Ser Gln Thr Pro Leu Val Thr
   1 5 10 15
   Leu Phe Lys Asn Ala Ile Ile Lys Asn Ala Tyr Lys Lys Gly Glu
   20 25 30
<210> 840
   <211> 17
   <212> PRT
   <213> Artificial Sequence
<220>
   <223> Description of Artificial Sequence: Synthetic Peptide
<400> 840
   Tyr Gly Gly Phe Met Thr Ser Glu Lys Ser Gln Thr Pro Leu Val Thr
   1 5 10 15
   Leu
<210> 841
   <211> 1
   <212> PRT
   <213> Artificial Sequence
<220>
   <223> Description of Artificial Sequence: Synthetic Peptide
<400> 841
   Tyr
   1
<210> 842
   <211> 8
   <212> PRT
   <213> Artificial Sequence
<220>
   <223> Description of Artificial Sequence: Synthetic Peptide
<400> 842
   Tyr Gly Phe Met Thr Ser Glu Lys
   1 5
<210> 843
   <211> 8
   <212> PRT
   <213> Artificial Sequence
<220>
   <223> Description of Artificial Sequence: Synthetic Peptide
<400> 843
   Tyr Gly Phe Met Thr Ser Glu Lys
   1 5
<210> 844
   <211> 16
   <212> PRT
   <213> Artificial Sequence
<220>
   <223> Description of Artificial Sequence: Synthetic Peptide
<400> 844
   Tyr Gly Phe Met Thr Ser Glu Lys Ser Gln Thr Pro Leu Val Thr Leu
   1 5 10 15
<210> 845
   <211> 16
   <212> PRT
   <213> Artificial Sequence
<220>
   <223> Description of Artificial Sequence: Synthetic Peptide
<400> 845
   Gly Gly Phe Met Thr Ser Glu Lys Ser Gln Thr Pro Leu Val Thr Leu
   1 5 10 15
<210> 846
   <211> 31
   <212> PRT
   <213> Artificial Sequence
<220>
   <223> Description of Artificial Sequence: Synthetic Peptide
<400> 846
   Tyr Gly Gly Phe Leu Thr Ser Glu Lys Ser Gln Thr Pro Leu Val Thr
   1 5 10 15
   Leu Phe Lys Asn Ala Ile Ile Lys Asn Ala His Lys Lys Gly Gln
   20 25 30
<210> 847
   <211> 6
   <212> PRT
   <213> Artificial Sequence
<220>
   <223> Description of Artificial Sequence: Synthetic Peptide
<400> 847
   Tyr Gly Gly Phe Met Lys
   1 5
<210> 848
   <211> 7
   <212> PRT
   <213> Artificial Sequence
<220>
   <223> Description of Artificial Sequence: Synthetic Peptide
<400> 848
   Tyr Gly Gly Phe Met Lys Lys
   1 5
<210> 849
   <211> 7
   <212> PRT
   <213> Artificial Sequence
<220>
   <223> Description of Artificial Sequence: Synthetic Peptide
<400> 849
   Tyr Gly Gly Phe Met Lys Arg
   1 5
<210> 850
   <211> 8
   <212> PRT
   <213> Artificial Sequence
<220>
   <223> Description of Artificial Sequence: Synthetic Peptide
<400> 850
<210> 851
   <211> 8
   <212> PRT
   <213> Artificial Sequence
<220>
   <223> Description of Artificial Sequence: Synthetic Peptide
<400> 851
   Ala Ala Ala Tyr Gly Gly Phe Met
   1 5
<210> 852
   <211> 26
   <212> PRT
   <213> Artificial Sequence
<220>
   <223> Description of Artificial Sequence: Synthetic Peptide
<400> 852
   Tyr Gly Gly Phe Met Lys Lys Met Asp Glu Leu Tyr Pro Leu Glu Val
   1 5 10 15
   Glu Glu Glu Ala Asn Gly Gly Glu Val Leu
   20 25
<210> 853
   <211> 26
   <212> PRT
   <213> Artificial Sequence
<220>
   <223> Description of Artificial Sequence: Synthetic Peptide
<400> 853
   Tyr Gly Gly Phe Met Lys Lys Met Asp Glu Leu Tyr Pro Leu Glu Val
   1 5 10 15
   Glu Glu Glu Ala Asn Gly Gly Glu Val Leu
   20 25
<210> 854
   <211> 12
   <212> PRT
   <213> Artificial Sequence
<220>
   <223> Description of Artificial Sequence: Synthetic Peptide
<400> 854
   Tyr Gly Gly Phe Met Arg Arg Val Gly Arg Pro Glu
   1 5 10
<210> 855
   <211> 12
   <212> PRT
   <213> Artificial Sequence
<220>
   <223> Description of Artificial Sequence: Synthetic Peptide
<400> 855
   Tyr Gly Gly Phe Met Arg Arg Val Gly Arg Pro Glu
   1 5 10
<210> 856
   <211> 22
   <212> PRT
   <213> Artificial Sequence
<220>
   <223> Description of Artificial Sequence: Synthetic Peptide
<400> 856
   Tyr Gly Gly Phe Met Arg Arg Val Gly Arg Pro Glu Trp Trp Met Asp
   1 5 10 15
   Tyr Gln Lys Arg Tyr Gly
   20
<210> 857
   <211> 3
   <212> PRT
   <213> Artificial Sequence
<220>
   <223> Description of Artificial Sequence: Synthetic Peptide
<400> 857
   Phe Ala Arg
   1
<210> 858
   <211> 5
   <212> PRT
   <213> Artificial Sequence
<220>
   <223> Description of Artificial Sequence: Synthetic Peptide
<400> 858
   Tyr Ala Gly Phe Gly
   1 5
<210> 859
   <211> 5
   <212> PRT
   <213> Artificial Sequence
<220>
   <223> Description of Artificial Sequence: Synthetic Peptide
<400> 859
   Tyr Ala Gly Phe Leu
   1 5
<210> 860
   <211> 5
   <212> PRT
   <213> Artificial Sequence
<220>
   <223> Description of Artificial Sequence: Synthetic Peptide
<400> 860
   Tyr Ala Gly Phe Met
   1 5
<210> 861
   <211> 4
   <212> PRT
   <213> Artificial Sequence
<220>
   <223> Description of Artificial Sequence: Synthetic Peptide
<400> 861
   Tyr Gly Phe Leu
   1
<210> 862
   <211> 5
   <212> PRT
   <213> Artificial Sequence
<220>
   <223> Description of Artificial Sequence: Synthetic Peptide
<400> 862
   Tyr Gly Phe Leu Arg
   1 5
<210> 863
   <211> 2
   <212> PRT
   <213> Artificial Sequence
<220>
   <223> Description of Artificial Sequence: Synthetic Peptide
<400> 863
   Gly Phe
   1
<210> 864
   <211> 2
   <212> PRT
   <213> Artificial Sequence
<220>
   <223> Description of Artificial Sequence: Synthetic Peptide
<400> 864
   Gly Phe
   1
<210> 865
   <211> 4
   <212> PRT
   <213> Artificial Sequence
<220>
   <223> Description of Artificial Sequence: Synthetic Peptide
<400> 86.5
<210> 866
   <211> 3
   <212> PRT
   <213> Artificial Sequence
<220>
   <223> Description of Artificial Sequence: Synthetic Peptide
<400> 866
   Tyr Gly Phe
   1
<210> 867
   <211> 3
   <212> PRT
   <213> Artificial Sequence
<220>
   <223> Description of Artificial Sequence: Synthetic Peptide
<400> 867
   Tyr Gly Phe
   1
<210> 868
   <211> 3
   <212> PRT
   <213> Artificial Sequence
<220>
   <223> Description of Artificial Sequence: Synthetic Peptide
<400> 868
   Phe Leu Arg
   1
<210> 869
   <211> 3
   <212> PRT
   <213> Artificial Sequence
<220>
   <223> Description of Artificial Sequence: Synthetic Peptide
<400> 869
   Tyr Gly Phe
   1
<210> 870
   <211> 5
   <212> PRT
   <213> Artificial Sequence
<220>
   <223> Description of Artificial Sequence: Synthetic Peptide
<400> 870
   Tyr Gly Gly Phe Leu
   1 5
<210> 871
   <211> 5
   <212> PRT
   <213> Artificial Sequence
<220>
   <223> Description of Artificial Sequence: Synthetic Peptide
<400> 871
   Tyr Gly Gly Phe Leu
   1 5
<210> 872
   <211> 5
   <212> PRT
   <213> Artificial Sequence
<220>
   <223> Description of Artificial Sequence: Synthetic Peptide
<400> 872
   Tyr Gly Gly Phe Leu
   1 5
<210> 873
   <211> 5
   <212> PRT
   <213> Artificial Sequence
<220>
   <223> Description of Artificial Sequence: Synthetic Peptide
<400> 873
   Tyr Ala Gly Phe Leu
   1 5
<210> 874
   <211> 6
   <212> PRT
   <213> Artificial Sequence
<220>
   <223> Description of Artificial Sequence: Synthetic Peptide
<400> 874
   Tyr Ser Gly Phe Leu Thr
   1 5
<210> 875
   <211> 6
   <212> PRT
   <213> Artificial Sequence
<220>
   <223> Description of Artificial Sequence: Synthetic Peptide
<400> 875
   Tyr Thr Gly Phe Leu Thr
   1 5
<210> 876
   <211> 6
   <212> PRT
   <213> Artificial Sequence
<220>
   <223> Description of Artificial Sequence: Synthetic Peptide
<400> 876
   Tyr Gly Gly Phe Leu Lys
   1 5
<210> 877
   <211> 6
   <212> PRT
   <213> Artificial Sequence
<220>
   <223> Description of Artificial Sequence: Synthetic Peptide
<400> 877
   Tyr Gly Gly Phe Met Arg
   1 5
<210> 878
   <211> 7
   <212> PRT
   <213> Artificial Sequence
<220>
   <223> Description of Artificial Sequence: Synthetic Peptide
<400> 878
   Tyr Gly Gly Phe Met Arg Arg
   1 5
<210> 879
   <211> 9
   <212> PRT
   <213> Artificial Sequence
<220>
   <223> Description of Artificial Sequence: Synthetic Peptide
<400> 879
   Tyr Gly Gly Phe Met Arg Arg Val Gly
   1 5
<210> 880
   <211> 8
   <212> PRT
   <213> Artificial Sequence
<220>
   <223> Description of Artificial Sequence: Synthetic Peptide
<400> 880
   Tyr Gly Gly Phe Met Arg Gly Leu
   1 5
<210> 881
   <211> 7
   <212> PRT
   <213> Artificial Sequence
<220>
   <223> Description of Artificial Sequence: Synthetic Peptide
<400> 881
   Tyr Gly Gly Phe Met Arg Phe
   1 5
<210> 882
   <211> 5
   <212> PRT
   <213> Artificial Sequence
<220>
   <223> Description of Artificial Sequence: Synthetic Peptide
<400> 882
   Tyr Gly Gly Phe Met
   1 5
<210> 883
   <211> 5
   <212> PRT
   <213> Artificial Sequence
<220>
   <223> Description of Artificial Sequence: Synthetic Peptide
<400> 883
   Tyr Gly Gly Phe Met
   1 5
<210> 884
   <211> 5
   <212> PRT
   <213> Artificial Sequence
<220>
   <223> Description of Artificial Sequence: Synthetic Peptide
<400> 884
   Tyr Ala Gly Phe Met
   1 5
<210> 885
   <211> 5
   <212> PRT
   <213> Artificial Sequence
<220>
   <223> Description of Artificial Sequence: Synthetic Peptide
<400> 885
   Tyr Ala Gly Phe Met
   1 5
<210> 886
   <211> 7
   <212> PRT
   <213> Artificial Sequence
<220>
   <223> Description of Artificial Sequence: Synthetic. Peptide
<400> 886
   Tyr Gly Gly Phe Met Arg Phe
   1 5
<210> 887
   <211> 5
   <212> PRT
   <213> Artificial Sequence
<220>
   <223> Description of Artificial Sequence: Synthetic Peptide
<400> 887
   Tyr Gly Gly Phe Met
   1 5
<210> 888
   <211> 5
   <212> PRT
   <213> Artificial Sequence
<220>
   <223> Description of Artificial Sequence: Synthetic Peptide
<400> 888
   Tyr Ala Gly Phe Met
   1 5
<210> 889
   <211> 4
   <212> PRT
   <213> Artificial Sequence
<220>
   <223> Description of Artificial Sequence: Synthetic Peptide
<400> 889
   Tyr Gly Phe Pro
   1
<210> 890
   <211> 4
   <212> PRT
   <213> Artificial Sequence
<220>
   <223> Description of Artificial Sequence: Synthetic Peptide
<400> 890
   Tyr Gly Phe Met
   1
<210> 891
   <211> 8
   <212> PRT
   <213> Artificial Sequence
<220>
   <223> Description of Artificial Sequence: Synthetic Peptide
<400> 891
<210> 892
   <211> 31
   <212> PRT
   <213> Artificial Sequence
<220>
   <223> Description of Artificial Sequence: Synthetic Peptide
<400> 892
   Phe Ala Glu Pro Leu Pro Ser Glu Glu Glu Gly Glu Ser Tyr Ser Lys
   1 5 10 15
   Glu Val Pro Glu Met Glu Lys Arg Tyr Gly Gly Phe Met Arg Phe
   20 25 30
<210> 893
   <211> 25
   <212> PRT
   <213> Artificial Sequence
<220>
   <223> Description of Artificial Sequence: synthetic Peptide
<400> 893
   Tyr Gly Gly Phe Met Arg Arg Val Gly Arg Pro Glu Trp Trp Met Asp
   1 5 10 15
   Tyr Gln Lys Arg Tyr Gly Gly Phe Leu
   20 25
<210> 894
   <211> 34
   <212> PRT
   <213> Artificial Sequence
<220>
   <223> Description of Artificial Sequence: Synthetic Peptide
<400> 894
   Tyr Gly Gly Phe Met Lys Lys Met Asp Glu Leu Tyr Pro Leu Glu Val
   1               5                   10                  15
   Glu Glu Glu Ala Asn Gly Gly Glu Val Leu Gly Lys Arg Tyr Gly Gly
               20                  25                  30
   Phe Met
<210> 895
   <211> 19
   <212> PRT
   <213> Artificial Sequence
<220>
   <223> Description of Artificial sequence: Synthetic Peptide
<400> 895
   Ser Ser Glu Val Ala Gly Glu Gly Asp Gly Asp Ser Met Gly His Glu
   1 5 10 15
   Asp Leu Tyr
<210> 896
   <211> 6
   <212> PRT
   <213> Artificial Sequence
<220>
   <223> Description of Artificial Sequence: Synthetic Peptide
<400> 896
   Leu Val Val Tyr Pro Trp
   1 5
<210> 897
   <211> 2
   <212> PRT
   <213> Artificial Sequence
<220>
   <223> Description of Artificial Sequence: Synthetic Peptide
<400> 897
   Pro Gly
   1
<210> 898
   <211> 4
   <212> PRT
   <213> Artificial Sequence
<220>
   <223> Description of Artificial Sequence: Synthetic Peptide
<400> 898
   Tyr Pro Phe Pro
   1
<210> 99
   <211> 5
   <212> PRT
   <213> Artificial Sequence
<220>
   <223> Description of Artificial Sequence: Synthetic Peptide
<400> 899
<210> 900
   <211> 4
   <212> PRT
   <213> Artificial Sequence
<220>
   <223> Description of Artificial Sequence: Synthetic Peptide
<400> 900
   Tyr Pro Pro Pro
   1
<210> 901
   <211> 6
   <212> PRT
   <213> Artificial Sequence
<220>
   <223> Description of Artificial Sequence: Synthetic Peptide
<400> 901
   Arg Tyr Leu Gly Tyr Leu
   1 5
<210> 902
   <211> 8
   <212> PRT
   <213> Artificial Sequence
<220>
   <223> Description of Artificial Sequence: Synthetic Peptide
<400> 902
   Ala Ala Ala Tyr Gly Gly Phe Leu
   1 5
<210> 903
   <211> 8
   <212> PRT
   <213> Artificial Sequence
<220>
   <223> Description of Artificial Sequence: Synthetic Peptide
<400> 903
   Ala Ala Ala Tyr Gly Gly Phe Leu
   1 5
<210> 904
   <211> 18
   <212> PRT
   <213> Artificial Sequence
<220>
   <223> Description of Artificial Sequence: Synthetic Peptide
<400> 904
   Tyr Gly Gly Phe Met Arg Arg Val Gly Arg Pro Glu Trp Trp Met Asp
   1 5 10 15
   Tyr Gln
<210> 905
   <211> 6
   <212> PRT
   <213> Artificial Sequence
<220>
   <223> Description of Artificial Sequence: Synthetic Peptide
<400> 905
   Tyr Pro Phe Pro Pro Leu
   1 5
<210> 906
   <211> 7
   <212> PRT
   <213> Artificial Sequence
<220>
   <223> Description of Artificial Sequence: Synthetic Peptide
<400> 906
   Tyr Val Pro Phe Pro Pro Phe
   1 5
<210> 907
   <211> 2
   <212> PRT
   <213> Artificial Sequence
<220>
   <223> Description of Artificial Sequence: Synthetic Peptide
<400> 907
   Trp Gly
   1
<210> 908
   <211> 4
   <212> PRT
   <213> Artificial Sequence
<220>
   <223> Description of Artificial Sequence: Synthetic Peptide
<400> 908
   Tyr Arg Phe Lys
   1
<210> 909
   <211> 7
   <212> PRT
   <213> Artificial Sequence
<220>
   <223> Description of Artificial Sequence: Synthetic Peptide
<400> 909
   Tyr Met Phe His Leu Met Asp
   1 5
<210> 910
<211> 7
   <212> PRT
   <213> Artificial Sequence
<220>
   <223> Description of Artificial Sequence: Synthetic Peptide
<400> 910
   Tyr Ala Phe Asp Val Val Gly
   1 5
<210> 911
   <211> 7
   <212> PRT
   <213> Artificial Sequence
<220>
   <223> Description of Artificial Sequence: Synthetic Peptide
<400> 911
   Tyr Ala Phe Glu Val Val Gly
   1 5
<210> 912
   <211> 4
   <212> PRT
   <213> Artificial Sequence
<220>
   <223> Description of Artificial Sequence: Synthetic Peptide
<400> 912
   Tyr Pro Trp Phe
   1
<210> 913
   <211> 5
   <212> PRT
   <213> Artificial Sequence
<220>
   <223> Description of Artificial Sequence: Synthetic Peptide
<400> 913
   Tyr Pro Trp Phe Phe
   1 5
<210> 914
   <211> 2
   <212> PRT
   <213> Artificial Sequence
<220>
   <223> Description of Artificial Sequence: Synthetic Peptide
<400> 914
   Tyr Arg
   1
<210> 915
   <211> 1
   <212> PRT
   <213> Artificial Sequence
<220>
   <223> Description of Artificial Sequence: Synthetic Peptide
<400> 915
   Tyr
   1
<210> 916
   <211> .2.
   <212> PRT
   <213> Artificial Sequence
<220>
   <223> Description of Artificial Sequence: Synthetic Peptide
<400> 916
<210> 917
   <211> 4
   <212> PRT
   <213> Artificial Sequence
<220>
   <223> Description of Artificial Sequence: Synthetic Peptide
<400> 917
   Pro Gln Arg Phe
   1
<210> 918
   <211> 18
   <212> PRT
   <213> Artificial Sequence
<220>
   <223> Description of Artificial Sequence: Synthetic Peptide
<400> 918
   Ala Gly Glu Gly Leu Ser Ser Pro Phe Trp Ser Leu Ala Ala Pro Gln
   1 5 10 15
   Arg Phe
<210> 919
   <211> 17
   <212> PRT
   <213> Artificial Sequence
<220>
   <223> Description of Artificial Sequence: Synthetic Peptide
<400> 919
   Phe Gly Gly Phe Thr Gly Ala Arg Lys Ser Ala Arg Lys Leu Ala Asn
   1 5 10 15
   Gln

<210> 920
   <211> 3
   <212> PRT
   <213> Artificial Sequence
<220>
   <223> Description of Artificial Sequence: Synthetic Peptide
<400> 920
   Tyr Phe Phe
   1
<210> 921
   <211> 4
   <212> PRT
   <213> Artificial Sequence
<220>
   <223> Description of Artificial Sequence: Synthetic Peptide
<400> 921
   Tyr Pro Leu Gly
   1
<210> 922
   <211> 4
   <212> PRT
   <213> Artificial Sequence
<220>
   <223> Description of Artificial Sequence: Synthetic Peptide
<400> 922
   Tyr Pro Trp Gly
   1
<210> 923
   <211> 7
   <212> PRT
   <213> Artificial Sequence
<220>
   <223> Description of Artificial Sequence: Synthetic Peptide
<400> 923
   Val Val Tyr Pro Trp Thr Gln
   1 5
<210> 924
   <211> 9
   <212> PRT
   <213> Artificial Sequence
<220>
   <223> Description of Artificial Sequence: Synthetic Peptide
<400> 924
   Leu Val Val Tyr Pro Trp Thr Gln Arg
   1 5
<210> 925
   <211> 2
   <212> PRT
   <213> Artificial Sequence
<220>
   <223> Description of Artificial Sequence: Synthetic Peptide
<400> 925
   Pro Leu
   1
<210> 926
   <211> 17
   <212> PRT
   <213> Artificial Sequence
<220>
   <223> Description of Artificial Sequence: Synthetic Peptide
<400> 926
   Phe Gly Gly Phe Thr Gly Ala Arg Lys Ser Ala Arg Lys Leu Ala Asn
   1 5 10 15
   Gln
<210> 927
   <211> 8
   <212> PRT
   <213> Artificial Sequence
<220>
   <223> Description of Artificial Sequence: Synthetic Peptide
<400> 927
<210> 928
   <211> 8
   <212> PRT
   <213> Artificial Sequence
<220>
   <223> Description of Artificial Sequence: Synthetic Peptide
<400> 928
   Ala Lys Ser Gln Gly Gly Ser Asn
   1 5
<210> 929
   <211> 8
   <212> PRT
   <213> Artificial Sequence
<220>
   <223> Description of Artificial Sequence: Synthetic Peptide
<400> 929
   Leu Glu Asp Gly Pro Lys Phe Leu
   1 5
<210> 930
   <211> 5
   <212> PRT
   <213> Artificial Sequence
<220>
   <223> Description of Artificial Sequence: Synthetic Peptide
<400> 930
   Arg Lys Asp Val Tyr
   1               5
<210> 931
   <211> 13
   <212> PRT
   <213> Artificial Sequence
<220>
   <223> Description of Artificial Sequence: Synthetic Peptide
<400> 931
   Gly Glu Gln Arg Lys Asp Val Tyr Val Gln Leu Tyr Leu
   1               5                   10
<210> 932
   <211> 28
   <212> PRT
   <213> Artificial Sequence
<220>
   <223> Description of Artificial Sequence: Synthetic Peptide
<400> 932
   Ser Asp Ala Ala Val Asp Thr Ser Ser Glu Ile Thr Thr Lys Asp Leu
   1               5                   10                  15
   Lys Glu Lys Lys Glu Val Val Glu Glu Ala Glu Asn
               20                  25
<210> 933
   <211> 10
   <212> PRT
   <213> Artificial Sequence
<220>
   <223> Description of Artificial Sequence: Synthetic Peptide
<400> 933
   Tyr Gln Ala Lys Ser Gln Gly Gly Ser Asn
   1 5 10
<210> 934
   <211> 28
   <212> PRT
   <213> Artificial Sequence
<220>
   <223> Description of Artificial Sequence: Synthetic Peptide
<400> 934
   Ser Asp Ala Ala Val Asp Thr Ser Ser Glu Ile Thr Thr Lys Asp Leu
   1 5 10 15
   Lys Glu Lys Lys Glu Val Val Glu Glu Ala Glu Asn
   20 25
<210> 935
   <211> 52
   <212> PRT
   <213> Artificial Sequence
<220>
   <223> Description of Artificial Sequence: Synthetic Peptide
<400> 935
   Tyr Arg Gln Ser Met Asn Asn Phe Gln Gly Leu Arg Ser Phe Gly Cys
   1 5 10 15
   Arg Phe Gly Thr Cys Thr Val Gln Lys Leu Ala His Gln Ile Tyr Gln
   20 25 30
   Phe Thr Asp Lys Asp Lys Asp Asn Val Ala Pro Arg Ser Lys Ile Ser
   35 40 45
   Pro Gln Gly Tyr
   50
<210> 936
   <211> 12
   <212> PRT
   <213> Artificial Sequence
<220>
   <223> Description of Artificial Sequence: Synthetic Peptide
<400> 936
   Tyr Arg Gln Ser Met Asn Asn Phe Gln Gly Leu Arg
   1 5 10
<210> 937
   <211> 37
   <212> PRT
   <213> Artificial Sequence
<220>
   <223> Description of Artificial Sequence: Synthetic Peptide
<400> 937
   Ser Phe Gly Cys Arg Phe Gly Thr Cys Thr Val Gln Lys Leu Ala His
   1 5 10 15
   Gln Ile Tyr Phe Thr Asp Lys Asp Asn Val Ala Pro Arg Ser Lys Ile
   20 25 30
   Ser Pro Gln Gly Tyr
   35
<210> 938
   <211> 31
   <212> PRT
   <213> Artificial Sequence
<220>
   <223> Description of Artificial Sequence: Synthetic Peptide
<400> 938
   Thr Val Gln Lys Leu Ala His Gln Ile Tyr Gln Phe Thr Asp Lys Asp
   1 5 10 15
   Lys Asp Asn Val Ala Pro Arg Ser Lys Ile Ser Pro Gln Gly Tyr
   20 25 30
<210> 939
   <211> 48
   <212> PRT
   <213> Artificial Sequence
<220>
   <223> Description of Artificial Sequence: Synthetic Peptide
<400> 939
   Glu Leu Arg Met Ser Ser Ser Tyr Pro Thr Gly Leu Ala Asp Val Lys
   1 5 10 15
   Ala Gly Pro Ala Gln Thr Leu Ile Arg Pro Gln Asp Met Lys Gly Ala
   20 25 30
   Ser Arg Ser Pro Glu Asp Ser Ser Pro Asp Ala Ala Arg Ile Arg Val
   35 40 45
<210> 940
   <211> 33
   <212> PRT
   <213> Artificial Sequence
<220>
   <223> Description of Artificial Sequence: Synthetic Peptide
<400> 940
   Ser Leu Pro Glu Ala Gly Pro Gly Arg Thr Leu Val Ser Ser Lys Pro
   1 5 10 15
   Gln Ala His Gly Ala Pro Ala Pro Pro Ser Gly Ser Ala Pro His Phe
   20 25 30
   Leu
<210> 941
   <211> 52
   <212> PRT
   <213> Artificial Sequence
<220>
   <223> Description of Artificial Sequence: Synthetic Peptide
<400> 941
   Tyr Arg Gln Ser Met Asn Phe Gln Gly Leu Arg Ser Phe Gly Cys Arg
   1 5 10 15
   Phe Gly Thr Cys Thr Val Gln Lys Leu Ala His Gln Ile Tyr Gln Phe
   20 25 30
   Thr Asp Lys Asp Gly Val Ala Pro Arg Ser Lys Ile Ser Lys Ile Ser
   35 40 45
   Pro Gln Gly Tyr
   50
<210> 942
   <211> 20
   <212> PRT
   <213> Artificial Sequence
<220>
   <223> Description of Artificial Sequence: Synthetic Peptide
<400> 942
   Ala Arg Leu Asp Val Ala Ala Glu Phe Arg Lys Lys Trp Asn Lys Trp
   1 5 10 15
   Ala Leu Ser Arg
   20
<210> 943
   <211> 53
   <212> PRT
   <213> Artificial Sequence
<220>
   <223> Description of Artificial Sequence: Synthetic Peptide
<400> 943
   Tyr Arg Gln Ser Met Asn Gln Gly Ser Arg Ser Thr Gly Cys Arg Phe
   1 5 10 15
   Gly Thr Cys Thr Met Gln Lys Leu Ala His Gln Ile Tyr Gln Ile Tyr
   20 25 30
   Gln Phe Thr Asp Lys Asp Lys Asp Gly Met Ala Pro Arg Asn Lys Ile
   35 40 45
   Ser Pro Gln Gly Tyr
   50
<210> 944
   <211> 40
   <212> PRT
   <213> Artificial Sequence
<220>
   <223> Description of Artificial Sequence: Synthetic Peptide
<400> 944
   Ser Thr Gly Cys Arg Phe Gly Thr Cys Thr Met Gln Lys Leu Ala His
   1 5 10 15
   Gln Ile Tyr Gln Phe Thr Asp Lys Asp Lys Asp Gly Met Ala Pro Arg
   20 25 30
   Asn Lys Ile Ser Pro Gln Gly Tyr
   35 40
<210> 945
   <211> 20
   <212> PRT
   <213> Artificial Sequence
<220>
   <223> Description of Artificial Sequence: Synthetic Peptide
<400> 945
   Ala Arg Leu Asp Thr Ser Ser Gln Phe Arg Lys Lys Trp Asn Lys Trp
   1 5 10 15
   Ala Leu Ser Arg
   20
<210> 946
   <211> 13
   <212> PRT
   <213> Artificial Sequence
<220>
   <223> Description of Artificial Sequence: Synthetic Peptide
<400> 946
   Ala Pro Ser Gly Ala Gln Arg Leu Tyr Gly Phe Gly Leu
   1 5 10
<210> 947
   <211> 10
   <212> PRT
   <213> Artificial Sequence
<220>
   <223> Description of Artificial Sequence: Synthetic Peptide
<400> 947
   Gly Asp Gly Arg Leu Tyr Ala Phe Gly Leu
   1 5 10
<210> 948
   <211> 5
   <212> PRT
   <213> Artificial Sequence
<220>
   <223> Description of Artificial Sequence: Synthetic Peptide
<400> 948
<210> 949
<211> 8
   <212> PRT
   <213> Artificial Sequence
<220>
   <223> Description of Artificial Sequence: Synthetic Peptide
<400> 949
   Asp Arg Leu Tyr Ser Phe Gly Leu
   1 5
<210> 950
   <211> 17
   <212> PRT
   <213> Artificial Sequence
<220>
   <223> Description of Artificial Sequence: Synthetic Peptide
<400> 950
   Val His His Gln Lys Leu Val Phe Phe Ala Glu Asp Val Gly Ser Asn
   1 5 10 15
   Lys
<210> 951
   <211> 11
   <212> PRT
   <213> Artificial Sequence
<220>
   <223> Description of Artificial Sequence: Synthetic Peptide
<400> 951
   Gly Ser Asn Lys Gly Ala Ile Ile Gly Leu Met
   1 5 10
<210> 952
   <211> 5
   <212> PRT
   <213> Artificial Sequence
<220>
   <223> Description of Artificial Sequence: Synthetic Peptide
<400> 952
   Arg Glu Arg Met Ser
   1 5
<210> 953
   <211> 17
   <212> PRT
   <213> Artificial Sequence
<220>
   <223> Description of Artificial Sequence: Synthetic Peptide
<400> 953
   Ala Lys Glu Arg Leu Glu Ala Lys His Arg Glu Arg Met Ser Gln Val
   1 5 10 15
   Met
<210> 954
   <211> 43
   <212> PRT
   <213> Artificial Sequence
<220>
   <223> Description of Artificial Sequence: Synthetic Peptide
<400> 954
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val Ile Ala Thr
   35 40
<210> 955
   <211> 42
   <212> PRT
   <213> Artificial Sequence
<220>
   <223> Description of Artificial Sequence: Synthetic Peptide
<400> 955
<210> 956
   <211> 40
   <212> PRT
   <213> Artificial Sequence
<220>
   <223> Description of Artificial Sequence: Synthetic Peptide
<400> 956
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val
   35 40
<210> 957
   <211> 11
   <212> PRT
   <213> Artificial Sequence
<220>
   <223> Description of Artificial Sequence: Synthetic Peptide
<400> 957
   Tyr Glu Val His His Gln Lys Leu Val Phe Phe
   1 5 10
<210> 958
   <211> 14
   <212> PRT
   <213> Artificial Sequence
<220>
   <223> Description of Artificial Sequence: Synthetic Peptide
<400> 958
   Glu Asp Val Gly Ser Asn Lys Gly Ala Ile Ile Gly Leu Met
   1 5 10
<210> 959
   <211> 28
   <212> PRT
   <213> Artificial Sequence
<220>
   <223> Description of Artificial Sequence: Synthetic Peptide
<400> 959
   Asp Ala Glu Phe Arg His Asp Ser Gly Tyr Glu Val His His Gln Lys
   1 5 10 15
   Leu Val Phe Phe Ala Glu Asp Val Gly Ser Asn Lys
   20 25
<210> 960
   <211> 40
   <212> PRT
   <213> Artificial Sequence
<220>
   <223> Description of Artificial Sequence: Synthetic Peptide
<400> 960
   Val Val Gly Gly Val Met Leu Gly Ile Ile Ala Gly Lys Asn Ser Gly
   1 5 10 15
   Val Asp Glu Ala Phe Phe Val Leu Lys Gln His His Val Glu Tyr Gly
   20 25 30
   Ser Asp His Arg Phe Glu Ala Asp
   35 40
<210> 961
   <211> 42
   <212> PRT
   <213> Artificial Sequence
<220>
   <223> Description of Artificial Sequence: Synthetic Peptide
<400> 961
   Asp Ala Glu Phe Gly His Asp Ser Gly Phe Glu Val Arg His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val Ile Ala
   35 40
<210> 962
   <211> 40
   <212> PRT
   <213> Artificial Sequence
<220>
   <223> Description of Artificial Sequence: Synthetic Peptide
<400> 962
   Asp Ala Glu Phe Gly His Asp Ser Gly Phe Glu Val Arg His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val
   35 40
<210> 963
   <211> 11
   <212> PRT
   <213> Artificial Sequence
<220>
   <223> Description of Artificial Sequence: Synthetic Peptide
<400> 963
   Tyr Glu Val His His Gln Lys Leu Val Phe Phe
   1 5 10
<210> 964
   <211> 14
   <212> PRT
   <213> Artificial Sequence
<220>
   <223> Description of Artificial Sequence: Synthetic Peptide
<400> 964
   Glu Asp Val Gly Ser Asn Lys Gly Ala Ile Ile Gly Leu Met
   1 5 10
<210> 965
   <211> 28
   <212> PRT
   <213> Artificial Sequence
<220>
   <223> Description of Artificial Sequence: Synthetic Peptide
<400> 965
   Asp Ala Glu Phe Arg His Asp Ser Gly Tyr Glu Val His His Gln Lys
   1               5                   10                  15
   Leu Val Phe Phe Ala Glu Asp Val Gly Ser Asn Lys
               20                  25
<210> 966
   <211> 40
   <212> PRT<213> Artifical Sequence
   <220>
   <223> Description of Artificial Sequence Synthetic Peptide
   <400> 966
   Val Val Gly Gly Val Met Leu Gly Ile Ile Ala Gly Lys Asn Ser Gly
   1               5                   10                  15
   Val Asp Glu Ala Phe Phe Val Leu Lys Gln His His Val Glu Tyr Gly
               20                  25                  30
   Ser Asp His Arg Phe Glu Ala Asp
           35                  40
<210> 967
   <211> 42
   <212> PRT
   <213> Artificial Sequence
<220>
   <223> Description of Artificial Sequence: Synthetic Peptide
<400> 967
   Asp Ala Glu Phe Gly His Asp Ser Gly Phe Glu Val Arg His Gln Lys
   1               5                   10                  15
   Leu Val Phe Phe Ala Glu Asp Val Gly Ser Asn Lys Gly Ala Ile Ile
               20                  25                  30
   Gly Leu Met Val Gly Gly Val Val Ile Ala
           35                  40
<210> 968
   <211> 40
   <212> PRT
   <213> Artificial Sequence
<220>
   <223> Description of Artificial Sequence: Synthetic Peptide
<400> 968
   Asp Ala Glu Phe Gly His Asp Ser Gly Phe Glu Val Arg His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val
   35 40
<210> 969
   <211> 11
   <212> PRT
   <213> Artificial Sequence
<220>
   <223> Description of Artificial Sequence: Synthetic Peptide
<400> 969
   Asp Ala Glu Phe Arg His Asp Ser Gly Tyr Glu
   1 5 10
<210> 970
   <211> 5
   <212> PRT
   <213> Artificial Sequence
<220>
   <223> Description of Artificial Sequence: Synthetic Peptide
<400> 970
   Ile Ile Gly Leu Met
   1 5
<210> 971
   <211> 4
   <212> PRT
   <213> Artificial Sequence
<220>
   <223> Description of Artificial Sequence: Synthetic Peptide
<400> 971
   Ile Gly Leu Met
   1
<210> 972
   <211> 11
   <212> PRT
   <213> Artificial Sequence
<220>
   <223> Description of Artificial Sequence: Synthetic Peptide
<400> 972
   Met Leu Gly Ile Ile Ala Gly Lys Asn Ser Gly
   1 5 10
<210> 973
   <211> 15
   <212> PRT
   <213> Artificial Sequence
<220>
   <223> Description of Artificial Sequence: Synthetic Peptide
<400> 973
   Asn Trp Cys Lys Arg Gly Arg Lys Gln Cys Lys Thr His Pro His
   1 5 10 15
<210> 974
   <211> 20
   <212> PRT
   <213> Artificial Sequence
<220>
   <223> Description of Artificial Sequence: Synthetic Peptide
<400> 974
   His His Gly Val Val Glu Val Asp Ala Ala Val Thr Pro Glu Glu Arg
   1 5 10 15
   His Leu Ser Lys
   20
<210> 975
   <211> 38
   <212> PRT
   <213> Artificial Sequence
<220>
   <223> Description of Artificial Sequence: Synthetic Peptide
<400> 975
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met Val Gly Gly
   35
<210> 976
   <211> 28
   <212> PRT
   <213> Artificial Sequence
<220>
   <223> Description of Artificial Sequence: Synthetic Peptide
<400> 976
   Asp Ala Glu Phe Arg His Asp Ser Gly Tyr Gln Val His His Gln Lys
   1 5 10 15
   Leu Val Phe Phe Ala Glu Asp Val Gly Ser Asn Lys
   20 25
<210> 977
   <211> 16
   <212> PRT
   <213> Artificial Sequence
<220>
   <223> Description of Artificial Sequence: Synthetic Peptide
<400> 977
   Asp Ala Glu Phe Arg His Asp Ser Gly Tyr Gln Val His His Gln Lys
   1 5 10 15
<210> 978
   <211> 40
   <212> PRT
   <213> Artificial Sequence
<220>
   <223> Description of Artificial Sequence: Synthetic Peptide
<400> 978
   Asp Ala Glu Phe Arg His Asp Ser Gly Tyr Glu Val His His Gln Lys
   1               5                   10                  15
   Leu Val Phe Phe Ala Glu Asp Val Gly Ser Asn Lys Gly Ala Ile Ile
               20                  25                  30
   Gly Leu Met Val Gly Gly Val Val
           35                  40
<210> 979
   <211> 35
   <212> PRT
   <213> Artificial Sequence
<220>
   <223> Description of Artificial Sequence: Synthetic Peptide
<400> 979
   His Asp Ser Gly Tyr Glu Val His His Gln Lys Leu Val Phe Phe Ala
   1               5                   10                  15
   Gln Asp Val Gly Ser Asn Lys Gly Ala Ile Ile Gly Leu Met Val Gly
               20                  25                  30
   Gly Val Val
           35
<210> 980
   <211> 35
   <212> PRT
   <213> Artificial Sequence
<220>
   <223> Description of Artificial Sequence: Synthetic Peptide
<400> 980
   Glu Gln Val Thr Asn Val Gly Gly Ala Val Val Thr Gly Val Thr Ala
   1               5                   10                  15
   Val Ala Gln Lys Thr Val Glu Gly Ala Gly Ser Ile Ala Ala Ala Thr
               20                  25                  30
   Gly Phe Val
           35
<210> 981
   <211> 24
   <212> PRT
   <213> Artificial Sequence
<220>
   <223> Description of Artificial Sequence: Synthetic Peptide
<400> 981
   Leu Val Phe Phe Ala Glu Asp Val Gly Ser Asn Lys Gly Ala Ile Ile
   1 5 10 15
   Gly Leu Met Val Gly Gly Val Val
   20
<210> 982
   <211> 11
   <212> PRT
   <213> Artificial Sequence
<220>
   <223> Description of Artificial Sequence: Synthetic Peptide
<400> 982
   Gly Tyr Val Ile Ile Lys Pro Leu Val Trp Val
   1 5 10
<210> 983
   <211> 17
   <212> PRT
   <213> Artificial Sequence
<220>
   <223> Description of Artificial Sequence: Synthetic Peptide
<400> 983
   Val His His Gln Lys Leu Val Phe Phe Ala Glu Asp Val Gly Ser Asn
   1 5 10 15
   Lys
<210> 984
   <211> 11
   <212> PRT
   <213> Artificial Sequence
<220>
   <223> Description of Artificial Sequence: Synthetic Peptide
<400> 984
   Gly Ser Asn Lys Gly Ala Ile Ile Gly Leu Met
   1 5 10
<210> 985
   <211> 5
   <212> PRT
   <213> Artificial Sequence
<220>
   <223> Description of Artificial Sequence: Synthetic Peptide
<400> 985
   Arg Glu Arg Met Ser
   1 5
<210> 986
   <211> 17
   <212> PRT
   <213> Artificial Sequence
<220>
   <223> Description of Artificial Sequence: Synthetic Peptide
<400> 986
   Ala Lys Glu Arg Leu Glu Ala Lys His Arg Glu Arg Met Ser Gln Val
   1 5 10 15
   Met
<210> 987
   <211> 43
   <212> PRT
   <213> Artificial Sequence
<220>
   <223> Description of Artificial Sequence: Synthetic Peptide
<400> 987
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val Ile Ala Thr
   35 40
<210> 988
   <211> 42
   <212> PRT
   <213> Artificial Sequence
<220>
   <223> Description of Artificial Sequence: Synthetic Peptide
<400> 988
<210> 989
   <211> 40
   <212> PRT
   <213> Artificial Sequence
<220>
   <223> Description of Artificial Sequence: Synthetic Peptide
<400> 989
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val
   35 40
<210> 990
   <211> 11
   <212> PRT
   <213> Artificial Sequence
<220>
   <223> Description of Artificial Sequence: Synthetic Peptide
<400> 990
   Tyr Glu Val His His Gln Lys Leu Val Phe Phe
   1 5 10
<210> 991
   <211> 14
   <212> PRT
   <213> Artificial Sequence
<220>
   <223> Description of Artificial Sequence: Synthetic Peptide
<400> 991
   Glu Asp Val Gly Ser Asn Lys Gly Ala Ile Ile Gly Leu Met
   1 5 10

<210> 992
   <211> 28
   <212> PRT
   <213> Artificial Sequence
<220>
   <223> Description of Artificial Sequence: Synthetic Peptide
<400> 992
   Asp Ala Glu Phe Arg His Asp Ser Gly Tyr Glu Val His His Gln Lys
   1 5 10 15
   Leu Val Phe Phe Ala Glu Asp Val Gly Ser Asn Lys
   20 25
<210> 993
   <211> 40
   <212> PRT
   <213> Artificial Sequence
<220>
   <223> Description of Artificial Sequence: Synthetic Peptide
<400> 993
   Val Val Gly Gly Val Met Leu Gly Ile Ile Ala Gly Lys Asn Ser Gly
   1 5 10 15
   Val Asp Glu Ala Phe Phe Val Leu Lys Gln His His Val Glu Tyr Gly
   20 25 30
   Ser Asp His Arg Phe Glu Ala Asp
   35 40
<210> 994
   <211> 42
   <212> PRT
   <213> Artificial Sequence
<220>
   <223> Description of Artificial Sequence: Synthetic Peptide
<400> 994
   Asp Ala Glu Phe Gly His Asp Ser Gly Phe Glu Val Arg His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val Ile Ala
   35 40
<210> 995
   <211> 40
   <212> PRT
   <213> Artificial Sequence
<220>
   <223> Description of Artificial Sequence: Synthetic Peptide
<400> 995
   Asp Ala Glu Phe Gly His Asp Ser Gly Phe Glu Val Arg His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val
   35 40
<210> 996
   <211> 11
   <212> PRT
   <213> Artificial Sequence
<220>
   <223> Description of Artificial Sequence: Synthetic Peptide
<400> 996
   Asp Ala Glu Phe Arg His Asp Ser Gly Tyr Glu
   1 5 10
<210> 997
   <211> 5
   <212> PRT
   <213> Artificial Sequence
<220>
   <223> Description of Artificial Sequence: synthetic Peptide
<400> 997
   Ile Ile Gly Leu Met
   1 5
<210> 998
   <211> 4
   <212> PRT
   <213> Artificial Sequence
<220>
   <223> Description of Artificial Sequence: Synthetic Peptide
<400> 998
   Ile Gly Leu Met
   1
<210> 999
   <211> 11
   <212> PRT
   <213> Artificial Sequence
<220>
   <223> Description of Artificial Sequence: Synthetic Peptide
<400> 999
   Met Leu Gly Ile Ile Ala Gly Lys Asn Ser Gly
   1 5 10
<210> 1000
   <211> 15
   <212> PRT
   <213> Artificial Sequence
<220>
   <223> Description of Artificial Sequence: Synthetic Peptide
<400> 1000
   Asn Trp Cys Lys Arg Gly Arg Lys Gln Cys Lys Thr His Pro His
   1 5 10 15
<210> 1001
   <211> 20
   <212> PRT
   <213> Artificial Sequence
<220>
   <223> Description of Artificial Sequence: Synthetic Peptide
<400> 1001
   His His Gly Val Val Glu Val Asp Ala Ala Val Thr Pro Glu Glu Arg
   1 5 10 15
   His Leu Ser Lys
   20
<210> 1002
   <211> 38
   <212> PRT
   <213> Artificial Sequence
<220>
   <223> Description of Artificial Sequence: Synthetic Peptide
<400> 1002
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met Val Gly Gly
   35
<210> 1003
   <211> 28
   <212> PRT
   <213> Artificial Sequence
<220>
   <223> Description of Artificial Sequence: Synthetic Peptide
<400> 1003
   Asp Ala Glu Phe Arg His Asp Ser Gly Tyr Gln Val His His Gln Lys
   1 5 10 15
   Leu Val Phe Phe Ala Glu Asp Val Gly Ser Asn Lys
   20 25
<210> 1004
   <211> 16
   <212> PRT
   <213> Artificial Sequence
<220>
   <223> Description of Artificial Sequence: Synthetic Peptide
<400> 1004
   Asp Ala Glu Phe Arg His Asp Ser Gly Tyr Gln Val His His Gln Lys
   1 5 10 15
<210> 1005
   <211> 40
   <212> PRT
   <213> Artificial Sequence
<220>
   <223> Description of Artificial Sequence: Synthetic Peptide
<400> 1005
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val
   35 40
<210> 1006
   <211> 35
   <212> PRT
   <213> Artificial Sequence
<220>
   <223> Description of Artificial Sequence: Synthetic Peptide
<400> 1006
   His Asp Ser Gly Tyr Glu Val His His Gln Lys Leu Val Phe Phe Ala
   1 5 10 15
   Gln Asp Val Gly Ser Asn Lys Gly Ala Ile Ile Gly Leu Met Val Gly
   20 25 30
   Gly Val Val
   35
<210> 1007
   <211> 35
   <212> PRT
   <213> Artificial Sequence
<220>
   <223> Description of Artificial sequence: Synthetic Peptide
<400> 1007
   Glu Gln Val Thr Asn Val Gly Gly Ala Val Val Thr Gly Val Thr Ala
   1 5 10 15
   Val Ala Gln Lys Thr Val Glu Gly Ala Gly Ser Ile Ala Ala Ala Thr
   20 25 30
   Gly Phe Val
   35
<210> 1008
   <211> 24
   <212> PRT
   <213> Artificial Sequence
<220>
   <223> Description of Artificial Sequence: Synthetic Peptide
<400> 1008
   Leu Val Phe Phe Ala Glu Asp Val Gly Ser Asn Lys Gly Ala Ile Ile
   1 5 10 15
   Gly Leu Met Val Gly Gly Val Val
   20
<210> 1009
   <211> 11
   <212> PRT
   <213> Artificial Sequence
<220>
   <223> Description of Artificial Sequence: Synthetic Peptide
<400> 1009
   Gly Tyr Val Ile Ile Lys Pro Leu Val Trp Val
   1 5 10
<210> 1010
   <211> 63
   <212> PRT
   <213> Artificial Sequence
<220>
   <223> Description of Artificial Sequence: Synthetic Peptide
<400> 1010
   Arg Leu Glu Lys Arg Asp Val Lys Ala Val Cys Ser Gln Glu Ala Met
   1 5 10 15
   Thr Gly Pro Cys Arg Ala Val Met Pro Arg Trp Tyr Phe Asp Leu Ser
   20 25 30
   Lys Gly Lys Cys Val Arg Phe Ile Tyr Gly Gly Cys Gly Gly Asn Arg
   35 40 45
   Asn Asn Phe Glu Ser Glu Asp Tyr Cys Met Ala Val Cys Lys Ala
   50 55 60
<210> 1011
   <211> 39
   <212> PRT
   <213> Artificial Sequence
<220>
   <223> Description of Artificial Sequence: Synthetic Peptide
<400> 1011
   Asn Ser Ser Cys Gly Glu Gly Asn His Leu Pro Thr Thr Pro Cys Tyr
   1 5 10 15
   Leu Gln Trp Gly Thr His Arg Glu Phe Leu Arg Arg Phe Ser Ile Trp
   20 25 30
   Asn His Gly His Leu His Met
   35
<210> 1012
   <211> 39
   <212> PRT
   <213> Artificial Sequence
<220>
   <223> Description of Artificial Sequence: Synthetic Peptide
<400> 1012
   Ala Gly Arg Gly Lys Gln Gly Gly Lys Val Arg Ala Lys Ala Lys Thr
   1 5 10 15
   Arg Ser Ser Arg Ala Gly Leu Gln Phe Pro Val Gly Arg Val His Arg
   20 25 30
   Leu Leu Arg Lys Gly Asn Tyr
   35
<210> 1013
   <211> 21
   <212> PRT
   <213> Artificial Sequence
<220>
   <223> Description of Artificial Sequence: Synthetic Peptide
<400> 1013
   Thr Arg Ser Ser Arg Ala Gly Leu Gln Phe Pro Val Gly Arg Val His
   1 5 10 15
   Arg Leu Leu Arg Lys
   20
<210> 1014
   <211> 37
   <212> PRT
   <213> Artificial Sequence
<220>
   <223> Description of Artificial Sequence: Synthetic Peptide
<400> 1014
   Lys Trp Lys Leu Phe Lys Lys Ile Glu Lys Val Gly Gln Asn Ile Arg
   1 5 10 15
   Asp Gly Ile Ile Lys Ala Gly Pro Ala Val Ala Val Val Gly Gln Ala
   20 25 30
   Thr Gln Ile Ala Lys
   35
<210> 1015
   <211> 32
   <212> PRT
   <213> Artificial Sequence
<220>
   <223> Description of Artificial Sequence: Synthetic Peptide
<400> 1015
   Ser Trp Leu Ser Lys Thr Ala Lys Lys Leu Glu Asn Ser Ala Lys Lys
   1 5 10 15
   Arg Ile Ser Glu Gly Ile Ala Ile Ala Ile Gln Gly Gly Pro Arg Cys
   20 25 30
<210> 1016
   <211> 31
   <212> PRT
   <213> Artificial Sequence
<220>
   <223> Description of Artificial Sequence: Synthetic Peptide
<400> 1016
   Gly Ile Met Ser Ile Val Lys Asp Val Ala Lys Asn Ala Ala Lys Gly
   1 5 10 15
   Ala Leu Ser Thr Leu Ser Thr Leu Ser Cys Lys Leu Ala Lys Thr
   20 25 30
<210> 1017
   <211> 24
   <212> PRT
   <213> Artificial Sequence
<220>
   <223> Description of Artificial Sequence: Synthetic Peptide
<400> 1017
   Phe Leu Gly Ala Leu Phe Lys Val Ala Ser Lys Val Leu Pro Ser Val
   1 5 10 15
   Lys Cys Ala Ile Thr Lys Lys Cys
   20
<210> 1018
   <211> 13
   <212> PRT
   <213> Artificial Sequence
<220>
   <223> Description of Artificial Sequence: Synthetic Peptide
<400> 1018
   Ile Leu Pro Trp Lys Trp Pro Trp Trp Pro Trp Arg Arg
   1 5 10
<210> 1019
   <211> 18
   <212> PRT
   <213> Artificial Sequence
<220>
   <223> Description of Artificial Sequence: Synthetic Peptide
<400> 1019
   Arg Gly Gly Arg Leu Cys Tyr Cys Arg Arg Arg Phe Cys Val Cys Val
   1 5 10 15
   Gly Arg
<210> 1020
   <211> 18
   <212> PRT
   <213> Artificial Sequence
<220>
   <223> Description of Artificial Sequence: Synthetic Peptide
<400> 1020
   Arg Arg Trp Cys Tyr Arg Lys Cys Tyr Lys Gly Tyr Cys Tyr Arg Lys
   1 5 10 15
   Cys Arg
<210> 1021
   <211> 35
   <212> PRT
   <213> Artificial Sequence
<220>
   <223> Description of Artificial Sequence: Synthetic Peptide
<400> 1021
   Arg Trp Lys Ile Phe Lys Lys Ile Glu Lys Val Gly Gln Asn Ile Arg
   1 5 10 15
   Asp Gly Ile Val Lys Ala Gly Pro Ala Val Ala Val Val Gly Gln Ala
   20 25 30
   Ala Thr Ile
   35
<210> 1022
   <211> 12
   <212> PRT
   <213> Artificial Sequence
<220>
   <223> Description of Artificial Sequence: Synthetic Peptide
<400> 1022
   Arg Leu Cys Arg Ile Val Val Ile Arg Val Cys Arg
   1 5 10
<210> 1023
   <211> 21
   <212> PRT
   <213> Artificial Sequence
<220>
   <223> Description of Artificial Sequence: Synthetic Peptide
<400> 1023
   Thr Arg Ser Ser Arg Ala Gly Leu Gln Phe Pro Val Gly Arg Val His
   1 5 10 15
   Arg Leu Leu Arg Lys
   20
<210> 1024
   <211> 14
   <212> PRT
   <213> Artificial Sequence
<220>
   <223> Description of Artificial Sequence: Synthetic Peptide
<400> 1024
   Tyr Pro Pro Gly Pro Leu Ala Pro Pro Gln Pro Phe Gly Pro
   1 5 10
<210> 1025
   <211> 22
   <212> PRT
   <213> Artificial Sequence
<220>
   <223> Description of Artificial Sequence: Synthetic Peptide
<400> 1025
<210> 1026
   <211> 15
   <212> PRT
   <213> Artificial Sequence
<220>
   <223> Description of Artificial Sequence: Synthetic Peptide
<400> 1026
   Ala Asp Val Leu Thr Thr Gly Ala Gly Asn Pro Val Gly Asp Lys
   1 5 10 15
<210> 1027
   <211> 42
   <212> PRT
   <213> Artificial Sequence
<220>
   <223> Description of Artificial Sequence: Synthetic Peptide
<400> 1027
   Met Ala Ala Val Ser Met Ser Val Ala Leu Arg Gln Ala Leu Trp Gly
   1 5 10 15
   Arg Arg Val Ala Thr Val Ala Ala Val Ser Val Ser Lys Val Ser Thr
   20 25 30
   Arg Ser Leu Ser Thr Ser Thr Trp Arg Leu
   35 40
<210> 1028
   <211> 23
   <212> PRT
   <213> Artificial Sequence
<220>
   <223> Description of Artificial Sequence: Synthetic Peptide
<400> 1028
   Gly Ile Gly Lys Phe Lys His Ser Ala Gly Lys Phe Gly Lys Ala Phe
   1 5 10 15
   Val Gly Glu Ile Met Lys Ser
   20
<210> 1029
   <211> 29
   <212> PRT
   <213> Artificial Sequence
<220>
   <223> Description of Artificial Sequence: Synthetic Peptide
<400> 1029
   Leu Arg Asp Leu Val Ser Tyr Cys Arg Ala Arg Gly Lys Gly Arg Glu
   1 5 10 15
   Arg Met Asn Gly Thr Arg Lys Gly His Leu Leu Tyr Met
   20 25
<210> 1030
   <211> 4
   <212> PRT
   <213> Artificial Sequence
<220>
   <223> Description of Artificial Sequence: synthetic Peptide.
<400> 1030
   Ser Gln Asn Tyr
   1
<210> 1031
   <211> 12
   <212> PRT
   <213> Artificial Sequence
<220>
   <223> Description of Artificial sequence: Synthetic Peptide
<400> 1031
   Arg Leu Cys Arg Ile Val Val Ile Arg Val Cys Arg
   1 5 10
<210> 1032
   <211> 25
   <212> PRT
   <213> Artificial Sequence
<220>
   <223> Description of Artificial Sequence: Synthetic Peptide
<400> 1032
   Asn Gln Gly Arg His Phe Cys Gly Gly Ala Leu Ile His Ala Arg Phe
   1 5 10 15
   Val Met Thr Ala Ala Ser Cys Phe Gln
   20 25
<210> 1033
   <211> 2
   <212> PRT
   <213> Artificial Sequence
<220>
   <223> Description of Artificial Sequence: Synthetic Peptide
<400> 1033
   Pro Phe
   1
<210> 1034
   <211> 18
   <212> PRT
   <213> Artificial Sequence
<220>
   <223> Description of Artificial Sequence: Synthetic Peptide
<400> 1034
   Cys Asn Leu Ala Val Ala Ala Ala Ser His Ile Tyr Gln Asn Gln Phe
   1 5 10 15
   Val Gln
<210> 1035
   <211> 19
   <212> PRT
   <213> Artificial Sequence
<220>
   <223> Description of Artificial Sequence: Synthetic Peptide
<400> 1035
   Tyr Arg Val Arg Phe Leu Ala Lys Glu Asn Val Thr Gln Asp Ala Glu
   1 5 10 15
   Asp Asn Cys
<210> 1036
   <211> 15
   <212> PRT
   <213> Artificial Sequence
<220>
   <223> Description of Artificial Sequence: Synthetic Peptide
<400> 1036
   Lys Trp Lys Leu Phe Lys Lys Ile Gly Ala Val Leu Lys Val Leu
   1 5 10 15
<210> 1037
   <211> 35
   <212> PRT
   <213> Artificial Sequence
<220>
   <223> Description of Artificial Sequence: Synthetic Peptide
<400> 1037
   Lys Trp Lys Val Phe Lys Lys Ile Glu Lys Met Gly Arg Asn Ile Arg
   1 5 10 15
   Asn Gly Ile Val Lys Ala Gly Pro Ala Ile Ala Val Leu Gly Glu Ala
   20 25 30
   Lys Ala Leu
   35
<210> 1038
   <211> 12
   <212> PRT
   <213> Artificial Sequence
<220>
   <223> Description of Artificial Sequence: Synthetic Peptide
<400> 1038
   Thr Tyr Ile Cys Glu Val Glu Asp Gln Lys Glu Glu
   1 5 10
<210> 1039
   <211> 29
   <212> PRT
   <213> Artificial Sequence
<220>
   <223> Description of Artificial Sequence: Synthetic Peptide
<400> 1039
   Cys Tyr Cys Arg Ile Pro Ala Cys Ile Ala Gly Glu Arg Arg Tyr Gly
   1 5 10 15
   Thr Cys Ile Tyr Gln Gly Arg Leu Trp Ala Phe Cys Cys
   20 25
<210> 1040
   <211> 34
   <212> PRT
   <213> Artificial Sequence
<220>
   <223> Description of Artificial Sequence: Synthetic Peptide
<400> 1040
   Ala Leu Trp Lys Thr Met Leu Lys Lys Leu Gly Thr Met Ala Leu His
   1 5 10 15
   Ala Gly Lys Ala Ala Leu Gly Ala Ala Ala Asp Thr Ile Ser Gln Gly
   20 25 30
   Thr Gln
<210> 1041
   <211> 24
   <212> PRT
   <213> Artificial Sequence
<220>
   <223> Description of Artificial Sequence: Synthetic Peptide
<400> 1041
   Gly Val Leu Ser Asn Val Ile Gly Tyr Leu Lys Lys Leu Gly Thr Gly
   1 5 10 15
   Ala Leu Asn Ala Val Leu Lys Gln
   20
<210> 1042
   <211> 6
   <212> PRT
   <213> Artificial Sequence
<220>
   <223> Description of Artificial Sequence: Synthetic peptide
<400> 1042
   Val Glu Pro Ile Pro Tyr
   1 5
<210> 1043
   <211> 11
   <212> PRT
   <213> Artificial Sequence
<220>
   <223> Description of Artificial Sequence: Synthetic Peptide
<400> 1043
   Arg Arg Trp Gln Trp Arg Met Lys Lys Leu Gly
   1 5 10
<210> 1044
   <211> 21
   <212> PRT
   <213> Artificial Sequence
<220>
   <223> Description of Artificial Sequence: Synthetic Peptide
<400> 1044
   Asp Ala Glu Ala Val Gly Pro Glu Ala Phe Ala Asp Gln Asp Leu Asp
   1 5 10 15
   Glu Arg Glu Val Arg
   20
<210> 1045
   <211> 7
   <212> PRT
   <213> Artificial Sequence
<220>
   <223> Description of Artificial Sequence: Synthetic Peptide
<400> 1045
   Ser Ile Gly Ser Leu Ala Lys
   1 5
<210> 1046
   <211> 23
   <212> PRT
   <213> Artificial Sequence
<220>
   <223> Description of Artificial Sequence: Synthetic Peptide
<400> 1046
   Gly Ile Gly Lys Phe Leu His Ser Ala Gly Lys Phe Gly Lys Ala Phe
   1 5 10 15
   Val Gly Glu Ile Met Lys Ser
   20
<210> 1047
   <211> 23
   <212> PRT
   <213> Artificial Sequence
<220>
   <223> Description of Artificial Sequence: Synthetic Peptide
<400> 1047
   Gly Ile Gly Lys Phe Leu His Ser Ala Lys Lys Phe Gly Lys Ala Phe
   1 5 10 15
   Val Gly Glu Ile Met Asn Ser
   20
<210> 1048
   <211> 27
   <212> PRT
   <213> Artificial Sequence
<220>
   <223> Description of Artificial Sequence: Synthetic Peptide
<400> 1048
   Tyr Leu Val Leu Phe Phe Tyr Pro Leu Asp Phe Thr Phe Val Cys Pro
   1 5 10 15
   Thr Glu Ile Ile Cys Pro Thr Glu Ile Ile Gly
   20 25
<210> 1049
   <211> 15
   <212> PRT
   <213> Artificial Sequence
<220>
   <223> Description of Artificial Sequence: Synthetic Peptide
<400> 1049
<210> 1050
   <211> 19
   <212> PRT
   <213> Artificial Sequence
<220>
   <223> Description of Artificial Sequence: Synthetic Peptide
<400> 1050
<210> 1051
   <211> 4
   <212> PRT
   <213> Artificial Sequence
<220>
   <223> Description of Artificial Sequence: Synthetic Peptide
<400> 1051
   Asp Glu Val Asp
   1
<210> 1052
   <211> 4
   <212> PRT
   <213> Artificial Sequence
<220>
   <223> Description of Artificial Sequence: Synthetic Peptide
<400> 1052
   Asp Glu Val Asp
   1
<210> 1053
   <211> 3
   <212> PRT
   <213> Artificial Sequence
<220>
   <223> Description of Artificial Sequence: Synthetic Peptide
<400> 1053
   Val Ala Asp
   1
<210> 1054
   <211> 3
   <212> PRT
   <213> Artificial Sequence
<220>
   <223> Description of Artificial Sequence: Synthetic Peptide
<400> 1054
   Val Ala Asp
   1
<210> 1055
   <211> 27
   <212> PRT
   <213> Artificial Sequence
<220>
   <223> Description of Artificial Sequence: Synthetic Peptide
<400> 1055
   Asp Pro Met Ser Ser Thr Tyr Ile Glu Glu Leu Gly Lys Arg Glu Val
   1 5 10 15
   Thr Ile Pro Pro Lys Tyr Arg Glu Leu Leu Ala
   20 25
<210> 1056
   <211> 4
   <212> PRT
   <213> Artificial Sequence
<220>
   <223> Description of Artificial Sequence: Synthetic Peptide
<400> 1056
   Val Ala Asp Met
   1
<210> 1057
   <211> 4
   <212> PRT
   <213> Artificial Sequence
<220>
   <223> Description of Artificial Sequence: Synthetic Peptide
<400> 1057
   Asp Glu Val Asp
   1
<210> 1058
   <211> 20
   <212> PRT
   <213> Artificial Sequence
<220>
   <223> Description of Artificial Sequence: Synthetic Peptide
<400> 1058
   Ala Ala Val Ala Leu Leu Pro Ala Val Leu Leu Ala Leu Leu Ala Pro
   1 5 10 15
   Tyr Val Ala Asp
   20
<210> 1059
   <211> 7
   <212> PRT
   <213> Artificial Sequence
<220>
   <223> Description of Artificial Sequence: Synthetic Peptide
<400> 1059
   Tyr Val Ala Asp Ala Pro Lys
   1 5
<210> 1060
   <211> 20
   <212> PRT
   <213> Artificial Sequence
<220>
   <223> Description of Artificial Sequence: Synthetic Peptide
<400> 1060
   Ala Ala Val Ala Leu Leu Pro Ala Val Leu Leu Ala Leu Leu Ala Pro
   1 5 10 15
   Asp Glu Val Asp
   20
<210> 1061
   <211> 4
   <212> PRT
   <213> Artificial Sequence
<220>
   <223> Description of Artificial Sequence: Synthetic Peptide
<400> 1061
   Tyr Val Ala Asp
   1
<210> 1062
   <211> 4
   <212> PRT
   <213> Artificial Sequence
<220>
   <223> Description of Artificial Sequence: Synthetic Peptide
<400> 1062
   Tyr Val Ala Asp
   1
<210> 1063
   <211> 4
   <212> PRT
   <213> Artificial Sequence
<220>
   <223> Description of Artificial Sequence: Synthetic Peptide
<400> 1063
   Tyr Val Ala Asp
   1
<210> 1064
   <211> 2
   <212> PRT
   <213> Artificial Sequence
<220>
   <223> Description of Artificial Sequence: Synthetic Peptide
<400> 1064
   Val Phe
   1

<210> 1065
   <211> 3
   <212> PRT
   <213> Artificial Sequence
<220>
   <223> Description of Artificial Sequence: Synthetic Peptide
<400> 1065
   Leu Leu Leu
   1
<210> 1066
   <211> 4
   <212> PRT
   <213> Artificial Sequence
<220>
   <223> Description of Artificial Sequence: Synthetic Peptide
<400> 1066
   Tyr Val Ala Asp
   1
<210> 1067
   <211> 4
   <212> PRT
   <213> Artificial Sequence
<220>
   <223> Description of Artificial Sequence: Synthetic Peptide
<400> 1067
   Val Glu Ile Asp
   1
<210> 1068
   <211> 1
   <212> PRT
   <213> Artificial Sequence
<220>
   <223> Description of Artificial Sequence: Synthetic Peptide
<400> 1068
   Asp
   1
<210> 1069
   <211> 7
   <212> PRT
   <213> Artificial Sequence
<220>
   <223> Description of Artificial Sequence: Synthetic Peptide
<400> 1069
   Asp Glu Val Asp Ala Pro Lys
   1 5
<210> 1070
   <211> 21
   <212> PRT
   <213> Artificial Sequence
<220>
   <223> Description of Artificial Sequence: Synthetic Peptide
<400> 1070
<210> 1071
   <211> 8
   <212> PRT
   <213> Artificial Sequence
<220>
   <223> Description of Artificial Sequence: Synthetic Peptide
<400> 1071
   Ala Asn Arg Leu Phe Gly Glu Lys
   1 5
<210> 1072
   <211> 19
   <212> PRT
   <213> Artificial Sequence
<220>
   <223> Description of Artificial Sequence: Synthetic Peptide
<400> 1072
   Gly Gly Gly Gly Asp Ile His Gln Gly Phe Gln Ser Leu Leu Thr Glu
   1 5 10 15
   Val Asn Lys
<210> 1073
   <211> 14
   <212> PRT
   <213> Artificial Sequence
<220>
   <223> Description of Artificial Sequence: Synthetic Peptide
<400> 1073
   Gly Asn Thr Ala Ala Gln Met Ala Gln Ile Leu Ser Phe Asn
   1 5 10
<210> 1074
   <211> 25
   <212> PRT
   <213> Artificial Sequence
<220>
   <223> Description of Artificial Sequence: Synthetic Peptide
<400> 1074
<210> 1075
   <211> 35
   <212> PRT
   <213> Artificial Sequence
<220>
   <223> Description of Artificial Sequence: Synthetic Peptide
<400> 1075
   Thr Gly His Gly Gly Pro Gln Phe Val Ala Asp His Pro Phe Leu Phe
   1 5 10 15
   Leu Ile Met His Lys Ile Thr Asn Cys Ile Leu Phe Phe Gly Arg Phe
   20 25 30
   Ser Ser Pro
   35
<210> 1076
   <211> 9
   <212> PRT
   <213> Artificial Sequence
<220>
   <223> Description of Artificial Sequence: Synthetic Peptide
<400> 1076
   Ala Pro Arg Leu Arg Phe Tyr Ser Leu
   1 5
<210> 1077
   <211> 8
   <212> PRT
   <213> Artificial Sequence
<220>
   <223> Description of Artificial Sequence: Synthetic Peptide
<400> 1077
   Ala Pro Arg Leu Arg Phe Tyr Ser
   1 5
<210> 1078
   <211> 7
   <212> PRT
   <213> Artificial Sequence
<220>
   <223> Description of Artificial Sequence: Synthetic Peptide
<400> 1078
   Ala Pro Arg Leu Arg Phe Tyr
   1 5
<210> 1079
   <211> 5
   <212> PRT
   <213> Artificial Sequence
<220>
   <223> Description of Artificial Sequence: Synthetic Peptide
<400> 1079
   Arg Leu Arg Phe His
   1 5
<210> 1080
   <211> 5
   <212> PRT
   <213> Artificial Sequence
<220>
   <223> Description of Artificial Sequence: Synthetic Peptide
<400> 1080
   Arg Leu Arg Phe Asp
   1 5
<210> 1081
   <211> 23
   <212> PRT
   <213> Artificial Sequence
<220>
   <223> Description of Artificial Sequence: Synthetic Peptide
<400> 1081
   Leu Lys Lys Tyr Lys Val Pro Gln Leu Glu Ile Val Pro Asn Ser Ala
   1 5 10 15
   Glu Glu Arg Leu His Ser Met
   20
<210> 1082
   <211> 13
   <212> PRT
   <213> Artificial Sequence
<220>
   <223> Description of Artificial Sequence: Synthetic Peptide
<400> 1082
   Gln Lys Leu Gly Asn Gln Trp Ala Val Gly His Leu Met
   1 5 10
<210> 1083
   <211> 7
   <212> PRT
   <213> Artificial Sequence
<220>
   <223> Description of Artificial Sequence: Synthetic Peptide
<400> 1083
   Trp Ala Val Gly His Leu Met
   1 5
<210> 1084
   <211> 14
   <212> PRT
   <213> Artificial Sequence
<220>
   <223> Description of Artificial Sequence: Synthetic Peptide
<400> 1084
   Glu Gln Arg Leu Gly Asn Gln Trp Ala Val Gly His Leu Met
   1 5 10
<210> 1085
   <211> 14
   <212> PRT
   <213> Artificial Sequence
<220>
   <223> Description of Artificial Sequence: Synthetic Peptide
<400> 1085
   Glu Gln Arg Leu Gly Asn Gln Trp Ala Val Gly His Leu Leu
   1 5 10
<210> 1086
   <211> 8
   <212> PRT
   <213> Artificial Sequence
<220>
   <223> Description of Artificial Sequence: Synthetic Peptide
<400> 1086
   Phe Gln Trp Ala Val Gly His Leu
   1 5
<210> 1087
   <211> 13
   <212> PRT
   <213> Artificial Sequence
<220>
   <223> Description of Artificial Sequence: Synthetic Peptide
<400> 1087
   Gln Arg Leu Gly Asn Gln Trp Ala Val Gly Phe Leu Met
   1 5 10
<210> 1088
   <211> 13
   <212> PRT
   <213> Artificial Sequence
<220>
   <223> Description of Artificial Sequence: Synthetic Peptide
<400> 1088
   Gln Arg Leu Gly Asn Gln Trp Ala Val Gly Phe Leu Leu
   1 5 10
<210> 1089
   <211> 13
   <212> PRT
   <213> Artificial Sequence
<220>
   <223> Description of Artificial Sequence: Synthetic Peptide
<400> 1089
   Gln Arg Tyr Gly Asn Gln Trp Ala Val Gly His Leu Met
   1 5 10
<210> 1090
   <211> 13
   <212> PRT
   <213> Artificial Sequence
<220>
   <223> Description of Artificial Sequence: Synthetic Peptide
<400> 1090
   Gln Arg Tyr Gly Asn Gln Trp Ala Val Gly Phe Leu Met
   1 5 10
<210> 1091
   <211> 21
   <212> PRT
   <213> Artificial Sequence
<220>
   <223> Description of Artificial Sequence: Synthetic Peptide
<400> 1091
   Met Pro Leu Pro Pro His Pro Gly His Pro Gly Tyr Ile Asn Phe Ser
   1 5 10 15
   Tyr Glu Val Leu Thr
   20
<210> 1092
   <211> 6
   <212> PRT
   <213> Artificial Sequence
<220>
   <223> Description of Artificial Sequence: Synthetic Peptide
   <400> 1092
   Pro Phe Tyr Gly Pro Val
   1 5
   <210> 1093
   <211> 47
   <212> PRT
   <213> Artificial Sequence
<220>
   <223> Description of Artificial Sequence: Synthetic Peptide
<400> 1093
   Tyr Leu Tyr Gln Trp Leu Gly Ala Pro Val Pro Tyr Pro Asp Pro Leu
   1 5 10 15
   Glu Pro Arg Arg Val Cys Leu Asn Pro Asp Cys Asp Glu Leu Ala Asp
   20 25 30
   His Ile Gly Phe Gln Glu Ala Tyr Arg Arg Phe Tyr Gly Pro Val
   35 40 45
<210> 1094
   <211> 6
   <212> PRT
   <213> Artificial Sequence
<220>
   <223> Description of Artificial Sequence: Synthetic Peptide
   <400> 1094
   Leu Val Val Tyr Pro Trp
   1 5
   <210> 1095
   <211> 46
   <212> PRT
   <213> Artificial Sequence
<220>
   <223> Description of Artificial Sequence: Synthetic Peptide
<400> 1095
   Tyr Leu Tyr Gln Trp Leu Gly Ala Pro Val Pro Tyr Pro Asp Pro Leu
   1 5 10 15
   Pro Arg Arg Val Cys Leu Asn Pro Asp Cys Asp Glu Leu Ala Asp His
   20 25 30
   Ile Gly Phe Gln Glu Ala Tyr Arg Arg Phe Tyr Gly Pro Val
   35 40 45
<210> 1096
   <211> 13
   <212> PRT
   <213> Artificial Sequence
<220>
   <223> Description of Artificial Sequence: Synthetic Peptide
<400> 1096
   Gly Phe Gln Glu Ala Tyr Arg Arg Phe Tyr Gly Pro Val
   1 5 10
<210> 1097
   <211> 13
   <212> PRT
   <213> Artificial Sequence
<220>
   <223> Description of Artificial Sequence: Synthetic Peptide
<400> 1097
   Pro Tyr Gln Glu Ala Phe Arg Arg Phe Phe Gly Pro Val
   1 5 10
<210> 1098
   <211> 48
   <212> PRT
   <213> Artificial Sequence
<220>
   <223> Description of Artificial Sequence: Synthetic Peptide
<400> 1098
   Val Pro Ile Tyr Glu Lys Lys Tyr Gly Gln Val Pro Met Cys Asp Ala
   1 5 10 15
   Gly Glu Gln Cys Ala Val Arg Lys Gly Ala Arg Ile Gly Lys Leu Cys
   20 25 30
   Asp Cys Pro Arg Gly Thr Ser Cys Asn Ser Phe Leu Leu Lys Cys Leu
   35 40 45
<210> 1099
   <211> 48
   <212> PRT
   <213> Artificial Sequence
<220>
   <223> Description of Artificial Sequence: Synthetic Peptide
<400> 1099
   Val Pro Ile Tyr Glu Lys Lys Tyr Gly Gln Val Pro Met Cys Asp Ala
   1 5 10 15
   Gly Glu Gln Cys Ala Val Arg Lys Gly Ala Arg Ile Gly Lys Leu Cys
   20 25 30
   Asp Cys Pro Arg Gly Thr Ser Cys Asn Ser Phe Leu Leu Lys Cys Leu
   35 40 45
<210> 1100
   <211> 48
   <212> PRT
   <213> Artificial Sequence
<220>
   <223> Description of Artificial Sequence: Synthetic Peptide
<400> 1100
   Val Pro Ile Tyr Glu Lys Lys Tyr Gly Gln Val Pro Met Cys Asp Ala
   1 5 10 15
   Gly Glu Gln Cys Ala Val Arg Lys Gly Ala Arg Ile Gly Lys Leu Cys
   20 25 30
   Asp Cys Pro Arg Gly Thr Ser Cys Asn Ser Phe Leu Leu Lys Cys Leu
   35 40 45
<210> 1101
   <211> 28
   <212> PRT
   <213> Artificial Sequence
<220>
   <223> Description of Artificial Sequence: Synthetic Peptide
<400> 1101
   Ala Gly Ala Thr Val Gln Val Thr Leu Asp Gly Val Pro Ala Ala Ala
   1 5 10 15
   Pro Gly Gln Pro Ala Gln Leu Gln Leu Asn Ala Thr
   20 25
<210> 1102
   <211> 5
   <212> PRT
   <213> Artificial Sequence
<220>
   <223> Description of Artificial Sequence: Synthetic Peptide
<400> 1102
   Phe Leu Phe Leu Phe
   1 5
<210> 1103
   <211> 30
   <212> PRT
   <213> Artificial Sequence
<220>
   <223> Description of Artificial Sequence: Synthetic Peptide
<400> 1103
   Ala Cys Tyr Cys Arg Ile Pro Ala Cys Ile Ala Gly Glu Arg Arg Tyr
   1 5 10 15
   Gly Thr Cys Ile Tyr Gln Gly Arg Leu Trp Ala Phe Cys Cys
   20 25 30
<210> 1104
   <211> 3
   <212> PRT
   <213> Artificial Sequence
<220>
   <223> Description of Artificial Sequence: Synthetic Peptide
<400> 1104
   Met Leu Phe
   1
<210> 1105
   <211> 4
   <212> PRT
   <213> Artificial Sequence
<220>
   <223> Description of Artificial Sequence: Synthetic Peptide
<400> 1105
   Met Leu Phe Lys
   1
<210> 1106
   <211> 4
   <212> PRT
   <213> Artificial Sequence
<220>
   <223> Description of Artificial Sequence: Synthetic Peptide
<400> 1106
   Met Leu Phe Lys
   1
<210> 1107
   <211> 6
   <212> PRT
   <213> Artificial Sequence
<220>
   <223> Description of Artificial Sequence: Synthetic Peptide
<400> 1107
<210> 1108
   <211> 25
   <212> PRT
   <213> Artificial Sequence
<220>
   <223> Description of Artificial Sequence: Synthetic Peptide
<400> 1108
   Met Leu Thr Glu Leu Glu Lys Ala Leu Asn Ser Ile Ile Asp Val Tyr
   1 5 10 15
   His Lys Tyr Ser Leu Ile Lys Gly Asn
   20 25
<210> 1109
   <211> 19
   <212> PRT
   <213> Artificial Sequence
<220>
   <223> Description of Artificial Sequence: Synthetic Peptide
<400> 1109
   His Trp Asp Thr Thr Gln Ser Leu Lys Gln Leu Glu Glu Arg Ala Ala
   1 5 10 15
   Trp Asn Val
<210> 1110
   <211> 20
   <212> PRT
   <213> Artificial.Sequence
<220>
   <223> Description of Artificial Sequence: Synthetic Peptide
<400> 1110
   Gly Pro Val Ser Ala Val Leu Thr Glu Leu Arg Cys Thr Cys Leu Arg
   1 5 10 15
   Val Thr Leu Arg
   20
<210> 1111
   <211> 21
   <212> PRT
   <213> Artificial Sequence
<220>
   <223> Description of Artificial Sequence: Synthetic Peptide
<400> 1111
   Ile Gln Arg Thr Pro Lys Ile Gln Val Tyr Ser Arg His Pro Ala Glu
   1 5 10 15
   Asn Gly Lys Ser Asn
   20
<210> 1112
   <211> 11
   <212> PRT
   <213> Artificial Sequence
<220>
   <223> Description of Artificial Sequence: Synthetic Peptide
<400> 1112
<210> 1113
   <211> 19
   <212> PRT
   <213> Artificial Sequence
<220>
   <223> Description of Artificial Sequence: Synthetic Peptide
<400> 1113
   Tyr Ser Asp Asp Glu Gly Ala Ser Trp Ser Asp Leu Asp Ile Val Ser
   1 5 10 15
   Phe Ser Lys
<210> 1114
   <211> 18
   <212> PRT
   <213> Artificial Sequence
<220>
   <223> Description of Artificial Sequence: Synthetic Peptide
<400> 1114
   Val Ala Arg Thr Leu Leu Val Phe Glu Val Gln Gln Pro Phe Leu Phe
   1 5 10 15
   Val Leu
<210> 1115
   <211> 14
   <212> PRT
   <213> Artificial Sequence
<220>
   <223> Description of Artificial Sequence: Synthetic Peptide
<400> 1115
<210> 1116
   <211> 10
   <212> PRT
   <213> Artificial Sequence
<220>
   <223> Description of Artificial Sequence: Synthetic Peptide
<400> 1116
   Cys Asn Thr Gly Gln Leu Cys Pro Val Glu
   1 5 10
<210> 1117
   <211> 18
   <212> PRT
   <213> Artificial Sequence
<220>
   <223> Description of Artificial Sequence: Synthetic Peptide
<400> 1117
<210> 1118
   <211> 19
   <212> PRT
   <213> Artificial Sequence
<220>
   <223> Description of Artificial Sequence: Synthetic Peptide
<400> 1118
   Leu His Glu Trp Thr Lys Pro Glu Asn Leu Asp Phe Ile Glu Val Asn
   1 5 10 15
   Val Leu Pro
<210> 1119
   <211> 29
   <212> PRT
   <213> Artificial Sequence
<220>
   <223> Description of Artificial Sequence: Synthetic Peptide
<400> 1119
   Val Leu Glu Leu Pro Tyr Gln Gly Glu Glu Leu Ser Met Val Ile Leu
   1 5 10 15
   Leu Pro Asp Asp Ile Glu Asp Glu Ser Thr Gly Leu Lys
   20 25
<210> 1120
   <211> 19
   <212> PRT
   <213> Artificial Sequence
<220>
   <223> Description of Artificial Sequence: Synthetic Peptide
<400> 1120
   Thr Tyr Gly Ala Asp Leu Ala Ser Val Asp Phe Gln His Ala Ser Glu
   1 5 10 15
   Asp Ala Arg
<210> 1121
   <211> 12
   <212> PRT
   <213> Artificial Sequence
<220>
   <223> Description of Artificial Sequence: Synthetic Peptide
<400> 1121
   Glu Arg Pro Pro Leu Gln Gln Pro Pro His Arg Asp
   1 5 10
<210> 1122
   <211> 29
   <212> PRT
   <213> Artificial Sequence
<220>
   <223> Description of Artificial Sequence: Synthetic Peptide
<400> 1122
   Tyr Glu Arg Pro Pro Leu Gln Gln Pro Pro His Arg Asp Lys Lys Pro
   1 5 10 15
   Cys Lys Asn Phe Phe Trp Lys Thr Phe Ser Ser Cys Lys
   20 25
<210> 1123
   <211> 20
   <212> PRT
   <213> Artificial Sequence
<220>
   <223> Description of Artificial Sequence: Synthetic Peptide
<400> 1123
   Ser Ala Leu Pro Leu Glu Ser Gly Pro Thr Gly Gln Asp Ser Val Gln
   1 5 10 15
   Asp Ala Thr Gly
   20
<210> 1124
   <211> 31
   <212> PRT
   <213> Artificial Sequence
<220>
   <223> Description of Artificial Sequence: Synthetic Peptide
<400> 1124
   Thr Gly Leu Leu Thr Phe Leu Ala Trp Trp His Glu Trp Ala Ser Gln
   1 5 10 15
   Asp Ser Ser Ser Thr Ala Phe Glu Gly Gly Thr Pro Glu Leu Ser
   20 25 30
<210> 1125
   <211> 4
   <212> PRT
   <213> Artificial Sequence
<220>
   <223> Description of Artificial Sequence: Synthetic Peptide
<400> 1125
   Arg Gly Asp Cys
   1
<210> 1126
   <211> 4
   <212> PRT
   <213> Artificial Sequence
<220>
   <223> Description of Artificial Sequence: Synthetic Peptide
<400> 1126
   Arg Gly Asp Ser
   1
<210> 1127
   <211> 4
   <212> PRT
   <213> Artificial Sequence
<220>
   <223> Description of Artificial Sequence: Synthetic Peptide
<400> 1127
   Arg Gly Asp Val
   1
<210> 1128
   <211> 4
   <212> PRT
   <213> Artificial Sequence
<220>
   <223> Description of Artificial Sequence: Synthetic Peptide
<400> 1128
   Arg Gly Glu Ser
   1
<210> 1129
   <211> 7
   <212> PRT
   <213> Artificial Sequence
<220>
   <223> Description of Artificial Sequence: Synthetic Peptide
<400> 1129
   Gly Arg Gly Asp Ser Pro Ala
   1 5
<210> 1130
   <211> 5
   <212> PRT
   <213> Artificial Sequence
<220>
   <223> Description of Artificial Sequence: Synthetic Peptide
<400> 1130
   Arg Gly Asp Phe Val
   1 5
<210> 1131
   <211> 12
   <212> PRT
   <213> Artificial Sequence
<220>
   <223> Description of Artificial Sequence: Synthetic Peptide
<400> 1131
   Pro Leu Tyr Lys Lys Ile Ile Lys Lys Leu Leu Ser
   1 5 10
<210> 1132
   <211> 49
   <212> PRT
   <213> Artificial Sequence
<220>
   <223> Description of Artificial Sequence: Synthetic Peptide
<400> 1132
   Glu Cys Glu Ser Gly Pro Cys Cys Arg Asn Cys Lys Phe Leu Lys Glu
   1 5 10 15
   Gly Thr Ile Cys Lys Arg Ala Arg Gly Asp Asp Met Asp Asp Tyr Cys
   20 25 30
   Asn Gly Lys Thr Cys Asp Cys Pro Arg Asn Pro His Lys Gly Pro Ala
   35 40 45
   Thr
<210> 1133
   <211> 7
   <212> PRT
   <213> Artificial Sequence
<220>
   <223> Description of Artificial Sequence: Synthetic Peptide
<400> 1133
   Gly Arg Ala Asp Ser Pro Lys
   1 5
<210> 1134
   <211> 37
   <212> PRT
   <213> Artificial Sequence
<220>
   <223> Description of Artificial Sequence: Synthetic Peptide
<400> 1134
   Phe Asn Lys His Thr Glu Ile Ile Glu Glu Asp Thr Asn Lys Asp Lys
   1 5 10 15
   Pro Ser Tyr Gln Phe Gly Gly His Asn Ser Val Asp Phe Glu Glu Asp
   20 25 30
   Thr Leu Pro Lys Val
   35
<210> 1135
   <211> 16
   <212> PRT
   <213> Artificial Sequence
<220>
   <223> Description of Artificial Sequence: Synthetic Peptide
<400> 1135
   Ala Asp Ser Gly Glu Gly Asp Phe Leu Ala Glu Gly Gly Gly Val Arg
   1 5 10 15
<210> 1136
   <211> 17
   <212> PRT
   <213> Artificial Sequence
<220>
   <223> Description of Artificial Sequence: Synthetic Peptide
<400> 1136
   Tyr Ala Asp Ser Gly Glu Gly Asp Phe Leu Ala Glu Gly Gly Gly Val
   1 5 10 15
   Arg
<210> 1137
   <211> 13
   <212> PRT
   <213> Artificial Sequence
<220>
   <223> Description of Artificial Sequence: Synthetic Peptide
<400> 1137
   Gly Val Asn Asp Asn Glu Glu Gly Phe Phe Ser Ala Arg
   1 5 10
<210> 1138
   <211> 14
   <212> PRT
   <213> Artificial Sequence
<220>
   <223> Description of Artificial Sequence: Synthetic Peptide
<400> 1138
<210> 1139
   <211> 14
   <212> PRT
   <213> Artificial Sequence
<220>
   <223> Description of Artificial Sequence: Synthetic Peptide
<400> 1139
   Gly Val Asn Asp Asn Glu Glu Gly Phe Phe Ser Ala Arg Tyr
   1 5 10
<210> 1140
   <211> 18
   <212> PRT
   <213> Artificial Sequence
<220>
   <223> Description of Artificial Sequence: Synthetic Peptide
<400> 1140
   Tyr Glu Lys Pro Gly Ser Pro Pro Arg Glu Val Val Pro Arg Pro Arg
   1 5 10 15
   Gly Val
<210> 1141
   <211> 8
   <212> PRT
   <213> Artificial Sequence
<220>
   <223> Description of Artificial Sequence: Synthetic Peptide
<400> 1141
   Trp Gln Pro Pro Arg Ala Arg Ile
   1 5
<210> 1142
   <211> 5
   <212> PRT
   <213> Artificial Sequence
<220>
   <223> Description of Artificial Sequence: Synthetic Peptide
<400> 1142
   Glu Ile Leu Asp Val
   1 5

<210> 1143
   <211> 8
   <212> PRT
   <213> Artificial Sequence
<220>
   <223> Description of Artificial Sequence: Synthetic Peptide
<400> 1143
   Glu Ile Leu Asp Val Pro Ser Thr
   1 5
<210> 1144
   <211> 6
   <212> PRT
   <213> Artificial Sequence
<220>
   <223> Description of Artificial Sequence: Synthetic Peptide
<400> 1144
   Gly Arg Ala Asp Ser Pro
   1 5
<210> 1145
   <211> 5
   <212> PRT
   <213> Artificial Sequence
<220>
   <223> Description of Artificial Sequence: Synthetic Peptide
<400> 1145
   Gly Arg Gly Asp Ser
   1 5
<210> 1146
   <211> 6
   <212> PRT
   <213> Artificial Sequence
<220>
   <223> Description of Artificial Sequence: Synthetic Peptide
<400> 1146
   Gly Arg Gly Asp Ser Pro
   1 5
<210> 1147
   <211> 7
   <212> PRT
   <213> Artificial Sequence
<220>
   <223> Description of Artificial Sequence: Synthetic Peptide
<400> 1147
   Gly Arg Gly Asp Ser Pro Cys
   1 5
<210> 1148
   <211> 6
   <212> PRT
   <213> Artificial Sequence
<220>
   <223> Description of Artificial Sequence: Synthetic Peptide
<400> 1148
   Gly Arg Gly Asp Thr Pro
   1 5
<210> 1149
   <211> 5
   <212> PRT
   <213> Artificial Sequence
<220>
   <223> Description of Artificial Sequence: Synthetic Peptide
<400> 1149
   Gly Arg Gly Glu Ser
   1 5
<210> 1150
   <211> 6
   <212> PRT
   <213> Artificial Sequence
<220>
   <223> Description of Artificial Sequence: Synthetic Peptide
<400> 1150
   Gly Arg Gly Glu Ser Pro
   1 5
<210> 1151
   <211> 5
   <212> PRT
   <213> Artificial Sequence
<220>
   <223> Description of Artificial Sequence: synthetic Peptide
<400> 1151
   Gly Gly Asp Ser Pro
   1 5
<210> 1152
   <211> 15
   <212> PRT
   <213> Artificial Sequence
<220>
   <223> Description of Artificial Sequence: Synthetic Peptide
<400> 1152
   Gly Gln Gln His His Leu Gly Gly Ala Lys Gln Ala Gly Asp Val
   1 5 10 15
<210> 1153
   <211> 3
   <212> PRT
   <213> Artificial Sequence
<220>
   <223> Description of Artificial Sequence: Synthetic Peptide
<400> 1153
   Gly Pro Arg
   1
<210> 1154
   <211> 10
   <212> PRT
   <213> Artificial Sequence
<220>
   <223> Description of Artificial sequence: Synthetic Peptide
<400> 1154
   Gly Arg Gly Asp Ser Pro Ala Ser Ser Lys
   1 5 10
<210> 1155
   <211> 9
   <212> PRT
   <213> Artificial Sequence
<220>
   <223> Description of Artificial Sequence: Synthetic Peptide
<400> 1155
   Gly Gly Arg Gly Asp Ser Pro Cys Ala
   1 5
<210> 1156
   <211> 6
   <212> PRT
   <213> Artificial Sequence
<220>
   <223> Description of Artificial Sequence: Synthetic Peptide
<400> 1156
   Gly Arg Gly Asp Ser Pro
   1 5
<210> 1157
   <211> 6
   <212> PRT
   <213> Artificial Sequence
<220>
   <223> Description of Artificial Sequence: Synthetic Peptide
<400> 1157
   Gly Arg Gly Asp Asn Pro
   1 5
<210> 1158
   <211> 8
   <212> PRT
   <213> Artificial Sequence
<220>
   <223> Description of Artificial Sequence: Synthetic Peptide
<400> 1158
   Trp Gln Pro Pro Arg Ala Arg Ile
   1 5
<210> 1159
   <211> 5
   <212> PRT
   <213> Artificial Sequence
<220>
   <223> Description of Artificial Sequence: Synthetic Peptide
<400> 1159
   Tyr Ile Gly Ser Arg
   1 5
<210> 1160
   <211> 3
   <212> PRT
   <213> Artificial Sequence
<220>
   <223> Description of Artificial Sequence: Synthetic Peptide
<400> 1160
   Leu Asp Val
   1
<210> 1161
   <211> 5
   <212> PRT
   <213> Artificial Sequence
<220>
   <223> Description of Artificial Sequence: Synthetic Peptide
<400> 1161
   Leu Asp Val Pro Ser
   1 5
<210> 1162
   <211> 4
   <212> PRT
   <213> Artificial Sequence
<220>
   <223> Description of Artificial Sequence: Synthetic Peptide
<400> 1162
   Lys Gly Asp Ser
   1
<210> 1163
   <211> 6
   <212> PRT
   <213> Artificial Sequence
<220>
   <223> Description of Artificial Sequence: Synthetic Peptide
<400> 1163
   Gly Ala Val Ser Thr Ala
   1 5
<210> 1164
   <211> 6
   <212> PRT
   <213> Artificial Sequence
<220>
   <223> Description of Artificial Sequence: Synthetic Peptide
<400> 1164
   Trp Thr Val Pro Thr Ala
   1 5
<210> 1165
   <211> 5
   <212> PRT
   <213> Artificial Sequence
<220>
   <223> Description of Artificial Sequence: Synthetic Peptide
<400> 1165
   Arg Gly Asp Ser Pro
   1 5
<210> 1166
   <211> 11
   <212> PRT
   <213> Artificial Sequence
<220>
   <223> Description of Artificial Sequence: Synthetic Peptide
<400> 1166
   Thr Asp Val Asn Gly Asp Gly Arg His Asp Leu
   1 5 10
<210> 1167
   <211> 4
   <212> PRT
   <213> Artificial Sequence
<220>
   <223> Description of Artificial Sequence: Synthetic Peptide
<400> 1167
   Arg Glu Asp Val
   1
<210> 1168
   <211> 10
   <212> PRT
   <213> Artificial Sequence
<220>
   <223> Description of Artificial Sequence: Synthetic Peptide
<400> 1168
   Arg Gly Asp Ser Pro Ala Ser Ser Lys Pro
   1 5 10
<210> 1169
   <211> 4
   <212> PRT
   <213> Artificial Sequence
<220>
   <223> Description of Artificial Sequence: Synthetic Peptide
<400> 1169
   Arg Gly Asp Thr
   1
<210> 1170
   <211> 3
   <212> PRT
   <213> Artificial Sequence
<220>
   <223> Description of Artificial Sequence: Synthetic Peptide
<400> 1170
   Arg Asp Ser
   1
<210> 1171
   <211> 5
   <212> PRT
   <213> Artificial Sequence
<220>
   <223> Description of Artificial Sequence: Synthetic Peptide
<400> 1171
   Ser Asp Gly Arg Gly
   1 5
<210> 1172
   <211> 4
   <212> PRT
   <213> Artificial Sequence
<220>
   <223> Description of Artificial Sequence: Synthetic Peptide
<400> 1172
   Ser Asp Gly Arg
   1
<210> 1173
   <211> 5
   <212> PRT
   <213> Artificial Sequence
<220>
   <223> Description of Artificial Sequence: Synthetic Peptide
<400> 1173
   Tyr Arg Gly Asp Ser
   1 5
<210> 1174
   <211> 42
   <212> PRT
   <213> Artificial Sequence
<220>
   <223> Description of Artificial Sequence: Synthetic Peptide
<400> 1174
   Tyr Ala Lys Leu Leu Gly His Gln Asn Leu Lys Gln Lys Ile Lys His
   1 5 10 15
   Val Val Lys Leu Lys Asp Glu Asn Ser Gln Leu Lys Ser Glu Val Ser
   20 25 30
   Lys Leu Arg Cys Gln Leu Ala Lys Lys Lys
   35 40
<210> 1175
   <211> 4
   <212> PRT
   <213> Artificial Sequence
<220>
   <223> Description of Artificial Sequence: Synthetic Peptide
<400> 1175
   Phe Met Arg Phe
   1
<210> 1176
   <211> 4
   <212> PRT
   <213> Artificial Sequence
<220>
   <223> Description of Artificial Sequence: Synthetic Peptide
<400> 1176
   Phe Met Arg Phe
   1
<210> 1177
   <211> 4
   <212> PRT
   <213> Artificial Sequence
<220>
   <223> Description of Artificial Sequence: Synthetic Peptide
<400> 1177
   Phe Met Arg Phe
   1
<210> 1178
   <211> 4
   <212> PRT
   <213> Artificial Sequence
<220>
   <223> Description of Artificial Sequence: Synthetic Peptide
<400> 1178
   Phe Met Arg Phe
   1
<210> 1179
   <211> 5
   <212> PRT
   <213> Artificial Sequence
<220>
   <223> Description of Artificial Sequence: Synthetic Peptide
<400> 1179
   Leu Pro Leu Arg Phe
   1 5
<210> 1180 .
   <211> 6
   <212> PRT
   <213> Artificial Sequence
<220>
   <223> Description of Artificial Sequence: Synthetic Peptide
<400> 1180
   Tyr Leu Pro Leu Arg Phe
   1 5
<210> 1181
   <211> 3
   <212> PRT
   <213> Artificial Sequence
<220>
   <223> Description of Artificial Sequence: Synthetic Peptide
<400> 1181
   Trp Arg Phe
   1
<210> 1182
   <211> 5
   <212> PRT
   <213> Artificial Sequence
<220>
   <223> Description of Artificial Sequence: Synthetic Peptide
<400> 1182
   Tyr Phe Met Arg Phe
   1 5
<210> 1183
   <211> 4
   <212> PRT
   <213> Artificial Sequence
<220>
   <223> Description of Artificial Sequence: Synthetic Peptide
<400> 1183
   Tyr Met Arg Phe
   1
<210> 1184
   <211> 8
   <212> PRT
   <213> Artificial Sequence
<220>
   <223> Description of Artificial Sequence: Synthetic Peptide
<400> 1184
   Ser Asp Arg Asn Phe Leu Arg Phe
   1 5
<210> 1185
   <211> 8
   <212> PRT
   <213> Artificial Sequence
<220>
   <223> Description of Artificial Sequence: Synthetic Peptide
<400> 1185
   Ser Asp Arg Asn Phe Leu Arg Phe
   1 5
<210> 1186
   <211> 6
   <212> PRT
   <213> Artificial Sequence
<220>
   <223> Description of Artificial Sequence: Synthetic Peptide
<400> 1186
   Asp Pro Phe Leu Arg Phe
   1 5
<210> 1187
   <211> 8
   <212> PRT
   <213> Artificial Sequence
<220>
   <223> Description of Artificial Sequence: Synthetic Peptide
<400> 1187
   Glu Ile Leu Glu Val Pro Ser Thr
   1 5
<210> 1188
   <211> 30
   <212> PRT
   <213> Artificial Sequence
<220>
   <223> Description of Artificial Sequence: Synthetic Peptide
<400> 1188
   Gly Trp Thr Leu Asn Ser Ala Gly Tyr Leu Leu Gly Pro His Ala Val
   1 5 10 15
   Gly Asn His Arg Ser Phe Ser Asp Lys Asn Gly Leu Thr Ser
   20 25 30
<210> 1189
   <211> 19
   <212> PRT
   <213> Artificial Sequence
<220>
   <223> Description of Artificial Sequence: Synthetic Peptide
<400> 1189
   Gly Trp Thr Leu Asn Ser Ala Gly Tyr Leu Leu Gly Pro His Ala Val
   1 5 10 15
   Gly Asn His
<210> 1190
   <211> 30
   <212> PRT
   <213> Artificial Sequence
<220>
   <223> Description of Artificial Sequence: Synthetic Peptide
<400> 1190
   Met Ala Arg Gly Ser Ala Leu Leu Leu Ala Ser Leu Leu Leu Ala Ala
   1 5 10 15
   Ala Leu Ser Ala Ser Ala Gly Leu Trp Ser Pro Ala Lys Glu
   20 25 30
<210> 1191
   <211> 24
   <212> PRT
   <213> Artificial Sequence
<220>
   <223> Description of Artificial Sequence: Synthetic Peptide
<400> 1191
   Glu Leu Arg Pro Glu Asp Asp Met Lys Pro Gly Ser Phe Asp Arg Ser
   1 5 10 15
   Ile Pro Glu Asn Asn Ile Met Arg
   20
<210> 1192
   <211> 35
   <212> PRT
   <213> Artificial Sequence
<220>
   <223> Description of Artificial Sequence: Synthetic Peptide
<400> 1192
   Thr Ile Ile Glu Phe Leu Ser Phe Leu His Leu Lys Glu Ala Gly Ala
   1 5 10 15
   Leu Asp Arg Leu Leu Asp Leu Pro Ala Ala Ala Ser Ser Glu Asp Ile
   20 25 30
   Glu Arg Ser
   35
<210> 1193
   <211> 29
   <212> PRT
   <213> Artificial Sequence
<220>
   <223> Description of Artificial sequence: Synthetic Peptide
<400> 1193
   Gly Trp Thr Leu Asn Ser Ala Gly Tyr Leu Leu Gly Pro His Ala Ile
   1 5 10 15
   Asp Asn His Arg Ser Phe His Asp Lys Tyr Gly Leu Ala
   20 25
<210> 1194
   <211> 16
   <212> PRT
   <213> Artificial Sequence
<220>
   <223> Description of Artificial Sequence: Synthetic Peptide
<400> 1194
   Gly Trp Thr Leu Asn Ser Ala Gly Tyr Leu Leu Gly Pro His Ala Ile
   1 5 10 15
<210> 1195
   <211> 29
   <212> PRT
   <213> Artificial Sequence
<220>
   <223> Description of Artificial Sequence: Synthetic Peptide
<400> 1195
   Gly Trp Thr Leu Asn Ser Ala Gly Tyr Leu Leu Gly Pro His Ala Ile
   1 5 10 15
   Asp Asn His Arg Ser Phe Ser Asp Lys His Gly Leu Thr
   20 25
<210> 1196
   <211> 40
   <212> PRT
   <213> Artificial Sequence
<220>
   <223> Description of Artificial Sequence: Synthetic Peptide
<400> 1196
   Thr Lys Glu Lys Arg Gly Trp Thr Leu Asn Ser Ala Gly Tyr Leu Leu
   1 5 10 15
   Gly Pro His Ala Ile Asp Asn His Arg Ser Phe Ser Asp Lys His Gly
   20 25 30
   Leu Thr Gly Lys Arg Glu Leu Pro
   35 40
<210> 1197
   <211> 21
   <212> PRT
   <213> Artificial Sequence
<220>
   <223> Description of Artificial Sequence: Synthetic Peptide
<400> 1197
   Gly Trp Thr Leu Asn Ser Ala Gly Tyr Leu Leu Gly Pro Pro Pro Gly
   1 5 10 15
   Phe Ser Pro Phe Arg
   20
<210> 1198
   <211> 20
   <212> PRT
   <213> Artificial Sequence
<220>
   <223> Description of Artificial Sequence: Synthetic Peptide
<400> 1198
   Gly Trp Thr Leu Asn Ser Ala Gly Tyr Leu Leu Gly Pro Pro Pro Ala
   1 5 10 15
   Leu Ala Leu Ala
   20
<210> 1199
   <211> 24
   <212> PRT
   <213> Artificial Sequence
<220>
   <223> Description of Artificial Sequence: Synthetic Peptide
<400> 1199
   Gly Trp Thr Leu Asn Ser Ala Gly Tyr Leu Leu Gly Pro Arg Pro Lys
   1 5 10 15
   Pro Gln Gln Trp Phe Trp Leu Leu
   20
<210> 1200
   <211> 41
   <212> PRT
   <213> Artificial Sequence
<220>
   <223> Description of Artificial Sequence: Synthetic Peptide
<400> 1200
   Glu Leu Glu Pro Glu Asp Glu Ala Arg Pro Gly Gly Phe Asp Arg Leu
   1 5 10 15
   Gln Ser Glu Asp Lys Ala Ile Arg Thr Ile Met Glu Phe Leu Ala Phe
   20 25 30
   Leu His Leu Lys Glu Ala Gly Ala Leu
   35 40
<210> 1201
   <211> 26
   <212> PRT
   <213> Artificial Sequence
<220>
   <223> Description of Artificial sequence: Synthetic Peptide
<400> 1201
   Leu Gln Ser Glu Asp Lys Ala Ile Arg Thr Ile Met Glu Phe Leu Ala
   1 5 10 15
   Phe Leu His Leu Lys Glu Ala Gly Ala Leu
   20 25
<210> 1202
   <211> 17
   <212> PRT
   <213> Artificial Sequence
<220>
   <223> Description of Artificial Sequence: Synthetic Peptide
<400> 1202
   Thr Ile Met Glu Phe Leu Ala Phe Leu His Leu Lys Glu Ala Gly Ala
   1 5 10 15
   Leu
<210> 1203
   <211> 20
   <212> PRT
   <213> Artificial Sequence
<220>
   <223> Description of Artificial Sequence: Synthetic Peptide
<400> 1203
   Gly Trp Thr Leu Asn Ser Ala Gly Tyr Leu Leu Gly Pro Gln Gln Phe
   1 5 10 15
   Phe Gly Leu Met
   20
<210> 1204
   <211> 15
   <212> PRT
   <213> Artificial Sequence
<220>
   <223> Description of Artificial Sequence: Synthetic Peptide
<400> 1204
   Gly Trp Thr Leu Asn Thr Ala Trp Trp Leu Leu Gly Pro His Ala
   1 5 10 15
<210> 1205
   <211> 15
   <212> PRT
   <213> Artificial Sequence
<220>
   <223> Description of Artificial Sequence: Synthetic Peptide
<400> 1205
   Gly Trp Thr Leu Asn Thr Ala Trp Trp Leu Leu Gly Pro His Ala
   1 5 10 15
<210> 1206
   <211> 20
   <212> PRT
   <213> Artificial Sequence
<220>
   <223> Description of Artificial Sequence: Synthetic Peptide
<400> 1206
   Gly Trp Thr Leu Asn Ser Ala Gly Tyr Leu Leu Gly Pro Pro Pro Ala
   1 5 10 15
   Leu Ala Leu Ala
   20
<210> 1207
   <211> 24
   <212> PRT
   <213> Artificial Sequence
<220>
   <223> Description of Artificial Sequence: Synthetic Peptide
<400> 1207
   Gly Trp Thr Leu Asn Ser Ala Gly Tyr Leu Leu Gly Pro Arg Pro Lys
   1 5 10 15
   Pro Gln Gln Trp Phe Trp Leu Leu
   20
<210> 1208
   <211> 12
   <212> PRT
   <213> Artificial Sequence
<220>
   <223> Description of Artificial Sequence: Synthetic Peptide
<400> 1208
   Asp Glu Pro Asn Ser Asp Gln Phe Ile Gly Leu Met
   1 5 10
<210> 1209
   <211> 120
   <212> PRT
   <213> Artificial Sequence
<220>
   <223> Description of Artificial Sequence: Synthetic Peptide
<400> 1209
   Lys Lys Lys Asp Lys Val Lys Lys Gly Gly Pro Gly Ser Glu Cys Ala
   1 5 10 15
   Glu Trp Ala Trp Gly Pro Cys Thr Pro Ser Ser Lys Asp Cys Gly Val
   20 25 30
   Gly Phe Arg Glu Gly Thr Cys Gly Ala Gln Thr Gln Arg Ile Arg Cys
   35 40 45
   Arg Val Pro Cys Asn Trp Lys Lys Glu Phe Gly Ala Asp Cys Lys Lys
   50 55 60
   Phe Glu Asn Trp Gly Ala Cys Asp Gly Gly Thr Gly Thr Lys Val Arg
   65 70 75 80
   Gln Gly Thr Leu Lys Lys Ala Arg Tyr Asn Ala Gln Cys Gln Glu Thr
   85 90 95
   Ile Arg Val Thr Lys Pro Cys Thr Pro Lys Thr Lys Ala Lys Ala Lys
   100 105 110
   Ala Lys Lys Gly Lys Gly Lys Asp
   115 120
<210> 1210
   <211> 61
   <212> PRT
   <213> Artificial Sequence
<220>
   <223> Description of Artificial Sequence: Synthetic Peptide
<400> 1210
   Ala Asp Cys Lys Lys Phe Glu Asn Trp Gly Ala Cys Asp Gly Gly Thr
   1 5 10 15
   Gly Thr Lys Val Arg Gln Gly Thr Leu Lys Lys Ala Arg Tyr Asn Ala
   20 25 30
   Gln Cys Gln Glu Thr Ile Arg Val Thr Lys Pro Cys Thr Pro Lys Thr
   35 40 45
   Lys Ala Lys Ala Lys Ala Lys Lys Gly Lys Gly Lys Asp
   50 55 60
<210> 1211
   <211> 10
   <212> PRT
   <213> Artificial Sequence
<220>
   <223> Description of Artificial Sequence: Synthetic Peptide
<400> 1211
   Cys His Ser Gly Tyr Val Gly Val Arg Cys
   1 5 10
<210> 1212
   <211> 13
   <212> PRT
   <213> Artificial Sequence
<220>
   <223> Description of Artificial Sequence: Synthetic Peptide
<400> 1212
   Ala Asn Phe Leu Val Trp Glu Ile Val Arg Lys Lys Pro
   1 5 10
<210> 1213
   <211> 50
   <212> PRT
   <213> Artificial Sequence
<220>
   <223> Description of Artificial Sequence: Synthetic Peptide
<400> 1213
   Val Val Ser His Phe Asn Asp Cys Pro Asp Ser His Thr Gln Phe Cys
   1 5 10 15
   Phe His Gly Thr Cys Arg Phe Leu Val Gln Glu Asp Lys Pro Ala Cys
   20 25 30
   Val Cys His Ser Gly Tyr Val Gly Ala Arg Cys Glu His Ala Asp Leu
   35 40 45
   Leu Ala
   50
<210> 1214
   <211> 50
   <212> PRT
   <213> Artificial Sequence
<220>
   <223> Description of Artificial Sequence: Synthetic Peptide
<400> 1214
   Val Val Ser His Phe Asn Lys Cys Pro Asp Ser His Thr Gln Tyr Cys
   1 5 10 15
   Phe His Gly Thr Cys Arg Phe Leu Val Gln Glu Glu Lys Pro Ala Cys
   20 25 30
   Val Cys His Ser Gly Tyr Val Gly Val Arg Cys Glu His Ala Asp Leu
   35 40 45
   Leu Ala
   50
<210> 1215
   <211> 10
   <212> PRT
   <213> Artificial Sequence
<220>
   <223> Description of Artificial Sequence: Synthetic Peptide
<400> 1215
   Cys His Ser Gly Tyr Val Gly Val Arg Cys
   1 5 10
<210> 1216
   <211> 10
   <212> PRT
   <213> Artificial Sequence
<220>
   <223> Description of Artificial Sequence: Synthetic Peptide
<400> 1216
   Cys His Ser Gly Tyr Val Gly Val Arg Cys
   1 5 10
<210> 1217
   <211> 6
   <212> PRT
   <213> Artificial Sequence
<220>
   <223> Description of Artificial Sequence: Synthetic Peptide
<400> 1217
   Pro Pro Gly His Phe Lys
   1 5
<210> 1218
   <211> 6
   <212> PRT
   <213> Artificial Sequence
<220>
   <223> Description of Artificial Sequence: Synthetic Peptide
<400> 1218
   Arg Thr Gly Gln Tyr Lys
   1 5
<210> 1219
   <211> 11
   <212> PRT
   <213> Artificial Sequence
<220>
   <223> Description of Artificial Sequence: Synthetic Peptide
<400> 1219
   Phe Asn Leu Pro Leu Gly Asn Tyr Lys Lys Pro
   1 5 10

<210> 1220
   <211> 24
   <212> PRT
   <213> Artificial Sequence
<220>
   <223> Description of Artificial Sequence: Synthetic Peptide
<400> 1220
   Pro Ala Leu Pro Glu Asp Gly Gly Ser Gly Ala Phe Pro Pro Gly His
   1 5 10 15
   Phe Lys Asp Pro Lys Arg Leu Tyr
   20
<210> 1221
   <211> 10
   <212> PRT
   <213> Artificial Sequence
<220>
   <223> Description of Artificial Sequence: Synthetic Peptide
<400> 1221
   His Ala Glu Lys His Trp Phe Val Gly Leu
   1 5 10
<210> 1222
   <211> 12
   <212> PRT
   <213> Artificial Sequence
<220>
   <223> Description of Artificial Sequence: Synthetic Peptide
<400> 1222
   Cys Met His Ile Glu Ser Leu Asp Ser Tyr Thr Cys
   1 5 10
<210> 1223
   <211> 12
   <212> PRT
   <213> Artificial Sequence
<220>
   <223> Description of Artificial Sequence: Synthetic Peptide
<400> 1223
   Asp Val Val Asp Ala Asp Glu Tyr Leu Ile Pro Gln
   1 5 10
<210> 1224
   <211> 12
   <212> PRT
   <213> Artificial Sequence
<220>
   <223> Description of Artificial Sequence: Synthetic Peptide
<400> 1224
   Cys Tyr Ala Ala Pro Leu Lys Pro Ala Lys Ser Cys
   1 5 10
<210> 1225
   <211> 18
   <212> PRT
   <213> Artificial Sequence
<220>
   <223> Description of Artificial Sequence: Synthetic Peptide
<400> 1225
   Tyr Phe Asn Lys Pro Thr Gly Tyr Gly Ser Ser Ser Arg Arg Ala Pro
   1 5 10 15
   Gln Thr
<210> 1226
   <211> 14
   <212> PRT
   <213> Artificial Sequence
<220>
   <223> Description of Artificial Sequence: Synthetic Peptide
<400> 1226
   Ala Leu Leu Glu Thr Tyr Cys Ala Thr Pro Ala Lys Ser Glu
   1 5 10
<210> 1227
   <211> 12
   <212> PRT
   <213> Artificial Sequence
<220>
   <223> Description of Artificial Sequence: Synthetic Peptide
<400> 1227
   Gly Tyr Gly Ser Ser Ser Arg Arg Ala Pro Gln Thr
   1 5 10
<210> 1228
   <211> 8
   <212> PRT
   <213> Artificial Sequence
<220>
   <223> Description of Artificial Sequence: Synthetic Peptide
<400> 1228
   Ser Arg Val Ser Arg Arg Ser Arg
   1 5
<210> 1229
   <211> 9
   <212> PRT
   <213> Artificial Sequence
<220>
   <223> Description of Artificial Sequence: Synthetic Peptide
<400> 1229
   Tyr Ser Arg Val Ser Arg Arg Ser Arg
   1 5
<210> 1230
   <211> 3
   <212> PRT
   <213> Artificial Sequence
<220>
   <223> Description of Artificial Sequence: Synthetic Peptide
<400> 1230
   Gly His Lys
   1
<210> 1231
   <211> 22
   <212> PRT
   <213> Artificial Sequence
<220>
   <223> Description of Artificial Sequence: Synthetic Peptide
<400> 1231
<210> 1232
   <211> 24
   <212> PRT
   <213> Artificial Sequence
<220>
   <223> Description of Artificial Sequence: Synthetic Peptide
<400> 1232
   Gly Lys Lys Glu Lys Pro Glu Lys Lys Val Lys Lys Ser Asp Cys Gly
   1 5 10 15
   Glu Trp Gln Trp Ser Val Cys Val
   20
<210> 1233
   <211> 6
   <212> PRT
   <213> Artificial Sequence
<220>
   <223> Description of Artificial Sequence: Synthetic Peptide
<400> 1233
   Ser Phe Leu Pro Ser Ser
   1 5
<210> 1234
   <211> 15
   <212> PRT
   <213> Artificial Sequence
<220>
   <223> Description of Artificial Sequence: Synthetic Peptide
<400> 1234
   Ser Ala Gln Thr Asn Arg His Ile Leu Arg Phe Asn Arg Pro Phe
   1 5 10 15
<210> 1235
   <211> 18
   <212> PRT
   <213> Artificial Sequence
<220>
   <223> Description of Artificial Sequence: Synthetic Peptide
<400> 1235
<210> 1236
   <211> 18
   <212> PRT
   <213> Artificial Sequence
<220>
   <223> Description of Artificial Sequence: Synthetic Peptide
<400> 1236
   Leu Ala Gly Glu Thr Gly Gln Glu Ala Ala Pro Leu Asp Gly Val Leu
   1 5 10 15
   Ala Asn
<210> 1237
   <211> 14
   <212> PRT
   <213> Artificial Sequence
<220>
   <223> Description of Artificial Sequence: Synthetic Peptide
<400> 1237
   Ala Leu Lys Arg Gln Gly Arg Thr Leu Tyr Gly Phe Gly Gly
   1 5 10
<210> 1238
   <211> 30
   <212> PRT
   <213> Artificial Sequence
<220>
   <223> Description of Artificial Sequence: Synthetic Peptide
<400> 1238
   Gly Met Asp Val Leu Gly Arg Pro Lys Ile Pro Leu Glu Thr Pro Ala
   1 5 10 15
   Tyr Thr Gly Gln Pro Trp His Cys Gln His Cys Phe Leu Leu
   20 25 30
<210> 1239
   <211> 43
   <212> PRT
   <213> Artificial Sequence
<220>
   <223> Description of Artificial sequence: Synthetic Peptide
<400> 1239
   Met Asn Thr Ile Thr Ile Cys Lys Phe Asp Val Leu Asp Ala Glu Leu
   1 5 10 15
   Leu Ser Thr Val Glu Gly Gly Tyr Ser Gly Lys Asp Cys Leu Lys Asp
   20 25 30
   Met Gly Gly Tyr Ala Leu Ala Gly Ala Gly Ser
   35 40
<210> 1240
   <211> 38
   <212> PRT
   <213> Artificial Sequence
<220>
   <223> Description of Artificial Sequence: Synthetic Peptide
<400> 1240
   Gly Ala Asp Lys Thr Val Lys Gly Pro Asp Gly Leu Thr Ala Leu Glu
   1 5 10 15
   Ala Thr Asp Asn Gln Ala Ile Asp Tyr Gly Gly Phe Met Glu Val Val
   20 25 30
   Tyr Val Asp Ala Thr Lys
   35
<210> 1241
   <211> 16
   <212> PRT
   <213> Artificial Sequence
<220>
   <223> Description of Artificial Sequence: Synthetic Peptide
<400> 1241
   Cys Lys Gln Leu Gln Arg Asp Arg Gln Val Tyr Arg Ala Thr His Arg
   1 5 10 15
<210> 1242
   <211> 17
   <212> PRT
   <213> Artificial Sequence
<220>
   <223> Description of Artificial Sequence: Synthetic Peptide
<400> 1242
   Cys Glu Gly Asn Val Arg Val Ser Arg Glu Leu Ala Gly His Thr Gly
   1 5 10 15
   Tyr
<210> 1243
   <211> 16
   <212> PRT
   <213> Artificial Sequence
<220>
   <223> Description of Artificial Sequence: Synthetic Peptide
<400> 1243
   Cys Gly Ala Gly Glu Ser Gly Lys Ser Thr Ile Val Lys Gln Met Lys
   1 5 10 15
<210> 1244
   <211> 15
   <212> PRT
   <213> Artificial Sequence
<220>
   <223> Description of Artificial Sequence: Synthetic Peptide
<400> 1244
   Cys Asn Leu Lys Glu Asp Gly Ile Ser Ala Ala Lys Asp Val Lys
   1 5 10 15
<210> 1245
   <211> 16
   <212> PRT
   <213> Artificial Sequence
<220>
   <223> Description of Artificial Sequence: Synthetic Peptide
<400> 1245
   Cys Lys Gln Leu Gln Lys Asp Lys Gln Val Tyr Arg Ala Thr His Arg
   1 5 10 15
<210> 1246
   <211> 11
   <212> PRT
   <213> Artificial Sequence
<220>
   <223> Description of Artificial Sequence: Synthetic Peptide
<400> 1246
   Glu Glu Gln Gly Met Leu Pro Glu Asp Leu Ser
   1 5 10
<210> 1247
   <211> 15
   <212> PRT
   <213> Artificial Sequence
<220>
   <223> Description of Artificial Sequence: Synthetic Peptide
<400> 1247
   Pro Gly Thr Cys Glu Ile Cys Ala Tyr Ala Ala Cys Thr Gly Cys
   1 5 10 15
<210> 1248
   <211> 15
   <212> PRT
   <213> Artificial Sequence
<220>
   <223> Description of Artificial Sequence: Synthetic Peptide
<400> 1248
   Pro Asn Thr Cys Glu Ile Cys Ala Tyr Ala Ala Cys Thr Gly Cys
   1 5 10 15
<210> 1249
   <211> 16
   <212> PRT
   <213> Artificial Sequence
<220>
   <223> Description of Artificial Sequence: synthetic Peptide
<400> 1249
   Asn Asp Asp Cys Glu Leu Cys Val Asn Val Ala Cys Thr Gly Cys Leu
   1 5 10 15
<210> 1250
   <211> 32
   <212> PRT
   <213> Artificial Sequence
<220>
   <223> Description of Artificial Sequence: Synthetic Peptide
<400> 1250
   Ser Thr Pro Leu Met Ser Trp Pro Trp Ser Pro Ser Ala Leu Arg Leu
   1 5 10 15
   Leu Gln Arg Pro Pro Glu Glu Pro Ala Ala His Ala Asn Cys His Arg
   20 25 30
<210> 1251
   <211> 33
   <212> PRT
   <213> Artificial Sequence
<220>
   <223> Description of Artificial Sequence: Synthetic Peptide
<400> 1251
   Tyr Ser Thr Pro Leu Met Ser Trp Pro Trp Ser Pro Ser Ala Leu Arg
   1 5 10 15
   Leu Leu Gln Arg Pro Pro Glu Glu Pro Ala Ala His Ala Asn Cys His
   20 25 30
   Arg
<210> 1252
   <211> 31
   <212> PRT
   <213> Artificial Sequence
<220>
   <223> Description of Artificial Sequence: Synthetic Peptide
<400> 1252
   His Asn Lys Gln Glu Gly Arg Asp His Asp Lys Ser Lys Gly His Phe
   1 5 10 15
   His Arg Val Val Ile His His Lys Gly Gly Lys Ala His Arg Gly
   20 25 30
<210> 1253
   <211> 32
   <212> PRT
   <213> Artificial Sequence
<220>
   <223> Description of Artificial Sequence: Synthetic Peptide
<400> 1253
   Tyr His Asn Lys Gln Glu Gly Arg Asp His Asp Lys Ser Lys Gly His
   1 5 10 15
   Phe His Arg Val Val Ile His His Lys Gly Gly Lys Ala His Arg Gly
   20 25 30
<210> 1254
   <211> 32
   <212> PRT
   <213> Artificial Sequence
<220>
   <223> Description of Artificial Sequence: Synthetic Peptide
<400> 1254
   Ser Thr Ala Pro Leu Pro Trp Pro Trp Ser Pro Ala Ala Leu Arg Leu
   1 5 10 15
   Leu Gln Arg Pro Pro Glu Glu Pro Ala Val His Ala Asp Cys His Arg
   20 25 30
<210> 1255
   <211> 33
   <212> PRT
   <213> Artificial Sequence
<220>
   <223> Description of Artificial Sequence: Synthetic Peptide
<400> 1255
   Tyr Ser Thr Ala Pro Leu Pro Trp Pro Trp Ser Pro Ala Ala Leu Arg
   1 5 10 15
   Leu Leu Gln Arg Pro Pro Glu Glu Pro Ala Val His Ala Asp Cys His
   20 25 30
   Arg
<210> 1256
   <211> 17
   <212> PRT
   <213> Artificial Sequence
<220>
   <223> Description of Artificial Sequence: Synthetic Peptide
<400> 1256
   Val Gln Gly Glu Thr Ser Asn Asp Lys Ile Pro Val Ala Leu Gly Leu
   1 5 10 15
   Lys
<210> 1257
   <211> 72
   <212> PRT
   <213> Artificial Sequence
<220>
   <223> Description of Artificial Sequence: Synthetic Peptide
<400> 1257
   Ala Pro Val Ala Asn Glu Leu Arg Cys Gln Cys Leu Gln Thr Val Ala
   1 5 10 15
   Gly Ile His Phe Lys Asn Ile Gln Ser Leu Lys Val Met Pro Pro Gly
   20 25 30
   Pro His Cys Thr Gln Thr Glu Val Ile Ala Thr Leu Lys Asn Gly Arg
   35 40 45
   Glu Ala Cys Leu Asp Pro Glu Ala Pro Met Val Gln Lys Ile Val Gln
   50 55 60
   Lys Met Leu Lys Gly Val Pro Lys
   65 70
<210> 1258
   <211> 9
   <212> PRT
   <213> Artificial Sequence
<220>
   <223> Description of Artificial Sequence: Synthetic Peptide
<400> 1258
   Val Gln Gly Glu Glu Ser Asn Asp Lys
   1 5
<210> 1259
   <211> 13
   <212> PRT
   <213> Artificial Sequence
<220>
   <223> Description of Artificial Sequence: Synthetic Peptide
<400> 1259
   Ile Leu Asn Gly Ile Asn Asn Tyr Lys Asn Pro Lys Leu
   1 5 10
<210> 1260
   <211> 22
   <212> PRT
   <213> Artificial Sequence
<220>
   <223> Description of Artificial Sequence: Synthetic Peptide
<400> 1260
   Phe Val Gln Gly Glu Ala Ile Pro Met Ser Ile Pro Pro Glu Asp Lys
   1 5 10 15
   Ile Pro Val Ala Leu Gly
   20
<210> 1261
   <211> 13
   <212> PRT
   <213> Artificial Sequence
<220>
   <223> Description of Artificial Sequence: Synthetic Peptide
<400> 1261
   Ala Pro Val Pro Pro Gly Glu Asp Ser Lys Asp Val Ala
   1 5 10
<210> 1262
   <211> 21
   <212> PRT
   <213> Artificial Sequence
<220>
   <223> Description of Artificial Sequence: Synthetic Peptide
<400> 1262
   Met Val Lys Gln Ile Glu Ser Lys Thr Ala Phe Gln Glu Ala Leu Asp
   1 5 10 15
   Ala Ala Gly Asp Lys
   20
<210> 1263
   <211> 10
   <212> PRT
   <213> Artificial Sequence
<220>
   <223> Description of Artificial Sequence: Synthetic Peptide
<400> 1263
   Ala Tyr Val His Asp Ala Pro Val Arg Ser
   1 5 10
<210> 1264
   <211> 7
   <212> PRT
   <213> Artificial Sequence
<220>
   <223> Description of Artificial Sequence: Synthetic Peptide
<400> 1264
   Pro Arg Lys Leu Tyr Asp Lys
   1 5
<210> 1265
   <211> 12
   <212> PRT
   <213> Artificial Sequence
<220>
   <223> Description of Artificial Sequence: Synthetic Peptide
<400> 1265
   Tyr Thr Thr Asn Pro Arg Lys Leu Tyr Asp Tyr Lys
   1 5 10
<210> 1266
   <211> 24
   <212> PRT
   <213> Artificial Sequence
<220>
   <223> Description of Artificial Sequence: Synthetic Peptide
<400> 1266
   Arg Asn Pro Asp Gly Asp Val Gly Gly Pro Trp Ala Tyr Thr Thr Asn
   1 5 10 15
   Pro Arg Lys Leu Tyr Asp Tyr Lys
   20
<210> 1267
   <211> 12
   <212> PRT
   <213> Artificial Sequence
<220>
   <223> Description of Artificial Sequence: Synthetic Peptide
<400> 1267
   Arg Asn Pro Asp Gly Asp Val Gly Gly Pro Trp Lys
   1 5 10
<210> 1268
   <211> 8
   <212> PRT
   <213> Artificial Sequence
<220>
   <223> Description of Artificial Sequence: Synthetic Peptide
<400> 1268
   Pro Arg Lys Leu Tyr Asp Tyr Lys
   1 5
<210> 1269
   <211> 12
   <212> PRT
   <213> Artificial Sequence
<220>
   <223> Description of Artificial Sequence: Synthetic Peptide
<400> 1269
   Tyr Thr Thr Asn Pro Arg Lys Leu Tyr Asp Tyr Lys
   1 5 10
<210> 1270
   <211> 11
   <212> PRT
   <213> Artificial Sequence
<220>
   <223> Description of Artificial Sequence: Synthetic Peptide
<400> 1270
   Tyr Thr Thr Asn Pro Arg Lys Leu Tyr Asp Tyr
   1 5 10
<210> 1271
   <211> 24
   <212> PRT
   <213> Artificial Sequence
<220>
   <223> Description of Artificial Sequence: Synthetic Peptide
<400> 1271
   Arg Asn Pro Asp Gly Asp Val Gly Gly Pro Trp Ala Tyr Thr Thr Asn
   1 5 10 15
   Pro Arg Lys Leu Tyr Asp Tyr Lys
   20
<210> 1272
   <211> 23
   <212> PRT
   <213> Artificial Sequence
<220>
   <223> Description of Artificial Sequence: Synthetic Peptide
<400> 1272
   Arg Asn Pro Asp Gly Asp Val Gly Gly Pro Trp Ala Tyr Thr Thr Asn
   1 5 10 15
   Pro Arg Lys Leu Tyr Asp Tyr
   20
<210> 1273
   <211> 7
   <212> PRT
   <213> Artificial Sequence
<220>
   <223> Description of Artificial Sequence: Synthetic Peptide
<400> 1273
   Arg Lys Leu Tyr Asp Tyr Lys
   1 5
<210> 1274
   <211> 6
   <212> PRT
   <213> Artificial Sequence
<220>
   <223> Description of Artificial Sequence: Synthetic Peptide
<400> 1274
   Arg Lys Leu Tyr Asp Tyr
   1 5
<210> 1275
   <211> 7
   <212> PRT
   <213> Artificial Sequence
<220>
   <223> Description of Artificial Sequence: Synthetic Peptide
<400> 1275
   Pro Arg Lys Leu Tyr Asp Lys
   1 5
<210> 1276
   <211> 6
   <212> PRT
   <213> Artificial Sequence
<220>
   <223> Description of Artificial Sequence: Synthetic Peptide
<400> 1276
   Pro Arg Lys Leu Tyr Asp
   1 5
<210> 1277
   <211> 8
   <212> PRT
   <213> Artificial Sequence
<220>
   <223> Description of Artificial Sequence: Synthetic Peptide
<400> 1277
   Pro Arg Lys Leu Tyr Asp Tyr Lys
   1 5
<210> 1278
   <211> 4
   <212> PRT
   <213> Artificial Sequence
<220>
   <223> Description of Artificial Sequence: Synthetic Peptide
<400> 1278
   Asp Gly Glu Ala
   1
<210> 1279
   <211> 6
   <212> PRT
   <213> Artificial Sequence
<220>
   <223> Description of Artificial Sequence: Synthetic Peptide
<400> 1279
   Leu Gly Thr Ile Pro Gly
   1 5
<210> 1280
   <211> 23
   <212> PRT
   <213> Artificial Sequence
<220>
   <223> Description of Artificial Sequence: Synthetic Peptide
<400> 1280
   Asn Ile Ser Ser Glu Glu Lys Ala Ser Trp Thr Arg Pro Glu Lys Gln
   1 5 10 15
   Glu Thr Leu Asp Gly His Met
   20
<210> 1281
   <211> 17
   <212> PRT
   <213> Artificial Sequence
<220>
   <223> Description of Artificial Sequence: Synthetic Peptide
<400> 1281
   Phe Asn Leu Asp Val Arg Phe Leu Val Val Lys Glu Ala Val Asn Pro
   1 5 10 15
   Gly
<210> 1282
   <211> 17
   <212> PRT
   <213> Artificial Sequence
<220>
   <223> Description of Artificial Sequence: Synthetic Peptide
<400> 1282
   Ala Pro Arg Gln Arg Gln Thr Leu Val Leu Phe Pro Gly Asp Leu Arg
   1 5 10 15
   Thr
<210> 1283
   <211> 24
   <212> PRT
   <213> Artificial Sequence
<220>
   <223> Description of Artificial Sequence: Synthetic Peptide
<400> 1283
   Cys Ser Cys Ser Pro Val His Pro Gln Gln Ala Phe Cys Asn Ala Asp
   1 5 10 15
   Val Val Ile Arg Ala Lys Ala Val
   20
<210> 1284
   <211> 24
   <212> PRT
   <213> Artificial Sequence
<220>
   <223> Description of Artificial Sequence: Synthetic Peptide
<400> 1284
   Cys Ser Cys Ser Pro Val His Pro Gln Gln Ala Phe Cys Asn Ala Asp
   1 5 10 15
   Val Val Ile Arg Ala Lys Ala Val
   20
<210> 1285
   <211> 13
   <212> PRT
   <213> Artificial Sequence
<220>
   <223> Description of Artificial Sequence: Synthetic Peptide
<400> 1285
   Asn Val Ile Gln Ile Ser Asn Asp Leu Glu Asn Leu Arg
   1 5 10
<210> 1286
   <211> 35
   <212> PRT
   <213> Artificial Sequence
<220>
   <223> Description of Artificial Sequence: Synthetic Peptide
<400> 1286
   Val Pro Ile Gln Lys Val Gln Asp Asp Thr Lys Thr Leu Ile Lys Thr
   1 5 10 15
   Ile Val Thr Arg Ile Asn Asp Ile Ser His Thr Gln Ser Val Ser Ser
   20 25 30
   Lys Gln Lys
   35
<210> 1287
   <211> 15
   <212> PRT
   <213> Artificial Sequence
<220>
   <223> Description of Artificial Sequence: Synthetic Peptide
<400> 1287
   Tyr Lys Val Gln Asp Asp Thr Lys Thr Leu Ile Lys Thr Ile Val
   1 5 10 15
<210> 1288
   <211> 15
   <212> PRT
   <213> Artificial Sequence
<220>
   <223> Description of Artificial Sequence: Synthetic Peptide
<400> 1288
   Ser Cys Ser Leu Pro Gln Thr Ser Gly Leu Gln Lys Pro Glu Ser
   1 5 10 15
<210> 1289
   <211> 10
   <212> PRT
   <213> Artificial Sequence
<220>
   <223> Description of Artificial Sequence: Synthetic Peptide
<400> 1289
   Glu Asp Val Asp His Val Phe Leu Arg Phe
   1 5 10
<210> 1290
   <211> 7
   <212> PRT
   <213> Artificial Sequence
<220>
   <223> Description of Artificial Sequence: Synthetic Peptide
<400> 1290
   Thr Ser Phe Thr Pro Arg Leu
   1 5
<210> 1291
   <211> 8
   <212> PRT
   <213> Artificial Sequence
<220>
   <223> Description of Artificial Sequence: Synthetic Peptide
<400> 1291
   Asp Pro Ala Phe Asn Ser Trp Gly
   1 5
<210> 1292
   <211> 8
   <212> PRT
   <213> Artificial Sequence
<220>
   <223> Description of Artificial Sequence: Synthetic Peptide
<400> 1292
   Asp Pro Gly Phe Ser Ser Trp Gly
   1 5
<210> 1293
   <211> 8
   <212> PRT
   <213> Artificial Sequence
<220>
   <223> Description of Artificial Sequence: Synthetic Peptide
<400> 1293
   Asp Gln Gly Phe Asn Ser Trp Gly
   1 5
<210> 1294
   <211> 8
   <212> PRT
   <213> Artificial Sequence
<220>
   <223> Description of Artificial Sequence: Synthetic Peptide
<400> 1294
   Asp Ala Ser Phe His Ser Trp Gly
   1 5
<210> 1295
   <211> 8
   <212> PRT
   <213> Artificial Sequence
<220>
   <223> Description of Artificial Sequence: Synthetic Peptide
<400> 1295
   Gly Ser Gly Phe Ser Ser Trp Gly
   1 5
<210> 1296
   <211> 8
   <212> PRT
   <213> Artificial Sequence
<220>
   <223> Description of Artificial Sequence: Synthetic Peptide
<400> 1296
   Asp Pro Ala Phe Ser Ser Trp Gly
   1 5

<210> 1297
   <211> 8
   <212> PRT
   <213> Artificial Sequence
<220>
   <223> Description of Artificial Sequence: Synthetic Peptide
<400> 1297
   Gly Ala Ser Phe Tyr Ser Trp Gly
   1 5
<210> 1298
   <211> 10
   <212> PRT
   <213> Artificial Sequence
<220>
   <223> Description of Artificial Sequence: Synthetic Peptide
<400> 1298
   Ile Ile Gly Gly Arg Glu Ser Arg Pro His
   1 5 10
<210> 1299
   <211> 38
   <212> PRT
   <213> Artificial Sequence
<220>
   <223> Description of Artificial Sequence: Synthetic Peptide
<400> 1299
   His Ser Asp Gly Ile Phe Thr Asp Ser Tyr Ser Arg Tyr Arg Lys Gln
   1 5 10 15
   Met Ala Val Lys Lys Tyr Leu Ala Ala Val Leu Gly Lys Arg Tyr Lys
   20 25 30
   Gln Arg Val Lys Asn Lys
   35
<210> 1300
   <211> 30
   <212> PRT
   <213> Artificial Sequence
<220>
   <223> Description of Artificial Sequence: Synthetic Peptide
<400> 1300
   His Ser Asp Gly Ile Phe Thr Asp Ser Tyr Ser Arg Tyr Arg Arg Gln
   1 5 10 15
   Leu Ala Val Arg Arg Tyr Leu Ala Ala Val Leu Gly Lys Arg
   20 25 30
<210> 1301
   <211> 21
   <212> PRT
   <213> Artificial Sequence
<220>
   <223> Description of Artificial Sequence: Synthetic Peptide
<400> 1301
   Phe Thr Asp Ser Tyr Ser Arg Tyr Arg Lys Met Ala Val Lys Lys Tyr
   1 5 10 15
   Leu Ala Ala Val Leu
   20
<210> 1302
   <211> 38
   <212> PRT
   <213> Artificial Sequence
<220>
   <223> Description of Artificial Sequence: Synthetic Peptide
<400> 1302
   His Ser Asp Gly Ile Phe Thr Asp Ser Tyr Ser Arg Tyr Arg Lys Gln
   1 5 10 15
   Met Ala Val Lys Lys Tyr Leu Ala Ala Val Leu Gly Lys Arg Tyr Lys
   20 25 30
   Gln Arg Ile Lys Asn Lys
   35
<210> 1303
   <211> 27
   <212> PRT
   <213> Artificial Sequence
<220>
   <223> Description of Artificial Sequence: Synthetic Peptide
<400> 1303
   His Ser Asp Gly Ile Phe Thr Asp Ser Tyr Ser Arg Tyr Arg Lys Gln
   1 5 10 15
   Met Ala Val Lys Lys Tyr Leu Ala Ala Val Leu
   20 25
<210> 1304
   <211> 22
   <212> PRT
   <213> Artificial Sequence
<220>
   <223> Description of Artificial Sequence: Synthetic Peptide
<400> 1304
   Phe Thr Asp Ser Tyr Ser Arg Tyr Arg Lys Gln Met Ala Val Lys Lys
   1 5 10 15
   Tyr Leu Ala Ala Val Leu
   20
<210> 1305
   <211> 38
   <212> PRT
   <213> Artificial Sequence
<220>
   <223> Description of Artificial Sequence: Synthetic Peptide
<400> 1305
   His Ser Asp Gly Ile Phe Thr Asp Ser Tyr Ser Arg Tyr Arg Lys Gln
   1 5 10 15
   Met Ala Val Lys Lys Tyr Leu Ala Ala Val Leu Gly Lys Arg Tyr Lys
   20 25 30
   Gln Arg Val Lys Asn Lys
   35
<210> 1306
   <211> 33
   <212> PRT
   <213> Artificial Sequence
<220>
   <223> Description of Artificial Sequence: Synthetic Peptide
<400> 1306
   Phe Thr Asp Ser Tyr Ser Arg Tyr Arg Lys Gln Met Ala Val Lys Lys
   1 5 10 15
   Tyr Leu Ala Ala Val Leu Gly Lys Arg Tyr Lys Gln Arg Val Lys Asn
   20 25 30
   Lys
<210> 1307
   <211> 23
   <212> PRT
   <213> Artificial Sequence
<220>
   <223> Description of Artificial Sequence: Synthetic Peptide
<400> 1307
   Gln Met Ala Val Lys Lys Tyr Leu Ala Ala Val Leu Gly Lys Arg Tyr
   1 5 10 15
   Lys Gln Arg Val Lys Asn Lys
   20
<210> 1308
   <211> 11
   <212> PRT
   <213> Artificial Sequence
<220>
   <223> Description of Artificial Sequence: Synthetic Peptide
<400> 1308
   Gly Lys Arg Tyr Lys Gln Arg Val Lys Asn Lys
   1 5 10
<210> 1309
   <211> 8
   <212> PRT
   <213> Artificial Sequence
<220>
   <223> Description of Artificial Sequence: Synthetic Peptide
<400> 1309
   Tyr Lys Gln Arg Val Lys Asn Lys
   1 5
<210> 1310
   <211> 29
   <212> PRT
   <213> Artificial Sequence
<220>
   <223> Description of Artificial Sequence: Synthetic Peptide
<400> 1310
   Asp Val Ala His Gly Ile Leu Asn Glu Ala Tyr Arg Lys Val Leu Asp
   1 5 10 15
   Gln Leu Ser Ala Gly Lys His Leu Gln Ser Leu Val Ala
   20 25
<210> 1311
   <211> 29
   <212> PRT
   <213> Artificial Sequence
<220>
   <223> Description of Artificial Sequence: Synthetic Peptide
<400> 1311
   Asp Val Ala His Glu Ile Leu Asn Glu Ala Tyr Arg Lys Val Leu Asp
   1 5 10 15
   Gln Leu Ser Ala Arg Lys Tyr Leu Gln Ser Met Val Ala
   20 25
<210> 1312
   <211> 20
   <212> PRT
   <213> Artificial Sequence
<220>
   <223> Description of Artificial Sequence: Synthetic Peptide
<400> 1312
   Ser Asp Glu Asp Ser Asp Gly Asp Arg Pro Gln Ala Ser Pro Gly Leu
   1 5 10 15
   Gly Pro Gly Pro
   20
<210> 1313
   <211> 52
   <212> PRT
   <213> Artificial Sequence
<220>
   <223> Description of Artificial Sequence: Synthetic Peptide
<400> 1313
   Gly Glu Ser Arg Ser Glu Ala Leu Ala Val Asp Gly Ala Gly Lys Pro
   1 5 10 15
   Gly Ala Glu Glu Ala Gln Asp Pro Glu Gly Lys Gly Glu Gln Glu His
   20 25 30
   Ser Gln Gln Lys Glu Glu Glu Glu Glu Met Ala Val Val Pro Gln Gly
   35 40 45
   Leu Phe Arg Gly
   50
<210> 1314
   <211> 29
   <212> PRT
   <213> Artificial Sequence
<220>
   <223> Description of Artificial Sequence: Synthetic Peptide
<400> 1314
   Pro Glu Gly Lys Gly Glu Gln Glu His Ser Gln Gln Lys Glu Glu Glu
   1 5 10 15
   Glu Glu Met Ala Val Val Pro Gln Gly Leu Phe Arg Gly
   20 25
<210> 1315
   <211> 16
   <212> PRT
   <213> Artificial Sequence
<220>
   <223> Description of Artificial Sequence: Synthetic Peptide
<400> 1315
   Glu Glu Glu Glu Glu Met Ala Val Val Pro Gln Gly Leu Phe Arg Gly
   1 5 10 15
<210> 1316
   <211> 49
   <212> PRT
   <213> Artificial Sequence
<220>
   <223> Description of Artificial Sequence: Synthetic Peptide
<400> 1316
   Gly Trp Pro Gln Ala Pro Ala Met Asp Gly Ala Gly Lys Thr Gly Ala
   1 5 10 15
   Glu Glu Ala Gln Pro Pro Glu Gly Lys Gly Ala Arg Glu His Ser Arg
   20 25 30
   Gln Glu Glu Glu Glu Glu Thr Ala Gly Ala Pro Gln Gly Leu Phe Arg
   35 40 45
   Gly
<210> 1317
   <211> 48
   <212> PRT
   <213> Artificial Sequence
<220>
   <223> Description of Artificial Sequence: Synthetic Peptide
<400> 1317
   Gly Trp Pro Gln Ala Pro Ala Asp Gly Ala Gly Lys Thr Gly Ala Glu
   1 5 10 15
   Glu Ala Gln Pro Pro Glu Gly Lys Gly Ala Arg Glu His Ser Arg Gln
   20 25 30
   Glu Glu Glu Glu Glu Thr Ala Gly Ala Pro Gln Gly Leu Phe Arg Gly
   35 40 45
<210> 1318
   <211> 49
   <212> PRT
   <213> Artificial Sequence
<220>
   <223> Description of Artificial Sequence: Synthetic Peptide
<400> 1318
   Tyr Gly Trp Pro Gln Ala Pro Ala Asp Gly Ala Gly Lys Thr Gly Ala
   1 5 10 15
   Glu Glu Ala Gln Pro Pro Glu Gly Lys Gly Ala Arg Glu His Ser Arg
   20 25 30
   Gln Glu Glu Glu Glu Glu Thr Ala Gly Ala Pro Gln Gly Leu Phe Arg
   35 40 45
   Gly
<210> 1319
   <211> 50
   <212> PRT
   <213> Artificial Sequence
<220>
   <223> Description of Artificial Sequence: Synthetic Peptide
<400> 1319
   Tyr Gly Trp Pro Gln Ala Pro Ala Met Asp Gly Ala Gly Lys Thr Gly
   1 5 10 15
   Ala Glu Glu Ala Gln Pro Pro Glu Gly Lys Gly Ala Arg Glu His Ser
   20 25 30
   Arg Gln Glu Glu Glu Glu Glu Thr Ala Gly Ala Pro Gln Gly Leu Phe
   35 40 45
   Arg Gly
   50
<210> 1320
   <211> 19
   <212> PRT
   <213> Artificial Sequence
<220>
   <223> Description of Artificial Sequence: Synthetic Peptide
<400> 1320
   Leu Ser Phe Arg Ala Pro Ala Tyr Gly Phe Arg Gly Pro Gly Leu Gln
   1 5 10 15
   Leu Arg Arg
<210> 1321
   <211> 51
   <212> PRT
   <213> Artificial Sequence
<220>
   <223> Description of Artificial Sequence: Synthetic Peptide
<400> 1321
   Asp Asp Gly Gln Ser Glu Ser Gln Ala Val Asn Gly Lys Thr Gly Ala
   1 5 10 15
   Ser Glu Ala Val Pro Ser Glu Gly Lys Gly Glu Leu Glu His Ser Gln
   20 25 30
   Gln Glu Glu Asp Gly Glu Glu Ala Met Ala Gly Pro Pro Gln Gly Leu
   35 40 45
   Phe Pro Gly
   50
<210> 1322
   <211> 52
   <212> PRT
   <213> Artificial Sequence
<220>
   <223> Description of Artificial Sequence: Synthetic Peptide
<400> 1322
   Tyr Asp Asp Gly Gln Ser Glu Ser Gln Ala Val Asn Gly Lys Thr Gly
   1 5 10 15
   Ala Ser Glu Ala Val Pro Ser Glu Gly Lys Gly Glu Leu Glu His Ser
   20 25 30
   Gln Gln Glu Glu Asp Gly Glu Glu Ala Met Ala Gly Pro Pro Gln Gly
   35 40 45
   Leu Phe Pro Gly
   50
<210> 1323
   <211> 26
   <212> PRT
   <213> Artificial Sequence
<220>
   <223> Description of Artificial Sequence: Synthetic Peptide
<400> 1323
   Gly Glu Leu Glu His Ser Gln Gln Glu Glu Asp Gly Glu Glu Ala Met
   1 5 10 15
   Ala Gly Pro Pro Gln Gly Leu Phe Pro Gly
   20 25
<210> 1324
   <211> 17
   <212> PRT
   <213> Artificial Sequence
<220>
   <223> Description of Artificial Sequence: Synthetic Peptide
<400> 1324
   Gln Glu Glu Glu Glu Glu Thr Ala Gly Ala Pro Gln Gly Leu Phe Arg
   1 5 10 15
   Gly
<210> 1325
   <211> 2
   <212> PRT
   <213> Artificial Sequence
<220>
   <223> Description of Artificial Sequence: Synthetic Peptide
<400> 1325
   Pro Gly
   1
<210> 1326
   <211> 1
   <212> PRT
   <213> Artificial Sequence
<220>
   <223> Description of Artificial Sequence: Synthetic Peptide
<400> 1326
   Lys
   1
<210> 1327
   <211> 4
   <212> PRT
   <213> Artificial Sequence
<220>
   <223> Description of Artificial Sequence: Synthetic Peptide
<400> 1327
   Gly Phe Ala Asp
   1
<210> 1328
   <211> 6
   <212> PRT
   <213> Artificial Sequence
<220>
   <223> Description of Artificial Sequence: Synthetic Peptide
<400> 1328
   Asp Tyr Val Pro Met Leu
   1 5
<210> 1329
   <211> 6
   <212> PRT
   <213> Artificial Sequence
<220>
   <223> Description of Artificial Sequence: Synthetic Peptide
<400> 1329
   Asp Tyr Val Pro Met Leu
   1 5
<210> 1330
   <211> 11
   <212> PRT
   <213> Artificial Sequence
<220>
   <223> Description of Artificial Sequence: Synthetic Peptide
<400> 1330
   Gln Lys Arg Pro Ser Gln Arg Ser Lys Tyr Leu
   1 5 10
<210> 1331
   <211> 12
   <212> PRT
   <213> Artificial Sequence
<220>
   <223> Description of Artificial Sequence: Synthetic Peptide
<400> 1331
   Pro Leu Ser Arg Thr Leu Ser Val Ala Ala Lys Lys
   1 5 10
<210> 1332
   <211> 14
   <212> PRT
   <213> Artificial Sequence
<220>
   <223> Description of Artificial Sequence: Synthetic Peptide
<400> 1332
   Ala Arg Arg Pro Glu Gly Arg Thr Trp Ala Gln Pro Gly Tyr
   1 5 10
<210> 1333
   <211> 6
   <212> PRT
   <213> Artificial Sequence
<220>
   <223> Description of Artificial Sequence: Synthetic Peptide
<400> 1333
   Arg Gly Tyr Ser Leu Gly
   1 5
<210> 1334
   <211> 13
   <212> PRT
   <213> Artificial Sequence
<220>
   <223> Description of Artificial Sequence: Synthetic Peptide
<400> 1334
   Lys Lys Ala Leu Arg Arg Gln Glu Ala Val Asp Ala Leu
   1 5 10
<210> 1335
   <211> 25
   <212> PRT
   <213> Artificial Sequence
<220>
   <223> Description of Artificial Sequence: Synthetic Peptide
<400> 1335
   Ile Thr Ser Phe Glu Glu Ala Lys Gly Leu Asp Arg Ile Asn Glu Arg
   1 5 10 15
   Met Pro Pro Arg Arg Asp Ala Met Pro
   20 25
<210> 1336
   <211> 20
   <212> PRT
   <213> Artificial Sequence
<220>
   <223> Description of Artificial Sequence: Synthetic Peptide
<400> 1336
   Leu Lys Lys Phe Asn Ala Arg Arg Lys Leu Lys Gly Ala Ile Leu Thr
   1 5 10 15
   Thr Met Leu Ala
   20
<210> 1337
   <211> 10
   <212> PRT
   <213> Artificial Sequence
<220>
   <223> Description of Artificial Sequence: Synthetic Peptide
<400> 1337
   Pro Leu Ser Arg Thr Leu Ser Val Ser Ser
   1 5 10
<210> 1338
   <211> 10
   <212> PRT
   <213> Artificial Sequence
<220>
   <223> Description of Artificial Sequence: Synthetic Peptide
<400> 1338
   Pro Leu Arg Arg Thr Leu Ser Val Ala Ala
   1 5 10
<210> 1339
   <211> 17
   <212> PRT
   <213> Artificial Sequence
<220>
   <223> Description of Artificial Sequence: Synthetic Peptide
<400> 1339
   Leu Gln Asn Arg Arg Gly Leu Asp Leu Leu Phe Leu Lys Glu Gly Gly
   1 5 10 15
   Leu
<210> 1340
   <211> 8
   <212> PRT
   <213> Artificial Sequence
<220>
   <223> Description of Artificial Sequence: Synthetic Peptide
<400> 1340
   Cys Leu Arg Arg Ala Ser Leu Gly
   1 5
<210> 1341
   <211> 7
   <212> PRT
   <213> Artificial Sequence
<220>
   <223> Description of Artificial Sequence: Synthetic Peptide
<400> 1341
   Phe Lys Lys Ser Phe Lys Leu
   1 5
<210> 1342
   <211> 13
   <212> PRT
   <213> Artificial Sequence
<220>
   <223> Description of Artificial Sequence: Synthetic Peptide
<400> 1342
   Lys Lys Ala Leu His Arg Gln Glu Thr Val Asp Ala Leu
   1 5 10
<210> 1343
   <211> 6
   <212> PRT
   <213> Artificial Sequence
<220>
   <223> Description of Artificial Sequence: Synthetic Peptide
<400> 1343
   Lys Arg Thr Leu Arg Arg
   1 5
<210> 1344
   <211> 14
   <212> PRT
   <213> Artificial Sequence
<220>
   <223> Description of Artificial Sequence: Synthetic Peptide
<400> 1344
   Arg Thr Lys Arg Ser Gly Ser Val Tyr Glu Pro Leu Lys Ile
   1 5 10
<210> 1345
   <211> 26
   <212> PRT
   <213> Artificial Sequence
<220>
   <223> Description of Artificial Sequence: Synthetic Peptide
<400> 1345
   Gly Ile Gly Ala Val Leu Lys Val Leu Thr Thr Gly Leu Pro Ala Leu
   1 5 10 15
   Ile Ser Trp Ile Lys Arg Lys Arg Gln Gln
   20 25
<210> 1346
   <211> 9
   <212> PRT
   <213> Artificial Sequence
<220>
   <223> Description of Artificial Sequence: Synthetic Peptide
<400> 1346
   Arg Arg Lys Ala Ser Gly Pro Pro Val
   1 5
<210> 1347
   <211> 17
   <212> PRT
   <213> Artificial Sequence
<220>
   <223> Description of Artificial Sequence: Synthetic Peptide
<400> 1347
   Arg Phe Ala Arg Lys Gly Ala Leu Arg Gln Lys Asn Val His Glu Val
   1 5 10 15
   Lys
<210> 1348
<211> 18
   <212> PRT
   <213> Artificial Sequence
<220>
   <223> Description of Artificial Sequence: Synthetic Peptide
<400> 1348
   Arg Phe Ala Arg Lys Gly Ala Leu Glu Gln Lys Asn Val His Glu Val
   1 5 10 15
   Lys Asn
<210> 1349
   <211> 14
   <212> PRT
   <213> Artificial Sequence
<220>
   <223> Description of Artificial Sequence: Synthetic Peptide
<400> 1349
   Ser Phe Val Asn Ser Glu Phe Leu Lys Pro Glu Val Lys Ser
   1 5 10
<210> 1350
   <211> 11
   <212> PRT
   <213> Artificial Sequence
<220>
   <223> Description of Artificial Sequence: Synthetic Peptide
<400> 1350
   Ser Tyr Thr Asn Pro Glu Phe Val Ile Asn Val
   1 5 10
<210> 1351
   <211> 15
   <212> PRT
   <213> Artificial Sequence
<220>
   <223> Description of Artificial Sequence: Synthetic Peptide
<400> 1351
   Ala Gly Asn Lys Val Ile Ser Pro Ser Glu Asp Arg Arg Gln Cys
   1 5 10 15
<210> 1352
   <211> 18
   <212> PRT
   <213> Artificial Sequence
<220>
   <223> Description of Artificial Sequence: Synthetic Peptide
<400> 1352
   Gly Pro Lys Thr Pro Glu Glu Lys Thr Ala Asn Thr Ile Ser Lys Phe
   1 5 10 15
   Asp Cys
<210> 1353
   <211> 29
   <212> PRT
   <213> Artificial Sequence
<220>
   <223> Description of Artificial Sequence: Synthetic Peptide
<400> 1353
   Leu Leu Tyr Glu Met Leu Ala Gly Gln Ala Pro Phe Glu Gly Glu Asp
   1 5 10 15
   Glu Asp Glu Leu Phe Gln Ser Ile Met Glu His Asn Val
   20 25
<210> 1354
   <211> 14
   <212> PRT
   <213> Artificial Sequence
<220>
   <223> Description of Artificial Sequence: Synthetic Peptide
<400> 1354
   Asn Tyr Pro Leu Glu Leu Tyr Glu Arg Val Arg Thr Gly Cys
   1 5 10
<210> 1355
   <211> 9
   <212> PRT
   <213> Artificial Sequence
<220>
   <223> Description of Artificial Sequence: Synthetic Peptide
<400> 1355
   Val Arg Lys Arg Thr Leu Arg Arg Leu
   1 5
<210> 1356
   <211> 8
   <212> PRT
   <213> Artificial Sequence
<220>
   <223> Description of Artificial Sequence: Synthetic Peptide
<400> 1356
   Cys Asp Asn Gln Ile Lys Lys Met
   1 5
<210> 1357
   <211> 5
   <212> PRT
   <213> Artificial Sequence
<220>
   <223> Description of Artificial Sequence: Synthetic Peptide
<400> 1357
   Ile Tyr Gly Glu Phe
   1 5
<210> 1358
   <211> 5
   <212> PRT
   <213> Artificial Sequence
<220>
   <223> Description of Artificial Sequence: Synthetic Peptide
<400> 1358
   Ile Tyr Gly Glu Phe
   1 5
<210> 1359
   <211> 17
   <212> PRT
   <213> Artificial Sequence
<220>
   <223> Description of Artificial Sequence: Synthetic Peptide
<400> 1359
   Thr Tyr Ala Asp Phe Ile Ala Ser Gly Arg Thr Gly Arg Arg Asn Ala
   1 5 10 15
   Ile
<210> 1360
   <211> 11
   <212> PRT
   <213> Artificial Sequence
<220>
   <223> Description of Artificial Sequence: Synthetic Peptide
<400> 1360
   Gln Lys Arg Pro Ser Gln Arg Ser Lys Tyr Leu
   1 5 10
<210> 1361
   <211> 9
   <212> PRT
   <213> Artificial Sequence
<220>
   <223> Description of Artificial Sequence: Synthetic Peptide
<400> 1361
   Arg Arg Glu Glu Glu Thr Glu Glu Glu
   1 5
<210> 1362
   <211> 7
   <212> PRT
   <213> Artificial Sequence
<220>
   <223> Description of Artificial Sequence: Synthetic Peptide
<400> 1362
   Arg Arg Lys Ala Ser Gly Pro
   1 5
<210> 1363
   <211> 13
   <212> PRT
   <213> Artificial Sequence
<220>
   <223> Description of Artificial Sequence: Synthetic Peptide
<400> 1363
   Arg Arg Leu Ile Glu Asp Asn Glu Tyr Thr Ala Arg Gly
   1 5 10
<210> 1364
   <211> 15
   <212> PRT
   <213> Artificial Sequence
<220>
   <223> Description of Artificial Sequence: Synthetic Peptide
<400> 1364
   Pro Leu Ala Arg Thr Leu Ser Val Ala Gly Leu Pro Gly Lys Lys
   1 5 10 15
<210> 1365
   <211> 32
   <212> PRT
   <213> Artificial Sequence
<220>
   <223> Description of Artificial Sequence: Synthetic Peptide
<400> 1365
   Lys Glu Ala Lys Glu Lys Arg Gln Glu Gln Ile Ala Lys Arg Arg Arg
   1 5 10 15
   Leu Ser Ser Leu Arg Ala Ser Thr Ser Lys Ser Gly Gly Ser Gln Lys
   20 25 30
<210> 1366 .
   <211> 20
   <212> PRT
   <213> Artificial Sequence
<220>
   <223> Description of Artificial Sequence: Synthetic Peptide
<400> 1366
   Tyr Ser Phe Val His His Gly Phe Phe Asn Phe Arg Val Ser Trp Arg
   1 5 10 15
   Glu Met Leu Ala
   20
<210> 1367
   <211> 21
   <212> PRT
   <213> Artificial Sequence
<220>
   <223> Description of Artificial Sequence: Synthetic Peptide
<400> 1367
   Val Ala Pro Ser Asp Ser Ile Gln Ala Glu Glu Trp Tyr Phe Gly Lys
   1 5 10 15
   Ile Thr Arg Arg Glu
   20
<210> 1368
   <211> 6
   <212> PRT
   <213> Artificial Sequence
<220>
   <223> Description of Artificial Sequence: Synthetic Peptide
<400> 1368
   His Arg His Phe Leu Arg
   1 5
<210> 1369
   <211> 25
   <212> PRT
   <213> Artificial Sequence
<220>
   <223> Description of Artificial Sequence: Synthetic Peptide
<400> 1369
<210> 1370
   <211> 14
   <212> PRT
   <213> Artificial Sequence
<220>
   <223> Description of Artificial Sequence: Synthetic Peptide
<400> 1370
   Met Tyr Ser Asn Val Ile Gly Thr Val Thr Ser Gly Lys Arg
   1 5 10

<210> 1371
   <211> 25
   <212> PRT
   <213> Artificial Sequence
<220>
   <223> Description of Artificial Sequence: Synthetic Peptide
<400> 1371
   Phe Tyr Lys Ala Asp Gly Val Val Phe Ser Ile Tyr Asp Val Pro Gly
   1               5                   10                  15
   Arg Gln Val Pro Leu Ser Ala Arg Gly
               20                  25
<210> 1372
   <211> 20
   <212> PRT
   <213> Artificial Sequence
<220>
   <223> Description of Artificial Sequence: Synthetic Peptide
<400> 1372
   Trp Ser Pro Gln Glu Glu Asp Arg Ile Ile Glu Gly Gly Ile Tyr Asp
   1               5                   10                  15
   Ala Asp Leu Asn
               20
<210> 1373
   <211> 20
   <212> PRT
   <213> Artificial Sequence
<220>
   <223> Description of Artificial Sequence: Synthetic Peptide
<400> 1373
<210> 1374
   <211> 11
   <212> PRT
   <213> Artificial Sequence
<220>
   <223> Description of Artificial Sequence: Synthetic Peptide
<400> 1374
   Cys Ser Pro Cys His Ala Met Lys Met Asn Ile
   1 5 10
<210> 1375
   <211> 27
   <212> PRT
   <213> Artificial Sequence
<220>
   <223> Description of Artificial Sequence: Synthetic Peptide
<400> 1375
   Glu Val Ser Phe Leu Asn Cys Ser Leu Asp Asn Gly Gly Cys Thr Pro
   1 5 10 15
   Leu Leu Pro Arg Gly Gly Gly Leu Ala Ala Leu
   20 25
<210> 1376
   <211> 33
   <212> PRT
   <213> Artificial Sequence
<220>
   <223> Description of Artificial Sequence: Synthetic Peptide
<400> 1376
   Met Ala Pro Glu Glu Ile Ile Met Asp Arg Pro Phe Leu Phe Val Val
   1 5 10 15
   Arg His Asn Pro Thr Gly Thr Val Leu Phe Met Gly Gln Val Met Glu
   20 25 30
   Pro
<210> 1377
   <211> 36
   <212> PRT
   <213> Artificial Sequence
<220>
   <223> Description of Artificial Sequence: Synthetic Peptide
<400> 1377
   Cys Glu Asn Gly Gly Phe Cys Ser Gly Val Cys His Asn Leu Pro Gly
   1 5 10 15
   Thr Phe Glu Cys Ile Cys Gly Pro Asp Ser Ala Leu Val Arg His Ile
   20 25 30
   Gly Thr Asp Cys
   35
<210> 1378
   <211> 24
   <212> PRT
   <213> Artificial Sequence
<220>
   <223> Description of Artificial Sequence: Synthetic Peptide
<400> 1378
   His Pro Pro Cys Cys Leu Tyr Gly Lys Cys Arg Arg Tyr Pro Gly Cys
   1 5 10 15
   Ser Ser Ala Ser Cys Cys Gln Leu
   20
   <210> 1379
   <211> 48
   <212> PRT
   <213> Artificial Sequence
<220>
   <223> Description of Artificial Sequence: Synthetic Peptide
<400> 1379
   Glu Asp Asn Cys Ile Ala Glu Asp Tyr Gly Lys Cys Thr Trp Gly Gly
   1 5 10 15
   Thr Lys Cys Cys Arg Gly Arg Pro Cys Arg Cys Ser Met Ile Gly Thr
   20 25 30
   Asn Cys Glu Cys Thr Pro Arg Leu Ile Met Glu Gly Leu Ser Phe Ala
   35 40 45
<210> 1380
   <211> 35
   <212> PRT
   <213> Artificial Sequence
<220>
   <223> Description of Artificial Sequence: Synthetic Peptide
<400> 1380
   Gly Gly Cys Leu Pro His Asn Arg Phe Cys Asn Ala Leu Ser Gly Pro
   1 5 10 15
   Arg Cys Cys Ser Gly Leu Lys Cys Lys Glu Leu Ser Ile Trp Asp Ser
   20 25 30
   Arg Cys Leu
   35
<210> 1381
   <211> 18
   <212> PRT
   <213> Artificial Sequence
<220>
   <223> Description of Artificial Sequence: Synthetic Peptide
<400> 1381
   Cys Asn Cys Lys Ala Pro Glu Thr Ala Leu Cys Ala Arg Arg Cys Gln
   1 5 10 15
   Gln His
<210> 1382
   <211> 60
   <212> PRT
   <213> Artificial Sequence
<220>
   <223> Description of Artificial Sequence: Synthetic Peptide
<400> 1382
   Trp Gln Pro Pro Trp Tyr Cys Lys Glu Pro Val Arg Ile Gly Ser Cys
   1 5 10 15
   Lys Lys Gln Phe Ser Ser Phe Tyr Phe Lys Trp Thr Ala Lys Lys Cys
   20 25 30
   Leu Pro Phe Leu Phe Ser Gly Cys Gly Gly Asn Ala Asn Arg Phe Gln
   35 40 45
   Thr Ile Gly Glu Cys Arg Lys Lys Cys Leu Gly Lys
   50 55 60
<210> 1383
   <211> 60
   <212> PRT
   <213> Artificial Sequence
<220>
   <223> Description of Artificial Sequence: Synthetic Peptide
<400> 1383
   Arg Ile Cys Tyr Ile His Lys Ala Ser Leu Pro Arg Ala Thr Lys Thr
   1 5 10 15
   Cys Val Glu Asn Thr Cys Tyr Lys Met Phe Ile Arg Thr Gln Arg Glu
   20 25 30
   Tyr Ile Ser Glu Arg Gly Cys Gly Cys Pro Thr Ala Met Trp Pro Tyr
   35 40 45
   Gln Thr Glu Cys Cys Lys Gly Asp Arg Cys Asn Lys
   50 55 60
<210> 1384
   <211> 36
   <212> PRT
   <213> Artificial Sequence
<220>
   <223> Description of Artificial Sequence: Synthetic Peptide
<400> 1384
   Phe Thr Asn Val Ser Cys Thr Thr Ser Lys Glu Cys Trp Ser Val Cys
   1 5 10 15
   Gln Arg Leu His Asn Thr Ser Arg Gly Lys Cys Met Asn Lys Lys Cys
   20 25 30
   Arg Cys Tyr Ser
   35
<210> 1385
   <211> 36
   <212> PRT
   <213> Artificial Sequence
<220>
   <223> Description of Artificial Sequence: Synthetic Peptide
<400> 1385
   Met Cys Met Pro Cys Phe Thr Thr Asp His Gln Met Ala Arg Lys Cys
   1 5 10 15
   Asp Asp Cys Cys Gly Gly Lys Gly Arg Gly Lys Cys Tyr Gly Pro Gln
   20 25 30
   Cys Leu Cys Arg
   35
<210> 1386
   <211> 13
   <212> PRT
   <213> Artificial Sequence
<220>
   <223> Description of Artificial Sequence: Synthetic Peptide
<400> 1386
   Glu Cys Cys Asn Pro Ala Cys Gly Arg His Tyr Ser Cys
   1 5 10
<210> 1387
   <211> 31
   <212> PRT
   <213> Artificial Sequence
<220>
   <223> Description of Artificial Sequence: Synthetic Peptide
<400> 1387
   Ala Cys Ser Gly Arg Gly Ser Arg Cys Gln Cys Cys Met Gly Leu Arg
   1 5 10 15
   Cys Gly Arg Gly Asn Pro Gln Lys Cys Ile Gly Ala His Asp Val
   20 25 30
<210> 1388
   <211> 14
   <212> PRT
   <213> Artificial Sequence
<220>
   <223> Description of Artificial Sequence: Synthetic Peptide
<210> 1389
   <211> 17
   <212> PRT
   <213> Artificial Sequence
<220>
   <223> Description of Artificial Sequence: Synthetic Peptide
<400> 1389
   Arg Asp Cys Cys Tyr His Pro Thr Cys Asn Met Ser Asn Pro Gln Ile
   1 5 10 15
   Cys
<210> 1390
   <211> 13
   <212> PRT
   <213> Artificial Sequence
<220>
   <223> Description of Artificial Sequence: Synthetic Peptide
<400> 1390
   Tyr Cys Cys His Pro Ala Cys Gly Lys Asn Phe Asp Cys
   1 5 10
<210> 1391
   <211> 12
   <212> PRT
   <213> Artificial Sequence
<220>
   <223> Description of Artificial Sequence: Synthetic Peptide
<400> 1391
   Gly Cys Cys Ser Asp Pro Arg Cys Ala Trp Arg Cys
   1 5 10
<210> 1392
   <211> 13
   <212> PRT
   <213> Artificial Sequence
<220>
   <223> Description of Artificial Sequence: Synthetic Peptide
<400> 1392
   Ile Cys Cys Asn Pro Ala Cys Gly Pro Lys Tyr Ser Cys
   1 5 10
<210> 1393
   <211> 19
   <212> PRT
   <213> Artificial Sequence
<220>
   <223> Description of Artificial Sequence: Synthetic Peptide
<400> 1393
   Arg Asp Cys Cys Thr Arg Lys Cys Lys Asp Arg Arg Cys Lys Met Lys
   1 5 10 15
   Cys Cys Ala
<210> 1394
   <211> 24
   <212> PRT
   <213> Artificial Sequence
<220>
   <223> Description of Artificial Sequence: Synthetic Peptide
<400> 1394
   Cys Lys Ser Gly Ser Ser Cys Ser Thr Ser Tyr Asn Cys Cys Arg Ser
   1 5 10 15
   Cys Asn Tyr Thr Lys Arg Cys Tyr
   20
<210> 1395
   <211> 25
   <212> PRT
   <213> Artificial Sequence
<220>
   <223> Description of Artificial Sequence: Synthetic Peptide
<400> 1395
   Cys Lys Gly Lys Gly Ala Lys Cys Ser Arg Leu Met Tyr Asp Cys Cys
   1 5 10 15
   Thr Gly Ser Cys Arg Ser Gly Lys Cys
   20 25
<210> 1396
   <211> 26
   <212> PRT
   <213> Artificial Sequence
<220>
   <223> Description of Artificial Sequence: Synthetic Peptide
<400> 1396
   Cys Lys Gly Lys Gly Ala Pro Cys Arg Lys Thr Met Tyr Asp Cys Cys
   1 5 10 15
   Ser Gly Ser Cys Gly Arg Arg Gly Lys Cys
   20 25
<210> 1397
   <211> 26
   <212> PRT
   <213> Artificial Sequence
<220>
   <223> Description of Artificial Sequence: Synthetic Peptide
<400> 1397
   Cys Lys Leu Lys Gly Gln Ser Cys Arg Lys Thr Ser Tyr Asp Cys Cys
   1 5 10 15
   Ser Gly Ser Cys Gly Arg Ser Gly Lys Cys
   20 25
<210> 1398
   <211> 10
   <212> PRT
   <213> Artificial Sequence
<220>
   <223> Description of Artificial Sequence: Synthetic Peptide
<400> 1398
   Lys Thr Lys Cys Lys Phe Leu Lys Lys Cys
   1 5 10
<210> 1399
   <211> 19
   <212> PRT
   <213> Artificial Sequence
<220>
   <223> Description of Artificial Sequence: Synthetic Peptide
<400> 1399
   Arg Asp Cys Cys Thr Lys Lys Cys Lys Asp Arg Gln Cys Lys Gln Arg
   1 5 10 15
   Cys Cys Ala
<210> 1400
   <211> 37
   <212> PRT
   <213> Artificial Sequence
<220>
   <223> Description of Artificial Sequence: Synthetic Peptide
<400> 1400
   Glu Phe Thr Asp Val Asp Cys Ser Val Ser Lys Glu Cys Trp Ser Val
   1 5 10 15
   Cys Lys Asp Leu Phe Gly Val Asp Arg Gly Lys Cys Met Gly Lys Lys
   20 25 30
   Cys Arg Cys Tyr Gln
   35
<210> 1401
   <211> 37
   <212> PRT
   <213> Artificial Sequence
<220>
   <223> Description of Artificial Sequence: Synthetic Peptide
<400> 1401
   Gly Val Glu Ile Asn Val Lys Cys Ser Gly Ser Pro Gln Cys Leu Lys
   1 5 10 15
   Pro Cys Lys Asp Ala Gly Met Arg Phe Gly Lys Cys Met Asn Arg Lys
   20 25 30
   Cys His Cys Thr Pro
   35
<210> 1402
   <211> 37
   <212> PRT
   <213> Artificial Sequence
<220>
   <223> Description of Artificial Sequence: Synthetic Peptide
<400> 1402
   Gly Val Glu Ile Asn Val Lys Cys Ser Gly Ser Pro Gln Cys Leu Lys
   1 5 10 15
   Pro Cys Lys Asp Ala Gly Met Arg Phe Gly Lys Cys Met Asn Arg Lys
   20 25 30
   Cys His Cys Thr Pro
   35
<210> 1403
   <211> 22
   <212> PRT
   <213> Artificial Sequence
<220>
   <223> Description of Artificial Sequence: Synthetic Peptide
<400> 1403
   Ile Lys Cys Asn Cys Lys Arg His Val Ile Lys Pro His Ile Cys Arg
   1 5 10 15
   Lys Ile Cys Gly Lys Asn
   20
<210> 1404
   <211> 39
   <212> PRT
   <213> Artificial Sequence
<220>
   <223> Description of Artificial Sequence: Synthetic Peptide
<400> 1404
   Thr Ile Ile Asn Val Lys Cys Thr Ser Pro Lys Gln Cys Leu Pro Pro
   1 5 10 15
   Cys Lys Ala Gln Phe Gly Gln Ser Ala Gly Ala Lys Cys Met Asn Gly
   20 25 30
   Lys Cys Lys Cys Tyr Pro His
   35
<210> 1405
   <211> 4
   <212> PRT
   <213> Artificial Sequence
<220>
   <223> Description of Artificial Sequence: Synthetic Peptide
<400> 1405
   Phe Asp Arg Ala
   1
<210> 1406
   <211> 44
   <212> PRT
   <213> Artificial Sequence
<220>
   <223> Description of Artificial Sequence: Synthetic Peptide
<400> 1406
   Ala Asp Cys Ser Ala Thr Gly Asp Thr Cys Asp His Thr Lys Lys Cys
   1 5 10 15
   Cys Asp Asp Cys Tyr Thr Cys Arg Cys Gly Thr Pro Trp Gly Ala Asn
   20 25 30
   Cys Arg Cys Asp Tyr Tyr Lys Ala Arg Cys Asp Thr
   35 40
<210> 1407
   <211> 31
   <212> PRT
   <213> Artificial Sequence
<220>
   <223> Description of Artificial Sequence: Synthetic Peptide
<400> 1407
   Ala Phe Cys Asn Leu Arg Met Cys Gln Leu Ser Cys Arg Ser Leu Gly
   1 5 10 15
   Leu Leu Gly Lys Cys Ile Gly Asp Lys Cys Glu Cys Val Lys His
   20 25 30
<210> 1408
   <211> 35
   <212> PRT
   <213> Artificial Sequence
<220>
   <223> Description of Artificial Sequence: Synthetic Peptide
<400> 1408
   Arg Ser Cys Ile Asp Thr Ile Pro Lys Ser Arg Cys Thr Ala Phe Gln
   1 5 10 15
   Cys Lys His Ser Met Lys Tyr Arg Leu Ser Phe Cys Arg Lys Thr Cys
   20 25 30
   Gly Thr Cys
   35
<210> 1409
   <211> 14
   <212> PRT
   <213> Artificial Sequence
<220>
   <223> Description of Artificial Sequence: Synthetic Peptide
<400> 1409
   Phe Leu Pro Leu Ile Leu Gly Lys Leu Val Lys Gly Leu Leu
   1 5 10
<210> 1410
   <211> 14
   <212> PRT
   <213> Artificial Sequence
<220>
   <223> Description of Artificial Sequence: Synthetic Peptide
<400> 1410
   Ile Asn Leu Lys Ala Ile Ala Ala Leu Val Lys Lys Val Leu
   1 5 10
<210> 1411
   <211> 14
   <212> PRT
   <213> Artificial Sequence
<220>
   <223> Description of Artificial Sequence: Synthetic Peptide
<210> 1412
   <211> 14
   <212> PRT
   <213> Artificial Sequence
<220>
   <223> Description of Artificial Sequence: Synthetic Peptide
<400> 1412
   Ile Asn Leu Lys Ala Leu Ala Ala Leu Ala Lys Ala Leu Leu
   1 5 10
<210> 1413
   <211> 14
   <212> PRT
   <213> Artificial Sequence
<220>
   <223> Description of Artificial Sequence: Synthetic Peptide
<400> 1413
   Ile Asn Leu Lys Ala Leu Ala Ala Leu Ala Lys Arg Leu Leu
   1 5 10
<210> 1414
   <211> 14
   <212> PRT
   <213> Artificial Sequence
<220>
   <223> Description of Artificial Sequence: Synthetic Peptide
<400> 1414
   Ile Asn Leu Lys Ala Lys Ala Ala Leu Ala Lys Lys Leu Leu
   1 5 10
<210> 1415
   <211> 14
   <212> PRT
   <213> Artificial Sequence
<220>
   <223> Description of Artificial Sequence: Synthetic Peptide
<400> 1415
   Ile Asn Trp Lys Gly Ile Ala Ala Met Ala Lys Lys Leu Leu
   1 5 10
<210> 1416
   <211> 21
   <212> PRT
   <213> Artificial Sequence
<220>
   <223> Description of Artificial Sequence: Synthetic Peptide
<400> 1416
   Phe Met Ser Ser His Gln Ser Gln Ala Ser Leu Glu Leu Ala Ile Lys
   1 5 10 15
   Gln Trp Gly Ser Gln
   20
<210> 1417
   <211> 20
   <212> PRT
   <213> Artificial Sequence
<220>
   <223> Description of Artificial Sequence: Synthetic Peptide
<400> 1417
   Phe Asn Lys Ile Thr Pro Asn Leu Ala Glu Phe Ala Phe Ser Leu Tyr
   1 5 10 15
   Arg Gln Leu Ala
   20
<210> 1418
   <211> 34
   <212> PRT
   <213> Artificial Sequence
<220>
   <223> Description of Artificial Sequence: Synthetic Peptide
<400> 1418
   Trp Ser Pro Lys Glu Glu Asp Arg Ile Ile Pro Gly Gly Ile Tyr Asn
   1 5 10 15
   Ala Asp Leu Asn Asp Glu Trp Val Gln Arg Ala Leu His Phe Ala Ile
   20 25 30
   Ser Glu
<210> 1419
   <211> 36
   <212> PRT
   <213> Artificial Sequence
<220>
   <223> Description of Artificial Sequence: Synthetic Peptide
<400> 1419
   Tyr Thr Ser Leu Ile His Ser Leu Ile Glu Glu Ser Gln Asn Gln Gln
   1 5 10 15
   Glu Lys Asn Glu Gln Glu Leu Leu Glu Leu Asp Lys Trp Ala Ser Leu
   20 25 30
   Trp Asn Trp Phe
   35
<210> 1420
   <211> 38
   <212> PRT
   <213> Artificial Sequence
<220>
   <223> Description of Artificial Sequence: Synthetic Peptide
<400> 1420
   Asn Asn Leu Leu Arg Ala Ile Glu Ala Gln Gln His Leu Leu Gln Leu
   1 5 10 15
   Thr Val Trp Gln Ile Lys Gln Leu Gln Ala Arg Ile Leu Ala Val Glu
   20 25 30
   Arg Tyr Leu Lys Asp Gln
   35
<210> 1421
   <211> 36
   <212> PRT
   <213> Artificial Sequence
<220>
   <223> Description of Artificial Sequence: Synthetic Peptide
<400> 1421
   Tyr Thr Asn Thr Ile Tyr Thr Leu Leu Glu Glu Ser Gln Asn Gln Gln
   1 5 10 15
   Glu Lys Asn Glu Gln Glu Leu Leu Glu Leu Asp Lys Trp Ala Ser Leu
   20 25 30
   Trp Asn Trp Phe
   35
<210> 1422
   <211> 36
   <212> PRT
   <213> Artificial Sequence
<220>
   <223> Description of Artificial Sequence: Synthetic Peptide
<400> 1422
   Tyr Thr Gly Ile Ile Tyr Asn Leu Leu Glu Glu Ser Gln Asn Gln Gln
   1 5 10 15
   Glu Lys Asn Glu Gln Glu Leu Leu Glu Leu Asp Lys Trp Ala Asn Leu
   20 25 30
   Trp Asn Trp Phe
   35
<210> 1423
   <211> 36
   <212> PRT
   <213> Artificial Sequence
<220>
   <223> Description of Artificial Sequence: Synthetic Peptide
<400> 1423
   Tyr Thr Ser Leu Ile Tyr Ser Leu Leu Glu Lys Ser Gln Thr Gln Gln
   1 5 10 15
   Glu Lys Asn Glu Gln Glu Leu Leu Glu Leu Asp Lys Trp Ala Ser Leu
   20 25 30
   Trp Asn Trp Phe
   35
<210> 1424
   <211> 36
   <212> PRT
   <213> Artificial Sequence
<220>
   <223> Description of Artificial Sequence: Synthetic Peptide
<400> 1424
   Leu Glu Ala Asn Ile Ser Lys Ser Leu Glu Gln Ala Gln Ile Gln Gln
   1 5 10 15
   Glu Lys Asn Met Tyr Glu Leu Gln Lys Leu Asn Ser Trp Asp Ile Phe
   20 25 30
   Gly Asn Trp Phe
   35
<210> 1425
   <211> 36
   <212> PRT
   <213> Artificial Sequence
<220>
   <223> Description of Artificial Sequence: Synthetic Peptide
<400> 1425
   Leu Glu Ala Asn Ile Ser Gln Ser Leu Glu Gln Ala Gln Ile Gln Gln
   1 5 10 15
   Glu Lys Asn Met Tyr Glu Leu Gln Lys Leu Asn Ser Trp Asp Val Phe
   20 25 30
   Thr Asn Trp Leu
   35
<210> 1426
   <211> 41
   <212> PRT
   <213> Artificial Sequence
<220>
   <223> Description of Artificial Sequence: Synthetic Peptide
<400> 1426
   Cys Gly Gly Asn Asn Leu Leu Arg Ala Ile Glu Ala Gln Gln His Leu
   1 5 10 15
   Leu Gln Leu Thr Val Trp Gly Ile Lys Gln Leu Gln Ala Arg Ile Leu
   20 25 30
   Ala Val Glu Arg Tyr Leu Lys Asp Gln
   35 40
<210> 1427
   <211> 38
   <212> PRT
   <213> Artificial Sequence
<220>
   <223> Description of Artificial Sequence: Synthetic Peptide
<400> 1427
   Gln Gln Leu Leu Asp Val Val Lys Arg Gln Gln Glu Met Leu Arg Leu
   1 5 10 15
   Thr Val Trp Gly Thr Lys Asn Leu Gln Ala Arg Val Thr Ala Ile Glu
   20 25 30
   Lys Tyr Leu Lys Asp Gln
   35
<210> 1428
   <211> 46
   <212> PRT
   <213> Artificial Sequence
<220>
   <223> Description of Artificial Sequence: Synthetic Peptide
<400> 1428
   Tyr Thr Ser Val Ile Thr Ile Glu Leu Ser Asn Ile Lys Glu Asn Lys
   1 5 10 15
   Cys Asn Gly Ala Lys Val Lys Leu Ile Lys Gln Glu Leu Asp Lys Tyr
   20 25 30
   Lys Asn Ala Val Thr Glu Leu Gln Leu Leu Met Gln Ser Thr
   35 40 45
<210> 1429
   <211> 54
   <212> PRT
   <213> Artificial Sequence
<22u>
   <223> Description of Artificial Sequence: Synthetic Peptide
<400> 1429
   Ala Ser Gly Val Ala Val Ser Lys Val Leu His Leu Glu Gly Glu Val
   1 5 10 15
   Asn Lys Ile Ala Leu Leu Ser Thr Asn Lys Ala Val Val Ser Leu Ser
   20 25 30
   Asn Gly Val Ser Val Leu Thr Ser Lys Val Leu Asp Leu Lys Asn Tyr
   35 40 45
   Ile Asp Lys Gln Leu Leu
   50
<210> 1430
   <211> 53
   <212> PRT
   <213> Artificial Sequence
<220>
   <223> Description of Artificial Sequence: Synthetic Peptide
<400> 1430
   Gly Glu Pro Ile Ile Asn Phe Tyr Asp Pro Leu Val Phe Pro Ser Asp
   1 5 10 15
   Glu Phe Asp Ala Ser Ile Ser Gln Val Asn Glu Lys Ile Asn Gln Ser
   20 25 30
   Leu Ala Phe Ile Arg Lys Ser Asp Glu Leu Leu His Asn Val Asn Ala
   35 40 45
   Gly Lys Ser Thr Thr
   50
<210> 1431
   <211> 48
   <212> PRT
   <213> Artificial Sequence
<220>
   <223> Description of Artificial Sequence: Synthetic Peptide
<400> 1431
   Tyr Thr Ser Val Ile Thr Ile Glu Leu Ser Asn Ile Lys Glu Asn Lys
   1 5 10 15
   Cys Asn Gly Thr Asp Ala Lys Val Lys Leu Ile Lys Gln Glu Leu Asp
   20 25 30
   Lys Tyr Lys Asn Ala Val Thr Glu Leu Gln Leu Leu Met Gln Ser Thr
   35 40 45
<210> 1432
   <211> 34
   <212> PRT
   <213> Artificial Sequence
<220>
   <223> Description of Artificial Sequence: Synthetic Peptide
<400> 1432
   Tyr Thr Ser Val Ile Thr Ile Glu Leu Ser Asn Ile Lys Glu Asn Lys
   1 5 10 15
   Cys Asn Gly Asp Ala Lys Val Lys Leu Ile Lys Gln Glu Leu Asp Lys
   20 25 30
   Tyr Lys
<210> 1433
   <211> 34
   <212> PRT
   <213> Artificial Sequence
<220>
   <223> Description of Artificial Sequence: Synthetic Peptide
<400> 1433
   Thr Ser Val Ile Thr Ile Glu Leu Ser Asn Ile Lys Glu Asn Lys Cys
   1 5 10 15
   Asn Gly Asp Ala Lys Val Lys Leu Ile Lys Gln Glu Leu Asp Lys Tyr
   20 25 30
   Lys Asn
<210> 1434
   <211> 34
   <212> PRT
   <213> Artificial Sequence
<220>
   <223> Description of Artificial Sequence: Synthetic Peptide
<400> 1434
   Val Ile Thr Ile Glu Leu Ser Asn Ile Lys Glu Asn Lys Cys Asn Gly
   1 5 10 15
   Asp Ala Lys Val Lys Leu Ile Lys Gln Glu Leu Asp Lys Tyr Lys Asn
   20 25 30
   Ala Val
<210> 1435
   <211> 34
   <212> PRT
   <213> Artificial Sequence
<220>
   <223> Description of Artificial Sequence: Synthetic Peptide
<400> 1435
   Val Ile Thr Ile Glu Leu Ser Asn Ile Lys Glu Asn Lys Met Asn Gly
   1 5 10 15
   Asp Ala Lys Val Lys Leu Ile Lys Gln Glu Leu Asp Lys Tyr Lys Asn
   20 25 30
   Ala Val
<210> 1436
   <211> 33
   <212> PRT
   <213> Artificial Sequence
<220>
   <223> Description of Artificial Sequence: Synthetic Peptide
<400> 1436
   Val Ala Val Ser Lys Val Leu His Leu Glu Gly Glu Val Asn Lys Ile
   1 5 10 15
   Ala Leu Leu Ser Thr Asn Lys Ala Val Val Ser Leu Ser Asn Gly Val
   20 25 30
   Ser

<210> 1437
   <211> 33
   <212> PRT
   <213> Artificial Sequence
<220>
   <223> Description of Artificial Sequence: Synthetic Peptide
<400> 1437
   Ala Val Ser Lys Val Leu His Leu Glu Gly Glu Val Asn Lys Ile Ala
   1 5 10 15
   Leu Leu Ser Thr Asn Lys Ala Val Val Ser Leu Ser Asn Gly Val Ser
   20 25 30
   Val
<210> 1438
   <211> 33
   <212> PRT
   <213> Artificial Sequence
<220>
   <223> Description of Artificial Sequence: Synthetic Peptide
<400> 1438
   Val Ser Lys Val Leu His Leu Glu Gly Glu Val Asn Lys Ile Ala Leu
   1 5 10 15
   Leu Ser Thr Asn Lys Ala Val Val Ser Leu Ser Asn Gly Val Ser Val
   20 25 30
   Leu
<210> 1439
   <211> 33
   <212> PRT
   <213> Artificial Sequence
<220>
   <223> Description of Artificial Sequence: Synthetic Peptide
<400> 1439
   Ser Lys Val Leu His Leu Glu Gly Glu Val Asn Lys Ile Ala Leu Leu
   1 5 10 15
   Ser Thr Asn Lys Ala Val Val Ser Leu Ser Asn Gly Val Ser Val Leu
   20 25 30
   Thr
<210> 1440
   <211> 33
   <212> PRT
   <213> Artificial Sequence
<220>
   <223> Description of Artificial Sequence: Synthetic Peptide
<400> 1440
   Lys Val Leu His Leu Glu Gly Glu Val Asn Lys Ile Ala Leu Leu Ser
   1 5 10 15
   Thr Asn Lys Ala Val Val Ser Leu Ser Asn Gly Val Ser Val Leu Thr
   20 25 30
   Ser
<210> 1441
   <211> 33
   <212> PRT
   <213> Artificial Sequence
<220>
   <223> Description of Artificial sequence: Synthetic Peptide
<400> 1441
   Leu Glu Gly Glu Val Asn Lys Ile Ala Leu Leu Ser Thr Asn Lys Ala
   1 5 10 15
   Val Val Ser Leu Ser Asn Gly Val Ser Val Leu Thr Ser Lys Val Leu
   20 25 30
   Asp
<210> 1442
   <211> 33
   <212> PRT
   <213> Artificial Sequence
<220>
   <223> Description of Artificial Sequence: Synthetic Peptide
<400> 1442
   Gly Glu Val Asn Lys Ile Ala Leu Leu Ser Thr Asn Lys Ala Val Val
   1 5 10 15
   Ser Leu Ser Asn Gly Val Ser Val Leu Thr Ser Lys Val Leu Asp Leu
   20 25 30
   Lys
<210> 1443
   <211> 33
   <212> PRT
   <213> Artificial Sequence
<220>
   <223> Description of Artificial Sequence: Synthetic Peptide
<400> 1443
   Glu Val Asn Lys Ile Ala Leu Leu Ser Thr Asn Lys Ala Val Val Ser
   1 5 10 15
   Leu Ser Asn Gly Val Ser Val Leu Thr Ser Lys Val Leu Asp Leu Lys
   20 25 30
   Asn
<210> 1444
   <211> 33
   <212> PRT
   <213> Artificial Sequence
<220>
   <223> Description of Artificial Sequence: Synthetic Peptide
<400> 1444
   Val Asn Lys Ile Ala Leu Leu Ser Thr Asn Lys Ala Val Val Ser Leu
   1 5 10 15
   Ser Asn Gly Val Ser Val Leu Thr Ser Lys Val Leu Asp Leu Lys Asn
   20 25 30
   Tyr
<210> 1445
   <211> 33
   <212> PRT
   <213> Artificial Sequence
<220>
   <223> Description of Artificial Sequence: Synthetic Peptide
<400> 1445
   Asn Lys Ile Ala Leu Leu Ser Thr Asn Lys Ala Val Val Ser Leu Ser
   1 5 10 15
   Asn Gly Val Ser Val Leu Thr Ser Lys Val Leu Asp Leu Lys Asn Tyr
   20 25 30
   Ile
<210> 1446
   <211> 33
   <212> PRT
   <213> Artificial Sequence
<220>
   <223> Description of Artificial Sequence: Synthetic Peptide
<400> 1446
   Lys Ile Ala Leu Leu Ser Thr Asn Lys Ala Val Val Ser Leu Ser Asn
   1 5 10 15
   Gly Val Ser Val Leu Thr Ser Lys Val Leu Asp Leu Lys Asn Tyr Ile
   20 25 30
   Asp
<210> 1447
   <211> 33
   <212> PRT
   <213> Artificial Sequence
<220>
   <223> Description of Artificial Sequence: Synthetic Peptide
<400> 1447
   Ile Ala Leu Leu Ser Thr Asn Lys Ala Val Val Ser Leu Ser Asn Gly
   1 5 10 15
   Val Ser Val Leu Thr Ser Lys Val Leu Asp Leu Lys Asn Tyr Ile Asp
   20 25 30
   Lys
<210> 1448
   <211> 33
   <212> PRT
   <213> Artificial Sequence
<220>
   <223> Description of Artificial Sequence: Synthetic Peptide
<400> 1448
   Ala Leu Leu Ser Thr Asn Lys Ala Val Val Ser Leu Ser Asn Gly Val
   1 5 10 15
   Ser Val Leu Thr Ser Lys Val Leu Asp Leu Lys Asn Tyr Ile Asp Lys
   20 25 30
   Gln
<210> 1449
   <211> 70
   <212> PRT
   <213> Artificial Sequence
<220>
   <223> Description of Artificial Sequence: Synthetic Peptide
<400> 1449
   Gly Thr Ile Ala Leu Gly Val Ala Thr Ser Ala Gln Ile Thr Ala Ala
   1 5 10 15
   Val Ala Leu Val Glu Ala Lys Gln Ala Arg Ser Asp Ile Glu Lys Leu
   20 25 30
   Lys Glu Ala Ile Arg Asp Thr Asn Lys Ala Val Gln Ser Val Gln Ser
   35 40 45
   Ser Ile Gly Asn Leu Ile Val Ala Ile Lys Ser Val Gln Asp Tyr Val
   50 55 60
   Asn Lys Glu Ile Val Pro
   65 70
<210> 1450
   <211> 56
   <212> PRT
   <213> Artificial Sequence
<220>
   <223> Description of Artificial Sequence: Synthetic Peptide
<400> 1450
   Tyr Thr Pro Asn Asp Ile Thr Leu Asn Asn Ser Val Ala Leu Asp Pro
   1 5 10 15
   Ile Asp Ile Ser Ile Glu Leu Asn Lys Ala Lys Ser Asp Leu Glu Glu
   20 25 30
   Ser Lys Glu Trp Ile Arg Arg Ser Asn Gln Lys Leu Asp Ser Ile Gly
   35 40 45
   Asn Trp His Gln Ser Ser Thr Thr
   50 55
<210> 1451
   <211> 35
   <212> PRT
   <213> Artificial Sequence
<220>
   <223> Description of Artificial Sequence: Synthetic Peptide
<400> 1451
   Thr Leu Asn Asn Ser Val Ala Leu Asp Pro Ile Asp Ile Ser Ile Glu
   1               5                   10                  15
   Leu Asn Lys Ala Lys Ser Asp Leu Glu Glu Ser Lys Glu Trp Ile Arg
               20                  25                  30
   Arg Ser Asn
           35
<210> 1452
   <211> 35
   <212> PRT
   <213> Artificial Sequence
<220>
   <223> Description of Artificial Sequence: Synthetic Peptide
<400> 1452
   Leu Asn Asn Ser Val Ala Leu Asp Pro Ile Asp Ile Ser Ile Glu Leu
   1               5                   10                  15
   Asn Lys Ala Lys Ser Asp Leu Glu Glu Ser Lys Glu Trp Ile Arg Arg
               20                  25                  30
   Ser Asn Gln
           35
<210> 1453
   <211> 35
   <212> PRT
   <213> Artificial Sequence
<220>
   <223> Description of Artificial Sequence: Synthetic Peptide
<400> 1453
   Asn Asn Ser Val Ala Leu Asp Pro Ile Asp Ile Ser Ile Glu Leu Asn
   1               5                   10                  15
   Lys Ala Lys Ser Asp Leu Glu Glu Ser Lys Glu Trp Ile Arg Arg Ser
               20                  25                  30
   Asn Gln Lys
           35
<210> 1454
   <211> 35
   <212> PRT
   <213> Artificial Sequence
<220>
   <223> Description of Artificial Sequence: Synthetic Peptide
<400> 1454
   Asn Ser Val Ala Leu Asp Pro Ile Asp Ile Ser Ile Glu Leu Asn Lys
   1 5 10 15
   Ala Lys Ser Asp Leu Glu Glu Ser Lys Glu Trp Ile Arg Arg Ser Asn
   20 25 30
   Gln Lys Leu
   35
<210> 1455
   <211> 35
   <212> PRT
   <213> Artificial Sequence
<220>
   <223> Description of Artificial Sequence: Synthetic Peptide
<400> 1455
   Ser Val Ala Leu Asp Pro Ile Asp Ile Ser Ile Glu Leu Asn Lys Ala
   1 5 10 15
   Lys Ser Asp Leu Glu Glu Ser Lys Glu Trp Ile Arg Arg Ser Asn Gln
   20 25 30
   Lys Leu Asp
   35
<210> 1456
   <211> 35
   <212> PRT
   <213> Artificial Sequence
<220>
   <223> Description of Artificial Sequence: Synthetic Peptide
<400> 1456
   Val Ala Leu Asp Pro Ile Asp Ile Ser Ile Glu Leu Asn Lys Ala Lys
   1 5 10 15
   Ser Asp Leu Glu Glu Ser Lys Glu Trp Ile Arg Arg Ser Asn Gln Lys
   20 25 30
   Leu Asp Ser
   35
<210> 1457
   <211> 35
   <212> PRT
   <213> Artificial Sequence
<220>
   <223> Description of Artificial Sequence: Synthetic Peptide
<400> 1457
   Ala Leu Asp Pro Ile Asp Ile Ser Ile Glu Leu Asn Lys Ala Lys Ser
   1 5 10 15
   Asp Leu Glu Glu Ser Lys Glu Trp Ile Arg Arg Ser Asn Gln Lys Leu
   20 25 30
   Asp Ser Ile
   35
<210> 1458
   <211> 35
   <212> PRT
   <213> Artificial Sequence
<220>
   <223> Description of Artificial Sequence: Synthetic Peptide
<400> 1458
   Leu Asp Pro Ile Asp Ile Ser Ile Glu Leu Asn Lys Ala Lys Ser Asp
   1 5 10 15
   Leu Glu Glu Ser Lys Glu Trp Ile Arg Arg Ser Asn Gln Lys Leu Asp
   20 25 30
   Ser Ile Gly
   35
<210> 1459
   <211> 35
   <212> PRT
   <213> Artificial Sequence
<220>
   <223> Description of Artificial Sequence: Synthetic Peptide
<400> 1459
   Asp Pro Ile Asp Ile Ser Ile Glu Leu Asn Lys Ala Lys Ser Asp Leu
   1 5 10 15
   Glu Glu Ser Lys Glu Trp Ile Arg Arg Ser Asn Gln Lys Leu Asp Ser
   20 25 30
   Ile Gly Asn
   35
<210> 1460
   <211> 35
   <212> PRT
   <213> Artificial Sequence
<220>
   <223> Description of Artificial Sequence: Synthetic Peptide
<400> 1460
   Pro Ile Asp Ile Ser Ile Glu Leu Asn Lys Ala Lys Ser Asp Leu Glu
   1 5 10 15
   Glu Ser Lys Glu Trp Ile Arg Arg Ser Asn Gln Lys Leu Asp Ser Ile
   20 25 30
   Gly Asn Trp
   35
<210> 1461
   <211> 35
   <212> PRT
   <213> Artificial Sequence
<220>
   <223> Description of Artificial Sequence: Synthetic Peptide
<400> 1461
   Ile Asp Ile Ser Ile Glu Leu Asn Lys Ala Lys Ser Asp Leu Glu Glu
   1 5 10 15
   Ser Lys Glu Trp Ile Arg Arg Ser Asn Gln Lys Leu Asp Ser Ile Gly
   20 25 30
   Asn Trp His
   35
<210> 1462
   <211> 35
   <212> PRT
   <213> Artificial Sequence
<220>
   <223> Description of Artificial Sequence: Synthetic Peptide
<400> 1462
   Asp Ile Ser Ile Glu Leu Asn Lys Ala Lys Ser Asp Leu Glu Glu Ser
   1 5 10 15
   Lys Glu Trp Ile Arg Arg Ser Asn Gln Lys Leu Asp Ser Ile Gly Asn
   20 25 30
   Trp His Gln
   35
<210> 1463
   <211> 35
   <212> PRT
   <213> Artificial Sequence
<220>
   <223> Description of Artificial Sequence: Synthetic Peptide
<400> 1463
   Ile Ser Ile Glu Leu Asn Lys Ala Lys Ser Asp Leu Glu Glu Ser Lys
   1 5 10 15
   Glu Trp Ile Arg Arg Ser Asn Gln Lys Leu Asp Ser Ile Gly Asn Trp
   20 25 30
   His Gln Ser
   35
<210> 1464
   <211> 35
   <212> PRT
   <213> Artificial Sequence
<220>
   <223> Description of Artificial Sequence: Synthetic Peptide
<400> 1464
   Ser Ile Glu Leu Asn Lys Ala Lys Ser Asp Leu Glu Glu Ser Lys Glu
   1 5 10 15
   Trp Ile Arg Arg Ser Asn Gln Lys Leu Asp Ser Ile Gly Asn Trp His
   20 25 30
   Gln Ser Ser
   35
<210> 1465
   <211> 35
   <212> PRT
   <213> Artificial Sequence
<220>
   <223> Description of Artificial Sequence: Synthetic Peptide
<400> 1465
   Ile Glu Leu Asn Lys Ala Lys Ser Asp Leu Glu Glu Ser Lys Glu Trp
   1 5 10 15
   Ile Arg Arg Ser Asn Gln Lys Leu Asp Ser Ile Gly Asn Trp His Gln
   20 25 30
   Ser Ser Thr
   35
<210> 1466
   <211> 35
   <212> PRT
   <213> Artificial Sequence
<220>
   <223> Description of Artificial Sequence: Synthetic Peptide
<400> 1466
   Glu Leu Asn Lys Ala Lys Ser Asp Leu Glu Glu Ser Lys Glu Trp Ile
   1 5 10 15
   Arg Arg Ser Asn Gln Lys Leu Asp Ser Ile Gly Asn Trp His Gln Ser
   20 25 30
   Ser Thr Thr
   35
<210> 1467
   <211> 35
   <212> PRT
   <213> Artificial Sequence
<220>
   <223> Description of Artificial Sequence: Synthetic Peptide
<400> 1467
   Thr Ala Ala Val Ala Leu Val Glu Ala Lys Gln Ala Arg Ser Asp Ile
   1 5 10 15
   Glu Lys Leu Lys Glu Ala Ile Arg Asp Thr Asn Lys Ala Val Gln Ser
   20 25 30
   Val Gln Ser
   35
<210> 1468
   <211> 35
   <212> PRT
   <213> Artificial Sequence
<220>
   <223> Description of Artificial Sequence: Synthetic Peptide
<400> 1468
   Ala Val Ala Leu Val Glu Ala Lys Gln Ala Arg Ser Asp Ile Glu Lys
   1 5 10 15
   Leu Lys Glu Ala Ile Arg Asp Thr Asn Lys Ala Val Gln Ser Val Gln
   20 25 30
   Ser Ser Ile
   35
<210> 1469
   <211> 35
   <212> PRT
   <213> Artificial Sequence
<220>
   <223> Description of Artificial Sequence: Synthetic Peptide
<400> 1469
   Leu Val Glu Ala Lys Gln Ala Arg Ser Asp Ile Glu Lys Leu Lys Glu
   1 5 10 15
   Ala Ile Arg Asp Thr Asn Lys Ala Val Gln Ser Val Gln Ser Ser Ile
   20 25 30
   Gly Asn Leu
   35
<210> 1470
   <211> 35
   <212> PRT
   <213> Artificial Sequence
<220>
   <223> Description of Artificial Sequence: Synthetic Peptide
<400> 1470
   Val Glu Ala Lys Gln Ala Arg Ser Asp Ile Glu Lys Leu Lys Glu Ala
   1 5 10 15
   Ile Arg Asp Thr Asn Lys Ala Val Gln Ser Val Gln Ser Ser Ile Gly
   20 25 30
   Asn Leu Ile
   35
<210> 1471
   <211> 35
   <212> PRT
   <213> Artificial Sequence
<220>
   <223> Description of Artificial Sequence: Synthetic Peptide
<400> 1471
   Glu Ala Lys Gln Ala Arg Ser Asp Ile Glu Lys Leu Lys Glu Ala Ile
   1 5 10 15
   Arg Asp Thr Asn Lys Ala Val Gln Ser Val Gln Ser Ser Ile Gly Asn
   20 25 30
   Leu Ile Val
   35
<210> 1472
   <211> 35
   <212> PRT
   <213> Artificial Sequence
<220>
   <223> Description of Artificial Sequence: Synthetic Peptide
<400> 1472
   Ala Lys Gln Ala Arg Ser Asp Ile Glu Lys Leu Lys Glu Ala Ile Arg
   1 5 10 15
   Asp Thr Asn Lys Ala Val Gln Ser Val Gln Ser Ser Ile Gly Asn Leu
   20 25 30
   Ile Val Ala
   35
<210> 1473
   <211> 35
   <212> PRT
   <213> Artificial Sequence
<220>
   <223> Description of Artificial Sequence: Synthetic Peptide
<400> 1473
   Lys Gln Ala Arg Ser Asp Ile Glu Lys Leu Lys Glu Ala Ile Arg Asp
   1 5 10 15
   Thr Asn Lys Ala Val Gln Ser Val Gln Ser Ser Ile Gly Asn Leu Ile
   20 25 30
   Val Ala Ile
   35
<210> 1474
   <211> 35
   <212> PRT
   <213> Artificial Sequence
<220>
   <223> Description of Artificial Sequence: Synthetic Peptide
<400> 1474
   Gln Ala Arg Ser Asp Ile Glu Lys Leu Lys Glu Ala Ile Arg Asp Thr
   1 5 10 15
   Asn Lys Ala Val Gln Ser Val Gln Ser Ser Ile Gly Asn Leu Ile Val
   20 25 30
   Ala Ile Lys
   35
<210> 1475
   <211> 35
   <212> PRT
   <213> Artificial Sequence
<220>
   <223> Description of Artificial Sequence: Synthetic Peptide
<400> 1475
   Ala Arg Ser Asp Ile Glu Lys Leu Lys Glu Ala Ile Arg Asp Thr Asn
   1 5 10 15
   Lys Ala Val Gln Ser Val Gln Ser Ser Ile Gly Asn Leu Ile Val Ala
   20 25 30
   Ile Lys Ser
   35
<210> 1476
   <211> 35
   <212> PRT
   <213> Artificial Sequence
<220>
   <223> Description of Artificial Sequence: Synthetic Peptide
<400> 1476
   Arg Ser Asp Ile Glu Lys Leu Lys Glu Ala Ile Arg Asp Thr Asn Lys
   1 5 10 15
   Ala Val Gln Ser Val Gln Ser Ser Ile Gly Asn Leu Ile Val Ala Ile
   20 25 30
   Lys Ser Val
   35
<210> 1477
   <211> 35
   <212> PRT
   <213> Artificial Sequence
<220>
   <223> Description of Artificial Sequence: Synthetic Peptide
<400> 1477
   Ser Asp Ile Glu Lys Leu Lys Glu Ala Ile Arg Asp Thr Asn Lys Ala
   1 5 10 15
   Val Gln Ser Val Gln Ser Ser Ile Gly Asn Leu Ile Val Ala Ile Lys
   20 25 30
   Ser Val Gln
   35
<210> 1478
   <211> 35
   <212> PRT
   <213> Artificial Sequence
<220>
   <223> Description of Artificial Sequence: Synthetic Peptide
<400> 1478
   Lys Leu Lys Glu Ala Ile Arg Asp Thr Asn Lys Ala Val Gln Ser Val
   1 5 10 15
   Gln Ser Ser Ile Gly Asn Leu Ile Val Ala Ile Lys Ser Val Gln Asp
   20 25 30
   Tyr Val Asn
   35
<210> 1479
   <211> 35
   <212> PRT
   <213> Artificial Sequence
<220>
   <223> Description of Artificial Sequence: Synthetic Peptide
<400> 1479
   Leu Lys Glu Ala Ile Arg Asp Thr Asn Lys Ala Val Gln Ser Val Gln
   1 5 10 15
   Ser Ser Ile Gly Asn Leu Ile Val Ala Ile Lys Ser Val Gln Asp Tyr
   20 25 30
   Val Asn Lys
   35
<210> 1480
   <211> 35
   <212> PRT
   <213> Artificial Sequence
<220>
   <223> Description of Artificial Sequence: Synthetic Peptide
<400> 1480
   Ala Ile Arg Asp Thr Asn Lys Ala Val Gln Ser Val Gln Ser Ser Ile
   1 5 10 15
   Gly Asn Leu Ile Val Ala Ile Lys Ser Val Gln Asp Tyr Val Asn Lys
   20 25 30
   Glu Ile Val
   35
<210> 1481
   <211> 47
   <212> PRT
   <213> Artificial Sequence
<220>
   <223> Description of Artificial Sequence: Synthetic Peptide
<400> 1481
   Thr Trp Gln Glu Trp Glu Arg Lys Val Asp Phe Leu Glu Glu Asn Ile
   1 5 10 15
   Thr Ala Leu Leu Glu Glu Ala Gln Ile Gln Gln Glu Lys Asn Met Tyr
   20 25 30
   Glu Leu Gln Lys Leu Asn Ser Trp Asp Val Phe Gly Asn Trp Phe
   35 40 45
<210> 1482
   <211> 35
   <212> PRT
   <213> Artificial Sequence
<220>
   <223> Description of Artificial Sequence: Synthetic Peptide
<400> 1482
   Trp Gln Glu Trp Glu Arg Lys Val Asp Phe Leu Glu Glu Asn Ile Thr
   1 5 10 15
   Ala Leu Leu Glu Glu Ala Gln Ile Gln Gln Glu Lys Asn Met Tyr Glu
   20 25 30
   Leu Gln Lys
   35
<210> 1483
   <211> 35
   <212> PRT
   <213> Artificial Sequence
<220>
   <223> Description of Artificial Sequence: Synthetic Peptide
<400> 1483
   Gln Glu Trp Glu Arg Lys Val Asp Phe Leu Glu Glu Asn Ile Thr Ala
   1 5 10 15
   Leu Leu Glu Glu Ala Gln Ile Gln Gln Glu Lys Asn Met Tyr Glu Leu
   20 25 30
   Gln Lys Leu
   35
<210> 1484
   <211> 35
   <212> PRT
   <213> Artificial Sequence
<220>
   <223> Description of Artificial Sequence: Synthetic Peptide
<400> 1484
   Glu Trp Glu Arg Lys Val Asp Phe Leu Glu Glu Asn Ile Thr Ala Leu
   1 5 10 15
   Leu Glu Glu Ala Gln Ile Gln Gln Glu Lys Asn Met Tyr Glu Leu Gln
   20 25 30
   Lys Leu Asn
   35
<210> 1485
   <211> 35
   <212> PRT
   <213> Artificial Sequence
<220>
   <223> Description of Artificial Sequence: Synthetic Peptide
<400> 1485
   Trp Glu Arg Lys Val Asp Phe Leu Glu Glu Asn Ile Thr Ala Leu Leu
   1 5 10 15
   Glu Glu Ala Gln Ile Gln Gln Glu Lys Asn Met Tyr Glu Leu Gln Lys
   20 25 30
   Leu Asn Ser
   35
<210> 1486
   <211> 35
   <212> PRT
   <213> Artificial Sequence
<220>
   <223> Description of Artificial Sequence: Synthetic Peptide
<400> 1486
   Glu Arg Lys Val Asp Phe Leu Glu Glu Asn Ile Thr Ala Leu Leu Glu
   1 5 10 15
   Glu Ala Gln Ile Gln Gln Glu Lys Asn Met Tyr Glu Leu Gln Lys Leu
   20 25 30
   Asn Ser Trp
   35
<210> 1487
   <211> 35
   <212> PRT
   <213> Artificial Sequence
<220>
   <223> Description of Artificial Sequence: Synthetic Peptide
<400> 1487
   Arg Lys Val Asp Phe Leu Glu Glu Asn Ile Thr Ala Leu Leu Glu Glu
   1 5 10 15
   Ala Gln Ile Gln Gln Glu Lys Asn Met Tyr Glu Leu Gln Lys Leu Asn
   20 25 30
   Ser Trp Asp
   35
<210> 1488
   <211> 35
   <212> PRT
   <213> Artificial Sequence
<220>
   <223> Description of Artificial Sequence: Synthetic Peptide
<400> 1488
   Lys Val Asp Phe Leu Glu Glu Asn Ile Thr Ala Leu Leu Glu Glu Ala
   1 5 10 15
   Gln Ile Gln Gln Glu Lys Asn Met Tyr Glu Leu Gln Lys Leu Asn Ser
   20 25 30
   Trp Asp Val
   35
<210> 1489
   <211> 35
   <212> PRT
   <213> Artificial Sequence
<220>
   <223> Description of Artificial Sequence: Synthetic Peptide
<400> 1489
   Val Asp Phe Leu Glu Glu Asn Ile Thr Ala Leu Leu Glu Glu Ala Gln
   1 5 10 15
   Ile Gln Gln Glu Lys Asn Met Tyr Glu Leu Gln Lys Leu Asn Ser Trp
   20 25 30
   Asp Val Phe
   35
<210> 1490
   <211> 35
   <212> PRT
   <213> Artificial Sequence
<220>
   <223> Description of Artificial Sequence: Synthetic Peptide
<400> 1490
   Asp Phe Leu Glu Glu Asn Ile Thr Ala Leu Leu Glu Glu Ala Gln Ile
   1 5 10 15
   Gln Gln Glu Lys Asn Met Tyr Glu Leu Gln Lys Leu Asn Ser Trp Asp
   20 25 30
   Val Phe Gly
   35
<210> 1491
   <211> 35
   <212> PRT
   <213> Artificial Sequence
<220>
   <223> Description of Artificial Sequence: Synthetic Peptide
<400> 1491
   Phe Leu Glu Glu Asn Ile Thr Ala Leu Leu Glu Glu Ala Gln Ile Gln
   1 5 10 15
   Gln Glu Lys Asn Met Tyr Glu Leu Gln Lys Leu Asn Ser Trp Asp Val
   20 25 30
   Phe Gly Asn
   35
<210> 1492
   <211> 35
   <212> PRT
   <213> Artificial Sequence
<220>
   <223> Description of Artificial Sequence: Synthetic Peptide
<400> 1492
   Pro Asp Ala Val Tyr Leu His Arg Ile Asp Leu Gly Pro Pro Ile Ser
   1 5 10 15
   Leu Glu Arg Leu Asp Val Gly Thr Asn Leu Gly Asn Ala Ile Ala Lys
   20 25 30
   Leu Glu Asp
   35
<210> 1493
   <211> 34
   <212> PRT
   <213> Artificial Sequence
<220>
   <223> Description of Artificial Sequence: Synthetic Peptide
<400> 1493
   Leu Glu Arg Leu Asp Val Gly Thr Asn Leu Gly Asn Ala Ile Ala Lys
   1 5 10 15
   Leu Glu Ala Lys Glu Leu Leu Glu Ser Ser Asp Gln Ile Leu Arg Ser
   20 25 30
   Met Lys
<210> 1494
   <211> 34
   <212> PRT
   <213> Artificial Sequence
<220>
   <223> Description of Artificial Sequence: Synthetic Peptide
<400> 1494
   Leu His Arg Ile Asp Leu Gly Pro Pro Ile Ser Leu Glu Arg Leu Asp
   1 5 10 15
   Val Gly Thr Asn Leu Gly Asn Ala Ile Ala Lys Leu Glu Ala Lys Glu
   20 25 30
   Leu Leu
<210> 1495
   <211> 34
   <212> PRT
   <213> Artificial Sequence
<220>
   <223> Description of Artificial Sequence: Synthetic Peptide
<400> 1495
   His Arg Ile Asp Leu Gly Pro Pro Ile Ser Leu Glu Arg Leu Asp Val
   1 5 10 15
   Gly Thr Asn Leu Gly Asn Ala Ile Ala Lys Leu Glu Ala Lys Glu Leu
   20 25 30
   Leu Glu
<210> 1496
   <211> 34
   <212> PRT
   <213> Artificial Sequence
<220>
   <223> Description of Artificial Sequence: Synthetic Peptide
<400> 1496
   Arg Ile Asp Leu Gly Pro Pro Ile Ser Leu Glu Arg Leu Asp Val Gly
   1 5 10 15
   Thr Asn Leu Gly Asn Ala Ile Ala Lys Leu Glu Ala Lys Glu Leu Leu
   20 25 30
   Glu Ser

<210> 1497
   <211> 34
   <212> PRT
   <213> Artificial Sequence
<220>
   <223> Description of Artificial Sequence: Synthetic Peptide
<400> 1497
   Ile Asp Leu Gly Pro Pro Ile Ser Leu Glu Arg Leu Asp Val Gly Thr
   1 5 10 15
   Asn Leu Gly Asn Ala Ile Ala Lys Leu Glu Ala Lys Glu Leu Leu Glu
   20 25 30
   Ser Ser
<210> 1498
   <211> 34
   <212> PRT
   <213> Artificial Sequence
<220>
   <223> Description of Artificial Sequence: Synthetic Peptide
<400> 1498
   Asp Leu Gly Pro Pro Ile Ser Leu Glu Arg Leu Asp Val Gly Thr Asn
   1 5 10 15
   Leu Gly Asn Ala Ile Ala Lys Leu Glu Ala Lys Glu Leu Leu Glu Ser
   20 25 30
   Ser Asp
<210> 1499
   <211> 34
   <212> PRT
   <213> Artificial Sequence
<220>
   <223> Description of Artificial Sequence: Synthetic Peptide
<400> 1499
   Leu Gly Pro Pro Ile Ser Leu Glu Arg Leu Asp Val Gly Thr Asn Leu
   1 5 10 15
   Gly Asn Ala Ile Ala Lys Leu Glu Ala Lys Glu Leu Leu Glu Ser Ser
   20 25 30
   Asp Gln
<210> 1500
   <211> 34
   <212> PRT
   <213> Artificial Sequence
<220>
   <223> Description of Artificial Sequence: Synthetic Peptide
<400> 1500
   Gly Pro Pro Ile Ser Leu Glu Arg Leu Asp Val Gly Thr Asn Leu Gly
   1 5 10 15
   Asn Ala Ile Ala Lys Leu Glu Ala Lys Glu Leu Leu Glu Ser Ser Asp
   20 25 30
   Gln Ile
<210> 1501
   <211> 34
   <212> PRT
   <213> Artificial Sequence
<220>
   <223> Description of Artificial Sequence: Synthetic Peptide
<400> 1501
   Pro Pro Ile Ser Leu Glu Arg Leu Asp Val Gly Thr Asn Leu Gly Asn
   1 5 10 15
   Ala Ile Ala Lys Leu Glu Ala Lys Glu Leu Leu Glu Ser Ser Asp Gln
   20 25 30
   Ile Leu
<210> 1502
   <211> 34
   <212> PRT
   <213> Artificial Sequence
<220>
   <223> Description of Artificial Sequence: Synthetic Peptide
<400> 1502
   Pro Ile Ser Leu Glu Arg Leu Asp Val Gly Thr Asn Leu Gly Asn Ala
   1 5 10 15
   Ile Ala Lys Leu Glu Ala Lys Glu Leu Leu Glu Ser Ser Asp Gln Ile
   20 25 30
   Leu Arg
<210> 1503
   <211> 34
   <212> PRT
   <213> Artificial Sequence
<220>
   <223> Description of Artificial Sequence: Synthetic Peptide
<400> 1503
   Ser Leu Glu Arg Leu Asp Val Gly Thr Asn Leu Gly Asn Ala Ile Ala
   1 5 10 15
   Lys Leu Glu Ala Lys Glu Leu Leu Glu Ser Ser Asp Gln Ile Leu Arg
   20 25 30
   Ser Met
<210> 1504
   <211> 34
   <212> PRT
   <213> Artificial Sequence
<220>
   <223> Description of Artificial Sequence: Synthetic Peptide
<400> 1504
   Leu Glu Arg Leu Asp Val Gly Thr Asn Leu Gly Asn Ala Ile Ala Lys
   1 5 10 15
   Leu Glu Ala Lys Glu Leu Leu Glu Ser Ser Asp Gln Ile Leu Arg Ser
   20 25 30
   Met Lys
<210> 1505
   <211> 22
   <212> PRT
   <213> Artificial Sequence
<220>
   <223> Description of Artificial Sequence: Synthetic Peptide
<400> 1505
   Tyr Leu Cys Glu Phe Cys Leu Lys Tyr Gly Arg Ser Leu Lys Cys Leu
   1 5 10 15
   Gln Arg His Leu Thr Lys
   20
<210> 1506
   <211> 8
   <212> PRT
   <213> Artificial Sequence
<220>
   <223> Description of Artificial Sequence: Synthetic Peptide
<400> 1506
   Ala Ser Thr Thr Thr Asn Tyr Thr
   1 5
<210> 1507
   <211> 8
   <212> PRT
   <213> Artificial Sequence
<220>
   <223> Description of Artificial Sequence: Synthetic Peptide
<400> 1507
   Ala Ser Thr Thr Thr Asn Tyr Thr
   1 5
<210> 1508
   <211> 8
   <212> PRT
   <213> Artificial Sequence
<220>
   <223> Description of Artificial Sequence: Synthetic Peptide
<400> 1508
   Ser Gln Asn Tyr Pro Ile Val Gln
   1 5
<210> 1509
   <211> 6
   <212> PRT
   <213> Artificial Sequence
<220>
   <223> Description of Artificial Sequence: Synthetic Peptide
<400> 1509
   Glu Leu Asp Lys Trp Ala
   1 5
<210> 1510
   <211> 11
   <212> PRT
   <213> Artificial Sequence
<220>
   <223> Description of Artificial Sequence: Synthetic Peptide
<400> 1510
   Arg Gly Val Val Asn Ala Ser Ser Arg Leu Ala
   1 5 10
<210> 1511
   <211> 10
   <212> PRT
   <213> Artificial Sequence
<220>
   <223> Description of Artificial Sequence: Synthetic Peptide
<400> 1511
   Arg Gly Arg Arg Gln Pro Ile Pro Lys Ala
   1 5 10
<210> 1512
   <211> 23
   <212> PRT
   <213> Artificial Sequence
<220>
   <223> Description of Artificial Sequence: Synthetic Peptide
<400> 1512
   Leu Thr Thr Gly Ser Val Val Ile Val Gly Arg Ile Ile Leu Ser Gly
   1 5 10 15
   Arg Pro Ala Val Val Pro Asp
   20
<210> 1513
   <211> 14
   <212> PRT
   <213> Artificial Sequence
<220>
   <223> Description of Artificial Sequence: Synthetic Peptide
<400> 1513
   Gly Ser Val Val Ile Val Gly Arg Ile Ile Leu Ser Gly Arg
   1 5 10
<210> 1514
   <211> 27
   <212> PRT
   <213> Artificial Sequence
<220>
   <223> Description of Artificial Sequence: Synthetic Peptide
<400> 1514
   Pro Leu Gly Phe Phe Pro Asp His Gln Leu Asp Pro Ala Phe Gly Ala
   1 5 10 15
   Asn Ser Asn Asn Pro Asp Trp Asp Phe Asn Pro
   20 25
<210> 1515
   <211> 26
   <212> PRT
   <213> Artificial Sequence
<220>
   <223> Description of Artificial Sequence: Synthetic Peptide
<400> 1515
   Met Gln Trp Asn Ser Thr Thr Phe His Gln Thr Leu Gln Asp Pro Arg
   1 5 10 15
   Val Arg Gly Leu Tyr Phe Pro Ala Gly Gly
   20 25
<210> 1516
   <211> 11
   <212> PRT
   <213> Artificial Sequence
<220>
   <223> Description of Artificial Sequence: Synthetic Peptide
<400> 1516
   Met Gln Trp Asn Ser Thr Ala Phe His Gln Thr
   1 5 10
<210> 1517
   <211> 8
   <212> PRT
   <213> Artificial Sequence
<220>
   <223> Description of Artificial Sequence: Synthetic Peptide
<400> 1517
   Tyr Gly Ala Val Val Asn Asp Leu
   1 5
<210> 1518
   <211> 21
   <212> PRT
   <213> Artificial Sequence
<220>
   <223> Description of Artificial Sequence: Synthetic Peptide
<400> 1518
   Cys Thr His Gly Ile Arg Pro Val Val Ser Thr Gln Leu Leu Leu Asn
   1 5 10 15
   Gly Ser Leu Ala Glu
   20
<210> 1519
   <211> 7
   <212> PRT
   <213> Artificial Sequence
<220>
   <223> Description of Artificial Sequence: Synthetic Peptide
<400> 1519
   Ser Glu Asn Tyr Pro Ile Val
   1 5
<210> 1520
   <211> 7
   <212> PRT
   <213> Artificial Sequence
<220>
   <223> Description of Artificial sequence: Synthetic Peptide
<400> 1520
   Lys Ala Arg Val Phe Glu Ala
   1 5
<210> 1521
   <211> 15
   <212> PRT
   <213> Artificial Sequence
<220>
   <223> Description of Artificial Sequence: Synthetic Peptide
<400> 1521
   Arg Ile Gln Arg Gly Pro Gly Arg Ala Phe Val Thr Ile Gly Lys
   1 5 10 15
<210> 1522
   <211> 8
   <212> PRT
   <213> Artificial Sequence
<220>
   <223> Description of Artificial Sequence: Synthetic Peptide
<400> 1522
   Ala Ser Thr Thr Thr Asn Tyr Thr
   1 5
<210> 1523
   <211> 8
   <212> PRT
   <213> Artificial Sequence
<220>
   <223> Description of Artificial Sequence: Synthetic Peptide
<400> 1523
   Ala Ser Thr Thr Thr Asn Tyr Thr
   1 5
<210> 1524
   <211> 15
   <212> PRT
   <213> Artificial Sequence
<220>
   <223> Description of Artificial Sequence: Synthetic Peptide
<400> 1524
   Arg Ile Gln Arg Gly Pro Gly Arg Ala Phe Val Thr Ile Gly Lys
   1 5 10 15
<210> 1525
   <211> 38
   <212> PRT
   <213> Artificial Sequence
<220>
   <223> Description of Artificial Sequence: Synthetic Peptide
<400> 1525
   Asn Asn Leu Leu Arg Ala Ile Glu Ala Gln Gln His Leu Leu Gln Leu
   1 5 10 15
   Thr Val Trp Gly Ile Lys Gln Leu Gln Ala Arg Ile Leu Ala Val Glu
   20 25 30
   Arg Tyr Leu Lys Asp Gln
   35
<210> 1526
   <211> 10
   <212> PRT
   <213> Artificial Sequence
<220>
   <223> Description of Artificial Sequence: Synthetic Peptide
<400> 1526
   Arg Gly Pro Gly Arg Ala Phe Val Thr Ile
   1 5 10
<210> 1527
   <211> 3
   <212> PRT
   <213> Artificial Sequence
<220>
   <223> Description of Artificial Sequence: Synthetic Peptide
<400> 1527
   Phe Phe Gly
   1
<210> 1528
   <211> 36
   <212> PRT
   <213> Artificial Sequence
<220>
   <223> Description of Artificial Sequence: Synthetic Peptide
<400> 1528
   Tyr Thr Ser Leu Ile His Ser Leu Ile Glu Glu Ser Gln Asn Gln Gln
   1 5 10 15
   Glu Lys Asn Glu Gln Glu Leu Leu Glu Leu Asp Lys Trp Ala Ser Leu
   20 25 30
   Trp Asn Trp Phe
   35
<210> 1529
   <211> 2
   <212> PRT
   <213> Artificial Sequence
<220>
   <223> Description of Artificial Sequence: Synthetic Peptide
<400> 1529
   Thr Gln
   1
<210> 1530
   <211> 6
   <212> PRT
   <213> Artificial Sequence
<220>
   <223> Description of Artificial Sequence: Synthetic Peptide
<400> 1530
   Trp His Trp Leu Gln Leu
   1 5
<210> 1531
   <211> 5
   <212> PRT
   <213> Artificial Sequence
<220>
   <223> Description of Artificial Sequence: Synthetic Peptide
<400> 1531
   Gly Pro Gly Ala Gly
   1 5
<210> 1532
   <211> 2
   <212> PRT
   <213> Artificial Sequence
<220>
   <223> Description of Artificial Sequence: Synthetic Peptide
<400> 1532
   His Gly
   1
<210> 1533
   <211> 5
   <212> PRT
   <213> Artificial Sequence
<220>
   <223> Description of Artificial Sequence: Synthetic Peptide
<400> 1533
   Phe Val Phe Leu Met
   1 5
<210> 1534
   <211> 3
   <212> PRT
   <213> Artificial Sequence
<220>
   <223> Description of Artificial Sequence: Synthetic Peptide
<400> 1534
   Thr Ser Lys
   1
<210> 1535
   <211> 3
   <212> PRT
   <213> Artificial Sequence
<220>
   <223> Description of Artificial Sequence: Synthetic Peptide
<400> 1535
   Lys His Gly
   1
<210> 1536
   <211> 16
   <212> PRT
   <213> Artificial Sequence
<220>
   <223> Description of Artificial Sequence: Synthetic Peptide
<400> 1536
   Lys Asn Arg Trp Glu Asp Pro Gly Lys Gln Leu Tyr Asn Val Glu Ala
   1 5 10 15
<210> 1537
   <211> 10
   <212> PRT
   <213> Artificial Sequence
<220>
   <223> Description of Artificial Sequence: Synthetic Peptide
<400> 1537
   Leu Arg Ala His Ala Val Asp Val Asn Gly
   1 5 10
<210> 1538
   <211> 14
   <212> PRT
   <213> Artificial Sequence
<220>
   <223> Description of Artificial Sequence: Synthetic Peptide
<400> 1538
   Trp Ser Lys Met Asp Gln Leu Ala Lys Glu Leu Thr Ala Glu
   1 5 10
<210> 1539
   <211> 4
   <212> PRT
   <213> Artificial Sequence
<220>
   <223> Description of Artificial Sequence: Synthetic Peptide
<400> 1539
   Thr Pro Arg Lys
   1
<210> 1540
   <211> 12
   <212> PRT
   <213> Artificial Sequence
<220>
   <223> Description of Artificial Sequence: Synthetic Peptide
<400> 1540
   Gly Glu Leu Gln Asn Gln Leu Ile Arg Lys Ser Asn
   1 5 10
<210> 1541
   <211> 17
   <212> PRT
   <213> Artificial Sequence
<220>
   <223> Description of Artificial Sequence: Synthetic Peptide
<400> 1541
   Gly Glu Tyr Gln Lys Met Leu Asn Leu Arg Ala Glu Val Lys Lys Asn
   1 5 10 15
   Ala
<210> 1542
   <211> 9
   <212> PRT
   <213> Artificial Sequence
<220>
   <223> Description of Artificial Sequence: Synthetic Peptide
<400> 1542
   Pro Phe Cys Asn Ala Phe Thr Gly Cys
   1 5
<210> 1543
   <211> 8
   <212> PRT
   <213> Artificial Sequence
<220>
   <223> Description of Artificial Sequence: Synthetic Peptide
<400> 1543
   Glu Lys Ala His Asp Gly Gly Arg
   1 5
<210> 1544
   <211> 14
   <212> PRT
   <213> Artificial Sequence
<220>
   <223> Description of Artificial Sequence: Synthetic Peptide
<400> 1544
   Pro Phe Thr Arg Asn Tyr Tyr Val Arg Ala Val Leu His Leu
   1 5 10
<210> 1545
   <211> 39
   <212> PRT
   <213> Artificial Sequence
<220>
   <223> Description of Artificial Sequence: Synthetic Peptide
<400> 1545
   Ala Pro Arg Leu Pro Gln Cys Gln Gly Asp Asp Gln Glu Lys Cys Leu
   1 5 10 15
   Cys Asn Lys Asp Glu Cys Pro Pro Gly Gln Cys Arg Phe Pro Arg Gly
   20 25 30
   Asp Ala Asp Pro Tyr Cys Glu
   35
<210> 1546
   <211> 8
   <212> PRT
   <213> Artificial Sequence
<220>
   <223> Description of Artificial Sequence: Synthetic Peptide
<400> 1546
   Lys Gly Asp Glu Glu Ser Leu Ala
   1 5
<210> 1547
   <211> 9
   <212> PRT
   <213> Artificial Sequence
<220>
   <223> Description of Artificial Sequence: Synthetic Peptide
<400> 1547
   Trp Ala Gly Gly Asp Ala Ser Gly Glu
   1 5
<210> 1548
   <211> 20
   <212> PRT
   <213> Artificial Sequence
<220>
   <223> Description of Artificial Sequence: Synthetic Peptide
<400> 1548
   Gln Ala Thr Val Gly Asp Val Asn Thr Asp Arg Pro Gly Leu Leu Asp
   1 5 10 15
   Leu Lys Tyr Tyr
   20
<210> 1549
   <211> 17
   <212> PRT
   <213> Artificial Sequence
<220>
   <223> Description of Artificial Sequence: Synthetic Peptide
<400> 1549
   Thr Lys Arg Arg Ala Ile Gly Phe Lys Lys Leu Ala Glu Ala Val Lys
   1 5 10 15
   Cys
<210> 1550
   <211> 8
   <212> PRT
   <213> Artificial Sequence
<220>
   <223> Description of Artificial Sequence: Synthetic Peptide
<400> 1550
   Ser Ile Ile Asn Phe Glu Lys Leu
   1 5
<210> 1551
   <211> 6
   <212> PRT
   <213> Artificial Sequence
<220>
   <223> Description of Artificial Sequence: Synthetic Peptide
<400> 1551
   Phe Gln Val Val Cys Gly
   1 5
<210> 1552
   <211> 6
   <212> PRT
   <213> Artificial Sequence
<220>
   <223> Description of Artificial Sequence: Synthetic Peptide
<400> 1552
   Ala Arg Met Ala Pro Glu
   1 5
<210> 1553
   <211> 4
   <212> PRT
   <213> Artificial Sequence
<220>
   <223> Description of Artificial Sequence: Synthetic Peptide
<400> 1553
   Gly Gln Pro Arg
   1
<210> 1554
   <211> 3
   <212> PRT
   <213> Artificial Sequence
<220>
   <223> Description of Artificial Sequence: Synthetic Peptide
<400> 1554
   Thr Val Leu
   1
<210> 1555
   <211> 49
   <212> PRT
   <213> Artificial Sequence
<220>
   <223> Description of Artificial Sequence: Synthetic Peptide
<400> 1555
   Leu Leu Ser Lys Arg Gly His Cys Pro Arg Ile Leu Phe Arg Cys Pro
   1 5 10 15
   Leu Ser Asn Pro Ser Asn Lys Cys Trp Arg Asp Tyr Asp Cys Pro Gly
   20 25 30
   Val Lys Lys Cys Cys Glu Gly Phe Cys Gly Lys Asp Cys Leu Tyr Pro
   35 40 45
   Lys
<210> 1556
   <211> 10
   <212> PRT
   <213> Artificial Sequence
<220>
   <223> Description of Artificial Sequence: Synthetic Peptide
<400> 1556
   Gly Phe Asp Leu Asn Gly Gly Gly Val Gly
   1 5 10
<210> 1557
   <211> 18
   <212> PRT
   <213> Artificial Sequence
<220>
   <223> Description of Artificial Sequence: Synthetic Peptide
<400> 1557
   Ala Val Gln Ser Lys Pro Pro Ser Lys Arg Asp Pro Pro Lys Met Gln
   1 5 10 15
   Thr Asp
<210> 1558
   <211> 4
   <212> PRT
   <213> Artificial Sequence
<220>
   <223> Description of Artificial Sequence: Synthetic Peptide
<400> 1558
   Thr Lys Pro Arg
   1
<210> 1559
   <211> 11
   <212> PRT
   <213> Artificial Sequence
<220>
   <223> Description of Artificial Sequence: Synthetic Peptide
<400> 1559
   Tyr Leu Asn Phe Thr Pro Asn Trp Gly Thr Tyr
   1 5 10
<210> 1560
   <211> 5
   <212> PRT
   <213> Artificial Sequence
<220>
   <223> Description of Artificial Sequence: Synthetic Peptide
<400> 1560
   Asp Leu Trp Gln Lys
   1               5
<210> 1561
   <211> 17
   <212> PRT
   <213> Artificial Sequence
<220>
   <223> Description of Artificial Sequence: Synthetic Peptide
<400> 1561
   Trp Tyr Glu Pro Ile Tyr Leu Gly Gly Val Phe Gln Leu Glu Lys Gly
   1               5                   10                  15
   Asp
<210> 1562
   <211> 39
   <212> PRT
   <213> Artificial Sequence
<220>
   <223> Description of Artificial Sequence: Synthetic Peptide
<400> 1562
   Ala Pro Arg Leu Pro Gln Cys Gln Gly Asp Gln Glu Lys Cys Leu Cys
   1               5                   10                  15
   Asn Lys Asp Glu Cys Pro Pro Gly Gln Cys Arg Phe Pro Arg Gly Asp
               20                  25                  30
   Ala Asp Pro Tyr Cys Glu Asp
           35
<210> 1563
   <211> 4
   <212> PRT
   <213> Artificial Sequence
<220>
   <223> Description of Artificial Sequence: Synthetic Peptide
<400> 1563
   Thr Lys Pro Arg
   1
<210> 1564
   <211> 6
   <212> PRT
   <213> Artificial Sequence
<220>
   <223> Description of Artificial Sequence: Synthetic Peptide
<400> 1564
   Glu Glu Val Val Ala Cys
   1 5
<210> 1565
   <211> 4
   <212> PRT
   <213> Artificial Sequence
<220>
   <223> Description of Artificial Sequence: Synthetic Peptide
<400> 1565
   Ser Asp Lys Pro
   1
<210> 1566
   <211> 6
   <212> PRT
   <213> Artificial Sequence
<220>
   <223> Description of Artificial Sequence: Synthetic Peptide
<400> 1566
   Arg Phe Trp Ile Asn Lys
   1 5
<210> 1567
   <211> 13
   <212> PRT
   <213> Artificial Sequence
<220>
   <223> Description of Artificial Sequence: Synthetic Peptide
<400> 1567
   Cys Gly Tyr Gly Pro Lys Lys Lys Arg Lys Val Gly Gly
   1 5 10
<210> 1568
   <211> 1
   <212> PRT
   <213> Artificial Sequence
<220>
   <223> Description of Artificial Sequence: Synthetic Peptide
<400> 1568
   Leu
   1
<210> 1569
   <211> 5
   <212> PRT
   <213> Artificial Sequence
<220>
   <223> Description of Artificial Sequence: Synthetic Peptide
<400> 1569
   Asp Asp Asp Asp Asp
   1 5
<210> 1570
   <211> 6
   <212> PRT
   <213> Artificial Sequence
<220>
   <223> Description of Artificial Sequence: Synthetic Peptide
<400> 1570
   Asp Asp Asp Asp Asp Asp
   1 5
<210> 1571
   <211> 8
   <212> PRT
   <213> Artificial Sequence
<220>
   <223> Description of Artificial Sequence: Synthetic Peptide
<400> 1571
   Asn Pro Asn Ala Asn Pro Asn Ala
   1 5
<210> 1572
   <211> 8
   <212> PRT
   <213> Artificial Sequence
<220>
   <223> Description of Artificial Sequence: Synthetic Peptide
<400> 1572
   Val Ala Ile Thr Val Leu Val Lys
   1 5
<210> 1573
   <211> 6
   <212> PRT
   <213> Artificial Sequence
<220>
   <223> Description of Artificial Sequence: Synthetic Peptide
<400> 1573
   Val Gly Val Arg Val Arg
   1 5

<210> 1574
   <211> 4
   <212> PRT
   <213> Artificial Sequence
<220>
   <223> Description of Artificial Sequence: Synthetic Peptide
<400> 1574
   Val Ile His Ser
   1
<210> 1575
   <211> 5
   <212> PRT
   <213> Artificial Sequence
<220>
   <223> Description of Artificial Sequence: Synthetic Peptide
<400> 1575
   Val Pro Asp Pro Arg
   1 5
<210> 1576
   <211> 4
   <212> PRT
   <213> Artificial Sequence
<220>
   <223> Description of Artificial Sequence: Synthetic Peptide
<400> 1576
   Val Thr Cys Gly
   1
<210> 1577
   <211> 3
   <212> PRT
   <213> Artificial Sequence
<220>
   <223> Description of Artificial Sequence: Synthetic Peptide
<400> 1577
   Arg Ser Arg
   1
<210> 1578
   <211> 11
   <212> PRT
   <213> Artificial Sequence
<220>
   <223> Description of Artificial Sequence: Synthetic Peptide
<400> 1578
   Ser Ala Lys Leu Cys Pro Gly Gly Asn Cys Val
   1 5 10
<210> 1579
   <211> 6
   <212> PRT
   <213> Artificial Sequence
<220>
   <223> Description of Artificial Sequence: Synthetic Peptide
<400> 1579
   Asp His Ala Arg Trp Lys
   1 5
<210> 1580
   <211> 7
   <212> PRT
   <213> Artificial Sequence
<220>
   <223> Description of Artificial Sequence: Synthetic Peptide
<400> 1580
   Pro Gln Asp Pro Gln Asp Leu
   1 5
<210> 1581
   <211> 6
   <212> PRT
   <213> Artificial Sequence
<220>
   <223> Description of Artificial Sequence: Synthetic Peptide
<400> 1581
   Gln His Phe Arg Trp Gly
   1 5
<210> 1582
   <211> 19
   <212> PRT
   <213> Artificial Sequence
<220>
   <223> Description of Artificial Sequence: Synthetic Peptide
<400> 1582
   Asn Gln Glu Gln Val Ser Pro Leu Thr Leu Leu Lys Leu Gly Asn Gln
   1 5 10 15
   Glu Pro Gly
<210> 1583
   <211> 20
   <212> PRT
   <213> Artificial Sequence
<220>
   <223> Description of Artificial Sequence: Synthetic Peptide
<400> 1583
   Leu Ser Ala Leu Ser Leu Asp Glu Pro Phe Ile Gln Lys Asp Val Glu
   1 5 10 15
   Leu Arg Ile Met
   20
<210> 1584
   <211> 15
   <212> PRT
   <213> Artificial Sequence
<220>
   <223> Description of Artificial Sequence: Synthetic Peptide
<400> 1584
   Ala Pro Glu Ala Gln Val Ser Val Gln Pro Asn Phe Gln Gln Asp
   1 5 10 15
<210> 1585
   <211> 27
   <212> PRT
   <213> Artificial Sequence
<220>
   <223> Description of Artificial Sequence: Synthetic Peptide
<400> 1585
   Glu Tyr Gly Gly Thr Lys Val Leu Asp Asp Lys Asp Tyr Phe Leu Phe
   1 5 10 15
   Arg Asp Gly Asp Ile Leu Gly Lys Tyr Val Asp
   20 25
<210> 1586
   <211> 16
   <212> PRT
   <213> Artificial Sequence
<220>
   <223> Description of Artificial Sequence: Synthetic Peptide
<400> 1586
   Lys Ala Tyr Ile Asn Lys Val Glu Glu Leu Lys Lys Lys Tyr Gly Ile
   1 5 10 15
<210> 1587
   <211> 24
   <212> PRT
   <213> Artificial Sequence
<220>
   <223> Description of Artificial Sequence: Synthetic Peptide
<400> 1587
   Ser Gln Gln Ser Ser Ser Tyr Gly Gln Gln Ser Glu Lys Pro Tyr Gln
   1 5 10 15
   Cys Asp Phe Lys Asp Cys Glu Arg
   20
<210> 1588
   <211> 15
   <212> PRT
   <213> Artificial Sequence
<220>
   <223> Description of Artificial Sequence: Synthetic Peptide
<400> 1588
   Ala Thr Glu Ser Ile Ala Tyr Leu Ala Pro Pro Tyr Ala Phe Arg
   1 5 10 15
<210> 1589
   <211> 16
   <212> PRT
   <213> Artificial Sequence
<220>
   <223> Description of Artificial Sequence: Synthetic Peptide
<400> 1589
<210> 1590
   <211> 13
   <212> PRT
   <213> Artificial Sequence
<220>
   <223> Description of Artificial Sequence: Synthetic Peptide
<400> 1590
   Val Ser His Pro Tyr Ser Gln His Leu Glu Gly Lys Gly
   1 5 10
<210> 1591
   <211> 7
   <212> PRT
   <213> Artificial Sequence
<220>
   <223> Description of Artificial Sequence: Synthetic Peptide
<400> 1591
   Ala Leu Thr Asp Phe Phe Arg
   1 5
<210> 1592
   <211> 20
   <212> PRT
   <213> Artificial Sequence
<220>
   <223> Description of Artificial Sequence: Synthetic Peptide
<400> 1592
   Gln Ala Ile Gly Leu Met Gly Tyr Arg Leu Ser Pro Gln Thr Leu Thr
   1 5 10 15
   Thr Ile Val Lys
   20
<210> 1593
   <211> 17
   <212> PRT
   <213> Artificial Sequence
<220>
   <223> Description of Artificial Sequence: Synthetic Peptide
<400> 1593
   Met Gly Phe Asn Ala Phe Lys Glu Leu Trp Ala Ala Leu Asn Ala Trp
   1 5 10 15
   Lys
<210> 1594
   <211> 16
   <212> PRT
   <213> Artificial Sequence
<220>
   <223> Description of Artificial Sequence: Synthetic Peptide
<400> 1594
<210> 1595
   <211> 17
   <212> PRT
   <213> Artificial Sequence
<220>
   <223> Description of Artificial Sequence: Synthetic Peptide
<400> 1595
   Glu Leu Asp Ala Lys Ile Pro Ser Thr Gly Asp Ala Thr Glu Trp Arg
   1 5 10 15
   Asn
<210> 1596
   <211> 20
   <212> PRT
   <213> Artificial Sequence
<220>
   <223> Description of Artificial Sequence: Synthetic Peptide
<400> 1596
   Pro Asp Thr Arg Pro Ala Pro Gly Ser Thr Ala Pro Pro Ala His Gly
   1 5 10 15
   Val Thr Ser Ala
   20
<210> 1597
   <211> 20
   <212> PRT
   <213> Artificial Sequence
<220>
   <223> Description of Artificial Sequence: Synthetic Peptide
<400> 1597
   Gly Lys Glu Ile Leu Val Gly Asp Val Gly Gln Thr Val Asp Asp Pro
   1 5 10 15
   Tyr Ala Thr Phe
   20
<210> 1598
   <211> 15
   <212> PRT
   <213> Artificial Sequence
<220>
   <223> Description of Artificial Sequence: Synthetic Peptide
<400> 1598
   Glu Leu Ser Leu Ala Gly Asn Glu Leu Gly Asp Glu Gly Ala Arg
   1 5 10 15
<210> 1599
   <211> 22
   <212> PRT
   <213> Artificial Sequence
<220>
   <223> Description of Artificial Sequence: Synthetic Peptide
<400> 1599
   Met Phe Ile Val Asn Thr Asn Val Pro Arg Ala Ser Val Pro Asp Gly
   1 5 10 15
   Phe Leu Ser Glu Leu Thr
   20
<210> 1600
   <211> 35
   <212> PRT
   <213> Artificial Sequence
<220>
   <223> Description of Artificial Sequence: Synthetic Peptide
<400> 1600
<210> 1601
   <211> 27
   <212> PRT
   <213> Artificial Sequence
<220>
   <223> Description of Artificial Sequence: Synthetic Peptide
<400> 1601
   Gly Leu Pro Gly Arg Asp Gly Arg Asp Gly Arg Glu Gly Phe Arg Gly
   1 5 10 15
   Glu Glu Gly Asp Pro Gly Leu Pro Gly Ala Ala
   20 25
<210> 1602
   <211> 21
   <212> PRT
   <213> Artificial Sequence
<220>
   <223> Description of Artificial Sequence: Synthetic Peptide
<400> 1602
   Cys Lys Thr Cys Gln Arg Lys Phe Ser Arg Ser Gly His Leu Lys Thr
   1 5 10 15
   His Thr Arg Thr His
   20
<210> 1603
   <211> 15
   <212> PRT
   <213> Artificial Sequence
<220>
   <223> Description of Artificial Sequence: Synthetic Peptide
<400> 1603
   His Val Ser Ser Glu Ala Phe Arg Met Cys Asp Val Cys Leu Glu
   1 5 10 15
<210> 1604
   <211> 5
   <212> PRT
   <213> Artificial Sequence
<220>
   <223> Description of Artificial Sequence: Synthetic Peptide
<400> 1604
   Pro His Ser Arg Asn
   1 5
<210> 1605
   <211> 5
   <212> PRT
   <213> Artificial Sequence
<220>
   <223> Description of Artificial Sequence: Synthetic Peptide
<400> 1605
   Pro His Ser Cys Asn
   1 5
<210> 1606
   <211> 28
   <212> PRT
   <213> Artificial Sequence
<220>
   <223> Description of Artificial Sequence: Synthetic Peptide
<400> 1606
   Met Gly Pro Gly Ala Pro Phe Ala Arg Val Gly Trp Pro Leu Pro Leu
   1 5 10 15
   Leu Val Val Met Ala Gly Val Ala Pro Val Trp Ala
   20 25
<210> 1607
   <211> 15
   <212> PRT
   <213> Artificial Sequence
<220>
   <223> Description of Artificial Sequence: Synthetic Peptide
<400> 1607
   Met Val Lys Ser His Ile Gly Ser Trp Ile Leu Val Leu Phe Val
   1 5 10 15
<210> 1608
   <211> 9
   <212> PRT
   <213> Artificial Sequence
<220>
   <223> Description of Artificial Sequence: Synthetic Peptide
<400> 1608
   Cys Ser Leu Pro Gly Ser Ala Ala Ala
   1 5
<210> 1609
   <211> 15
   <212> PRT
   <213> Artificial Sequence
<220>
   <223> Description of Artificial Sequence: Synthetic Peptide
<400> 1609
   Arg Gly Leu Lys Arg Gln Ser Asp Glu Arg Lys Arg Asp Arg Glu
   1 5 10 15
<210> 1610
   <211> 57
   <212> PRT
   <213> Artificial Sequence
<220>
   <223> Description of Artificial Sequence: Synthetic Peptide
<400> 1610
   Gly Arg Ser His Met Val Leu Asn Ser Ala Leu Glu Gly Ala Arg Gly
   1 5 10 15
   Gly Pro Gly Gly Glu Glu Ile Pro Glu Arg Phe Ser Ile Pro Glu Leu
   20 25 30
   Gln Trp Met Leu Ser Asn Ala Glu Leu Ala Pro Val Gln Ala Asp Glu
   35 40 45
   Pro Pro Gln Ser Arg Met Asp Leu Val
   50 55
<210> 1611
   <211> 35
   <212> PRT
   <213> Artificial Sequence
<220>
   <223> Description of Artificial Sequence: Synthetic Peptide
<400> 1611
   Gln Ala Arg Ala Val Gly Leu Ala Gly Thr Ser Arg Ala Phe Leu Ser
   1 5 10 15
   Ser Arg Leu Gln Asp Leu Tyr Ser Ile Val Arg Arg Ala Asp Arg Ala
   20 25 30
   Ala Val Met
   35
<210> 1612
   <211> 15
   <212> PRT
   <213> Artificial Sequence
<220>
   <223> Description of Artificial Sequence: Synthetic Peptide
<400> 1612
   Met Val Lys Ser His Ile Gly Ser Trp Ile Leu Val Leu Phe Val
   1 5 10 15
<210> 1613
   <211> 29
   <212> PRT
   <213> Artificial Sequence
<220>
   <223> Description of Artificial Sequence: Synthetic Peptide
<400> 1613
   Leu Arg Asp Leu Val Ser Tyr Cys Arg Ala Arg Gly Lys Gly Arg Glu
   1 5 10 15
   Arg Met Asn Gly Thr Arg Lys Gly His Leu Leu Tyr Met
   20 25
<210> 1614
   <211> 33
   <212> PRT
   <213> Artificial Sequence
<220>
   <223> Description of Artificial Sequence: Synthetic Peptide
<400> 1614
   Gly Lys Arg Ser Ser Pro Glu Thr Leu Ile Ser Asp Leu Leu Met Arg
   1 5 10 15
   Glu Ser Thr Glu Asn Val Pro Arg Thr Arg Leu Glu Asp Pro Ala Met
   20 25 30
   Trp
<210> 1615
   <211> 30
   <212> PRT
   <213> Artificial Sequence
<220>
   <223> Description of Artificial Sequence: Synthetic Peptide
<400> 1615
   Asp Val Glu Pro Leu Leu Gly Phe Leu Ser Pro Lys Ser Gly Gln Glu
   1 5 10 15
   Asn Glu Val Asp Asp Phe Pro Tyr Lys Gly Gln Gly Glu Leu
   20 25 30
<210> 1616
   <211> 20
   <212> PRT
   <213> Artificial Sequence
<220>
   <223> Description of Artificial Sequence: Synthetic Peptide
<400> 1616
   Phe Cys Lys Cys Arg Leu Glu Pro Met Lys Ala Thr Cys Asp Ile Ser
   1 5 10 15
   Glu Cys Pro Glu
   20
<210> 1617
   <211> 20
   <212> PRT
   <213> Artificial Sequence
<220>
   <223> Description of Artificial Sequence: Synthetic Peptide
<400> 1617
   Lys Ser His Gly Arg Thr Gln Asn Pro Val Val His Phe Phe Lys Asn
    1               5                  10                  15
   Ile Val Thr Pro
               20